# EUROPEAN PATENT APPLICATION

(11) **EP 2 527 466 A2**
(43) Date of publication of application: **28.11.2012**
(21) Application number: 12153902.7
(22) Date of filing: 26.03.2008
(51) Int. Cl.: C12Q 1/68

(54) **Genetic variants on CHR2 as markers for use in breast cancer risk assessment, diagnosis, prognosis and treatment**

(30) Priority: 26.03.2007 IS 8625; 25.05.2007 IS 8648
(62) Divisional of application: 08720170.3
(71) Applicant: Decode Genetics EHF., 101 Reykjavik (IS)
(72) Inventor: Sulem, Patrick, 105 Reykjavik (IS); Manolescu, Andrei, 105 Reykjavik (IS); Stacey, Simon, 201 Kopavogur (IS)
(74) Representative: Arnason Faktor

(57) **Abstract**

The invention pertains to certain genetic variants on Chr2q35 and as susceptibility variants of breast cancer. Methods of risk assessment and diagnosis of increased and/or decreased susceptibility to breast cancer, using such variants are described. The invention further relates to kits for diagnosing a susceptibility to breast cancer.

SEQUENCE LISTING

<110> deCODE Genetics ehf

<120> GENETIC VARIANTS ON CHR2 AND CHR16 AS MARKERS FOR USE IN BREAST
      CANCER RISK ASSESSMENT, DIAGNOSIS, PROGNOSIS AND TREATMENT

<130> 2506-PC00

<160> 6

<170> PatentIn version 3.5

<210> 1
<211> 600
<212> DNA
<213> Homo sapiens
<400> 1
caggataaga ttatttctga ggctgcttcc atcagtggtt ctgtgagctg gcccatgtat 60
tcagccagtc agtaggccat caagcatgca tctacgcaag gcactagaag ggttacatac 120
aatgggaaac tgaggcccag aaggaggaaa gatgaagtgc ccaaagttaa ggagataaaa 180
gcagaagtgc aggtaagaga aggagtattc atattactgc aggaatccaa attctacaag 240
aacatacgct tgttctttaa agggagccct cgctttaagc attatttgcc gatcaatacc 300
mattaataat tacaggacat gggcaaatat tctagcaaag gtcctgaggt cttcctagat 360
gtgcccaaaa taacaaggac tccccccgca ggtggcacca aaagtcggag gatgtgttgg 420
aagctgacct tccccacctg tccctgcctc acccacagtc acccacccct ctaggatgta 480
atggtgaatc attcaacaca tgggccacct tgggaagtcg cttaacctct ctcccttttc 540
acgttataaa atgggcatag tcattacaca aatgcataaa tcaacctcaa ttttctcatc 600

<210> 2
<211> 600
<212> DNA
<213> Homo sapiens
<400> 2
ctgacatagc cgcctgtgca ggtttttggg accaggtctg ccatctgtat tcccaggctg 60
ggctggagca cctgaggcta ctatggttac agaaaggaac ctggcatccg aaggtatagg 120
ctcaaatccc agctctcatg attgctagct ttgaggtttc aggttcattt cttaagtgct 180
ctgagctttg gtgccctcct cttcaaaatg ggaatgagga gcatgtctag ctccctgttt 240
tgttgcagtg aatagctaca tgaagaacag ctaaaccaga acagaaagaa ggcaaatgga 300
rgctacagaa accaaggatt tccttgttga atcgaatctt ccttcaatct tccttcacca 360
cactagtgga tctccctgtg ggagggatgt tgagagtgct ccgtgttttt tttgtttttt 420
tttttttttt ttttgtgatg gagtctcact ctgtcaccca gactggaatg cagtggtgcg 480
atctcggctc actgcaacct ctgcctccca ggttcaagtg attctcctgc ctcagcttcc 540
caagtagcca ggactacagg catatgccat cacacccagc taatttttgt atttttagta 600

<210> 3
<211> 600
<212> DNA
<213> Homo sapiens
<400> 3
gctagtcctt ggctgttctg tgatcaaatg caattgtttc ccatttctta agaaacagaa 60
tctttattag actcagttct tttcttggtt atttttacaa ttttattgtt ctatggttat 120
taaaaaataa catgtcatat aaattaagcc tagaaacatg gatgtttatc aaagaaagga 180
ttgtcatcca aagcaccaac tatgagagat atctatgtgc aatggtatat agatctgtca 240
tagaagggtt taattatatc tgcctaatga ttttctctcc ttaatgcctc tatagctgtc 300
ycttagcgaa gaataaaact gtggactgac ccccacccat ttgcgaagaa agtactgggt 360
cttcagcttt cattgttcag ccggtggtct ttgtggacaa caccaggggc ccctcctgct 420
gaagactgtc tggacttcct gattttcact cctgtataaa ccccctcccc cattactacc 480
tttgtctcct tggggaaaaa aaatgttttt cacttaagca aaataataac tacccagtat 540
ttgtttctgg agtgaaattg taaacttcat tgtacattct tcctggcttt gatgcttatc 600

<210> 4
<211> 94080

<212>  DNA
<213>  Homo sapiens
<400>  4
ggttgacaca aacacgggaa gattctgcag gaaacacaag gcagccacgg cctctgtccc      60
tgagaggagc agtgagggag ccgcagggct gggctgcggc gtgacactag ggacgtgggg     120
ggaatgcaag tgggtccaga cctgggggcc accagtgcag gagcaagggc aggccggcca     180
tcagagctgg gcggggaggg gggcgctgag cttggcgtgg gagtttcccc aacggactgt     240
gtcttccagg gaccctcgcc cctcatgccc ctctgcgtcc tgaagggagg aggcaccacc     300
cctctgtggc atctgctttt tagggctgac gatggaacaa gctcatggac aggagtgaag     360
cccctggcac atagcaggtg ccacagaagg gtggtgaagt ctgccctaga tcagagatca     420
ttcagggggt ctgttaactt ggatgaagaa gaaatacact cttatcacta acatcaggaa     480
ggtaagcaag gaaccacaca tagaattgac aataagtgtg actttgccac caagaagaat     540
cacaaagttt tattttattt tattttattt tattttactt taagttctgg gatacatgtg     600
cagaatgtgc aggtttgtta cataggtata cttgtgccat ggtggtttgc tgcacccatc     660
aacccgttat ctaggtttta agccccgcac gcattaggta tttgtcctaa tgctctcctt     720
cccctcgctc cccaccccca aacaggcccc ggtgtgtgat gttcccctcc ctgtgtccat     780
gtgttctcat tgttcaactc ccacttatga gtgagaacat gtggtgttta gttttctgtt     840
cctgagaatc atagagtttt catagcccag tacaataatc acagatacat gacatagagt     900
tgtgtgacat gtagacccca atagtccggt taatgggatg ctttagttaa aaagtacata     960
gattactgta ttataagttt atgttttagg atattttgat gattactttt tagaaatagt    1020
ttcctttgta acctttattt tacttaatgc atatgaaaga atcattcaga ggaggggtcc    1080
atggcttcac caagatgaaa caggtcattg gcatgaaaaa ggttaaaacg ctgtgccctg    1140
gaaccaggag tttatgatcc aggcctgcag catgctgtat gaccttggcc acataacttc    1200
attgtgcctc agttgcctca actataaaat ggggataata aacctgtgac atatgatgct    1260
tggggggtta aatgtgacaa aagtcttagc actctgtgtg ccatacagtg agggccccag    1320
catgggcatt gatgcaccgt cttgattcct cctgcctttc ccaggtacag ggagcttccc    1380
cagcctgcct gcatctgaga agattccacc agagaaacag aaccaatagg agacatatat    1440
taagagttgt gttgcggcca ggcctggtgg cccatgcctg taatcccttt gggattacac    1500
tttgggatta cagcactttg ggaggccaaa gcgggcctat cacctgagtt caggagttcg    1560
agaccagcct gaccaacgtg gagaaacccc gtccctacta aaaatacaaa attagccagg    1620
cgtggtggca ggcatctgta atcccagcta ctcgggaggc tgaggcagga gaatcacttg    1680
aacttgggag gcggaggttg cagtgagctg agatggtgcc attgcactcc agtctgggca    1740
acaagagcaa aactccatct caaaaaaaaa aaagagagag atgtgttgca agaaatcggc    1800
ttacgtgacc gtgggggctg gatccatagc gcaggctctc aggaggggca gtccagaact    1860
ctcaggctga tgctgcaggc cacaggtggg atttcttctc aagaaagcct cagctctgct    1920
cctaaggcct ttcaactgat tgactcatgc ccactcagat tattgagggc tatctccttt    1980
atttaaggtt aaccggccca gtgcagtggc tcacgcctat aatcccagta ttttggcagg    2040
aagattgctt gaggccagga gtttgagaca ggactggaca acagagcaag agcctcatct    2100
ctacaaaaca taaaaaaatt agcccggtgt ggtggcacac tcctatactc ctagctactc    2160
tggaggccaa gccgggagga tcacttgagc ccaaaagttc aaggctatac tcctagctac    2220
tctggaggcc aagcggggag gatcacttga gcccaaaagt tcaaggctgc agtcagctat    2280
gatcaagcca cttatactcc agcccagaaa acagagtgag accctgtctc taaaacaaca    2340
acaacaaaaa accccaacca attgtagata tcaactacat tgctgtggct tgaatgtctc    2400
ctccaaaact caatctgaaa cttaatccct aatttaatag tattggcagg tgacagggcc    2460
tttcagaggt agttgggtca tgtgggcatt catggattaa tgggttaata gattaatggg    2520
ttatcatggg agtagattag ctatcacaag agtgagtctg ttacaaaaat ccagcttggc    2580
tctctcttgt gagcccctcc ccatacgatg ccctgcactg accattagca agaaagcctt    2640
catcagatgg accccctcaa ccttggacat ctcaatcttc agaactctaa gaaataaatt    2700
tcttttcttt ataaattgcc cagtctctgg tattcagtta tagcaacaga aaatggacta    2760
agatacacat ctacgagata cctccacagc aactcctaga ttagtgttga tggaataact    2820
ggggattgta gcctagccaa gtcgacacct aagaccggcc ctcatgtcac ccaaggacaa    2880
gacagcgagg tcctgaagag tttcatgaag aggaggggaa ggagagagtg gggagctcaa    2940
tgtggacccc tcaattaatt tcccaagtgc tcaatgaggc ctgccccacc tctcatccag    3000
ctccttggcc aggccctgcc cccactcacc tctcttatgt gggacacgtg agcccctcca    3060
gtgtgccagg agcacatggg tacaggcaca catagagaat gccgtgtcca caggaaaccc    3120
agtgagcctg aagggccttg gcgtgccatt tacaggtcac tcacttgggc caggcctcac    3180
tagggatgcc agggtgaaca agacgtggcc agtgctcttg aagggctccc acctggtgga    3240
ggtagcagac aagtaagggg acaaaggccc ctcagaagta cacatctggt gggccgggcg     3300
cggtggctca catctgtaat cccagcactt tgggaggccg aagcaggcat cacttgaggt    3360
caggagttcg tgaccagcct ggccaacagg tgaaacccag tctctactaa aaatacaaaa    3420
attagccaga cgtggtggcg cgtggctgta atcccagcta ctcgggaagc tgaggcagga    3480
gaatcacttg aacccgggag gcggaatttg cagtgagccg agatcacgcc attgcactcc    3540
agcctggaca cagagcgaga ctccatctca aaaaactaaa aaaataaata ataaaaagta    3600

caaattaggt gtggttatcg cctggagagc caggagcatc cttgggaggg gacttgggga 3660
ggggtccgct gggctgctct agcccactct gggccagaat cgtgattcta attgcaggta 3720
ggctttaccc tatcaaagcc ccctgggctg gggactctgt gaagaagcaa gatgtgcttg 3780
aaacctccct ctctgctgaa aaatctagac aagctcctga ccggctgcat tgctgcttca 3840
gcccctcctc agggaagcgg gaggccctgg gtctctacgg gtgctctgtg aaagactgca 3900
gaggctggtg gggctgcatt cctgtctgtc tgcctaggac agtgcctact gacttctgac 3960
tgctgcccca tggtccaggg ctccaggtac accactgcaa tgtcactttg tgcttggggt 4020
tccaactaga caaaagcccc acgttgcaca gcccttgagc cgtcccgggt agcatgcccc 4080
ctggtggccg tgggcagtta ttaccgttaa tcctttcttg cccagatgcc tctagcgtag 4140
gccacagcag ccggccgggt tcgttgtgtc tggaaagacc agagaagggt tcccggagaa 4200
agaggccgtc tgatcggagt tttatgaacc agaggataaa gaaaggaagg gatttctggg 4260
cctcagaaaa ccacatgcca acgcatgggg gtattaagga taggggctct tgaacgccca 4320
ggctggtaaa atctagaacg gcagagggag gaggggtaat ttttctggtt ggtagaaata 4380
aagtggggat gaggaagagc acgaaagtct gtcggcaaac ctgaaagtgg gagcgagctt 4440
ttgtcaaatc cagggtcaac ctgagctctc attaattggg aaactggaga gtcagatcct 4500
ctaaagaaag tggaaatatt tgttctaaag aacccctgcc aggtccccgt ttacgtgact 4560
cataaagagg ccttcaggca gaaaggatcc cagtgcacca gtaacagaag ggctccagga 4620
ccagagggaa tggacaggga accaccaccc acaccaagca caggcccatt gtcggcatga 4680
ggagcctcgc ccctgcagat cacctcccag aggggagcgg atcagagatt aaattccaca 4740
cctttctaca aaatcatagc agtttgaaag tgccaagggg agtaggtgag gaggtttcga 4800
ggcatgtatg cagccgtttg gagacaccag ccatgtcgcc tgagttttct gatgtaatcc 4860
tcataacaac cattccagat ttaagtgatt agtcctgttt tacagatggg gaaactgaag 4920
ctcagagagg ttaagcaact tccccaaggt catacagtag ccaagtagca taagtggtat 4980
tcgaacccag ttctatttgg agccttcaca ggctttttgc tgcattccac aagagtctgt 5040
atattcagaa gcaagtcttc cacagccatc ataagtcatc ctcacagccg gctggcaggg 5100
agggaacatt gccactgtag ctgggatcat agatatcgtg gtctccattt gacggataag 5160
actgaggtct ggaagagagt cagtcagtct gtgtgataag cctgaaccac cctctccttc 5220
agttcctccc ctaacccaac atcctccggg tctggaagga cagtgtcttg agcaacttct 5280
tgcagctata cttctccagc taatagtcta gaaaaccgag cctggtcccg agtctcctcc 5340
tcggggtacg agccgagggc actgatgggg gtcaggaggt gctggctaaa agacggtggt 5400
gcaggtgccc ctggctttaa gcgcgcccag gttataaaaa gcttggttcc catgctggcc 5460
ctaattctcc atgcactgag taccttaggt cccttctctg ggtctgttgc tcccctgtaa 5520
aacaaggggg tgggagaaat gaccttcaag ctcaaacacc atatgtccac ctgggttggt 5580
atagcagatt gcattagttt gttaggactg ttttaacaaa tgccacagac tgagtggctt 5640
aaacaacaga catttattct ctcacagctc tggaggctgg aagtctgaga tcaaggcagt 5700
ggctggcagg gttgtttctc ctgaggcctc tctccttgta gccacccctc ttctccctgt 5760
gtcttcacat ggttgtccct gtgtgtgtgt gtctgtgtcc taatcacctc ttcttataag 5820
gacaccagtc acagaggatt accggccacc cataggactt cattttacct taattatctc 5880
tttaaagtct ccatctccaa attagtcaca ttctgaggta ctgggagtga ggacttaaac 5940
atacgaattt ggaggatagg acacaattca gcctataaca cagatagaag acactgctca 6000
ttttaaaaaa gaagtagcag gctgggcgca gtggcccata cctgtaatcg cagcactttg 6060
ggaggccgag gcgggaggat cacctgaggt caggagttcg agaccagcct ggccaacatg 6120
gtgaaacccc gcgtctacta aaaatacaaa aattagccga gcatggtggc atgctcctgt 6180
aatcccagct actcgggagg ctgaggcagg agaatcactt gaacctggga ggtggaggtt 6240
gcagtgagct gagatcgcac tgctgcactc cagcctgggt gacagagcaa gactccgtct 6300
caaaaaaaaa aaaaaaaaaa gaagtagcag tcccagccct ttaacagcga acttctgata 6360
catttaaaac agaatctgat tctcactttt tcttccaggc ccacttcaat gccactaatg 6420
aggcctatct gtctgcaatc tgatattaga ggccaaaaat aatctttcta tttgccactt 6480
actgggcacc aatgtcctac ctgacacatg ctggctcagg tgggggagga ggtacggtcc 6540
ctggccctag gaacgctcca tcttagaaag agacagaatg tgctggggtc agagtaaaaa 6600
gtgcatgggc ttcagcagca gatcccagca tggctaccac cagctggggg accttggcca 6660
agagccccct gtgagcctca gttttctcac ctggtcaatg ggactcacac tactgcggca 6720
cacattgtgc gcccacattg tctcggtgca caggggccac ccttcaaact gccagggtct 6780
gcacctctgt gcccaaggtg cagaaagggc tggctgccag aacccactct gcattttgcc 6840
tctttgcacc cgaggctgga agagccctgg aatcactgac tcgctggagc atccattcac 6900
cagtggccat gggagctgat gcataaatat tccagcgctc tggtccttcg agagggacac 6960
tctgtgacat gcgttgctca cctgtgacat gcccctccca gcgtctcctg acctcccctc 7020
ccaaatgcac tacttgcact tgctctcatt cccattctgc ttgctcacag gctggatcac 7080
acaagaccac acatgcgtgt acactcgtgc ctgcccatgg caagtattca aaaactgctt 7140
gctattttgt gggtggtggt gaagggaata gtatatttat aatacttcag gccaggaata 7200
tacatctcct ctgttttctg acccttccag gagtagggtg gattccaccc gcttttttgt 7260
aagccctcag aagtgctcca ggctctctgc acacccccag atctcccctg agcttcagcc 7320
ctcacccagc tccccagtca atttggcccc tgtgacgatg ttggtggggg gggcgggggc 7380

aggggaaaca gctggccctg gctcctgaca ggcactctgt tcccatctca gctgcctggc 7440
cctgcctgtg gcatcctcag agacccagtg gtgagtactt ccttggccct ttaggcccct 7500
ttccccaatc cagtagtgag acacggggag gggctggcag agacacaagt ctgccaccct 7560
ttcctggaca gccttctcct ctctggcagc aggaaaccac cccctactcc cactccagac 7620
ctacgctcac actccccgtc aaccccaagt gccactccag tctggggtcc ctccctgagc 7680
tttcataagc attgcaaatg cccaattcct tcattggctg gggagggcaa agcctaaatg 7740
ggcagtgggc tatagatggt ggccttccag gcactggcag caggacactt cagggcatca 7800
ttcaaatgca gaggggagag cattctgccc aagggcaggc ctatttgggg tgggcagggc 7860
agacttacaa tggagactac catacttacg tggctctctg caggccacgt gaccccttcc 7920
ctctgctcca tgtgcccagg attcaaactc aggctagtct gattcccaaa actgtgggcc 7980
tcagttttgg gaaaactgag aaaattgggc ctcagaatag ggatagggat ggagatggag 8040
atggaagaaa ataaaccagg ccctctgcct gatcccctcc ctactttggc tctggggttg 8100
ggagatgctg cagaggcttc atccactctc tgccccgacc ccctaactcc ctctcctgct 8160
ctgtccactg ggctggaccc tgtctgctgg gcctatcagg gctccccgtg tgtcctccac 8220
cagccagcaa tggccagaga gcccagctgg ccccagccta tcccctgccc accctgggcc 8280
ctgctcagag gacaaggctg aactgattct gtcaggaagg gttagtcttg tctcaggcct 8340
ggctgaatgc aaaaggacga gacaaatgca tgtggacagc ctcgatccgg agtgctctgt 8400
caaacatgct ggccacctct ccgggaagat cactcagact ccttgcatcc catcttggga 8460
aggctggcct gaggctccag agagatgttg ggagatgcca ggtgcctgca agccaagggc 8520
cctccgggga agggtgtcag gacccgctgc ttcttgcctc ttgcagagag ggcaacagct 8580
ggctccttct cctgccccag gtgaagactg atcagatact gattccagga tgaatccagc 8640
aacattaggc cctgcctggt cccatggtag catctggctg ggcttttggc ctatagtcga 8700
gtagatagag ggagggctga taactaagtg actacaacag gacctccctg tgtaagggtt 8760
ctagcttatt tccacactct tctggcacag acaggttggc agggcagctg gtagcaggca 8820
cagcagatgg aatgtcagga atgaaatcca caagactcca agcccctccc agggtgagct 8880
tgccacaggc cacaccagga agctgagctc ctcacccaaa gcctgccagt gcctacgtgg 8940
cagtcattcc cttgtcaaca gacttcccac tggcttctct tgctccatta ttaaaagcaa 9000
gaaccaaaaa cttcactgag gggatagaat cccactaata agcatgtcca cgctttgggt 9060
catcttaggg gcacagaaaa tctgagtatc ccccatagca tcaggcagag cagatttctt 9120
gtcaattagc tcagttggtt ggaggctgca gccatgaaca tgctggcctc agggccctca 9180
caacacaggt gggttaggga atgaatgaaa gttcagctat tattcctgcc agagactcca 9240
ggcccaacct ggcctctaat gagctatgtg actttgggca ggcagaaaag tgtctctgct 9300
atggtttgaa ggcatccccc aaaggcctcc aatgcaacag tgttgagaag ggggaccttt 9360
aagaggtacg tgggtcacga gggctctgcc ctcacgaatg gatttgtcct catcgcagga 9420
gcgggctcat tatcatgaga gtgggttggt tataaaattg aatgtggccc cattccttct 9480
ctccctttcc cccatgtaag gccccaccag acatggccct ccagtggtgg agttcccaac 9540
ctccagaacc acgagacaaa tgaactgtta ttttttcata aatgactcag cctgcagtac 9600
tctgttacag catcacaaaa tatgctaaga cacccctgag actcatcggc cacctgtctg 9660
tcagctttgc cgcaaaaatg atttgggagc agctatagtc aactttcctc agactacctc 9720
ctgctggcac attacggcca cctgctgtgt gcctggtgtt tttctaggtt cttggaataa 9780
agtgaacagg ccgggtacgg tggctcacgc ctgtaatccc agcactttaa gaggctgagg 9840
tgggcagatc acctgaggtc aggagttcga gaccagcctg gccaacatgg tgaaaccccg 9900
tctctgctaa aaatacaaaa atgtgctggg catggtggca ggtgcctgta atcccagcta 9960
ctcgggaggc taaggcagga gaatcgcttg aatccgggag gcagaggttg cagtgaaccg 10020
aggtctcgtc agtatactcc agcctgggca ataagagcaa aactccaagt caaaaagaaa 10080
aaagaaaaaa gaaaaaaaaa acagaaagaa agaataaagt gaacaaagca ggcaaaaata 10140
cctgccccca gaaagatcac attcagtgga tgtgcccatg ttccttcctc ttcctaaagc 10200
actcacaaat atcaacattc ccctgaactc accactgaga gtcacttctc agcgtgacaa 10260
tgttgaccag tcttcacctg ctgtgtaaag caggcctgcc ctatagtgac cccccaaaca 10320
ggtgcagaag gcaggtccac catcagccca tggccagcac accagttaga tacctgctgt 10380
tgccaggcag ctgtctgcca gcccgtaggc actagagagc cttggggcct ttcctgagcc 10440
cttttaaaga agtgcttcct ttagacatgg actctcaggc ctgctcagag cggggagggt 10500
gctgctccgc cccaaggcct tccctccatc ctttcctctg gcaatcaccg tgatagcagc 10560
aacgcccagc ctgaggcctt gggtgctgct tccctcagga aaactggcca aagagtagac 10620
agcagatact ttctccatat tcccaccccc acaacacagg ccagcagcac agcagctgta 10680
gacaagattt cataaagaac tggaggacaa gaaaggggag attaaggagc cctaggctct 10740
agggcctggc tcacacaaga atggtcttcc tcctgccaac ttcttggcct ttacaaaggc 10800
cccacctgac ccagcccagc ctcctgggca ctggccaagt tccacaccaa gactaccaga 10860
ccccgccaag tccaggctca agagagaaag ccagaccctc tctccttcca cctacctgag 10920
agaggcctgt cctcaggtgt ctggctcaca gatgggggac ccaactggga gtcactccct 10980
tctggaagcc ccaggcccta taaacaggtc cccggcctca ctgctgggcc tcggcctgga 11040
gacagcggct cagtgcaaca caaccacgcc agagctgatc aggggaaacc cacgcagcag 11100
ttgagggggc tggggctttg cgggtgcagt gaggggactg cttcccttca gtggagttgc 11160

tgccgcaggt gaggggagga agatgaatct gaaggttcta acgtcagccc acaggctgaa 11220
gcgaaggctg gcctgaagtg ttccacctgg gcccccacct gagcggctga agagttctgt 11280
ttcctcaagg cccacagagc tcctcagagg gaagctttag tagctatttc cttaggaagg 11340
caagtatgtg tgcaacattt cctaataaaa tgcaaaggct gttggaggca ctccagcccc 11400
ctcatttagc tctgaggtgt aattaataca taaattaagc tcctgcagac tcacaaatgt 11460
ccaagaaaaa aaatgaaaaa tataattagt ttgcccatct gactccatct gtcagctctg 11520
agctcaatgc tcaacgtagc aaatcctgag ggctttggtc tcgcattggc tgggcccggc 11580
gctgctcctg ccctgctcca gggaagtggg ccctccgcag cagctgccca gagcccgggc 11640
tctggactca gcctccaggg cagcaaggac tctggagtac acagactgca ggctgggagc 11700
cggggcactc ggcagagcag ccaggacctt ctctgtggcc ctgagcgctg gctaggggtg 11760
gcaagggcac cccggggaat ttgcatgtgc ctcttgctgc catgttccta tttgtgcaga 11820
cctggaagtg ggcggcagag ccctgaatgg gctaagactc gccctcctcc tccaacctgt 11880
cacctctgtc gcttactatc tgtaagaaga gaaaggctgc aaaggggaaa gggaactcga 11940
gaggaaggca ccgaggagaa gagaggagac aagagggcat tgggggagca aaggaggcca 12000
ctcacacagg aggattagaa ttaacaacta cccccacccc agctcccccc aacacacact 12060
tacacacgct tgtattacac cggggatctc acacacatcc ggtcactcag tattcagcac 12120
tctaggaaaa gaacaaacaa atggcttgtc tttcatcccc caaaattact tatgtcataa 12180
aattaatcca tatttgtttc agaaacattt aaataagaca taaggggtat ggcattaaac 12240
gtacccccaa atgcctcttc cccttacaag tggggatcat cattaaaaac ttttgtccca 12300
gaagtggtga ctcaggcctg taatcccagc acttagggag gctaaggtgg gaggatcact 12360
tgaggccagg aggtcaagac cagcctgggc aacactgtga ggtcctgtct ctaacagaaa 12420
aaagaacagg aaaatttaaa aggaggaatg aaaacaattg tttaaataat aatttggtgt 12480
gtattattta aagtacatta atatttggtg caatcgtttt tgaaagggag attgtactat 12540
acatgttgtt tggcaactta ggtgctcgct caggcaccag gccacacaca tcttgggcat 12600
tccacatctg ggttttgaaa gtaaaattat aataagaaaa taagcaaagg tcaaaatggg 12660
ttggaaaaaa attatttaca cagtgagttt tctactttat cataccaaga agatgacttc 12720
tctaattatt attatttttt tttaagatgg agtttcactc tgtcacccag gctggagtgc 12780
agtggcgcaa tctcagctca ctgcaacctc cacctcctgg gttcaagcga ttctcctgcc 12840
tcagccttcc aagtagctgg gatgacaggc tcccaccacc acatcaggct aatttttgta 12900
tttttagtag acatggggtt tcatcaggtt ggccagggtg gttgcgaact cctgacttca 12960
ggtgatccac ctgcctcggc ctcccagagt gctgggatta caggcgtgag ccaccgtgca 13020
cccggcccct aattattatt tacgtagaac aatatcaaag atatttttct tcctaactct 13080
atactccttg acaagaaaaa aaaagaaaag aaaggaagag cccgcgatat ataatggtac 13140
atttgaaaat gaagcatttg tatgtgcgct cacatgagta catatatatg tattacatcc 13200
atatgcatgt atgtgcatag actgtttcta gaagaattca caagaaactg gtaacatttg 13260
ttgcctctgg gaaacagatt ggcaactggt agaaggtgac tgacttcatt ataaacatat 13320
tttgtaatgt tttaattttc tgtcacatgc attattactt ttttccttag aaaggatttt 13380
ggttttgtct attttatcgg aagaggcaaa ttttaacaat aaaaacaaaa tgactgacat 13440
tcagccatag tcccacctct ctgaattact cagatctact gatttacatg tagattaaat 13500
gcaaatgagg gtggcactat ggttgattat ttagcagctc agcaatatga ccaatgccct 13560
gttcccttct acccttctgc tctgccccct cggcttgtag actttgtcct tgggcttcct 13620
aacctcattg ctacaagagt caggcacatg atcaaaacaa gacactgccc caaggtagga 13680
aagaagccac ctcttccttc ctatttttag gagaaaaatc tttcccccaa agccctcctg 13740
cagacttagc ccacatatcc cactggccat atttatttcc catgtctatg cctgagccca 13800
tctctggcaa gggaaaggga cccctttgga catcccaggg caagcaggcc tcctcagtgt 13860
tatcagacca actacccctc ttgaaaacaa tgactgtatt cttgtaacca cccaacctca 13920
atccagaaac aaaattcaca gatcacagaa tgtacctaca aaaacatatt tcaaaaaaga 13980
atcaaaacga agccctagct gtcacgtaaa ggagaaagta aaagtaattc aagacaaaag 14040
agtatgtttt ataataagca aaggctcagg cagcagactc tacagaacat aatgaagtgg 14100
tgagatgctt gcagctgcac acggaaatgc caaggttgcc ccatcataca tatagacaga 14160
tgcgggagat gactcaccca gcacgaggcc attgccactg gcggcatgat tttccaaaat 14220
ggcgaacaac tgttggtaaa gcttcaaaca aaacacagtg caacctcccc ttgatttata 14280
cagtaggata cttcctagga aatttagggc atatttaaaa acatgcaaaa agatacttgg 14340
agctcataca tcaaatggaa tgaagtttta ggcttaaaaa ggactttcag ctacataaaa 14400
gtctcatgag acatcagaaa gtcacgtggg acatggagcc atttcctctt agaaaggact 14460
gtctcacaca ttgtaaggta tttagccttc ctagaccccg tttattgaaa gatgatagca 14520
agggccctcc ccacaaaatg tcaaagcccc caccaatttc cagaacacac ttagggaatg 14580
gttctgtccc tttcgagaac tcctggctta ggccaacctg cctctcccag cccaggtgac 14640
ctcagagctg ggtggttgtc tgaacaacat tcaggttctt gcaggaagca tagaaggcga 14700
ttagaggttg gatagatagt caaccgtctg ttcatcacag aagcatttgc acatatttgt 14760
ctctagcttt gcctttgtat acatattttt aaatacaaat aaaatacgta tttaaaatat 14820
ggccaggtgt gttgactcat atctgcaatc ccagcacttt gagaggctga ggtgggagga 14880
tcaacttgag cacatgagtt tgacaccagc tgggcaacat agtgagatcc catctctata 14940

aaaaattagc caggtgtggt ggcatgtgcc tgtggtccca gctactcaag aggctgatgt 15000
gggaggatca cttgagccct ggaggttgag gctgcaggga gcactccagc ctgggcaaca 15060
gtgaaactgt ttcaaaaaaa aaaaaaaaaa aagacaaaca actgatttac cgaatgggca 15120
gaacttcatg gcacatactt ggctttcttt ttcttttccc cccatcactg tttttcagtg 15180
ctggctggtc agctctcctc tttcagccct tggatgacag ctggggctgt cccatagctt 15240
ttcagtcact ttgagctatg tcataactgt ggtcatattt tcaagcatga aaagtacaga 15300
tttgtctcaa tcaaaactca gtctcacaaa agccactttc ctccccagct cagtgcccac 15360
ccagtgggca ggaggagggg caggtctgtt ccttttctcc ctcgctgcca ccctcagtcc 15420
aagttcgggt ggggaagagt tgattagagg atggggcaaa gtcttagagg gccggacgtg 15480
ggtgatccat cacacatacc cattctatct tggcctctag ggtggatttt gaggagcccc 15540
ctccccatga tgatctctgc actccacaag gagttcctcg atgtgacggt gcactctcta 15600
gctggtcttc tgtggcatgc cccctctggt acccagtggt tcacccctcc caaccagcct 15660
ttccctgggg gacctctcct acccagcagg tgagtctcct gagtgggctt ctttcaagat 15720
acctgaaaac ccaactcctg ggaggttcac ttgctccaag gaaacttgta catctttgcc 15780
ttgcctaaga gtggaggagc agcttcccca caactgaccc tgtaatagaa aaaaacaagg 15840
tgttttgcct attctcacac acagaactcc caacaccaga tgtgtggagc atttcccaca 15900
taccaggcaa ttctcccttg gacaccagct gggtgtcctc taattcagtt cagttttgat 15960
cctatctacc tggagatagc atcagatccc acaggtcgat ggctcagtcc tgcgagactg 16020
cccctctaga gatgccaatc tcaagtgcag gttgtgacct gtgctttgaa ccattgacta 16080
tatattgggg ctccctcgac tccctcgttg ggtgagatta atctgctaga gcagctcata 16140
aaactcaggg aaatgctcta tttatgttta ctggtttatt aataaaagat gtaataaaga 16200
atacagataa gcaccagatg aggagataca tagggtaagg tctagagggc cagcagcttc 16260
tgtctctgtg gagttggagt gtaccaccct cccagcaagc agatgtgttc tccaacatgg 16320
aagttctctg aacctcatac ttagctgagg gatttttttt tttttttttt gagacagggt 16380
ctcactccgt cacccaggct ggagtgcagt ggtgccatct cggctcactg caacctccac 16440
ctcccgggta caagtgattc tcgtgcctca gcctcttgag tcgctgggat tacaggcgct 16500
tgccgccaca cccaactaat ttttgtattt ttggtagaga cagagtttca ccgtgttggc 16560
caggctggtc tcaaactcct gatgttaggt gatcccccca cctcggcctc ccaaagagct 16620
gggattacag atgtgtagct cagggatttt tatggagtct tcatcacata ggcatgattg 16680
attttcaagt ccatttccag cccctttctc ctctccagaa aatgggtcat ggggctgaaa 16740
gctccaagct tccaatcatg gcttggtctt tctgctgaca agcccccatc cagaagccca 16800
ccaagagttg cctcaataga acaaaagatc ctccttttac ccaggaagtt ccagggactt 16860
taggagctct gtgtcaggag ctagggtcaa aaaccagata ttagaacaaa agattctcct 16920
agcacctgta tctacaagga tattaggagt gctgtctcgg gaataaggac agagaccaaa 16980
cattggaaca aaagattatc ctgacacttc tatcacttag aaaatcacag ctgacccttc 17040
aacaatgcaa gggttaggag tgctgacaac tgcccaactc ctgcctcgca cacggttgaa 17100
aatttgtgta taacttttga ctcccccaaa gacttaacta caagcctact gttgactgga 17160
atccttatgg gtaacataaa cagtcaatta acagatatat cgtatatatt ttatatactg 17220
tattcttaca ataaagtaag ctagagaaaa gaaaacatta ttaagaaaat cataagagaa 17280
aatatattta tggtgctcta cggtatttat tgataccata ggtctgcacc atgtgtttac 17340
aacatgtatc atctgtctga aacgtcaggc aacctcagct gctgacctta gtctatggta 17400
catatcgagc aattcaactt gttcctgtga ggtcatgacc ttacttctta ggagctcttc 17460
cagcatcact agtggcactt cgtatgggtc ccatgatgct attcaaggtt tacagtattg 17520
cactaaccat gaaaataaat gagaactgag agagatcatg gttttcactg caatatgcaa 17580
tttactggag attgcaatag taaattgtaa attgctatat gcaatctact gctcacgtgg 17640
agatggtcag tatttcatag tgttttaagc agacactcgc aacacgagct caccgcaata 17700
gcaacaggag gtagctacaa aaatattaca gtagtacagt gcttactaca gtgaatttta 17760
tgtagttatg attgaatact tcatctttac atttgtttac atttccctca actacaaatg 17820
gtgccatgta tgatctgtgt tttttgcatg ttttcataaa ttttaacttt ttataataga 17880
tttgtgtaat tttatagtag taacttataa aatagactag tactcgcata tactttatgc 17940
attcatgaca tacctaactt tttgttgttc ttttttgata tttctgggct acacaagggc 18000
agtgacatga tcgtagcgca ctgtaacctg taactgtaag ttttttcaaa ctgtcacaaa 18060
tctcaaaaaa aaaatctaat atatttaatg aaaactatcc aagtataagt tgacccttgc 18120
agttcaaacc tgtgttgttc aagggtcaac cttacaagga ttttaggaac tctgctggga 18180
actggggaca gagagcaatg tgtatatttt gtgttatttc aggtgcgttt gctcatgcct 18240
ataatctcag cactttggga ggctgaaggg ggagaattgc ttgaggccag cagtttgaga 18300
ccagcttggg caacacagag agatcatgtc tctaccaaaa agatcccaaa aattcatatt 18360
ctccctcctc actttacctc acacgatgct ccaaatcagt ctcccacctt aagaattctc 18420
cataagcaaa agcccatctt gaatcccaca ctctcaggga cccttgccac actatccccc 18480
caattttctt ttctttcttt atttatttat ttatctttaa gacagagtct tgctctgtcg 18540
cccaagctgg agtgcagagg tgcgatctcg gctcagtgca acctccacct tctggattca 18600
agcaattctc ctgtctcagc ctcctgagta actgggacta caggtacatg ccaccacagc 18660
tggctaattt atatatatat atatttagta gagatggagt ctcaccatat tggtcagtct 18720

ggtcttgaac tcctgacctc agatgaccca cccgcctcag cctcccaaaa tgctgagatt 18780
acaggtgtga gcccccgtgc ccggcctccc aattttctca ttggccagtc actctggtgc 18840
cctggggact agcatccagc taagggatag atgccagctg ctctctcagt gctatggttt 18900
gaatgtttgt gcgccctcca caattcatgt tgaaaatccc cagcacaaca gtatcgaggg 18960
gtggggtcct taggaggtga tgaggacatg aggcctctgc agtcatgaat ggattagtgc 19020
cctatagaag ggctttagag gctgagtccc accttccatc cctccaccat gtgaggacac 19080
agcatttgtc ccctccagag gatacagcac aggtgccatc ttttaagcag tgagcagccc 19140
tcaccagaca ccaaacctgc cagtgccttg aacttggact tccgtcctct agaactgtga 19200
gaaataactt tccattattt ataaactacc caggctcaag tgttttgtta cagtggcaca 19260
gacgtactaa gacttgtggg tacccacaat tccataagcc attttctttg cttggcctgg 19320
gcatgacccc ctgtgctgtc cccgtgaaga aaaaggataa accaacaaac tctatgaatt 19380
aacttcagtg aatctcgtat gtttctaatc ttctgctttg gagctttggc gataaagaaa 19440
gattaggaaa ttagattaaa aaaaactaac attatcttaa atgacccata ttcccaatca 19500
atatctcttc ccttgaaact tggtgtgtat aaatccaata ctttttaatg tatttgtata 19560
tacatatgta aacatataca aatatataca tcatatatat gtatgtatct gcctaaataa 19620
ccacctggag ttagcaggtt ccatacctgc aggatgcaag cagggatggg cagaggcagg 19680
ctggtcagct tccaggtata tatatatata tatactcgga aaatacttgc tattagtttg 19740
gtggttttat tattaacgta aatgatgtct tgctgcacat attattctgc aagttggttt 19800
tcccacataa tgtgttggac atcttttagc cccatttcac actcttagcc tgccttttgc 19860
acaagagttg ctgtggcaac aagctgcccc caggtgtaac ctgacagctt cacttcagct 19920
gcaccaggta tgtctttctt tctgccccag gcatcacggt atacaggcaa atcttgaagt 19980
gccagggagt caaccctccc tggagcaatc cttgcccaat aggagaagga agccaatgaa 20040
taaatatgcc aggcctcgag gtcttaggcc ctcagtctaa ggcatagtct acatggtttc 20100
tctgggagtc ctcagttaaa ctgagccccg actgcccaca gcaatggcca gtgttaaaag 20160
aacaacctta accagattaa atttaacaca gtttaactga gcaaaggaca attcgtgact 20220
agggcagtct gctgagccag agtaggttta gacagactct ggtggaagat ttatggacag 20280
aaaaaggaaa gtgaagcaca gaaaacagaa gtgaggttca gaaacagctg aatgggtcgc 20340
agctcgtcgt ttgccttatt tgaacacagt ttgaaaagtt ggccaccttt gattggccaa 20400
aactcagtga ttgacacaag agtacgttac agcctgttta catttccctg taggctatag 20460
tttactatgc acagagaaac ctttaggctg aacttaaaat atgtaaggag gaagctttaa 20520
gctaaatttg atttttgttg ttgttgttgt tttgttttgt tttgtttttt gttttttgag 20580
atggagtttc actcttgttg cccaggctgg aatgcagtgg catgatctcg gctcactgca 20640
acctccgcct cccaggttca agtgattctc ctgcctcagc ctcccgagta gctgggatta 20700
caagtgtgca ccatcatgcc caattaattt ttgtatattt agtagagggg gtggtttcac 20760
catgttggcc aggctggtct ctaactcctg acctcaggtg atccatctgc ctcagcctcc 20820
caaagtgctg ggattacagg cgtgagccac cgcgccaggc cactaaacct cagcctccca 20880
aagtgctggg attacaggcg tgagccaccg caccaggcca ctaaacctca gcctcccaaa 20940
gtgctgggat tacaggcgtg agccactgcg ccaggccact aaatttaatt taacagtagc 21000
ttaacacatg gctggatggg cttttcctac ttccttgttt cattcttccc agtcctccaa 21060
ctcttgactc ctagatttat ggacccaaat aaatgactca aatgcagtcc ctttgtctca 21120
ggtttttggt ggaatccaag ctaaaacact aaataaaaaa tgttgttaaa aataagttta 21180
ttttggtcat tattgatcca cttggagtgg tcctcaaaat gggatctagg gaatcggtcc 21240
ctgaggctgg tttctgggaa tccacaaatg caaaactatt tccatagtca cactaaggag 21300
gtgtggcctt ttcaagaaat tacattaccc gtaaagtgca atagacggaa aacagaagcc 21360
catatgagaa tccagctatc ttctagcaag ccagaagtaa aagatgccag gtgcagtgga 21420
tcacatctat aatcccagca ctgaggattg cttgagcctg ggaatttgtg accagcctgg 21480
gcaccatagt gagaccccca tctctacaga taatttttaa aattagccag atgtggtggt 21540
gtgtgcccat gatcccaact actcgggagg ctaggaagga ggatcacctg agtcaggagg 21600
tcaaggctgc agtgagctgt gattgcacta ccacactcca gcctgaatga tagagtgaga 21660
ctctgtcaag aaagaaagaa agaaagaaag aaagaaagaa agaaagaaag aaagaaagaa 21720
agaaagaaag aaagaaagaa agaaggaagg aaggaaggaa ggaaggaagg aaggaaggaa 21780
ggaaggaagg aaggaaggaa agaaagaaaa agagatctgc aaaaaaatgt aaaacaatac 21840
cactcttctc actaaatttt tgtacttgga aaacatagtc atttttcaca aaaatgctat 21900
ttatattaac ataagtattt tgatgtgcaa tggacttatt tttgttattt tcacataaag 21960
attgtttaaa tttcttagtt ttaatttcta atactgtaaa tatcagtaga tataacccat 22020
gtaaacaaaa gctttttggg gtcctcaata attttcaaga gagaaaaaga ttgccaagac 22080
taaaaagttt gatagctatt aatagagttc attgctttta acttctgagt cccatgcata 22140
cttcattatt attattatta ttatttgtaa agacagggtc ttgctgtgtt gcccaggctg 22200
gtctcaaact cccaggctca agtgattctc ccatcttgga ttcccaaagt gctgggatta 22260
caggtatgag ccacagtgcc ctgcctcttc ttttaaaatc taattatgta tttgttatct 22320
cttacatttt tcctatcttt cttttttctg attatggccc aaaaggggaa gatttctgcc 22380
catgctcatg ttactatctt gaaattattt cttttcagac tctctagttc aggaaaaagg 22440
tcccctgcca catcaagtgg ccacggagga gcttgggcct gtgatgtccc ctcccttctg 22500

cctaaatgac cacctggagg cagcaggtta catacgtgga ggatgcaggc ggggatgggc 22560
agaggcagac tggtcagttt ccagggcaca cttcgggggc atacaaggga caaaaatgtg 22620
acggtggctc aggagggcac caaagatggc caaaagcttc tgcttttaag gtggcagtag 22680
gaggcagtta atggaaaaat agaaaccaat caagctacgt ctgccaccaa tgaaaatgaa 22740
ttattattta ttcaacaaaa tataagcttt ttaagaaaaa aaaaatagca aacgctcaac 22800
ccacttgcta agttccgttg gggtataatg gcatcagctc tgagtgccaa cttaccttgg 22860
caaggtggtt gcacgccctg aggatatgta caccttctcc gctgctggcc agctagatga 22920
ccttgagcaa gccacccacc cttctgaact tcgtctattt ggaaaataca gacattaata 22980
cctcatagtt tgtaatgagg ccttagagtc cacatattaa tcgttcagaa cacagctcaa 23040
aaaaatctaa aacttctggt ggggcacagt ggctcacacc tatgatccca gcactttggg 23100
agcctgaggt gggaggatag cttgagccta ggagtttgag accagcctgg acaagataga 23160
aaggccccat ctctatacaa aatataaaaa ttagccaggg gtggtggcac atgcctgtag 23220
tcccagctac tcaagagact aaggtaggag gatcgattga gcccaggagt ttgaggctgc 23280
agtgagctat gatcacacca ctgcactcca gcctgagtga cagagcaaga ccctgtcacc 23340
aagaaaaaaa agagagagag agagagagag aggtggcttc agtgggccga atagcagaga 23400
tccactggga gcattagcaa catcagggcc attgcttcag cagcctggaa ctaaagagga 23460
agaaagcaag aagcttcagg agattgaaca gtaggaagga aggaaggaag gaaggaagga 23520
aggaaggaag gaaggaagga aggaaggaag gaaggaaaga aggaaggaag ggagggaggg 23580
agggagggag ggagggaggg agggggagag ggaagcatat gagaaagcga ggctaggagt 23640
cttggagtca tccttgactc atctcttatt gtagtcattg ccagtactca cagctctcag 23700
acaacagtga ggaaacaagt tattttcaac atttcaggaa gctaaatgga aaccatgcac 23760
acaacaaggt ttcaaagtta attgaggatc aagattttct gctgttgtct ggtaagttat 23820
caattctgat ttttttaaac ctgattacag gcatccacaa aaaaatatgc aactaacatc 23880
atgtttaatg ttgaaagact aaatgctatc ctctgggatt gggagcaagg aaagggtatc 23940
cactctcatc actcctgtgt acttaaaaaa aaaaaagaaa taaaacatat gttcacacaa 24000
aagtctgtat gcaaatgttt atagtggctt tatttataat tcaccccaac tggaaacaac 24060
ctaacatgac cttcagctgg tgaatgaata agcaaaccat ggtacatcca tacaatgaat 24120
tactagacag caaaaaaaga aactggtgat ataggcaaca acctgatgaa tctcaaatgc 24180
actgtgctta gtgaaagaag ccaggcccaa aggactacat actgtatgat tcaatgttgt 24240
gacatactgg aaaaggtaaa attataggaa caaatcagta gttgtcaggg tctagtggct 24300
agggaaataa ttgactacaa aaggacagca agggggaatt ttggggggaa ttggaactgt 24360
tctgtatctc aaagatgggc atgattgatt acatgattat gccaaaattc attcatcaaa 24420
aatgtaaatt tttatgtatg tagatagatt ttattttaag tgaataaaag gaacaggcaa 24480
cttggaagca aaagagcagg agccgggcca ggcctgcggc tagattcaga aatagggtta 24540
gagggaatga tcgaggggaa tgacccaggc agccacagct ctggagagac ggctgcaggt 24600
ggacaaaaag caggtggaag cgcccagcag gaccttgacc agacaggcaa tgggaacata 24660
aaagacagca acagattgca acatcatgtc catggctccc caaacctgat atctcagaat 24720
cacttccaga gctgattgga aagacagatt ccattgctaa gcatagtggc ctgcacctgt 24780
agttccagct actccagagg gcttgaaccc aggaggttga ggctgcagtg agctatgatc 24840
gcaccacagc gctccagact gggtgacaga atgagacaag tcagaatgaa agaaaggaag 24900
aaacaaagaa agagagaggg ggaaagaaag aaagaaagag agagagaaag aaagagagag 24960
agagaaagaa atagaggggg gaaagaaaga aagagagaaa gaaagagaga gaaagaaaga 25020
aaagaaagaa agaaagaaag agagagagag agaaagaaag aaagagagaa agaaagaaag 25080
agagagagaa agaaagaaag agagagagaa agaaaaggaa ggaaggaagg agaaagagca 25140
aaagagacag agggaaggaa ggaaggaagg aagggaggga gggagggagg gaagagggag 25200
agaaggtgag aaggagggag agagggaggg aagagggaga gaaggtgaga aggaaggagg 25260
gagggaggaa agaaggaagg aggggaggga ggaaggaagg aaggagggga gggaggaagg 25320
aaggaaggaa agaaggaaag aaggaaggaa ggagggaggg agggagggaa gaagggaaag 25380
aaagaaagga aggcagattt ctactgaggc tgcctcttta ttcccctctt tattcccctc 25440
agagaggtca tttaacctct ctgatctctg tggttgctgt agggactaaa ggacacaatc 25500
cttgggaaag tgcttggcac agaacttttc agcttgctat ggggagtgcc agccaccggc 25560
aacaggtgtg actgcagggg aaagccccgt gcaggtgaga gaaaggggcc agaatgagaa 25620
gggctttgta aggctgggac cactatgttg ggtctgctat ggagggggga gctcaggagt 25680
aggcagggca gatttgaaca tgggaagggg aaagctgctg ctggaagcag tggcccatgc 25740
ccaggtgcat ggagaagaga gcctggaagc ctctcttggt ccctctaggc tggcagaggc 25800
agggccccac attggcagtg gtgtgaacaa tttcccctca tcatggggct gctcatggct 25860
gtgaaacatg ggatggcact agatttggtc attaagtaaa taggataaag aaataattgg 25920
aaaatggatc cctgcctgga aggagtggaa ttgttcctgg ggctattgtt gtccggcatc 25980
ctgctttctg agtaatctcc cgagccaggc aaggaaggat gtcaggctta gggctgatga 26040
gaaacttaca cttgaaaaga gctcaaaatt acccatgaca atggaaaagc ctttgtatcc 26100
tgaacagaga gaggaaaaag actctgaatc ttctctgggg cgggaggaga gggaagatag 26160
gagggcagtc tcgagtcaca gaatgtcaaa cccaggaccc ctgtgtcttt ccaagttggg 26220
ggcactggcc agggaaggat cctggttctt ccggtgtctg ggatgatgag gctggtcctc 26280

aggcagtggg aggctggagg agggggaatg tgtgaagacc acatgctttc gcctgcaaga 26340
gcatccatcc atgtcttggt cactgcaatt tacatcctcc cattcaaccc tcagcacacc 26400
caccaaacca agctcctcct tgcaccttga tgggcagaaa gttggagtga tgattatcta 26460
agacagagcc ctgaggtgaa gaactggttt tctggtggag ggggtgaggg gaggatggag 26520
ggagctatcc cttccctgtc cccgcaaatc cttcagcaaa aaatccatag tgttgagtca 26580
aaatgccaat cagatgtgcc tctcagcagc tttaggcctg gactttgacc tcagccccaa 26640
cccccacccc cacaggcact cagcctaagc tcccattctg caccctcccc accctggttt 26700
gcaaaatgca tcaaagcaga agctgtgctt ttgcctttcc ttgtaacttc ttgtctgcaa 26760
gctgtattag atgcttgctg gtgtttaaaa cattttctat ttctctaaaa aaacaagttg 26820
gggcacccca aaatgaagcc tcccaagttc ggtcaaacat gcaagattgc cactcaattt 26880
ctccattcta gatgagaata gcgagtccca aaggctcgtg ggtgtctgcg gcgttgcact 26940
cacttaactc acttgttcaa aatacaggtg ccagggcctc ccccccgagc cacagaacct 27000
aaatccccag aatcaataac tttctttaaa taactagatg cttgatttaa agggaaaact 27060
ctatttaaag tgctgtatcc ctccttaaag acagattgtc ctacacgaag cagctacatg 27120
caaaatgaat tataggagtg tccttggttt tacaaaaaat gaaaagcatt gcaattctcc 27180
aaacatgata agaaaggaat ttttttgtta aactctgaga gcaaaacaat ttactagaag 27240
atacagatgc aagttgagta agcaagccaa taatggagaa aggaggaatt tttacagaag 27300
cagtttttcc tcaccccatc tgcatttcat gtctattaaa acacatgtca tgcaacgaaa 27360
aaatcaaagc agtatggttg ggtacacaca ttagtggctt tctgtatggt tagtgagatg 27420
aaaggacaga gaaagctatg ctggagtcat caggacatgg tagagccctc ttgtgagctg 27480
ctggaacaca tcagttgcac gtacgttctc ttgctggtgc agcccatccg gtcagaatct 27540
gtcgctcaca gtagctgtta ctgaatactg tctgcttctt agctgaaacc ttaccgtagg 27600
accatccggc cctgctcctt tcccgttaac atgtaagttg ttctcagacc ctaccttttc 27660
ccttggtttc tcatggaccg tggccaggac ttgggtgtcc acagccacac ccacaaaaga 27720
agtgcctgtt ccacccagga cctctgggtt aaatgacctc tctgaggcca cacaggatct 27780
acagcacaaa ctcagctcag attataaaac ttgcactctg aacgactgcc ctagccagcc 27840
ccaccaaggc catgaggggc ctcccagagc caagtgcgac ggcttctctt ggcctctgcc 27900
tccttgacct gcaagcaata tttgacccca cttcccttcc cgtctcacac cctcctgacc 27960
tcctcagctc tcctttctgg tctctcttcc cctctcctca cacctccaat tggatcttca 28020
gccttcctct cctctcactc cacatttttc agctggattc ctgtggtttt cactcttatc 28080
tctccctatg cctgccaaat ctgtgtgttc agctgtccga acttctcagc tctacatgtg 28140
tgtttgcaaa ggcttcacca tcctccccac acttcctcta ccagaggctg caccatgggt 28200
cactgggcct caatcttcct gcccactcag gtagaggctc tagggtcagc tttgcctcct 28260
gccggatgct gcactgctgg aatcagctgc ccacacttct ctccttttgt atggtgtcct 28320
cactgaaact tgggcggtgt cccttggagt gccttgagag ctgatgattg ccaccctctc 28380
taagagccta cagacccctg atggatatgt gtcctggagc ccctcacaca agcccagccc 28440
tctgtgttag ctagacagtc aagtgggtgc acacagggct agcaccaccg cggctcagaa 28500
gccaccttcc cctggaagcc actccttata tctgggcact ttctcctctg gactaccctt 28560
gcattgtgtt gttctatagg aggtgctgga gacagtgata agaacccatg gcccctgccc 28620
tcaagaagtt ctccagtttt attggtggga gcaaacaaat aaacagacag acatggagaa 28680
aatgcatcag gtcgggtttg catgcaggaa gttgtcactt gcgagggaat gacagaagca 28740
aaagtgggca gagggagaag ttaaaccaag gtgcagtccc accagaggcc tcagaagaac 28800
ccatgagaag ctctgaaact ggatgggctt tcagagttgt accaaatgga ggcaagaggg 28860
ctggcctcta cccctgtctc atccactaga atgtgggctg ctcccagaga gggtgtgagt 28920
gagttggggc aatacatctg ccttcagtgg aaagcaatta attcccagag agggttgcag 28980
ccattgctcc tccagcagcc agggatatga gtacctgtgt gcagatgggg acctgggcaa 29040
catgccacag catccatgac acggcacaga cagggatgcc caaagtgggg gctggccaag 29100
tcaaagatgg gggaagtaat gaggtgatca tcagatcttc ccatttcacc tgggcattga 29160
cctacatgta ggtgtttttc aagtggacaa tctttagttc cctctgctcc tcatcataca 29220
agtataccag caatggctaa tgttatcttt gcacttgcta catgcccagc tgtgtcttag 29280
ctcttcacat gcatagctta tttaatattc acaagaaccc tacgaattaa gtaatactgg 29340
ctaggcacag tgggtcctgc ctgtaatccc agcactttag aaggtcaagt ttggagaatt 29400
gctagagccc aggagttcaa gaccagcctg ggcaacatgg tgaaacctca tctctacaaa 29460
aaatacaaac aatttgctga gcatgatggc agacacctat agtacctgct actcaggacc 29520
ctgaggtggg aagatcactt aagcctggga ggcagaggct gcagtgagcc aggatcgcac 29580
cactgcactc cagcctggat aacagaatga gaccctgtct caaaataata aataaataag 29640
tgggccaggc acggtggctc ttgcctataa tcccagcact ttgggaggcc aaggcgggtg 29700
gatcacttga agtcgggagt tcaagaccag cctggccaac atagtgaaac cccatctcaa 29760
ctaaaaagac aaaaattagc cgggcatggt ggcacactcc tggaatccca actacttggg 29820
aggctgagac aggagaatca cttgaacctg ggaggcggag gttgcagtga gctgagatca 29880
tgccactgca ctccagcctg ggcaacagag caagactcca tctaaataaa taagtaaata 29940
aataaataaa tacataataa aactattgcc cctgtcttaa tgtaaatgga acatgaggct 30000
tagagaggta aaatgactca aggtcacaca gtaagtcaaa ggctgagcta tctctgccct 30060

ttcttcttcc tggtgtacac ctttcaccct gcaaagctca gtgcctacct tacttgctct 30120
atcaaacctt ccccatttcc ctcagaaaga ccaagtcccc ctcctggtct ctagagaaca 30180
agctgccctg gaccacacac cttggcttcc agccccttca ccatcctaga cgaggtcacc 30240
tagaaggttc catccagact gtgaatgtcc ctctttaact acagtctcca gtgaacccac 30300
aacctcaggg acatatgacc ttggagggtt aagtgggatt tcatttccct gactctggac 30360
ctgggctttg ctagcacacc tctcccacta tgatagcatt cgcttaaggc agctgagccc 30420
cgtcaggggc tccctgagct tgtggtcagc tcaagccatc acatctttct catctgtgct 30480
ctgggaagct gcctctgctt ggtctacaca gctgagacaa tggagcctgg gtgggctcag 30540
gggccccttc tgctgggaac ctgctttgaa acaacatatc cattgcattc actggaggag 30600
catcagaagt agacggtaat ttgtgttttt tcttcactgg atgccctagg gtgggttccc 30660
ccgaagcaga ccctgagatg agcatttatg caactgatcg aggatgtgcc caggagaacc 30720
ggataaggga ctcagagttg gagggaagat actaagaaaa tcatgctcca ggcaaagtcc 30780
cagcctcggc ttgatataaa ttacactgac ttgaggcaag ggagctgggc tctcacaccc 30840
cggctccagt cagccactgt ttcagggctg ccccaggagg acaccagctc ccagcactct 30900
tgccgtctgt gcatgcattc agcacactgg caccccctca aaggccagcc atttaagagg 30960
ggcacaggtg ctggggccac aaagcaccca gagcccagtg atgggtgcac aggcactgta 31020
aaagtgatat gagggccggg tgcagtggct catgcctgta atcctagcac tttgggaggc 31080
tgaggtgggc ggatcaccag gtcaggagat tgagaccatc ctggttaaca cggtgaaacc 31140
ctgtctctac taaaaataca ataaattagc tgggtgtggt ggcacacgcc tgtagtccca 31200
gctactcggg aggctgaggc aggagaatca cttgaaccca ggaggcagag gttgcaatga 31260
gctgagattg caccaccgca ctccagcctg ggcgacagag tgagactcac ctcaaaacaa 31320
aacaaaacaa aacaaaacaa aaaaagggat atgagaggat cagagcagcg tccccttgcc 31380
tcctgttacc aagaaggtac caagccgttg ttccctcagg ttgttcctca ttccatgttg 31440
ctcagccagc caaaatcaga agctgccaag ccacctgggg aaacaaggca cccaccgaag 31500
ctcttgtttt caaaagtttg agctaattaa agttgatgga gctggatttc ccctgaagga 31560
aaggatacca atgtttggga aggttaggat tcagagaaag aagcatttga agtcttcccc 31620
agggcctaga aattaaagtc tagagatttg ggagggtctc tagaagggca ggaacgcagg 31680
ccttcacctg aaccacttcc taagcagata gccaacctca tatgggagat tcctaaaggg 31740
gctgacatca aatcctgaga ctccatagtg aagaccccaa gctgcagcct cgcctggaag 31800
tataggaact taccagagta tccaatgaca ggagaagtcc agagggctct accctgaatg 31860
ttcaccatgg ataagtcctg ccccaaacct gacagataca gatttctcat ctatcttgac 31920
aaatgggaac ttgcagccag tttaatttgc ctcaaagaaa ataaaataat gtgacattcc 31980
ttcaccccca gcaagaacta atccctttct ccagtgctcc tacagcaggg aggacatttc 32040
cctgatattg atttttcatg actgtccttc ctccctagga gaccctgatc tccttaggga 32100
agaggtggtg ctttttaaac catttctgca gcccaggtgc ctatcaaggc cccagcatcc 32160
agtgggtctc aataaatgag acctatcata attaacattc ccctacctca gcacatgact 32220
catcaaagta ttacatgttg tttgcacttc tctaccccct cccccttccc atcacctcaa 32280
agctctccaa gcacacatga gccactgctc ataaatgttg catgaaagaa gggatggatg 32340
gatggatgga tggatggatg gatggatgga tggatggatg aatggatgga tggatagaca 32400
gacagatggc ctgatggatg aggctggtga gcaagtgaaa gagtgcttgt tttagaataa 32460
gctatttagg ccactgccat ttcctgttgg ctgagcaatg agcatgccac agggccaatc 32520
agctctaaat gcaaggcggt gcatggccag aggaaagttc ctgctgtctc tccttcaccc 32580
tgacagccat ggccccaagg gctaacagag gcccttgaga ccaattctgc catcaggaag 32640
gaaaagccct gccaaaatat ttgcattcag aaagttagca tttataacac agtcagagaa 32700
ctatgaaata tcttccaggc caagcactgg gcaatgccat gttggtgggt tctgatgagc 32760
cctgggaaac ttacttgcac cttttcagcc cctaacccta ttacacaggc ccctgcatgg 32820
aacaaatgcc ttcatcctca catgttgctg ttgggagtgt actttgatac agcctctact 32880
caagaacaat ttgggaatac ctatagacat tttactaaca aatgtgcacg ccatttgacc 32940
caggaattct actcctagga attaatcctg cacatatact cacgtatatg tacaaaatcc 33000
ccatatacaa tgtttgtaaa aagcacggtg tgaaagccat ctaactcttc atcagaggga 33060
ctgggtaaat aaaggatgtt ctgcagccat caaaaagaat gaaagttcta catgttctga 33120
tatggaacaa tccccaaaat atattgctaa gaaggaaaaa acaaggtgca gatcagtgtg 33180
catcatttac taccatttgt gatggtctta tatagcacag accagttctg gaaggatcca 33240
taagaatggg tcccaagggc taggtgcggt ggctcatgcc tgtaatccca gcactttggg 33300
aggccgtggc gggcagatca cctggggctg ggagattgag accagcctga tcaacatgga 33360
gatatctcat ctctactaaa aacacaaaat tagccaggcc tggtggcaca tgcttatagt 33420
cccagctact caggaggctg aggcaggaga attgcttgaa cctgggaggc ggaggttgca 33480
gtgagccagg atcgagccat tgcaccccag cctgggcaac aagagtgaaa ctcggtcaca 33540
aaaaaaaaaa aaagaagaat gggtcccagg agttgcctcc aaggaaggga ccatggggac 33600
tggggactga ggactgggag acaagagtag agaaatgctt ctcatattgc acaatttgga 33660
atttttttgt atcatgtgta tgtattactt atgcttaatc aaatactcaa ttttaatatt 33720
aatttaaaaa aataataata tccccatcca cccagccagg tgccatagta tctgacattc 33780
tgccaatagg gagtcacaag gttgaagtct tagattgaac attgtttcaa catcatttct 33840

cactggaagc tctttataat gcaagcctta ctagaactgg tttaagtgtg atccaaccta 33900
tcagaccaac tttcctcttt cgggcataca aaaaacttca ctgggcattg actttgtcct 33960
acacacttgc ccaggcactg gagagacagg gagaacaggg ctgacctcac agtcagccag 34020
gtggacagct gcacgcacag agtggtggca gtgcacaggg agtggtggct ctgcacatgg 34080
actgtgggcc ctgcatggga ctagtggcac tgtgtacaga atgatggccc tgtacaagga 34140
gtggcagccc tgcataggga cctatggccc tgcacaggga gtcgtggcct tgcatgaggc 34200
cggtggcggc ctgcacggga ctgatggcac tgcacagaga gtgtggccct gcacaaggag 34260
tggtggccct gcatagggac ttatggccct gcacagggag ttgtggccct gcaaaaggag 34320
tggtggccct gcacagggac ggggcagata taccctgaca tttgaggaga ctgaggcaag 34380
gatataagtg ggagagagtt agaatttcga attctcagac tccttgggtt tcatgctgga 34440
acacgctggc atgagagaac caggccttgg cctgtggccc agctccctcc tcttcccacc 34500
tcctgccctg tcccacccca tcaggggctt gcacacacac acacacagac ccttcagccc 34560
tcagagccaa gctccttgca cacagactct ttccacagtc agcgccctta gctcctgtca 34620
agcctccagg tacacacaaa cctgcgtctc tgctcgcctg cacaaggaag gacccagcca 34680
agtgtcccaa tagggcctgg aagcgatctt gatgccatcc gggcagaaaa tcccaggtgg 34740
agtacagccg gataaagaca gcccaggttt caggttggca tgttcctccg gttctgcaga 34800
tccctcgggc cctggagaag gagatggcca gaggaggata gcaaggcctt ccaaagtgca 34860
gtcccgggtc acagcccacc tgcctggatc taaggacggt gtcgctgagg ctgtggcagt 34920
gggaggttcc aagacacggt gaaggcctga aggagggggt ggcccttgct aagacctgtg 34980
gccagctgac agggctcccc agcactctgt cagtaaggcc cttccaggaa gagggagtgg 35040
ctggacaaga ggctgtgtgt ggaaggcaaa ggggagaagg gagagagagg gctggtaacc 35100
caattcctct tcctgcctcc ctacacttca gcatcaccct gttcacgccc cttgcagatc 35160
tcccacagca ctccctgtgt acacctttgc tgctcaaagg gtctacacaa gccaagcaca 35220
gtggctcttg caaaggtggg aagatgtctt gaggccagga gtttgagacc accctaggca 35280
acatagtaag actctgtctc tattaaaaat aataataatt tttttttttt ttggagacag 35340
agtcttgctg tgttacccag gctggagtgc agtggcgtga tctcggctca ctgcaacctc 35400
cccttcccca gttcaagcta ttcccgtgcc tcagcctccc aagtagctgg gattaccggc 35460
acctcccatc acacccagct aatttttgta tttttagtgg agacagggtt tcaccatgtt 35520
gcccaggctg gtctccaact cctgacccca agcaattcgc ccacctcggc ctcccaaagt 35580
gctgggatta gaggcgtgag ccaccacaac cagccaataa taaaaatttt ttaaagggtg 35640
atctaagatc agcagcatgg cctcggaaat tggaaatgca gaccctctgg ccccacctca 35700
gaaccactga attagaattt gtatttggat aaaagtctca ggtgagttct gcaaactttg 35760
taatttgaga agcactggtc tacaccacac tccagcacct taacgcagct ccatttagca 35820
gtggtggaca ttgcatcatg cagtgcatgg ttccttcttg agtgcagctt atggaaggag 35880
ggggagtgac aggactgaac ttattgttca aatccccttg tggtcttggg gttcttggtt 35940
gctcactgca gagtttatag gtgaaatgag cagctgtcct cacctctcca gcacccttgg 36000
aatggaagag gctgccctga gctgggctgc catctcctgg gtcctcagtc tcaagccgct 36060
ttctgatgtc agtgagctct ttgaagtcag gtccgcatcc tacacagggt aggtacaggg 36120
ggccgggaag gcatagaata cacattaggt tggatctagc atctgatcca cctccccatg 36180
tttcacgaat gggccagtgg gtgagtctta gggagggcta ggccctgcct cttagaggaa 36240
ggcttaaaag gccccaacct actcttcttg gcctggtcag ctggaggcag gcagatgccc 36300
caagttcaga cagtcagata ctcccaccag ggatttgggt ctatggtgag tgacatctag 36360
aagcagggcc agccagaagt ccttcttggg gctgggcaca gtgactcgtg cctgtaatcc 36420
cagcactttg aggggctaag gcaggaggat tgcttgaagc taggagtttg agaccagcct 36480
ggacaacata gcaagaccct catctccaca aaagtaaaaa taataataat aataaccagc 36540
catattagag cattcctata gttccagcaa ctcaagaggc tgaggtggga ggatcactgg 36600
agcccacgag gtcaaggctg cagtgagcta cgattgccct cctacagccc agcaattgca 36660
cacctaaccc cacatacaca taatcaaata gagattatac attcacgatt gaagccaaag 36720
tgttatgaca caaaacttac ccttaggaca tgtcataaga tctgatgttt tatattttta 36780
ttcttttttg ttaaagaaag agcttgttga tccaaccctc taataaagct gaaagccaac 36840
atttgcaaac actggcaggt ggctgcgtgt cagggtgagg tgtcactgaa cagggtgata 36900
aagcaagatc ttgtctctaa aataaataat ataatataat aaatataata taattaagta 36960
gaataatgaa atatcctgct ggccaaggtg gcatccccgc ccccagcagc aaagtcagca 37020
gggccagtgg tggcaagctg gtgtccacac cagtggccac agtgtcatgt ccaggcaatc 37080
tctgccctat gacctcagct gcctttccag ctgtctttgg tttctgcctg tttcccttca 37140
gcctccctag attctgggag gggccggtgt cctgctccag gtaggcagag tccaagtctg 37200
ttgtgggcag aactctgatg atcattgcag gggtgggagg agggatggag ggagcatgat 37260
gctggcagaa cctccatggc atctggggaa ttgaaggctg ttttttgctc agttctcacc 37320
atccaaagca gcatgaacag ttttcaccac gcctctcttg tggatgcctg tgttcctggt 37380
acacacctct gctatgacac ttatcacctg gtattagtta agcatctgcc tccttcattt 37440
gctgtgggta tttagaaagc aatccatatt tcctctctgt atgcctcaat ttctccacct 37500
ttattagagg acccaggata agattatttc tgaggctgct tccatcagtg gttctgtgag 37560
ctggcccatg tattcagcca gtcagtaggc catcaagcat gcatctacgc aaggcactag 37620

aagggttaca tacaatggga aactgaggcc cagaaggagg aaagatgaag tgcccaaagt 37680
taaggagata aaagcagaag tgcaggtaag agaaggagta ttcatattac tgcaggaatc 37740
caaattctac aagaacatac gcttgttctt taaagggagc cctcgcttta agcattattt 37800
gccgatcaat acccattaat aattacagga catgggcaaa tattctagca aaggtcctga 37860
ggtcttccta gatgtgccca aaataacaag gactcccccc gcaggtggca ccaaaagtcg 37920
gaggatgtgt tggaagctga ccttccccac ctgtccctgc ctcacccaca gtcacccacc 37980
cctctaggat gtaatggtga atcattcaac acatgggcca ccttgggaag tcgcttaacc 38040
tctctccctt ttcacgttat aaaatgggca tagtcattac acaaatgcat aaatcaacct 38100
caattttctc atctctaagt gagaaggttg gactggaatt aacaagagga tccttgaagt 38160
tctttcgagc tctgacatcc tcatccattg caaagcggct gtgaaactgg attatgtaag 38220
gtcctgtggc cccaccaatt gtttggcaca tcctgtcttt ttccctagct agataatgat 38280
gtggctgagg gcagggactg tgcccacact caccaggtgc atgtgagtca gcgtctggat 38340
tgtgtggccc cgctggggct gccctggctg tatcacatgc ctcttgtgcc catctcatac 38400
ccctcagcca ccctctgacc acagcattgc ccggcgtgcc ccctgcaggc ttttattctc 38460
ctgcagcccc agggcctcct caacactatg atgtgagtca cctgagaagc ccagtcccac 38520
aggcatgcac agctcaggag tgcaggactg tcaaggactc ttgcagcaac cctcagccat 38580
gtggtaggta tgtggggtgc agggtgggga ggcagtggca tacacactcc ccagaagctt 38640
cagtgggatg gagatcagct caacaacaca cctttgactt gacttcccct ccttctccac 38700
ttcactcccc cagcccctca ccctgctccc tgagctctcc tcccaaataa actgcatgca 38760
tgcaggcaca catgaccagg ctgaggctct gctcttagtg ggataccagg caaagacacc 38820
tgccctaggg attggagcct aagccagagg aggcaggagg gtttagggca ccagccaaga 38880
agtgctttcc agatgctgag tgggatcccg aataagggaa agggcatcta acatagaatg 38940
aggaggagat aacacaaggg tagaaataac caagaaaggg gatttctctc agatggcttt 39000
agaggtgctc ccagccattg ctgggctcac atggctcaca tgtcataatt tttgccaagg 39060
cggtttcact gccaccacca ccccgctcag cctccagtgg cttgtccacc catggctgtg 39120
gcctctaact gggcaggatt ttataggcta ggaagaaaag tagggaatgt taagggagac 39180
ggaagtcaag ttcaacttgg aaccatccca gaatgtccag caggaaaggc ccatggatgt 39240
tataggctac aaccctctca gtgctcagcg gaggaactag gagcagagag ggtgagtaag 39300
tcaaaggcac ccagaccttg tagggaatcc tgtgtccctg caggatgcag aatggatgct 39360
tctgaaccct gccacaccct cagggtacat gtcagagcac gtgtgtggct gggacaatcc 39420
tccatctatg ttcctcactt acaccggctc cagtttagga aacccaaaag gctacaacca 39480
acccaccacc acagtgacca caatgtactt ggtgacacct caccttgaca cgcagccacc 39540
tgccagtgtt cacaaatgtt ggttttcagc tttatgcgag ggtaggatcc aacaagctca 39600
ttctttaatg aaaaagaatg aaattgtaaa atatcagatc ttattacatg tcctaagggt 39660
aacttttatc tcataacact tttgcttcaa tcatgagtgt ataatttcta tttgattttg 39720
tgtctatggg gttaggtgtg caatgtagaa aagcttgaaa accattgcct tatacccatt 39780
tgaaaaatga accaccaaac aaaggcttaa aaacatgaat atttgtcaac caaaaggaga 39840
actcctgggc ctggaagaaa catcacgatt caagtgctgc agcacgaaag aagttgaggc 39900
tggatttgta ttcagcttga ctgtttgtaa agcaccatgc accatgcaac tgccttcagt 39960
tccttttttt tttctttctt tttttttttt tttttttttg agatggagtt tcgttcttgt 40020
cgcccaggct ggagtgcaat ggcacaatct cggctcactg caatctccgc ctcccaggtt 40080
caagagattc tgctgcctta gcttcctgag tagctgggat tgcaggtgcc tgccaccaag 40140
cctggctaat ttttgtattt ttagtagaga tggggttttg ccatgttggc caggctggtc 40200
tcaaactcct gacctcaggt acttggcctc caaagtgctg ggattacagg catgagccac 40260
catgcccagc ctgccttcag ttctaaccac taaaggattc gtgttctcaa ctgagaaaca 40320
gccatatgga ggggcttcct gcaagggcac tgaagatgtc agagatgatg cctgtggagc 40380
acctggcaca gaggagctgc cctgctgact gcagcacagc cagttcacag ggtgagcccc 40440
cggcatcagc ttagggagca tcccttcttg cagctcctcc ccctagcaca ctctcccacc 40500
tctgctcgca caggacctct gtccaggagt gcttacggca aggtttctgc ctcaaaaaca 40560
gggcttttgg ggctaactgg gagtgtaagg gtaaaaggaa agcttccact aggccccccc 40620
gattgttcac tggaaatgaa gggacaatag gagattaata ggagaagaag gcatacaaat 40680
gtattaacat acataaacac aggagccatg aaaatatgag gctcaaagaa gagccagatg 40740
actgaagttt aaatagcacc ctcttcatag gagagaggga aatgggggta ggtaattttc 40800
aaggttagta aataattttg agggaaatca aatggaccca gaaggcagac attttcctgt 40860
aaatgtcctc ttaggaagct gaatgggact gcaatttaag ggaaagtgag gggcaaaact 40920
gcattgtgaa caaaggttgt cttattacat aggtaaagtc tccctagtca tccctcaaag 40980
ctgccctcag aagaatagat gaaaagcctg ggcttagtga cagcttttac tcttttctcc 41040
ttgcaggtgg gtggttaatc tttcctggtt atttgatgag atttctaggg aggggccctt 41100
aagacagttg catttctttt ggaaagaagt gtccatagtc agatatggaa attctagaca 41160
gcctcttttt cctgcacttg gggagggggc agaggagaaa caagagaaga ttggaaaatt 41220
cttggttctg aggcagcttc taaggacttg cattttcctt taattcaaag tgctcagcct 41280
gccaacactt cctactttgg ggtatcattc tctgtgaccc cacaggggaa tggggtctgt 41340
cctcagagaa tgggggagga cctcaaggca gcagggcaga actccagggt gagagtcaaa 41400

gatggtttcc tggacctgag actccagtgg ggttcatgct agagtcaccc cagtcttcag 41460
gctcactgcc caaacacgca cttgaggaag tctgtggggg cagctccttg ttttgttcct 41520
cactgtcatt ttccaaaagg cagacaagcc ctggccgcct tatgcccagg gctgagtcca 41580
caccatgcta cacgagcacc tgaggggagg ctcctctcca aagtgcatta ccagggcctg 41640
tgctaaattt ttcagcagcc tcttgtctca ctaatggtcc cgggaaatag actaagcctc 41700
catccccaac taagtgctca ggcctccccc acctctgtcc tgcctgggca agaagcccag 41760
cgccgcccca cagcccagct ctccatgcag cctcagctcc cagtggccct ctgccaagga 41820
cttgcgcctc tgcagtggga tgcagtgcct cattttatcc ataattgtac gtggtccctg 41880
tgtgatgtcc ggggacgtga aaagttgtgt ctcatttcat ttgggatcca gttaaattcc 41940
caaatgctgc aactgagcag gaatctcagt gctcagaggc cagcagtagg aggagtctgg 42000
ctgctgggcc cggacacctg gcctctccgg cctctgacca gctctggcca ttggtcacca 42060
ccctggcttt gttgggtatt aatccaccac actctgcctg gctgatccct gagtcagagc 42120
caggctggga tttgtgggtc tctgagccca ccagcctgtg actcccaaaa agagagcacc 42180
cttgctactt ggaaggcttg ccaccatgcc tagccagcac tcttgctttc taagctgaaa 42240
tcattataag atagtcccct actaccttct acccagaaat gtatcccctc acaaagtata 42300
tgttcccacc ctatcccaat cctaccatcg tgatttctga gtactttcca gaaaactgtc 42360
tccactggct cccaagggca gagcacttcc tccagcctct ggaatgtcca ccatgttgat 42420
ggtgcatgga tgataatgac cccacggatg agctctctcc agtgggatca cgaaggagaa 42480
atgggatccc tgactcccca cagaacagct cggcccagct gtgaggttga gcaaatacac 42540
tcaaagctca gcgttaaccc aagtccagcg ctctcagctt acacaggtgg cagcccaatc 42600
ttagtgctcc actcaatcca tgtctatgta tacttaattc tctggaacta aaatggggaa 42660
tagggccagg caccatgact caagcctgta atcccagcac tttgggaggc cgaggtaagt 42720
ggatcaccta aggtcaggag ttcgagacca gcctggccaa catggtgaaa ccccatcttt 42780
actaaagata caaaattagc caggtgtggt ggcgggcgcc tgtaatccca gctacttggg 42840
aggctaaggg aggagaatca cttgaaccca ggaggtggaa attgcagtga gccgagatcg 42900
agccattgcg ctccagccta ggtgacaaga gtgaaactct atcttaaaaa ataaaaataa 42960
aataaaataa atttttttaa aggccggagg gactaagatg ggagtgggaa ggagggaggt 43020
tccagctcaa tcctcaccag cagatattcc tgcatcctcc atacctgagc cttcagacca 43080
atgctcccca ggcccaaagg ggacctccct ttcaagtata aggctcccct aattcctcac 43140
cttaggatct ttaaggcaga tgactgcctt aaagaacata tattccttgc agcaagaaaa 43200
cccaactccc aacaccccac agagtgtggt ctggcctgga caagtctgcc ctaagaaaaa 43260
agcgacaaag acccttagta attgaaacag actttaagta ccataaagat gagctttcaa 43320
tcattaaaaa aaaaaacgtt tactgagcca ggcaccaggc ctaggaacag agaggtgcat 43380
ccaggctgcc atgttaggtg ggcctgggcc ccactgctgg ggtcagaagg tattagaaag 43440
ggtctggtct gctgggcgcg gtggctctca cctgtaatcc cagcacttag ggaggccgag 43500
gcaggcagat cacctgaggt ctgcagctcg agaccagcct ggccaacatg gtgaaacccc 43560
gtctctacta aaaatacaaa aattagttgg gcatagtgat gggcacctgt aatcccagcc 43620
agctactcag gaggctgagg caagagaatt gcctgaaccc aggaggcgga ggttgcagtg 43680
agccaagaaa gcgccactgc actccagcct ggatgacaga gtaagagtcc atctcaaaaa 43740
aaaaataaaa taaagaaagg gtctggaagc tctaggtgac ctcatgctca catctatacg 43800
cacaccttga tacattattt atggatcagg tggcacagca ttgcaggcag cagcagggca 43860
aagacagtgt ggaatcagag tcgggcagcg ttggatggga ttccggttct gccaccactg 43920
gctatgtgat ggaaatctac cacatctctc caagccttgc tgtcctgttt tatcaaatga 43980
ggacattgta ctgaatcaca aggtctgtac aatgcccgtg catgtgcctg gaatcttccc 44040
ctctggtgtc ccctcaatgc ccatggtgct tcctgcacag ggtgtaccat agttgcttgc 44100
tgtctcctgc tgaagcctgc tgtgaagagg gactgtgtgg aggtaaggca gtgggcctct 44160
ccctgtgagg tcctcagggg gctgaaggca aagtcagaag taaaaaaaga gaggggggac 44220
ccatccccaa ccagctgcca tcccagggca ctcagcctaa ggccactgca tttgcatgct 44280
atttacataa atctgcatat acactgtatt cccactctat cttgtaggca attattgcat 44340
ttaggcagct ctgcattcca ggaaggggca caagcagaga tgtgatcccc tgaacttcat 44400
gacaagaagg tacccgaagc tcctctccca cctccagcag caaatgtctc ccctggggct 44460
gtcctgtgtt gtgtttgaac cctgctctgg ggctcctgcc cgccacctgt agtggctacc 44520
atcagaagtt cctgttaatt ggggatggca gggtcaccac atctctactc ttctcccact 44580
tctcagcagt tctaatttaa accaggttag aatcttccca cttggtagtc tggtagggaa 44640
attatagtta acaataattt attaataagt taaaatagcc agaagagaag aactgtaatg 44700
ttcccaacac acacaaaaaa gataaatgtt tgaagtgaag cattgaagtg atgctccaat 44760
tatcctgact tgatcattac atattgcatg caagtaccaa aagatcacat gtacccaaaa 44820
atatatacaa ctatgatata tcaattaatt ttttaattga ttttttttct tattttcctg 44880
tgtagtggca gaatcacagt tcactgcagc ctcaacctcc tggggttcaa acaatcctcc 44940
caactcaacc tcctgagtag ctgggactat aggccctcac aaccatgccc agctaatttt 45000
ttgtgtgttt ttgtagagat gggggtctca ttttgttacc cagctggcct gtaactcccg 45060
ggctcaaggg attctcttgc ctcagccttc caaaatgatg agattacaag tgtgagccac 45120
cgactctggc tgatatatca attttttaaa aaaaaggttc ttctccaggc ttcaatgcga 45180

cttcacctat accaggtgtg agcggccctc atgtccaggt gggtggggcc aggcatctgc 45240
agggacctca ggcttcttgg attgtttggg atggaggccc aaggaggcgg aaggtctggg 45300
gtctcaccca agctggctct ccccaaggaa taagagtaat cacatgggcc tggagcagaa 45360
aagggaagag aagggtcccc aggaatgatg atttctgggg cttcaggaaa gagtgtggaa 45420
ctgaatggca gagctaaggg aagactagga cttacaacca ccaaaatcag gccctccagc 45480
tgggttcggg gccaagggag agtgcagcct ggcttctgcc attctccttt catccccctg 45540
gagcttctga aatccacaga attgtggtga aggacaagga gccaagggct cacaggcgag 45600
gggaggcggg gaattcaatg tgttcaaggc ctacattgca catctgccct tggtttactc 45660
tatctaatgt tcccatttga ccggtaagaa aactgaggct caagattcaa acccaggtcc 45720
ttgtaacttc aaaaaccttg ttctttctag gtgtcaaagt caatagatgg gccacggggc 45780
tagcagtcac ttctcttcct ggacttggca gtcaagggat aggacctgaa ctaaattgcc 45840
tttctacaga cttttcagcc ccaaccagag gcctgctgca ggcagttgtt tgaactcctt 45900
gattaaatgt tcagcccctg gcctgggcgg cagactccca acacacaaga cttactgagt 45960
cccccaaata gacttcaaaa cacagtcctg agactctgcc tgccagttgc ccattaagct 46020
catcccagcc tggcatcttc ctttgttggt ccactctccc tatggctacc agggagaaag 46080
tcttttcaga gatgaataag ggcgtccaga caacagagat gtagtagtag gaggtggaac 46140
caaaacagaa ccaggggtct gaatcctggc tctgccactt tctgagatat gtcacatcag 46200
acaggtgact taacctcctg tgtctcagtt tccaaattca aaaacggaaa atgtattatg 46260
acgatcagat gagttaatac agtcaaaatg cttcttagaa cagcatctgg gatgtagcaa 46320
gcactcaaaa acatattagc tattatgatc acacagagat gcatacctcc taaaatcttt 46380
ctgggctccc accgccagca tgataaagcc tcacttctca ccaaaggcaa atcaggcctc 46440
tcatcatctg cctcctccct ccccagcccc atctcccgaa gcgcctctca gggcacctct 46500
gcccttgcac aaggcagggc ctgttcctag caactctcag agtagcagtg gaagtttcca 46560
acaccaagat ctcagatacc tcaagctggc aggagtgcga ggtccaacaa gaagagattt 46620
tatgaatcag ccagccatgg gggtggggca tggccaggtt tcctgattcc cagaccaggg 46680
tgccaacaac acctactgtt catgtgagaa aaggagaaag aggaggaggg gccactggcg 46740
tcttttgccc acctggggtt cctcgctgtg ggagaggggg aggccagagg acagcaagtt 46800
ccctgagagc ctctcagaga aaaggagggt ttagatcatc acacaggata aggaagaaga 46860
ccttttccaa gggggtggag cagagcctgc ctggaggtaa atttcctttg ggaactgtct 46920
tcttcagtat ttcccttcct ctctgaacac cgtttctttt cctttcagat tatctgacag 46980
ccttttaatg aacacctact aagtgcccag cactttcctg ggaacttaat tcttacaacc 47040
tgttaagagg tctatacact cattttacag tggaggaaat ggagacttag cggagatgtg 47100
aaatttgaac caggtccgtg tggtttcaaa gcctgcgcat gttcctttac cctatggaag 47160
cctccagaag ccaaggtctc atctcccctc ttccctggag gccccaaacc tgggtagctg 47220
tgcccctaga cctttgaggg agggtctcga gccattatct cccttacctt ttcttgcagt 47280
gacttggctt ctcccaggct gccgtaacca gcaaacgcct aaagatggct ctgagacgat 47340
ggccttctgt gtgtgtgtcc atgaaatcag gtagtacacc acacagaacc agactctcag 47400
tgacatgcct gatattccac agacctgcat ccgagctgtg cagatctcag gtagacacaa 47460
ccagtgattg gggatggctg gtaggatctt gcctaggcag gccagggagc tctgtgagga 47520
gtgtttggga gaaaatcccc caggttcatc tccctgcatg gactgcccct gctgggccct 47580
cacactaagc tggtcctggt cctcagtact gagggatcac cacaggtgtg taggccacag 47640
ctcacaggag ccctccacct agcgggtcaa aggtagtgga agatctgaga gtcaggcaga 47700
cctggtccaa atcccggatc tgccatttcc tcactgcatg atactaaaaa agtcacttct 47760
tcatctgaaa aatggggctg atcccaccca ttctgcaggg tctcagtggg gattgaaggg 47820
ggctgacctc aatgtagcac gatgcctgcc ccaggtggga cttcagcaaa ctctctttcc 47880
tttcccttcc ctccccttga aggccaagac caaggaaaca gaattgggca tgccagaaca 47940
tatcccagcc ctgtggggtg tatttacctc cacttctgat cacagtgtgt ttgcgtatga 48000
ccttgaggcc agtgactttt acaccttgcc tatgctgggc tgggctgtga cttggctgtg 48060
acttgcctgc cctgaagcca agcctcagct gacatgtgtc tttccaccac ccctgctgtg 48120
ctgagtgagc accagccacc tgtcccattg ttcctgcccc aggctgtgtg tggagagtgg 48180
tctgacaggg cagacaacac cctacactcc agatgtgttg aggtgagctc tgggggtcag 48240
gaaagaagca gggaggggca cagtgtgaac atcctgtgca gatttccccc caactggccc 48300
cccagctggt tgggtttctt tttctcagta actcctggca gaagtccaag gaggcctcct 48360
tctcccaaac cctctgtcct tggctcaaaa atccagcaga ggacgtgcca cgaccacgaa 48420
aggtgacagc agtcaagggg aaagaggact cccttactgg agtgcacaga agtttcctat 48480
ttattttgct tacgcggttg actttttcca aaggctgctt gggacggctt acctcgaaaa 48540
gcttagcaat aaggctagta gtaaatcaga caaaaaaatc atggactggg ggaaagaaag 48600
tagcatttaa atatatttaa taaggacgat atagtcgtga acatgtgcct gagtatcctg 48660
gctaccacag taaaagggga aatatgatca gttatgggaa aagagtaaaa atattaatca 48720
tgattttttt tttcttggag ctttgtgtaa taggcactga tgttgaaaag ggtagagtta 48780
tttttccata tttgtagaaa ttcagaagat tatcagacta tttgcctcac aaggactttt 48840
tagcaagcaa aaagtatgat attgtcatgc accctggtga ggggaagtgt catccagttc 48900
taggtttctg tgaatctacc ttgatgcagg tgttcaggga gtagtgagtt tcctcagttg 48960

taatgtagac cagcgcttct caaacttaaa cacgtataca acctcctagg gggtcttgtt 49020
aaaatgccat tctaatttgg gagctttggg gtggcctgac actctgccat tctaaaaccc 49080
tcccaggcca tcatgatcac caccttccga gcagcaaggg gttaaaaccc aggcatctcc 49140
catgatctag tagtgagtgc ctttctaatc gcccccagct gaaaggcaat gcaatgttat 49200
catagacggg caagaagaat ctcttccttg gctctgtaaa tgcttccggg atgtgctggg 49260
tcagcctccc tccattctgg ttggcgatct tggtctagtc acaagccaag accgttgatc 49320
atacacaaaa gtttaagtac ctactacgcg ccaggaagct ccctcagaaa gatggttggt 49380
cctgctcccc cacccaccct ccaccctggc atcttttatc cactcttcct tcagcaactg 49440
cctttatcaa tgctcccttg agccaaaata aacaaataaa aagcaacttc ctttggaagc 49500
tactccatac tcttgcgctg gccacagtta catctccctg cagatacaca gattctcatg 49560
acacagagct cagacagtgc ttcctctctg gagggctcat gtttcgagag aagactggaa 49620
ggaggacaac cttatgctcc agtagaattc caaattctgg ggtgcaggga catttcgtgt 49680
gtgtatgtgg aggggggggt gtcctaatgc cctgtaagcc acactttata atgctgtaca 49740
atattgggtc accatcatgg gccatggaaa gcagaatctg tgaggttcag tcccaggctc 49800
cagcacccct ttgtcagctg gcctccccgc tcagtgccct ctcccactac ctgccctgcc 49860
tcagctctga ctgctgctga tgtgcctcca cccatcaggt cattgtgatg gcaaagacaa 49920
tgcctccatt tgccctgaat gcagccttac ataggaaaga gcttcctgtg ctccagaact 49980
tggaggagtc ctgcagcacc tggtctagtg ggagagcatc cagtggggag cggcaccttt 50040
cctgcagcca cggcccactg ggcaggacca gatccaagtg gcctggcttc tggctagggt 50100
gtcagagtac attggggcat acagcttccc gtggccatct cgttttcatg gaggatttat 50160
tttgtgaagt ggacctgctc tgtcccatcc tataaatgct ggattaatcc aagaagctga 50220
gggtgagcaa catccctctc cttcctgatc aaataagaaa taaatctaga ttttctcctg 50280
gatgctattc tcttcttagc caaacagcta accttgtctt caaggaaaaa gggcaatccc 50340
aggggctctg taaattgaat attcagcaac tccaggggca gacatcccta agtgcataag 50400
tggagcaccc ctctgaatca gcatcacacc agctgggggc ataaaaagga gagcagagta 50460
ggcaggagct gtggggacat ttctgagatg ctaaaagagg caaggaagtg tgaggacggg 50520
tgtgcattca tgcaacaggt atttactgag cacctactat gagccaggca gtgctctatc 50580
cattagagat acggaattga ataaaacata aaaatccctg cccaggagga gctcacattt 50640
ctatgggaga agacagttac tgaaaaacag tccaaaaaac tctattttat agtgtgttca 50700
gaggtaatac aggctatggg gaaaaaaaga aagcaagcag gtacatggat gaggaatggg 50760
ggcgatcagc gtgggcctca cagaaatgat gacctttcag ccaagacttg gaggaggtag 50820
ggagggtacc tggggatgcc tagggtccag gcaaggggaa cggcaggggt caggatcctg 50880
tcagattcat ctgaggtggg actgaatgtg agcagagaga gggagacaga ggatgagggc 50940
agagagaagg ccaatcacac tgggccacgg ctattgcaag gactttactt ttgctggggg 51000
agggggtaga gttttgagca gaggagaaat gcgatctggc aaacatcttg caaggatggc 51060
tctgactgtt ctatccttgg cagaggacac cagagtctgg cccatagaag gtgctcaaca 51120
aagctcagca cattgcactc ctccctggag taggaaaggg ctcgtctgct ttgggaaatt 51180
tggctctcaa gcttctggaa acacctgacc ccatgccagg tgagacaagc tcacactcaa 51240
ggccccctta gtccaacggt cagtggacag aacaagcacc aaaggtcaac ggcgtgggcg 51300
gtggcctctc tgccaaagtg tgtgggccca agtagggtat tcacctccct ccctgtctgt 51360
gagaaatttc agacccctct actttttcca gcaggagttc atgttccttt taagataatc 51420
ctctatagac ctctggcagc ccagccttca aaatgtctcc ccctgtctct cttactcatg 51480
gagagccccg cttccccgcc tcaaaaaaaa gtcttcctct ctcttactca tgcatggaga 51540
gccccacaaa aagacactgg ttcccaggga ttcttccaaa gatgctggaa gacctctcag 51600
tgtttgcatt tcccaagctc ggtttccttg tgctgtcctt gggcattaaa ggaaggctat 51660
tcagccatcc ccatcagcag gagcaggcat caaaagaaca gctactgcag ggggagcata 51720
ttaggggcac cagagggggc taggaactgg gtcttgtctt ctcaaagttt acaacccact 51780
gcaggggcca ctcaccagcc ctggcaccct aagggcagga acctccattg ggcacgtgtg 51840
tctccagcac tgggagcact gtctggcacc tggggcactg tgagtgttga atggataaag 51900
gacctgtaag aattgccata agaggtgttg ccctggggca aggtgggaca ggaaagttct 51960
tgcaagagaa gagaggctct ggccttgggc cctggctgag cagagagctg ggaggtgggt 52020
attcgggtag gaggaaagct gtggaggggg aaagcaaact gcctgcgggg gtgggggggt 52080
tctcggcggg agttgggggt ggagggaaag gcgctttaca gcagcactcc ctccacaggg 52140
agaccagagc tctgcctctt cctccccacc gtgccctttg gggtccagtt gtggggcctt 52200
ccttgaggcc cagctcctgc ctctggcctc cggaggctta tctccccaac agaggctggc 52260
tccccagaaa acaggattct tagacttcct aaaaataacc tgaagctcct tatcagaggc 52320
taatagtgta tgtctcccag gaatactcta gaaagagtac caggggtcag gaacccccac 52380
tcagcctcca cgaatctgag cggaaaagcc cgggtctcct cacctgtaaa gaaaataact 52440
gcagccggca aatgtgacca caggtgagaa ggggcttcgg accagggcgc acacctctag 52500
agaggtgccc acagacagcc tccccttact tacgccctgc ttcgctgcag tgctgtgaag 52560
ccatcggcat tggaagtgcg gagggcagcg ggcagtgccc cagggggaag gggggacggg 52620
tagggtttca gagcccagaa ggcctagaca gggaggcagc tccgggagaa aggtacccgc 52680
catcccaccg gtcttggaac ctgaggactg ctgggaaggg tcaaaagtct gccctaaggg 52740

tggacaaggg acagagattg gggggggacc acaaaggcct gggcgcaacc ttgttgagag 52800
ggaagggatc cggacccaat ctggagccct ggtctcttcc cacctgcaac cgtggctgcc 52860
acttggcctc ggttataaaa tggacaagga ttcgaggtgc tcctcaggtt aagagagggg 52920
ggcctagagg tcaccctggc ctcggtgccc tccgtggccg ggttaagagg aggtcccgga 52980
gttctgctca cttcagccgt gtgccgggca ctgcaaatca ggaagtgttg gcgccggctg 53040
gcgacctccc gcctggggcc aggggaggag ggtggttgga cgctgccacc gctgccgggg 53100
ctgtgcaggg ctgggcgggg agcgaggacc cggcggctcc tgattgcggc cccgggggag 53160
gtggccgagc cggataagct gcggcgggct ggagggcggc cacctcccct gcaggtccgg 53220
ccctcccggg cgggtggggc gcgggggagg aggagcctcg ggccgagcca ccgccttcgc 53280
cgcggacctt cagctgccgc ggtcgctccg agcggcgggc cgcagaggtg agtgtaccct 53340
cccccggtct ccgcggggct gcgtgctgcg cccggtcccc gagacgcccg cccggttgca 53400
ccctgcgccg tcgctgcgcg gacctcgggt gccgccacac gtctggaggc gacttctgtc 53460
ccctgggacc gagccacgtg cgcccggcgg cagagaaacc gggttccggg gcccccaccc 53520
cgtgtgcctt ccttccctag gcgtggaagc cgcttgcgcc gcgcaggtta ggcagggccg 53580
gcggcgacag tggcggggag caggctccgg agccccgggt gcagatgtgg gcgcccctcc 53640
ggatgacccc ggctgagtcc acaggtcccg tgtgccccac ggctggggct ctggccagcg 53700
gtcccaaagg agggctggta gctgtgggcg gggatcttcc aggctcttcc tcgccagacc 53760
tcccagtccc ttcgcagccg aacagggcag ggacaggggc aggtgcaggg gctgggcagg 53820
ggcgcccgac cttggtcccc aggaggaggc tggcctcacc agtgcccgcc tctgcccgcg 53880
gagcccttgc ctggcaaagt tccccctcct gcccggcaaa gctccccctc ctgcccactt 53940
ccccgaaatt tggggacagg ctgcccggag aggtgtggtt ggaactttgg tcactctggg 54000
taaatgaagg ggagggtgtg cccagataat atgcggtttg gggaccagaa agccggatcc 54060
gtggggagaa gcaggaaggt cccgctggag ccctggcaga gggcacagag aggcctccca 54120
aatatccagg gccacctcag agatgggctc cagaagtggg gattctggaa atgcccccag 54180
ggaggtattt ctgtccatca gagtgcagcc ataatataat taacagcaga tattcccatt 54240
tatgtgtgcc aggcatcttg ctaagggctt tgagcacaat atcccagtta atcctcacaa 54300
caggaaggca ccattatctc ttgttaaaaa tggggaaaca gcctcagaga gtccacgtat 54360
tttgcaagga tacacagcta ggaagttgca gaagtagaat ttgaacccag agttatctga 54420
cttggagccc agaggaacct accactgggg cgaaacaggc tgtgtcctca cggggattct 54480
ggcagcccca agaatacagt gctgagagcc agaaggtcca caaaaccaaa gggtctcacc 54540
tgggtgtgtc ctctcagtca cctgcaaggc ttgttaaaaa tatagatccc caagtcccat 54600
ccgaagctga ctgaatcagc taggggcacc tggaggaccc agactatgtc ttactcatct 54660
ctgtccctgg tggcctgcag aggggctggc aaggagcagg tgcttagtga atgtctaatg 54720
ggtaagtggg cggggtgggg tagatggtgt cactagtgtc ctgaaggggg cttaaatgga 54780
gcaggtggcc tcagccaggc aatccggcag caggtatgga agtgacacag tctaatgctg 54840
gcctcagagc cagcagggag ctcaggctgg gccacttgtc cacctggggg ttgaacagcc 54900
tgccccaggt ttgctgaggt ccccaggacc aggccaggtc aagctgggat cacacatgtt 54960
ccctctccag cctagagatg tcaaacaggt agattcctct cccattcata tctcctatcc 55020
ttggcccaca gcccttccct tcttggactt atcagagacc aaggtgctgg gcagggcttc 55080
aggtggttaa aaagtgaaag ttcttgagtg aagtccaaag gcgcacacct gagagctgag 55140
tgggcaaaag gtcgctggct gagtgctggg gatagtctgg ctttggagtc agatggacga 55200
gtccaaatct cagctcctta ccccgtaaca tgaagccctc agctctctga acctctgttt 55260
atttgcaaaa ccttgccaag ggcttcaaac aggatatcct cataaaacaa gtatgcctat 55320
ttgggggact aagtgtgtga agtgctgagt tttaggagtg cagtaaatat cggatccctc 55380
cttcttcacc tcaggtttgc gagttaacca gtgagggccc ttccaggcct agtgctcttg 55440
tagaccgaac cactagcttt cctagagaag gcaactgagg cctagagagt ggcctccagg 55500
ttccgtgctc tgtggcagag ctggccttgg ctccaagtcc cagggttccc agctcagtct 55560
cagctgatgc ctcccagcct ggcggtctac gctgcctgct acattccctg tccagctaca 55620
aatgaacaac tgagagggct ggggtgagat tcaggcaggc tggggcaagg gaaggaagca 55680
ggtcatttgg ggagtcgggc tctctggtct tccagctgag gctggatcca ctctttcctg 55740
tccacccacg gtgcgtagtg gccaggtctt cctgagctga gcaggttgag cactatgaaa 55800
ctgctttcta gcatctagac cagggagttg cctcttgatt taaaatgagg atgtttacca 55860
gtttaagtag ttattaaaac ctaaggccaa gttggtgcct ttcctccctc taggagttgg 55920
aagggtgtgt gcttttttct gctcctaccc cacccctgcc aaaccatccc aatttgagag 55980
gctgtggaga ttacagaact tggagaggag aggggcaggg atcggggttg gagactagat 56040
ggttttcaaa cattgggcta cagaagtccc tcttcctccc atctgtctcc tgcagctggg 56100
tccacccacc tgtccaacac agcctcccac aggtcccggg cctctttgtg ctccaaccgt 56160
cacatccttg gcatgccaca caccctcacc atcctgcctg aaatgccagt cctcatcact 56220
gacattcagc ctcccatgtg ccagcatccg ggccaaacac tttatctaca ggagctcatt 56280
tgtacctgga gactcttagg aggtcgataa tgttattctc ccaaccatgt gactttccaa 56340
ctccatatgg accccaacct ctttttttcc acccctaacc tgagactcct agccactaac 56400
taactgccac attctcctgt ccaaattctg cctcatcggt tattcattca acaaacactt 56460
accgagcact aactttgagc caggcactct aagaccagct gagtcccact tctggcagga 56520

tgagtgttgt tgtcatcaac caccccacaa acacccgtgc cctctgcgta cttgcgaatc 56580
tgagtgagga gacacggtgg cagtaaggct caggacctat agctcatgtg agtgagcaag 56640
gatgcaaaac caaagacacc cgctcccctc acccactgag ctggagggat caaccactgt 56700
gcctgccatc agggctgatg ggcgactgct tgtggatcac aaactcttgc gttgtcactt 56760
caacccaggg gcagagtgtg ttcctcttca ggtgtggcct ggtctcgtga gccctgcact 56820
gaatgggcca ggcgttcatt gcttcagcac cgtaagaaca ctgtcaccgg agaattgttc 56880
gatactcaac tcttcccagt cagtctgtct tctgcatgcc cacagttacc gggcagcgtg 56940
ggcaacaagt gagcactgat tgttccgcaa tcaggctgtg acttactcca tctctgcctt 57000
ctgcttaatg agagccaggt gggagttcag caaagatcac atgtttaagc cttgttatta 57060
aacacgttaa aaaaatactg ccccaagatt ataaagccga cttcggtaca tataaagaga 57120
tttggggtcg gtgaatgggc ccactaagct ttattttgtg gtgtcagctg tcttggttca 57180
tgtgtagaca gcagggctcc caaagccagc atgctggggt ctgtgaggga acaagcttcc 57240
catttcttgt atttatttat ttatttattt atttatttat tttttggaga cagaatctcg 57300
ctctgtcacc taggttggag tgcagtggtg ccatcttggc tcactgcaac ctctgcctcc 57360
cgatttcaag caattctcct gcttcagcct cctgagcagc tgggattaca ggtgtgctcc 57420
accacaccca gctaattttt gtattattag tagagatggg gtttgccatg ttggccaggc 57480
ctggttttga actcctgacc tcaggtgatc cacctgcctc ggcctcccaa agtgctggga 57540
ttacaggtgt gagtcaccac agccggctgc ttcccatttc tataagcatc cgactggtgg 57600
ggtggccagt aggctgtggg gagtggtgcg tgattttcct ggtgggcttg agagagtaac 57660
agtatacccc cagtcagggc aagtgtctct ctgaaggtga actctgggtg cacagctaga 57720
ggatacagtg gctgtgagct gggccctcca ggtgaggtct ggcctgccca ttgccacagt 57780
gggcttgctg gcatccaggg gggctttctg ggtgactgcc caggcctgca catgagtcag 57840
tagcacgctg aatgcagtgg agtttgaggg gacccaccac tgtccatcaa atcgccccaa 57900
cctgggtgaa aggccagagc agctgcggga agtgacacca ggccctctgg agatgaccct 57960
ctggcccttg gagatgtctc tggactggcc agaaagaagg tggtgcctgg aactggaagg 58020
gaaggaagca agacagtgct gggcccagca gccagaaatc agggtgatgg gtgggtggag 58080
gggcaggcat ctgggaaagc cctgccctcc tggtgttccc agatctgaag gctggaggct 58140
ggaaaccttg tgtcacctgt gctggtgtga gttccattgg ggctgccgct taccctccca 58200
tgacctttaa catctggcat ggcttgtggg gggtgctgag aggaggagaa gtctcaaggc 58260
tgagggagct cagggtagag ccaaggtctc agtgagaacc ccagggggca gctgtcacag 58320
ctgacctgga gccctccagg aacgcgtact ctccagctcc tgtactctca aaagcaacag 58380
aaaggtttgc tttcctcagg tgcggtttcc tcacagtgca gcttctccaa agagatctgg 58440
agacccccca gcagctttga caggaaaata gagtcctatc caccccacac caacacactg 58500
aagtagagac tctgggcctg ggctgcccca gaaacccacc cacttaaact gcccgggcga 58560
ttctggtgtt cacaaatatt tgaaagcact gcctgacacc tgctggctca acaggtgtcc 58620
acctaggctg gagtacagtg gtgccatctc ggctcactgc aacctctgcc tcccaatttc 58680
aagcaattct cgtgcctcag cctcccgagc agctgggatt acaggtgtgc tccaccacac 58740
ccagctaatt tttgtattat tagttcacct aagcagagag cgatgtgagg ggctgtgagg 58800
atgtgcaggg gtggagggca cttaggtccc tccctcagct gaccatagag cacccagagt 58860
ggcagagggg ccacgtgcag tgagtggcca cagcagagca aggaaaggtg cacacaagaa 58920
cagtcctaag gaagacatgt gtttaggggt ttcatattca ctgtttaatc atcaggacag 58980
agagggaaag caacctaccc aacgtcacac agctagcaca tggctggacc ggaattcaca 59040
cacatctctg tccagctagg tcccccaact aacacagctg cctgtctgaa ttcaaagccg 59100
ggagagattg tcaggggtgg gcgttggggg agatggcagg tgccttaagt gcccaaggcc 59160
ttgggctgga tctttaagga agggggattg ggatggccca ggaggttgtc aagattggag 59220
atggaaagaa tgccattggc tgtggtgggg ggtggagtgg gcagggactg gaaggagggg 59280
tagtggaagt cagtccctgc ctacaggcct gtgttagact ccgcggagat tcaggaatct 59340
agccctgccc acaggggctt tcactccagt agaagagagg agacccaggg ggccacagca 59400
gtcagagaac aatgaggcct tggcttggtg tttggtgatg ataggggaaa cctgcccaga 59460
acctgtttat cagatgctca gggatctgaa aaacaggttc tgcatgtggg cttgagataa 59520
agatgcccgg gatacttaga tgtgccctat tccaagatcg ctaggttcca aacaagttga 59580
aaacaataga atacacttat ccctaagaat gggcttgcac ataattcact tcaacaatgc 59640
aaaagcttcc aaacctccag cctcctttgg gctgccggtg tggcctccaa ggtccactga 59700
aaagccatcc ccagccagca gaaacatgcc tgtgtcttct tcagcccccg tccatgcttt 59760
gccctggaga caagtgctgt catctgtttg tgggtctgtc tgtctgtctg tctgtccgtc 59820
tttctcccca ctgtgagcac tgacaagcaa gtaaagatga caatgaagcc tttgtgggca 59880
ccaaatgctg acagcattat cactaagcca gtggcagcca tggtgactgc aataaatttc 59940
ccccacagaa attgataaat tgccactttc tgcagccatt atgtttgcct ttgtatcccg 60000
aagagtaaac gcctctctcc ctttctctct ctcgctcacg tgtgtgtgcg cgcgttagat 60060
gacatttatt cattttatgc atcctgggtt ctactggtcg tcccacctca gttcctgtag 60120
caaagagact tgagtctgag ccactaatta tcacccgtga ggtttcctcc ccgagcagga 60180
agcagcaggc cagagctgcg ctctctcagt gcactctcca accaagcatc agtcaccact 60240
cccggtccag cccctgtggc caagagctgg cgtgcaggct gcgggaggca gctggctgtg 60300

caagaccctg gcagggccct cgcctcctga gaaaccgaga gtcagaacca aagccaggct 60360
gtcctggttg gagactgagc cagaaagggt ggctcacctc acggtgaggc tgtcgagtga 60420
cctgagagcc tcagaccctc acgtcagccg gatgtcgcac cagcctgctg ttggtaagtc 60480
tggctaggac gcagatccaa gggggcatgg gtcgtgtgca aacccctccc accagctccc 60540
tcaccacctg acccctgccc tgctggaggg tgccccttga atctccagga ggtccgagga 60600
actcgacacc tcccagagat ctgtactgcc ttggaacagg ctgcaaaaag tgcagtatga 60660
aatgggaaat atgtatcagc tgcacgtttt catggccccc tttggaggaa agggagtcca 60720
ggttattaag tcaggaggct tttaaatttc aggggttcaa ggatttatca cacacagttg 60780
cgtgtgtgtg tgtgtgtgtg tgtgtgtgtg tgtgtgtgtg tgtttaagga tgttaatgtt 60840
tatgatgcat ttctgtggaa tgcaggccca ttgggaattg taaatttttc ttattggtaa 60900
ccatttctgc attggtttag cacggaactg ctgctgcaag taaatgaacc agatttttct 60960
ggaatgattc gaaaatctag ctgtttgtaa aaagctctct aaattattca ttcactttta 61020
ttttgcacag ttgtgtgacg gtaaactctt ttccatcaga cgaaacttgc gcgcacgtgt 61080
atttctgcgg ggattggctc gagtgtgctt gagttttctg tattctcttt tgtaggaact 61140
tctcagcctt ccttttaaaa agataataat tttttaatga tgtctaaata atgattccag 61200
gcagtattac aagaaatata acagtaaaac gtttgtaaga gtccatcaga aagggaaaat 61260
gcccacagag aaaaatagaa atctaccaat tagatgtgtg tggctataag tgagctttta 61320
aaccatgatt tgagactggc atcctctggc ctctctgccc tcaggtcagc tgtgggaggc 61380
taggggtggg atagcaaaag ggacctggga tcaggtgggg cttcctggac cccaggccca 61440
gtaagaatga agcctagggg ttctaggata gggggcagcc ctggatttgc agaaacagcc 61500
tgagaatgag gggctggaaa gtatgagttg gagcagctca gtgaggagac agaaaactga 61560
agcagaagaa agcagaagac accagagtct ggtgctgact gggtgactgg ctgttactca 61620
catcctgagc aatgagccac agaccctccc caccccagag gcagcttgaa tgcagggact 61680
ggatgggccc agggcacagt ggatccgggg caggcaagag ccaactcggg gcccagagga 61740
ggacctttaa cctgactggg cctcagtttt ctcttctgca aagtagaagg aattatagcc 61800
ttctgcaggt caaaccctgt gcccacatcc acaggcacga tccccgactt cgcctcgctg 61860
gcacttctct gcatttgact cctggttatt gcagctgtgc ccatgccagg catttgttag 61920
gtgctgggat gtgcctgccc cgctcctctc tgttggggaa gacggaattc atgctgtagg 61980
ggaggcccgg agtttctaca tggggcagtc acagggtggg ggacttccct gaaaacttct 62040
tatttgcata aaatgtgcgc tgcttttgac ttatattgta aatttcagat ccataaaatg 62100
ggaaagactt tgcagaagct gttattcact ttacctgact gggagatgta gattgttcat 62160
atccaagggt gttagtttta atactaacat tagcatgtgg ggaagggtga tcgagatggt 62220
ggggagtagg gccccccacc acctacccct gggacctcta agggaaagta cacaccaccg 62280
tatgctagtt gcatcctgtt acctgtgctg cagagccaag gggccagagg cagggaaatt 62340
aattctgcct tgtaaaggca ctgggccttc ctgttgaatt ttgctacttg cggggaaggc 62400
acttcccagg caaaaagatt aagcctataa cctaagcaga aagcaagcaa gagctttggg 62460
tgggaagtgg ccagagacat agatcaggca gacccaggtt tggaagggcc aaggagacag 62520
cacatctgcc aatggcagca gctgggcctc acctgccctg acacccccac ctaatgggga 62580
ctgggcagaa ccctccaaat ggttgccatg ccctggccca ggagcctcca aaaaccctga 62640
agccattgac tcagcatccc tgaagcctcc ttccatgagt acccccatac caggctgcac 62700
ctcaatgcac cttagatctg gtgtttgatt ttcactttga ccagatctgc agcagtctct 62760
ttgttaatat ttatttccag tgtttacatt catctttgca taagtcctca gtgttactaa 62820
gtttatcaaa tagcgaaatg cttggccagg tctaacaatg tcaccaagct gcagaaggga 62880
ttagaaactc atagtattat tatttcatca ccactgagta caagagagcc ccagctaggt 62940
tctggggaga acagctatga ctcacaaata ctcccctccc aactcacaaa tactcccctt 63000
agaatctagt ccttaaatca ttcaaaccac aaaaatgtat tgagcactta ctgtgtgcta 63060
ggccccggta ggtgttagag gcacagaaat gagtttttta ttggccccca tccaatgagg 63120
tgacagacaa gagaaccagt gtgtgcagcc aatgttaatg tgacaaaaag tgcagagtta 63180
gtcccagagc gtgaagtcct ccctgtccta ggcccaggtg gaggccagga gctgctcccc 63240
gggctgcctg gaccagactt ttgagctctt gggaccctgg cgaacatcaa agctgttgca 63300
aaagtcagag ctcttgcaaa agttggagct ggcccatggc aggtggttga gaagtggcta 63360
catgactctt cccatgcaga gcggttagag ggtggaggcc gatttccagt atccctggga 63420
atccaaagaa agaggccact gaggggcagg ggagggcccc tccgaggctt ccagaaaaag 63480
gcagtgcagc agctgggcaa accaggataa ggcatgcatc cctaagccca aaaatcaggt 63540
gatgtgaggc aggccgagct ccatggcaga catgctgagc tgcctctgtg tgtcttggct 63600
aactctgccc tttcctaacc ccacggctca ccctgcccag gagcagggat gaggcccatc 63660
cctgattcag caaatcctgt gccataacaa ggggaggggt gggggcagcc acagcccaca 63720
agacccacca gtcccagggt ttgtgcagct ccaggtctcc ctggaaagca agaaaccacc 63780
cagctccaga cgctgaggct gcagattcag ctgccactgc ctgatggcct ccaacctggc 63840
tggattaact ggcccatagg gagctggctg cccccacaag ccagctccct ggcctccctg 63900
ggcaaactaa agccctcctc cctggctcca tagcgcccgg tcatcccctc attccagcac 63960
tttgcttctt cctgcattgt gtagctggcc tttctcctaa gagccttaaa agggcaggtc 64020
atctttggcc acctgcatgg ccccggcgcc ttgcttcatg ctgggtacac agcaggtact 64080

ctgggaataa caaataagag aatttgtcag aggaggtttg gcaggatgga ctattgcata 64140
gaactgcaca gactgtgcac tgcacgcctc cagagggcac tctccacagg gattactatg 64200
taaatggcac ctcctaaagt tgtgcagcat agggacccta gggcttgagc atgccccacc 64260
cgcctccgct gcttcaagcc cctggccttg gtgctgactg ttgggctttc ctcttcccct 64320
tcccaaccag cactgaccat catccttccc atacgctggc gatgggtagt aatgaactgt 64380
cccagtgagc agttctccca gactgggctg tcaaagcatg ctagtgagag attcctggat 64440
ggcaggcagg ccatctggat tcttgctcag ccaggaaact ctgtcctctg ggaatatcct 64500
cccagtcagg gtgttagggg gctcctggat ccccaagatc ttcatggtcc cctagaactg 64560
ctcctctccc cagcctgcag tgggcctaat gacccacatg tagccaggga gtggtcccca 64620
tggggtttgt tttattgact cagctaacaa tgccacaatg actctgattg ccttcttctc 64680
acctgagaga cccagctctg ggcagccaag gggtacgtag ggacagagca tatgcatgtg 64740
accctcaaga tctagaggag ctcagagaca gcatggatac tcgaagcccc cgtgagcagg 64800
gaggactttc tggaccaagt gggctgggag ctgggccaac tgtaaagacc cagggaaaaa 64860
aaaagtcaaa actgagttgc tcttgcatgc aagtgagtgg ttagttcatt gactccgtcc 64920
acagcccttt cctgagcagc tactgcagtg gttttcaaag tgtggtcccc acaccagcag 64980
caccagcaat tctggatgtg gagcccagca atatgttttc atcagtcctc caggtgaccc 65040
tgatgcagct caagtttgac ctgatgtgtc ctaagtgctg gggtttgcaa aagtgagttt 65100
tacttagttc tcaccttgag gagcttggag tgggggagaa gatagacagg aaagaaaaca 65160
gtactgtcca gtgagacgta ttgctctgga gggccatgga ggcagaggag cagttggtat 65220
aggcttgatc cttgcaaggg taagtggcct ggagacccca gtggcatgac tgtcctcggc 65280
agggaaccag atgttgggac cagcatcccc tagagggctc aaggagccca gtgccgaccc 65340
cagtgcccca gtgcccagcc accagggctg tggccctgag cagcaggtcc tcctactctt 65400
ccctccaccc cagatcttct gctctgctct tcccccttct tcttcccact cccaagtccc 65460
aaatagtagc aggtcttttc ctccgattgt cttggggaga aatgggcatg tacctccagg 65520
ccattatctt agagcacatt tttagaagat tttaaagtaa atttttccat taggcagaga 65580
acaccgtgta ttgatttggg tgcatctatt tctgctttac ccagacaaaa agatttacaa 65640
aatatcctag agaggaatag aattaaaaat gagtaaattt aacaggctca gagcgtagca 65700
ctttatttgt ctcatataat ccttgccaca cctcattaag gtgaggaaca tcagcccata 65760
gtgcagatga ggaaatgggg ctcaggaagg tgaagtgact tggccgggcc gccagctagc 65820
aagtggtctg gtgggatatg aaattccatc cctctgctgg gcccttgctg gctgaccttg 65880
cagtttacta aggtgactga tcctgaataa atccagagtg ttctaacgtt ttgctgagcc 65940
cgtgtcctgt tggctgcatt gtggggagtg gcctggcctg gctagggaag ctctcagtga 66000
ccaattgtgg cttcagctgt gttatttaga gaatcccagg ggctgaaatg atcctctgcc 66060
tggttaggga agggatgact ttaggaggaa gagggaccta atgccccact tagcagatct 66120
gcagctgtgt gcatgtttcc acctgcacat ccttctataa accctcagcc atgtgggtga 66180
gcagcagacg gtgcctcccc cactccccct ggaacacatc tgccctgttc actgacctgg 66240
gagctggtgg ggtggacctc agaccccagc acttggcaca cacacatcat cccctgggcc 66300
ctggaatggc ttgcatggat ggcagagaaa tagctgtgtg agcaaaggcc aagagaacag 66360
tttagcccaa ggcaccattt ttacaacaac aacaggtaat ggtactgatt atataagcct 66420
cacttgtcca gaggtcacgg ggcttaggaa cctcaattat gtggaaaaca aataggacat 66480
aacttcagaa ggttgcagag ccactgtgct agtgtggtct ttgtgttgct ataaaggaat 66540
acctgaagct ggggaatttg taaagagaaa aggtgttttt caaaaggaag acaaacattt 66600
gaatgctctg gatttattca ggatcagtca ccttagtaac ctgcaaggtc agccagcaag 66660
ggcccagcag aggagtggat tcatatccca cctgaccact tgctagctgg tggcctgggc 66720
aagtcacttc accttcctga gccccatttc ttcatgtgca ccatgtgtgc agagatcacc 66780
tggggagaga ggaagtaaga gagagagaga gggtagggag gtggcaggct ctttttaaca 66840
agctgctctc gaggaaacta atacagtgag aactaacagg gaacttactt gctcctctgc 66900
cctcccccca gccagggagg gaatccaccc ccatgaccca aacacctctt attaggtgca 66960
cctccagtat tggagatcag aattcaacat gaggtttttg ggggacaaac ctccaaacca 67020
cagcagccac caacattggt gttacaaaat ccatccaatg acataggctc atggagcgcg 67080
cttaagctgt catccaaaac atgtttactc ttggtttaca cacactagta gcagtctgcc 67140
atacctcaat ttcctttttt attttaggta acaacttttt aaataacctt actgagctat 67200
gcctcacatc accacgcaat tcacccattc aaatggtgag ttgcatggtg tacccgtgag 67260
gatcactccc cattttctcc aaatccccca ttctggccac cactaatcta ctttctgtct 67320
ctattgattt gtttattcta gacatttata taagtgtaat tatgtaatgt ctggcctttt 67380
gtaattggct tctttccctt aatgctttca aggttcaccc ctgttggatg catacttagt 67440
tctctttatg gctgaatatt aatccaccgg atggataggc cacattttgt ttaaccattc 67500
ctcagttgat ggacgtttga attgtttcca ctttttgggt attatgaata atgggtgaaa 67560
atgtttttgt gtggatatac attcttcctt ctcttggata tatagctagg agtggagttg 67620
ctgggtcata tagaaactgt ttaacgtttt gaagaattcc caggctgttc tccacggtgc 67680
ctgcaccatt ttacaatccc agcagcagtg taggagggtt ctgatttctc cacatcctcc 67740
ctgtctttta aaaaaatata tcattgtagc catcctagtg ggtgtgaagt ggtatctcat 67800
tgtggttttc atttgcattt ccctgagggc taatgatgct gaacatcttt tcatgtgcat 67860

ttgtatatct tttttggaga agtgtctttt ccagtctttt gcccacttta tgtaggttgt 67920
ttgtcttttt aataccgagg tgtaagagtt cttcagatat ttagaaacag gtccccttat 67980
cagttatatg atttcctgaa cttttctcca ttctgagttg gctttgcact ttctcgatgc 68040
tgtgctcttt cgatttcgtc agcagcgaat tagcaggacc tgggggcttt taagaggtga 68100
gcaggcagac agacagcgcc cacaaagtcc tgaggatgag agaagaggca gagcttacac 68160
aaaggctctg tgaaaactga agatggaccc caggcaggtt ggggtgatcc ctggggtgct 68220
ggatgaggat ctgattttgt actgcatttt ggaaggattt ctgcccccag aggctacata 68280
ggctccagtg gtgtgtgcag tcaggcagag agatgcacag aacctctaca gtgtgccagg 68340
catggtgtgg ggttctgggt gcatgtggaa caacacacag caatcttgcc ctcccaaagc 68400
tcagggaagt gggggcgggg ggtggacaac gagcacatga gaaaaagacc acaggggaag 68460
aactcccaga aacatcccag ccacagcgca atgccccagg tgtatatggc ccttggctac 68520
ttcatgccct tgagggaaaa tcggtatttt cattttacac ttgatgaaac aggttagaga 68580
aaggactggc gtgtccagta cctcccagtg agaagatact cagagtagac cccagatcca 68640
ggtccttcct ctttccctct ctacacgttt tcagtctaga aaagagttct gtgcgagaat 68700
ttttttgttt ttgtttgttt gtttgagacg gagtctcact ctgtcgccca ggctggagtg 68760
cagtggcgcg atctcggctc actgcaacct ccgcctccca ggttcaagcg attctcctgc 68820
ttcagcctcc ggagtagctg ggattacagg cacgtgccaa cacacccagg taatttttgt 68880
atttttagta gagatggggt atcaccatgt tggccaggct gtgtgcaaga atttatgttt 68940
tcacttgtgg cttgctgagc cctggtgctg gctgtgggca catactcgtt cacccaggga 69000
catgcactgt tgatgggtcc ttgcactctg ggctatcttg ggccccgttt gctcaatgca 69060
ggcccctctt tctgaagcgc ttttcttggg ctcactctca attcgtcaaa gttcggctca 69120
ggcccacctc ctgcaggaat cctcccagtc cccgctgtcc gaagtcagta cccttgtcag 69180
gagaccttaa acactttagt ccactgcctg gccctcccat ggggcaaagg tctccagggt 69240
gtgtgtgcac ttccttgggg cttcttaagg ctcattccct gggattaggg aggacataca 69300
gagcctcctt gcacctacat ttgtttacat attacttata ccacatacaa cgttataggg 69360
cactgctgca taagtgtaca ttttgaatgt attttactta taaagaaata gagcagtaat 69420
aggaacatgt gcccctaaat gtaattttac tgaccagaca aaaaaaattt tttttaattg 69480
aaaataagta gctggacgtg atagcatgcg cctgtagtcc cagctactca gactcagaag 69540
tctgagacaa gagaattgct tgaacctggg aggcagaggt tgcagtgagt cgagatcgcg 69600
ccactgcact ccaggctggc gacacagcga gactccgtct caataaataa ataactaata 69660
aataaacaaa aatttaaaaa aattgaagat aactgtgggc tgcttgagga caagaactgg 69720
gttattttct ctctctttgc tcagtcagtc cccacatggg aggcgtgcga ggcaccccgt 69780
tcttggcctg caggccgccc ccactccatc tggctgcagg gtgccacagc cagcctctga 69840
ttgccagtgg ccccagcacc tcaatccata ccgacctcac agcaaaccct ccgtggccac 69900
agcctgccct ggccactcat gtctgaaagg tgatcggtgt ccatgaagaa gaggccatgg 69960
ggctgtggat gtcatgtggc caactgttag tggcctgcgc aagagcagac aggagctggc 70020
aggcaggccc atgggccagg cgtggctccc agccataaac acaggctgta gccctgccgg 70080
gaagaaggag gggacagaca agcctgggca gagacccaga ggggaccagg acacccgatc 70140
ctccatggca gttctgtcca cagcttggga ggagaggcag gcaggtctgc aggcaggaga 70200
caggcctcgg cccacagagc tgcaatctga tcccatctgc tccctctcca gttctataaa 70260
tagaaccccc agggagccag ggcccagaca gggttagtca caccctcacc tcaggccgca 70320
gggacaggcc tctgggactt ccaagacatg taccacctgt cctgccccca acccctgcca 70380
gccttcagca ggagggttca tgctgagcat caggaggggg cagaagaatt gaacgtggcc 70440
actgtccatg aagaagttag agaagagtac acacaacaca catgcacaca agaataggac 70500
acgtgcacac acacacacac atgcatacaa gattaggaaa cacaagtgca cacacaacca 70560
gtcagaaatg agctctgagc ttttacacat attaactcat ttcatcctca aacagtcttt 70620
agagaggtat aattagtgtt cccgttttaa aggtgaggga actaaggcac agaggagcta 70680
tgtaactcac ccaaaggcac ccagctactg gcatgtgagg ccatgatttc tccccagcag 70740
ccccacccac agcccgtgcc tttaaccacc tgcctgaccc ctcccagcca agtggctccg 70800
tccctacatc agaaaggtca aggaagaggc cgcctaggga gcagtacaag gcagcattac 70860
aacctggcat tttcctgttg cagactgcat tttggtggca agagaagctt tggaggtgct 70920
taacgtttag tttgcattta tcctaccgct gaaaagcagg gacccttagg accataagtc 70980
taggactttt catagcaccc tagactgctg gggatgaaag gggcttggag ctgagctggt 71040
gcagcagctc ctgttagaga agccacccag agaggcccag ggacctccta tgtcccagcg 71100
ccagttaggg gcagacgtgg gaccagctcc cctcggctgc agcggcctcc atatcagctc 71160
cctggaaggg agcagatgcc aagtcaggtt ctgagcaaca ccctcaaggc ccagccctgg 71220
caatcccttc ccacctgtga ttaggtctgg ggttcaacaa aaataggact caaaatctgg 71280
atcttctcgc cttgcttcac tttgggctgg ttcagttttg tttcgaggta aatcattcga 71340
ggtttttcag ggatcctcag atgcatgtct gcgtgaagcc gtcctggctt ccccttcggc 71400
tgccccctgg gcaagagcct gggccatacc tgtgccattc ctgagctctg tctctagctg 71460
ccctatgtcc agtcattggc gatgggtctt attcaagccc cctttccccc aacccacact 71520
agattgtaag ccctcagggt ggggaccgct tctcactcat ccttatatgc ttaggagtgt 71580
tcagcacaaa gtcactcagg agatggcagg cagcggggag agggcagaga cagaggagag 71640

gcttctgtcc agagggttcc tacttaggat catccaaatc atcagcaaat gttggaaaga 71700
tggtctgggg gcctcaggct ggctactctt cctccccatc agggcaaaac tccccggggg 71760
cttgtgtctc cctgtggttg gggacaggcc tgggtgtatc tgtcccagcc ttgactgacc 71820
acaaggggag acttggccct cccgaccaca ggagaagagg gatttgaatt cacctcggcc 71880
caaattgcag atgctggcct gcgagccctg aggccatctg caaggctcct gggctgacat 71940
aactgtcacc cagctcttca tcctccttcc ccggggctga aggggtgcct gcagggtctt 72000
ctgccctccc tccacctggg ccctactgct ggcacggagc ttcatttctg ttggcagcca 72060
gtgtttgctt agtgattact gaatgccgtt tgctgggtgc ttatgatgtg ctcagcactg 72120
ttctaagtgc tttacacaat tgacttattt actcctacaa cctgcccata agggaagtgc 72180
tgccatggtc tcccttttat agctgagaaa actgaggcac agataggtcc atggtcacac 72240
agttaacata aggcagagtc aggaccagaa tccaggcgga ctggctccag agcccggctc 72300
ctggaccgag cagccttgag tagggacctg gtgcagaccc gcctgccgtg gactcactat 72360
aggatgggtc aggaggcccg ttgtcaccta caaaatgtag cccaccaccc aggaaacgct 72420
cccagaacca ggcagaaaat actgtctcct attcacaggg tggctgtgcc tggcgtgagt 72480
gtcttggcgg gtgcctgcat gtctgtgcag gagtgccacc cccatctgtg gactcggaaa 72540
gcatgatccc cacaaaagca acccctcaga gtctcagctc agccaatccc tgttatcctg 72600
tgacaggcag ggccatgttt caaaaccatg ttaaagatga gcaagaggct aaggaagttt 72660
cccatgctgg cagaggcagg acaggaacac atgtcccctg actcttgaca ttgagaatgc 72720
acccccttcc acccacctgg cctcttgtcc cagccagtgt ggtcccagct atgcagctct 72780
ctgggacatc tctggaaagc tcattgttct ctgtctgggc agtaagacgg agtgaggctc 72840
tgactgccga ggcagacaga cctgcctcct gctcttcctc ccctcaatta tttgtgctgc 72900
ctgaaacaag ttcctgagcc tccctgagcc tcagttccct cctctgtaaa gtgggaatga 72960
taactgaatc tacttcctgg ggttcttgtg aggaccagag gcccatcacc cgaggaaagg 73020
cctgacagca gcaatgggtg tatggtaggc atccattaat aggaactgct gctgctgctg 73080
ctgccttata aaaggaaaaa tcacagtttc aaaaacctct taagattgca tgatttgtca 73140
tctgagagca gagattgtga tctccattga caaagatgga agctggggcc cagggaggca 73200
cacagacttg tctgaagaca cacagctgct tcatagcaga gccgggccca gcctcctggt 73260
tcccctgccc catctttatg tcagtccctg gatccactcc ctcacctggt caggtgtcca 73320
gaggttccag gagagtgttg tggaaaagta ggaagaggca gacgcccatg gagggtgttg 73380
aaagccaccc cagctgctct gctgcttcag ggcaggggat cttaagccct gtggaactgt 73440
aggctggtga agcttgtgga cttctgctca gaataatgtt cttacatgca ccagacaaaa 73500
tagcttaggg ttacaaatga aatatattat atggaaacac agcgaaaata ttttttaaaa 73560
aagactacag tagcattagg catgcttctt tattagcaca ttaaatgaca agctctagtg 73620
gcagatctaa tgtctaccat aattttattt ttacttttat tattattatt attattatta 73680
ttattattat tattattatt ttgagatagt cttgctctgt cacccaaact ggagttcagt 73740
ggtgcgatca cttcagtctt caactcctgg gctcaagcga tcctcctgcc tcaatgtcct 73800
gagtagctgg gacgacaggc gtgtgccact gtgcctggct aatttttttt ttattttttt 73860
agagatggga cctccctgtg tggcccaggc tggtcttgaa ctcctgacct caagcgattc 73920
tcctgccttg acctcctgaa gtgctaagat tacaggtgtg agccactgca cctggctagc 73980
atggttttaa atttgtgctc aatttaaatg gtattaggat aaattattta caggtaaatt 74040
tatgatatga tcttggtcag tattaatgaa aaattatgtt gcctaccttc atacttaaag 74100
gaaatagcaa attttagtta gaagttggtg acaattaata tataataatt tttccatcca 74160
agtttactgt agaccgatgg cccgagttca gggtttcctc cacatccact catatcttgg 74220
gctcagcaaa gaagatcaca gggcacccac tctatcttag gggcccattc acttgtctct 74280
cagtcctgcc ctcagagatc aaggtttggt gttcattcat tcattcattc agcaaacact 74340
gagcactgcc tccgtgccag gctatgatac acagtgaggt ccaaggtcag atgagatgag 74400
gtcctgccct caagctgttc accatgcttc agggagagca taagaagaac tgctccaaga 74460
tgcaaaatgt agacaagcat agggacatgc tcacccttcc tggtcatcaa acacccagcg 74520
atggtgaggt tggggttaga tggtcactcc tggctagtga cactgtccat tgcctaatcc 74580
ttttgaaaag aagtatggca ctgcatcaca ttggtcatgg aaattccaga gttccctttc 74640
ccagcactct cactctgcca gtttttcccg aggaaatcat tcagcaggag ggacaaaggg 74700
tggagggcag aagtcatccc tgctcttcct gagtctgtgc tctccccagg gaaggggctg 74760
tgttgtgttc agcttgttcc cctggtgctg acacgtggta gcactcggtg aacacctgct 74820
gggtgaatgt ctgactgccg tgtagttacg atgtataaga ggagggaggt ggcagaaggg 74880
gcagtccagg agccctccac tgaccaggtt cctgacttct cttgcagcca ccaaaatgcc 74940
agaagagatg gacaagccac tgatcagcct ccacctggtg gacagcgata gtagccttgc 75000
caaggtcccc gatgaggccc ccaaagtggg catcctgggt agcggggact ttgcccgctc 75060
cctggccaca cgcctggtgg gctctggctt caaagtggtg gtggggagcc gcaaccccaa 75120
acgcacagcc aggctgtttc cctcagcggc ccaagtgact ttccaagagg aggcagtgag 75180
ctccccggag gtcatctttg tggctgtgtt ccgggagcac tactcttcac tgtgcagtct 75240
cagtgaccag ctggcgggca agatcctggt ggatgtgagc aaccctacag agcaagagca 75300
ccttcagcat cgtgagtcca atgctgagta cctggcctcc ctcttcccca cttgcacagt 75360
ggtcaaggcc ttcaatgtca tctctgcctg gaccctgcag gctggcccaa gggatggtaa 75420

caggcaggta ggttctgggg gaataatacc catcgtaaca ataaatataa atggctaatt 75480
ttcatcgagt gctgccagca tgctcttttt gatatgttat cataactaat cctgcatcac 75540
tgcaaaattc cattttacag aacaggaaat tgtggctcaa agacattaat gatcttactg 75600
aaaattccat agctggtaag ttgggatttg aacccagttc ctatggatct gaaacccatg 75660
ttctttctgc tatggtaaca gggtttccta gagggcaagg aggaaaaggt tgatggcttt 75720
ttgctatttg gagccaagaa ggagagagac ttggctttta gaattggact cagaaggagg 75780
aaagtaacac acttatgtac ccggccctga ggaaggcagg catgtctcaa attttatcgt 75840
atttattcta agggcttcca agaactgatt tctcagaaaa gtcaacagca ggtttttctg 75900
gggctagata atggctccac ggaacagcta gagctgcata cgtttcttgc cacgaggagc 75960
tcttaccaag acaaggtggt ttttaatgcc accttggttt ttaatgcatt ttcttggcga 76020
aagaatgcca gactccatgt gtgagaactt ccaccagtga gtcgagccca ggtgccaggc 76080
tctgtaggac agtttaaagg tgcccagcca tacctgacta cagcacctta aatgcaggct 76140
gcatcatggg actcccaggt atggaccagg aggaacagtg gagagtgttg gatgcagggc 76200
ctggagggaa cggagcaagc aagctggctg gaaaagactt ggaggagggc tttgacctgg 76260
gcccacctca tgctgtcagg ctcagggagc agggagtgca ttccagtctg agggaacaat 76320
ctgaaaaggc acagagataa gaaacaatgc tcctagacct cagttttctc acctgaaaaa 76380
tgaagactat agtacctacc tgcgggctca tggcaaggct gagagatcat atatgtaaag 76440
ctcagcatag agtaggtgtt cagtctgtgt ttggagacta aactgaccaa tgcagtgtca 76500
acaaggccca cggggtgctt ggggatctga ggactgcaat gtgcctgcag ggggacctcg 76560
tgggtcagag cagcgggctt caaggcggag agggcaggct ggagctggct tgtagaaggc 76620
cttatgtgct ggctgagggc tctactgagc cgagggacat catcaagggg ggtgattctg 76680
aactgaggga tggagctgca gccacagctg cccattctgc accatacgca aaactctttt 76740
gctgagcggc tcccagggca ggccacggcc ttggagacag ccatgtcggg ggcccatggg 76800
gactacctgg atgagcattt tgcttgtggt tgctgatgtc cacgagctgg gcaagatgct 76860
tactgtttgc agcttccctc cttgagtagt tggcctggct tggcaggtgg gccagaggct 76920
gggaggcctg ggcagcagag aaaagacccc tagcctggga gctagaaccc cattggctcc 76980
cacctgtacc attgactggc agctcacttg aacaagtccc tgcccctctg gccctcggtt 77040
tcctcagctg ctcagtgagg ccctgcccac tcctgtggcc tgtgacgccg tctgactgcc 77100
ccacttttct cccgcaggtg cccatctgcg gtgaccagcc agaagccaag cgtgctgtct 77160
cggagatggc gctcgccatg ggcttcatgc ccgtggacat gggatccctg gcgtcagcct 77220
gggaggtgga ggccatgccc ctgcgcctcc tcccggcctg gaaggtgccc accctgctgg 77280
ccctggggct cttcgtctgc ttctatgcct acaacttcgt ccgggacgtt ctgcagccct 77340
atgtgcagga aagccagaac aagttcttca agctgcccgt gtccgtggtc aacaccacac 77400
tgccgtgcgt ggcctacgtg ctgctgtcac tcgtgtactt gcccggcgtg ctggcggctg 77460
ccctgcagct gcggcgcggc accaagtacc agcgcttccc cgactggctg gaccactggc 77520
tacagcaccg caagcagatc gggctgctca gcttcttctg cgccgccctg cacgccctct 77580
acagcttctg cttgccgctg cgccgcgccc accgctacga cctggtcaac ctggcagtca 77640
agcaggtacc caccccatgc ccttcctccc tctggcaact cagcacatgc ttgtccagca 77700
cctccccccc ccaccaacca ggtgcagccg atacccacgg gtgcagcctt accaggtgcc 77760
aactgcagat tctgttccaa tggggcagca cccacatcct cctgcccaag attttgagta 77820
tcagtgaaca aagcagatga aaattcctgc ccccatgggt acagcccagt tggggcagac 77880
ttaaacaaaa tacagcagtg agtcatagtg ggcgttgaga ggatcaggag tgctaaggcg 77940
caaaggaaag caagcgtggg cggtgggggt gcaggtttga agatggtcca ggaggacttg 78000
cagagtgagt gctatgtgag cagagctgga aggaggggag aggggccggt ggcctgctgg 78060
ggtgatgttg caggccacgg ggcatagtcc ttgggcagaa gtgtgcctgg tacgttggag 78120
gagcagccag gaggcccatg tggctaaagc tgaacaagga gggggtggca ggcgaggcct 78180
gagaggaaga gcttcagcct taggctccga gtgaaatagg gagctggtga aggtttgaat 78240
ttaggtgttg atcggcctga cattttaagt ggttctctct ggtttttggc tgagaacaga 78300
ctgtaggagg tcaaaggcag ctagctgctg gggccagcga ggaagccgtg catatgccca 78360
gatttgaggc agtggaggct ggaggcagtg ggagcagtgg aggagcaagg agatcctgaa 78420
ggcacatgtg aagctggagt gcatggaatt gccgctggaa tggccccagg gttttgtggg 78480
tgatcaggga tgactctggg gtttgggtct gagcacctga aagagagagt tgccatcagc 78540
tgagatgggg aaggctgcag gagtgagcat tccaggttta ggtttgttgt tgttgttgtt 78600
gttctttgtt tgttttactg ggggtggcac taatcattca tttagaaaat tcatatgggg 78660
cttggttgcc agcagggttc ctgggaagtg gaggaccttc aggccacacc aggtggggac 78720
ccaggcatca gctggcacct gtgcccacag aaacaggccc ctgatgctcc ctcctggtcc 78780
acaagctcat ggggcttgat tcttagtccc aagctcacat cttcagcaac actgtcttta 78840
ttttctccaa attaattcca cctccagtgc ccagccccgg ggctggttga tgcataggtc 78900
ctgcccactc caggacgcta gtcacccaac agggttcagc ccaggtgctg gtgagacact 78960
tctcatccac ccctcattcc agcctggcag cccctgcagc tcctgctcct gctcagaatg 79020
cagggacctt ggtgaggctg ggccctcccc taaacaccta gcacagggcc atcccctccg 79080
agcatggggg attggcagga ctgggaagca gggctgggca cctcctgaga accacagacc 79140
tcagccccta ctcactgtca gggttgctgc ttctctgcca gcagctactc gactgccctc 79200

gtcctttgcc acccacagat accggcatca ttgcatctgt gggctcagaa tttcctaaga 79260
caaaggaaga accccagact tcaggcagaa aagcaagatg cttgatggaa atgagaggaa 79320
gggaagaaat cagtcatgaa cccttaacaa gagctcgctg tgttccagga accattctaa 79380
gagcttcgta caccactgtg aattcattta accccatctc atccctgtag gccaggtgct 79440
attatatcct tatttcatag atgagaaatc tgaggcacag aggggtcaag taacttggcc 79500
aaagtcacac agtcagtaag tagcacagct gggattcaga tccagctatt ttccagatcc 79560
atgcttatac ccacgctgct ttctacaggc aaaccaaatt cacaattgag taaatgattt 79620
cagaactagg tcattcattc actcattcac tgagcaccta ctctgccagg ccctggggcc 79680
acagaaagga gtaagaaaga cacagaccat ctggctcctc gcctcagctg gtgtggggcg 79740
gtgtggaggc ctgaaggcta gaggctggac tcctgctctc cagcatcgct gactggaaca 79800
aagatcttga ccttgagcaa gtcacagccc ctcttttgtc ctcgaggccc tgatgtgtag 79860
atcaagctct ctcctagtgc taatgttttc aatgctcttt gacgatattt tcttactgac 79920
ttctacaaag caccagcccc ttggcaggag gctctgcggg gctccctggg gactagtaca 79980
atgttgctgc ccaggacggg gcagatctgc atggaggagc ccactccctc tcccctgagc 80040
tggcaggtat ttccagcagc agtgggaaag acggggacgg tgcccactac cccccagggt 80100
aaaagacaca ccactgctcc ctccctcccc tgccaccctt caactgcctt tgtctaaatt 80160
ctctgccctg ccaaggctct ccctgatatt ctgttaaccc aattctgccc tgatgcaaat 80220
accagggcac agaggggcgc atgcaaggta attttccccg tgtcccaaaa atctttaggg 80280
ctgcaggccc tggtgaggtt gccaggcaac catccctccc gcaggacgag atcagtcaga 80340
gacaccgaag gcctctcatg caaaggggct taaacttgtt ctgcagagtt cttaagggca 80400
aaaccagggc tgatgtggtt atgtcggggt gggtggtcgg agggaggcag gggcttgaca 80460
cacagcagcc cagatacggg gaggtgccca agcctgggca catccaagca gattctggat 80520
ggtccatggt aagccatgcc cgcggttctg ggcctgggca cctgaggctg ttctgccttc 80580
agccccaaac agcagggact cctgggtggc tcaggcttga tgtgtggctt ctgctgccag 80640
cccatgggca cctcaccaca gggtccccac atggccactc accaccccaa ctgcccacta 80700
ggcccttggg tcaagggagc acattagtct aggcacaaga aactcctcca agatttcctt 80760
acagccctga gtctgcagat taggaggcaa aggtgtatgc cagctgtaga gaaaaccttt 80820
gagaccatgt ttacagacag tattattacc aacaataacc gatatttgta cagggcattt 80880
ggatttgctt gtgtatttcc ctggaaatac acatttcaac atcaaactca aagatcaaga 80940
tcaaagaaat gtttaacctg cacactgttc tcctgttacc ttcaccccgt tctcctatta 81000
ccttcacccc aagctacatc tgcacatggc tggcacagga tggtctgaaa ctctcatctg 81060
accatgtcac tgctgaaaag atccagtgac cccagtgtcc ttgggataat ggtagaaccc 81120
tcactatgca gctagactcc ctccgcagcc tcatctcccc ttctcctgtg acgtgtcaac 81180
acttgggtcc catactgtcc tctaaatggg cagagctcat tcctctcggc ttggaacgcc 81240
caccccagga caccttgaca gcttgcaaac tcctattcct ccttcaaaac cctacctctg 81300
ttaagctccc ccagactatc cccttacttc cccatacctg cctctgtaat ctgttgcatt 81360
ttgtaaatac ttttttatta tttttcttac acactacttc atacccgact cagaggtcca 81420
tgactgggtc ttctacacct tggggtccat ggttcctaac acaggttcac agtgcatgct 81480
cactgagtga ctgattttta aattggccac cacagagcca atatcaggca tcttaagagg 81540
agtgcacgta gagcttggag caagaactag tctaactgcc ttgctgccac taagaacaac 81600
agccatttat tacattaagt gcccactgtg tgcctgttcc atgctactca ttccacaaac 81660
gtaatgcttt tagtcctgca gtaacacagc aagatacaat gatgctatga gccaggaaag 81720
ggactgccca gggtctcatg actgcccacc agtggcagag tcagcgtcag aatccaggcc 81780
tatggtctct atgccccaag gttgcccttc cacaccctgc caccccctcc tcctctgagt 81840
ctggcaggca gtgagttggt accactctct tcccatccat cacccactat gaagacatgg 81900
agaggggcta atgctgcagc ctcactggtc cttcagcgtc tgacctgcaa cctagaggga 81960
aatcccacac ctgaatgctg tctttccatc tcttgttgat gaacactctc cttggccttc 82020
tcaaggagaa ggtggtgaac ttgatcatct ctaaacatag agacttactt actaggggag 82080
ctgttacatc tgcgttccag ttagccatgt gtgccactgg tcaccaggga gctctgagca 82140
caacactgtg gtttaatagg ctggctgaga atctcatgga ctcctaaggc tggcttaggt 82200
tctgtcctca tgcgggtctt tgccatccac tgctccctag cccacctcat ccctcccttc 82260
ccagcgtcac tgcagactgg aaatgttaga ggaaaaaaac aaggcatttt acaagccaat 82320
gtggtatacg tgtgtgtaat catccaaact ctgacttgtg acattaactg cctgatcttc 82380
ccgaggcgca gagaggaatg actaatgaag cagagttaaa gaacaaacaa cttccagcga 82440
ggagatgact tactcaattc atgctgtgag gtgtgagtaa gacttctctg ggcagaggta 82500
cggaggagac ctctctgccc tctgcacgcc tttccagcta atggtgactt caaccacagc 82560
aggcacataa gcaaaatcaa gagaatttca gacatgacct catccaaacc tcctaggtag 82620
agaaactgag gcccagagac agtcttgcca gagtcacatg ccactggtgg tcaggacaag 82680
aggctgggtc cctagactcc tggtccctta gtggcttaaa gtcaccaaag tgagccaggc 82740
ctgccctaaa atagaacaga gccaatcatt tttcacagat tgcaggaaaa gcactcaatg 82800
gatggcattt tctctgtttc tttaaacttt ctcttataag ctgtacaacc attttgctca 82860
gcccagtgga ttcactttta atattttatg tggtaactga gctcgtgttt ttttttttct 82920
gctgaaattg tttctcaaaa aacctgtatg agtcattctg ttttgagagg ctgcctatta 82980

aagtgtagtc acatcttcta atggaaatac tgttccttgt tctgctgaca ttatagaata 83040
aaagtaccag catttcacag tgtttcacag agaacacctt gacttccagg aactctgcta 83100
cccggtctag ttgcaccttg ataaaagttc ttcctcatga gggcactatg cgagtgagtg 83160
ttggctgggc taggaggggg ctcagtggca aggtggggag agaagggtca gaaatctcca 83220
ggttcatagg ggctccccaa tttatatgtc ctggaacttg gtgggggggc ccagatctct 83280
gtgagggcag ggtaagggca ggtatcttta ttttctgtag cccagggcct ggcacacagt 83340
aggtgcttcg taagtggtca ctgaactgct tggtgaatgg ccgtcagtct ctcttgcctg 83400
gcaaagttga gccaaggctg tgtgcctcag gcagaagtag agaactgggt aggaagaggg 83460
gaggctgaga gctttgggtc ggggagtgtt gggggctcag tgtctgtatg tgtctttgta 83520
tgtcctgagt gtgagccagt gatgggggca gtgtggggct ccaggaagac agagggctca 83580
gggtgagaca ggctgggaag ggaagcagcc agtaaggtgt gagctcattg gctgagggga 83640
ttcagagcta ggagagctcc tctctcctgt cctgggattc tggggccgga aaaccgtgag 83700
acggagccat gcttaagggc tgtatttctg gttgactcct tactctgctg atgtgactgt 83760
tctgattggc tgccccgggg cagatatgtg aaagccccag gagccccggt cattggcacc 83820
aggctgagtg ggtcctgatc ccagctctgc atctgttagc tacatgtgac cttgggaaag 83880
tgatttgatc tttgtgcctc atttcctcca tctctaaaat gggctaatag tgatctctac 83940
tttgtggggt ggttttgagg attcgatgag ttaatgcctg agaaacgctt atcacagagc 84000
caggtagcgt caggtgcagt gtgagcgccc gccgtcttgt ctttcttgtg tccttcatca 84060
ttgccctccc ggggcaccag cctttgccac agtgttcatg gttttccttc catccatgtc 84120
aggtcttggc caacaagagc cacctctggg tggaggagga ggtctggcgg atggagatct 84180
acctctccct gggagtgctg gccctcggca cgttgtccct gctggccgtg acctcactgc 84240
cgtccattgc aaactcgctc aactggaggg agttcagctt cgttcaggta aagtagtctc 84300
tagtctgcca gccagcttca gcgtggccct ggcccacctc tcatgagtaa gtgctctgcc 84360
tttgagaact gcctgggctc tgatcatctg cacagcagga ccactcggtg agatgccagg 84420
ccttcctgac ccagagggcc catccaagcc tcttgatagg ggctgcctgc tacaagctta 84480
acctcccaac acatgaggaa gccagacaca ggcatgtgaa aaaattacag accaggacac 84540
tgagccctgg gtccaccatt ctcaagcttc tagaattctt gagctttcca aacaaaattg 84600
aatgacatga agtcattgga cattttgctg gtctaggtag gagtcatcat ctacctagac 84660
atccatctag gtaggagtct agggagtgag cgagcctagc ccaccaccct ccacccctca 84720
tctcaacctg gctctgttgt tccccttaac cccttgattc atgcagcgtg gatgagacac 84780
aagcattagg accccagttt ataggggatg cgacacagct ttttcccaag gatgccattc 84840
ctatgaaaaa atccagaaaa aggaggtctg tcatcagatg aatttgagaa aagatttact 84900
tccccctaag agatcacaat gcactttggc acatcaaaga ttcgaataac atctacaaca 84960
aacaaacctt tgttaatcag acttattgga gtgttagcac cctccccctc cttttttcca 85020
aataacagct atcagcactc cacaagctgg gcgctgtggc atgtggctgg gatcccagct 85080
actaacagag gctgaggtgg gagaatcact tgagcttgag cgattcttcc aggagttaga 85140
agttcaagtg tccagcctgg acatcatagc aagaccttgc ctcttaaaaa aaaaaaaata 85200
ctccacagaa catactctgg gaaatgctgc tagtggcaaa agtttctttt tgcatttgtt 85260
cattcaacaa acatttattg agcacttact gcaggccagg catcctgcta ggcctgtgat 85320
gcaatagaga acaagacagg cagagccatc tttccatgat aaaggaaacg gaggcaggga 85380
cattgtttac atttctttgg catctcccac tgttctccac acagaatctg cggtcagtgt 85440
tggcggatta actcctcccc tcccatgagt gggagctgac taatgctgta tcctgccatt 85500
gtaacagggc agagaacagc catcctatcc acccccacta tcaacagaaa tctgtgcagg 85560
gaggacgtgg tacaggactc agggacagtg acagcgtaat gctcttacgt aatccaaatt 85620
aattgctcct gaccagggat gcttcacatc cattggaaca acacagagtt gtagccgtgt 85680
gttttttaaa gtgtgtctgc ctctgaatct ccctggctct gctcatctca gaaggtccct 85740
ggaggagggc tcatggctga cacatgttag catacgagaa ggggaagaat tttatttatc 85800
tgccactaat ccccatgtgt ctcataccca aatctaggat tttcctcttc ccacctggag 85860
tatccggcgt gcatggttta tccacaatta aagcagactg caggctgctc ccctcaccag 85920
aaagccgctc ccttaagcag ccatagatta catgccctgg ctctgcctgc cagagaaagc 85980
cggtggattt gattttgtta gcaagagctg actgagaccc tagcagctgc attctacttt 86040
acatattaaa gagtggaaat acaaatagga cagtcctttt gccactgcaa gagcaggggg 86100
ggttaggtgg gtagaaaggc aggattttac ttgtggaaat cagtgcctgc cttccatcca 86160
caaaaaccag atgtcaatat ggaggaggca catgtcagcg gcgcctggga agcctgagtt 86220
ccagctttcc ctccccacaa ccctgctctg taactgtggg cagagcatgt ctgctccaag 86280
agcctcagtt tacacccctg aaaaatggag ttaatgcctt acagggattt tatgagccac 86340
agtaagagat tatgtgtgaa agaaaaataa aaagcatttt gctattattc ttagggtatc 86400
tggccacatt tccattatct ctgactaagg taccaaccac cgatttctga gttcagtagg 86460
agacagtgag acagaggcca aggaacccca accctgggcc taaaagtggt ccagtcagtg 86520
tttgcaaact ggcggacact cactagtggg tcatgcaatt gattccatgg atcacaagca 86580
gcattttaaa aatgaaataa aatagaatga caaagaaaac agtggagtgt tttgcatggt 86640
gtcgggatac tttttcagtt atatggaatt gctagtttta tttgcaaatg tagcatacct 86700
cgttgtctac cggacagcag agtaaaacat ggttcttact gtgagtccct gtcaaatagg 86760

ctgaagccag agccctcttg gagaaacaga gataacccag gcctgcctcc catccgtgga 86820
ctcagatgct gagggatggc tctggcaatc tatttttgca gctccctaag tgactgatag 86880
gtgcgcatgc cgcagtgtgt ggcaacttcc agtgcaggaa acacaggcag tggaacccga 86940
agacctgaat ctcagtccca agacccccac ttacctgccc cgcatcatca gacaagtttc 87000
ctaggccctc ggagcttctg ctgctcactt gtgcctgtgt ccaccccata tggtcatcaa 87060
gaggatttga gctggacacg ttaaatgcag gatgcgtgca gccaacagtg gcatgctggc 87120
ttttgaggta agctgcaacc ttctccttta aagttagcgt ggctagtgag aagttacctg 87180
agccccatca ccctccccta cacacatgtc cacagcagac accaggctat ggggacagcc 87240
atggttcttg aggggccttg gctgacccct agatctgtaa tgcttttgtt accggaaaag 87300
ggtcccgatc cagaccccaa gagagggtta ttggacctca cgcgagaaag aattcaggac 87360
ctgtccatag agtaaagtga aagcaagttt attaagaaag caaaggaata aaagaatggc 87420
tactccatag tcagagcagc ggcatggctg ctcagctgct tatacttatt gttacttctt 87480
gattatatgc tgaacgaggg gtggattatt catgagtttt ccaggaaagg gatggcaatt 87540
cccagaacta agggttcctt acctttttag accatatagg gtaacttcct gacattgcca 87600
tggcatttgt aaactgacat ggcgctagtg ggagtgtttt ttaacatgcg aatgcattat 87660
aattagtgta taatgagcaa cgaggtcact ctcgttgcca tgttggtttt ggtgcttttg 87720
gctggcttct ttactgcaag ctgttttatc agcaaggtat ttatgacctg tatcctgagc 87780
cgacctccta tgtcatcccg tgactagcaa cgtttaacct cctgggaatg cagcccagta 87840
ggtctcagcc tcattttacc cagcccccca ttcaggtgaa gtctctctgg tttgaacacc 87900
tcagacactt cgtttagcta aaaaccattg aaacagtgta ttgattccac aaagaggtga 87960
cttgtgcttg catttatcct tttgttccca aatctgtttt caaagtttta catgttgttt 88020
tgtgttcctt tgggtatttt tttttttttt tttttttttt ttttgagacg gagtctcact 88080
ccgtcaccca ggctggagtg cagtggcaca atctcggctc actgcaacct ctgcctcccg 88140
ggttcatgcc attctcctgc ctcagcctcc caagtagctg ggactacagg cgcccggcac 88200
cacacctggc taattttttt tttttttttt ttttgtattt ttagtagaga tggggtttca 88260
ccgtgttagg caggatggtc tcaatctcct gacctcatga tccgcctgcc tcggccttcc 88320
aaagtgctgg gattataggc gtgagccacc gcacccagcc ttcctttggt atttttttgt 88380
tattgttttg gtttggtttg ggttgtgcta agtgtttcaa ttcagccagt tttaactggc 88440
tgttagatat tagactgcaa aatactccag tcagagcagc tcacctgcgc agaaaaatga 88500
gggttcagct tttagaacct aagttctttc ccagagttag aggaactgcc tgtgaccttg 88560
cccactccag ccacctgcct gaaataatga caagaccagc ctgaccccag cttcatgagc 88620
aattgtccaa agcccctttg tgccgtagta attgagcacc agcaggagct acaacgacct 88680
ggctgtttct tttttgctaa caagcttacg tataataaaa atggctctag cttggggatc 88740
ttattaatcg cctcatgaca atccctctcc ttttgggctt ctttgggttc tgggattttt 88800
aaattgttcc caaggagagt tttgggaaaa caaactccct ggaatcatgc aggcttccca 88860
ctgggacagg aaagtcaaag gccctttgtt attggctggc ctttggctgt cagcccttgc 88920
tatgaaccac tggcctgagg ctggaacttc ctgctgtctg caccatggga ttttgaatgg 88980
cacaagaatg aggctatatg aacaggagga gctgactgcc actggtgggc ctgggagcag 89040
cgtctccaca ctgtccctgt gcagagccca gagtgcccag caaaattcat cactctccag 89100
atcaggggct ggggacagtg gcccacacag accaaacact ggagctacag ggcatgtcac 89160
ccgagggaga tgcaaagacc tcactggggc tactaaggct ggctttccag gcctggatgg 89220
gctcccattg ctgcacctcc tgggaaagcc cacgacacag aggtgggcgc caaaaggctc 89280
tcagtggggc tttgtctaag gaacagatag gtccaggtct tcctcaggca agtgacctga 89340
gcagaggggc ccgtgtggac ttttcaccct gaagctagaa gggtgtggtg acgacagtct 89400
cctaaatgag tggctttagt cctagctgcc catcagcctc atccgccgaa ccttaaaaaa 89460
caacagatgc ccccgcccaa accaacttaa tctgaatttc tggaagtggg gtttgggcac 89520
tagtatttta cccaagctcc ccgggtgatt ctaatgtgta gcaaggtttg aaaaccacag 89580
tctaaaagcc acagactgtt aaactcttgg ggataaaggg catcacccag ccatcttggc 89640
aacaccaata agtccacaat gaggaggggt gggagggccc acagttagtt agggctgggc 89700
acctggctgg ggaacatcag ggtgattgac ttcaagactt actctttttt ttttttttct 89760
ttttttttga gacggaattt cactcttgtt gcccaggctg gagtgcaatg gcgtgatttt 89820
ggctcactgc aacatccacc tcctgggttc aagcgagtag ctgggattac aggcatgcgc 89880
caccacacct ggctaatttt gtatttttag tagagacggg gacggggttt atccatgtcg 89940
gtcaggctgg tcttgaactc ccaacctcag gtgatctgcc cgcctcggcc tcccaaagtg 90000
ctgggattac aggcgtgagc caccgcaccc agccaagact tattcttgat ggtcagtgct 90060
ggggactttt ttaaccacag tcttccacac taagagatta gaatttagaa aagttggggt 90120
ggctcagaag ttgcagggga tttggaattc ttgacagaca cttctggaag tggcaaggat 90180
ttgcttatgt gcaactgaca gtcaaggagt cttctctttg ggaggtgacc acattaaaat 90240
gataggaatt cactgtgggt gacagcataa agccaatcgt ggcttcctca ctcttcgtgg 90300
tgagccagaa gttgttaaag actgggccaa gaagattcca aaaatggaca caaagccaag 90360
aagaaccaga gagggtgagg aaggatagag aaggttttat ttggttattt tacttgggcc 90420
caagaatgtt gaacgtgaga acctgtctgt gaaattgtag tgtgcagcca ttggaaaatg 90480
tttattatta tctttagcaa aagctttaca agcaattctc ccacaccatg ttctttcact 90540

cactgacgtt cactgtcctg gtgtctgtcc tggagcagga aatgaaggga cacacacaca 90600
cacacaccag ggaggagcaa gaacggggcc caatgtggaa tcagacccac ttagaaacac 90660
ttctgagggg agtgcaaatg agaagcgtct gtcagccgag gctctggatt gatgccaaca 90720
agctgggcta gaagggaatc caggaggctc agtccatgca gaccacccag aggtctcagt 90780
gtgccgccac acttaaatta caccctcgca atgtctcctt ggagaaaatg catatgtgtt 90840
cattgcttga tgggtcatcg ctatttccat ccctgtgtaa tagacgtggg aggtctctcc 90900
caaagctctc agtggctcac cagtgctagc agaattcagt cagagcaccc ctgtgccacg 90960
actccctcgc tctcggtgcc ctctgctgtg tcacttgcct gccctgtcta ggggactaga 91020
tggaggctac tgcaactact cagcgcttcc ccctctgact ttagctttgg aaattactta 91080
tctcccactg aagttgatta agtcgcctgc ccagggttca tatcagttga accagcacca 91140
acagtagaac ctgcatctct tccttcgtgg tccacgagaa ctcgtacatg ccaagaggct 91200
ggagtcatag agcagcactt cccagccatt ttcacattgc atctcttaca gaaagtcatg 91260
ctttgggtgg tggatcagcc actctcggct ggaagtgacc cacaaaagcc cacacattgt 91320
tcaaggggtg tgaagctcca ttgtaaaatg gccgggacca tttccattta gagttgggat 91380
ggtctcttct cccaacggtc actaatctcc tccagatgga agagtaggcc cagcggggtt 91440
tccacccggg ttgtttataa cccagtgtgg ggtgggaggt gaaggtctag catgcacaga 91500
gggcatctgt gtggtgttct ggggctttct atacggtgcc aggtcaccca gaccacaggc 91560
cccgtgagtc cccttcgcta ctggtgccaa atccacgtgc catctgtaaa ttatttactc 91620
tgcatttatc ttcaagctaa tttaaaatca atccactttt tgtctgatct taacttaggc 91680
caaatgttat ctgtgaaatt atgattttgg tatgcaagca gacacttgtt actctaatat 91740
acattaaaat acacacataa ctagtaaaat ttatacacac acacacacac acacatatac 91800
atgtatatgt atatatatat ttagagacag ggtcttgctc tgttacccag gctggagtgc 91860
agtagtgcag tggcacaatc ttggctcact gcaaccttga cctcttgggc tcaagtgatc 91920
ctcccatctc agcctcccaa gtagctggga caacaagcac atgctgccac agcctggcta 91980
atttttttgt attttttgta gaaacagggt ctcactatgt tgcccagggt ggtcttgaac 92040
ccctgggctc aagcagtcca cccacttagg cctcccaaag tgttgggatt acaggcgaga 92100
gccaccgcag caggccttaa atgtttttta aacttctttg aacttaattc catctgaaat 92160
catccagcag cctccagaga taacaaataa caaagttaag gaaaaagcat ggtcatatag 92220
ttattgtatg gaatccgtgt gaattggtgg gtccttagac accttcagca tagaagcatt 92280
attcccattt tcctgttgtg gaagctgagg cacagagagt tgagagttat cataagatca 92340
cactaagaga gtgacaggac tggggttcca acccatagcg gtgagtacta aagccaccct 92400
ccttcccctc cgccaggcca gcagatgagt cgttggcagg atcactgcat ctgtcatccc 92460
ctcgccctca ctccagcctt tttttccctc cacagtcctc actgggcttt gtggccctcg 92520
tgctgagcac actgcacacg ctcacctacg gctggacccg cgccttcgag gagagccgct 92580
acaagttcta cctgcctccc accttcacgc tcacgctgct ggtgccctgc gtcgtcatcc 92640
tggccaaagc cctgtttctc ctgccctgca tcagccgcag actcgccagg atccggagag 92700
gctgggagag ggagagcacc atcaagttca cgctgcccac agaccacgcc ctggccgaga 92760
agacgagcca cgtatgaggt gcctgccctg ggctctggac cccgggcaca cgagggacgg 92820
tgccctgagc ccgttaggtt ttcttttctt ggtggtgcaa agtggtataa ctgtgtgcaa 92880
ataggaggtt tgaggtccaa attcctggga ctcaaatgta tgcagtacta ttcagaatga 92940
tatacacaca tatgtgtata tgtatttaca tatattccac atatataaca ggatttgcaa 93000
ttatacatag ctagctaaaa agttgggtct ctgagatttc aacttgtaga tttaaaaaca 93060
agtgccgtac gttaagagaa gagcagatca tgctattgtg acatttgcag agatatacac 93120
acactttttg tacagaagag gcttgtgctg tggtgggttc gatttatccc tgcccacccc 93180
acccccacaa cttccctttt gctacttccc caaggctctt gcagagctag ggctctgaag 93240
gggagggaag gcaacggctc tgcccagagc catccctgga gcatgtgagc agcggctggt 93300
ctcttccctc cacctggggc agcagcagga ggcctgggga ggaggaaaat caggcagtcg 93360
gcctggagtc tgtgcctggt cctttgcccg gtggtgggag gatggaggga ttgggctgaa 93420
gctgctccac ctcatccttg ctgagtgggg gagacatttt ccctgaaagt cagaagtcac 93480
catagagcct gcaaatggat cctcctgtga gagtgacgtc acctcctttc cagagccatt 93540
agtgagcctg gcttgggaac aagtgtaatt tccttccctc ctttaacctg gcgatgagcg 93600
tcctttaaac cactgtgcct tctcaccctt tccatcttca gtttgaatga ctcccaggaa 93660
ggcctagagc agacccttta gaaatcagcc caagggggag agcaagagaa aacactctag 93720
ggagtaaagc tccccgggcg tcagagttga gccctgcctg ggctgaagga ctgtcttcac 93780
gaagtcagtc ctgaggaaaa atattgggga ctccaaatgt cctctggcag aggacccaga 93840
aaaccacact ggctccaact tcctcctcat ggggcattac acttcaaaac agtggggagc 93900
aacttttcca ccaaagctac aaacctaaaa tgctgctgcc ccaaagcaca agagggaaga 93960
gcaccgccgg ggccacagga cgtctgtcct ccagtcacag gccatccttg ctgctcccta 94020
ctgactctag cttacttccc ctgtgaagaa acaggtgttc tcggctgagc ccccaaccct 94080

<210> 5
<211> 82154
<212> DNA

<213>  Homo sapiens
<400>  5
   cattaatagt gataactatt aataatatct gagtgttaac tatgtatcaa acactgtgct    60
   atgggctttc tttaactatc tcacttatct ctcacaataa ccctgtgagg caggtactat   120
   tacagaaggg gaaacggagg ctaataaaga ttaaatgagt ttcttagggt tgcccaccaa   180
   gtggaagatc ctgaatatga atcttacagt ctttctcagc ctgcattctt taccacaagg   240
   ctgggactcc cagaggacag catcctctca tgcctgggtg gcaagcagcc agccacagac   300
   atgctgaaga actaggggta gctacagtta gaaaacattc attgcttttt caaggaagag   360
   ctaaggagac ccaggacgca gatgtgagtt agcagagaac accagcaaat tacttaactt   420
   gacagaatta ttttctctcc tgggtcagtt agcttctact tgaccctggt gcttgttgga   480
   gtttgtgtca cccccatggc tctgccttct gctctgaagc acaaggttac cattagcata   540
   catcaggctg tggttaattt ggctgccccc tctttaagct aaggagacat ggtcagggcc   600
   tcagaccctg tgtgtggcca ttcagctttg ccctgttctg tggccacagt ccatgctcta   660
   agtactgacc agatgtctta caatgcacac ggcacacagt tatggtctct gtggggatgg   720
   gggaataagg tgtggacaaa tctgttcagc tctaaatcct tgaccagagg agcagctcag   780
   agcatgaatc cacagggcag tggagccatg tgcaggatca agcacagcgg catcactgac   840
   ttgcaggaca ctggagtgct ggggcgtggt tctagagaaa tgccctcaag tcctcgatag   900
   atcctaaatg tggttagttc tcttcattac aaagacagtg gaagctgagg tccatgcaga   960
   ttaaaaggct aagctgtaca gtaattatga ggtccttttg attagcagaa ctccatctat  1020
   tccaaaacaa attcatagaa agacatcttt tctatgccta attgcctttc catctttgtt  1080
   gaaaatcaac agaccataaa cgtggagtct gttttgggac ttgacattct gttctgctaa  1140
   tctatgtgcc tctctttgcg ccaatacttt actctcttga ttactgtagc tttttagtaa  1200
   atcttgacat cagatatttt aagtcctcca actttatttt tgcttctaag ttgttttgtg  1260
   tttacataca aaatttagaa tcaatttgtt caatttctac caaaaaagtc tgcttgggtt  1320
   ttggttggga ctgcattgaa tccatagatc aactattggg gattaatttt tttctctacg  1380
   ttttgactgg tttgtatacc acagattaga ggattctggg atggagcttc agaatccata  1440
   gcacaaacat gaagatattc agaacaatta acttttatgc tgatttaaag ctaataagaa  1500
   taaatcttta catcaaatat aagccacctg gagccagtat tattgggact tcattgataa  1560
   taagattatt tacccaaagg ggaatgattg tcaaagtacc tactgtgagc catatttata  1620
   aaatgtaagc catatttgaa aaatacttaa ttttcacaag aagctgatga ggtaggcatt  1680
   acctgctgta ctttacaaat ggggaaacta aagtacaaag agatgtagca gttaagctga  1740
   ggtcacacag gtaattaaat ggtagcattg tcacttaaac ccaggcttgt ctaattccat  1800
   tctgtggtac ttgctgtacc tcaatggttt tcagactgtg tccagagtgc cccaggacct  1860
   gagagagaga gagagaaaga aagagaaaaa agagagagag aaagagaaga gagtgagaga  1920
   gagtgagtgt gtttgaggga aggaggaaga aagtggtctg gggttcagaa tcccagactt  1980
   ggtctcagtg agttttcctt tgtcctattt tgcagatggg gcctccacgt gaaaagtaac  2040
   tacaaaacag aaccagaaaa tagtcatgct gttcaagtga gttggcatat cacttcatgc  2100
   tactttgaaa taaaatttcc tcttattttc ttatggataa aggtctcgaa aggtttgaga  2160
   tgatcacagc ctgtgttaag gctgaccagg gtccagggca gtacagtgga gctgcttttc  2220
   ttctggattc tgaaccaccc tcagccttca gggcctgttg tctggtgggc tcatggagtc  2280
   accagtgaac gtgaaagcca ctgctcctct ggtgtccatg tctattctga caggtggatc  2340
   tcagccccag gttgactgat tgctcgcagg tgaggctact gcctccgcag tgctcaccaa  2400
   atgctccggc cgctccagtc tgcacgttcc tgagcttggg ccacctcctg cttttcacag  2460
   gcagcttctc ctcgatctcc tcctctcttt tttcttcatg agcctcaccc ttggcagcca  2520
   ccccaagaaa ttcagccttc ttcatctcct ttctctctca gaaactggaa agatttttac  2580
   tgaattcctg atatttttgt ctttgccaaa acccaaaggc cccaatctgc catgggccaa  2640
   gaagtgccca gagaggttgt ttggacaaat ccagaaatga ccttgaatct ttactcagca  2700
   cttagatgac aggttctctc cttctccacc acaccctagc acacgtgctt cttgtcggat  2760
   caggaaatgt tttattgctt tttttccttt tcactttctc tacccaccct tctttgtgag  2820
   cccttcccgg ttatgctgtc cgtattgcct ctcttctgga aaagtaatca gagagtgaaa  2880
   atcccaggac aggaagggac cttgggaagg cattaaccag accttttcct ccaggcagca  2940
   gccacagtcg ccggggtaaa gaaccctctt ttccatcttt aaactttcat cggtttctgc  3000
   atcttctcat tgaagcatct ctgggttttg ctttttgttg tcttatttgt tactttcatt  3060
   tttaaaagtt ttctttcatt cagaggtttc ttgagactgc ctggtgcaca tcatgcgtat  3120
   ccgctgatag atgctgttgt gacttggtga aacatgttgc gtgctcttca ggcagatgta  3180
   tgtcaacaaa cccttttcct ggctcagcca ggatttctgg gagtcagatt tgattatggg  3240
   gtgtcatagg taaaaactgg aggctgtctg tggtggccca gctgtgcagt tagctgactt  3300
   ggaggaatct ttggctataa ttggatgcca agagtggagg tggcacgaga gatggtacaa  3360
   gcctatgatg atgtctaccc aggtgaaaaa tgatctaggt gcttgtaacc atcaccctga  3420
   gcattcagtt ctacatccag ctattgtgga acaaaatgat ttatattgat taattgacta  3480
   gttggttcat tttagcttga agagtactga tcagctgtgt gcattaggca gtttcttgac  3540
   acactgctgt gctgaggaca gatttcccag agcctggatg tgtataattc tacatgctcc  3600
   tttttccaag gacttaagca aacagagtca actcctctgt tttccttgtt tgggtatatt  3660
   tttgaaaatg tctcttcttc tttcctttca tttacatttt accgttgttt atccaccttt 3720
   tcctcttcct tcaaagctct agacctgcct ccctgcctgt tcttagccca cttttgagaa 3780
   aggactttcc aaccccatcc tcgaaagggc agggtctctc tccagggctc caacagagtt 3840
   ctgaaagaac acggcttcca ccattgtgtc ttctctgttt tgtcaactgc tgaaagactg 3900
   acacagtaga caatgctgca gaccccttgg gagatagtcc aagtttaggg tggggaggga 3960
   atggtgcatt ctgggtgtcc acatatttgt tgttgcttta ggaagaggag ctgggaggtt 4020
   ggagtcgcca cacatgttga aagatgtggc aaaaaacaaa caaacaaaac acctcataga 4080
   gagaaatcct gactttgtgc acaagaaaag ggagtgaagt gtgtggggtt tttaaagtgg 4140
   tggtgggggg gcattaggag atacccagaa gctctcaaga ttccaaacag tctgcttttc 4200
   ttttcagaca ttacactcca agaaaagaaa ctctagtgta taacagaggg gctctgtcaa 4260
   aatttaacaa cataaatatt gggcaaatga agttgtttct ctggtgctat ctcaacctgc 4320
   caatggagaa atatctcaaa aagtcaagaa ttgaagccat tactcagaga cagcatccta 4380
   tggcatggtg atccaaaaca agtacacctc taccttttcc ctttcagccc caaaccccaa 4440
   aacactggag aatattgggg caggcaaggc ccgtgaatag gtgagcagag ctggaagaag 4500
   agactcctgc ccctggtggg cctcaggcta tttgtggaat gatgtttgtg agatgaacat 4560
   gttcgctccc tcacccggat gttttggaca taggagcctg gagtaaagag tgtatatctt 4620
   gggctgtcat tttcccgtgt gaaaggtgtt ttgttttctc cccgtttgca caacttgttt 4680
   cccttaaact gtgctgtgca tggctaggaa gctcagcaat agactaaaca gtttgcgatc 4740
   ccacagtttt gctttctgtt tctcaggcct gacccaacac tccatgggtt ccctagccct 4800
   tcgtaggtgc ttcgaaacac tcatggggtc cttgacctga tcccagtggg ggtttgtgtg 4860
   tggaagttag ggaacctctc tttgagagtt ctggtgcccc taactctgta gggtctgact 4920
   tcacttctgc ccaataagaa atgaaaggct ttgccagtag tgcagccctc tcctcccaaa 4980
   gtcctagagg caattactct gaaatatgga agaaagaggc acatctgaaa gcccagtgct 5040
   gtcttcttgt cctcacatcc ctcaggatct caaggtcagc ctagtagagg cattgcttta 5100
   ccatagtagc aactcaggga atatctgtgg aactggattc ctctgaagtt ggacagttgc 5160
   tttatccctg ctagaggctc tcactactta gacccacaca gtcctataga tagaagaaac 5220
   ctagagagaa ccacctctca cacttttatt ttatacatgg agaaccaaag gcctcaaatg 5280
   gtggagtgat ttgctggggt gctacagtca ttgtcacaac ttacattgtg acatgggctt 5340
   catatccagt taaaacaaaa gcctctattc actgccccaa ggtgtgaagc cttgtctcca 5400
   ggagatggag agtcaatgac tccaaggaga taagacttca gactcttcct gctgcaattt 5460
   tttttttttt ttaagtaagt gccaaccaat aactcctggg gaaagtaaaa taatctgggg 5520
   gaggaaaatt gttctctcct ggagatggga ctcctctgat ttggaactcc tctgtctgct 5580
   ttcacttctt ggataggatt ttaaatccag cacccagcct gctgctgtga agttcacaat 5640
   aactttatca cctgagttcc cttggtgctg cagcagatat gagttacaca cctggtcctc 5700
   cgagagtggt ttaatccaca ccagagtcct gacttactag tttcttccta ttgcagagcc 5760
   tatgcagact gcttctctga gcaccaacat cattatgcac tgtcctttct attcccaaga 5820
   cttcctagtt gctgcaagtc aagttcgaca ttcaactatt tcctcggggt ccttttttgg 5880
   gagaagcaag gctttgagtt cactctttga cttcaagtga agtccatttt cctcttagac 5940
   actaccccag tggttctcac actcaaggaa gcaccaaagt ccctggagga cccgtctaaa 6000
   tgaaggatgc taagccccac ctcacagaga ttgatgcagt acgtcccagg aatctgcatg 6060
   ttaaaggagc tctgcaggtg attctgatgg aggtggccct tgcaggatgc tttgagaaac 6120
   tatatgttct atccctcgaa ctctgcctgc agaacagcct gagatggctc ttccatgctt 6180
   tgaccatttg gccctccaca gtagtccctg agctgcgtct tcttcggacc atgagcagaa 6240
   tgtgtgtccc ctgactgact gtccttttcc ttgagaacca ggcatagtgg tgcactggta 6300
   agagcatgga ctctggagcc acactgggtt tgaattatgt ctctgccact tactagctga 6360
   gtgaccctag gcaactttct aaatctctgt gcttttgttt cttcatctgt aaaatgggga 6420
   taataatact acttgcccca tagggttgtt atgaagatga accaagttaa tatctctaat 6480
   aaatgtctct aaaatcccat gattgcaaaa agtagaaaat atgtcatgta gcctattata 6540
   tcattatatt ataaggtgag gctattgaag gcaagagggg aaatgacttg ctgaaggcaa 6600
   cagagctggt ctgcagcatg gccaagacca aaaacagctc ttttcgctcc cagataaatt 6660
   gtctttttta tttttttccc aaactgcctg tctaaccccc ctttcatctg gttataatga 6720
   atgccattgc ttgtgtatag ttgcctacat taatagacaa caatgcaata ttctccttat 6780
   ggaaacccta cctagattgg gaggcaccat gaagccactt tttcttcttt ctccatctct 6840
   accctgatta gcaaacatct ctaattattg tgactcccaa ccccagtcag atcattttat 6900
   tctttttcca gtatctatgc agggaagcct ggcaaaggct taaggatggg ggtggggtgt 6960
   agcatgtcag caggatttgg aactatgggc cccatacttt tggcacctgc tgcattttct 7020
   ccccacctgc tgcaccataa ttcatctgct tgtatgagtt tagcagaaag agcccccagt 7080
   tccctgtaag aaacacatac ggcttttcca ggttacactg tggggattga gaaaatgcac 7140
   agtatccatg tctggaaatc aatggacaac tcactgcctg aatcgtttgt gtcttgtaac 7200
   ttgcctcaag caacatctgt ctgaaaaatc tggtgctttg ggctaaatga ctggatattc 7260
   tcctaaggaa tggcagtgag tgggttaatt tcacctctca tcttacttcc tgcctggccc 7320
   ctctaccttg cccaccatgc ctggctttgc tgctgatctt cggctgacct gaaattgaac 7380
   accttaactt aaggacaact aagggtgtag gtagattggg aagtatgtgt tcttcttgct 7440
   ttgccaataa ttgtcttctg gattcattgg cctttcttct ggtttccttc ttcaacttct 7500
   ttaacaaatg acttcagcca tgccaagcct tcttttaagc actttccctc tcactctcct 7560
   gtcctataaa gatatctttc ttgaagcccc tccgtgccag gcgtccaaag tgaggcacat 7620
   caactcggtc cttgaacgtc tcatgtttat cccaatagat ggtctagatg tggatgaatg 7680
   taaaggaaat gtgcaaataa gatactttta aaattcagac agaattatac agggtgggaa 7740
   atttcagtat acatagaagg cttaattcaa aatgatataa tgtcaacatt tggtataaga 7800
   aattatttat ctggcagaat atatactcac agatgtcaaa atgggtctag agagtagaga 7860
   atggaggcaa ttatccagac actgaggccc aaggcacaat acttttaagt aatcatgccc 7920
   tcaactggca tttccatagt cttaagccca aaagcattgg gtggaaccat tctataagca 7980
   aagcctcagc tatgaaaaca acttttaaaa ttgacattca aaagataaga tcaacaacag 8040
   gtgccctttg ctttcggcag taggtgtgtt cttgaaaagt tggttgtcaa tatttccttt 8100
   tcccattaat ctaatcacat tctaaatata acagggggct ggatgtttga agaaatcctg 8160
   tagttgatct cattgcaaag tccttttaga gaggtaaaaa tagtttgttt tattttcaaa 8220
   aattaggttt tcttagacta aaacttcttg gaagtggggg catatcatgt tcctgtatat 8280
   tgcctacagg taaagaatgt aagaatgtat ggcttagtca cagagagcta gggcttcaga 8340
   aatgctatat gtatcaaata ttttatatct atatttactc tgaagatatg acaatagctt 8400
   ttaatgtggg tcgagagata tctaatcttt cgcttctctt tcaaccttcc aaaaccatgc 8460
   aatcctcatt ccttcttttc aataacctcc acatgccccc ttcatctgtc ccacccaaag 8520
   gaaactcttt taacttacca tttaattgat caactctgat gacctttccc taagctctct 8580
   gtgctaaaat aatctggtac tttgggctga agaagtattc tcctaacgat ggaggtaggt 8640
   ggcagtcaaa tgacgcagag attatgatag ggccctgtct aggctgtcac ataaggtaac 8700
   attagccaca tgtggctact gtgtacttga aatgtgatga atgccattga gaactgaatt 8760
   tttaatttaa tttaaaaatg gagtcgcatt tctgttttag gagaacttct aagtatgtct 8820
   gaaacaactt gatcatgttt caagtgtaag ttttattaga tctaaatcta aatcaagtat 8880
   ttcgaaaaag tattttagca tttgaataga gatgtgctct aagtataaaa cacacagtgg 8940
   attttgaaga ttagtaagaa aaaaatgaat gtaaactatt tcattaataa atttttatat 9000
   tggttacatg ctgaaatgat aatattttgc atatattggg tgaaattata tatattattt 9060
   aaattaactt tccttttttt ttttaaactt tgtgtggcta caaggacact gaaattagaa 9120
   acatggctcg aattatgctt gcactgaaca aggctagtct aaggtatgca attgccctgc 9180
   ttcttcacta ctttcaagtg taaacaaata tgtcagcatt atccctagaa ggagggttta 9240
   ggggttttaa gttgaatact cttcagctga agaaaatcca ttaatctcca gttttattct 9300
   tgagttagct ttcaaatcta cattgcgaca ataattactt tgggatcttt gaatttcttt 9360
   tcatgccttt ctgttctatg aagcccacat gcgtagttta tgcattttcc aatttaggct 9420
   agaagtccaa tgtgctgtcc attgcaccac agagcctgtt tgctatgcat ttttaagagt 9480
   aaaattttca gaaaatactt aaattaaaaa gatagatttg gaaagcatat ccaaaatgtt 9540
   gctttgaact tcttattctg gacaattcct ggactgggga gaaggcatgc ccaagtacac 9600
   attgcttgca gtggcacctc caaaccaatg ctggtcggga ctcttcctat tacctcgcaa 9660
   cagcaatgac tgggccccat aatacagcca cggtcattat aaaagatcat tacaaagcat 9720
   caaattaact gctattaatg aaagccagaa acacgtcatt caggaggtcg aatggctggg 9780
   atggaacata cagggttgaa taatggtagg agcctgggtt tggaattaga aaactgggac 9840
   aggaatccta cttctgctat gaatccattg caagtgactg ggcaagttgt tcctcactcc 9900
   ctactgcctt gctcatctat cagctgagaa tgtcaaggca gaaggtctcc agggctcctg 9960
   caggctccta caagccagat ctcaatccgt cttatcctac cctcttcctg acctctagct 10020
   acaagaacag caaggcacag ccccctggtt ttgagaatgc taatgcttgc cagcattctt 10080
   gtcctgcagg gcatgaagta tagtcctgca caaccagcag gctctgtgac tgcctaacct 10140
   catccttggt cactgacctc tgtcttagta gcagttcgtg ccatcctacc tagctttgct 10200
   gggtcaagtg cgttcacatc ttaaaaccgt ttgaagcgtt tgtctaatta acatgcagaa 10260
   agcatatact gatttttatt ttcaccacca tgtatgtgag ggcctatttt atggcatgca 10320
   aagcatctca tttttatttc aattctctgt cttgtaactt taaatgaggc tgaaatttcc 10380
   ctccatatgt ttattagcat cttctatttt atctcttata cactgactgt ttgcatcttt 10440
   tgccccattt ccctgaatgg tttttttctc aatgatttgt aagcactctt tatatttaaa 10500
   tatgttaact ctttcttgtc acatatttta aaccctttct tacaaacgtt gttttccggt 10560
   ttgtgactta ccttttaatt ttgctcataa attactttta acttacagat agtaatattt 10620
   ttgaaaggtt acattcatca gtctttgtat tgttaaaaag agtctttatt gttatacttt 10680
   taaagtagct tttccactcc tagtcagata ctttcacata taatagttgt ctactttcct 10740
   cctgaattaa tggggattta ttttggcata tggtgtgaag taagaagcta aattcttttt 10800
   cttttctctg catcatcaat gatctcttac actgttaaat aaactagtct tttccttctg 10860
   ctttccacac ccatttaacc tagcccactg ttgacatcgt cagagaaaac ttttcatcag 10920
   aaagtctgaa cagcacaatt atactgtatc aatccagctt aaattacaca tgattgttag 10980
   gatgggtaga aggtatgata aaatggggtg aaagtaaaat gaatgatcat cgatatacat 11040
   gatgtcaaaa atttgggtat aactgaagaa ttttagtaat ttaaagattt cagaatttaa 11100
   ataatgaaat atctgataca ttcaattgga aatttggttg agatcagctg acttgttaat 11160
   tggacttcac tgataaacat ttgccttaaa atctgtagaa tctaaagcag ggtataccta 11220
   gtatgcgagt aagacatcat ttctggaatc aaggggcctt ttcagtgttg accagatgtt 11280
   tatcactgca tgggcaaatg gcccaaggag tatggttgtg gcttggctta tttcttctta 11340
   acgcagatgc agttgctttc tttctgggtg gcagtgtgga ggataactgg ggtcggtggg 11400
   gacataaagt ctcacaaggg gaggaaagcc cctctgtggg ctctctgggc cagccaccct 11460
   ccttacaaag tgggatgtca gcttctgctc tgaccagagt caaagtaggc atcagctatt 11520
   gccattactg ttgttgtata atatggtgaa taaaggaaat gtgttatata tatatttttt 11580
   tcttattatt atactttaag ttttagggta catgtgcaca atgtgcaggt tagttacata 11640
   tgtatacatg tgccatgctg gtgtgctgca cccactaact ggtcatttag cattaggtat 11700
   atctcctaaa gctatccctc cccccctccc cccaccccac aacagtcccc agagtgtgat 11760
   gttccccttc ctgtgtccat gtgttctcat tgttcaattc ccacctatga gtgagaatat 11820
   gtggtgtttg gtttttgtcc cttgcgatag tttactgaga atgatgattt ccaatttcat 11880
   ccatgtccct acaaaggaca tgaactcatc attttttatg gctgcatagc attccatggt 11940
   gtatatgtgc cacattttct taatccagtc tatcattgtg ggacatttgg gttggttcca 12000
   agtctttgct attgtgaata gtgccgcaat aaacatacat gtgcatgtgt ctttatagca 12060
   gcatgattta tagtcctttg ggtatatacc cagtaatggg atggctgggt caaatggtat 12120
   ttctagttct agatccctga ggaatggcca cactgacttc cacaatggtt gaactagttt 12180
   acagtcccac caacagtgtc aaagtgttcc tatttctcca catcctctcc agcacctgtt 12240
   gtttcctgac tttttaatga ttgccattct aactggtgtg agatggtatc tcattgtggt 12300
   tttgatttgc attcctctga tggccagtga tagtgagcat ttttttcatg tgttttttgg 12360
   ctgcataaat gtcttctttt gagaagtgtc tgttcatgtc cttcgcccac tttttgatgg 12420
   ggttgtttgt ttttttcttg taaatttgtt tgagttcatg gtagattctg gatattagcc 12480
   ctttgtcaga tgagtaggtt gcgaaaattt tctcccattt tgtaggtttc ctgttcactc 12540
   tgatggtagt ttcttttgct gtgcagaagc tctttagtta atgtgttata ttttaagagc 12600
   tcagttatct gttacctatt tgccagtgac aggaactcat gtcttctgag gcagttggtc 12660
   tcatttatgg tagatataat taattattat ctaattatta ttgcccttga attatttgaa 12720
   gatatcatca ggccctctct taaacacttc cgttcatgtg ctttagcttc tttatatggc 12780
   cagccactct ctgtggatat gtccaggttt gttgatgtcc ccttgacatc agccaaccat 12840
   aacttaacac tggatgagag ctaagcagtg ctaaacttat cacttccctc gaatgtgtca 12900
   ttatactttt attagtgctg cttgggacta gatttccttt ctagcagcca agtcatgttt 12960
   tgggctcata tgcagcttgt agctaagaaa gccttgagtt gttttcctat aaaccgtctg 13020
   aaactatttt tagaattcaa gttacaactc tgaagtcctg ggtcttactt aatcaatgat 13080
   accttctgag ataaatactg aaaatccttc aagtatagaa caaggacatc ctaactctgg 13140
   gcatctgatg tctgttaaat gtatcacatt ttatttattt gtatttttta aaggaagtga 13200
   gaaggagaag tgagatgctg agcatcaagc tgggcagagt tgtcatgttg tgatctgaga 13260
   ttctgtcctg ttcttcagga tttaaggagt cgctaaccaa tgccaagcat gggctcatcg 13320
   ggtgcagctg gggaggcaat tacttcaact caggagtttt caaaaacttt ccttatgcct 13380
   gctcacagga tttcatggtg tttttcttct ttgttgttac ttttgtggaa aaggaaagtt 13440
   accagtgaac aatctctgtg accttcctct caatcccaaa aatatgcttg tgataaatca 13500
   gcagtttatt ttaatgcctc atttggaggc attgcttttc caagaatgaa tctttgcata 13560
   ttatctgaga catcattttg gtgtaaggta gaattgtacc aggtatgtat ctttgtggcc 13620
   aactatactg tcaaaactga gtttcagtca tccaataaaa aggaagctgt ctctgaattc 13680
   agatattcct ccattctaag ggaactgaag tataacataa aatacttaac attctttctg 13740
   tatatgtgaa aaaaccatac ctaaggaatt ttattattca gcccatgttt ctttattgta 13800
   tccagtataa catactcgaa gatgtatata tacacatata tagatataca aagcatctat 13860
   acatctagac atgtatatat acatatatat atacacacac gtatatactg tacttcaatc 13920
   atctatatta aattgtgata atatcacatt accacatgtt gatggcattc ttgtaatctg 13980
   cctgaaatcc atgaaaacac atgcagtggt ctggctcatg ttgtctcagg gagtgcattc 14040
   tggctcccag tgatcatcaa ttacttttat aaaagttcac caatcacttg ttggtttccg 14100
   gaaaacaaat ttaaagcaaa cattattaat gtcaaactta cacattttgc tttctggaat 14160
   caactttttt attttaaaaa ttgagaatat aatttctgtc ttttgtttct ttgggcattt 14220
   ttcttattct ccacaattat tcaaagatcc ttagccatag gtctgaaagt gtttgtttca 14280
   gctctcatcc cccttccttg caggtaaggc tggcattcca cagccctgga taactcctct 14340
   gtgctaggtc tatgcctgct cctgggacac tgaacactcc taggtttgca gggtccacgg 14400
   tgtccttcgt gtccctccct gcccagcctc agcaggagcc ccctgctcct agaccactcc 14460
   cttactgagt cctgtagatg ttccagcaca cacagtgcct cccttctcaa ggccccagcc 14520
   ccagcccctt ctcaggtcat ttgggggctt acatcacctg ctacctgtgt ggagatggag 14580
   gtctgtttgc cagcactgcc ttgcttttcc tcctgcccca ctgcatttaa tcactgtgat 14640
   aagaggaaac aaagagggaa cgtcattggt cccattcctc tgaatcttga ccacgaaaat 14700
   atcttgaatt gcctccgtgt ccccacccaa catcgcctct tgcactgcta cctctgcctg 14760
   agtgcacatc agccaagagc caggaaccac tgcccaacca cgacccaggc acagaccctt 14820
   atctctagcc tagagaagag gtgtgtttct ttctaatcat ctggtatgta tccaaggaaa 14880
   ccataaattg atcattattt ttgtatgtgg aattagaaat attcatatag acatacattc 14940
   tttggttttt atttttttat catccctaaa ataaatagca gatgttctca aaatggttta 15000
   ttaatttcag tcctaagatt gtataattga ttgaaaccat aaatggcaga cagaagaaca 15060
   gcaacagtga aaaccaagat taattctaat aaattcatta caatggcagg aatcaagtgt 15120
   actttaagct cactgtaaag ttttcagtgt gttgtggatt cagaggcagt aggtgatgac 15180
   ttttattata tattcttaag tacctggttt tggagggtga tggcttgttg gagggaatga 15240
   tgctgaagga acataataaa acaatatgat aaatacaggg agagaagaaa gacaaaggaa 15300
   aagaggaaac aaatgtcaag aataagatga ataggtaggg gagaagaaaa aataagttgt 15360
   cataatttta tattccaagg actacgctag tccttggact acgctggtaa aaagaaatct 15420
   aatcatgagg cagagaattt tatttatttc cccattcctt ttggctcgtg gcttgagttt 15480
   gctctggctt tcattgaata catgggccaa ggcaggatga gcttctgcct gaaccgctgg 15540
   gatgttattc ctgatctcat ggaaagacat gtcgcggctt ctgaccctgt gcctctgtat 15600
   cactgtgttc cctgtattcc tgtgcccctc gtattagttt tcacattgct aatgaagaca 15660
   taccagagac tgggtaattt ataaaggaaa gaggtttaat ggactcacag ttccacatgg 15720
   ctggtgaggc cttacaatca tggcagaagg cgaaggaaga gcaaagggac ttcttacatg 15780
   gtggcaggca agaaagagca tgtacagggg aattcccttt tataaaacca tcagatctca 15840
   tgagacttat tcattaccag gagaacagta tgggggaaac cgctccccat gattcagtta 15900
   tctccacttg gccctaccct tgacacatgg ggattattac aaaatcaagg tgagatttgg 15960
   gtggggacac agtgagacta tatcacccct gtatccctgt gtctctatat tcctgtatcc 16020
   ctggctgaat gggtcaaagt aatgatcagc ccacagatat gcatttgcct ctgctgagaa 16080
   tagaaaggaa tataaatgaa gtctttttct tcatggagct tattaactag atgaagagag 16140
   aaagcctgtg ggaaaagaca aatagaacct tcaggttcta tgcagtgagt gcccaagtgg 16200
   tgattgtgaa aatgaaagtg cagccctgca gatgagcaga agctcatggt gggacctttc 16260
   atgcaagcta tgcagaggag agagcatagg atttggggtt gggacacaca gattcaaatc 16320
   atggtcctgc cactttcctg ctacatgaac ttgaccatgt tattttaccc ccatgaagtc 16380
   tgagttttca gatgtgtaaa ataataccac taatgcatac ttttttaaaa tttaaattta 16440
   aatttttttt tgacactgag tctcactctg tcaccaggct ggagtacagt ggcacgatct 16500
   tggctcactg cagcctccac ctcctgggtt caagcaattc tcctgcctca gcctcctgag 16560
   tagctggaac tacaggtgtg taccaccatg tccagccaat tttgtatttt ttagtagaga 16620
   cggggtttca ccatgttggc caggatggtc tcgatctctt gacctcgtga tccgcctgcc 16680
   tcggcctccc aaagtgctgg gattacagat gtgagccact gcgcccagcc taatgtgtac 16740
   ttttaaaagc acttttcaaa tacttcatct tgtttaatct tcttggcagt tctgtaaagt 16800
   gtgaagcatt gtacaaagtt aactctcatt attatattgg aacccagggt tttcagagga 16860
   gaatcctcaa ggagataggg ctttcctgcc ttgtgcccat ataaggttgt ggatcccaag 16920
   ggacgaaagc attttcatca gactgggaga ggcaagaaaa ggtcactggc agtatagatt 16980
   cttctggagt tcactggcaa cttgtcccac ttcagttctc ccttaatctt tccagatgtg 17040
   cagatgtata gcctgggtgg atgagccaaa atgaattcaa ggctaaacag gtcagctggc 17100
   tcagcccatg ggaagcatgc tcatcccaaa gcaaaggtct ctggcagaaa tttctctgca 17160
   cttatttacc ctgctgtctt cacattgggg tgcggagtct aattcaaaaa ctccaaggca 17220
   tgagtccaag ccagggagct gttagaggtg cacatgggag atcttagcaa gagaaaaata 17280
   tatgttttaa accctagaaa gaagaaagta aatgcctctc atttacttta aacaaataat 17340
   atggccaagg tcacatagca ggaaagtggc aaggggtctg tcctcttttc actgttcaat 17400
   catgaccata ttgtcctgga ggcctagggg caggatataa tgaagaaaca tccaattttg 17460
   acagagtggg taaatgggat tgtgggactt gtagtgagct gatctgtctt cattcatgta 17520
   attatcaata tcatttcaat gaccatgatg ttgatatgga aaatgatgag gatgatagta 17580
   cagaaagaac atgaatatta gctgatgtct attgagtgtt tcctgggtgc cagacaccac 17640
   cgttaagtgc tgaacaagga ttatagaatt taattctcta gtgacctttt gagataggtt 17700
   atatgattgt cagctttcca caaatgagga agccaatatt cagaaggata cggtgctgaa 17760
   gcttatgcag taagaaatag tagaagcaga attcaaactc aggcaattga acaccaaaat 17820
   tcaagctctt aattactatt tctagaaccc tctgactgac tgattcctcc ctcaccacag 17880
   ccaacttttg catcccccat ttctggagag catctcccca catggtatat actcaaaggt 17940
   cccatagtga tcctcttccc aaagagcgtg aatgaggggt ttgaaactgc cagaactctg 18000
   gtccagagct tggacttttg atgcgaatga tggagaaacc aagaggttga tttcctcagc 18060
   cttcaagaga gctcatcctc agctctctaa agctgaccag aaagaggaga gtgggaccta 18120
   tgaaaacagc aggagatggg agggcttgaa ttcctaatgc tatacccata ggtcattgtg 18180
   ccatggtcct ctgcaccccc aacaccccca acactgatcc tgaaggtctc aacaatcttc 18240
   atctgcctct ctcctttcac cttctttaca ctctcattga tagccttggt tttttctata 18300
   cctccagtat tcccattccc atgatagcca tttaaggaca gggcacttcc tggtcaccat 18360
   aaggaatatt gtgggaatgt tctgctcatg gatattgcat cagaataggt ctggaaatga 18420
   aacacaccaa caaagtcaac agaggagagt ccaagcaaag cgtctgttta caaagttgtg 18480
   gaaggtttag gggaactcat tatgagatag ttgaagcaac ccagagctag gaacagtggc 18540
   gctcttacca caccaaggtt tgaaggagct cgggtttctg gaacccagac atagcagcag 18600
   cagttgtgat agaggagttc ctggcagaag ccatagcttt cagttgagaa ggtggtcact 18660
   gccaacccat ggctcagctg gaaggtaacc agagaataaa ttccccaaac tctttctctt 18720
   cctttcctgt gctctcttat tggtgcctcc cattgagaga acccaaccaa agccagctgt 18780
   aagggaaact aatgaggcat ttccatatag ctcagcctgc tgggaaacat gtactataga 18840
   aaagggtgga gagcggatct agagaggcaa aaaaattaaa caacggcacc gatgtttaca 18900
   tttctttctg atgtagagac ctcataccac ttttctgtac atcttacttt aatgtgtaaa 18960
   aaggcctata ggctctttct ctaggttcag taaaagatga actaggaaac atatgccaca 19020
   tttgagcttc ctcacagctt ggcccctgca aagctgtccc cttctaagaa ctaagtgccc 19080
   tgggtattca gtgctgtgtt gtctggtgac ttcgcagccc tgtcaacatt gtttttctgt 19140
   ttgtttgttt gtttaattat actttaagtt ctagggcaca tgtgcacaac gtgcaggttt 19200
   gttacatatg tatacatgtg ccatgttggt gtgccgcacc cgttaactcg tcacttacat 19260
   taggtatgtc tcctaatggt atccctcccc catccgccta ccccacgaca ggccccagtg 19320
   tgtgatgttc cccaccctgt gtccaagtgt tctcattgtt caattcccac ctatgagtga 19380
   gaacatgcgg tgtttggttt tctgtctttg cgatagtttg ctcagaatga tggtttccag 19440
   cttcatccat gtcactacaa aggacatgaa ctcatccatt ggctcctcct cagaaaggtg 19500
   gaaaagcaga acctcttaaa ctgctccagg tttcctggag agccttgcct taaaaaaggc 19560
   cctttttttg ggtggggggg gacaccaatg ccgtgactcc ttcaagaatg tgattctagc 19620
   aagagactga gctctattct ttttcttcct ccaccctctc ttaaggcaaa ttgagtcaaa 19680
   ccatctcagt ctacccacac catccttctt taatttctgt agttaagagc ataaatccct 19740
   tccctattct gagtgtggtt catcgaccta cagcatcagc atcacctggg agtctgtcag 19800
   aaatgcaaaa tcttaggctc cagcccacat ccctgagcca aatctgcctt ttgcaagatc 19860
   cccaggtgat atacatgtac attaatttta ggaggattga tgtagcctgt atgccatggt 19920
   aagaggcatg caatgtaatg ttgagtttct ttctttagaa tttatagtag cagccgggac 19980
   cggtggctca cgcctgtaat cccagcattc taggaggctg aggctggtgc atcacttgag 20040
   gtcaggagtt ggagatcagc ctggcctggc caacatggca aaaccctgcc tttaccaaaa 20100
   aatacaaaaa ttagccaggt gtggtggtat gcccccgtag tcccagctac tcaggaggct 20160
   gagatgggag agtagcttga acctgggagg cggaggttgc agtgagccaa gatcacacca 20220
   ctgcactcca gcctgggtga cagagtgaca tagtgagtcc ctgtctaaaa aaaaaaaaaa 20280
   aaaaaaaaaa aaaaaagaat ttatagtagc cacaaaagcc tttttgagta attgaagaaa 20340
   ttctgaagtt attgtgtgct ttgaaattta gtatttattg aatagtttat atctttcaaa 20400
   aagcttttat aactcattgg agtctcgtca taactccatg aaatacgtaa ggtagataat 20460
   aggagccaca ttttccacat gaggatctga ggcatgatta agtaaaatca gtcctctgct 20520
   aggtggaatg gttttgagac cagatactag ggctcagaac tcattttaaa gctgttgtgg 20580
   cagctatcaa ttctgaagaa agtcaacatg gcatagcatg cctcttacca tggcatacag 20640
   gctgtatcaa tcctcctaaa actaatgtac atgcacacca cctgagggtc ttgcaaaagg 20700
   cagatttggc tcagggatgt gggctggaac ctaagatttt gcatttctaa tagactccca 20760
   ggtgatgctg gtgctgtagc tccctccgtc ccccagcttt cccccatcag tatttctgaa 20820
   cacttgatcc acacccaatc tgcctcaaaa tcacttgtgg tggaaaatgc ttgttaaaca 20880
   ggtagattgt tgtgccccat accaagacat aatggatttc ttatggtctc taaatgtgag 20940
   aatcactttt ttgtattggt ttgaatgttc tgatttaaga gtatgggatg agggaatttt 21000
   aggttcattg tgttcacatc agtaggactt aaacctatgg aagactttgc agcagaatag 21060
   gaagattatt ctcccagcct acccaacagt tgaaaaacga tcagaaggac ttggaaggga 21120
   gcaatggagg attgatgaca atggcaaaga ctgagcagtc cccaggcgtg acaacaaaaa 21180
   ggaaagcaat cagctgtgga gagagaaatg gtgttgatga tattatgaaa gtcgggatat 21240
   aaacaagaat gagaaaggga ggaatgggga aactgaatga gacagaagca cagaaaaaag 21300
   aagaaaggaa aaaagaagga aaaaaggagg aaaagtggga gaagagacag atggagagtg 21360
   agagaaggga ggcaggtagg taggcagaag aatgaggaag aaagcaaagg agttgagact 21420
   gggaatggac agaaggaaac ctgtacatgc tgtgatctgg gctttctatg ttccctgatc 21480
   cagggtcctg agatcttggg gtactcgcca ctgcattgta agtttctgtg gatgaagctg 21540
   gcatataaat acatctgtaa agctgtaact cctgggcatt gtccctgaaa ttttactgat 21600
   gtctagattc taggtatggg attaaatggg tccacatttt gtgtttttct gagtcaccaa 21660
   ttacaacaaa acttgctcat atacttatgc ctatcttaaa aaataattta ttttaataaa 21720
   ataataataa ggcaagaaaa aaagacaccc agatttgaaa aagcaaaaaa tgtctttatt 21780
   tgcagacaaa atcatgtatg tagaaaatcc taaggaatct acaaattagc tactagaact 21840
   aataagaaaa tttaatgatg tctcaggata cagggtcaat atttaaaaat tattgtattt 21900
   caacttataa gtgatgaatg tttggataat ggggtttaaa taatccattt acaacaacat 21960
   ccaaaatata aaataactag caacagactt tataaaaaca tacaatgaaa attgaagcac 22020
   gttgctggga aaaagaaaaa agaaaacaaa attcataaaa agacattata ttcactgaac 22080
   agaacactta gtatttttaa gacgtcagtt cttatcaaat ttatgtgtag actcaattca 22140
   atcccaatca acattccaac atcaacattt ggctagagag caaagttggg gtcagattgt 22200
   gtagggctgt gttaaagagt tgagcattac atgagataat ggatgtgaag aaagtgctta 22260
   atagggggtc gtgcagagga agtgctcaag aaatagtatc attgtcatta ttaccattac 22320
   tgtattgttg ttattattta acaaatgttt tcaagcccac tttggcttgt gtatgggttg 22380
   aggaaaacgc tacccaggca gaaagataaa acttattctc catgcctctt gagaaaagaa 22440
   gcagcaatcc ccagatgtag ggaggagggg agccctgctg gcttttagtc agcctcacac 22500
   aggaaaggag aggtagagat tctgcagcga acaagaatca tgtccgggac ttcacagaca 22560
   gggacactca aaggttggat gatgctcaat tgcagcatcc acctgcaggg gcctcacccc 22620
   acttctcact taatgattgt ctctgctgtt agggagccct tgaaagccag gtgttttgtc 22680
   tccttcccaa agcctcagaa gatagacggg ggccatgagg gaggtggggg cagggaaggg 22740
   ggagttcctg tctgttcttt tctgcccttc cctgtgctct ccacctctat cccctcttcc 22800
   ttcccaaaca ggtggcagga atgttaagac tatgaattcc atcaagagca agcagggttc 22860
   cttagggcac cagttggtaa gtgtaagagg cctcatccgc ttgcaccagc agcagaaaga 22920
   agctgcagga agagtctctt ttgggcatga gaagctaaag gaggctggtg gttttctggt 22980
   taactgcaca aggcctgcaa gttctacaga tgtcctttga ggaccagagc agcctccttt 23040
   cctcccacac tctccagaca tgggaggcag taggagttca aagcacttct cagaaattga 23100
   gcctttggtt ctgctgtcag caaaaggctg tgaagctcag tccccgacac tgggtaggag 23160
   aagggtgaat gaagactagg gggtctggtg aaggaagtgg ggaaacaaac tcttcatttt 23220
   taaagagagt gacctggcaa aacacagaga agatactttg ggtttggagg aacgtgcaag 23280
   aggtcgagag tcactgccga ggggttttga tgcggcactg tgctccggca atgggaaggg 23340
   ctgtggtcag gaaacaaagc agcaggtgtg gcccatgtgg aattgtcagg caggatttac 23400
   ctgtggtggc ccgtgccgca cagcctggcc caccttctgt ccacctctgc tttccttctt 23460
   aggacctcct agaaagaaag ctggaccttc cgaaaagtag cttgggaggc aaataacatt 23520
   tatcgaatgc atcaatgtct taggcctcaa gcaagggttt aacatgagaa tctctttgag 23580
   ttcccctagc atcctatgag gtagacacta ttaatatccc ccaaggctca gagaagttaa 23640
   gtcacttacc aggaaacaca actattaaat caggcctgcg tttgatccca gggatgttaa 23700
   atttgaggcc tgagctcttt ccatcatact gcgaagtctc aattgtggca tcactgcaaa 23760
   agaaaaacca actgaacgtt gctgtcacat gcagagagag agacaaagaa gagaaagggg 23820
   gatgcagcta aatgctgggc tttttgctct tgcaggacca tgccacagag tccaccgtct 23880
   ggggagcagg gcctgtgagc tacagattct cgaacaatac aatcatgggt caatgcctgg 23940
   gcagagaata ggttctcttt gaagttcatt atctccagaa agcttttcca gcttccagtc 24000
   actgcttcac tccagttttc tttcttcttt tttcatcttg gcttatttta gttggcttct 24060
   atgcccatga ctatcaccct caaccaaaga cagagaggct ccaatgcagg aggcagacag 24120
   atttgggctt gaacccaatg ccgcttcctt gctgtgtgag ctttgtcaag ttattctatt 24180
   gcctaatttt tggggattgt attatgttat gttacatgtt atgttttgtt acatgatatt 24240
   gtgttatatt actatcttct tcatggtgtt attatattat taagattgat gagataatgt 24300
   gtgtcagatt tctagcaggt ttcttggcat attgcaacca ccaatacact gaacatatac 24360
   tgtctatgta actttctcag actcagagat ttttgtctct tttatctccc acaattcttt 24420
   tcttttcaaa atgtcacttc cagaattttt tcccagaaga atccagtcca ttaaataaga 24480
   atgttttttc actaaaattt tctttctgca tactcttagg attgtatgat gttcgtctgt 24540
   ctcccccacc tacaacctcc agcagagaga agagggctga gccagtagtg acaagggtgg 24600
   gactttcaca tctcttttat tccccctgta gctttttgct aagggctaac tgatttcact 24660
   taaaaaaaaa ttttttttta agaggccttc catcctggtc agacttcacc tttttgcacc 24720
   ttagtattct cggctagaaa atggaaataa taagtcctcc cccactcact ttacagggtc 24780
   atcataaaaa ctcgataaga tatattgttt aaaatattta aaaaccttgg aatgcttcat 24840
   gaatataagg cattgttatt caaatacaga gcttcttaag agctttactt tttagctctg 24900
   tgatcagtta acttttgatg tggagcaggc aaccacgaat tctcagaggc gtgcaacaat 24960
   aaacatttat ttctcacaca tctgtggagc tgagctgatg taggctgggc ttgcttctcc 25020
   atctgggggt ggtgtcgttt ggctgaccgt gttgggcctg gctgggttag tatggctcca 25080
   catgtttctc atcctcctcc caggattagt gggcagccca agcatgtctt tcttcaaaat 25140
   gatgctggat acataagaga gcaagcaaaa acatacatgg cttcttgaag cccaggcttg 25200
   gaattggtat gccattgctt ttactggcca agcaagtcac atgggtggca tcagggatgg 25260
   gcttatgcat catctactgt gggaggagtt ttctaagtat gatgagggca cagatacagg 25320
   gagcggtgac cattacgaac attaatgcga atcctccaca ctgtgtttct tgatctgcca 25380
   ggacagggtt ctctgaaggc cagtagaggt tcagtgcttc caaaaaagga tgctgggctc 25440
   aggtaggtag acagagttca tcacctctat catgactgat tggctctaag gcccaatgac 25500
   aaaactcttt acttctgtaa agtgttagac agtgaagaag atgaaaaggc catcatgaaa 25560
   ttcagtctct gcttttagtt tttgtttgaa attcggtctc attgttgtgt ttgttttgtt 25620
   ttgttttgtt tttccatatt ttctcccaag ggttattact ttcacaacag tggaaaaata 25680
   ttccttatgt tctctcccct ctccttccca ggattccttt agattcctgt gtgtgcctga 25740
   gggagctaga agagaaatac ataaaacata taactgtggg actcttgtct gagcccatct 25800
   ccattcttaa gtcccattca cccccacaaa agctttcctt agagccaatt atgttcctta 25860
   ggctaccata tccccataaa cagacgtgag ccactgtggg ttcctagtaa tgagattttg 25920
   tttggcagtt ttatttgcaa atgactccaa agatctgagt aaaacatctt agagcaagaa 25980
   attctgggtt agaaattttc aaaccagaac acttcttcaa aggaagatga ggttgagaga 26040
   gggggtggcg gtgaggcagg gattgatttt aatgggccag tatccagggc tccagatgtt 26100
   tcttcaaaag gaatctccca aattattgag cccaagttac agaataagcg agaaagaaaa 26160
   tggtctacag aatctttgat tgggtccgca tgaattcatt aaaagtggtt gtcagggaga 26220
   tgtgaggggt ggggaggctt gcacatatgt atattaacac acacacacac acacacacac 26280
   gcacacgcac gcacacgtac acaaagagct tggtgtactg gtcttgtcaa atgctttcct 26340
   ccctctgtcc ctccccacga aattaaatcc aaggtctgga gttttgccct ttaggttaaa 26400
   aacaaaaaaa acaacaaaaa aaacaaactg ctgcttttcc aaaaagcatt aaagagagaa 26460
   aaaatataac agcagctact gacttgaatt gaacttaaaa tttcccagga agaatctgct 26520
   cagagccgaa ctcagaaact tgttgcaagc tccccagaca tgagaaaata aacttctatt 26580
   aagctggcac tgaagtgcct ggaagggtgt tcaaccctta gaacaactct tagctttccg 26640
   aggagtttca gggttggaag aacaactttg ccaattctac cctcaacttg gaacaaagca 26700
   ttatacatct ggaggagtct ggtggtttaa tgctttcttt tccatccaag attattcatt 26760
   cattcaataa atatttagtg agaacatact acctactggg cacggtgcta ggtgttgaga 26820
   tgtggtgatc agggtgaaca aaacagacat agtcattgtc ctcctggaat ttgcacttta 26880
   ttggaggtag gggagcacaa ttaactaatt acgtttactt gagataagtg tagttaaaga 26940
   aacaaacaag atccagagac agaaagtagc aaggacttac aggatgatca gggaagtctt 27000
   ttccatgaag gtaacattta aactacataa tgctgatatt ttgaatctga acctgaggat 27060
   ctcagaattt tccacttctc cctctgaaac agaggaggtt ttggtccaca ggagaggcta 27120
   agggttgatt cagtcaaagt ctttggagtt cagattagcc ctcgcagtta gctagaagtg 27180
   gggatggaaa cttaatacac ttgaattcac acacctccgg aagtatttat ggctacttct 27240
   ggttctgacg gcaaggtcag gtgaatcaac ttggtcttat gaatatagtt gttttccata 27300
   tcccttgttg aaaatagaag caggtttgaa tcaacgtaaa gaggaagttg ttaaacacca 27360
   tgagaaactt ccaaatcagg tgtgtcatgg actctttctc tacctgagat aaagatgagt 27420
   ctgaataaaa taactgagaa gtggacttag gtgaaaatga gaagatagta aagatgattt 27480
   gtcacaaatt gtagggaaca aaatccaaca ccacactaag aaaataatat actatgacaa 27540
   ggtgggacct attacaagaa tgccaatttt gttcagtttt agaaaataca ttagaataat 27600
   atacattagt ataactcagg aaaaatattg tgattatctc cagagatggt gaaaaagcct 27660
   ttgagcaaac tgaacaccta ctcctaacaa aagcactcaa gaatatacaa attaatggct 27720
   gtttctttga caagataaga aatatatagt ttggtcttaa caccagtatc tgtgctaatg 27780
   agaaaatatt aggggcatat ttattaagat cagaaataaa taatatcctc tctccccttt 27840
   atttgacatt gtactggaga tattagccaa tgcaattaaa caaggtaaat taaaggtata 27900
   aaaattagaa acaaataagt taaactgtca gtatttgcag atgatatgat agtatacatg 27960
   gaaaattcaa tcaattataa aatgaactca aacaataaaa tattttgcta aatttagaaa 28020
   agataagaga atttaaaatt aatactcaga aatcaatagt tcttatatac aaaaccgaac 28080
   accagttaga ggatatgata gggaaaacct catttaaaat ccaaagggga gaactttaaa 28140
   gcttccttaa tgaaacaaaa gtagacctga acaaacagaa agacaacttt ttgctttttt 28200
   tttttttttt gcttaggatg ttttaatatc ataaggttgc cattgttctc ttagctaact 28260
   tataaattga ctacaatcct aataatcaca ttaaaatttt ttatagtgaa attgatactg 28320
   aagttatatg gaaaaaataa gaataactag agaaatatta agaaattaaa agagagctac 28380
   aatgaaagat taactctgcc taaatttaaa aggcattcta aagtttctgt gattgaacta 28440
   gtaagaacct tgtgcgtaag tggaaagacc aactaatgaa ataaaatatg atctgaaaga 28500
   gacccatatg catgtgaaaa tttagtatat aataaaaatg caagttctaa tcacttgata 28560
   gtcattcctt taaagagata caatgaaatc cataccttaa accatataaa agaataaacc 28620
   tcaaatgtat gaaagatagt atatatagaa aataaaacct tataagtcca caattctagt 28680
   tagaagaaaa tatcattaat ttctgacttg atctgacctg ggtctaacta taatttggaa 28740
   tcctgtaata ataaatgata atagtaatca aaatgatgat aaagaataaa atttgactag 28800
   gtaaagttta gaaacaacaa atttgaatgt tcaaaaatat tatcagcagt aaagtcaaaa 28860
   gccaagtgac gaactggtag aaaatgtttc tatatatatc agagataaaa agttaatgtc 28920
   cccaaagtat aaagaatttt tgaaattgag aataagcgga aaaccaaaaa ccaatataaa 28980
   tgggcaaaag atacgttcat ataatttaag tggtccctaa atatataaaa atatcttcaa 29040
   ctttcaaata tccttaaaca tataaacata cattcaaata tgtttcttgt aataagagga 29100
   atgaaaatta aatataatga gataccattt ttcacctatc agaatggtaa aacaattttt 29160
   tttaaactct taacacactg ttgacaaagc tgtgagaaga aatactctca gtgttaggag 29220
   gaatgaaaaa taatgcaact ctttggagag gaagttgcaa tctctaacac aaatacatat 29280
   gtgtttctct cttgaggtgg caatccctat tctagaaatt tactctgaag ctatacctct 29340
   aacaatatga atatgaaaat acatgtacac aaagttattc actgcagcaa tgtttataat 29400
   tgcaaaatat tagaacaaac ctaaaatttc catatgtact agactgaatg tgcaatgata 29460
   catccactca atggagtagt atggagttgt gaaacaaatg aggaagatct ttatgcttga 29520
   cacagagtga ttttcaggat attttattga gtggaaaaag caaagagcaa aagagtattt 29580
   ctgcaatgta tactatacta cattttgtgt aaaaaagaat atatacaatg tatgctatgc 29640
   tacgttttgt agaagaggaa gacagatgaa aatagacata tttacaatat ctgtctaatt 29700
   ttgctggcag aaacacagga agaataaacc agaaatggat acaatttgtg atctatagag 29760
   gatgtgggga aatgggatgg aaaggacgag tggtgggcgt aggggacggc acttctctga 29820
   atataccttt tgtatcattt ttagttttgg aaccatgctg acattttacc taccaagaaa 29880
   aagcactaac tatatcataa atgaagaaca ttaccacact gaactaggga aaaaatttaa 29940
   aaactaatac aagtaacatt tgaactcaat tctttgacta tagaccatca gactaaatac 30000
   aaaaggaaac ataaacatta aaatgtagtt aacagttttt gtctaacaca tagtgttagc 30060
   aattcagaaa ccactttatg tgaaacgtag aaatgagtga gtgagcaaat gcataactat 30120
   attaaggata aaggagagta ggtttctgac tgttgaagac aattaaaaat atgtacagag 30180
   ggccgggcac agtgactcat gcctgtaatc cctgcacttt gggaggctga ggcgggtgga 30240
   tctcttgagg tcaggagttc gagaccagcc ttgccaccat ggtgaaactc catctctact 30300
   aacagtataa aaatttagcc ggccgtgtgg cacgtgcctg taatcccagc tattcaggag 30360
   gctgaggaag gagaatcact tgaacccagg aagcggaggt tacagtgagc tgaggattgt 30420
   gccattgcac ttcagcctgg gcaacagagt gagacttagt ctcaaaaaaa taaataaata 30480
   aaataataac gtacagagga aaatctcgaa tgtaccaata gtaataatct tgggttaaaa 30540
   ttgttggcat cagcaaaaac tcatggtttt attaaatata ttaacattta ataaataata 30600
   tgtattaata tataatttca tatatacgta catctatatg tatcttatat gtatttcttt 30660
   tttatatata acatctgtgt gtgtgtgtgt gtgtgtgtgt gtgtgtgtgt gtacacccct 30720
   tcagtcctat gaatagctgt gttcactgaa ggaagcaatg gcacttcaat aaaaacgagc 30780
   acatctagtc ccaagacctt gtttctaagt accattcctc aacaaaaaga atcctcaaca 30840
   aaaagacatt tcctgaagaa atgtctgatg ccataggctg gttggagcaa agaaggccta 30900
   acatgatcgt gaattacctt attgtaccag aaaaaaaaaa aaactatgaa aaaatgatgg 30960
   cgatatatca aaaagactca ggatccagtg tgagcagctc ttattgggca agtcttggaa 31020
   atttgaacaa caaaatcaac aacggttagt tacacattaa catgtttgac aaaataagaa 31080
   tccatgagtc cagactgata taaattaatg agtgaataaa tgaataggtg attgagaaga 31140
   aaggaaagct tctcttcaca atagaatgcc aattaacaaa tacaaaagga acaatggagt 31200
   tagaaaagag ttagaacact accattttgc agcaattaca ataataatta gtttaggtaa 31260
   gaatcatgaa tgggtgatac atttacggag actgtttgat gaggaactgg atattacata 31320
   gcctcacagt gtttctcata ggctatttct aattctagag gaaaaatagt aacttcacag 31380
   taagaagcat ggtggacacc accttaacca aatgattcaa gttatcaata acaacgggac 31440
   aatctgacat gtgtttccca atatacagat gaaaaagagc atagcatcac aattttacaa 31500
   cataaatcta agcaaaagga aagaaaagac aaacccaatt gaaggagagc cacattaagt 31560
   aattggcttg aactcttcaa aatgcgaagg ttatagaagt caaaggaaag actgggttaa 31620
   tattcttcta acactgtaag tctcagccta aatccaaagg cctgagaacc aggagtgcag 31680
   acatccaaga gcaggagaag agagatggcc tggctcaagc agagagtcaa tttgcccttc 31740
   cttcatcttt ttgttctatt caggccctgg agggattaaa tgatacccag ccttcactgg 31800
   ggaaggtaat cttcattact cagtctgctg attcaaatgt taatctcttg cagaaacacc 31860
   ctcacagtca cacccagaaa taatgtttta acagctacct ggtcatccct tacctcagtc 31920
   aagtcgatac ataattaacc atcatagagc cagaacatgc aataacttgg aaataattca 31980
   gtcattgtgc aaggagttac ctagactctg ttttaggctg gaaggtggga gtgagatata 32040
   aaatgcatac agtgatcagg aggctacgag tactagaaag aactagaaag ctttaactct 32100
   ttcatggcaa taataaaatt caagattgtt actaggaata aactgtacca gagttatgtg 32160
   agacccaaac acagtcagaa ttagactttg gaggtatttg ttgaattgaa ctgaacccta 32220
   tagctcccca ttgttcatag agtgcctaca acctgtggcc actgcactag gcattcagga 32280
   ttcagaatgt tgagaggact atgagaagta acgtggctaa gcatggaacc agagagagtt 32340
   ggcccaaccc tttcatttac aaatgaggaa actgatgccc acagtgagac tggcctggcc 32400
   caacattaca caaccagttt atggcaaagc aaagagaaac acctgtttgg gtagtgaggg 32460
   ttgcctttta ggtgccctga ccccattttt accaccaagg attgaggagg aattgcagta 32520
   caaagagaca agagatagaa aaaatttgag cgttgctgct gctatagacc ctccatccag 32580
   caaagccttg agagagatat tctaccaccg ggctgggact gtaaactagt tcaaccattg 32640
   tggaagacag tgtggcgatt cctcaaggat ctagaactag aaataccgtt tgacccagcc 32700
   atcccattac tgggtatata cccaagggat tataaatcat gctgctataa agacacatgc 32760
   acacgtatgt ttattgtggc actattcata atagtagact tggaaccaat ccaaatgtcc 32820
   atcagtgata gactggatta agaaaatgtg gcacatatac accatggaat accatgcagc 32880
   cataaaaaag gatgagttca tgtcctttgt agggacatgg atgaagctgg aaaccatcat 32940
   tctgagcaaa ctattgcaag gacagaaaac caaagaccac atgttcccac tcataggtgg 33000
   gaattgaaca ttgagaacac ctggacacag ggtgggaaac atcacacact ggggtctgtc 33060
   gtggggtggg ggaaggagtg agggatagca ttaggagata cacctaatgt aaatgacgag 33120
   ttaatgggtg cagcacacca acatggcaca tgtgtacata tgtaacaaac ctgcacatgt 33180
   accctagaac ttaaagtata ataataaaaa taatatatat tctaccaccg ggctgctaac 33240
   ttggcaaggg ctgaaagaat ccaaccgaga agaaaatagg ccatcattgt gcccctctcc 33300
   acatcaaaat gcaattactg agcttttctt aagagctcag cactgtgcta gatgttgtcc 33360
   atgttgatct tcacaactgc agaggctgaa ttactatatg tttatgtgat aactaaatat 33420
   tctattatgc ttgcatttgt ctttggtgaa tattattgaa gtcgtttact gctacggttt 33480
   cctctgcagt ttttctacta gtccatcaca ctcttctttt tggccctatt tttacccata 33540
   ctcagttagt ttcttgtggc aggctacgtg tgtgtgaggt tctgtgtgta tgtgtgtttg 33600
   cgcaaacatg gttttgtgtc agtatacctc atagatatga aaacagtttc tgttcaaaca 33660
   gtacccacac ttagactcca tattaatctt ggctcaattc tgtgaaaact ggccctgaag 33720
   tctgtgaccc cccttactca cctctttacc tcatctcaac caaaagaatc cacttcaaga 33780
   acaccttccg cacaagctct tcaaatatct caagttctct tttccagtat ggtgttgcag 33840
   ggtgtagtga aaaatgcatg agtttggagt caaacagatt cccgttccat ttctggttgt 33900
   atcctatatt accacttgga gttgttggca gtcttaggtg aacacaggag gcaattcttt 33960
   ttttacagtg actgacaaag aggtgagcaa gaaatgtgag tctcttattt attcccagcc 34020
   cctctatctt aacttgttga aggtaaagac cagtggatct gaatttagat tatttaccct 34080
   cccatgtgtg gggaacacac acacgtgcac acacacacca ggctggaatt tcttcagttt 34140
   gttcctccgg gtctactcta tacttcatgg aagctgatct tatgacttac atcaatggtc 34200
   tcccttgtcc tctggcttct attagatttg gcaaatgggg agccctggca gatcactgaa 34260
   gggaggacag aagttgtagt atttattcct gtgatattct ctcctggtgt gtcaccttgg 34320
   gctgtctgcg tcccttgaca gaaggtcacc ctcttgtcag gcatcacttt tctcacagtt 34380
   ctctctgtct ctgggttcca acaccactcc ctcttcttga cccttaacgc ctaggggtga 34440
   ttatagctcc agctgttatt agcacggagg actgcacgat cccttatgat ttcactacac 34500
   ccagtaaata gaacctttat taagcttttc ccaaataacc caatttcagt gcactgtctg 34560
   ttttctgctt ggactttcac tcacgtatgt gcatgcacac acacacacac atacacacac 34620
   gcatgcacac tcatacccac gtactctcta tatacgtgta cattgagtgc tatggaattt 34680
   ttagggttct ttgaggacaa cttcctggaa gaagaataat gggagatgtg ctcagaggct 34740
   ggtgagcgtt taattgacct ggttgtgagc acaatgggaa tgcttttctg aaccttccat 34800
   ttgcctccaa tttagaataa ctgcttagtc agcaacatgt ctccaattaa catgacagat 34860
   tttttggacc acagatctct ttgggaatct tatgtaagct gtgaaacttc tcccttaaaa 34920
   aacctacata tttaattctg tctgtaattt taggacttca catatcccca aagaccattc 34980
   atgatccacc agggttaaag acttgtttta agtaaagaaa atattcacag cctctggagt 35040
   agtagacatc tccagagaaa gtctggcatt ctttggtaac tcatggcatt tattacatta 35100
   gctttggatg gaacaacccc caacccccaa gtctgagcat gtggtttgat gctgcttcct 35160
   ttccattttc actctgctct cttggattct atgtagcaaa aaggtacctt ttctagggtc 35220
   acatgtccac ttctcaaact ctgtcttgca ctggggattt gagggtggac tatcagagca 35280
   taatggaaga ggcaggaaag gatagcacta attagagaac ttgataggcc ttttgctact 35340
   gataaagatt ctgtcctgta tcatccatga ggtttggatt tgcacttcta aaaaatagct 35400
   gtgtgttttt gacaatagcc actccctttt tgctcctttc tggtctcttt cctaaatgac 35460
   tttccaggat agtataaaat aagcaatcta tttagaggaa caaaacaaag aactacacca 35520
   ttagcattaa aatatttcta gggtagttta ggtaacatag aactttgtag ctcagcagtc 35580
   ttggaatcac tagactctaa gccccatgta gtcagagact gcctgctttt ataattcatc 35640
   cccaacagca ggctattgca taacacattg ttaccaccca caccattttg ttgagtaaat 35700
   gaatgcatgc atgaacgaat cagaggattc tgttgtctaa ataaactttg gaggtgactt 35760
   agtcttctct tccctctgat gcttagatct tctttatctc tcagttcaac atatattgct 35820
   caaatggttc ctaggtgcaa gggatctgga agttattaaa atgaaataga ttttggacca 35880
   tgaccttgac aaattatgat ctactaggag agataatatg gaaatcagta taatactaca 35940
   gagacagtaa aaaggaaaaa gaaatgtttg cacttaataa ttattccacc attgttccaa 36000
   aagtttcaat tatagggaac taagttccac tactaataaa agtcaatacg cattgtatgc 36060
   ttactgcatg ctaaatatat tatgtcatta taatatttat gttcaggtgt atactatgat 36120
   tgtatgtatt ttacgacaag gaaattgagg ctcagagagg ttaaagaact tgctcaaggt 36180
   gacagagcca gtaggtggta gaacaaagac tcctacattc tgacctagtc catatgtaag 36240
   cagatttatt gctagaaata tcttgatgtt gcattgaaat ctgcctctcc ttattttcca 36300
   tttggaacta attccactct cccgagccag aaaaaccaag cctctcatta cttgaaaaac 36360
   caattaatga gttaaattac atcaagttag tttaacagtc agcaattccc tgttgtaatc 36420
   atagttaggc tgctaccatt tttgtcccaa tcattggccc acactgaatt ttgtgtctaa 36480
   ttcattgctt tctgtctcct taatttcatc ctctgccccc agagatacta tgctgagtat 36540
   tgatgctgca gcaacatatt aatttttatg ttttctgact catttacccc aaagccatag 36600
   tgcatcaaat gcttttgggt ttgtgaccat tttatctttg gtttcagtca ctgtttatct 36660
   ggtcgcatct ttgttcattt aacaaatatt tgctatgaag tgcaagttta gtgggtagca 36720
   aacactgctc tggcaagggg tatatgaaga aagacatcat tccttcctcc aagactagtc 36780
   ttgtttggaa gctggacaaa caaacagtta caatacaatt aaaatagggt taattaagaa 36840
   atgtgccaac tgtcaagggt aatcaaaggg gaccctttca gtctgtccca aggatgctag 36900
   gtaagtcttc acagagaaac tggtattagg gctaagatct agcggaggac taggggcttt 36960
   ctagacagac aatggaggca ggacacttct gagagaggaa tgaagccttg aggtgtgaga 37020
   cagcatgtgg tagagcatgg agggttcagg cacttggagg agttgggtat ggggagaaat 37080
   acagagcacc tgtgagagcc tggggcatgg ctggagaggt aggcaggggc cggcttgcaa 37140
   agggctttac ctgtcatgct gaagggctaa ctgctgttct ggagaatttg cagagccact 37200
   gaaggttttg aagcatccta atgttaggat tcacttcaag ttttagaaag tttagtcttt 37260
   tggctcaagg gtggggaggg aataaaagaa gcgagagtga aggcatggga ccagtttaga 37320
   gactagtgaa gtctgatgaa aaggagatga gagaagccta aagtggacaa gcagctggac 37380
   acggcggctc acacctgtaa tcccagcaat ttgggaggac gaggtgggcg gatcacctgt 37440
   agtcgggagt tcaaggccag cctggccaac atggtgaaac cccgtctcta ctaaaaatac 37500
   aaaattagct gggtgtggtg gcagacacgt gtaatcccac ctactcagga ggctgaagcg 37560
   ggagaatcgc ttgaacctgg gaggcggagg ttgcagtgag ctgagttggc accactgcac 37620
   cccagcctgg gcaaaaagag tgaaactctg tctcataata aataaataga gtggacaagg 37680
   aaatactgat gggggtgaga aaacaatttg agagacattt gagaagtagt gagataacat 37740
   ttggttactg attgaatatg gagtgttagg ataaccctgg gatcctcatt ttgggcaatc 37800
   acaaagctac tggctacagc aaaaggaggg gaagaaaagt ggtggtggga gggaggcatg 37860
   agaaatttgc tgctggccat gttaagctcc acctcctgtc accaagtggt gatgctcagg 37920
   agatgtttgt gtccacagga ggagagtgat ctgggtggga ggtttacatg tccctccttc 37980
   catctcccgg tccttctttc cctcctccct ctttctttgt ttggtacttc ctttcttctc 38040
   tcttttcttt ttctgccttc ttttgagaat gaaacctaag agtgtttata tgtagaggga 38100
   aaaagtggag ctagaagttt aagacacagg aaagggagga aaaatgtcct agtgtgagat 38160
   tccaaggagg aagaagggcc aggataaggt ggggagaggg atgcctcttt tgctcattgc 38220
   ctttatctgt ctttgtctgt cctggattga ttgtcctatc cttttcttcc acttcgtgtc 38280
   ttctcccgta tttctacaca aggcacagtc aaataagggc tggcacgtgc gcatctttgt 38340
   gcacacatgt gtgtgcacaa gcattgaggg atgcacaggt gcacacacct tgtagagcct 38400
   ctagtagctc aagtcacatt ttttcgcatt tccagattgt aagccttact gaggggcaga 38460
   tgggacgttc tcacaggaaa aaggctgacc tcagggagaa agatgtggcc cccacctaca 38520
   aaccaatgat gtctactccc aacaggggac gtctttcccc tgcgtgaagc aacaggacag 38580
   atttggttag gtgtggcatc gatgtcctaa tctcctatga gcacacaatg aatgaaggtg 38640
   gggggctctg ttaactggag ggaatcaggg gctgccctgc aaagcatgtg caaagaaaat 38700
   aaatgcaatt ggagtgttgt ctacacatcg tcctcagtgg gcacaaaaga ggcacagtgg 38760
   gatggggctc tgacccagtt tccatttggc cagccgactg gaaacagcta ctcactgggc 38820
   cactgggttc tttccaaaga acagtgaaga acagtggttt ggggtaggtg ggggcagggg 38880
   ctggtggggc aagaatgtca gaagcaaatg caggcgtggc tggtccagta gttccagtac 38940
   caaagaagcc gctatctgga agagtgggac aacccatcct aaacatggga agaagaaaca 39000
   ggaaatttca aaagatacaa ctactaatta aatgaaagca ctccttaacc acccctttga 39060
   taattagatc atcttctcct agaatgagaa ggaatattag tctgactttg ccctccatat 39120
   ggagaaacat ggcaggcagg ctctctgtgc ctggagggtc tggccagact ttgtctggca 39180
   tgggtatttt gactccagcc tattttttag ggatcctcct gcagctggtc ctcaagaggt 39240
   ctgaactttg ccgtctcatt tctaaagcct ctttcagcag gtactggggt tcccaggtcc 39300
   agggaagcct ggtcattgct agaacacatg gcaacatcct tcaggaaact cagctcagac 39360
   tgtctggaaa gatgctggaa aggctgaatc catgcaagta ttatctaacc agccccaagt 39420
   catgttttca cacttagatt tctacaggat attcataaat tctggaaaca aagatattgc 39480
   cactttattt tatttttaca cattgagaag tttccttggc aacataacat gtatttgcct 39540
   atgtttccaa ggtttatgga tgaatgtggg tggtcaatgg gattcattca ttttgccaag 39600
   caaacgatat gtttgaagcc atattaagtg ccaggtacca tagaggatac agatgttcat 39660
   aatgtaaact gccctctagc agcttacaga tactacctag gaataatgct tacactgcag 39720
   taaacaacat aaggcagaat atatatatat gtctttgtac tcataacaaa atgtaaaagt 39780
   caaagtcagg gctcttttaa ctttcttagt agcagaaacc caaattgaac ttaagcagaa 39840
   ggggaattat ttatttaaaa cacaaagaaa ttatttattg aaagcaaaca ggaaattgat 39900
   tattgcttct tacaactcat gagctaaaag acagagctgg gttcagatac agatatatcc 39960
   agagttgtga tgctatcagg acgctgtctg gtaatgaaac caaccacagc acagagcctg 40020
   gagtggaggg acagaccaac tctcttcaga tgccacatcc tcacccagca gacctcctcc 40080
   tctccagcac ccattcatca aaccttcgag aagaccctgg ttgctactgc aaggctttca 40140
   gcaccaaatg ggaaccaact cctcctcctt ggacatggaa tacttgaact ggccattctg 40200
   ggacaggggc ctgcccatgt gaatggaggg aggaagaagt gaaatcctta aagggttgct 40260
   gggtcagcat atcacaaatt tcccttgccg tgagccagat ctgatgggat ggtattagtc 40320
   aataggtaga taataggggg aagagggggt gaagaatgag atggttctac aaaaagaaag 40380
   gcagggtgcc agaaggtaga atttgggcaa tagtgagtca cagaattgat tgtgtcttgg 40440
   agcccatgga agggagctga ggcacctacg gttggagggg gaggtgggga cccttgggca 40500
   cagctaaaga gtctgctttt gggtaggatg tacagagcaa ttagaattat ttagcagtga 40560
   cttaaagcca agagaacaaa tctggcttca gagtgttgac atggaagaaa ggaagaggca 40620
   aatggaagag gctattccaa agggagaagg tctgagcttg gtggtgcctg ggaaggaagg 40680
   acactgcagc caagctgagc ctgtgtccag ctgaacacgg acatttactt ctgcttctga 40740
   ggggaaatat tgtcatgtct tatgggtgta cccagcaaaa gatgtttcca ctaaaagact 40800
   atagagaatt gcttatttct aaggactgaa ttccgaatgt ttttggtcag gttctctctg 40860
   aacagagaac cattgcccct gagcagaagc ggtcttcaca caactagcaa ccatgtagtg 40920
   ttttctcatt caaatggcgt ttggtattca cctgcctgtc ctggaacaaa tgcaaagtca 40980
   gggttgtttc tagctctccc ccaatattgg aaaaatgctg atgaactgag cctggttcca 41040
   ttttacacct gacaaaagga actcagatga gagtgttggt aactgggttt aaggaggagt 41100
   cagaggtgac tgctgaaatt tctgccagac tacaactaaa ttaaaaaagg aattcggttt 41160
   aagaagccaa tcttcaacta atttgaacat gaggatgctc aacttagaag cctcccatgg 41220
   ggccctggag cccagaaggg actgcatact ctttgctcct gcaggtaact cagggctttg 41280
   ttctggtgtc tctgacaggc ttctaacctt ctgcaattct gttgccacaa tttctgtggc 41340
   accaggagaa aattcatgca tctaggctag ggtggtgctc tcatgtgtct ccaggagagt 41400
   gtggcctggg ttagcttaag actaggaatg tgctgacagc tgggaagaaa gtcaaaagaa 41460
   ggcatggcat gtggttatgg ggccggaggg accgcttggg gggagaggtg gaagcgctga 41520
   catgagcttg ctctgcatcg ctggttccca gaggtggcct gataggagag tgccagctgg 41580
   tggtatgccg tgggctctgg ggaaggggct agaatgttgt tgcagcccag aggggctggg 41640
   ctagtgagga gcaaaagaaa acataagctg gggggaaact gcctaataga ccaattagga 41700
   gacagggttg tgccctaagg gatgggtggg agcagggggt gctagaagta gacagagagt 41760
   aactctcagc tctgaacagc gtcccacata ggaggaagaa cactgatgtg aggccaagtg 41820
   gcttggaagg gcgttgacac cccattccct acaacaaagg gtttggcaaa ctcatctcat 41880
   ttgctacttg tcctataggt aaggatggac accaaaccca aagagacttt tgggatgtgg 41940
   ataaaaattg aaaactttgc ctctcattaa tcccttggag aatatagcat ctccttcaaa 42000
   ctggactgct ctccttgatc ccttgtcttt acctgaaaat atctttcaag gcctagctca 42060
   tgctatcttg gccataggaa ctttccttat cacctccatc aatgctgttt gtccctccac 42120
   tagaatcttc tgttttggta ctcctcttac tcagcctcaa gttaaattta tgtgtactgg 42180
   ttttaacccc agtacccagt aaaggttctg aatacacgtg atgaaataag tctctcctat 42240
   cccatggcac ctagtatagc atttagttgc tatactaatt attataatta gcatttatta 42300
   cgggcttact atgtgcaaag tgccattatt ccatttatta ttcacaaaaa tatttagtct 42360
   ctactattct ctaatttatt agataaccta atataaatct tttttgtttt gattgttgct 42420
   agctgattga aggaaataga attgtggtgt ggtagtaggg gatgttgagt gagtcttcct 42480
   cattcttaat atttccttcc actaggataa cgcccctggg ctctcttcat caggggagga 42540
   ggagtgactt catctgatag attcggaagg gaaaatgtta cccagtcgct tctcagtcct 42600
   ctttctcttg gagcgagccc atctctggag cacatacact ctcccctgct cctgtaggtc 42660
   tcgagttctc aggggaaggg tttgccctgt aagagagctt gtccttctcc agtgaggcct 42720
   gtcactggga agataattct gtacagtgct gggattcaaa tttagaaagc ccacttgccc 42780
   cataattaag ccacattcac cagctttaac aatgacagag gtgatattga gttctccagt 42840
   gatattgagt tctccatctg ccttatgctt ttctgcttat ttccccattg gttcagaatt 42900
   ctggtgggaa aaggggtgct gtttgtggga tgccttccca ccctcatgtg gccctccata 42960
   agttcttcat ccctcattgc attcaataac ttgggtgtgg tgtgggtgga gtgcatgtgc 43020
   catatgaccc tctcccttga gtctttgaaa cctatttctt actttatgac tttggacttc 43080
   cttttctttg ggagtataaa agtggatacc accattccac cccttacccg tcatcaggag 43140
   aagttagttg ggaagcaaag gcggagtgac gtggagggaa gagtctaaaa acagctctac 43200
   acttagcttt gtccttgcat tgatgacaag cgatgttgga tcctgaaagg tggggatggg 43260
   aggtaggaag acccaggaag ttgctgtgaa ggctaaatcc taaagacagt cactaaaagt 43320
   ttccagaagg ggcatctatc actccacagg caagcaagat gagaccatgt tgaaaaggtc 43380
   ttaaagaagc cagggaagag gtgaaacatc tctctgtgtg ctgaggacca taccaggtcc 43440
   ttcattatca tattctacac attgaaaatg aaggtaagag agcaagcaac ttgccctagg 43500
   ctacccaggg aatagggagg aaagtctgga tttgaaccta ctgctgcctg actccgaagc 43560
   cctgttcttt ctctcaccct ggaggatgga tgagggtggg atggggcagt taaaagttga 43620
   ggggtatctc taggagaaag atgagacaca tgaccattgg gtctaaggag ccagagagag 43680
   gccattgtgt ctgagcctgg acagccaagc ttcctagaga cagaggatgg gatgagatga 43740
   cttctggctg actctgcctc cttgagctct ttaggatccc ctgcagaatc tcccaaaatg 43800
   atgctcattg gaaacagaga gagcacattc aagtaagtat ttgtgccaca ctatgattgt 43860
   tccaatgacg tcaagtctgg gatggcaaca gcagcggttc catttctttt cttcaggacc 43920
   tggattcttg ctccaaggtc ttccacctcc ctcctcattt tcttccactc ccactgcagg 43980
   ctccttcctt ggaggagctg gggcagggag taggggtgga ggttgtgtgt gcacttgtgc 44040
   acatgcatgc ctgtctctta tgtgttgctg agtcaggaat ggcaggaagg gagaggcttg 44100
   gacagcaagg cagccctggg caatctggtg atgaacttga aacgggagag ggtctattgt 44160
   gcatttgcac tgactctgtg caggaagggt gggggcaggc caaggaggac tggcttggct 44220
   gagacatgag atgtctcagg ggagggtttg gctgaggcag tggaagaatg cttgtcggga 44280
   accggcctgc ctggaattgt aacctcccct gctgtctctg caccacactc agagtacagc 44340
   tgtatgcctc ttagctggta gattcctttt ctagctgggg acaccgtgga cagggacagt 44400
   ggggaagagc attggactca gagacaactt gacatcaagt cctgcttgga atcctatttg 44460
   ggtttgtaat tcgttgaagg cagacacaga cataaactaa tccttagttt tcccatccct 44520
   tcaataagga gtacaacacc tatcacacaa ggctacaaga ataaaagaga gaacttgtac 44580
   aaaggcaggg cctccacttg gcacatctta gatcctagac aaatgctgaa caaggcagca 44640
   tggtgtgatg gtgaagggtt acagccagac tgactgactg gtcctgtctt cgtttgaagt 44700
   tttgatattt tgttcctcgt gggttttttg catttttact atttatttat ttatttattt 44760
   atttatttat tgagagacag agttttactc ttgtttccca ggctggagtg caatggcgtg 44820
   atctcggctc actgcaacct ccgcctccca ggttcaagcc attctcctgc ctcagcctcc 44880
   tgagtagctg ggattacagg catgtgccac cacatccggc taattttgta tttttagtag 44940
   agatggggtt tctccatgtt ggtcagcctg ttctcaaact cccgacctca gatgatccac 45000
   ctgcctcagc ctcccaaagt gctgggatta caggtgtgag ccaccacacc cggccaattt 45060
   tttttttttt aaaatatggc attaaaatgg tatttatctt gactactgag tttttggtta 45120
   accccttaca ttttgtgccc aagacatctc actcatgtta tcctaggccc agcatggctg 45180
   gccttgtctc ttaaattcaa atttttaaat ttttcattcc tcaggcttct cacttgtaaa 45240
   atagaaataa taatagcatc tcccccacag gcctgtgctg agagtgaaat gtgttactac 45300
   acgtgcctca ccttcatttt caacaggacc tattgccagt aagtgctcgt taatgttagc 45360
   cattattctt ggcattgctg ttgttattag tgtcagccat cattctctca gccaagagat 45420
   ccttccagaa gagaagttct ccatggaggg ggatgggaga tgagctgaac cctggaaaca 45480
   gaaatggatc agatcaggga gcctcagtgc cctgaagcca gggcaggggt gcatggggct 45540
   tctgcaaaac ccagggccaa gcatgcaggc aagaggcagc cttgccaagc ccttgcagaa 45600
   aatgcttgca gtgcaactcc tctctggaga cgccacaagt tgccccgaca gcttttcctt 45660
   ttggggcttg tttaacattt caaacaaaaa taaaataaat aactgcagtg tttatggaaa 45720
   cctaagacag aaatgaagac gacttagaag aaagttaagt ggaagtgggc caggaggggg 45780
   tttagaagat gcccctttcc ttggtgttag cagttttgtg cttcagtttg tcagctcttc 45840
   ctactgctct cccctagcaa ctcctctgcc agtgtgcaca aatgcatgtg tgcacacatg 45900
   tgcatgtgaa tgcatgtaca cacacatgac acacacacac tcatcccaac gagaggcaca 45960
   catttcagac accatctcac acacacacat ttctctcttt ctggaaatgt gtccacccac 46020
   tttctccttt atcttcttca ctgtgttttt ttgttttttt ttttttttct ttttagacaa 46080
   agaatctaat taactcattc tggccagatg gtgtacatta ctttaactcc ttcactcctg 46140
   tgaaaatttt tggcctcaac cttcactctg aatagaccaa gccaaactcg ttttatgtgg 46200
   ataagccatt tccaagtcaa agttccgtgg caggaagtgg gagagtgggc agagggagca 46260
   ggtccttaca agcaaagact tgtataatgc tttgcctaga tttgcttatc agtatgttaa 46320
   atatatgtgg actattttat tcttccacct tttattttgg tatcttcttt gtctatccag 46380
   tctcactttg ccagactcca ttatgcaaga gaggctttgc cagaagcaag gatgctgtag 46440
   aggaaagtaa tgaaaatgtg aggatttctc agaaggccac atacaatctc taggtctttc 46500
   cagtacacac agcttcaaaa gccaggtgct gtgttcccca agcaggtcag ggcatttcca 46560
   ggagccctgg attccagaag ctcaggacac ctcagtggta aagagaccct aatgccggtg 46620
   acagcacaga ccctggaaac cctagattgc ctccgtgtgt tcctcttggc ctcagcaggg 46680
   ctggtttccc cctcctgtag gagcagagga ggctctcctc tcttgagccc agggcctagc 46740
   atagtgacca taaggaagca tagaagatcc agctgcagat ttggaaaaca gagccctcat 46800
   ctctggaaga gccaaaactg atcctcaaca tgtgccatgc acatcagata tcaaacgctt 46860
   cattcatttt gccttcgctg ggtaaccagt gaccttgaga agtcacctct cttgagtctc 46920
   aatttcttca agttgggaac attacatgac aggacaggac tgtgaggatt aaatgtaaac 46980
   aacatttttt ttttttctat agaacatact gtggggccag caccatgcaa ggtgctttta 47040
   tctacaattt atttctttcc cttcccatgc cttctgcctc tttgaataac tgctgcaatg 47100
   gctgtagaga gataaatgca gtagaccaag acttttagtg atttttaaac aatgttctag 47160
   ggcaaataca tgggtatgtt tacatactcc acaaagtgaa aaaattatgc tagtcagtgt 47220
   gcaaagtgtg tttttccaga cctgcaaaaa ctgagatcaa cttccatatt tccaacttcc 47280
   atattccctt catcccttaa gctaccaggc agaggatggg agataaaatt tggccataaa 47340
   cttgtcatgg gtccatcttg gtcctaatta gaattatcta cttcctaatc cattaactgc 47400
   atggatagat agtctgaggg cctcaagggc aagagatgac ttctacccct ccacccatcc 47460
   agccctgacc cacccttctc atatacattg gagaaagctc tgctctgtgt aaaacacatc 47520
   aagtgcagga catggttctt ttcttcaact atctacagaa atgaatagcg gccctagaga 47580
   aatgcaaaag atgcctgcaa tccaaggcca aattggagaa catttcaagg agaactgggc 47640
   ctttattgta gagtttggga aatgagtaac atgtagatct gtgaatagat atgtctgagg 47700
   aagcaccagg cagcggaaat agctggttgc aggagcagtt tgtcctgccc acctgaatct 47760
   tcaggttcct tcagctaagc cttaggagag gaggctggag agctgctttg ctggatcatc 47820
   tcctttggcc cccctcagac tagaactgca cctcctctct acctagctct gtgctccaca 47880
   ggctgcctct gggctgcact gatgggctcc cttgactgct ggctcctgtt cagggttgga 47940
   ggaggaggat gaagtcatgg aatagtgtcc ccatgctggg ctgcaggtac agttgctgct 48000
   tctctactga agacccctga cagcaaccct gtcttacagc cagggtgttt gtagttgctc 48060
   cctcctccct gtccctttag agtggtgatg gcactatatg ttagatatgg tcagagcagg 48120
   agacatcatt ttcccttaat tctattctgc cttgtaaccc ctttcttaga ctctctgctg 48180
   agtctctgct tatgtgttgg gattgcattg atgcagatgc tttagggcca cgttgtggag 48240
   gatacaagat gatgagacag gaggcagcgt atggtgaatc acttctcaca gctcctaact 48300
   aagttctggg cacatgaatg ggtactcagc acatacctac tgaccaagcc tatggagagc 48360
   tgaaaactca taggctgact ttgaagcgag gcctttgaaa ccatcgtccc aggggaaata 48420
   atctaggtca aagagacggc tgtggcaagg gtgcgagctg gcaagcatga gggctgacaa 48480
   agcaggcagc actcctcctg agcagatgac cagccccgca gtgctggatg ggtacgacta 48540
   ttccaaaccc tggtgtaatt ctgtcctcta acgtggctgg tttgactcag cactgctggg 48600
   gagccacaga gccttcttct ttagcattta tgagctcatt ataagcatcc aagaaaacat 48660
   caacaaaggg tgctaagtcc ctctagaact gccttttaaa tttttctttt gtaaaaagaa 48720
   tgatgcttta agtaaaacca caacttacag aattcagaca aaaccaggca gcccccaatg 48780
   ttgttatccc agctcctctt ggaataaatt tggaatgaaa caaaaagtca cagaagagaa 48840
   aactccagcc cacacccaca ccttttctga catagccgcc tgtgcaggtt tttgggacca 48900
   ggtctgccat ctgtattccc aggctgggct ggagcacctg aggctactat ggttacagaa 48960
   aggaacctgg catccgaagg tataggctca aatcccagct ctcatgattg ctagctttga 49020
   ggtttcaggt tcatttctta agtgctctga gctttggtgc cctcctcttc aaaatgggaa 49080
   tgaggagcat gtctagctcc ctgttttgtt gcagtgaata gctacatgaa gaacagctaa 49140
   accagaacag aaagaaggca aatggaagct acagaaacca aggatttcct tgttgaatcg 49200
   aatcttcctt caatcttcct tcaccacact agtggatctc cctgtgggag ggatgttgag 49260
   agtgctccgt gttttttttg tttttttttt tttttttttt gtgatggagt ctcactctgt 49320
   cacccagact ggaatgcagt ggtgcgatct cggctcactg caacctctgc ctcccaggtt 49380
   caagtgattc tcctgcctca gcttcccaag tagccaggac tacaggcata tgccatcaca 49440
   cccagctaat tttgtatttt ttagtagaga cggggtttca ccatgttggc caggatggtc 49500
   tcgatttctt gaccttgtga tctgcgcacc tctgcctccc aaagtgctgg gattacaggt 49560
   gttagccacc acaccagcct tctctgtgtg tttttaatca atagcaatat gtaccatatt 49620
   tagtagggac tataaagaaa ttgtgctaga aacacctggt tattttacag aactttgcca 49680
   gtgagtacaa aaatatgcgt agcccacaac ctctctcccc tagaaaatac ctcctgtggg 49740
   tagaatgaaa tgggtaggtg ttcttaccat tcattagcat ttcaacctgg ctatcttctt 49800
   cctagaattc cactactccc aattgcccat gtgtttccaa taaaaccaat tcaattgtgt 49860
   accaacttgg tgacaaaacc tttatgtagt tcttctatca ttttgtgggg ttttcaaaga 49920
   gggttgctct ttgatatggt ttgatgctgt gtccccaccc aaatctcatc tctaattgta 49980
   atctccatgt gtcaggggag ggcctggtaa ggaggtgatt gaatcatggg ggggggactg 50040
   ttcttgtgat agtgagtgag tactcacgag atctggttgt ttgaaagtct gtggcacttc 50100
   cccctttgct ctgtctccct ctttctctcc tgccaccatg taagacgtgc cttgcttccc 50160
   cttcaccttc tgccatgatt ataagtttcc tgaggcctcc ccagccatgc agaacagtgt 50220
   caattaaacc ccttccttat acattaccca gtctcagcta gttctttata gcagtgtgaa 50280
   aacagactaa cacactctta aaagtaaatg ttattaccca ccttttacag attaagaaac 50340
   ttaatattac atcaaggaat tagtttatag cactggatga tctggcctca aatctccagt 50400
   gtgatcagta tcaagctttt ctcactgtat caacagagtt ttcctaacaa atcataagac 50460
   acaagtagtt tctccaactt aatctattat acccataatt cacttgcctc cagggaaaaa 50520
   aattagatga aacagactgt agtggctttg tgtaaaacag cagctccatt cctttagccc 50580
   tattctaaaa caatagacca tgtgctgata gctagctaac ctccattcag tgcttcacta 50640
   tgtgtcaggc atttgttcca aacacatttt acatggatta tcccatttaa tctttacact 50700
   aaagtataat actcttatta ccccatttta aagaagagaa aactgaggca cagtgagatt 50760
   aaggaattca tccagtttct gctgcaagta gggtggctag cattgtaatc tgggcatttg 50820
   ccatgaagct gtgcccttaa ccactccact atgtgctata aggtggagct gacttcatgg 50880
   gggctaattc aaccagggga gtgtgctcat attaacagat ctgtgaactc cctggtttgt 50940
   ttgtgagaca attatctatt tgcttacctg aatcacagca ataatagtta tacctcccac 51000
   ttactgcatg cctacttggt agcatgtacc tggacagaag gttttcattt ttatgacaaa 51060
   cctgtaaaaa atggcttctt agcctggttt ttcagatgag gagtttgatt ctccatccaa 51120
   agtcatatga taaagaagtg gtgaaacagg aattccaacc caagtttgtt ttgttttgtt 51180
   tctgaaatcc atagactgga gtagaatctc aaattatatg acatttgtct atgcttattt 51240
   tttagtttcc aaggtgatgg attatttaaa aaccaaacga ttttgaatgt aaaacaagct 51300
   taattcaatt tacttataaa caatgagcac tgactcttgt atacatttaa gttatgagac 51360
   actccaatat taattcgacc cacacttttc tttctataat ctattgctcc caaatattct 51420
   atactatctg catgtctgcc ttagatacat aaggatccag ccctttccca tgtgtctctc 51480
   aggatggcac ctttgctctg ggccggatgc catggggttc tcagtgatgc gcactgaata 51540
   atgcaaatat gaggacataa tcacagaagg aagcccttga aattctaaag ataaggccca 51600
   cagaccaggg tactttgaat aagtcagtca tccttttcca aggttatact tttcccccct 51660
   aaaataataa ctgcagacac agttctcaaa gcaaacaggt ctggtatagt agaggcattt 51720
   ttagacaatt tttttctatc tacttgttaa gcacatttta aaatactgat tgccaataaa 51780
   gggctttcca tagaactcaa aattccaagt ctctgtccca agcttctggg agcattcctc 51840
   catttttctg tttttatttt ttgagatgga gcctcgctct tgctcaggct ggactgcagt 51900
   ggagcgatct cggctcactg caacctctgc ctcctaggtt caagagattc tcccacctca 51960
   gcctcctgag tagctgggat tacaggtgca tgccaccatg ctcggctaat tttgtatttt 52020
   ttagtagaga tggggtttca ctacgtaggt caggctggtc tagaactcct gacctcaagt 52080
   gatccactca ccttggcctc ccaaagtgct gggattacag gcgtgagcca ccccggctgg 52140
   tcgcattcct ctatttagtg ttaataccct aagactcccc agctgaccac caaatgtagg 52200
   caggtggagg gactgacctc cagggggcac atgtctcagg ttgacaaaca cattttctct 52260
   tgatcctttt tttcagtatg atgaagtatt ctacaagact ctcattgaac cttctgatat 52320
   cacattctaa ccatttatat aaaagtcaaa agctccttgt cttcaggatt ccaaagttct 52380
   tagcacaaag gtccaggaag tgtctgaatt ctttggtgtc tggttcagta tggatgttcg 52440
   ggtccctctt agacttgaga tagaacagga aagagatgaa tagacaatga cagaggtaga 52500
   gaaagtacca acccttccca ggctgtcggc ttatttgact gtgggaatca ataaaaagta 52560
   tttagtgact aaaatagaat ttgaacccag ttcagatctg taagctaaca ttttttaggc 52620
   aacaccatac agcctcttca tcattccctg gcctctttca tcctatccct ttttctcccc 52680
   cttcctcttt ctctccttta tttctcctta atcatcttgc ttctctttct tatatcttct 52740
   cagtaagtaa ataatccaca acctcacagg taaattaaag ttgccattcg cacatctgtt 52800
   ggctcccagg tatggttcca ggatactggg aatgttaggt agtgagaaag attcagatga 52860
   tattaatttc ctgcaaatgg aatattcatg gcaggcacag tctggccaat tgctcatggc 52920
   ctggcgagta cgactagcct cgattgtacc tggtctttgc gaatgtggtg gcatgttcac 52980
   tggcatcaca tactccctct catctcctga atggctgacc agtgcttgct tcaagttaga 53040
   ttagagtttg agctttgtaa cataagtccc cttctgtctg atcaatgaaa tgagggatag 53100
   gtacttgccc gagagttatg ggacctggca tttaacgtga gaacatgcat cttcagaagc 53160
   actggtgaga cttcagaatt cctgaaagca ctattcttgc aaggcttgaa tgtaccttct 53220
   ccactccacc atgtcctctc ccaaccctac tagcatgata tcaagcttgg gtggagagct 53280
   cgcttgcaac tttgtcagat acccatgagc cactctttga atttctgaca atgaacaagt 53340
   atgtgggagg ccaaccccat ccaacctgac ccatctgaac ccaatcacat gttttctatt 53400
   ttgaaataaa caggcagggt ctcaggaatt ttaagtgagt cctctatatc tttctctatc 53460
   ttccttttct tccactaatt attacagaat tgatctgcca gtaattacta ctaaattgaa 53520
   tttcaggggc agtgctgtat ctggccccaa taccctaagt ccagtaatag cctggaatgt 53580
   tgctgagttg ttaccgtcct taaagggtga agccacaatg atactttcta taacttctat 53640
   ttggtctttt taagcccagc acttagaagg attcacttgt ggagaccttt tcatttcttt 53700
   caactctaca gcttagtgcc tgaaaggcac atattggtct ctgtgaatct ctacgttaag 53760
   aatctgattt gcagcactgg aaaccttgct agtcttagct ctgggctgaa tcccatgaag 53820
   ccacgccatg cctagggctt tcatgctgta gtccatacct ctgaagaaaa cggagagaaa 53880
   catcttcctc ccttcttccc cccaagtgtg tgtctgttct cgtcagattc ttggcagggt 53940
   taccaactct cttctaagca acttggattc acaacagaat gattcctttt caatgccaat 54000
   ctaaacaaat gccatcaact cttatcccag ggagtatgaa aaacagaaga gtcctttgtg 54060
   actctgattg gctttgtgac ttccttgggg ctggtgttgg tctcaggctt gtgtctcgca 54120
   gtcatccaaa atgtgggtgg gccatgtccc accttaactg tggttgtgaa cgcagaggat 54180
   tcactcaact ttcagcatgc aacaataatc aaatgcagtt ttatggttca cagagggctc 54240
   caccttcatg atttcatttg atcctcctaa caaccctggg agctcagaga gactaaggaa 54300
   cttgtctcat atcacatacc caggagttga ctgcacggca tctgatgcca gcttctgtac 54360
   cacgtgtcct cctggtgata ctagagtaaa taaaacataa ctagacaagt aaatattttg 54420
   tacaaagtaa tctgctaaaa atgtggatgg cattttaggt tatttgttgg tttgggttat 54480
   gcctcttctt attttgtaga taactgaaag ctaagagcat tgtcttattt atttatttat 54540
   ttatttaggc agagtctcac tctctcaccc aggctggagc gcagtggtgc gatctcagct 54600
   cactgcaacc tccacctccc aggttcaagg attctcctgt ctcagcctcc caggtagctg 54660
   ggattacagg cccctgccac cacacctggc taatttttct atttttagta gagacagggt 54720
   ttcaccatgt tggccaggct ggtctcgaac tcccgacctc aggtgatcca cctgcttcgg 54780
   cctcccaaag tgctgggatt acagcacttt gcaatcccat gcgtgagcca ccgcacccgg 54840
   cccattgtct gtcttcataa gcagaaggct gtggcacagg gtaggagaag aacatgaaga 54900
   caaagttgag gggagttgct tatctcatgc ttcttgctgc tctgcctgaa gcaaaaatgc 54960
   tttatggata cttcacttat gatttaaaat aatagctcac attgtgtact tactgtgtgc 55020
   caggaactgt gctaattctt tatattatgc ccataaaacc tgcttctgaa gcattggggg 55080
   gtttgcactt gccgttccct ctgtctggaa tgcctcccta tgcctacctt catgaacctc 55140
   ttccctttac tttccccatc tctgttcagg tgtcacattt ttagttattc tttcctgacc 55200
   gctcagtata aagtagctca cacctctacg ttatcacact ccctcttacc tgctctattt 55260
   gtctttatag ccctgctcac aattatagtg tacatggatt tcttaatgtg tgctttatct 55320
   tccccctccc atcggaatat aaactccttg agagcaagga tgatattttc ccctgctgtt 55380
   ttctcagtac caagaagagt gcctggcacg tagtagctgt tcaacaaata tttgctgtgt 55440
   aaattatctc atttatagaa taaggaatct gaggttctaa gggagtaaat cattgacttc 55500
   aggggacaca acaggcaaga ggtggaacac agtcatgagc taaggcagtc ccagatcctg 55560
   cctgaccctt cactctcttc ctcattcagt gaatcgtaag gcacttgggc tgtttgagca 55620
   gtgcccttat ccataccctg cccagtcagg gcctcccaga accaggtaaa aggatctggc 55680
   cccctctgcc atcctcccac tggtgccctt cccctcccac ccaccgcaac ccccatctag 55740
   ggagaggaaa ctctttttcc acctaaattc cccctctgtc tagttcatcc aagtcatctc 55800
   ccttcccacc acactgttta cacttgtggg tctgtgttga tgcatatcac agccccctta 55860
   gtcattgttt agccattata ctttgcaggg gtaattttcc atccctctgt ttatattttt 55920
   gcttctcctt catgtgctcc ccctcccact tcttaagggc aaaggaagac atgttcagtc 55980
   atacaagcaa taatagagtt ataattaaga tgggaacatt ttgccaaaag gaggatttag 56040
   ttgtgatttg ttgaatgcca tcctgagtgc attatttaag agcaaattca agctctttca 56100
   tgagacttcc tgaacagtgc agtcctctga gcctgctgtc tctatagtct ttgtgtcagc 56160
   tttgtggggt caggattgcg cgtgaaggat acatgtttag caaggcctgg ttgctgaaaa 56220
   agcttaaaag gaaacttcag ggactatcta ggaagatctt ctgtgatctg ttctctgatt 56280
   ttgtgtgctc ttttgtggtt agccattgct cttggtaccg tgtgggactt agcaccctgt 56340
   gcctcaaggt aagaacacac ttccagccca gcttcagttt aatgttcact atgagagtct 56400
   ccagtaacaa cccacactct gcgtgggatt cactgccttt cccatgtcct ggtgttggac 56460
   atacatactg gacagagtta ctgccttttc ccctagtaat taggggaaaa ggaccacatt 56520
   ttcccctagt aataggggaa aaggtgtcca gtatcagagc taggtagagc tgaatttgga 56580
   tatgaacttg agctgtaccc catggtgtct tttcttgcag tcctagcctc cagcgccttt 56640
   agaatttgca ttctgtttgg tgggatgagg acggtggcag tggagacagg gtgagaggag 56700
   catggatggg tcctaagcct tcctgtcttc agcagcatct cgtgctctgc ggctctgctg 56760
   tgtgcctcag ggctgcccca ttttcaggtt gcatttatta gcttcctgga tgctctggct 56820
   tccagtaggg tttagcctgt gggaggcact ggatggagat gggagggagg gagagaggac 56880
   aagagaagtc atgttatttc tcctttgcct tcccagcctg ggaggtttct ccagtagcag 56940
   ctgcctctct tcttggattc cagaccccac tagacaggtg tgctgtggct ccagcttctg 57000
   tctggtgttg ctggcccctg gttcacctga ctccagctcc tcctcctcca tgcagctggg 57060
   tcctgctgct gcctccctct gctctgcctt gctgtcccct gtcaggcctc tcagctcctc 57120
   catcccctgg gttaccaact cgctatactg aaatccttct gttttagaaa ctcatatgtg 57180
   attttcattc ttcttggttg gacactgact gatattgagg gatttgtctt agaagtaaag 57240
   ctgtgtgagc atttctcttt ggtaagttct cattttccct taatcattag aagacacggc 57300
   acagtaaacg cgagctcatg agcagaaggc tacatagttg tatacatgtg tgtgcttttt 57360
   ttcacaccag agtttgatga agcaaaatac ccaaaggtct tttaaaagta aagaggtttt 57420
   aagagtaaag aaatttaaga aaaaaaacaa aaagaagtaa agatggttag gtctcctgtt 57480
   tgctctcctt ttgcaatcag tctgtctcgc tctgtgagct gactatgctg cagacttttc 57540
   ttttacctcc agcctttcgc ttgtagggtt tgaggctagg ggcggggaga gagagttcag 57600
   cccagcaggc agcaaaccct atgtgcaaac acgatgtgtg aacattgtgt gcttggggga 57660
   actgggatta tttagctgag aagacacgaa atctcctccc cttatttggg ctttgggatc 57720
   tgagttcatt ggaagctgaa atgtaagaga aagaagaaag ggggaaggag tgggacaaag 57780
   gagaggggag tgggagatca gactgggcac attctccctc acagagggaa gcgatttctt 57840
   tgttaggaaa gcagttctgt tttgagaccc ggatgccttg tttatgctca ggtgtgaagc 57900
   tggcttagga attgagttta gcttgcctct gtgcttttat gaatgtgttg gctgctcagc 57960
   aaatttagta cagaaacatt ttatgtaagc agaagagaaa attatatttt aaaagttgtt 58020
   ttaacagaag gtcagagtat gggattgaga attaaaaaaa aaaaaaaagg aaattctgtg 58080
   ttctaggctt taagacagag agtcacacac actaatttag accagctggg gtgatctcac 58140
   agggactcca tgggccagag ctgccgttcc tccatggaga gcacatttcc cctagaaatg 58200
   tgtactcagg tgcagctgaa gggtctgagg tcatggctgc atccctggcc agggtcctcc 58260
   caggctgcag cctctgcttc ctctgttgtc cattggtcta gagccatcct tcgagaccag 58320
   agccagacca tgagcgagcc aaatgtgcat gaggacagca tgaattatcc ttggagacca 58380
   gggggccctg ttcatccaga cattgtcttt ctttttaaag aagctaaatg cctctttttc 58440
   cctctttctt cttgcatgac ctctgggagc ttgggcagta aatttctctg ggacaggttg 58500
   tgaggaacct cttcaaagaa gcagagatga gacaaactgt ttatcagttc actctctacc 58560
   cctgctgccc tcccaatgct caggtgggcc ctagattcat ccagaagcca gggtgttgtg 58620
   cttcatgaaa gggccaatac ccctaatgtg ggacctatac aataaaaggc aagggcagaa 58680
   agagagaaga aataaaagga aaaggggtgt cttgagggga gtacatgata gactcctgtg 58740
   cctggaaggg actaggaaac ttatactgac tggttcactg catttgctca aagagtattc 58800
   atcttcttga gtttgtcagg tgactgtaag agctgggttc aagttctttg gttcactatt 58860
   atatgcctag ctgagcacaa tgcctgggac ttagagggaa ggtctgggac ttcaccagga 58920
   ttcccaaaat agaacaaagc atttgttatt tgcaaaaggt tcacaaagcc tgaaaaaata 58980
   tgtgttgtct ctggtggaaa agtaagggaa tatagcttaa gtacctacta cgctctgact 59040
   ctttggattt cacctgcagg gcttttagag gtggataatc agggccatct tttcgggggg 59100
   tgggggcgga aaacaaccac tgaagaggtg aagaatcttg tagttctaaa aggatcagag 59160
   agaggcttaa aacaactttt gcaaactgaa atatgccagc cagcttaaat tagcaaagct 59220
   ggaactgtgg cagaatgaat agcccgtgtc tgagtgagag ggaaggaaag ggtctactgt 59280
   gtgataggca atggttccaa agtaggagta ggggcctagt gtggtcatgc cttctgatat 59340
   gtcaagaaaa ggcaaaaagt tgatatttat ttgaaatatt ctgattttat aggttggtga 59400
   tcaattaaaa aactaacaaa tacagttaac aaacatgttt taatgcttgc agacaggatc 59460
   cttcctgccg atgtttggcc tttgtcttta atggaatttg ggctgcccca taacagccca 59520
   ccctaagcaa aacttctttt ctgcctttct tttatattgt tttttatgga atgtagaggg 59580
   atttttctgg aatttttgga acggggaaat tagtgacaat ggtggtgctg agattcctgt 59640
   ttatttcttg gcttttcccc aattccctga gggaactcat ttcttttgga tcttccataa 59700
   catcggtcac atagttgggc acagagaggg cccacacttg gtacatgttg atgaaataaa 59760
   tgtgtcttcc tctaagtcta gctgaatgga gaactgaaat attagggtgg gtgaaagtta 59820
   cctaaattgt ttgctgccaa attttgctgt gttctttgtc tccagttctg tgaggtgtgt 59880
   gttagatacc tattgctgta taacaaatca cccccaaaac atatctgttt aaagtaacaa 59940
   acatttatta tagcacacat ctgtaggtca ggaattcaag tgcctcttag ctggtactct 60000
   catggggctg taaaggaaat atcagttggt gctgcagtct catctgaagg atccactcca 60060
   aattcattca tgtggttatt ggcaggattc ggttcctcac cctatattgg actgagggcc 60120
   tcagttcttc actgattgtt tgaccggagc tctccttcca ttctttgcca tagggcctct 60180
   ccatagggca gccccgaaca tgggatctgg tttccattag agcaaacaag caaaggaaag 60240
   cagtgcaaga cagaagctgc aatgttttgg taacctaatc tcaggagtga ccctcctgtc 60300
   actgcttcta tgctgtattc tagtcattaa aggagaccat ctcacactca aggggagaga 60360
   ctacacaagc catagtgctg ccatggcaac acaaccatta ggatgatgcc ttgagccttc 60420
   cttcttcata tttcttagac ttcctttctg acctagagga gccagaagga cttgggactt 60480
   gtcataccgt ccaatcccaa gaaatgacag gaatgaagga atttttaaaa attcaactca 60540
   tttcaacaca cacacaaaaa atgagagaag tgagtcaatt tgagaactga aattctggta 60600
   gacttttggg gagacactag aagggccatg ttcatgagta ggaaccatag attctgagtc 60660
   aattgggcat gaaagttttt ctcctggtgt cccgatcttg gtcgtatagt gaccaagcat 60720
   ccaagaaggg gataacacaa tgttcttaca gtgttttctc tgttgtatta ataactcagg 60780
   catcagccag tggaatgggc aaagctaagt gaaggtttga atgccagaca gattctccat 60840
   tggctaaacc caatagacat gaataaggaa taaggccagg aatcagcatc cagatatatt 60900
   aatttatgaa tctgaggtta ttccaaaaag caagagaaca ggagcaccat tggctgagac 60960
   ggacaccaat accaagctct agagagtaat ttcaggcaga aaggaaacct taattaccag 61020
   aggttcaagg gataggcaca cagtaagtcc aaataggcag aaagaaggca tcagggataa 61080
   tctggcagag actgcattca aggtcaaggg cagggcatcc cagctgctgt tgagactggg 61140
   ctccaaggaa gtctccctgg agagactgaa aaacaatcct gggccttaat cactgaggac 61200
   tgaaacacaa cataggagaa aactagggct taagagaggc ttggtcaaga aataggcaag 61260
   aacctgatta ttggggaaca agagatccac agtcaggctg gaccagtagc aagacagcaa 61320
   gagtagacaa aaagttgacc actgccagaa accaagattg gtctctgagt ctggcatggg 61380
   gctgaaacta cttgttgaga tcaggctgtc tcacttgcca ggggtagagt catgctggaa 61440
   gtggagccag gacagacatt gaaccctggc taccctgtga tcaattgcac tccataccct 61500
   ttgatacctc atgggaaaat cagacaagtg ctgttctgaa aggcaattag gtttcatgat 61560
   ttcctccccc atccggacat cagcatgaaa ttgggggctt ggcttccaag cccatgttct 61620
   tcctttttgt gaagggcagg gtgagacaaa ggacaccaca ggccagagca accctctgct 61680
   tccatacagg ccctagaaga gaagtgagga cgcgggtgac ccacacggcc ttctgtgcct 61740
   cttcaacagc tactctccca gcctcccatt tctgccaagc cacattctcc acaggactca 61800
   aacatttagc tttactttag acttttccag gaatcttccc aaatctcctt tcctcatccc 61860
   ccctcacctc ttcctcacca cccacagccc cctcctccac ccccatgtac aacatccggc 61920
   ttttactcat gcttgtttct aattatgcat actaatttat tttctttcct tttttcctga 61980
   cctgcctata ttttcctact cattctttgg cctccactac cgaaaaatgg aatactcctc 62040
   ctgtacaggg tcagggtttt tccttcccca gtgcctaaag ctggattcag tccacagtga 62100
   cttctctaat aacagagtgt aagggactcc ttggttgaaa agaaggaaaa accccaactt 62160
   gcctttcagg gtctcttcaa tgagcttgat ttgtgttaaa tttaggggct caggaatttc 62220
   tgccgatttg cagcacttac tactcctacc cttcccaaca tttgtgcttt atcgaaacaa 62280
   ctaaattagg agcagcttga gggtaggggt cagagcacag acattttctg tattgcctgt 62340
   agaaactagc acagtacagg caccccctaa gtgcaaaaga agtccttgct gacccagatg 62400
   gaattttcct gaagtgagtg ctttgtgtct atctttagaa ctagactttc ctgataaaat 62460
   agtcaggaga tttggctgaa ttttccagta ttcaactggc tatgttgggg tcatgtagat 62520
   acaaccttta agaacataat gacctttaaa atatctattc ctgaagtcag gtttggtccc 62580
   tagtctgtgg gcagatgact gttttcatgg tatgctttgt tcgtgtcctt cctgggtggg 62640
   ggaggatttg gggggacagt ttgactctag ttatctacaa agttgagtta tgagaccagc 62700
   ccttttcaac tttacctctt gcaaattgat cctgtaataa attgacatga cgaaaacacc 62760
   aaagcaacca gagaaatctc acaaaggcaa gaacaccatc acacaagtcc tcaaaatctt 62820
   tcccaagatc cactaggggt taatgccact gatctgatac tggggcacag cctaggaggt 62880
   tttgctgtct ctgaacgcac atgtctccag cttacagctc acaggccagc tttgtttttg 62940
   aacagattgt gttgggggaa gggaggcttc tgagacagag ctgagagtcc ccagcaatgc 63000
   catccctggc ttctggccca tctgaatgcc ttactctttc atgtaagcaa gacatgaggt 63060
   cctgctgttt gagttggctt tgccctgctc cgtgatttca gttccttgtc tctccagcgt 63120
   gtgggctcat ttccgtcctc tgagcactat tgtttttctg acttgaacag tcatccagaa 63180
   aacctcagta ataaggcttt tgtatcatta tttactttgc taattatata tgtctttccc 63240
   atccccagtg cttgaagaca tcaaattgca gttgaagtaa cggcagaggg aaaagcaagg 63300
   gcaacaacaa caaaccactt cactcctctg ggtggaaaca tgcaattgta ccaagaggga 63360
   taaggaaaaa actgctcatg tttgcagaaa gaagttggaa taagggcgtg aaagcaggcg 63420
   ggctacttta gcatctgatg ccagggatat gagagagtag cccagtgtgt tcattcaaaa 63480
   agtaaggctc tgaggccagg accaggaaag atcctggagt ccccagtcac aatggcatga 63540
   agcaggatga tgcagagagg ctaagggggt gattcagtgg gttctttacc tataaaaatg 63600
   atggaaaccc caacatcagc tgggcaagtg gccctgttca gagctcctgg actcgccatg 63660
   actaaaacac tctgttttat ttcgtagttg atatctattg ttcttgcctc ccagcaagca 63720
   atggaacagc acctttactt tcctttgaga aactattcct ttcccattct cagggcttgt 63780
   gaggatcaag tgaggtcaca taatccagat ctggccaatc agcccatctc atgctactgg 63840
   ctacactaat tggtttaggg atagtaaagt actcaaattt gtttactgaa catttagccc 63900
   tggaattttg gtgatgggag gtgtagaggg taaactcttg atgatatcat ttgtgtacct 63960
   gggtccagcc atgcctgaac ttttcaatta cttgacccag taaatttcct tattcattta 64020
   aatgttagag ttgagttttg tctcttataa ctgaaagagt gacatgtgtt tatattaagt 64080
   gcaggtcact aatatagtct attggtttcc ccaggcttgt tctccttttc tcaaatagga 64140
   ctgatcagat agtaagtgct ccatacctgt aggatacata aaagagcaaa tgaacaagga 64200
   gcaggagata tggtggctga cggttggact ataatttttt cttgattttt ctgactttat 64260
   tttggctgtg atctaggaag tgaaatccag gtccttctaa accccaattt aactgtggct 64320
   cctgagggag attttcagat ctcaaatgat ctgtaagttt aagggaggat gattggtccc 64380
   tgtacactct ctgtgagagt tcctttggaa tactggtttc agaatgtaca gccacacagg 64440
   taactggatt agggacggcc agtggttttt tatgaatgct attgaagaca ctgaatggaa 64500
   gcaattctca ccagaactct ttttgctgtc atctttcagg ggctaagttg acactagaac 64560
   caatcaatga tccctgaact gggagaaatg ggtggccttg gctgtgattt ttggttgtgc 64620
   tgcacatttc aagatgacaa tattgtttta aattaacctg ttagagtgca cctttcctaa 64680
   agaactttac cttgatgtct gagtaagggc agtgcaggag gtcccccaac ttaggggact 64740
   gatcatgttc caaaggtcat ttctcagtct ggatgttttc atgagataat gaattccaag 64800
   atattgtaag gttcaatatt ataaatggtt cttagtttac taagataatg cattgcctct 64860
   ttgtgaagcc ttctctggtt accctaatca aaagtaactt ttctttcctc ttatatgtca 64920
   cacagtatta aatgagttgt tttttaaaaa catcttaaaa tcttatactt aagcaattat 64980
   gtagattatc ttggagtgtt gagcttattg ctctcattag atgttagtaa atggaaactg 65040
   agtccacaat tttcacactg ttctagttct ttttcattct gcttcatttt ctgttgtctc 65100
   ctgccaggtg tctgtgctaa ctcatcttgc ctgaggaccc ctaccccatg tctcactggt 65160
   ctgcttggct tacttctgaa gcccagcacc accttccctc agtggctctt aacaataaac 65220
   cctgacacag aagttctggc tggtccacct attcctttga gtcaggtcac aggtttccct 65280
   ccagctaact gatggatgga ctgtccttgg ctcaggggtc cactcttgtt caattagata 65340
   cattttgggg gcagggtcag aattcataaa atgcaggcat ttgaagaaca gggaatcaag 65400
   gtagagagtt cctagaagac agagcaggga ggtaaaatga aatattctag taaagtatta 65460
   atatttgtgg caggatatgg tcttttttct ttctttcttt tcttttgaga tggagtctta 65520
   ctctgtcacc caggctggaa tgccctggca taatcttggc tcactgcagc ctccgcctcc 65580
   cgggttcaag cgattctcat gcctcagcct ccctagtagc tggtattaca gatgtccgcc 65640
   actacaccct gctaattttt gtatttttag tagagacggg gtttcaccat ataggccagg 65700
   ctggtctcga actcctgacc tcaagtgatc ccccgccttg gatatggtct taactagaaa 65760
   tgaagagatc tgagttcaaa ttcagcattt gtcacatgta ccctctctgg tcccaaggaa 65820
   atcaagtaat atctccaatc ttcagatctt tcacatgaag aataaagagt attgatcacc 65880
   atatctctac acctccttcc agctctaaga cctccaggac tcacctagaa ccaaattcat 65940
   ccccacacag tggcggcagg gtcgtgttgt catgatgatg gcctctttcg gtgaagagag 66000
   gttgtcagac acctggagca atgccagaga cattatcctt ccctaggaga gctactgata 66060
   agaaaccact ggcagctcct cattcacttc tccatcattc actcttttct cctttctttt 66120
   tccttagagg cataaacaag gtgtagatca tcctgttatc cataatgaag gaagatttta 66180
   ggatattcca gcttagatcc aggtgtcccc agcttctata ctcactctga agaagtgtac 66240
   tttatctact ctttttacac catttatccc attttggcca tttctgaaag gaagatccta 66300
   gaagccaggc ctcttggaag aagttggagc tacagcattg ataggaaaac ctgacaccta 66360
   gaaaggcaag tcagttcttc ccatgcagtg gctttgggga ctgtaattta acaaagctct 66420
   tttttacttg tgtaaagtga tctccattcc acttttttct tgcactgatt ctaattaagt 66480
   ggaagcaatt gttatcatca tttaggatta tagttaaaat acccctcaaa ttgtttccat 66540
   ttggaatggg accttgtccc attctgctct tgctgaagga agtcttcatg gaatcacagg 66600
   actcaacaaa catttctcat taaaaattct ctcctttagg caacagtggc atagaaatcc 66660
   ttatgttcta ctgtctgacc cgtatgagaa cagcgcactt ctcaatttat aaaagaggca 66720
   ttttccctcc tcctgggaaa aatatttctc ttggtcactt gagtctagca tctaagtcat 66780
   ataatgctat tatagccttg ttttgctttt gatttaggtc atatcttctt tttctgatct 66840
   ttcttaaagt gaagggacca tttgctatgc tcacaaaaat tctcaaggct ggaactacag 66900
   catgctctgg tttctaagcc atacacatcc aggtagaagg gtcaatttaa ttgccaatga 66960
   tctcagcctt ttctaacact gttccttaag tttctttggc actgcatggc aagcaactga 67020
   gtcatgcatg aaaatttctc ttctagacaa cccataccta cagagggagg atttggttta 67080
   ctgcagatgc tgagtgcttc tgatggcaca cattccttct tattaatatc cccctggccc 67140
   ataatggttt gtgtggacaa cagctacttc cttcaagggt cctttgcttg gtaaatatat 67200
   gcctccagga gctttctggg tagaagcagt cacaaatcac aggggtatct atgaatggca 67260
   cggcccatgg aagggctcac actgtcctga tttttctacc ataagagcta aaataaggga 67320
   ataggcttcc aggaaataag tcagatgggt tgtctgacta agcacagggg cagggattgg 67380
   aggtgagatg acagagaaca gtcagcattg tctccgcttc cgctccaccc tgcagttctg 67440
   agctgaacag ataatttatc tgtggcacaa caaagtatat gacttggaag aatgttaaag 67500
   atcaagtcca ccttctcctt gtattatatg gctctgagag ccagagaggg gaggtgacct 67560
   tcccagaatg tgaatgtggt agagcctagc ttacaattca tgtccaactt aggtgtgaag 67620
   ttctatgaga ctatgctgcc ctctgcctca caatgctttc tcccatggga gaggaggctc 67680
   agaggtacaa agcagctgca gaaagactct ctcaagctat tgactcttgg atgacaacaa 67740
   ttaatgttca acttggcttt acccccaaca tgcactgcac agatgcctgc aggtctacaa 67800
   tgccgagacc aagtttgggg ggcatttccg cagctctgtc tattgtcctc ctttcctctt 67860
   ctgtggtcat tcttcccaag ggataggcct agtccagggt ggagaaaaac aattcttggt 67920
   ggtaggggca ttggaattgt atgggcaggg ttctaggcca actttggcct ttcacaattc 67980
   tgctaagcat tgaacagaat tttcacccta atttcacctt cccttccatc ccttccctaa 68040
   ttttgctagt gtgtatggag ttgatttgat gagacctcgt cttttttttt ttctcccttg 68100
   taacgtgggc cctatcttca agcctatctt catagccttt cttttccaga ccttgccaaa 68160
   aagcagtaga agagaggtta caaaatataa gaggaaacct taaaacaaac agggtagaac 68220
   tggagggaat cttaaataat gcctaaccca actccatatt ccactgatag gaaactgaga 68280
   tcctgagaca ccatccaaat tctgatgtaa tcagcagaac ttaatatgcc actttgagca 68340
   cagaaccaag tactttagag gtaagagagg ctatttgttt ccagcacagg ctgaaaccca 68400
   catgagaaag ttggacttta agtgaccata gtcacattct gataaattct aaaatgtctc 68460
   tctcattttc tctgacatca tattgtcatt tttcatacca caaatgtgtt acagtgaaaa 68520
   ccaggtatag gtcaatagat agtaccaaga aaaagaaaag ttatcattct ttaatgctct 68580
   ttagtttgaa aactttattt atttctttat tttgatggag gagtagagtg ggtcaagcta 68640
   agagttgcct tataaacctg ttgttctcag caaatgagtc agctgagata aaaacattag 68700
   tttccttggc gtgtcagcat ctctcccaaa ggagggtatt agttcatggg ttggcattgc 68760
   tatcacaaag aaggctctgg cagcacctga ttggcttggc caagcttttc ccagttggcg 68820
   tctgggcatc ttcctagcag tgggtcactc acccagcagt gatttggcct cctgttgaca 68880
   taaatatgcc taattcaaag cagaaagtca atttgttatt ttgcctaaag aaatgtaact 68940
   accaagcagg ctagatttct gtcttggcat aaccaggaaa tctttaccaa taatttatga 69000
   tgtggtcacc cttgttgtag gcattatgga agatggaaag atatataagg aatggcgtct 69060
   tgcaaaaaga actggacttt tttttttttt tttttttttg tagcaatgtg gagagtaaaa 69120
   gcccaggggt caaatggtaa ttatagaagg agattcattt aaagaatgtc ataaggaaat 69180
   agaaaataaa ggaaacattg caggaaacca tagggggcta gctgattcat ggaaaatgtg 69240
   aggttatggc tgggtctaag aataagtaaa tcattggtga ctatggttgt agaggttagg 69300
   acagagaaag ctggagcaac aagcaatcat ctgccagata agaccccggg ctgggtgaat 69360
   gcaaggaggg agcggacaat tttgtcttcc aactctgtgg agctggagcc ttatcttagg 69420
   agtccagtgg gcctggaggc ctgatcacca accagtgtaa tgttagaata agacttccct 69480
   aagcaaatca atgtgatgat tgatacaaac agccatcact ggggcctcct ctatttccaa 69540
   tctcttcctc agtttcccag ttctttcaga acctcactgg gaagttcagg gatatatgcc 69600
   tgcaggctgg cctggcgcag atggatgatg aagggtcctg ccttgagtgt agggtagagt 69660
   gattctgagt gctttccaac agactgtaaa ttaaagacac tccaaaaagc ccctggtggg 69720
   gatagatggg gagaaggggt atcactaagt cttcatcttt tcttctaggg ccagggtcag 69780
   tggttcaaat aaacatggtg ccccacagag atggcctgtg gccaagtagc tctcagtggg 69840
   ttgactcaaa gtcacctgga caggcctggc ggaaacgcag gatggcccag ccctttccta 69900
   ataggttttc cagagttgat ccatcattct gcccagaaaa ggggagaata aaaaatagat 69960
   cacgagcccc tatgactgtg acttgtgctt ccttggaaat gcaaacaggt gaaggagaaa 70020
   atgacacagc tagggtggta gggggcactg gaagtcaaca catttctggg ttcaaatgag 70080
   aagggtgagg tgctggcatg gaggaaactg gcactggccg tctccaggca ggactttctg 70140
   ggcactgggc aaggccatgg ctttgggagc tgccccaagc tgctggtcgt gccagtgctt 70200
   cattcaccct gtgaaatgta ttaattagtg agtggttttg gctcagagag agttgctgtg 70260
   gcttcagatg gatatagcag aaaaaaggca aatccatcta caggcgcctc tgagaaatct 70320
   cagctcttgt gagcctgaga agagcttcca tagagagaag gcgagacccc agcaaagttg 70380
   gacggagaaa attgagacac ccacaccaat tagtatgagt ctatcaatcc agtgccaccg 70440
   aaacacaagt ctaagcctaa cagaatctgt gctgcgaata agtaagattg ttggaaacag 70500
   aacaaaaaaa aactttgcaa atggtgcttc tttaagcttc tgcagttctc ttctctcact 70560
   cccttttcaa aaatggctgt aaaaaaaatt tttggtctgg tttagccaga gcgcgtcaga 70620
   gctggtagat gccttcaaaa ctgtgatctc cctttaattg tcagaggtta ggctgaggtc 70680
   cagggaggta aacagacttt tcctaagctc tgtagctggc tactggcagg attgagggaa 70740
   ggtggaacaa gcctcttcat tcccaattca ggtttctttc ttccatccca catctcatgc 70800
   aataaacatt tccaaatcta cctgcccagc ggggctaaga gcagagtggt ctccagaata 70860
   agtggaatag catctcaggg actgctggtc tgaacacgta ctccgaacat cacagctgct 70920
   gccaagcggg cagacgcctt agccccagcc cctctccctg ggctttcctg ggtcttgtcg 70980
   acacttctca catcactctg ccttaaattt ttctccccct agaaagagat aacttgcctt 71040
   tcattcatgc ctacctaact cagttcaatt caagtacata aatcctgctg acaaccctgg 71100
   cagaagcttt atgagtaggg agtaaagatt gtgagaaagg cagggaatta atttataagg 71160
   aaggaacaat gggagaggag agaaacaaag agaaggatgc aaagcaatta atgttaattg 71220
   atgcttaata tatgccaggc ccagtcatcc tagttatttc atttaatctt taaaataatc 71280
   cttgtgttgt catccatgcg ttttcagttg cagttccatg atttaagaaa agcacacagc 71340
   cagaaagtgg cagagaaaaa atcatatcct gagtgacagc tttaccggcc cagtgctttc 71400
   cattctaaca aatagtagaa agagggaaag agagaagaat cccttggtga taggaataag 71460
   gtagagagag gctgtgaaag agagaaacag ggctttgggg gatcctgggt tctgatgctg 71520
   acctctgtgt catttctgta tccattgtat gaatgtttat ggaaagtcag ctacgaagca 71580
   aattgttcta ggcagggggg tttcagcaga gcttgggaga gcagatggtg ggaggagtgc 71640
   atccatagga tagcctgggc tgtgggaata agagaacagc aagtcagggt gagtgctgca 71700
   aagacaatat aagagagcga caggcctcag agaacctggg aaaaggaatg aatggggtca 71760
   ccttaggaag ttcattagga ggggcctctc tgaagaggtg acctttgagt cgagacctga 71820
   ggcactggag ttacaggaaa tgtgatgttg cacgtggaga atttcttgga aatggagcag 71880
   caatgtagtc tgaccctatt gttgggccca ttaacttggg caatgtctca ccaaggtgga 71940
   ggatggtgac aggtcctcct gggatatggg ccatggcagg acgaggggac aatgtctgta 72000
   aaggatttaa aaagagtcat caattctaac gatgtcaatg ataaaatact tctccatatg 72060
   cagggggcca tttccatgac ccagctcata aggagtggag agagtgctat cttcctctag 72120
   aggcagctcc aggaagcagg agctggtaac ctgggggctg ctctgaaacc ttcaaaaggg 72180
   caaatcatgt aggagtctct ggccactttc ttttctgtaa gagaagattc actcaaggtc 72240
   aggggaccct aaggacccca gggaatcacc caaaaactcc agccagcccc tagaggaaga 72300
   caatccacag agctggcccc atcccttcgg cctgtggcta aggcccatct gcccatggtt 72360
   ccttcttgaa gtaattgctg taagaatttg tcattcacaa aggaagtctc ctattgtctc 72420
   ttttcggatg tcatgtaacc aatgcgtgtt aaaggtcagt tgcatcagtt ctcttaaatg 72480
   gcaattaagc tattgtctga gcaaaccaga ggcatccgga gtgcaagctt cgctcaaacc 72540
   tagggacctg ggaaaggctg ggaacagagg agcaagtagg acaccacagg ggagggccgt 72600
   ggcttcagga aacgtgggga acaccctgaa ctgtaaaagc agatgcgtca gcagacaaag 72660
   ggggtcattt cactaaagcc tcagtgaagc agagaaattc cagactttgc ccatcaccta 72720
   ctggcaggct ctccctccct ctgtcccctc cccaccctct cctcaccctt gtctttctct 72780
   gcttcattat ctcacgctct ctctttctct cccgcagttt ctctgtctct cagtctcgat 72840
   ttctcacatc ctgtttctct ttcttcttta ctctttctcc ctctccttca cctctcctgg 72900
   cttcactcca ctccccccac ccactctgtt ccctctcaca ttctcatttg gggatggggg 72960
   gcggctgaca aaccggattg ggggggtctg acaaactgga tttaaatttc agctccgcag 73020
   cgttctcagc tcttggtctt ggaaatgttc cctgagcttc ggtttttctc atctgaaaat 73080
   gaaaaaaata atacctagct ccaagtattg agcttgttgt gaggaatcaa acagataacg 73140
   tttctaaaat tccaaacaca gtgcctggtc cacggcagat gcttgactgg tgacagcttc 73200
   ctttcccagt gaggcccagg gaagtaggca cagtgcttgg caccctgcaa gcatgcgaca 73260
   cagcttcaca aaatacccac agggagccag gagcaccgct tgaattttat ggatgacaaa 73320
   attgtggttc aaggaaggta aaagtaccct ggtaggacac gtaattttta aaaagtctat 73380
   agctattgat attactatat attaattccc attctgtccc tccggtttct cccctgaaca 73440
   ctaatttcca aatccccaac actctatggt ttaaatgata aacgattcac ccaaatactg 73500
   tataattttt tttttccttt tgggtcaggg ccatgctctg tcacccaggc tagagtgcag 73560
   tggtacaatc gcagctaact gcagcctcaa cctcccaggc ttaagtgatc ctcccacttc 73620
   acagcctccc aagtagttgg gactacaggc atgcacaacc acaactagct aatttttaaa 73680
   atttttggca aagacagatc tcactacgtt gccctggctg gtctcgaact catggactca 73740
   aatgatcctt tctcctcagc ctcccaaagt gcttggatta cagatgtgag ccaccgcaca 73800
   tggccatgta caattcttga agaagctgaa actcatcttt ctttctcggt ataagaaaac 73860
   tcaggatatg tttggacatc ccctcccatc tgccctccca gcctcctctt ctccaggctg 73920
   aaccagatcc tgctgcatct ttctcaaaga ctctcttaca tcctcattat ggtttctgcc 73980
   ccagtcctcc atgcttctct gaagtttaga gtcaaaatgc ccttttttgc tttaggggtg 74040
   agaccttctc tagcaccgtg tgtctacaac acagagaggg caaacagaga tagtgcttgg 74100
   tattttattt ttaaaatttt taatcttgat tatattttgc aatgtttata ccattgcaca 74160
   acacacggaa gcattagtca caatttatat agctgatttc atggagctga attctatggt 74220
   gtttatagcc gttgagtgtc ttctcccaga aattggaaag atgccccatt tgctgtaaca 74280
   cacacgacct ccaccccatt cttttccttt gtaacccaaa ccacaatgtg taattgtgtg 74340
   tttattttgt gtttaatgtt tatctcttcg cccaggctct aaggctctat gagggcaggg 74400
   atcacatctg tttagttcac ccagatttca ttatatatcc agcattctac actctgtaac 74460
   tgcttagtct caactctgac cacacattaa gtcatcaagg gagttaacag aaaaaaaaaa 74520
   aaaacaataa acaagcaaaa tgtttactaa tgttccacaa gctcttttga atcaactggt 74580
   ccctgtggca cctggggatc cgtagtcatg aaaatcctgc cagggattct tgatccggga 74640
   tctggtgatc ctcggcagga caactcacca ttctgttcgg ttgcccgctt gcctcctccc 74700
   gctggctcca gcttctgtgc ctacaatgaa ggctgtgccc attccctcac gtgcccgccg 74760
   tggcctgaca ccttcctggg cccaccctaa cacattcctc cgttgctttt aacgactgtt 74820
   tacagctcgc tacctctagg acgtggcttc tgatgaaaac tgtccagacg ctctgcctcc 74880
   ggtgtaattc agagcccagc tctcctcctt aaaattcttt tacgctattt tccacaagca 74940
   tgtgcttctg ttggcagtgc cataagtgtg attgatcttt accataagtt ccttgaactt 75000
   tgctgtgacc tggatgctaa ggtggcggga gctgcctcca tccccacaat ttgactggta 75060
   caacccgaaa gtaggatgat agtctgttct ggtcaaacat taacgtgggg cagggacaat 75120
   ggagagaggg tttggtcatc tggcatgctg cagactgcca gaggagctgg aaaggctgag 75180
   tatgggaaag gggagggatt tcatggcctc ttcgggcttt gagtatttga agaaactgcc 75240
   tatggaagac cagacctgtg ccctgcctcc caagaaacac acagagctta gacggtggaa 75300
   gctatctggg gcaaattcag ctccctgtga ggaaggtctt ctagttgtta ggggtgtctt 75360
   ggtaggtcag ggagggtttt ccagcatcag tcattttcat accaatctct cggtttttgc 75420
   catattcctg taccacccgt gcttttattt acctaatctt ttttatttac cgaattttta 75480
   acctaatatt tcctttaaaa caactttaaa ttgacacact ttgtttcatt cctgttttta 75540
   aaaagtacct atggcaaaag atatgaatag acacttctca aaagaagaca tgcaagccgc 75600
   caacaaacat atggaaaaat gttcatcatc actaattatc agaaaaatgc aaaccaaaat 75660
   cacagtgaga tatcatctca caccagttag aatggccatt attaaaaagt caaaaccaac 75720
   agatgttggt gaggctgcag agaaaagaga acacttatat attcttgaga atttaaatta 75780
   gttcagccac tgtggaaaga agtttggaga tttctcaaag aacttaaaca gaactaccat 75840
   ttgacccagc aatcccgtga ctgggtatgt acctaaaaga aaacaaattg ttcaaccaaa 75900
   aagacatatg cactcacatg ttcatcatag cactattcac aatagcacgg ttcatggaat 75960
   caacgtaggt gcccaccaac aatagattgg ataaagaaaa tgtggtacat atataccatg 76020
   gaatactatg cagccatgaa aaagaaggaa atcacgtcct gtacagcaac atggatgcag 76080
   agggaggcca ttatccaaag caaattaaca caggaacaga aaaccacata ccacatgttc 76140
   tcacttataa gtgggaacta aacatcaggt actcgtggac ataaagatgg caacaatagg 76200
   ccaggcacgg tgctgatgcc tgtaatccca gcacttgggg aggctgaggc gggtggatca 76260
   tatgaggtca ggagtttgac accagcctag ccaacatggt gaaagcccat ctctactaaa 76320
   aatataaaaa ttagctgggt gtggtggcac acacctgaaa tcccagctac tcaggaggct 76380
   gaggtgggag aatttcttga acctgggagg cagagattgc agtaagccaa gaaggtgcca 76440
   ctgcattcca gcctgggcaa cagagtgaga ctctgtctcc aaaaaaaaaa aaaaaaaaaa 76500
   aaaaagatag caacaataga cactggtaac atacccaggt aacaacatac ccaatatatg 76560
   catgatatac ccacacaaca tacccaggta acaaacctgc atttgtactg cctggtgggt 76620
   gtggtttgtg aaccggtatg tgtggtctgt ggtatgtatg gatgtgtgta gtttgtgaac 76680
   tggtgtgtgt ggcatatggt gtgtatgtgg gtaactggca taactggggt atggtaggta 76740
   tgggtatgtg tggtatgtga actggtaagt gatatgtatg agtgcgtatg gtgtgtgttg 76800
   cgggtggtgt gtgaactggt gtgtggtgtg tggtatgcat cggtgcatgt gatgtgtgaa 76860
   ctggtgtgtg tggtatggta tgcatgggtg tgtgtagtgt gtgaactgat atgtgtggtt 76920
   tgtgaactgg tatgaactgg tgtggtgtgt gatgtgtggt atgtatgggt gtgtatggtt 76980
   tgtaaactgg tgcatgtggt gtgaactggt gtgtggtatg catggagatg tgtggtttgt 77040
   gaactggtgt gaattggtgt gtggtgtgtg gtatatatgg atgtgtggta tgtgaacttg 77100
   tatgtgtggt gtggtttgga tgtggtgcat gtggtatgtg cagtatgtgg tgagagtgtg 77160
   gcgtgtggtg tatgtggtgc atgtggtgtg tttggtatgt ggtatgcgta tggtgtgtgt 77220
   ggtgtatggg gcatatgtgg tttgtgtgtg gtgtggtgtt tgtagagtgt ggtgcgcatg 77280
   gtgtgtgtgg tatgtggtgt gtgtgtggca tgtggtgtat ggggtgtatg tggtttgtgt 77340
   ggggtgtgtg ggatacgtgg ggtatatgtg gtggggtgtg tggtgtctgt gatgtgtggt 77400
   atgtatgggg gtatgtggtg tgtgaattgg tgtgtgtggt ttgtaaactg gtgcatgtgg 77460
   tgtgaattgg tatgtggtgt gcatggaagt gtgtggtttt tgaactggtg tgaactgctg 77520
   tgtggtgtgt ggtatatatg gatgtgtggt atgtgaactt gtatgtgtgg tgtggtttgg 77580
   atgtggtata tgtggtgtgt gcagtatatg gtgtgtttgt ggcgtgtggt gtatgtggtg 77640
   catgtgtgcg gtatgtagtg tgtgtgtggc gtgtggtgtg tgtggtatag gggtatatgt 77700
   ggtttgtgtg gggtgtatgg ggtgcgtgag gtgtgtgtgg tgtgatatgt ggtgtctgtg 77760
   gtgtgtggta tgtatggggg tatgtggtgt gtgaattggt gtgtgcacca tggtatgtat 77820
   gggtatgtgt ggtctgtgaa ctggtagggg tgctgtggtg gggagtggtg tgtgtggtgt 77880
   gtggcatgta tggatgtgtg tggtgtgtgt cttgggagtg agggtgctta tcatgagcag 77940
   gtgctgcaga agccctcagg gaagatgact tcactgggag ctgagatgac ctcactgggc 78000
   gccctccttc agcagatgcg aggacaggcc cacaggccta aagtcaatta gaggcagatt 78060
   tccaagcaga atctacttct cctgacctct gattcagtgc cttttcctga caattttatc 78120
   cctgcgaccc cagccttgag aatgcaaaaa actaccccca acttcagcct gaggtcatga 78180
   aaccaaccag gagtgcaggc cgtctagggg aggtgcgagt gcccacctac tgggcagcca 78240
   ctctgagggc aaagtccacg ggagacacag cttcagccac caccggggct gcgacctgtc 78300
   ctcaggatcc tcctcagccc cctccaggag tcctcagctt tctgctttta gccccaaacc 78360
   cagattcact ttcccaggcc attcaccgtc tgaggcagcc cagtccgctt tgtccttcct 78420
   ccttcccgaa ggctctcagc ccctgggcag tatcagaatt tcactttgat ctccttttcc 78480
   ctgggagcca gaatagctta gtctcttcaa aaggagagtt agaaagtgtg ccgttcatct 78540
   aataaaaatt aactcctgag agttatccaa tcccaataca ggtcatttcg ctggaggcta 78600
   ccccgactag gaaacacatt ttaggacatc agcagaggta aagatgagag caatttgctc 78660
   aggcttttac ttggggttaa ggatcactag gccaccagga ccagggatgc cagacaacct 78720
   caggtttctt tccaagcttt ctccaaacta ggcctcactt ttatcatttt cctacccctg 78780
   ggctctatga ttttgtggtg ttgcttttgt gttttcaaaa acactgcatt cattcattag 78840
   ctctgcactt tccctcatgc aatgcacaca gaactgtaaa gacaaatgat aaagcccttt 78900
   cccgcttgga tcttgtgttg ttggccaggt tcattcaaaa ggccattgtt tgatggtgat 78960
   taagtgtgtt tgttttgaat attcacctaa acaaaaatga tcaatttaca caattcttgg 79020
   aattcatggg tgtcttggga aaacttgaag ccacaaaaaa tgttcaatac ataaatgcca 79080
   aggacctcct gctgtcaatt aagaccctca tgatagtctt ttgcttattt gctcgttaat 79140
   tcatgtaaaa gtggtttgtg agcaaattaa gcatcagttg gcatgccgga catggacaca 79200
   tagatacagt ttgtggtctg gaggggaggt ggaaatgaga gtctcacagg ctgccctcgg 79260
   cccagaactt ctgtgtggag gttttgcata aaaatcagaa tttctggatt cttataaaaa 79320
   ccctaaagtt ctagccccat tagggccact gttccactca cagccctgag taagcctccc 79380
   ccatcagccc aggcatggac cctttgtcct gcctctgttg tcactctccc gactgtctca 79440
   gacctaacca gctcctcttc tgtttgtgac ttgcccttat gggagcctga gggctcctac 79500
   ctactagatg aagctgatgg ggacattctc ccaaatgaga aagcgatgga aagttgtgac 79560
   catcagagga agatcagaga tcaagccttg ttgatgtaaa aggaattaaa gttgagaaac 79620
   catttcctgc aaccagaatt gtgaaagcat acaacgtagt accaaaggaa aattaccgaa 79680
   gcctccttcc ctgaagaacc ttcaggaagg ggaggccagc cgtctgacgt gatcacagtg 79740
   atgtcttgcc ctgaaacaag aagcattctt cccaggtcct ttccgccgga ggattcttat 79800
   tctgtgatca atgctgagct atgtggatga gtgtttgcgt ggatttcttt taaaactata 79860
   ttgctaaata atcttgcccc ctggccccat gcacgttctt tttttttttt ttttttgcac 79920
   ccagataaga aaaagacttg aatgtatttt tttctataaa atgcagtcaa tattgttctg 79980
   tgagggggaa agaagtagtg gaggatgaag atggaacagg ggatgaggtg gtgaccagca 80040
   tgccctcatg gatagacact gagcagggag aggcagggat atgcatggag taagccctct 80100
   ctctctctag gacaaagcaa attatgtctt catgccagtg gactctgcat attcattgaa 80160
   aaaaataaga tttaattaat ttagggaagc aatatgggct gaccagacag ccaagttctt 80220
   caagatagga cagtaaaaca ttttatgtga gagctggcca atgtctggat ggaagaggga 80280
   gggccaggga gactggagag atattgaggg ggtgaggagg tttgaggaat ccacagtggg 80340
   gtgagcttca tgaccagtgg aatgaaataa gggctattct ggatggacga ccaatgtcag 80400
   tgcaaggccc tcaatctctt gggataacac tggagttgga atacatatga aaaaaaaaat 80460
   ccacaagtat aaaagaagta agcagaggcc gggcatggtg actcacacct gtaatcccag 80520
   cactttggga ggccaaggcg ggcagattgc ctgaggtcag gagttcgtga ccagtctggc 80580
   caacatggtg aaaccccgtc tctactaaaa ttagccaggt atggtggcgt gcacctgtaa 80640
   tcccagctac tcaggaggct gaagcaggag aattgcttga cccagggagg tcgaggttgc 80700
   agtgagccga gatcgcacca ctgcactcta gcctgggcga cagagtgaga ctccatctca 80760
   aaaaaagact ggaatagagt acatttgtca taggccaact gcctccatgc tcttgtctca 80820
   cagagatgtt tataacaagc ttgagtagct acaactggat actttggtct atatatgctt 80880
   tcctcgtgat tctttttttc ctccttctta gcttaaacaa atgcacacag agggaagtgg 80940
   ataaactatt agcagagtaa gcacttgcca agaataccgg gggcaccaaa aacacataaa 81000
   aacaggcttg gttgggagcc cgatgtgggc agatcacaag gtcaagagat cgagaccatc 81060
   ttggccaaca tgatgaaacc ctgtctctag gaaaaataca aaaattagct gggtatggtg 81120
   acgcacgcct gtagtcccag ctactcggaa ggttgaggca ggagaatcac tgaaacccag 81180
   gagctgcagg ttgcagtgag ccgagattgt gccactgcac tccagcctgg cgacagagtg 81240
   agactctgtc tcaaaaaaaa aaaaaaaaaa aaaaagcgag gggatcttgg ggaacctagg 81300
   aagaagttac ttaaatttca gcacatgcta tatagttttc aggattagtc cctctctagg 81360
   tattcatctg gggctggcgc ctccttggct ggcaggggct ggtgatcggg actccccagc 81420
   gagacagatt gcacccagag cagtgaggcc ccgagcgctt cctggagcct ggggaagatg 81480
   gcattcattt ttaaagtcca tttattgtgc agttgttgaa agtgggacac agaaatttgg 81540
   actgatatgc ctaggccaca tgactactga ttgggccagg gtaaatgcag tctttttgtt 81600
   gatggagaag gtcttttctg ggcccagcac aaagcaatgg atcttcagtc ttaagaaatg 81660
   cattgaaaac catccttgtg cagtttgggg tccctggtag gccaagagct ggaaaggccc 81720
   ttagactctg agtgctgtag ccccccttct aggtgatggt caaaacttga aaacggctct 81780
   agactctgga cagaaatgca tgagaattga gtgttcaaag cttggtgctt tgacttcttt 81840
   taactttctg tagactgaaa tatgaataag aggaaggcat gaggcccctc tcagtttatg 81900
   gagccatagc tccaaacctc ccactttcca acccagcaac cctgtgtgtc ctaggaattg 81960
   tagcgggtgt cccagaatgc aaaaatcact ctgctagaac tggaaaagga gacgcctgcc 82020
   ttttctagca cgatctttag taaatagatt caggaaatgt gggtcctggt cagagctggt 82080
   gccacaagct gagtgccttc aggcagcaga cttagcctct tcaagtccaa tctcatcagt 82140
   agtatcaggg ggct 82154

<210> 6
<211> 99791
<212> DNA

<213>  Homo sapiens
<400>  6
ttcagtgagt cgacaccgcg ccactgcact ccagcctggg tgacaaagca agactctatc      60
tcaaaaacaa acaaacaaac aaacaaaaaa aaaaacagag agagaattac taatattttc     120
ttctccaacc ttaatgctca ctccctgagc tatgtggatt atactagtga aagtactgcc     180
ttaacccact aatgtaaaca gattcctttt aagaagcctc cctatcgaga atggtctgta     240
agagacaacc atgtcaacaa gtaaagttcc ttctgccttc cctcctgggg tgggctaaga     300
tggacccttc tcaccattcc caattacctg caggaaggca gcaacagaat agagaaggag     360
ggtaacacag tttcctgtac tttaaatacc tccctgcctc cgcaagtaaa acttgagcca     420
tgccaatgag ggaaaaagtg gccgcgattt cccaggtggc ttagtaatac acagttttct     480
gacacactgc aactccattc tttcctaaaa gttccttcca agcctgtagg cactgtaaga     540
caaacagcta agcacttcct gagtgcctac tatttgccag gctgtatgca gcaccaggaa     600
cacaaaaata aaagctatgg ccattcagga acaagatttc aaaatgaaaa ataggaggaa     660
aatctaacat ggatggaaaa aagccacact tttccatgct tcaagtttag tttccatttc     720
taaaatgtct gtcctcatat gttagccaga aagccagttt ccagcaagac aagcagaaat     780
aaatattatg caagtcaaac ctacaagaat gggtcttcaa gaaagggaaa taattttcgg     840
tcaaaatcta aaaatccata ttcaacaaaa aaagcacaga ttgagaaact aattcaatag     900
caaaaaagga aaattaccat tgatccaggt tgccattttt aataaaaagc agtaaggtct     960
taaagaaaat attagcattg aagttcaacc aaataaatta ctttgtcatc aaaatgaaga    1020
atgccagaaa gttctctgaa atacaatttt taacaaaact gagaaagcag aggaagtatt    1080
ttttaatatt acaaattttt gacaccaaca taagaattcc atgatatcct aaagagcaat    1140
gaacccaggt aatgcagttt ctagaaattt taaaacacta ccaaataata ttgcttcttg    1200
cattaagtat agcattggtt tgctagaata atcctaacaa tgacaatgca atgtacttta    1260
aattggggtc aaaaaacttt tttcttaaag taccagatag tcaagctttt aagctctatg    1320
ggtcatccac agacacactg caactataca aactatatca cccctgttgc aactacacga    1380
aaagcaggaa agcaaccaca gaagacagac aatacataaa caaatgagta tgactgtgtt    1440
ccaataacat tttattaata aaaacaggca actggcccat gggtggtaat ttgctgaaac    1500
ttggtttaaa ttattattag tatctacctt tttttaaaag atgatgtttt ttagggtctt    1560
tccatcatga aaattatgtt tttatgaaag tgatttggga agtattaaaa tacagaataa    1620
agagcaagcc ttgggctatg aagtcaaata ggactgggtt ccaggactgc tgtgtgactt    1680
tgggaaactg acctgccatc tctaggtgtt agtttcctca cttgtaaaat taagatgctg    1740
acagccctca cctcataggg tcactcactg caaggaccac acaagtgaac atgcatacag    1800
cacttagcat agttatggat aaccctgtac ctaccttatt atagcaattg ctatcaactt    1860
tcttctagct tatcattaat gatactataa aaacacccag cataacgcca gcataaggat    1920
gtgaatgaat gagcaaatga aaggagggag ggcaatggca aaattttacc ttggtttcca    1980
agctaaagta taacctgagt tttttaatgc tttacaaaaa ggaaaggcaa tcagaaccat    2040
aattaatatt taagctgtac tgacaggttt tccagggaaa attctataga acttgttatt    2100
ctccaacttc caaaaataag caataaattt tttttaaaga gctgttattt taagacttta    2160
tttgaaccaa agaatgtgtt tatggtaaca acatacatgt tgtaaattaa tcagtttcaa    2220
tgttataaaa taatacttcc aagaataaat gtatcatata atctatgtac ctcatgtcga    2280
taatttaaat aaatacatta aataaataca tcattccgaa gtccgcttta tcattgtttg    2340
taaaatccaa ttgtcatact ctggtatttg ttctccaaaa gaatcatcac tttcatattc    2400
actacagcat gagatgagat atttaataaa taataggaca tcataatcca gggattaagt    2460
agtttctttt agcaaaattt aatatgaggg tagcatatgc cagccgttcc tagttgagca    2520
acaatttctg tgttgggaga ggataacaaa gccaagtatt ttcccataag aagttcacac    2580
tctaaaaggc caggcgcggt ggctcacacc tataatccca gcactttggg tggctgaggc    2640
aggcggatca ctaggtcagg agatcgagac catcctggct aagacggtga aaccccgtct    2700
ctactaaaaa tacaaaaaaa ttagccaggc gtggtggtgg gcacctgtcg ttccagctac    2760
tcaggaggct gaggcaggag aatggcgtga acccaggagg cggagcttgc agtgagccga    2820
gatcgtgtca ctgcactcca gcctaggtga tagagcgaga ctccgtctca aaaaaaaaaa    2880
aaaaaaagaa gttcacactc taaaatgaag agaaatgaga aatgtctatg tgcacacaac    2940
ttcccacata cacacattag ttagagacca cagtcatgcc caccctccag ctcacgtgac    3000
atttcaaagc agagatgaga cttaaaggat ggaagaaatg gcgtttgttt catggatggg    3060
gtaacggagg cttttagggg cttatgggat ggacaagtaa aggatcaatg agacagaaag    3120
gcaaggcaag gcaaggcaag ggactcgatc tcaggaagtg gtgagtggag atgaaagaag    3180
tccaatggaa tggcagtcac tggagatata tttaaagaag agggtctttt tgtttgtttg    3240
tttgtttgtt tgtttgagat agagtcttgc tctgttgccc aggctggagt gcagtggcat    3300
gatcttggct cactgcaacc tctgcctccc gggttcaagc gattctcctg cctcagccac    3360
ccaagtagct gggatttcag gcccgcgcca ccacgcccgg ctaatttttg tatttttagt    3420
agagacgggt ttcaccatgt tggccaggct ggtcttgaac tcctgacctc aaatgctctg    3480
ctcatcttgg ccccacaaag tgctgggatt acaggcgtga accaccgcac ccggccaaga    3540
agagggtctt ttaaagaagc aagataggaa gatccgttaa agagagaact gcataggaag    3600
gaaaaggcct ggctaggatt ccctgaacct tctcatttca ctactctctt gttagtcatc    3660

aatgcagtta agaatcaacg caatcaaatc gaatcacata gtgattaatt tgtgccacca 3720
atagctctaa ttttgttaac ttctcattcc aataaaaata atcatctggt taatccattt 3780
cttcagcggg attacttgtt tgcttgtttc tttagctata ataaactctg aatgccagag 3840
tcaccaaact aagcattaaa caaactgatt gacaattcgg aagcatagga ataattacag 3900
atgatgctag gcaatatcac tagattgggt tttcatgaag ctcgtgactg gattttgcca 3960
caaaaatcac taactgcttt ggcttccaga gaatggcctc atggataagc cccaagttcc 4020
aaaaactaca aaatgtcaaa atcacattca actaattaat aattttatgt cacaataaga 4080
attaggaatc tctagaaggc tgggtacagt agctcatgtc tgtgatccca gcactttggg 4140
agatcgaggc aggcagatca cttaagccca gcagttcaag accagcctgg gcaacatagg 4200
gagaccccgt cctacaaaaa atataaaaat cagctgggca tggtggcaca tgcctgtagt 4260
cccagctacc caggaggctg aggtgggagg attgattgag cccaagagat ggcggctgta 4320
gtgagccatg atcacaccac tgcactccag cctgggtgac agagcaagac cctgtctcaa 4380
aaaaagaaaa gaatttctcg gaaagtctta atttttatct aaacctatct gaaaattcaa 4440
tgatgaattt tatgaactta gccctgaact gacattcctg gtgaaaactg caatcattgt 4500
aggacaagag ccgtcgtgtt aaatgctaag gctattttat acacatccaa tattttggga 4560
gcactgcaat taaaagtctt ctcacactaa aaccaatgca caaagcacct ctcaggttta 4620
agcctcacta ggctgatgcc tgaaacacag caaaatgctc atactcttta tacacatgtc 4680
caaaggtcta gctatcaaaa gacagataaa aaggagcatt ttcccccaac tgatggaagc 4740
aagtccaagg tgaagaaaga ggatagaacg agaaggtaaa ccatctctca aaggtgagaa 4800
atcaacacag agtcaaccaa tgtcttcatc tcaacccaac ccaccaagca atgtatcaga 4860
ttggattgga gacagattgg agactctatg aaagaactgg catgcacaac ccaagaacct 4920
cctggattct tggcccttct tttaaagaag gggggtttct agacaggaat agaacccaca 4980
gagtcatgga agccagcccc ctaccataaa ttgttcataa ccacaagctt attggtcagg 5040
tttataaagt gattcagact ctttagaggc tacaggaagg gtgcagataa gaggacactt 5100
atgctggggt ggggaattat tggagaggcc tccaacaatg agatatcacc tataattaca 5160
ttaaagattt aaaaacatga ttgaatcaca tgttaacaat ctcctaatta gagctctgac 5220
tctccttaga atgtccaaca tccactgcat ctaagaagca ctagtattta ttatgtagta 5280
ttgtttgtgt caaattccca aaaaaggtta tcttgaaggt taatggattt aagtagctgg 5340
aaaccctgat tagagttgcc catagaaatc tgaattctgc tcaaacagtt caaagctgac 5400
aacaaaacag tgatggactc tgaaagtgaa gagacccata gagttcacat gtaatgttaa 5460
aatgttcggc acttcacttc tcaactgggg tacatgtaca cctaagacct acatcttaga 5520
agtttccaga gttacagaat gaactggatc gtgtctcaga ttaacaattg attcaaagaa 5580
tggggataga aagggagcca tgacaaaata aaggaggggg ctgttttgta tcacagcaaa 5640
caaggagatc ctgtgtaaaa gaaattatca aaattgttaa tgccagaggg tagtggctca 5700
tgcctgtaat cccaccattt tggtaggcca aggcagaatt gcttgaggtc agaagtccaa 5760
gacaggactg agaaacacag tgagagtgag accccatctc cacacacaca aaaacatctt 5820
tgcattaaaa aatgaaacta gaggctttaa agaactgctg caggctacgt gacttaaaga 5880
atataagttc tctgccttct ttgtcatcag gagtgcttct gtgattacgt ataggaaggc 5940
aaaccttaaa ggaggtaact gaaatcttta cttcaacata ctcaattgta tgcacacagc 6000
ttggtaagca aaataagtca caaataagca atattgcttc aaaaaccttt tttaaaaaaa 6060
gatacataat caattatttt catattaata gagcaaagga ataccatgaa taatccaaga 6120
taccaggtaa tccagaagtc agatactgag aagtgaaata catcagataa tttttacagt 6180
gtgtctatga tcttaattac attttactaa gcctaatact agtttttctt tcttttttag 6240
aatttctaga ttacattatt tgaaagtaaa taatggcttc aaatcatgtt aggcaacaga 6300
attcccagga aggagtgaag gtaaatcaga tgactgaact atattacaat ttaagctgtg 6360
atgtttgcac aactataaca atattataaa tgagcttgag gtttcttact gaagatgttt 6420
aatatttgat ccaaaatatc gagcagtttt gactaagcac tgtaagcaaa gaaatacgct 6480
atcatagcag gacacctgta agtttccatt gtataagaaa atgtttcctt tgtttaattg 6540
gagagtcaat taaacataca aatatctgaa gacattatta tgaaggctga tttggaggaa 6600
atgtataatg cagatacatt ctgggtgtaa gtattaaatt catacaacca ttctggaaaa 6660
taattcaacg ttgccaagtc aagagcattc cctaaaaccc atgagttcct ctcccaagta 6720
aagacatgag aacaattctt gcacatgtgc aacagcgtgc atacacaagg gtgtttgcag 6780
gacccgccca tgatagcaaa gcactggtaa aaaatcaaat gtccagaggg agtatactca 6840
gaccatggaa tactgtacag tggcaaacca aaagaaagac acctgcatcc acttagaaga 6900
atattacaaa actaatattg aagcggggaa aagcaagtca gaaaataaaa tatataacat 6960
cattccactt ataaagaaac caggaatatg tacaactaag caatatatta ttaggggata 7020
aagagatgta tggtaaaatt ttatataaag aaaaaacaag ggaataataa atgtaaaatt 7080
caggaaagtg actatctctg ggggcaggaa gggagggagt tgcaactgag aagcgcacag 7140
agagaacttc aaaattattg ataactttca acaaagttgg atgttagaca aataggtgat 7200
cattttatta ctattattta aacatgctca taatttaaat tcacatgtat agctatatat 7260
tatgtattat agatataaaa caattcagag gtataaaatg caataaaaaa gtgtacaagc 7320
ccaaatctct agcatactgc caatcaaaag atgattgtta ttcataaagt atctcaaaaa 7380
ggaacattag actatttcca gaaatcctgg ttgctttatt attcatttat tatttattac 7440

cccaggaaga taaattttag gaagtttcta ctttgagtgt ctttcttcta agttgcaaat 7500
acaaataaat gcttatttgg aattataact atactggaag ctcatgtttc aaataaagct 7560
atttatatat agcttcatca ttgtttgaat gcttatcatt gcagcctcat tacaaagaac 7620
atctaaaaga tttttttcca acctaagaaa aaacagtgct ataataaggt atcttacatt 7680
catgggtaaa aatactcctt ttagagaaca taatataatt agtttctatt ccaagtctgt 7740
cagtgtgcac tgttaacctc tgacaattat gcatgaacct tttaattagc atgttatctt 7800
tggtgcaatt aataacttgt acttcatgtt ataggcaaat taaagttttc ccactaaaac 7860
caaattcttc agcatagttc taaaagtgtt ttgccattta aatgaaattt taacattcag 7920
acctcttgct cccaaaacac ataagccatt ttttccaaag ctagacagct tttctctatc 7980
cacgttcttt caaatatttt acctaagaca gagaatctca tattaaattc aatttcacac 8040
ttgccaatca cacacgccca ctcacaattc ctccagatga tgcaatgtgg cctaatgtgt 8100
atgcttatgt atgtacatgt gcacacacaa acaggcataa tgtttaaata catataaaca 8160
cattcattgc taggtattgg ttaaaacaaa caggcttaca gcataccaac ttattaaaca 8220
taatgattga tattagtagt ttcattaatg taattaacct ttagatttga attatcaagt 8280
tcaaagtcta agaccgagag cagtggttct caggccggta caataatggc aagtcatggt 8340
attcaagaga tgagtctaaa aggcccctca gtcctgcccc taccagaaac cagagatatt 8400
tgctgctggt aataatgagg agggggtttc taagtacaag atcagctcta ttctttcatt 8460
ccctggaaat ctgccctcgc ctctgatacc tcaaaaaata tctgttgcat ttccaaggga 8520
aagaacaatg atataagcct tgctgttaaa aaaaaaaaaa aaaatgccac tacttacaag 8580
ataaaaggca aggtgttttg tttttttccc ctttctctta gactaggcat gaataatctc 8640
aaaatatttg tttgtcaata aagtgattaa aataaagtga tagacaagac ataatgatta 8700
gagaaatgaa tcaaagtaat atttgggacc agggtcttac aaatctagag taagtatgtt 8760
gagcagaaat agatactaaa aatttacatt agaacaaaat aaaaatggat ttagtaatgc 8820
tggatttaaa ttggaaatag ctgtttaaac tcatgacctt aaagaaaatt tgtcttggcc 8880
aactgcggtg gctcacgcct gtaatcccag cactttggga ggccaaggca ggcagatcac 8940
ttgaggtcag aagtttgaga ccagcctggc caacatggtg aaatcccatc tctactaaaa 9000
atacaaaaat tagctgggca tggtggtaca tgcctgcagt cccagctgct tggggcgctg 9060
aggcaggaga accgcttgaa cctgggaggt ggaggttgca gtgagccgag atgggaccac 9120
tgcactccag cctgggaaac agacagagaa ggaccctgtc tcaaaaaaaa aaaaaaaaaa 9180
aaagaagaag aagaagaaga aaaagaaaat ttgtcttctg gctctctccc taaaatgaac 9240
taactgggga atcatttcta ccttccagaa gcatttgaat ggaacagctt gatatcatgc 9300
agagcaccta aattttggat gccactatgc tctatcaaag gaatcagagc tcctcagaga 9360
atagctgact ccatgtattg ggtcatgaga aaatcaagat ccactggaac ttgtggttcc 9420
cagaaaacaa cagtgctttc aagaatgcct gggggtaatg ctaaggggac caaagaggac 9480
atttaaaggg actcccgcag gtcatgctgt gaccatctga gcatcgacat gacaatatga 9540
attttaatta attagaacaa gtgcatccca taaaagctga gagtccagga tactcaaaga 9600
gaaacagtaa ggctggctgg gcacggtgac tcatgcctgt aatcccagca ctttgggagg 9660
ccgagaaggg tggatcacga ggtcaggagt tagagaccag cctggccaac atggtgaaac 9720
cctgtctcta ctaaaaatac aaaaattagc tggctgtggt ggcaagcgcc tgtaatccca 9780
gctactcggg aggctgaggc aggaaaattg cttaaacctg ggaggcggag gttgcagtgg 9840
gccaagattg tgccattgca ctccagctct ggacaacaga gcaagactcc atctcggggc 9900
aggagaagaa aaggtaaggc ctactgaaaa cacatttgta tcataatacc aaagaagagt 9960
gactataaga tgtcctaaaa atagatggtg aagtatttaa agattaggat actctctttc 10020
tcctgtgaag tatgtgcttc tttatgagga aagaatcagc atttttcttt ttgtgtgttt 10080
gtaggacaga atgaatgcag agaattctaa cccaaatagt tccagaaaag taggaatata 10140
ccatgaaaga tggcagcaat agtttgaaga gtgagtcaga aaacaagtag tttgagaacc 10200
gttagcctat attctaatga cttgaaagtt tagtgcatgg agaaaaaata tttaatgtaa 10260
agtcatgttt gcctttataa tttcaaagaa tacttaaagc agtatgggaa atgcctaaat 10320
ggacacattc agaactcctg agggaacgag cagaacacag agggcaagag aacttggaat 10380
cagaaattcc acgttcaagc accacgttgc ctattcctca actttaagaa tgatcaatcc 10440
tgccatatct gcctcccagg gtaagtctga ggaactctcc ctgtggttta tgtttgttca 10500
actgtatttg caggtccatc tttaagatag tttgcccatg aagatagcaa ggtacttttt 10560
atgaccctat ctcaaaatca ggacatattc tggtaaagac acataaatat tggctcaaat 10620
ggattttgtt ttacctgaat caaagggatt ttgttttacc tgaatcaaag ggatgaaaat 10680
ttgccccaga aatagttgac caagtaccag gaggtttcaa aggaaaaact aggaagctga 10740
cagattgaat gttcaaaatg attccaaccc aaaatgctct attacccaga acttgactcc 10800
agagataatc caaccaacat gaacctctgt tttctattta ctcagagcat agttgctgct 10860
caatacatcc attcatgcaa caagtgccat attcatgatc ccatgccagg cacgttctat 10920
gtgcttaaga tgcagttagt gacaaaagag gccaaagtcc atgcccttgg tgactgggga 10980
caggcaaagg tggatggcaa taaataagaa tattagatga aggaagtaaa ataagactat 11040
gtcacttaaa atgacttggt gggtagcata cttttgaaag gcttctcaga agaggtgaca 11100
tttttgagct gagacacaaa tgacaaggag ctggcattac aaagagctgg ggaaagaatt 11160
ccttgcagaa gaaagaatca atggaaaagc tctgagttag gaatgtggct ggcatgttat 11220

taaaaaaaaa gaagaagaag aaaagaaggc ttaaacaaag tgaatgccaa gaagtgttaa 11280
gatgtgagct tgcaggggaa ggaaagagct agagaacacg gtgtgagtaa cgtaagcgaa 11340
aacaataaat aatacctttc ttcacatcca aaagctgatc agatttcttt ttccaatgcc 11400
aaaatacatg taagtacata atgtcacgtt gcataccact gcggtggaac acaaggaaag 11460
aagagctata atgcaaacaa acagcaactg actctttggg gagcttaaga aaaaagaagt 11520
ccaggcacca acacaaaagg ctgaataaat cacaacctta ctcaaagaac aatattaagg 11580
gcataggctg tagaaaaggc aactggttca atgcttctca tgccctctga gagctatcac 11640
aattaatggc atcatcttcc aaacaacagg taatacaatg ttacttctgt gttatagaaa 11700
catttgggtt ataactggag atgtatggta tattcatcac aaagatgata atcatttcct 11760
ctttgtgtat tcaatggaca gaaatctcta agactaggcc tctgcttccc aaatattcat 11820
gcaactggaa ctgttacaag ggtaaaggca tgaattaaaa ttatagaaat ctagatgaga 11880
tggaaattga aaatacacta aaacctaaag tctcctatag ttaacatggg ataatatatg 11940
catagttcct aacagcgtac ctacaatcct ataagggctg agtaaatgtt agtttgcttt 12000
ccaaagtaaa agtgacctga tttttctgtg ttgctccccc ttgagatcta aagctaaacc 12060
tctactgaat aaaagcagag tgacttacaa ttgagatagg ccaatctaaa actgaaaagt 12120
ctaaaatgtc aaacttttaa aataagggga tgttcactgc ttatttgact cattaaaaat 12180
aaaaatacct agtgattgtg aatttaaaac tgtagactta tgatttgtgc attttatata 12240
tgttattctt caattagaag ttcaagaaat aataatggga ggcctgtatt aaaccattta 12300
tataaaagtt tctcaaccat aatggaaaga tcaagatgtc tagcataatt tgaaagtatg 12360
tttctatttg acagaaacct tggcttggaa aagttaaact ctaaatattt gtacagaaag 12420
aagtattctg ggtttcattc attaaaagtc attgaccact gcagaaaagg gagagcaaga 12480
gatttacata tatttttctt gacgttttat atgcattagg cctggcaatg aacttgaggt 12540
aggtattact atctccttat ttctaaggga atcagaatac tgtaaacaaa gacatggagc 12600
taaataatgg agtcaaatcc tggcttacta ggctccacat ccatgttgct ccacttatca 12660
gacggccacc caaacataca agtttaacaa attccaaatt attggggcct aaatagacac 12720
ttttttgttt caatattgcc cttcttccct tgtattctga atatcatgtc aaccatgaat 12780
cataagccaa tgcttacaaa tttaacttca cattatgagt tttgagccca actgatgcct 12840
aaccactaaa ccattcatat aacacactga aaagaagcca tttaagaaat gagaaaaata 12900
ggtattgttt tctctgtatg gctgtaaaag ctgagataag aacattttgg caagttcaat 12960
agagccgaaa ggcttgtgaa ggctatggga ggatgagtat attggaagaa gatcagtagg 13020
tcaagaatct aagaagagct atttatgtgg gagacaggaa ttaccttgaa ggaagagcag 13080
agccccagaa gaagaaaaac agaatataac agctaactat aaaaggaccc agggccaggt 13140
gcggtggctt aggcctgtaa tcccagcact ttgggaggcc aaggcgggtg gatcacgaga 13200
tcaggagatc aagaccatcc tggttaacat ggtgaaacct cgtctctacc accaatataa 13260
aaaatcagcc gggcatggtg gcaggcacct gtagtcccag ctacttggga ggttgaggca 13320
ggagaatggc atgaacccgg gaggtggagg ttgcagtgag ccaagatgac agccactgca 13380
ctccatcctg gaccacagag cgagactcca tctcaaaaaa aaaaaaaaaa aaaaaaaaaa 13440
aggacccaag agaatataag tcccatgaaa acagagctcg agtctgtctt gttttgtcac 13500
attacctatt tgatactcaa tatgtaattt aatttctgag tgaataaatt aagcaaatga 13560
ataacttaaa agtatagcta tgaatgttaa aaaaaaaaaa agaaaaagaa aaagaaagaa 13620
aaaagaaaaa attaagtggg gagaggcatg cctatggcct tgtccttggt cctgaataac 13680
tgtttggcca gatagtatgg tttcctaacc ctagctcatg gtgttcaaat tctactagtt 13740
tttctttgtt tgaactttcc atctccatga aactaaaaag tataattttt aagctgcaga 13800
acaaatcatt ccatgtctta ttttctgagg ttacatagct ggctgcatgt actttcaaca 13860
attctatcat gctttgacac ttagaaatat tttccctacc agctttggga agctgggcgg 13920
tttgtgttta ccatcctctc ctgatgttac gctttagttt aaaaataaca gtcattatca 13980
ctcaattatt cagtgatcaa gtgtaacacc aggtgcaaac aaaagttctg agaacactgg 14040
aacatgctct tgttactgca aacgatttaa gagctttgaa tttatgaagc cagtgatcat 14100
gttactctaa attagaagca ggtaaaacag aagaatggca taagctaaat tccttctttt 14160
cgtttcttta cttccttttt ccttttaagg atatctatag caaactatat tagctttcta 14220
ttttctccac tatttgcact agcattaatc aacatccaaa aagtgtacaa attattttat 14280
ctttataatc aatcttctga aatgctaact tgaaaatata aacagcacaa ttactttttt 14340
ctcttttctt tcccatttga aatgtgtgct tgtgtgtgtg tgtgtgtgtg tatctgaatc 14400
tgtacatata aatcaaatac acacacatat acttgtttta aaaaaatcct caaagaatca 14460
atcacatatt acagaactga aggaatattg gagttcatct ggtccaaagt gttggtattg 14520
taaaatcact ttcaaatttc acagttatcc aaatggtaat gaatattaat acctaagaaa 14580
atcaatcaaa gattaacatt aagacattag attttaaaaa tcattgcatt taatattttt 14640
tatccatttt caaatttcag aaactaggtc tatattgtcc tatagatcta acaagcacta 14700
taaaagaaaa attacaaaga tttcccaata tacagataga cacataaatg aaaactttta 14760
attgcaagag aaagggaact ggattcatca gttacatgca tttagaaaaa tctgtctcat 14820
tcatcagcag caatgaagga gaaaagttgg caacagagcc aagtatactt actttctaag 14880
tagccaataa ttggaggcat ttaaaaatct tttcactaaa aatatacatt ttttgctcaa 14940
actccagatt tctgacattt ccttattcta ctgataatga cttcatgtga gcaaaatgga 15000

atgaaagatt gaaaaaaatc agatatatgc tacccataaa ggtttatcaa tatgatatat 15060
ctaaacattt atgtacttat tggtttaact tagttgatat aaatgaatat gcatatcata 15120
cacatcatgt gaacagaaga gcagatcagt ggctgggggt aggggattaa ctgcaaaggg 15180
gtacaaggaa acttgtggtg atggaaatgt tctatattta ttatgtggtg gttgttcaca 15240
actgtacatg tttgtcaaaa cttaccaata gcacatttaa aattggcaga ttttattgta 15300
tgtaacatat ctgactcccc gaccaaaaaa accaaaactc tgttatacca tgacacctaa 15360
aaataattcc tacagagact tacctagaac atcatggaca ctcaaatatt aaccatgacc 15420
caagaaaaca ctataaagta tgcataaaca gtgattctcc tataaaatct acacacatac 15480
acaatatgtt aagaaatgag tacaaaaatt aggggagcta ccataataca tggcagaaac 15540
agatttcctg ccccctgcca taactatcat ttaccaacat tcaatcgaat ttaagaattg 15600
ggggaatttc agagagtatc ctaattcact tcctaattca cttcctctag ataccaccac 15660
tcaaagattc ccgtggttcc cagagccttc acttgacatt catacaactt tgaccaggaa 15720
agaagggtgc gccacctcca cctgtgcctc tgcctctata ctagaaagga ttaggcagag 15780
aaagagattc aaatactgga aggaaaagaa ggtattggga agccaggatg gtttgttggg 15840
gcaaagtatt ctcttgtcat acagatggct ctcttcttgg tggtgcccca gcccaccctc 15900
aagggtgtct ataagtagga gaagagaaaa gcactaatga tgattataac ataattaaat 15960
aatttatgaa ggataaaata tcagagtcat gagagctctg aaagaatagg tagactacat 16020
gcatcttacc tacacattga gtattaaaaa tcctagctgc cctctgcagt gatgccccac 16080
ccagaagacc aatccattgg aactattgca atctgatcag attaaacaca ccaaggtcac 16140
tctgaaagta tggaggaaaa caccaaatta agaacaaaat aatactttta aaatataaac 16200
cagtttaaaa gtatcaaaaa cgtttctatt tagtccatct ctgatctctc tcccaaacct 16260
ctgaccgaaa gagacacaga tttttgcttc taaaaacaaa gtatatttta ccaacaggat 16320
aatataagtg ctttcaattt tatctaataa aaagtagggc aaagtgaaaa acaggtgcct 16380
ctacagatgg agtatactaa atacatgtaa cacttctgat aatgttgaac caccccctcc 16440
ccttttggaa tttgaaacat cccgcataag atctgcattt ttcaaatatt gctaaatatt 16500
tagagtctaa aaagatgaga ttcattattt tgcacagtaa cacatctcct aataatattt 16560
caggccagtt ttgaagaact gaaattgata tcttttccgt atatacttaa cctcctacag 16620
atttatttga ttctcaataa ttactattct ctggtaaaag gcatatgtta ttacagtaca 16680
ctgcactccc acaagctgcc cattcagtgt attttaataa atctctgcca gagctgtata 16740
aatttgatcc tgtttaaatt taataaggat cagaatatat caaagcacaa atacccatgg 16800
gagaaataga tattttgcat agttattgaa tggcctattc agggatacaa tatgtaagac 16860
ccagaccatc aaccttgaat taaaaagaga gattaatgcg ttcaaagccg gctacttgtt 16920
aaataacata tgaaattagt atttaataaa acgtcttctc tatattgttg ctaatggtat 16980
ccatcctagt acagctttaa tatcaatatt ctcatacaga attctaatat ttacaagaat 17040
tgtcagcaat gtatttctta aatatctaca ttatcttgat aaaatgcaac actgtatgga 17100
acagagatga agaatacctc atagtaattt ggaattgttg agtttaatgt tcatttgagt 17160
aatataaata aaaagaacaa ccactttgct ggatatagtt ttcacagatt acaagcctga 17220
tgatcaatga actgctttcc cattctcttc tgtagctgga tatgtttctg atttgaatcg 17280
tatttcaata atcattaata tcctgtggtt gagcaaatca tttcaatttt ttccaaccaa 17340
aatctacact tgagtgtaaa caccaatggc aatgattaat gagcctggat ttagaggctg 17400
ttagaagata caggtagcaa aatgtcaggt atcaggaaat ccttcctcat agtaaggacg 17460
actcacaaga caactattgc caggaagatc aaagggaaat ggtatcatgc gaggcatgta 17520
gaaccaacct gttctagcgc taaatgcacc agacagatgg atgggctaat ccgtcttcag 17580
ttatctacaa atactgcggg ggccacaaat tgtttaaagc cactgaaact gacattcact 17640
catgcatgtt atatttgaat ctacacctca ttattcccaa ccactttcat cttattctta 17700
aactagaaat gtaagggtta gtgcattcat cagacaaact gataaaagac actatctggt 17760
tagcctgtcc agatccatta gaaacctcac cagaagatta ggtcttactt tcagataggt 17820
agttatcagg taatgaagtc tctcctcaat gcgcctggct cccgagcgag gttggcactt 17880
catcctccac tacatcttct atgagtatct aaatcctctc taccctcttc tgactcttct 17940
gtcctctgct ggatggggtt cagtgagaga aatgcatcct agctgagcag acgaaaaaaa 18000
aaacaacatg gttgggcaat cagctatttg tcacactgag aagacatcac ttcttaccaa 18060
gcaagaagta gtagaatggg ctgggcgcag tcgctcacac ctgtaatccc agcactttgg 18120
gaggccaaca cagcaggagc tcttgggcct gggagttcaa gaccaacttg ggcaatgtat 18180
tgagagctta tctctacaaa aaaaaaatta aaaaattaga tgagcagggt ggcacgcatc 18240
cacagtccca gctactcaag aggttgaggt gggaggattg cttgagcctg ggaggtcaag 18300
gctgcggtga gcacagtgag cactggtcac accactgcac tccagcctgc gtgacagagc 18360
aagactctgt ctcaaaaaca aaaaaaaaaa aaaaagaaga agaagaagga gaagaaatag 18420
tagtagatga gctacaaata caatgcccca atggctgaag gaaactgaag tatggatgca 18480
gatatgacat gtcccatata tctaatttta tgaaatatgg gtatataaaa tccaaacata 18540
aaaatcagat ccataagact aatgagtgta gttaaaattt cctgaaatag tatttctaaa 18600
tatatttaaa aactagcatt ggttgaacac cttcagtttt ccaataaaat tgtgtaattt 18660
attatgctat aatttttctt taaaactttt atttaaaaag cccaggcttg gcagcctggc 18720
caagactagc accttatctt acacaaaata cagtgctctg cctcttaaca aatatagatg 18780

taaccactgc cagctgttta caacaagcaa aagctgggaa gccctattag gtgttatgtt 18840
tgttctgctc gtgttaatag actcatgttc aatgtcagct cttcttccta gatcagtgtc 18900
tgccaaattt cagttatttg tatatcactt tcatgcattt tgccatttaa agcaggtcca 18960
cactcttcct tatccaaaac cttagggcca gatgtatttt acagttgaaa gttttcaaga 19020
ttaggaaagt aatatggtgc atattccctt attacataat acctctagtg gtctgaggca 19080
gcaccccata gtcaaacatg tggacatttt tgtagcaaaa tgtctgaata ttcacataaa 19140
gataaataca ggaaaaaaaa gtttcatgtg aattcaggtc aagttttgat gcaaatgtgt 19200
aaccctaaac atttttttta atttgttttt cagagcattt agaatttgga attatggcta 19260
aggtatagta ttgtgagcct ttatttttct ttaattcatg catttgcata aaaattaata 19320
tgcttttttg ttaacaaaag gacactttat ttcatacatg gaaaactggt attcctttct 19380
ataaatagca cataactatc aaaataaata caataaatac aaaataatct atttaattct 19440
agcaaaccaa agctacagaa gggttgtaac tgggtcctgt atgatctcct ccgtctctcc 19500
ctctctctct cttcccctat aatgtgtctc aatctctctg ctaaaaaaga gattagcaag 19560
tattaggtgt taaagacata ttcgcaccaa actgagattt tctcactgat gtaataagaa 19620
agcctgaaag gaaaatggaa atgctttcta tgtaattcaa tatggtttaa aaacatatcc 19680
ttgtagctaa aaatcaagct acagcctacc aatggtagaa gcccagtacc acctgcctag 19740
cttccatcta tcctatgcca accagcacac catgtttgct catgtccctg gccagttcta 19800
ccttctctgc cagatatttg gaaacataca agggttgtag tattcagctg gaaaaaaaaa 19860
aagttcagtg gtataagacc acaggggtcc aggaagtgct tcattttaca taaagcccat 19920
tttactttat gccccttaaa tatgttttgc ctttgtgcat ggccatctgt gccgatgaca 19980
ctgttaccac tagagtacct aaatatgagt gcagtgatca actgtgcgta cacagcttca 20040
gtggatgcaa gtatgatctc tccgatgatc ctgggtccct tcagaggcct ttggatggtt 20100
ctgagcagta gaactcaaac tgtgatccca ggaaggcagc atcagcatca cctagcaact 20160
tgttagaaat gcaaattctt gggccctacc ccagacctac tgaatcagaa actctgggtg 20220
tggagcggca acatggattc taacaaggcg ttcaggtgac tctcatgctc agtgaagttt 20280
gaaaagcctg tgggtaacga gcacagtctc tggaacctgc ctgttgattt gaaggaatcc 20340
cagatcaatc acctcctcac ctgagacttt gaacaagtta tttaatttca gccatgcttc 20400
catttccgtt tctgttaact gggacattaa tagtacttac cttatagagt cgtggaggat 20460
taaatttatt aatacagata gagtacttag aatagttcct gaaaatactg cacaaatatc 20520
aatagtattc catgtaagct acataaaacc tttacttaca gctaaataat agtattttct 20580
ccaaataata aagacaatta ataactacaa gcttcatttg atgctcagaa tgaggttcat 20640
aggctttgtc tcctaataaa gtggaagtgt ggaacttcag cagagacagc caaatgtcag 20700
gccacaggcc agatgcagac tttagacagt ttccaaatag tgctaacatt taaaaatgtt 20760
aaaatatcac agaaatcaaa atttctggtt ccccttgaaa aatcaggacc tcagacaaca 20820
ctactctagg attccctaga gctactggca ggcagagtgg aggagcaacc acctcctttg 20880
aagaagtctc gtctcttgca gtctcacaca aatgggcagt tcagttgcag tcatgtacca 20940
tcttcctcac acctatgaca caggggtttg tgagccctgt ccttctgcaa actctaaggc 21000
ctctaagtgt gtcctctgtg tgcaggacag ggtgacagct gtcccactgg gaaagcaagg 21060
gagttcccaa gacactggtc tttcatgcaa caacagggag agtcccagga aacctgggaa 21120
aaaatggtca ccctatctgt gcagatggtt cccaagtcta caatccaagt gtctctccta 21180
tctggtccct gtttccaagt accatgagaa gcattttttt gtttgtttgt tttttgtttt 21240
ttgcgaatcc cacttttgcc tcaaaacaca ctttccttca cgtttatcat gtgctattca 21300
agcacttcat ggatgaaggc ctcctctata gcaggcacag tcctggacac tggagatgaa 21360
gcaacgaaca aaagagacag aaatctctgc cttcgccagg ttatgttcta gcatggagga 21420
gagagacaat gaataaataa ggcagaggga atgttgaatg gtagtaagtg cctggagaaa 21480
acggaagcaa ggcaggagga cggagagtat cagggaaagc aatgaggtgc ccatcaaagt 21540
cctccctgag aaaaaggaca tctgaggaaa gacttgaaga gaatgaacaa ttgagccatg 21600
caaatatctg atgcaacggt atttgggcca gaggaaaaca ggacctgggc cttgaggtgg 21660
gcgtgtgcca ggggtttcca agtgatcaag aagagcgggg tatgtggtgg gagggaagtc 21720
agacaggcct tgatgaggtc aaggaaataa tgtccaccac tgagttaaga ccaggctttc 21780
ctctgaatga atcagggagc cattggagag ctccaagcag aggagtcaca gggacctagc 21840
ttacttattt ttctcaaata gccatcgaca tctagttgtt ttgacttgtt attcaaggag 21900
gtcagtccaa ggaaactaga ttccccacct tctataattt cagcatcccc tctcattgaa 21960
gacaggaacc acgtttaaca tcaatgagtc ctgtgggaat aaagactgac agggaaccaa 22020
acagcaaaag ggaagactta cctcagttta tttatgggga tttatatact gcaaactaat 22080
actctatttc actggaatat ttgtgtttat gaagacatcc tgatgaacga gacttttttt 22140
ttcagttcat gaaattttca ccataaaaga accctgaatt cctattttga aatccattgt 22200
gccattttta ttctgcctgt atttgaatat agcatcaata tttaactact cattaaatga 22260
attgtgactt ttatagtgaa aatgtcagtc tttgcagttc attagattca tttaaagcat 22320
agctgcttga tatttttttt ccctggaaga ataaaagaac tgtgaattga agacataaac 22380
tttaaattta agcatgtgtt ttcttcaaaa ttaacattca ggatctgtaa cattattcat 22440
gagattgagg ggctgtttgc atctttctgc atttacttag tgactgtcgt cttttatgga 22500
gtatagagct gtttatcaca gttggatatg accagtggaa tgtcactgtg gacattaaga 22560

tgccataagc tattatgaat ctctacaatt ctgcatgtag tattaaaaac attatcttga 22620
actatggcat cagaaaaaat aaaccctgga agatgtcaaa tttaataact cactgaatcc 22680
aatagggcct aaaagtggtc tatttgagtt ttaaaaaata attctgataa gactttagac 22740
tgcctgaaag aaaatgatat attaaaaatg gaatttgaga aatgaattca aatgaatttt 22800
ttagatgttt gcaattcttt cttcctaaaa ttactatgat ttacatgtat ggagcagtaa 22860
atgcaaatga tttccaatca ccacgggtac gtgtactcaa ttacaaagtc tcttaatgag 22920
cattgaaaag atttcagcat tcactgaaca agacataaag caaatgaact cttcagtgcc 22980
ctccccttcc cacagagggc tgaaagggac tcactcttgt tacaagaaca cggccactcc 23040
tccctctctt cctcttccca gtccatccct caccccagtt tcagccacca tccaaaaaga 23100
aatcgcagca tggcccttga agcacattga gcttaaaagt gcctagaaga taaagcctaa 23160
acttcagcac gacaggcaag acctttcaaa atctggcctc gaccatcctc tctgggtctc 23220
ctttctgaga acccctattt tctgcctttt cacaactcca cacttatgca tgatgctgtt 23280
ttccctccct ggctcaccca ccccagtgtt cttcttctgt gatggtaacc tccccagaaa 23340
gtctcttgag ctagagtgat cattattaaa ttaaaaaaaa aaaaatccta ctttgttcta 23400
ttctgatttc aaaaacctga aaggcaagat atttgaggga aaaaagagaa tattgttttg 23460
ttgcttttta aaaaataaat ttgcaggtcc tgacaatccc gtgctttgtg tgtgctgatc 23520
acactgcaat ctaatcaggt acagtaaata aaatgacttg agaacctttc tgacgaatgc 23580
aaaggctgat gatattttgt gggtggaggg ctattcatgt atcttctttg agtatactaa 23640
agatccattt aattaagttt ggggaggggg gttctctatc ttttcctctg tccttttttc 23700
ctgaagctct cctactggaa agggaatgga agaaaacctt atgtggagtc cggagtgcct 23760
acctaaatta ctaagacaat aaaggacata caaaagaaga taatcaaatg ttactttggg 23820
tacttgaaca cttgctaaga gcatgcatcc tgcagtcagt aacattacca tctatactca 23880
gagggcaaac gctaatttca aatccagagc aatgtcaagg atttatcact gcaacccaaa 23940
gtatctttgc tatcaaagac agtgggggca tgaactacaa aggcaataac taaaaagagg 24000
ggctcatctc acacctctac tcctctccaa tattgggaag tccatttgag ttctagagag 24060
tgcacatgcc aaaatgcaag tgcgtgcatg cctgcgtaca cacacacaca cgcacaccat 24120
tccaacattt tcccatattt tgtccactcc ctctcttcct tgttatgggt agcagcagtg 24180
ataatgcact ttaatggaaa cctgcctaat gaacccaaaa ggaaaacgat taggaaaaaa 24240
atatgcaata catcgcattg aaaggtgccc ataattaaat tacttgttac cgtttttaaa 24300
tagtgcaaat attagtatgc ccacaaagaa ctaaaggttt gtttgtttta atctagatct 24360
tccagaattg gctgttcagg aactgcccat gttgcccaaa gtagctactt tttataagaa 24420
tttcccactg cctttgaaat attctctgac attcccattg gcttaagtat tctcatactt 24480
ttctgcctaa atttgtagtt tcccaagttt aattacccca gaacaatctg gtcatcaata 24540
accaaataaa agaggatggt atttttttcc ctcataaatc tattcatcaa gattcgacca 24600
agcctgcctg gaatgtccag cgaaagcttc aataatcagg gagaaatgag ttgcagaaac 24660
tcaaagagaa cagaatagcc aaggcaggag attacgaagc caggagatga gggcacactg 24720
aattaaacat tcttaagcag acttacatac ataatgaatt ggtcaattct gtccaatgtt 24780
tctgaaagag attacccttc caacaagcca aatgagtgtt caaggatggt agctcggatg 24840
gattcctgaa caaaataagt gttaatcact ttatctcccc caatcttttt tgtgaagaag 24900
agcaactttt catttccata aagccttgaa atcgttggtc ggcaggggat gggggtggtg 24960
ggggagggct ggttagagga tacagacaat gaagtccctg aaaaggggga tttcctgaca 25020
ataaacccct gatcagagcc agagagggga cattgttgtc ttcagcactc ccctctcctt 25080
tgggaattcc aggcgtagta gagagaagag ggtcacagtg acaactgctt gggaaggctc 25140
taaccaggta gtctttgcga ttttctgttt gggaaaagct ggcctacaac aatacatttt 25200
cttggaatgt gttcttttaa acaaattgct acagactttc ttaagacatc ctgtcaattg 25260
ttccaaaaat gtgtaatcag cttatgaaat ctttaagcaa agaacattaa atgttagcat 25320
aaaatctcat aactagttat gtattgagat tctcctacac accaggcaca gtgctgggca 25380
ctaaacacat agcagcgaac aaacgaaaca cagcggtcac tactgtctgg aaatcacagt 25440
taggaaagac aaaactgctt cttaaatctc tgtgcactcc atgccagaca cagtagccat 25500
tccataaata tttgaggaat gaatgaataa aaagacttca gagcttgagg ttcatgtgac 25560
tcaagattta aaaataaaaa aataaaaaag taaaagaaaa gaaaaagcta gcaggtatac 25620
tagaaccaaa cacacaaaat aatgagccag agtgtgctcc cacaatatta ggagaaatgg 25680
aacaagaaat caaaaccctc gacatattgc tcctatttat tattttaata tcatcgttgc 25740
acagaaccag tcattggtgg tgctttaaca attcctccta aatagctttg tgacttagtc 25800
caagcaccca gagacagatg agccccaatt caggcaagtc ccctgttgaa ggacaactat 25860
tttctcaaat gaacaaagat cttctcttct caatccgcca gatcacacag ctttataagt 25920
ttgaaaagtc tttttgctaa cctagaggta ttctctaaca gaaagaggga gatggaaaaa 25980
gaaaacatct gcaaggtgct gaaacacgcg tagattattt tacctgatcc aggagcctcc 26040
ttccctggtg actacctact cctcagaatg agtgctggcc ttcttagcta ttctggggaa 26100
cacaatgatg gaggctcctt ttagctccat caggccctag agaaataact gccctatatt 26160
tagatagatg ccctttgtaa caaaggaata attcatccca gttagcactt tctaaatgac 26220
agaaccagaa agttttcgaa aatattcttc tagctcagtg gctctgaacc aggggacgtt 26280
cggcaatatc taaagacata ttttgttgtc acaactgggg gtgctgctgg catctagtgg 26340

gtagaggcca gggatgctgc taaacctcct atgatacaca ggaaagttcc ccaaaacaaa 26400
gaactattta gcccaaaaca tcaacagtgc tgagatgaag aaatcctgct taagttatac 26460
attaaataaa gaacagcagg gcatctactg gtgaccccag atagttctgt taaaatattc 26520
tgaagtagga ttcttctccc agagtctatg ttcagcaact taaccagatg ttaaaaactg 26580
acccgatctt aggatgttaa caatatttta aaataatact gatggtaatg gtaacattaa 26640
tgaaatgctt ggtatacaga gagttatagt gcatatttac aatgacccta tgttgcaaat 26700
gttattagtg tcctctagaa ggtaactgta ttaatcacat acaataactc tatggagtag 26760
gtgggtttat aaccccctag gaagtgtgtg ctgtgggcaa gacttgatct gttttttttt 26820
tcaccaccat ctgtccagca cccagcagag tgaaatctcc atttcagaaa tgatgatgct 26880
gagaggctca ggaacatgcc agagatcaca gggccaataa atggtggaac cccagttcaa 26940
accgggcaga ctgcctggtt ccctagcctc tctctctcgc accgtgccag ccggccaact 27000
gcttcatatg tggagtcatt gaaacctatt agacctcttg agtctaacct tagataagca 27060
gaaccaacct aagggggaag gaattaggct gctttccaaa gttaccaaaa gtttcccaat 27120
attcaagaga ctgattaaac aaaccagtga ccccaagaca atggtgaagt acatttgtat 27180
tgccatgaga cacttgaaag caatctaaga gggtggctgg ttttataaga ggttaactgc 27240
ttctcagttt gagcaagact catgtggcac caggcactag gcaccgggca ggcttcctga 27300
agctaaaatg gccactttct caaaggacaa atgccctgca tgagtgcaga aactctttgc 27360
aaccactagg gagctaactc agggatcatg ggatttacat gtggaccaga caacaacttg 27420
gccaccagtc caatggatcc ccagtgccaa gttaacctta gcacagtctt cgggtaaaat 27480
ctagatcaat gctgattatg tcaggaggtc aactttcagg aattaatact ttttgaatga 27540
taagagagtg caaacattaa tttaaagtct cagagagtat actaattttc ctgttttcat 27600
catttcagta gattctataa ttctaggggg aaatatccca taagaaataa attctagcaa 27660
gtactgtaaa taaagaattg tatccacctt tataaaaaga ctaaaagaag aagagataag 27720
agcttgtttt ttagttttac aatattgaca tagtaggctt tatttttttt taaatgtgga 27780
aatttccctt ttagaacgat aagaatacta gcctggacac tatacaaaca tagtcattgt 27840
acttataagt gctttaagta tttattcata gcctgccttt ctctacaaag gaagtgaggt 27900
gacttttaaa agcatatgtg atttttaaaa ggtaagtaac aggtgaatca aagcaaggaa 27960
aaagtaaatt tgagaaaata ataaagccta gatggccagc acacaggaat gcatatccta 28020
ctgccctaga cagctactaa atgggtttcc tgtttgactc caagcttctt ggccatcaat 28080
gcaaagaaag aacaagaatg gtaagattaa tgcccagtaa gatacagcga agtggatttt 28140
tttcagttct tcttgatata gtaacctaag aagattctct gtaaatcatc atttaaaaag 28200
acaccatgag gtgtaatgag ctatacattg taaatagaac tttttaaaaa aaatgatgtt 28260
cacgaaacta ttactcataa tcctctcaat acaaagtgaa gccttaatca gtgtgcaatt 28320
caataaagca gagagggcct gtgaaggcaa ctgtgaagtg gggtgccaca taatatgcca 28380
tcgtgagcat tcagctctct cacaagtctg gcagaaatct cttatcaaat taggttatct 28440
ggatgaacgg aaggaagcaa ggcatcttct tcatgccatt ctccttagat atattatttc 28500
ttccaacaga ttttattaac acaaactggg cagcagggac ttcaagaagg acaaaaatcc 28560
tgtgtcaaaa ccattacata tgttggcaac atttatcatc aggaaaagcg tcccaggaag 28620
ttgaattcta atgtatgtgt ggtgttacaa tggaaatttt agtgtgcaac tggaatcagg 28680
ccatgagttt tgcctgtcat caaatgtatc tgttaactta gggttgaact gccccccacc 28740
ccactttttt tttttttgag acagtgtctc actctcttgc ccaggctaaa gtgcagtgcc 28800
atgatctcaa cccactgcaa cctccgcctc ccaggttcaa gcaattctcc ggcctcaatc 28860
tcccgagtag ctgggattac aggtgcctgc caccacgccc ggctactttt tatatttgta 28920
gtagagacgg ggtttcacca tgttggtcat ggttggccag gatggtctgg aactcctgac 28980
ctcaggtgat atgccctgcc tcggcctccc aaagtgttgg gaatgtaggc gtgagccacc 29040
atgcctggcc cctctttttt tttttctttt tttttctaac tacacccttc tctttttcct 29100
ctctacaact gaatggtctt tctccctgat ataagaatct tctagatcac tcccatccat 29160
tgcattcttc ttggtcttct gttatcactc ttcttactca tcttactagc ttttaacttg 29220
actacgtttt cctttacttg gtttctgcct tggacttcca caagcttcac cctaataatc 29280
atgcagcctt gaaaaacatg gaggaaggaa aatgctgtca cctgccatct tgtacctgtt 29340
taaagctcca ctgttttcag tgtttaaact atgaagaaag gctccgatga tttaagctgt 29400
atagtcaaaa ctacagtgtc ttctaggaat ctaggattga gacaagatgt aaaaagagga 29460
acatgtaata tattatttta ccccactggc aattagaaat cgttagccaa agccaggatt 29520
ggggaggata ttcgaaaagt tattaatgca gacagagcaa ggcttttatg ccaccaagaa 29580
agttatttat tataaacaat gggttttgag tcacatggac aatagctatt ttaaaaacaa 29640
cgaatatatc caggaaaggt gcaaaaatat aaaggcaggc aatcctagtt tgtggtgctg 29700
atctattacc actgcaatga ctgtcaatag tgaccctgag cagaactcaa acagttaaga 29760
aaactcacca cctgttttcc aaggaatttt attttgcttt aaggtggtaa gtttgatact 29820
aaatacggag ggtgttcaat gtcaagttga ggacatagca atttaccctg caggccagtg 29880
ttcctagtgt ctactattca tattagatta acttaagtgg tgtataaaaa actttttaaa 29940
agtttaatag ctatgcattt attgtatggg taatctttac acattgcaag caatacaggg 30000
tcgccatatg caatggtgat acaaattaaa aagtgggtca atgtaaaaaa ttataaataa 30060
tagcagtgat aggtagatac agcagaaatg aatgtggcaa atctgcgaac acattgcaag 30120

acactggaag tgttcagaag gaaaatggta gctgtctaag ctatccttta gaagattaaa 30180
ccagaagtaa agcatagatt ccattgaagg tgagaaaaga tgacaagcag ggagagaggc 30240
ctgggctagg atactgatgg ctgaaagaaa agaaaaagat tatcagaagc ataataaaga 30300
agacatgaag actccctgga ctaagggaat gaaagaagcc aagtaatcta aattaatttt 30360
gaagttttgc acctggaaat acggcactga aaaaaacaga aattcaaagg ggagtttggg 30420
gggcagtggg gatacaggtt gccctctggc ttatgctgga tttgaactga cggcagacag 30480
ggaagcggtc atagcctgga gagagccact atgcaggaga tgagaaatgt agatcacagt 30540
agagatgagg atttgagacc ctggggtaaa ttgcaaaaat ggccacaatt ctccacacct 30600
ctgtctatcc atgcccttct gtaatgtaat tttgtggctc ctcccatcaa gaaatggatt 30660
ctgcgtcctc tcccctagaa cctgggctgg cccagtgact tgctttagcc agtagaatgt 30720
agtagaagga acagtgtgcc atttctgggc ctggactcaa gggttcttgt gtactttcct 30780
gctgtccgaa atactgccat catgaacaag cccaggctgg cgtgctggag gtgaaagata 30840
catgccagag aactgaggga ccccggtgga cagccagcca acctcctgaa gcaaaccctc 30900
cctgccaatc tgcagttgac tgcaaacaca taagagaccc agaataggat aagcactgcc 30960
cttctgagcc caacctaaat tgctgacatg cagaatcaga aacaaaatat gtgtttttga 31020
agtcacaaag tttagaggcg gtttatcact cgcgatagat aactggtaca gacaacacgg 31080
gtctgcctag aaagtgaaaa acatacgaag gcaaaacagt aagaggtcaa gctttaggag 31140
gatattccca attcctagga agaaggggga aagctgtaga caagacaaca gggcaatggc 31200
aatcagaaac attcaagtat ataatatcag ggaaccagga acatgggggc aggggacaca 31260
gaggataaaa gtgggggtag gagaagtcaa ggactcatca gggaagtata gcactgggct 31320
tagcagagaa tctgcctaga gaaggcactg gtatttttgc atgaatgatt gaagtttgat 31380
ctgtcaaggc agagctcgct ggagaagagt gagaagacag tgtcagtaac gtaacaagga 31440
atagaagtgg tctatggagt gttaaggagg gcctgggtgg gaaagatatg gaaccctagg 31500
taaagattac ttgtttgaag aatcattcag agagatgttg aagacgaggt caacagagtt 31560
ggacaaaagt gtaaatgtga atggtccttt ctctaggacc ctacaatcac aagtgtggct 31620
gggaccatcc atgccagcat cacttgggaa tttgttagca atgcccactc tcaggcccac 31680
cccagaccca ctacctgtct tttatccaca tccccatgtg ccattcaagt ttgagaagct 31740
ctgctctagg aagtacaaag acaaggaact gaagagcatt taatgacatc ttgagaaagt 31800
cattctgttt tgaattccct ttcaatcccc cagataaaat aacacaaagt cttgccttgg 31860
tactaagcca tcttcgacac ctccctaaca cttgcaaatc tcccttttca acagagttca 31920
acagagagca gagccttagg caagcaaggt ctccagtgag atttgaaaaa cactgtttac 31980
accccttttg tgtttatttt taggattttc ttttatgtgt ggaaagcaat caggttttcc 32040
atttgcagta gtcttaattt cacattaatt tatttaagag aaaaaaagag gtaaataaaa 32100
atatgtagca tataataatg tggatgcaaa tgagaagaca gcaaaagttg ttacaagtga 32160
ggtggcactc aaagcgaaga ctggaaaatc tatagtggaa gtcagaagac atctgggcac 32220
atttgaaggt agaaaggcaa gagacaggaa agatgggagc tgtttgcaga acaaagaata 32280
ttcaacaggc aggaaggact gttggaagga tttatcccag agtagaagct gccctctctg 32340
ggcctggaag aaagaaagat ctggtccctg aatgggtgag gatgacaaat cagagatcta 32400
aggcagataa tatcaaattc ttcataagta aaagattgga ggtcccctct aaaatgaggg 32460
taggttggtt tgactgggac tgcaaaagag tatgaaagac ttggaatatc cattgtggac 32520
aatgaccaag agaacaaaca gatgaaaagg attccgcagc aaaggcccag ggacagtcag 32580
gcagcattaa tttttagtaa ggaaaatgga cacagtttat cccaaagctg agaaatgaaa 32640
ctctaaggct gacacaaaaa atacaacagc aaagaaaggg ggcagaggag attaggtagt 32700
agtggcaagc atagctgaca agtcctcata gtcaaggtta catacaaagt caaaggttgg 32760
ctaggtgcag tggctcatgt ctgtaattcc aactctttgc agggccatgg cgagaggatc 32820
acttgaggcc aggagttcaa gaccagtctg gtcaacatag tgagacccca tctctacaaa 32880
aatattaaaa attagctggg tatggggtgt gcacttgcag tcctagctac tcaggaagct 32940
aaatcaggag gtttgcttga acccaggagt ttgaggctgc agtggctatg atcacaccac 33000
tgcactccag ccggggcaac acaacaagac cctgactctt aaaaaaaaca aaaaagagtt 33060
aatggaaaag gccagtagac tggactctgg gaacaagatt ctcagtggat atagggatat 33120
aagaagtgag aaataaacta ggtttgatca aaaaggaaaa acatcatgtt tttaaaaaac 33180
tggtttttaa aatgtcaact aaatattaac tagtgttcaa aattttgtta ccaagagttg 33240
ggacttgata aatggcacta caaaaactca ggaaaaattt tgaaaagaag agaaatttgg 33300
gcctaaaatg caggtaatca attaaaatta ggacataatg atgtgtggtt cttgcaaaga 33360
acataaatca ctgggtattt taagtgtgcc caaaataaaa tcccaaaact gaagaaaggc 33420
ctgaaaagat ataaatctta ttacagtgtg tctctctcac caaaagagga tcatccaaga 33480
agcacataaa aactaaaact ttgaagttta ggaaaaacaa gaacactaca gagtatacat 33540
aaaatttcaa acttggagat acccaagaag ggtttctcag acctcaccca tcgctcactc 33600
acacacagca cacttgctga actacctggt gtcccttcct ctccactcac tccttccaca 33660
gtaattgaga gtttcactgg gcacatggcc agccacaata taaagtacat ttcctgagta 33720
cccaagtgtg gccatgtgat gaaggctagc cagtgagata taagcaaagt ggcatgtggc 33780
agcttctgga aacctccgtt tgcccttgat ccccttcaat ccccttcgtc cttcttgctg 33840
gtcaaaccat ggaggttgac tagaggtcaa ccttgagcca taaggataag ggtcacagga 33900

tagctagagg cagcttggct cttcagtgtt taagaagcct ccatactagc cttggactac 33960
ttccctctag atttggttag ggaaacaaga aatgagtatc tacttcattt aagacgttgc 34020
tgttcaagat tttttctatt acatgtaagt tgaaccctaa tgtaccctct ttgctattat 34080
gggggcctcc tattccttga ccatttcttc aatggcctga atagttggat taaaagcagt 34140
cacttcaata ccacatttcc ctcaactcct gttctctcat ccctgcctag gcaaaatcca 34200
ccaccaagtc agcttcataa tcattgttct cttcctgcca ccaacacatc aaatactgat 34260
aaaaaaaatt aaaaataaaa tgtaaactta aacacttcta atagggatca tcacaactgt 34320
agtccaactc cttgaaaatg gacaagtgac ttcactgctg cattcctatt gcctagaagt 34380
gtctgaccca tactatgtca acacatattc actcaaatac taaactacaa ccagatcttc 34440
tgctcaacag tatttttatt catctctagt tcacttctta ccacatttta tgtgccaggc 34500
acttctctaa atgtgagatg taccatgtgc atcaaagtaa atacaccccc tatcctcatg 34560
aagtttacat ttcagtggaa agagatcaac aagagcagat gaataaatga ataatcaaga 34620
aattcaaata gggtacatgc tatgaacaaa attatgaagg tagagagata ttaattagag 34680
agatggggaa aaggatattt tagaaattga gattttttta atggtcagga catacttgaa 34740
ggtgatatct gggctcagac ataggtgctg agaagaggcc agccaagtga agacatttgg 34800
ggagagggct ctagacagag agaatatcaa gttcaaaaag atgggatgaa ggctggtacc 34860
gttaaaacac ataaagcgag ggtagagggg aggagtggta agaaaatgag gtaggaaaag 34920
tacacaggga tagatctcag gcttgtcaat caattcccat gcccatacat attgttctct 34980
ctctctctcc tctcttcccc aacatctttt ctactgaaat agatatacac tatatatata 35040
tatatatata tatatatata tatatatata tatatatata catgtgtata tatagaacaa 35100
gatatatttc tcactctata tccatacagc cactgagaat cttattccca tagtccaatc 35160
cactggccct tttccttaac tcttgacatt tttaagagtc aaggtcttgc tgtgtttccc 35220
aggctggaag tgcagtggtg tgtgtgtctg tgtgtgtgtg tgtgtacata tatatgtata 35280
tatacacatg tgtgcacata tttacatatt ttatatatat atgaatcttc agccatcttc 35340
cctttctttc ctattatcac aaagaaatag ttaccaaggc ttcatttaag ggctaactct 35400
cctttatcta cagtgttcgt tccaacagca actgaccacc caatccatca ttcaatagct 35460
ctctcactct ctctctaatc tcttcttcaa acatgttcag acattgtctt gaaaatatct 35520
tctcattagc cccacttcaa aatgtcaccc tctacctata cacacatata tgtatgtaaa 35580
cacatacata tgcaaatgta ccgagacaca cacatacaca gtttactagt acctactcaa 35640
tacaacctag gatctttcac cagacattct agtgtctcca cagtttgttc tcaaccaacc 35700
acatctccca ccatggaaag agaaaatctc atatccatag aatatccagg tgacctatag 35760
gtttcttgaa ggcagaccac atccaatctc agcctcaaca agaagggcgt tcctaatata 35820
aacaatattt atgatattta cattacataa gcacaggctc atttggtttt ccacaaagaa 35880
taataataaa catagacaat ggaaatcctc agaaatcttc aaaatcatca agcttcactc 35940
cagttagccg tttcaatgga ctttgctttc ctatcctaca cccacattct ctcattgtga 36000
catcagcact ttgtttttcc tccaaagaac cactgctcca tactcttagc tcctgtggct 36060
tccaagaaga tgacagtgtc cagctccccc actggtatgc ctacgtggct acgagcctgg 36120
gcatgggacc caagtctatc caatgagact tgatccagaa aagctgctga aacatgcaag 36180
aaagaatctc tccctcttga agaggctgag ctgtgagaat gtaacttgga gctgctgctg 36240
gacaacttcc cactacttga gtacagcctg gctgatatga taaaaaaaaa aaaaaaaaaa 36300
aaagaaagaa aaagaaaaaa acagatgaaa gcagagctaa tataagaaag tcttgatagc 36360
atcccttgag actctagatc cagctatgcc tgaaacaaaa cttacaatcc cttttctatt 36420
taactcagtt tgactgaagt ctttttctca cttgcaacca aagcattttt actaaaacaa 36480
tcattttaac cacttttcca ttaaacattt ttattagaat ttttaaataa gtcaaataat 36540
aagggttgaa tctctcatta aagataagtt ggcatcaagg agcagcacga tggatctacc 36600
gagtatattt tattaactgt ttgatatttc tagatttaac tcatcccctt ttgaagaaga 36660
tcaacttcca accaatcata catatttaat aaaagttttc tagtcaggtc agcattttga 36720
acttctactt cctacaccta ggagattttt taaagaaact gtagaatttt tattattcta 36780
cagtaaatcc cttgcttatt acaaattagc tgtagaaatt gctggaaaaa atctcacaat 36840
ttggtacagt tatactacat ccatttaaaa taaattgaat tttaaaactc actcttatca 36900
ccatctttga cataattctt ctgcataaac aatttagtca atgtagataa ggctacatta 36960
aatttgcaac atttggaata gaaggaccca gcttaaaacc tcactccccc aaaagaatga 37020
gtatatatca ttcaaatatg aatttagagt aaaaactcaa atgtgtcttc atattagccc 37080
atttgtctat cttcaaattc ttcttttaca aacaaagcaa aattgaatta agattaaaga 37140
tagaatgtta atcatacaag aagggccatt ttccagggcc tggcatgttt tacacaagca 37200
tgaaacatat ttaagagaac atggcctccc tgaggaagag attaaggata caaacctagg 37260
aaacaaagaa aataaaacga tctacctcaa agggataaga ttaacctgtg agaagctcat 37320
gcggacctta aataaaagcc tctactcctt atatttttaa acaggatttt tctttttttt 37380
ttagagcact aattccacct tcaaaatata tttgagtttg taagtatact caacaaaaat 37440
cctgtccttt cttattaacc atactcaaaa cagattctct aaaatagtta tgacaccatt 37500
aacatttcat gaaatccaaa acaaatgtgt cttccaaaaa aataaatgtt ttcattcata 37560
aatggctaat atggttttaa attatgttat cacaaatttt gcatcattcc cccttaataa 37620
ccatttaaga atgtttcctt ccaaatagct attaagagag aaaggatgtt tcatcatgca 37680

tacacacaca taaacacaca ctccttgttt atgtcattgt ttttaatggt gcagtaggat 37740
ttccaaattc ttcaagtttc acacattcaa gaatgtttcc ttccaaataa ctattaagag 37800
agaaaggatg tttcatcatg caaacacaca cataagcaca cattccttgt ttatgtcatt 37860
gtttttaaca gtgcagtagg atttccaaat tctctgtttc acatgttact tcctaaatta 37920
ctgcctcact gactgggaag ttgaattggc aacaccagcc aatcctcagg tccaaggatg 37980
ttgtccttgc atttcctgtc tccttcctct tgaaggactc aaattcattt cattcatctg 38040
ctttactaca aacagaaaga ctccatcttt ggttgttcag ccccaaactc tctcttgagt 38100
tccagtgcat gactctgtga gaatatacca ctgaaatatc caaaatgagc ttgatctggt 38160
tatctttcct caaaaccagc ttctcctctc tccgctctgc ttccactcaa ggtcttacca 38220
ttcgtccagg ccccaaattc aaacaccctc aatcgtttct gcttcctctt tctgccttcc 38280
agtcgggagg ttctgtttag tgctttcttt tcttttgcat ccaaattcac atctccttga 38340
ttcaaatcct tcccatacct tacctcaact gggtgatggc atcctactcc cggtctctcc 38400
tactcttgcc ggggcctaca ccattctact caaaaggtca gatcaatctt tacaaaagta 38460
aaactctaac caatccaccg gtccacttaa aatttttcag gctgggcgca gtggttcaca 38520
cctgtaatct cagcactttg ggaggtcaaa ggcaggagga tcactggcgg ccaagagttc 38580
aagaccaacc ctggtgacac agcaagaccc tatctctgca aaaaataaaa tgaaaaagtt 38640
agccaagcat agcgaacaca cacctgtggt cccagctact caggaggcct gagtagctgg 38700
gaccacaggt atgtgtgcat ctcttgagcc tgggaaattg agactacaat aagccatggt 38760
cactgcactc aagcctgggt gacacagcaa gactccatct caaaaaatat ttatgtgtat 38820
atatatattc ctcgaatgca ctcagttgca gacaccgttg actttccaac agccttcttc 38880
cttgctaaca gagcataaca ctaattaatt atgctcacgt agcaatgccc agcctcaagg 38940
gatattggat tggtctaaac tgatcaaagt aatcttcctc tttgctagtg agtagcctag 39000
ggttggtacg caacccattt ctgaccaata agataaaaga gacggtctag tggaaaagct 39060
tttccttctt gccaagagat gtacaggaag agaaagtact catttgtgac cactcccacc 39120
accatcacct tgcttcttat tttgtgtgtt ttcatgtgac gatgtgacac ccagagctac 39180
agcagccaac ttgtagccat gggaagactg ataaaactcc aaggatagca gaataaaaag 39240
aaaatcaaag aagggctctt caagacatga ttgaaccatc caaaacaacc tggcactgcc 39300
aacccaaaga cttctttgaa ggtaaacaag tgtcttttat ggtttatgcc accatttgtt 39360
gagttttctg ttctctgcag cccaaagcac tctaactgat aaacttagaa agagcaaact 39420
cttgacctta gaaggccctc tgcttgatgt gctactcaaa gtgtagtcta cagacccaca 39480
accttagcat tgcctgggag cccagccctg ggcccatgaa tcagaatctc caagggtaaa 39540
gtccaaaagc ctgtgttata acaagctccc aggtggttct tgtgcatggt aaagtttgag 39600
aaacactgcc ctacaaaatg atcacaaatt attttttacc atctcagcta ctaccgtaca 39660
tagcatgaga tgcacttgat ctgcaatttg tctggtggca gtaacctttc cgggtttgag 39720
catcatttgt tgctccttta gtttcttctc ctcttaatca aaatcttcaa acttgaagcc 39780
ttcaggtcct gagaggatta caaaggtctg aatatgagac aggctgtgaa acaggccgtg 39840
atgaactatg gggtacaggc acatctgaag gacaccgagc agatgttgcc atacaaggat 39900
attggttcag tgttgccaga tgtctaaaga aaagctggaa tccagattta tatgcaaagt 39960
tttttagata tagacaacca agcctttttt aatactctgc agattaaaca aattttcaag 40020
tggattcaca tccagctgag tttgtgacat gaagtagatt ccttgaaata tcgacacata 40080
ctttgaaaac tgtgaccacc tacatgcatt gacttcattt gaaatgacta aaattaattt 40140
tctttggcta actatatgct agtctctact gagcacaggg tcttcatcag tagcccagca 40200
tctggagtgg tcaaatagtt tgtggacctc tagttaggag cagcaggact gttttatcca 40260
ttagtcacta tacaccacac acttagaact gcaagcttct catgggccca caacaatgtt 40320
taaaatgtga gaaaaaaata aactggccac acatcaccag aaaactacaa aattacaact 40380
aatagatgtg aagctaaata tttacaaaat gtaaactaag tcaacggatc catatcaact 40440
cagatatggt atcatatcac attagattaa atgcaggatg tggatgcatt ttaatgtttg 40500
atggggcatg gaagggagct cagatcaata gtgcttagag ctgccaaggt tctaaaaagg 40560
ccctgggtac caacctgaag ccaaccattc tgtggtaagt ctggtcttca caggtgtcac 40620
aaatttcttt acgcagctct actccattct attgaactgt ctacatagtt ggacatggga 40680
gcagaactgt ccacatagtt gcatgtaaac cataaatgac ttattaaaaa gttatgtttg 40740
gatcctcatc tacatatgaa tttaagacaa gaagtacaag aatcagcaca caagcaagtt 40800
ttgagggtcc cacataatga ttttaattat tttttgttaa tttattccaa catttaaaat 40860
tcatgataat tcacataaaa acccagatta tcatcctctt gaaaaaagaa ttgtaaagta 40920
tagagatatt ggacccaaat gttggcatgg caacactcta ctaatgctga cgaaggctat 40980
ccctttaaat ggggtatgtt ctctacttgg ccgcaaaccc tgtctccatg taggctaact 41040
tgctgtccca agagacaatt gagtttatgc cccctagttt aaggctctta ggaaaacctt 41100
atttcttttt cacaatgtgg cttcaatatc ttacgtgaag acctgtcgtg ataggataca 41160
ggaatctctc aatctaagaa gagtttcaat caaattcccc ccaatatgtt ctcaatttga 41220
accatgatcc ccaagaagat tgacaaatga caaattaccc tgcagtttct tttgcccttc 41280
aagctagcca aggcagaagc ccaatcaggt ggagtcaaag cactaatata agaatgtctt 41340
aattaatcaa agtgatcaat aaataataca cataataatt agaaagcaaa gtctgggact 41400
gatagaataa aaacaacata gcactaatct aagtaagggt gagaggtcaa tagccataat 41460

tcaaaaggaa aagccctaaa aatatgcaga aatagctagt aactggagtt tagtccaagt 41520
ttgttaacaa cccactgtat gatttcacct cacacccaca ttaaacacag tatcttattt 41580
tgggatggca gaattctgac aactacaggt tgagtatccc ttatctgaaa tgcttagatc 41640
tagaagtttc agatttaggg gttctttgga ttttggaata tttgcattat acttaccagt 41700
tcagcatccc taatccaaaa atctgaaatc cgaaatgttc caatgagcat ttcctttgac 41760
tgtcacatca gtgctctaaa aagttttgga ttttggagta tgttggattt cagattagtg 41820
ctaaattata tttgctaatt gatattctgt gtattgtttt cttactggtg aaaagacaaa 41880
ttgaactcag gaaagaaaaa ttacaaatat atagtcaaag agtcaccaga tcatttgagc 41940
aacataatga gtttttattt cttaatatac agggtttaat ttgttaacca caccagaaaa 42000
agaaatgttc aaacagtcga ctttctactt gaaattaaaa tggaaattag cccttaggaa 42060
acagtgatga ctttaagttt atacatcagt ggctggttca tgaattatta actgatttta 42120
atgatcctgg ctatatttta tttgaagtta ttctcctact tgcaacgact tttgaaccca 42180
caaaactctt tcatccattc tccacagaca cagtacactt tcatgagcac tgacatttga 42240
aaaggatcca tatactcaca gaatgtaaag ataatacaca tgcatataat tgatacaaag 42300
gactctggtc atttgttaaa gagagtaatg aatgaaaccc tattattcac ggtttgtaaa 42360
taaatttaaa gataagcgct aactttcact tgttaagcct agagccaaga gttcggtgtt 42420
tcttctaaag tttcccagat ggtcattaaa acaagagagg gaagaaacat actttctttg 42480
aaatccatca tttcccatcc agaaagataa tacatgtcat caaaagttaa gtctttagag 42540
attaattaat aatgtgagta ttttcaaata aaataaatgc atgtctttat atgctagttt 42600
ttacattttc agtataaacc tatattgaac agcaccactt cagacacaca aattagctgt 42660
gaaacaaaat gctataaacc tttaattagc acttgctgct ttatacccat aaagttaata 42720
cttcctcttc tactatattc tctgtaaagg aaaattatgg gaaataaaat tgtgtttttc 42780
acttccgaaa ataaaagtag caaaatatag tattgtggct cagtttaaag atagttatat 42840
ttttacttgc aaggaagtca atacaaagat cccttacaat aagtataatt tgattcctac 42900
caatattaag cgctatgtct aactttcata acttgggtac acagagcact gatgacgatc 42960
agattgctaa ataagtaaga aagcaagtta tttcaggcaa atagctgcat taagaaaata 43020
cgacagtaag ccaacttgta tccacactcc tgaagctgaa caagcatctc aataaataat 43080
gtctgttttc aagctttata taccctccct gaaataaatg ttctgagctt aaatagactc 43140
aaaatagcct ttctcagaaa ataaaaaaga gacacacaca aaacaccccc aatttcctct 43200
ggtcccttga agtacccttg ggatcccagt gaacagagat ttgccacgca ttgcagtgac 43260
tgcatttgta caataggttt ctattttggt gtcattcgag tgttgcagat cttaaatcag 43320
agaaagaagg agttacatta ggcaaggtaa tggcttccaa aaataagagc atgcccagtt 43380
tatacaaggc ttgcatccac atgctaagtt gtaaggagat aaataatacc ccactggaac 43440
aattaggtag attctcagaa acactacata cttaattagg ctgcctaagt cccttactga 43500
gcatacatgg gggacttttt aaagcattta ataacctgag taaatccaaa atcagaggac 43560
acaaactaat ccttaactct ggctccagcg atcggccttt tcttgcctct ctataaatgc 43620
ttagcgtagc ccttagaatt cggagagcag gaacgctcca accaccaaag ggttaaagca 43680
cacggaataa agtcccctcg atctatattt tttgggaggc cctctgtgcc cagacgctga 43740
gctgggcgat gatgacacgc gagggcgagc aagagagacc ctaatggtct tttacaaact 43800
tcagcactag agttgggaac gcagtataaa caaatagaaa ggaacacgag cctccatcag 43860
ccacggggca cagcaaactg ttcctcactc tagatgcaat tcctcctctt cctgttattt 43920
attcattagt tttgtttagc acctttcttt cagcaggctc cttatttcct cctgttatcc 43980
aagcaatcac aaacaaacta cgatttgaaa gaaaaagaga gggaggaggg aggagcagga 44040
ggaaggggaa agaaaaccac attcaaaagg cacaggccat tcagagcccg gctgtttctc 44100
tgaatctcgc cctcagggtg atgttttcga caaattcggc gctgggggct tcagggagga 44160
cagaggacac cggatggtgg gggggaattc cgcgcggccc gggtaccagg gctgcgactc 44220
tcgcccgcct cgctgctccg agcgacgccc gctgcccttc tgaggtccac ctcgccacct 44280
ggaacgctcg gtcctcgaag ttggcgcagg gggaggtgga gaaggtggag tgaccccagg 44340
gtgtggtgtg gaggtctcca atcccgatct ccccgaaatg caaaacttag cccccccaaa 44400
aaaatgagag gaaagaagcc acttactcta agcggggagg ggcgggcacc ccggttccga 44460
ggtccaaagt gccctaacga aagggcagca aaggccacct gcggggtccc accacttccc 44520
cccactcccc tgtccaggca tactcaaaag tttgttcctt tccgtccgta agcggccccg 44580
aggccggctc tggagtaggt gggggaggta agaaggactg ggggtggggg agggcaagat 44640
catcggacca ggagatcagg gacccctgcg tagcccccac tcagtccagg ggggctgcga 44700
tcgtgtttac taataacagc aacaaacaat cacacgccag cccttctttc agtcctcggg 44760
ctctcttcgc tttttggggg cagcaaagga gagtctggcg ggaggtgggg agggagcgga 44820
ggggctgagg agagcgccgg ctgggggaca aaaagcggcg tgggatgggg cagaaagaga 44880
agcggggaga gctggacaga gacgaaggct caaaggtaga agagacatgg gaggaaagag 44940
gccaaagaaa agtgcagcag gcggtgagcc cgagcgcggg gcgcgcccag gatggggagg 45000
tggcccccgc cccccggccc accggcccag cccggtcacc ttgctgtagc cgtagtaccc 45060
caggcactgc gcgaagtcca ggctggcagg gtccccggcc gccgcggggt agaacctcac 45120
atccatgccg aagctgggcc cggggccggg ggccgggact ggggttcgcc ggggccggga 45180
cccgcctcct cgccgccgct agatccaccg tcgagggcgc ccgggggtgg cgcgtgggac 45240

tcgcggccgg aggggcgccg ggacccagag cccgaggagc tcgggagccg cggccgccgc 45300
acacaaaggc gcggccacgc gagccgcggg agagcgggag gcggccgggg ggacgcgccc 45360
cgccggggca ccgaggcagc gctgcgcgcg ggccgggcgc cggggcgcgg gggcgcggcg 45420
ctgggggcccg ggtcggcgag gcgagttcag gtgcgctggg cgaggctggg acggcggcgg 45480
cggcggcggc tggccccgct cctcctcctc ctccccgggc ggactgagga gacgagccgc 45540
ggagacaagg ggcccggccc ctcccctcct tctccccctc ctgcctcagc cgccggtccc 45600
ctccccgcgc cgccgccgct ccgcccctcc caccgcgggc agctggcgcg ccgcccgccc 45660
cgccggtgcg ctcctcggcc cggaccgcct ccgggagcgc ccaccccgcc ccgccggccc 45720
catcaccccc gccgcggccg ccccctctcc ggggcccctc ttctccctac ctcgcccccc 45780
ttcccgctcc ggtcctcttt gtccccactg cctcggagcg ctcctctctc ccctcctttc 45840
ctcggccgaa gcggacgcca gccctgcgtc ccgtgtgtcc caccctcctc ctgcgcgacc 45900
acagcttccc cgggccccag catccccaag ccgtctgctc ccttcctgtc gctttcctcc 45960
ccatgacccc cttccttctt catgggtgcc tgtccccggg cccctctcct cgaccccgcc 46020
ttgcctttcg aatgtcccct gcgtggacac acctaaccca gacgccaacc tgcacgagtg 46080
tctcccggta gccgggagcc gggaccccaa gttcacctgt aggatttccc agaaggggtg 46140
ttggggcctg acctttcaca ggtaggcaga cttgaagctt cccaggtgaa gcctcgtgtc 46200
ggttctcttc gtattctctt tgctcttgtt tgtattcgaa aggcatttga gtaactggat 46260
ctaggcagat cattcactgc tctgggagag aaaatgtccc tattttccta aaaggtccta 46320
gctgattaac ttccccactc tacactcacc cacccctctt tatttcttta tctttcttaa 46380
gctccatctt tttatttaat tttttttctc atcggcttta ttgctttgag ttaatctgag 46440
agtttggcct tcagccttgg cgaagggaag gggctttata ctaaatggga acgttgtgga 46500
gaggccgtga gatctgcgcc ctccccagtt tagttatctg ccagctactt taaccagagt 46560
ggcgacattc tgaaatattt cctatgacac tcgctaacag tttcatcccc tggtcacacc 46620
ttgctggccc agggcctgag ctttttcaga caagcatgac atgggttttc ttttcatttc 46680
ttctgttctc cagagtagat cgggcttgtg acacctgtgt gtgtaccttc actcaatatt 46740
atctttctgg aacggagcag aaaaaaataa acttcaatta ggtgaaagtt tgtgtatttg 46800
ctcttctctt agcaaaaact tgggtcagga gactactgca tgagagagca gtgtgtgttg 46860
agaaaagaaa caactgccca cgttgtccct gggttgactc tgatccccac cctcccgaaa 46920
aagctttggt ttcccctccc tagtctccat ttaacgttga caacccagcg gtgctcccta 46980
atggcccagc aggttgcaca aattgacagt gagctgagat attatcagga caacacccca 47040
ttgagcaagt tcttgaaacg cagaagtgga ggccctttct ctgggccact gggcgcctgt 47100
ggcctttcca ccaatcagag ctggaggttg cagtggccac ctgttgtctc gcaagagaac 47160
atcttttaag tttcgctatt agtatttaac aatggcctac agagcaggat ctggccacag 47220
gagaggagca gtgtgatgat ctggggtacc aaaaaagaat tttttaaaaa aaaaatttta 47280
cagatagctc ttacaaggag ccaaaactct ggaaagctaa gtttttcttt gcacactcac 47340
aatcctcacc ttctgctcat tctgttttgc atctgaagca atctgagctt gattaaagga 47400
tgaagaaaag tataatcaaa gttacagaga ctaaacctga acttgtaatc agacttcgag 47460
gatcaggaat ataaatacgc ttcgcagtgg aaacccgcta gctgcaagaa tgggaattgc 47520
aagtgggctg atgtactgca ttcagctttg ggcggtagag cctagaaaat gaagcgagaa 47580
ctttacccct aaggctctca ggagttagga atgctcggtg acaatcccta actcctggcc 47640
attgttttgg aaaagtcttt ctcttaaatt tcccttccct gttaggatgc aaataccttt 47700
gtgaagggaa caatactcat ctgtctgcat tttattattt gatttaggat ataaggtaag 47760
caaaagatct gagtttagat tttgatttag agggagggga agaatccggg tgatgagctg 47820
aagatgtgta tgttgaggca tttagaatga gaaaatcact ggaacagaaa aagggcattg 47880
gtgaaggctg ctgacttgac gatttagccc aatctccacg catttggcag ctatctttat 47940
ttattgtttt tttttttttt ttttttttga gtacctacca tgtgccagac atgattttag 48000
gcacttgtga atgagaaaga ggattcaatt cccgacatga cacatataga agtcctcaaa 48060
gaggactatg attttggctc ttggaacaca aaatacacac gaactcacat gcacacgtgt 48120
atacatgccc tgaaccatcc cagtcacctg gagatactgt catggcacag ccactagctg 48180
ctgttgagag aagcgacatg aaaaacaaac tcacatacag tgtgaaggat gccagcttct 48240
acacccccat ggtatttgta taagaaggct caaggatgaa ggcagtgcat cccttgctac 48300
atcattgctg gatcccaaga acctcagacc atgcttgata catagtttgt ttgcaggaaa 48360
taaatattca ttaaagaaag aaagtttaga aagaacatgg gaaggaagaa ggaaaaaaac 48420
acagaatgtt ttaatagtgt aatttatttg cagggaacct taaggcagga tttgagaaga 48480
gtatatccag gacctctgat gtgaattaga acaatctact ttgggtgggg tgggggcctt 48540
ttgcaccctg ctgttgtaga cccttcgcag acataaaaac taagatccta tgtggtttcc 48600
tttagaactg atctgattac actctccatt gctttcgcac tatcatctaa ttttaagagt 48660
tccagtggtt ttgaagacta tccattaaaa actttagaat aatgctttaa aaaattaaat 48720
gcaattgtaa ctgtcatggt atttgttatc ttaagggtgt cactattttc agtctgtcag 48780
cagtcttgtt tctgaaagcc atagacaaag gtttccttta tactggatta gccttgaatt 48840
tgaagaacct tctccaagaa catatcttaa atatgctttg aatttatgct gaacaatgct 48900
aacagttatt gatttagaag ggcagcagaa gaatcaattc tgcagccaaa atacaacttc 48960
tgggcataag aaaattcctt tagggttttg agatgattcg gatcaaaact gtccatctta 49020

ttataatgct tgatgtttct aattaaatag ttgatgtcat ccaagaatcc cttaaaaatg 49080
gggagaaaat acacaaactt tacattgtgg gaaaactacc ttacatcttg acggaacctg 49140
aaaactttct ctactttatc tggtttcaaa ttatgcaaga aagtccccat gaggacttca 49200
ttaaacctgc cagagtttta taaccaaaaa cctcactgga acccccactt ttacgaatct 49260
atgaaagtaa tttaacccct ctgtgttctg cttgcccaag atcagatgca aatactatta 49320
aacagtatat acagtttaat tctcttgagt tttggtgatg aaaaatccta tccaaatatt 49380
atgatccttg caatacgtaa tgtaaccaaa ttaacgatag ctccaggtgg aaattaacaa 49440
ggccagggct actcacgttt cctctcatct attcccatag taacagctaa tgtattatat 49500
tgctaaaata tcttatattc agttttatgt ctgaacttgg tgttgtgtca taaagcgtag 49560
cattcctatc cagactggat tcttaatatg tgtatcctca tgaaatttag gagagtttcg 49620
tgtctgcaaa tcaacacttc atgtgcatag tctaaaccac atgcagataa aagcaagtca 49680
gaacctacca agactctcac catagtgaac ctacacatgg tatagaggaa gaattcttac 49740
ctgggagacc tgatgacaca ttaccagtgc aggacctaaa gcgaatcacc atagagagat 49800
ccacacagga attatagtgt gaaaaaaaat ccccaatcat agacattgtt agcttcagtt 49860
ttcagtgaat ctaaactctt ctagtatgaa aatgtgagct attttgatat ttttactttg 49920
gtcaaaaaaa cacagaaatc caaaaaatac agaaatctgg gaaatgggat aaaaataagc 49980
aaacttttcc ttcatgataa taaatatata ttcagttgtg tacatgaggt tgtttcccat 50040
actactacaa ttttagtcct attggtctct gtattaccag gcacaagaag ggctctagaa 50100
aactaatcat tttgtcagtt gactattact tttacaaaga atagataaca cccataggtt 50160
ctgacagata catagtaatt atggaacagt cacctccaac accctggtag caaaaaacta 50220
ttaagccaag aattcattac cacaaatatg gaatgcagca agtatttgct tgtgacctaa 50280
gtagctcttc aataaagata ttttagaaac caaaacactc cacacaatca ggaggtgtgc 50340
aattttcact gtatttacac tctgccttaa aaaaaaattt gtatgttgtc ctgcctgttt 50400
tctgaaatat attctatttc ttttaaagtg aaagaatgac ctcttttgca ggaacgattt 50460
tcagagacag cgtgtttatc aaatggtttc tctgctttca taaaaacaat ttattttcac 50520
tgaactcttc acgataagca tcaaagccag gaagaatgta caatgaagtt tacaatttca 50580
ctccagaaac aaatactggg tagttattat tttgcttaag tgaaaaacat tttttttccc 50640
caaggagaca aaggtagtaa tgggggaggg ggtttataca ggagtgaaaa tcaggaagtc 50700
cagacagtct tcagcaggag gggcccctgg tgttgtccac aaagaccacc ggctgaacaa 50760
tgaaagctga agacccagta ctttcttcgc aaatgggtgg gggtcagtcc acagttttat 50820
tcttcgctaa gagacagcta tagaggcatt aaggagagaa aatcattagg cagatataat 50880
taaacccttc tatgacagat ctatatacca ttgcacatag atatctctca tagttggtgc 50940
tttggatgac aatcctttct ttgataaaca tccatgtttc taggcttaat ttatatgaca 51000
tgttattttt taataaccat agaacaataa aattgtaaaa ataaccaaga aaagaactga 51060
gtctaataaa gattctgttt cttaagaaat gggaaacaat tgcatttgat cacagaacag 51120
ccaaggacta gcagcaaaat aatctctcct aatttacata gatctgtgcg tgccaaacag 51180
atgagtgttt gcttttcgag taatagtgca ggtgcgtatc ctgcagtgac taaatctcta 51240
actcatcaaa ctgtttcgcc agaatgggac atgccctctt tttctggaac agtttcaagt 51300
tttctcccca gcattccagc cacctgttta tggagttcat cctcgggaga agatgcatgc 51360
aattagaaac aagaaaatga aaataaatga ccttgtttgt tatttataac aaagaaactc 51420
tgcaaaatta atgctgtgaa gcttgacctc ctgacccagg aaaaagagcc acctcatggc 51480
tttgtggcca atggacaata gtctggcagc tcccattgtc cagccaccct gacttttgga 51540
tggtcattaa agccacatat ccaaaatatt aaatgtttta caagttagat tagaggattt 51600
gcacttttta aaatgcgtgt tttaaaaact gaggttagtt agcacaagag attttctttt 51660
taaggacagc aggtaaaaat catttagctt catctactcc atctatcctg tgtcaacaac 51720
atgttgaagg aaaggaaaag atttaaaaat tctgaactag tgctttggga attgaagaat 51780
ggttaaatat ataatagaaa tcaaatagaa aggtggtctt tgaaagtggt tcagggaaaa 51840
cattgtgttt cctccctgct gaggcctcag ggaggaaagg gggaattaca atgttctgtt 51900
acataaatgc agtgtggatg gtgcccccta gagttgtgcg atgaaatagc attgctgttt 51960
ttccttccac ctcttatccc aacccacaca tctagaagcc cagggcagga aggaaatgaa 52020
ggtggtaatg gagttttgaa gactcttcag aactggtgct tgggtggaac atcttgaggt 52080
aggatggcga ctgaaaatga cactctctgg tgtggctgat tctgaagctg gcctctccta 52140
ctgcatcttg cctctttccc catttggtct cctccttcat tcacccacaa caaattccat 52200
gagtgtcctc tattagtcta aaccttagag taaaatccgt gtttgatttt tttccatcca 52260
agttgcttat ttattggcca atctgcccag tgctactaca tctttaattg ttttattttt 52320
ccccaggagg aagtaactgt ttattcaaga aggttctcta ctgttcctca aaatgtcttc 52380
ttcactgtga aataaagtca gtttcataag gcatgcagat tgtcatgcaa acaccaccct 52440
gagcattcta gatcatacct cctcaccttc ccttttcttt ctgatcccct gagggtagct 52500
cattatatgc ccctccctac cccttcttgg gaatgctgca gccataaatc tggagaggtc 52560
attagaatta cgaactctgg ttggtgtctc acatacctac aacagcaaca acaacaacac 52620
aattatagcc actgtagagc caatgtcctg gttaatgttt gtgtaactgg ccaattctca 52680
ggcccagaga aaggagttta agaaagtttt tgcatctcac cagtctaaaa cttcttctgc 52740
agtccaaaac cacatttcaa ctattgttta actgaaagac tgagccctct taatctggaa 52800

ccaccaacta aattccccct gtgtgcttaa acaccaagtt agagagctgt gttcagtgcc 52860
agtgagaaaa agtagagtgt gattacccca tcttacccac agctgaaacc tgtgtcattt 52920
atttttcaac aaatgctgac tgtgcctttc taactatatg ctcctacgtt tccagaaatg 52980
ggaaaaagca tgatccctgt ttatactaaa catgacaaga catgaggaaa gtagtataat 53040
aatgaaatat tgtaatgagt gccataggaa aagcattctt aggctagtgt agtcaacaaa 53100
catttattta cagaaattgt tacaggtaat atgttagtgt gctgcttaga aaaggcgggg 53160
atcatttatg atcaggataa tcagaggaaa cttcatggag aaggcatttg aaagtagata 53220
gcattttggt gaggcaaaag ggatgaaaaa atacactagt ggagggagat tttgaacaca 53280
gttattagag agaaaatatt ccatgtgtgg tgggaatttt gggttgcatc agccatttgt 53340
tgggtagatt aagcttcagt aacaaataac ctttaatatc ttagtgggtt accagcacag 53400
aggactattt ctcagccccg ttccagtcca ccacccatca gctgctgttc atttgcacat 53460
cttcatctca ggaccaaagc tgaaaaagaa gcttctatct ggggcatgtg ggtcttgtag 53520
cagaggaaaa agagagagtg gaaacccgct acagttgtca aagctgctca cctcattccc 53580
gctcactttt cactgggcaa agcaaatcag atggccaagc cccatgtcaa tggggcagaa 53640
agtatgatcc tcccggaggg actggtctgg caaagagtaa ggaggagtcc cctattttgc 53700
aataatgtag tcggtgccat ggaaagagtg ttttagagac ctaccaaaag tttcaggcag 53760
caagcaaaat tgtttagtaa aaggagtgag tgagtgttga ggggaagagt ttggtgagat 53820
aagatgccgg gaagtgatag ggggaaggag acaggctggg aagtgaaaca ttataaagta 53880
ttgaaggctg ggcagctgag ttagattcga tgcaaaagga tgtatgatgg catgaaaaaa 53940
cgcagctgtg agttctgatc taaagtttgt tttgacccat atagagcaga ctcatttaac 54000
atttaattag tttatactgt gtgtcagttg tggtggcagg tagacatttg catagatcta 54060
tattttaaat tttgatcctc atgacacctc tctgggttag ataagtatta ttctctttgt 54120
taagctgaag gaggctcacc aaggttgaat gacttgctta aggacacaaa agcagcacac 54180
agaactgtga tgcaaagtga ggtctttcta ctgcatcact gcagggcaaa gaaattaaaa 54240
tgtcaccttt ctttagtatt gacttgttat tctttatctt ctccttaaat acacagtctg 54300
aaagcaaggt taatatgatg ctttttctca tattaattaa aatgatgttt atataactaa 54360
tccttgcata gtacaacttc ataagtgaat aaaaaagaga cagatcttta actgaggtat 54420
ttattataaa atttcgtggc agagacagtt acttctaacc aaatacagaa tgctctccac 54480
tatgtatttc ccagtttttc ctgctgttag gtgaggagca gctgaggcaa taaagaattg 54540
gtgtgcttcc tccatatctt tcttgacctg ctgtggtgtc cttggaagac acatgcacca 54600
gatggaagaa gacaattcat gcctcagaat tcacatgagc gaaaccatgt cttgatggta 54660
ttgcactaca agatatccag tgtctatttg ttacagcagc atagcccaac ctatcctgat 54720
taatacagat ccttctgact ttaattgata caaggaaagt gaccatatct gtcttgcaca 54780
tcctcttatc cctactgctg aatacaggac ctcccacata atatatgctc aataaagttt 54840
gattagtgga aaatgaatga atctcaaatt gagtctggcc tctgtctact atttggttaa 54900
accacttatt tactgctttt agaaaccagt ggttgatcaa tctttttgat atatcatttc 54960
atcagatcac tcctcagatt aagactttat tatatattta atcaatttta aactccttcc 55020
tttgacattc aaggtcccca taatctcacc tagattccta caactttacc aatttattgc 55080
tctaatccaa tctaacctat cactactggc ctgtgacccc tggtcactat attcactgtt 55140
ctagggacaa acatttagtg ttcttaagca aatcatttca agtctctgag cctcagtttc 55200
tcattctgta aagttgttat ttgtagcaaa agacataatg catacaaaag tttaagttat 55260
gcagcattat gaaaatccaa atttattttg tacttgttgt tggtatatat taaataaatt 55320
gatagatcag aggagtgaca ttaaaaaatg tgttgccatt tcagtagcac atttgtcaaa 55380
atctcccatg acaatcttgc atggaattgc tgtctaggga gaaatggtaa aggaaaccag 55440
ggagaccctg ttggttatct actccaaatt cattccctct tttttttctt aactctacct 55500
ctattttatt taagttctgc ccttctcctt gggaccatat ttatctaggg aggatccttt 55560
acaatggccc tattttcact gccaatgatt actgtagtca tgaactctga tgcgtttctg 55620
gttgataagt tgtggaaaag agtgtaagag ggcttctaag aagggtttct gtgcttttaa 55680
aaagaaacac tcagaaaata tataataatt tcttcttctg gacagtgtca tgtctcgaaa 55740
tgttgcagcc agctcatcag catgaaagga tctctagccc gaggtcagag ctgacacgct 55800
gagatgagaa tggagggcca gaaacacgga aagaacatag atccttgatg atgtcaggag 55860
tcactgatta actaaccctg gagctccttc tgtcctgatt tctagttatg tgaaataact 55920
cataatctta tcttttgatc taattgagtc ctagtttctt gttacgtgca atggaaagca 55980
gtgaaaccct cttagactga gtccattagg gtacccgtaa caaaatacta taggctgggt 56040
ggctttcata acagaaattc atattctcat ggttatgaag gctggaagtc ccagatcaag 56100
gtgcagcagg tttagattct cttgaagcct ctctccttgg cttgcggatg gccaccttct 56160
tgctgtgtcc tcacatggcc ctccctctgt gagtgcacat ctctggtgtc tcttcctctc 56220
tcataaagtc acaaggcaga tttgattagg gcccacccta agggcctcat tttaacctaa 56280
ccacctcttt aaaggctctg tctccaaata cagtcacatt ctgaggtact gggggttaga 56340
gctttgaaat atgaattttt tttttctttt attttttttt tgagatggag tctcactctg 56400
tcgcctaggc tggagtccag cagtgtgatc ttggctcact gcaacctccg cctcctgtgt 56460
ttaagtgatt ctcctgcctc agcctcccga gtagctggga ttacaggcac atgccaccac 56520
acccggctaa tttttgtatt tttagtagag atgggtttca ccatgttggt caggctggtc 56580

tcgaactctt gacctcaagt gatccgccca cctcggcctc ccaaagtgct gggattacag 56640
gcgtgagcca ccgcacctgg cccaaatatg aattttgtgg gggacacagt tcagcctata 56700
acatttgctc aagccaaaaa aaggagttta aaatttcaga aaaaagcctc acagactcca 56760
agtgcacttt atcaaagtca gacatccaga gaactgggaa gccaataggc agctaatctc 56820
ttcatgtttt tccctcttca tggcttcaat tttacatttc tctcagcatg catgctttgg 56880
tttttttcttt ctcctcaccc ttctgcttat acaaagctca tcatgagtgc ccgatgatgg 56940
gcttcagccc ctaagtcaca tgacccttat gtttggctac ccctagctag caaactcagg 57000
ctccatgcct ctttgtgaaa ttcttgagat agagaatctg attggcccag tttgtatcaa 57060
gtgtttatcc ttgatccaat aggctgtggc caggggcatg ggtcaggcgg tacacaaggg 57120
ctgcctcttg tgatctgtgg ctaagttagc tcacccagag gacttgaatg ggaataaatt 57180
ccttagcagc tctagtggaa gagggtaatg tggataaaat tatagtgtag tcaagtggat 57240
ttgaagcaga tgaaacagtc atacacaaag gtgttgattc attactgaca ggctgtaaga 57300
agtctttagt aaagtcccac tttatcttgc cctcggttct gatttttttt ttaatgttcc 57360
caatatttta ataaacagat taggacatag aaaatatacc tacgaatttt tagttgacaa 57420
aatgatggaa acatttgtta agatagttag aaatgagatt cagactcaaa atcatcttaa 57480
agggctagaa atctgtgtag aatcaataag ataaaaatta gctcacataa atattaactt 57540
ccaaatttgg atttcagtgc tcacttgcaa agtgatggaa tggagttttc aaaatgaact 57600
cttaaataaa ttatagcact ctgtggatat gtgtctctct ttccaagaag gaagtccttt 57660
ttaattagtc gcatgctaaa gttagtttgg ctaagtaaat gttttcctag ggttgggaga 57720
tttctataat ttcagtttcc cttttgtttg cagatcaagt agtttaaatg acccagtaaa 57780
taatttggag tcaccacagt taactcacca accttgaaaa tccctgaacc aagaggcccc 57840
ttgatactgt aagggagggc taccataatt aatagcataa gtctaaccag ggtaccatac 57900
ctttccctat attgctggat attctgtaag tttggctttc ccaaattagg acacagagat 57960
aaggataaac tcactttcca gctatttcgt tatgcctttt ttatttcttt caacttttgg 58020
aaatgacctt atgaatttcc tcttattatg aaatatatct ttattgaaaa ctggagtcca 58080
aacattacaa aaaatacata gcataggaag tgaaatgaaa tactgcataa ccaatgtaaa 58140
tgattaggtg actcctcttt cagattacat tcctgagcat tttctaacaa attatcaata 58200
tttttatttt aatgaatata gaatcacacc ctacttattc ttttgtaatt tgcttttccc 58260
atgtaataat atggcctgca ggctgtaatg atagacttgt tttttgcaga acaatccatt 58320
agccaagggc aactagaaaa gccagacaaa atattcgatt gatttggttt gaaggcgtga 58380
gagagctacc atgtctgcaa ggacttgagg ggctaagatt tttgagaaaa gggaagtaca 58440
aggagtgtgc tcgacattca gtctgctttc ttctcaaggt ctttagctga ttcacaagta 58500
gtggctgaga gcagagaagc tgagctaagc ttctggtagt cttatcatac tgaagggaaa 58560
aattagaatt cagagcccta aaaggaaaag agtccgtgat aagcatccca aggtttttgt 58620
tgacgtccac caagggctat accctagaac ataggagtga accagaaata gaccaaccct 58680
cacaaagaac aaagtctagc tttgattaaa cattatccgt gattggatta ggtgatctgc 58740
ctctatgcta acagtttact aaaaataaaa atgaatcttc cttgaaagaa aataaaatta 58800
ccaagaggct caaattatct taatcttata tacacatctg gcatctaatt tcaaactacc 58860
agtcatttaa ggagacaaga ccacatgact gaagaaatag atgagagggg cggaccctaa 58920
gagtgtcggg atattggagt tgctagacat tgactttaaa ataactgtga ttacatggtc 58980
attctccact cacattactc aagagatact tagtacatgg acttagcaca tgagctgtgt 59040
atgtaatagt ccctcaacaa atatttaatg atatgaactg ataatccaaa ataatggtac 59100
taatatattt gcaattttaa cctaagtaat atatgggcaa ttggggtttc tgaacaccct 59160
attaagtctt actgtctgag ggctccatta aatcacctca ggttccttga gatgatttaa 59220
tggagcccac agacactaaa ttacatggag aaagtcactc ttttctattt tttttaagag 59280
aaggttctca ctctgttgtc caggctgtag tgcagtagtg caatcatagc tcactgcagc 59340
ctcaaactcc tgggctcaag tgatcctcct gcctcagcct cttgagtagc tgggactaca 59400
ggcatgcatc atcatatcta actaagtttc taacaaaaat tttgtaaaga cagggtctca 59460
ctatgttgcc caagctagtc ttaaactcct ggcctcaagt gatcctctta cctcccaaag 59520
tgctagaatt ataggtgtga gatacctggc cccttttctg tttcctttta gtccttctga 59580
ttatgccata gagggagtct cagcttggtg ctgacgtgtc ttttatacca tcctaatgct 59640
atttgatttt tttttttttt aacagagaaa gatctcaagc ttctcagtca atgtcttggt 59700
atctctactc tggtgttctt aaggaaaatg ctatgggctc ctttggacat ctggtaaagt 59760
ctatgaaccc tatatcaaaa taatgcttgt aaattcttac aataaaatac ctagtattac 59820
aaagtaaaga atatgttgaa atacagttcc atccaccatc cttttgccac tacagtttgt 59880
gattgttgta tttattttca tggttacttt ctacttatgg aaagtgcaat tggtttctgt 59940
ttatagtcat gacatacaat ttgcatttta aatacatttg tttaaattaa agagggaaga 60000
tgaattaagg aaataagtaa aagtaggaaa tatgacaaag ctaaaagtgg tgtgctgtga 60060
ttgagtgacg aatgcagact taacccaaga gatgacttta ttaatgggaa tttcaggcac 60120
cctttcacat tggggactgt actaaaccag gcttgacaca agcctcactt gtccatcagt 60180
caatgtccat tgattgagca gccccaccaa gtaccagaca ctgttccagg tgtgagggca 60240
gggcattgag caaggcaatt tattctcaag gtgtttacat tgtagggaaa ggatatagac 60300
aagaggcagg ccaaaacatt caattaaaag gtaacttcag atactgattt ttgtcctgaa 60360

gggcaaaagg gtgactagca gggtgactag agagggagtg acttggggta ggtgtaggac 60420
agtagcagtg gggtttgtgg cttggcccag ggctgcatta gaaaaagacc aggctcccac 60480
tgcagcagtg cccggaaaaa tgcagagacg agccaggatt tctggatcat taccatgagg 60540
aagggaataa atgtgtgttc tctttcagtt gctcctcttg acacctgggt aaatggctgt 60600
ttacacaggc agatcaccca gccgctccct caaaggtaaa cagctgcttt ctgcctcccc 60660
tgctagcaca gattggggcc ctgaaacagt gtgtacccag gcaaaagagg aggtattctc 60720
catttaggtt tccattcatt cattcaggca agatttcttg agtgcctcct aaaatgcagg 60780
caccaaggta ggtactggga acacagtaag gaacaataaa gaggtccctc ctcccccacc 60840
tctctctcct ctccacttag aatccaagct agaggtgtgc ctcagagtct caggccactc 60900
ctctcatgat acagatggaa atatcagaat cagaagaggc aaagtgactt gcccaagacc 60960
cggagctagc ccacttcaag caagtcaggt ctcaaaacgg tgccagctgc tttcattgcc 61020
tactacctga cttgctcaat cttggtcagt atttggattg gcacttttta gtgtattctc 61080
cattactaga ctattatggc ttagggaagg agaaaccaga ggtccatcct acaagcacac 61140
tgaaatgtaa tccaattcta agtccccgta ttacaacatg gactattgtg actgaccaca 61200
tttgccttat atttaccttc ttccgcaggg atatgcccga tttcagtttc ccatgttgaa 61260
atagcctaca gttgagacaa acatacatta catttaagca taccttccat tttttataag 61320
gtactatatt tttaattgtt tcaatcactg tgaaattaac aaatgttcat gagcacccac 61380
tgtgggagct cacagtgtgg gagttccgcc ctggtggagg tactgggatt taatgataaa 61440
ccaaagacaa atatatgctc aatctggtct ctgccctcat atctagtctg ggaggtaagc 61500
atttgtcaaa taatcgcaca tatatgattg tgtatgtatg tataaaacta aacgctgtca 61560
gttgtttaat ttaaaaaggt caaaaagaag tttgctttga agaatgagat caaggaataa 61620
aaattagtca gttaaaaaaa attacatata taaacataaa tttaccacca taatagatac 61680
ttgttttagt gcaggtcctc cgtgatgcac actaagacag gaataaacag gtaaggattt 61740
tattggggaa aatgcctgtg agtgaaaatt ggaggtcaag cacgatggct catgcatata 61800
atcccagcac tttgggaggc cgaggtaggt aggctgcttg agcccaggag ttcaaaatga 61860
gcctgggaaa cacagcaaaa ccccatctct acaaaaaaac atacaaaaat tagccagcta 61920
tggtgccgca tgcctgtcat cccacctact gggaggctga ggtgggagaa tcacttgagc 61980
cagggagttc caggctacag tgagtcttga tcacgccact gtgctccagc ctgggcaaca 62040
gaccgagacc gtgtctcaaa aaaataaata aataaaataa aataaatgaa aagaaaaagt 62100
aaaagaaaat ggtgggggag gctggagaag gccaggaaag ccatcagact gggaagtagt 62160
tctgtgaaga ggagagagga ggatggatgg tcttagattc agtgtcatcc taaggaagtt 62220
ctggcaaggc cacatttgcc catcagaaga gtcccccttt ctcaaggatg gaactggctt 62280
agttttcctg ctgggcttag ctacaagctg ggaacagcca ttgggaagtg tggtctctgc 62340
agacatgtgg tgatggattt ggagtacagc agctggggct gtccatcagt aagggatcta 62400
agaattacat gctcatggcc actgcaatgc taaaatagag caaaacacaa taaaaacaag 62460
ataaattaga gggacatgat ctggtctggg acaaggaaga ttgtttcaga aaatagtctc 62520
tgtcttacat tcacaaggga aaagagcaat gttccagaaa gagtggcgag tgcaaaggcc 62580
ctgtggcaga aggcagcatg ggatgttcaa gaaactgaaa gaaggccaga gcagcctaat 62640
ggaaataata taggagagag tggaatggga ggctgagagg tgagcattgc tggggacccc 62700
tacaaggcct ttaaggagag gacattgttt taaacacaca tctaaaactc actacttaca 62760
tgacagacat agggcagagg gtggagtact aggcgtgtgc ctgggtttcc aggtggctga 62820
actgtagcat gactggcggg actgaaggag tggagaggct aaggtcttga tgtcacctac 62880
ttttgttagg attagaacag gggtcacaaa ctgttggacc ccatggcaaa tgtaattttc 62940
tttttcaatt ttttattttt tgagacagtc tcactctgtc acacaggcta gagtgcagtg 63000
gcatgatctc agctcgcggc agcctcaaac tccctggctc aagccatgct cccccacctc 63060
agcctcctga gtagctggga ctacaggcac atgccactcc acccggctaa ttttttttct 63120
ttttcttttt tgtagagatg gggtctcacc ttgttgttca ggttgatctc gaattcttgg 63180
gctcaagcag tcctcccatc taggcttccc aaaatgctgg gattacagac atgagccact 63240
gcacccagcc acaaagataa cctaaagatg tgtttacttt gacccaggca gtagtttaaa 63300
aaagttttaa tttgttgttc acatttaaaa actggacaat ttctacataa aaatctgaat 63360
tactcatgtc tcttaaaaaa ataacatcta gcaatggtag gcccacattc cttcctgaaa 63420
ataattagct gggaaagagt agggactgac ccctttagac acggtataaa tagcatggga 63480
gttgatcagt aaatatttgc tgaatgaaag aatacatgaa tgaaaagtca gagccctata 63540
ggtcagcatg gacggcggta aaggaacctg gctgagcctg aaagagaatg tgatctaaga 63600
ttaaatccag gatatgctgg taaatgttta acagccaact ctttggggag gaaaaaagtc 63660
ccaatttgta gtgtttgccg attattgtga tgtaaatact cccatcatga ccaatttcaa 63720
gctaccaaca tgctgacact gaacttggag ttggaaggag atgaacaggc ataatcaggt 63780
ctcgtgagat ggcccaagcc ggccccagca ctccactgtt atatatgagg ctagaattac 63840
tacataactg gaatagcaac tttctggacc atatgcctgg aacacagcag gtgctgaata 63900
aatgtttgtt gatccaggaa ctgactgtgt tgaagcccac agatgggaaa tcagtagaag 63960
gcaggtaaga gtaaaaagaa gggcagagaa ttgggggtac agacccctga accataagtc 64020
agaggaatgt tgtacatgtt ttcagatccc tcactggtca aatgaaggca aagggttaga 64080
tctctccaaa tctttagagg gacatgatgt aactccatta agtaactcag tgattttcaa 64140

cattaaaaag tgtaattatc ttttcaaact aaatattaca aataaattct gataaaagga 64200
aatgtacgtc tgttgagacc cctgatccta taactggttt agtattccct gaagccactt 64260
gtagtagcct attcacatgg ggactcccca gtgaacattt cctcatatcc acactcttgt 64320
gaaggtcctt caccttgaat ctggactggg cctatggcct gatttgatgc atagaatgca 64380
cagatgagaa taggtgggtt tctggcataa gctcaaagaa ggcacagtgg cttccagagc 64440
cctgagctac tagtaagaaa gtccagctac cctgctggga aggccatgga gagaggccat 64500
ggggagctca tagagaaaga gagggaccct gagactacat ggagctagga agaacaacac 64560
ccagccatcc cagtgtccca gctgagcccc actccaaacc catctgccaa ctgagcacat 64620
ctacaagagt aaccactgac aggccagcac aagcccagcc aagtccagtt caaattgcag 64680
attgtaagca aataaagcag ttgatgtttt aaggttttta gatgatctta gagtttagat 64740
tatgttaagt atgtgagatc cctaaatagc catcaataaa aaaattgatc agtacaatgg 64800
agaactgcac aactattaaa agaagaaggc aaatccatgt ttaccgacat gaaagaaaat 64860
caccaagatt taccctaaca tgacaaagca agattcagaa gaatctgtag ggtatagtac 64920
gggatttgcc attgcatgaa aaagggaagg taagtatttg tgcatgcgtg catgtgcatt 64980
tgtgtgtgtg tgtgtgtgtg tgtgactata aatttgtaaa tgcatagaag atttctggaa 65040
agatacataa gacattagaa cagactggaa gacttgaaga agaggggaga ttgtgtatcc 65100
ttttgtacat attttcattg tgtatccttt tgtacatatt tttaccttat ttgcaggtat 65160
cacttattca aatcatagaa ataacatttt aacaaaaaag tgaggcctgc ttatataatg 65220
ttgaagacaa ctagctacca cactatggga acacactatt atattaaact tgatgattcg 65280
agttttgcct aaattttact cagaatcgaa tacaagaata gaggaaagaa tttaaaaccc 65340
atctggctaa tgcataccct tgccaactat tctattagaa aggaagatgg cagtggggag 65400
ggggttctta caatggaaaa ggtattggct tccctagtgc ctgaggtcgg gttctccaag 65460
aagcaaattc agagtgaagg atttaatgaa gagtaatcta tgtgggaagt ggtcccaaga 65520
agcaccaaaa cagaagtggg gaaaggacac agagaaggaa aggaagtcac tgaagggact 65580
gtgagttacc tttgtgggca actggggttt cgtcccactg gggacctctg ggagacactg 65640
taggacatgt ttcagggtgt cccatcctaa gtgggaggaa tctggagaat ttatactctg 65700
ctcccagggg catcaattct ccagtgtttc cagtcagctc tgcttgctgg ctgagatacc 65760
agagcaagtg ctggtctggg gaagttgtca gcgtgtagag gaggtgagta ctgagcagat 65820
acaggcaggg cactgtcagt gcatttgctt acctagacag caaagccaga gacaaaagct 65880
cgtgtgctta tgtggcagga gagagagagc gaaccatgca gggaagtgca ttactttatg 65940
attttttttt tttttgagat ggagttttgc tcttgttgac caggctggag tgcagtggtg 66000
ctatatcagt tcactgcaac gtctccctcc cagtttcaag caattctcct gccctcagcc 66060
tcccaggtag ctgggattac aggcgctcat caccaagcct ggctattttt tttttttttg 66120
tattttttag tggagatgga gttttgccat gttggccagg ctggtctcaa actcctgacc 66180
tcaggtgatc cacctgcctc agcctcccaa agtgctggga ttacaggcgt gagccaccgt 66240
gcccagccaa caagttgatt ttttagtttt atcagatgtt ttctaggaga ccataggaag 66300
ctactttgtc tacacattac gcctctgtaa aagaggaagg aagaaatttc caggggactt 66360
cggctcctct gcacttctgc attgtccatg taaaggtgct tattgagtat ggattgtggt 66420
gtcttgcacc ttggtggcag caggaaagtc ccggggcagg aagtgagtgg tacataatgc 66480
aggagagggg agaggaggtg cctgataacc ctgagagtct gggagctgct gccccagtga 66540
gctcctccat ctgtaacact ggcggaaact gtagtaagca ggctttacaa cagtgggaaa 66600
catgcatgcc gatgtcaggg cctcccatcc aaaagcaagg ccaggatgga tccagggtgg 66660
tgaataaatg aaacctaatc cagaacctaa caaaggagac tgcgccaagg ctaagaggag 66720
tgccctgaca gttcacagca tcttagagca cattgcacca cattatgcta tttcaccaag 66780
gagaaaattc ataaacattt ttaaaagtag attctcaaga ctagacatta ttttaatatt 66840
gcttattgat gattataagg taaagaatgt tggtgagcag gtagaaaaga aaggtaatta 66900
ctaggaccta attctggttc tccagaaata gatcatagtg taccaacaaa tttccatttt 66960
tgatagaatt actttgctga tatacattga tctaagcatt gcttctggcc atgtcactga 67020
taatattttt ctggacaaca tgccaaaaca caggtggaat gactgtacag tcaaaaatat 67080
tttttgacta tacagcagat ttcaatcaac tatactcagc tctctagaga tatgccacag 67140
gtgcttgttt ttactcttgt gtttattgta ttcgatattc tttcctcaat gatttggaag 67200
aagatgtagc aggcatactc accacattta tggaaggaat cattggcatg tttgatggca 67260
gaatcaggat tcaaattgta ctgacaggtc aggatgttag gtcaacacca accatatgca 67320
atttaagagt aatagaatct gtatttaaca ttaaaaagtc agttgtgttg gcacaggatt 67380
ggggagttgg attgacagca attgagttgg aaaagacctg tgggttttaa ttaacccaaa 67440
gttcaataag atccaacaat gaaacagctt attttttaaa gcaattttag gctgcagtaa 67500
taaaagcctt attctcagct gaagagcagt gatgatccta atagattctg tgaggctaca 67560
tctggagtat tgtgtactca gttctgggtg aaagagtgag caattacata caggagggat 67620
ttcaaatgca tgtcacacaa ggactggtta tcagcaccgg ggataaatgc gaacaaaatg 67680
ataatatttt acaggaataa tagtgacctc catctatttg atgagtattc aggtgtaaaa 67740
gggagtaaac ctattctttt tgctctatgc tttagtcagc tatggtcaca ttaataccat 67800
gttggcttgt aataacatct atttatctct tatgcattca cagataagct ggagtttgtc 67860
tgatctaatg taggcttggc tgggcttggc tccaggccat gattgtggtt caggtttgct 67920

gcatgtgttt ctttttcttt gggaatcagt aggtgcccct gggaatattt ttcttacagt 67980
gatagaagag gcacacgagg gtgcatgcat taaaagcctc tgctgatatc atgtctgcca 68040
atatccattg cccaagttaa gccatcccaa atttaagggg cttcagccac tgtgaggcca 68100
tgggaaggga actgcatgta ttcctattat ggagtagtgg aacaaataat ttaatttatt 68160
ccactccaga ctagcaggtc aaaatgaggg tcagtggtct gaagctatag gaagatagat 68220
tttggtaata aaaaactgga tgatcgttgg aactgtccaa agatggatgc aaacaactga 68280
agtctttgtc ccagaaggga ttagacagag ttcaatgggt catttcccaa gagcatccca 68340
gatggaattc cggtgtcatt tggaaagtta gagactgagc cagttagtat ctttcctact 68400
ccaacatcct caaattccat agtcctgttc tatcccatac tcttcctctt gactctgaca 68460
tcctgaggct tttactctct gtgccattcc ttggggaacc taattatata tggcttcttt 68520
gtgtctctaa gagattccat gagtatttct tgtcttccta attgctttgg aagcatttca 68580
gtgtgcaggc tgagtcttaa gtttctctta aattgcctat agctattagc tcagtgtcag 68640
gcaccaagcc cttagcatgg cattaaatca tcacccaaca cccagtgcag cctcaaacca 68700
tctttgcagc ctctctccta cctttcccat cttattatcc agccgaattg agatacctgt 68760
ccttcccgat acacagcccc tgcttttcaa gtctttgctc acttatagac cattatcctc 68820
ccttgtctag accccatcca tcccactcag tgcaaaccaa gtgctacctt ttccaagatg 68880
ttttcactga tgctccaacc aaaaagaagt ttctctacct cttctgggtg gtcttaacac 68940
ttctcatggt attcgagatg tttgtggtat agaaaagtga ttctccactc taactatcca 69000
tgatgacttg agaatactta aatcatacac gcccaggtcc catcccaaga tagtctcata 69060
ttattgatct ggaaacagaa actccaagta atagcagtgt cagcaataaa gcaggaactc 69120
acatagcaaa gtgtatggaa ataagttatt ccagggttgg tatggcagtt taatgatgcc 69180
accaagaacc taggctcttt cttcctgatc tgccattccc aatatatcag caatgtcttt 69240
cctcatggtt gcaaaacagc tgctatggct ccaagcatca catcctcaca ccagcatacc 69300
aggaaggaac aaagcaagca taggaaaagt gttcttctca catatctctc ttgctcgaga 69360
aagagaacca agaagtttcc tgcaaggaac aaagcaagca taggaaaagt gttcttctca 69420
catatctctc ttgctcgaga aagagaacca ggaagtttcc tgcagacatt tcctcacacc 69480
tcattggctg gaactgggtc acatgcacat ccttgaacta tcattggcaa agggaaatgg 69540
gtcatcaaaa ttgcttaagg ccaagcagga gccattgttg gggttaaact gtcctgaaca 69600
aaatctaggc tctgaatgat aggcaagaaa gaagagggga tggccacaac ttcctttaca 69660
cggtgaataa caagagcagc ttaagtaagt agatgcccct atggggcagt aatcttgctg 69720
tgggcagaaa catgtccatg cacacagaag ctttaggaat cttcagagag gttccatagt 69780
cacttgtgaa ccactgaagc ctctttctcc aagccattgt cttgaatgct gtttttaact 69840
gattacaatg catgcagctc ccgtgcccca cttattttag cttttgagga tgttcatttc 69900
cccggaatct ccaaatataa atcaggtttt cacagaaaac cacagagccc tcacaggtgt 69960
ggaaatcatc taaagatatg ctaatgattt gggtttctag aaaaatgtat atctatgaag 70020
gaggcaggga ttaagcacag ctagtatgta tgattttttc ttttcctttc atcacctgta 70080
ataaggcagg atggagactg aatgtatcag acaaaacagg gatcaaatac tagtttcaac 70140
atttgctgcc tgtttgacct tgggcaaatt ctttaacctc tctgagcctt ggtgccttgt 70200
tctcctcatg cataaaatga accctcctgc agggatgata tgtagaccaa cactgataat 70260
aatgacagta ataccagtta ccctgtcatg ggtgtccatt ccatgacagg cctgactcta 70320
ggcattttat acacattctc ttcttttttt tttttttgag acatggtctt gctttgttgc 70380
ttaggctgga gtggagtggt gcaattgcag ctcactgtag cctcaaactc ctgggctcaa 70440
gtgatcctac catctcagcc tcctgtctga atagctggga ctacaaattt cagctaattt 70500
tttttttcat ttttttgtag aggctgaggt tttgctatat tgcccaggct ggttctcaaa 70560
cttctagcct caagtgatcc tcctgctttg gcctcccaaa ttacaggcat gagccactgc 70620
acctggccac gtcatcgtca caaaaaatgc aatgagttag gggcttgtgt tgctcccaac 70680
gtttagagta ttgaagggac cagcccaaag tcacaaagct aatgggcaag ggttgaatcc 70740
atatgtctga ctccaaagcc agttctgcag catattaatt gcatatttct tggtttcttt 70800
ttttttcctc caattatttg gtgctatgcc aaaatcatca gaattgttcc tgtaggcatt 70860
ccttttctta attacatatt gcattgttct ctttctcact ttcccttgtt tgatccaagg 70920
taaattttta gtctttttaa aaatgctcat acattataaa tccttgctaa tatacagttc 70980
tttcagactg tcctgccttg cttgaggaag cagatgtggg tcagggagaa agaaggaccg 71040
atgaactcaa atagcctttt cacttttatt ctctcttttc acttcaaaat gctgtgtcat 71100
cagtgaatcc cctcctcata atctcttctt tcctggagaa taccagccta gtttcacttc 71160
ccttgtaaaa tatccacaga tctttgtgcc ccaatcactt tggctgtatt gcactgggcc 71220
ttgctaatgg cacaatatcc ttttcataat aaggtatccc acatgacaca gtagactttt 71280
gtacctggcc agaatcacta cattcatcgc agataccatg tgctgtctat tggataccct 71340
cattttctct tttcaggaag ggctggtgtc agctttcctc ctgcctctta tgaaccagga 71400
aatttttctc cctgtatctt ggggctgctg ttttccagcc tctgccaagt ttccattttt 71460
gtgggacatc agttgaccgg catacccgta ccctggacgg ggacttgggt taactgaggg 71520
ttggcattct ccccaaagcc tgttctgcat atatttttga aaacctattg gttttctgcc 71580
taccttcttt cctccatgag tagacttcaa tgaacacatc actctctttc ttcggtaatt 71640
acctggtctc acctgctcat cataaaagag ctattcttat tgttagggct gacagattca 71700

ctttgcaggt gatttaatct gcatctgtag tgtccacaga acatattcta gaagttaaaa 71760
gtacataata taatgaaaac cctgatgcaa cttttgcttg aaatatgagt atagcaaaga 71820
catttgagag aagagcctga aaataggtta atagtagttt aattgtatct aataagagct 71880
accattgatt aagcctataa taatactagt aactccccta aatatgctct acacattact 71940
tcatttaatc tacataataa cactcagagg aatgtattac catcccagat ttacagagta 72000
aatgtattac catcccagat tcacagagaa aattaaggct aagaatgtta ggtaattggc 72060
cgagtagcct gcagctagta agtatcagag aacggatttg aacccaggcc ttggctggtt 72120
tccaagtcca caagcttagc tgctgctacc cagcttcact gatttttgtt ccgtgcactg 72180
cctctaggat gtttccgtat ttgccttctg gttaatattt actccctcct ccctagcaca 72240
ccacattaag ggtgtggcat gggcttttca cttcctgaat gtttgttact cagtttcaac 72300
cagctttact ctctttcaaa gtgacataaa agtgacagcc ctttaagctc acagaaaaac 72360
aggagctgat tccagtacgc gattgactac catgcaagat tttgtctttc cttttatatt 72420
gttgaagatt ccttgatggc aacatggccc acacattatc taacctccag acaatcccag 72480
aggatctgca ggaaaccacc aagcacccca gaggggtcac tggcaacaaa tggattaagg 72540
aaatggcctg atttatggga ttattggggg tcggggtgga gaattataga caattttgtt 72600
tctactgcag aatcagcatg atagagtgaa tatggacttt ggagtcaggc ccaggcctgt 72660
aggatgacct gggaagcagt ctaggcagct ttggcccact tcagcttgta agatagagat 72720
aatacatcat cctagaaata tcgcatggat tatcagattt tgtatataaa gtccccagca 72780
gacacctggc acacagtaag tgctcccaaa accaaagcaa cagttcttag aagcttagct 72840
tggctcaggc tgaagtctca aatgatctag ggttcactct ccagccattt ctaagccatt 72900
gagtgaaact ggacatgtta gttctggtgt tgacttcagt atcctaagtt gggaaataga 72960
actcatcatt ccactcctcc ttttaagtat tttgataaga ataaccgaga agtccatgta 73020
gcattttgaa aatataaaat actattagat tggttttgtt ttgttttcat aggaaagaga 73080
aaggaagaag gaagggagag gagaaaaaga gaaaagaatt caggactgat attttcaaat 73140
ttccaaagtg agcaagattg gtgtttggta ctctttgatc tctccttttc ttaaaaaaaa 73200
gggggatgag tgtgttagag tcctacccca tctagaatta gagagagagg gaaacagaga 73260
ggtaaaaata tccttcctat tatccctcaa attttgccac agagaaaaca actcactctg 73320
cttttgtatc aatgacctta ctgcctcagt gtcctcatct gtcgaaatgt gtttaggaat 73380
agttgccctg cacacctgtc caaggtgtta gaagagccag atagataaga caatggggaa 73440
acttattttg aaaatcaagt agtattaaaa tcaaggtgga gagaaggaac accaggccaa 73500
gagtgagcaa atctgcattt gaacctgggc ttggatgatc agcagctttg tgactttggg 73560
caagtcattt cacctctcca cactgcaggt tcctcatctt gcaatgtgga aagcagaacc 73620
tctccgggtt ggaggatcct gtgggatccc acgtgagtca gcaccttgaa attgcacata 73680
cagggcacat catttctacc atcacctaag gtcaatccca ggttattgct ggccttttgg 73740
tttgctatgt tttccacctt tctcacaaat taaaaatgca gacatgtaaa ttctctatct 73800
atgtgcactt caggaacttt caagatccct ttagggttat atctttcctt agcctcttgc 73860
aaacctgaaa gcaaggtgac tgtaggacaa actgtgtggg cactggatgg ggaatgcatg 73920
cctctggtag aaggggaagc cagaattcag agccaaagaa caggcaccat gcaggactat 73980
gagtccagga tgtccaaatc tgacttctaa aggaatgcca gtgatactga ttgtttttaa 74040
tataaaatct accatttaca aaatacaggc tcaagaacaa ttgtaaacac tgcacagggc 74100
aagttttatt gtttttctta aaagagctga aaaccataat caagtgcatc tgccctttgt 74160
tgtagattgg ggcctgcaat ctagggagta gagaaaggct gcctgactgg ttagcaattg 74220
aaacacagag ctggggccat ctagtccgaa tgcagtgttc atcgttcttt gctaaacaca 74280
aggtgaccgt ggttgttgtg gttagcaagg tcacctagct tcctaggcag accttattcc 74340
accacaatgg gcttcctttg ctagaaagga catggaaaaa ggagtgacaa aagaggagaa 74400
agagctagtg aaggaaagag aggaagagag aggaaggtcc ccagttgtta ttacctaggg 74460
agtgcccagc atgcctttca gaggaacaca ggtgcctacc cctttctcct gcctgctaca 74520
gcccaccacc accaataaca actgtgcacc ctcactgttg cccccatgca tggagcagag 74580
atagagcaca gatagggaga aaagaatttt caagggtgag ttggaatagt gacttctgca 74640
agcctggtag gtttgtctct tttccggtat ccttgccact gcaccaggtg aaggggaagc 74700
agactgtaaa atactttgaa tattatcaag gttatttggg gaaaaaaaac agcaacgacc 74760
tcacctgatt gtgccaggtt tgtgatagga atctgggggg aagttgtcct caatgacctc 74820
catttcttga atgacccgtg gcccacctgc tacctctgag atgagtcagg ggacacaact 74880
ttcttgattt ccatcatcat ttgcctgcta ctgctgcaga gactcaagtg gaaaatgtca 74940
cttcttgaag tcatggcttt ggtgtctggt gacagggagt agatagagtt tggacagagc 75000
cacttgtgag ctgcgtggtt taatctctct gaacttcagt gtctctagct ctaaaatggg 75060
ggtagcagcc tcaatgagca aattaaagca aaataacaaa tgtaaagcac ttatcccaag 75120
acaaggcaca tgagaagtat tcaacaaagg acagctctag ttatggtggt gatggtctct 75180
aaagcttcca gactcagatt cctaaccaga cagccatgag acatggtccc cacagtggca 75240
aaagggtagg ctccaggctg cctaagttta aatatcctga ctctaccact tcagcaaggt 75300
actaaactct gtgcttcttg cctcatctgt aagatggaga tacctcattg ggctgttggg 75360
aagattgaca ggggtcaccc ttataatgtg ctcagcacaa tgcttggacc aggatggatg 75420
ctgcttgggt gatgttatca tccaggtggg cactgtggaa cacagactga tacttaagtt 75480

cagcacttgc ctcatggagc ttacagtcca gatagaatag gtgcacatga gatttttctc 75540
agcaaagttg tagtcagtgc tgcccgtgcc acccatctgg tctcagcatt gctgctcatg 75600
tgcactgtcg tgacccatct ttctgctgct gaccttaaga aaccttgcct taaggtatgc 75660
tttggggaaa ccaaattatg acagatataa actgaagtgc caagacgaat ctttcaaagt 75720
gctgtgatgt ccagatacgt ggcaagtgcc aggaatttgt gtttgggccc actgggagag 75780
aggtcctttt ccccggcttc ccgcagtgga aagctgctgt atgtctgata cctgtgctcc 75840
aaatctaccc tcgagttctt atttggcaac aggcttgctt agcaatgtcc ctggggtcag 75900
ggagccacgg gtgggctgat gcaagaaact gcagttgtgt ctctaggctg cgacagcatc 75960
acagctgtga gcttctcaat ttaaactcaa catctgggca caagaggaag caccctccat 76020
ccaggcaagt tgaagttttc tttcctgtat ctttaacaga ggttaaaatg taaacacaaa 76080
acaaagcaaa acccaactta actctctggg tcctgtttct gttaaaaaca ttttttaaaa 76140
aataataata attagaacaa tagacccagc tgagagaagt ctgtctcata ataacaagag 76200
agactaaaac acgctctccc acaaacagat aaggaaatag tgagctaaaa agtaaaactt 76260
aagagtgcta cttctcaact cagtaattga ttcatgaata agctctagcc caattttcta 76320
tgaaaaataa caacatagag aaaagaaaag ccactcagat gcagctatgt cacccaagtg 76380
ttcactggag aaatttaata tgtgtcagag aaaaatctgc cttctccaaa acattgcccc 76440
ctccaaaaaa aatgcttctc gttgtgggat tttttcctcc acactgacag agagtttgaa 76500
agtaaagcag ccgactagag tgtgggtgcc cacgccttag aacaaagaga ttaaagaata 76560
ggcttttctt caaataaatt cgattttttt gcaacaaatt cagggcctgg acagtttaat 76620
gaagaataaa aaatgctcag acccctctct ttcagctttt ccaaacaaac acagtcattt 76680
ccaaccactc acaggtgttt ttcttcccaa aacaaacaca gaaacaaata tttacagagt 76740
tatgaaaatt caatagaagt cacaggggca caaatttcct cacctgcatc caagatctct 76800
ggcctgggat tatcaaacag aacgtcacac ctactttcca ttacgggaca ggaaatcttt 76860
gagccaaaga gaatcagtct gcagaatgtg ttcgtaagga taaaggcctc atcttttcaa 76920
gaaagtgttt gcagctccaa ggaagaatgg actttggaca aaggaaacac cagtgacttt 76980
ttcctcatct gcccatacgt gcagaaatgc ccttgaagag ctcatgactg ccttgcgaat 77040
taatccctat gttgaccaac aacacaaaga gtggtgctga ctaccacact gaccaccagg 77100
aataatttcc tacttaggtg tttgcgggaa aggctgttgt ttgcttttct caaatgtccc 77160
ggatgtgaac aggtctcaaa attaggaaat gcagctggga atggctgcta tcaacaaccc 77220
ttatttcatc tttttctttt cttcaataga gctagagaag caggcaagaa aagaaaataa 77280
tgcaattatg aaaggaggga ggaaagacag agggaaggca gcggggcaga agggaagcgg 77340
aaaaaagatg ggagagaaag gaagaaaggg agggcagaat ggggacaaaa gatggcaggg 77400
agggagcgag ggaggaagga gcagaaagga gagggaagaa gaaaggaagg aaggagagag 77460
gcagagagag agacaaaaag aaagaatgag agagaaacct gctgttgtca gaacctgact 77520
ggtttacgct gaggaaccag atagcctctc agtgaaaagc tgagattaag ctcacaacat 77580
aatttccatt atgttaatgt tgctttaaaa tatattgcac agagtgctgc aacaatcaaa 77640
ttatcaatta taatgtctac tcagagtaga aaagtgttgc aaacagaggg ctgattactg 77700
cttcagtaca tccaatctgc atactcattg ttcagtacag ggaaaacaaa ttatcttgtg 77760
ttggccccag ttcaaattta attaaatgag tgcaaagcaa ttataataaa gtacaatagt 77820
gtagtaatga aaggaaaata actcattaac ctattaatct ttatttatgt tcctctcaaa 77880
taactatgta atttctttat caccgcatcc cataagaaaa agtagggtct cgctgctcat 77940
ggcctggttt aacaggttct atttggttaa gatgcctttg tgggcaccat ttatactcac 78000
tttgcttagc tttggggcca cgcacccccc agctgtctgt catgctccct gcccacacgt 78060
atgcgtacct gatctagatt tagcgctcct ctcccacttt tccctgacac ttgtcagtta 78120
cagaaggtaa atctctctgg aagtgagaag tgcttaccac agcatatggg aaagaggcat 78180
cttccagcat ctggtatgta aggcaatgca aaaaagggga gaggacagaa tcacttctgg 78240
tttagagaga gcccagtgct tgacagctga agagattcca ctaaaagcta acactccact 78300
gtgctttcca gttaataaag ctttttacat gtatgatttc actgaggcct cctgataaca 78360
ctgtgagggt gcctgatgta cagatgagga aacagaatga tggagtagaa aagtatggtg 78420
tccaagcctt gctactcatg agtagcagat ccaggattct aatttaggtc tacagcccct 78480
aaatcttcat ttcattcccc tagaaccttg ctactcaaag tgtggtccag ggaccaggag 78540
cattggcgcc acctggaagc tcattggaat cttgggcctc accctagacc tgcagaataa 78600
gactccacat tttataagat cctctagtga ttctcacaca ctgaagttta agaagcactg 78660
gaactattgg atagtacact gccttttgag agaatggtga ctcaggcaaa tctcccagca 78720
actacttggt taagcatttc cttgcaattt gcaacacctt gagatgacct tgagatgatc 78780
ttgggtgatc aaggttgatt ctggcttcca ttttcatgtt atctgaagac agggctggaa 78840
gtagaggaca tagtaaaagc agaaatgatt tctctttctg gatccacagc agacccgaaa 78900
atcttcccct ttttcttcca aagagaattt gtgaaataga gagaaaggag aggggagaaa 78960
ctgatgaatt aaggaaggaa acagacactc gttaagtttc taccctgttc ttggaggttt 79020
tccatgtatt ccttcattta atcttcacaa caagcacctt tttaagataa ggtgactgac 79080
gacaggttta agtgatatgc ccaaggtcac ccacactgct gggtaacaga aatggtattt 79140
ggcttggatt caaagtttac actttcactg tactctattt tctcttaaaa aaaaaaaagg 79200
aaaagggaag gaaatcaaga tgaaataaaa gggtggtgtg gtagtcatta gtgctgttca 79260

ccaagaatct ccagttttta gtctctgagg tatgtgaagg attatacttc ctgccctgct 79320
tagaagtagg tgggagccat gtaaataggt ttggccaatg aattggcagc agcggtttca 79380
tgtttcattt tcagatcaaa tatttaattg cttgtggaca ccttacagag ctatctctct 79440
ttcctctgcc atgaaaattg ccaatattca agatggtggt tgcatcataa acctgtatcc 79500
atagtgagtg aatatgctca ccagagaccc ccaccaacct atgatagaca cataatgaga 79560
gtgataaaag caataaacct ttgtgttatt ttgagcccct gaagtgtgat ggttgcttgc 79620
tactgtagca tcactgagcc catccccact cttccagatg ctaggaagac gtgtgaaggg 79680
aaacagttaa catttctaca ctgcaacttt ttcacttgag actcacagca ttcctatgag 79740
aaaggtaagg aatggctatt atttattatg tatttataaa ttactattta cagataattt 79800
aacactgaca tagcagttta agtgtcctgc ccaagatcac aaagtttata agtggaaggg 79860
ctaggtttgg agcacaaggc ttgtaatgac tgatctttct tcaatgcaga acacttcctc 79920
caaaaaagga ctggagcacc attacctgcc cttgggtata ctcagattgg tatatctagc 79980
tttgtactca cactcctctt cccccaccac tcaggaggaa tccaagttat tgggcaaaaa 80040
tcaggccaag gaaaaacaag tacatgcagt tgccagcaga tgacgacatt gggtctttgc 80100
tagagaaacc accagagaaa gttcacagtg ataaccctca atgaagtcaa ctgtaaggag 80160
cttcattgtc cagtttgagt gttttttgtt ttttgtttga gaaagggtct tactgcatcg 80220
cccaggctgg agtgcagtgg cacaatcact gctcattgca gcctcaacat cctgggctca 80280
agcgatcctc ctacctcagc ctcccaagta gttagaacca caggtgcgtg ctaccccacc 80340
cagcaaattt aatttttttt tttttttttt tagagacagg gtctccctat gttgctcaat 80400
ctcgtctcta actcctggga tcaagcgatc ttcctacgtt ggcctcccaa agtgctggga 80460
ttataggtgt gagccaccat gcctggcctg tgttcatctt tcatgtgatt gtttttgcaa 80520
gatagaaagg caaaagttag tagtagcagg attgaccttt gtaatatgat cttaaaaata 80580
ttgtctccta atttgcatat ggaagaatct caactaaata acctcctttt aaccagttca 80640
ctaaacaaac caaagttctc cctttttcct ataaaacatg cttctaatgc aggttagagg 80700
ctcttggcaa gtaggctact gtccagcatg tccaaggtcc tgctctccca tctttgagtg 80760
ctatgtttac taagagtcta ttaggttcat tcccaggctt gtttatgctc actgtatttc 80820
ttattgtcat tatgtacctt aatgtaatgt gagtcagaaa aaaacatatt tctgtgaaga 80880
ctggatagaa cgctttggaa agatttgata aaagcaagtt actaaaaaga gtgttggcag 80940
acctgtaaca aaaaattgac aaaagttatg gtctatgtgg aacctgccct cagaatgttc 81000
ttttagtttc ccaaggcttc acattctgtg ttaaagaaac aaactgaaat tcatggacaa 81060
tgcattgcga gtgtgattta tacaagaaag gcaatctaga cgtcttagca atggaatgta 81120
accatgtttt ttaaaagcct aagtcatata tcaaatgatt gtcaaaacga taacccaaaa 81180
caatgtatag gttttgtgtt aaaataaaat gaaatattta aagtatggat cgtttttaat 81240
catttctgaa ttcaagccct acctatttgt cccaattgct tctgatataa ggactttcac 81300
ataattactt ttcctgtttg ccattttccc atttcccctt ccacagacat actgtgctac 81360
ctttaagtgc gaggtctttc acatatcttt ttttatccaa ttacctcatt caacaatatt 81420
tattttaaaa aattttttaa agagatggga tctgcctatg ttgcccaggt tggtctcaaa 81480
ctcctgggtt caagcagtcc tcccaccttg gcctcccaaa gtgctgggtt acaggcatga 81540
gccaacacat ctggccctct acaaatattt cctggacaat tactatgtgc caagcactgg 81600
cctgggtcct gggcatagac caacgaactg tagagccaac agtagagaaa acagacttta 81660
aataatgaga gaatcatcat ttaataataa ctgtggtgtg cgttatggag aaattcagag 81720
ttcactctgt ttttatataa ctcagacctg ggatggattt cctacttctg aatggtaaag 81780
taagtgtctg agtagatgag ccaccagcct tcttccttgt attctatttt taatgcagtt 81840
tctttgggcc aaatgatcac cgtcatcctt accagctcca gactgtgatt ccatagtggc 81900
tggattgcct tggagcttag agcaggttat ttcagtttac tccatggata ttctctccac 81960
tcttctccac ttcatgatgg ttaatattga gtgtcaactt gattggattg aaggatgcaa 82020
attattgttc ctgggagtgt ctatgaaggt gttgccaaag gagattaaca tttgagtcag 82080
tggactgggg aaggcagact caccctcaat ctgggtgggc acaatttaat cagctgccag 82140
cgtaaccaca ataaaagcag gcaaagaacg tgaaaagact agactcgttt agtcttctgg 82200
cctacatctt tctcctgtgc tggatgcttc ctgccctcaa acatcagact ccaagttctt 82260
caattctggc ttccctgatc ctcagcttgc agacggccta ttgtgggacc tcatcctgtg 82320
accgtgagtc aacgctcgtt aataaactcc cttttatata tacatctatc ctattaattc 82380
catccctcta gagaacccta agatatctct attctgttcc ccaagaggtg gacctttatg 82440
gacccaatca actgcctccc tcaccctgtg cctccaggtg gcttggaaag caggaggcga 82500
tgaaggctgt gggttttgtt ctctatctat ctatctgcag aattgtcaag tgttggcagc 82560
ctctctcttg gcaaagccac cacccttgtc aagcagcact ctctttatag ctactctcta 82620
ggtcagggat tgtcaaacaa ggcccagact aaggggtggc aagcaaagtg ccttggaagc 82680
caaggttacc ctcggagtta tgcaaatgca gggcctgaga gtccattctt tcttaaattt 82740
tgcaccctgg acacctcact tgccttaccc ttgtcccggc cttgatgaaa acttcctttg 82800
taaaggacta gagaataaat attttaggct ttgtaggaca cacagtttct gatgcaagta 82860
ttcaattctg cttttgaaat gtgagggcag tcatagacaa tatgtaaaca aatgggcaca 82920
gctgtgtttc aataaaactt tattttcaga aacaggtaca gggccaggtt tgatccatgg 82980
gccatggttt gccaacacct gctctaagtg atacagttcc ccatggttgc ttactctggg 83040

acagcacggt cccttgtggt ttcctttaat cctgctcaca tctttgcaaa aagccccttt 83100
actaagctcc cctcgaatta tccaaattga gtgtgccatc tgtttcttgc taggacccta 83160
attgatgcac ttggctttgc atttctccaa cacaggcttt aattgcaaag taacaacaac 83220
aaaaaaaaca agaaaatgga gactacaacc aattactctg tagcagggaa gtggtggtgt 83280
ttacatgtat tcatccattc ccaaatatga tagaatattt cacaaagttg cagtacactc 83340
ttaaagttga tgggagctga aataaataag cctttttttt tcccctgggg cctgcactct 83400
caaccctgct gccctacttg tcctcttcta atggaatgga ggtctattct aattaaacaa 83460
gaaaacacat cttttcacaa agggagaacg gaggacattg tactctgttc ctcaaagaaa 83520
gcatgcttta ataagaatcc ttttataatc cctgctttta tttgtagctt cccaaagctt 83580
ttgctggtaa taaaaggatt gtaaagttga caaatataag cagggacaaa gaagtttggg 83640
gagataatgc tctagtgaat gggctagtat ttgaaagttt attgatcctg caacacctag 83700
aagatgatca cgaagagcca atgctgccat caatcagggt gatatttcta ttacaaagta 83760
aaagcccttc caggcagggc tatccatttc tcatatcatg taagaattat gctttgtgct 83820
gagaaaaata attcaggcca aaatgcaggg cttggcatct gaagatctgg attcaaatct 83880
ggcttcatag aacttctaac ctacttcttt atcatatgga agcagcaaat agagctgtaa 83940
caaatgcaat gtgttctgtt gtggggttgc agacatctgc tgtcattcca ccactatgac 84000
tatcacaagt agcatccatt actcctggaa aaagccaccc attttcactg aggttccctc 84060
actctcacac ttaaagcctc acactttcca cttctggtgg agctccatct ctgtggcaag 84120
ggtggaacat gtgaccaagc ctagaccaat taatatactg tataccttgg gcagagagat 84180
ttgtttaatt ataggcaaat gatccagtta cagccaatga gactttctag gaaagtgatt 84240
agacaggctg tcctttctca ctaggcttga atgtgagggc tggaactgct gcagccattg 84300
tggatatatg gggagcctga ggctgaagtc aacagagtag aaggatgaca ttgttcaagc 84360
cccacataga gccatgccta aagctggtat tggttgggag aaaatataga caagttttca 84420
caatttcctt tatctaaacc ctttgtaatg tgactttgca gattctccca ttgagaacat 84480
taagagatgg aaactacttc ctactccttg aatatggact gctcttgtag cttgctttga 84540
cccatgcatt ccagtggaag tgatggtgtg cacttttgct gtcagaatcc tgcaccatgc 84600
agaaaagcct aggtgaacct gctggaggat gagagacaat gtggatgaga gccaaggcat 84660
cccagccaac agccagaagt agtgttgcct agctgaccca cagctaactg cagatcaata 84720
agggagccca gttcagacca gaagaaacat ccaactgagc ccagcttaaa ttgctgactt 84780
gcagattttc tgcgaaataa acagttgttt taagctactg tatgttgagg tgatttatta 84840
ttcatcaata actgatacac atcccatccc tgaatttttc atttatatga accgataatt 84900
ttatttttga tgtagtcagt taggaatggg ttttctgtga cttgaaataa aacaattctt 84960
aacacattaa agatgtgtct ttatttcccc cactagatag catcattagg gattctgtct 85020
tatccttatc caaagcactt gccctatctg gatccaaagc tgagtgactg atcaataact 85080
acttagtgat tgactatgtc atattgacag attcacaaat cacatttgaa agagcaaaga 85140
atatatttgc tcttgagtct ccaatgtcat ctgtagggca attgtacttt atttggctta 85200
gagcctcaac agaaatttgg aagatacaag gtgcttctcc aaccatcaca atagctgaaa 85260
acaaatacat ctgagacatt gtatctaagg aacccaactc tgccatctcc ctcaaggaaa 85320
cccagaaaaa tctgggcttt aatcctaccc tccttgctat aaaaacctga aatgatgtca 85380
tctttttctt tctaaaataa aataaaggca tgtagtgctt ctaggcaaga ggtctagatg 85440
tatctgcctt tcctgtcctc caggacacca tccacttatt gctacaactt gctgcattat 85500
tcctgcaatt tattttcttc tttttgcaca aaaaaaaaag gaagaaaaaa ctaaagcagg 85560
gattccatag aattcatcct ctactttagt aaaaaggtag aatccacctg acctattctg 85620
tatgtcagag tggaatccag cagccatttc tctccaatta ttgaaaaatt gtgtgtgtgt 85680
gtgtgtgcgt gtgtgcgtgt gtgtgtgtgt gtagagagag agagaaaaaa aaggtagata 85740
tacaccagca tttttattct taaataagag gataatcagg cttactccca cccaagtgag 85800
tttacacttt aaattcttaa atggctggtt tttttttccc tcagatctgc agacgaaggt 85860
ccaaaggaag aagattttgt tgaagactga aaagagaaat atacttggtg gacagagata 85920
actcctttca gcacagaggt caggtaaggg aatcacagac tgacatttac ccttctctct 85980
gaactctccc aggcctcaac agctcctaat ctttcctttc ttcattctcc ctcctcctca 86040
ttcacatctc tttccccaag aagactcaag aaaaagtcca agataagaaa ttccatttat 86100
aagaaaagga aggcacaggt taatttggtt tatctcctcc aacctcaaca atggatgtga 86160
gttctgacaa ttgcttgtgg ccagaaccaa tcaaactttc cttcctcatt agatgacaag 86220
gctgcagcat gaagaagatg ccatattgtg gaattgggtg tcaaggagaa gacattcgaa 86280
ggtgcatgaa cagagtgaag gtggtgcaag caagtgcaga ggaggtgggg agactggagt 86340
gctgggaaaa gggtcagcag aagctagcaa acatgaaaga cacaacacaa aacaagcagt 86400
tgtgcaaatt aggatgataa ggggggagtg gtcaaaggtg tgtttctcat ggctcaacca 86460
gtgcaaactc agtcttatga ccctacatga ctgactgcac ctggcctctg catcccttca 86520
gcctcttccc caaaaggact cctccttatt gccagccaca caagccttcc ttcatttcct 86580
ctaacaggcc agacttgttc ctggctcccg gtcttggcat tcactgtttt ttctgccata 86640
gctggctcct tcccaaacat ggaatctcag cccaaacaat atctcctctg agacaccagc 86700
cttgatcacc tttatacaat acagatctgc tcctgcccat cactttctat ctcatgaccc 86760
tacaatgtgc cccttcgtga tagctcacac cagctaaaac catcttgttt gtggattgct 86820

cacttgcccc ctgcctgtct ctagctctag gaggtcggct ccctgtcact gtctggtcta 86880
ctgtgtcgta ccacccatgc ccagcaccta gccttgcaca ttataggact taatgcaaac 86940
ttcttgactg aaagaaaaat gaagaaactc agccttggga gagaaaacaa ccattaaatg 87000
attatttaat aaaatagagt attaatctat tttatattaa ttattaattt agttcataaa 87060
atattaattg tctttacaac gaatgtgtcc ttagtcattg gtgtttgtgc tttgtgaatc 87120
ttcacatccc atggggccag taatgggggt gggggtgtag tgtgggaagt ggggcaggga 87180
accaatctgg cctgcagggg ttacctgccc catgggtgcc tgcaggcttc atgtggtttc 87240
agtggcatgg caccctgatg ggaagagtgg ctcagaattg gtggagtatg gtgataataa 87300
aaagcaaaac aacttttagt tgattattgt tttttaattc tataccgact atggcccctt 87360
cactacctgc accctgtgag gagagttttc ccacatctgt cccactccca ccctcacact 87420
ccactggttt tttccccctt cttgagtgaa cacacgcact ggaagagcag gtgtccttga 87480
agggaggacc agctccatga caggacacat gtcctcccaa acacaaacat acgcaaacac 87540
agaacaaatt agaatctatc ctggctcctg agaacacttg ctctcaggcc actaaagcac 87600
cttgataata aattgtcctg tgcctgctac ctctgtctcc acctgctcct ggcagaaatc 87660
tcttccctca ttccagccac tctctggagg gcaggcaggg ttctgtttat ccatgtccac 87720
aggccatgcc aaccaatggc tcatggcttc cccacttcca ggaaagccac atggcccagg 87780
atacaataga gtagggaatg agtaggagga gaactggtct ctttatccca gcaatatgga 87840
ccttgtgatg gaaagagacc taaagacagt cacatttact tctcatctct acccgggccc 87900
tttcacacct aacagagtat ggcaattggt acatggatag cactgagctg agtgacccat 87960
cattacttcc tgccatcgga cacttatgtt ccatcccagt agggttccct agtcacaatg 88020
aagatccagt ctctatgcta cctccatctg atttcttcct tgatctctac tccttttatc 88080
ccactaacaa atttgtattt attacttttc atttattcaa aagtatacat ttgtttgtgc 88140
acaaaaaaat acaatttctt tttttgtatg gttcagattt aaatcccaca tacatgatat 88200
atgcaagcaa gttgctttgc tggggagatt tgtgaaattt cctcacactc atgttgtaag 88260
ctactttgtg gtcctcatct cttttcacat ggacagcact cttacagaaa ttaagaactt 88320
tatttttttg tagaaaacca gatgtaagcc cattgaaagg agaaaccttg gctatctttg 88380
tcattataat aaatctagta tgaaattgtt ggttagaaaa tattaaatga aaggaagaca 88440
gagaaggagg gaagaggaag gtaagaaggg aggagggagc tagagaaaag ggggcgggag 88500
ggagaaaaga aattaagttg tcctgaaact caactgctag gacagttact gtgagtaaag 88560
aggaaataac agtttataga aaaatatgtt tcttattttg aaaaaaaaaa gtttggaata 88620
aaacaaaaaa tacaaagacc cctatttttt agaattatga ggagttggag gagtcactta 88680
aatgtttgtt aaacatcact acttctacct ataaagggga aaatacttat aaagggaaaa 88740
ttttaactag agtgatattt gggacttaaa atgtctggta ccaattttat ccatttctca 88800
tcccagagca ggaagtttgc attgtttatg actgaatatt caagtatctt tcccccacta 88860
ggatttactg taggttcgtg gtagtgacag gggatttaaa tgaaaatttt taggtctcgg 88920
tcagtaaaac gacctgtgtc agcctagaga aagaggaaga gaggagacag ggttgaccca 88980
gcgctggacg agtctgaaga tgtgcatggg gagaaaataa ttgagctcat gtgacccaaa 89040
gtcaagtgta ctctgggttt aggtctcagc tgcagtattt atcggactgt gaggatcatt 89100
tcagctccct gagcctcagt ttactactca tctacagaat ggaattaata acattaaatg 89160
agatgatgca tcactagaca tttagctcag atcatggcat gtattacata tgccacaact 89220
gttagctagt gttatgataa tctttgttat tattatggtc aagatgccta ctgcaagact 89280
aaatatatat caaatgtttt ctgttgagca tacaacttga gcatattttc caacttacct 89340
tacagttcca tgtacccacg tggctggatt ctgaacaatg accacttcca gaccggactt 89400
acaaaatctc tctccacact ctctcatttc cctcatcttc cgagaagatc caatggagga 89460
cttcaaagtc ctagaagatg gcaaagcgac aagacagaaa gaatgtcatc catgaatgac 89520
cacgtggagc agagctccca ctccctgccc tgctgtccca cattgcacaa gaatgtgaca 89580
tagaaacaca atttcatttc tcttatgtca cggaaatttg aggactcttc cttatagttg 89640
ctagtattaa ttaccctagc taatgccaag gaagaccaaa aagtctttcc aatgctttgt 89700
aaggactaac cagaagacac acataaacac agctttgctc aaattcattt ttgtcacaaa 89760
tcacttcttc agggcctcag aatgtgaatt ccagctgctc tgtcaaccaa tgtctagaaa 89820
catttttgct gttgagtgca aagtcttcat tcatgagggt gctctccaaa cgcattcatt 89880
ccatggtgtt catttcttgg tatagaagca tatgaggaac agcacactcc ctggatctat 89940
atactacatt acctgtatta ataagaatgc taggctgtac ccattcatta tctaccagcc 90000
cgccagacaa aatgattcac cctagactta caggttgaag tcaattgagt atcctttgaa 90060
ctggagggag aggcgcttcc ataaagtggt gagagcttta tggcagaaag ttctattaat 90120
gaccccactt cagtgcttat caggagcagg ccatataatc ccttcccgga attacatgct 90180
ggtcggttca gataaataac ccagatctct gtcatttata atccaaaacg ttaacaatta 90240
atttccaaaa tcaatcctga agggggctag aagcctaaat cgctaggtcc aggcagaccc 90300
cactccatga atttctcatg agtcaaaatt taagcctcga aacttgtaac taactgttgt 90360
caagacacag ctgacttagg caatcaagac aacagtattc agcagtaacc tgggtgaaac 90420
atactctaag tgtgaattaa taagtcggct gacaccaggc cccagatgtt tcccactctg 90480
gcaatatgcc tgaagtgatg ataagcagat ttacatactt aactaatatg tttatttaaa 90540
agaaatttcc aaatccaaaa taacatccag atttcttatt tggctgcaga tcaaaagaca 90600

tcctatcaat aaagacagaa tccaggataa accttttcaa catctgctta aaaccaaaca 90660
acagatttga gagttttggt aaaacttaat tgcagacaaa tttcactgcc tcttacataa 90720
atatatattt tcatgtccct ggggtggttc tgacctcatc aattttcgtg ttcacaatac 90780
acaacattgt tcttttacca gcaccaacaa ttctgccttt atggagaatt cccatcccaa 90840
ggttttcttt cagactgaag gataaacaga ctttctctct ggccaggaaa agtcgaacaa 90900
aagtcagggg catgtgctaa cacttggaat gttgtagaaa agaaaaatcg acccctctac 90960
cagtaagatc tttgtttttc tataaatatt agaaagcatt tatttggtct ccatctccga 91020
taaatctgga aggagaatag aaaagtagag atgctccttg agtattacat tttttttttt 91080
tcttttcggt ggtgttgatt ccccaaggga gagcttagta actggatcaa agtcttaaca 91140
gatctgtgtg cttgtgaagg agatgataat aggaggggtg ttggccatag tagcacttat 91200
ccctttagtg tgaattagtt cactttcttc aggtcttagc tgtctgaaat tggaccatga 91260
tccttgggca ctgagacatt tttctcccat ctggccagtg ctgcaattga gctaaggatt 91320
tggttcagag actcttgttg cctggggatc tgtttaatgt caagaccgat attagctggt 91380
tgaccctggg gtcgggtgac ttagagagtc agaagtcatc catgtttgct gctgctccac 91440
cctttgaact tagtcatgat aaactcctgg gtccaatcct gttacagcca aaatagcaag 91500
aaaagagtgg tagaagcaat cagaaaaatg catagtaagg caccaatgag ataccatctt 91560
cctcttctca attggcatac actttaaaat agcggtgttc agtcttttgg cttccctggg 91620
ccacactgga agaagaattg tcttgggcca cacataaaat acactaacac caatgataac 91680
tgatgggcaa aaaaaaaaaa aaagaatcac aaaaaatttc tcataatgtt ttaagaaagt 91740
ttacaaattt gtgttgggcc gcattcaaag ccatcctggg ccacatgcag gacaagcttg 91800
ctttaaaata tcccatttga agacagttca gatctcccca gttatagatg atttatcaaa 91860
taactatgta tgtatgaaat taatatatca aaagccttaa aacatttcca acctttgacc 91920
aaaaaattgt tcttctagga tgtatcttaa gacataatta tgatttgtgc acaaatttag 91980
ctatgaaaat gtagttttac ttatgatatg acaaaaatat cacaaatgac ctaggtgtca 92040
aacaatagag accagttaat taaatgatgt tccacaactt atataattat taaaaacatt 92100
taggatattt acatttaata aaacccatag tgagggcatt tagattgttt ctaatttgga 92160
cttttatgaa taaagctact atgaaattat tgtatagttt ttctttttat aaatagatgc 92220
tttcatctgt tttgggtaac cctccatgag taaaatagct ggattatatg ggaaataaat 92280
gtttaacttt ataaagaact accaaacttg tttccaaagt gattatatca ttttacaaca 92340
ctgcaacaat gtatgaaaat tctagttgct ccacctcctt ctcaatactt ggtttcccaa 92400
tcattttaat tttagccatt ctggcaggtg tgtagtgata tgtcattatc gttttaattt 92460
acattcccct aataattaag aatgtgagca tcttttcatg tgcttattct ccacttgggt 92520
attttatttt gtgagttgac tattcaaatc ttcttttcat tttttattag gtcatttgtc 92580
ttattgagtt gtaggagttc ttcatatatt ctggatataa gccctttgtc agatgttttg 92640
caaatattat ctcccaacct gtggtttcca ttttcatttt gtttatagtt tctttctttc 92700
tttctttctt tctttctttc tttctttctt tctttctttc tttctttctc tttctttctt 92760
ttctttcttc ctttatttct ttttcttttc tttttttttt ttttggtgag acagtctcac 92820
tccatcaccc agactgcagt gcagtggcac catctcacct cactgtaacc tccacctgct 92880
gggttcaagt gattctcatg tagcagcccg gattacaggc acttgccacc acactcagct 92940
aatttttgta tttttagtag agacagggtt ttgccatgct ggccagcctg gtctctaact 93000
cttgacctca agagatctgc ccacctcggc ctctcaaagt gctgggatta caggcgtgag 93060
ctaccgtgac tggcttcatc atagtatctt tctaagtaca gaaattttga acttaagttg 93120
cttttgagaa aaagaaatgt tgtggtgctt agtgaaaaga ttcaagagca cctgcctttc 93180
aacccctgct cagcctctgt ttttttactt ggctgccaac tctcttgctg tgtggacttt 93240
tcctcatcaa tccaaccatc tggtgcctac tggtggaagc tgtgagtggg taaaggccaa 93300
tgaggtgtca gggaacaaca gtagaaacag aagccaaacc taacctgccc tggagcttcc 93360
catgtgctct agaaagggaa gtgccttgtc tgctgaggca ccttctagta ctttccagct 93420
ttaaacgaag catggatctt ttccttcaca attaacaaaa gcccttgttc aggtatgttt 93480
ggcacgtgct gtcatgcttc tttgtcacaa gcaaaacaag aaactaggga tccttcaaaa 93540
ttctattaga aaatagacct ttcaggaaat gttgcagaat tggctttctg ggatatggga 93600
ccaaatattc cttcattccc tgagaattga cttctttctg ctgtatgctc caaatgaact 93660
ccatatatac atacctcatc atgcctctaa atttttaggg cacacatttc tttacatgtc 93720
tacctcctgc aattacactg gaagcccctt gaaagcatga ccatgttttg tacatctttg 93780
tatgtatagt gcctgtctta gtccatttct attgctgtaa gaaaatacct taaatctggt 93840
aaattataaa tcatagagat ttatttctca caatcctgac gactgggaag tccaacatca 93900
gggcaccaga agatttgatg tctgcagagg gctgtctctg ctttcaagat ggtgccttct 93960
tggtgcatcc tcacatagca aaatgacaga gagtaaaaag gactaaacag acttctttaa 94020
gatgttttac aagggcacta attccattca taagggcagg gcctcataac ctaatcacct 94080
cctaatggcc ccacctctta ataccattgc attggtgatt aagttttaca aattttagaa 94140
agacacaaac attcaaacca tagcagtgcc taccacagaa ctgatacaga atgagaactc 94200
aatggatact acttgaatgg ctgaatgaat gaagtgaatt aattgttcgt atccccaaat 94260
ctgagagata tctctctgtt taataatcta ttttgttaaa actttgctct ggagttggac 94320
agacctggga tcacatctca gctccagttc ttgctagctg tgtaactgag acaaagttct 94380

cagcttcctt tgagtcccat ggcttattaa aggggaacaa tggttataat tataataact 94440
acctcatgag tgtgtatgga ctcaatgagt taacacaaag tgtgtaagcc agtgcctggt 94500
tcttagtaag tgctctgtaa atgttagctt tggttattta catgctgtgt tttatgtgac 94560
ccatttctta tgaaataatg tttgctacaa gtagaagatg atggtgatgg ccaaatgtgc 94620
attatatatt ttagtgttca taacaaccag ttattattat ccttttttca gttgtaattg 94680
aagtgacatg cgtgaggtca caaagccagt gataaagctg ggattcagac tcaggctgac 94740
catactttaa tctctaaaca tagtcactct gtcccttcac acaggaaagc attctctgat 94800
ttggaggaga gaattcctgt agccaaggat ggtggcataa gccatagaca tggggctgtc 94860
aaattgctta gcctagaaga acaagagtca gccaactttg gggactccct gttctatccc 94920
cctccctctt atggcaatag gattaagttc tgtgtattag tccattttca tgaggctgat 94980
aaagacatac caaagactgg gtaatttact taaaaaaaaa aaaaaagagg tttaatggac 95040
tcagagttcc acatggctgg ggaggcctca caatcatggc agaagacaaa agtcacatct 95100
tacatggtgg cagacaagag agaatgagag ccaagtgaaa gggggatctc tctttataaa 95160
accatcagat ctcgtgagac ttactcacta ccatgagaac agtatgagga aactgtcccc 95220
catgattcat ttctctccca ccaggtccct cccacaacac ttaggaatta tgggatctac 95280
aattcaagat gagatttggg tggggacaca gccaaatcat atcaagtata ttgcagaaaa 95340
catgaataac agtggctaaa acaagataca attttttttc tcttacataa gaaaagagca 95400
gcctaaagct tatttagcct aaagctggta tggtgggttc cagtccacag gaccagggtt 95460
ctactttttt gctccttgaa aataagcatc caggagaaat gctgttcttc ctcacggggc 95520
ctgagattct cctctcctac caagtgtcac ctttctgctg gtctgcagaa actcctctgt 95580
ctcttcaggc agcagcacca gatacaccaa gcagaccaaa acaacatcac aaaggctctg 95640
aaaattaaac tgtctttgta accacagtcc acgaaactat gccaagacct ccgtgctaaa 95700
cctaaacagg ctgactgcct gctaaaataa aagattaaaa caggatccag agtctcctaa 95760
tataatagcc aaaatgttca gtatacagta aaaagccatg acactgagaa ccaagcaaaa 95820
cacaagtgaa ctaaaaaaga ccatcaactg acagccatgc tcagatgaat cagatgctgg 95880
aattgcctaa caaggatttt aaaggagaca gaaatgcttc aacaatcaat tacacattct 95940
cttgaaacaa ataaaaaaaa tataaaatct gggcaaaaaa tagttttttt atttttttaa 96000
aaaaaggaaa ccacagaact ctaaaatatg cagaaactct aaaataaaac agaaatagaa 96060
agggcatgtc tcctagccag atcagccatt caaagaagtg cagaagaact attcaatgat 96120
tttcacctaa tttccttggc tactttccag ctgcaaggaa tgctgggaaa tgtagtcttt 96180
aagcaggcac accaatgata gactggattt ttgtttacca ggtaaaagga aagaatatgt 96240
atcgggttac taagctaccc aggtctgccc ctgacacaaa gggctcatac attgggaaca 96300
aaatcaaggc tctgggagtt tacattggaa tatgatctta ctgtggataa gaccaaagaa 96360
atcctgtgca cacggatgag gattataaat cctggttcag gctggcatga tccacagttg 96420
ccacgattcc tgtatgcctg cagaaagaca acttaatgtg gcctaatgtt actaacatct 96480
cgacaggctc accttttgag ggaaggccat ttaattacct gattaatttg ctcatcttaa 96540
ttaacttgtt cctattgttg ataaccttgt attgattgct ctgctggtag taattagaaa 96600
actggtaaag aagtaaggag agaagcccaa agaggggggc agcacagcac acagattgtc 96660
ttcctgtctc ctaagaatct actgtgtagg ctgtgagctt agcatatttg cttctctttt 96720
taggtaaata tgttatagac acaaatgatg ttctctgttg attatttata accactccac 96780
gttccttctt agagtatctg cccttttcat tttttcaaag actcagctct catgagcccc 96840
acatgacctg ctacccccat caaccacagc tgattggata agcacctgac ctagactgtg 96900
ccagtcacat ttgattgttt cagatttctg aatcagagca tgaagaaaca gtaccagtta 96960
actttggatg ctgaaactat aggacgatgc agacttggaa ggtggacaag gtagctattt 97020
cgtgatagcc accatggacc ctgtattccc accaacaatc agagagcact tgtcagttaa 97080
gttcaccaga ctcctaatgg cattatagtt ctcccaccaa acttcttgcc ccagggtgta 97140
tggactatct ctgattattt gatagaagtt tccctgtaca ttagctgact caagttcatt 97200
gccatttttt gcaacccaaa gagtcccttc tataacaaag cactctcatc attgacttct 97260
ctctatctta gttttaagtg tctctggtga aaatgcaaat ttaaaggacc tattaaaaac 97320
aacagagtgg gagttgctat caaataaagt ataaactgct aaactgctaa ggcctctatc 97380
aagaccaatg ctgtgctaac tctttccaac ccagtaaaac gaggctttat tgtccaccac 97440
taacatgtca ccacctcccc tattgacttc agtcctttgg gaccccctaa ctctcatacc 97500
ccacccagct tccgagaact ctattgcctc ccagaagtaa tcatagtcaa cacttagtgg 97560
tagttatttt ggcttgaggc catggccttg caaagctggt attgaagtga tgctctcata 97620
tctaatttga ctgaattgag tgttccagta agactggtga tgttttcctt gcttcagggt 97680
ccatcctaat gagcaaatgc tgccttactt tcacagaagg ctgcaaatgt agacttgtaa 97740
agaaaatctt tacaaacact tcttggtgtc ttagagatca cccaacacaa ttacacatgg 97800
ggatatgctg ggaagtacta aaataaacct gatttatcct tcacatatgc ctttcatttg 97860
ctattgtggc ttcaaagctc aaaaaaaata gagagatgtt tctctaaact ttcactattt 97920
attaactgag aatgtaacgg aagtgaaaca gccaacaaaa attaaaagca gcttactcag 97980
ggatacagag tcatgactat gtggagcccc atatctttac ttctttgtct aaagtcctca 98040
taagcactgt ctgagtttgc atgagtccta caaactagaa caactcccaa cttcattgag 98100
aagaatgagg acatctttgc aaagaaggca gaggaaagcc acatgctact tgtaacactt 98160

tgatggctac ccagtgccaa ggtttttttt ttttttttta acaatcctga cagcagaaat 98220
ctggaagctt gatcctagaa gactgaagag cccccaacat gaccctgaac tctcacataa 98280
gagaggaact cttagttcgt ttgccattaa gagaaaacca cagactaggt catttataat 98340
gaacagaaat ttattttctc acagttctaa atgctgggac atccaagatc atgggacccg 98400
catccgacaa gagtgttcta gctgcatcat tccatggtgg aatggccaga gagggtgagg 98460
gagagaagca aaagggggcc aaactcatcc ctctgtaagg aactccctcc tgagataaca 98520
gcattaatca ttcatcaggg cagagccctc acggcctaat caccttttaa aggtcctatc 98580
tcttaatact gttataatta aacttcaacc tacatttggg agggaacaaa cattcaagcc 98640
acagtagaaa ccagcttctt tttctgttgt taaagcaaca ctttttttct tctctgttac 98700
ttgcagccta accagatcct aataaataca caagcttcta tgtcgacaaa tatgtctaca 98760
atgctgttta aatggcagca tggtattcta ggatagggaa gggccaccat ttactcaacc 98820
agcccattct tggaggatgt ttaagctatt tccaatattt tgctatgttc aacaaagtat 98880
taaggatctc cctagtagct aagcacccat gtaaatcctt agatatctta ggataaacct 98940
gtaaactagt ttatgtgtcc tagtttagct gggcccagct ggaccatcac acatatttaa 99000
atttttttga aacttattgc caacatgcat ctccagagag gtagtacacc aaattatgct 99060
cctacaaaag ttgcagaaac ttgcctgtct atccatactg tcacccacaa tggtgattat 99120
tactttttat ctgtgacaat ttgatttatg ctgggatttt gcagccagca ccaaggtagc 99180
atgttgtctc tcagtctaat aataatttcc ctctccctca tcatcatcac cagcactacc 99240
aatttctctg cactatgggt actaacatca aaaatgttcc catcacattt acttcatttt 99300
gaggaaaaga acagttacta ccattcattc attcactcat cacaccaata ttactgaatc 99360
ctaccctgga ccagcattgt tatggtagat cttgataata tagtgacaga caataccatc 99420
cttcccacca tttacatagt agactgcctg gggctcatag tctactcaca tctcagcaca 99480
tgagatagtc ccattatcac attctcttct atctctctca caggcactca ctcaccagcc 99540
atggagaact taatgggcat taggttctta atcctgctgg gtataattgg agataaccac 99600
cctggctgac tgagcactat accaggctac tcatggatga tttggttatt agacgtaatc 99660
agagtggccc ttgtatgcta aaaaggagac tcagtctcca tcccatggaa gattacattt 99720
ttctgactgc catccctcag atctagatgg tattgtctcc ctgacatcca aatatcttca 99780
atcatataac c 99791

## Description

### BACKGROUND OF THE INVENTION

Breast cancer is by far the most common cancer in women worldwide. Current global incidence is in excess of 1,151,000 new cases diagnosed each year (Parkin *et al.,* 2005). Breast cancer incidence is highest in developed countries, particularly amongst populations of Northern European ethnic origin, and is increasing. In the United States the annual age-standardized incidence rate is approximately 131 cases per 100,000 population, more than three times the world average. Rates in Northern European countries are similarly high. In the year 2006 it is estimated that 214,650 new cases of invasive breast cancer will be diagnosed in the U.S.A. and 41,430 people will die from the disease [Jemal, et al., (2006), CA Cancer J Clin, 56, 106-30]. To this figure must be added a further 61,980 ductal and lobular carcinoma in-situ diagnoses expected in 2006. From an individual perspective, the lifetime probability of developing breast cancer is 13.2% in U.S. women (i.e., 1 in 8 women will develop breast cancer during their lives). As with most cancers, early detection and appropriate treatment are important factors. Overall, the 5-year survival rate for breast cancer is 88%. However, in individuals presenting with regionally invasive or metastatic disease, the rate declines to 81% and 26%, respectively [Jemal, et al., (2006), CA Cancer J Clin, 56, 106-30].

Increasingly, emphasis is falling on the identification individuals who are at high risk for primary or recurrent breast cancer. Such individuals can be managed by more intensive screening, preventative chemotherapies, hormonal therapies and, in cases of individuals at extremely high risk, prophylactic surgery. Mass screening programs constitute a huge economic burden on health services, while preventative therapies have associated risks and quality of life consequences.

### Genetic Predisposition to Breast Cancer:

The two primary classes of known risk factors for breast cancer are endocrine factors and genetics. Regarding the latter, approximately 12% of breast cancer patients have one or more first degree relatives with breast cancer [(2001), Lancet, 358, 1389-99]. The well known, dominant breast cancer predisposition genes BRCA1 and BRCA2 confer greatly increased breast cancer risk to carriers, with lifetime penetrance estimates ranging from 40-80%. The presence of BRCA1 and BRCA2 mutations can account for the majority of families with 6 or more cases of breast cancer and for a large proportion of families comprising breast and ovarian or male breast cancer. However such families are very rare indeed. BRCA1 and BRCA2 mutations are found much less frequently in families with fewer cases or in families characterised by breast cancer cases only. Together, mutations in BRCA1 and BRCA2 can account for 15-20% of the risk for familial breast cancer. In non-founder populations, if all common BRCA mutations could be detected, between 2-3% of incident breast cancer patients would be expected to harbor a mutation [Gorski, et al., (2005), Breast Cancer Res Treat, 92, 19-24; (2000), Br J Cancer, 83, 1301-8]. This low "chance to find" statistic precludes the responsible use of BRCA mutation testing outside families with an obvious hereditary predisposition (Anon[(2003), J Clin Oncol, 21, 2397-406]). Rare, high penetrance mutations are known to occur in the TP53 and PTEN genes, however, these together account for no more than 5% of the total genetic risk for breast cancer [Easton, (1999), Breast Cancer Res, 1, 14-7]. Linkage studies have been largely unsuccessful in identifying any more, widespread mutations conferring high risk for breast cancer[Smith, et al., (2006), Genes Chromosomes Cancer, 45, 646-55].

Recent epidemiological studies have indicated that the majority of breast cancer cases arise in a predisposed, susceptible minority of the population [Antoniou, et al., (2002), Br J Cancer, 86, 76-83; Pharoah, et al., (2002), Nat Genet, 31, 33-6]. Data from twin studies and observations of the constant, high incidence of cancer in the contralateral breast of patients surviving primary breast cancer indicate that a substantial portion of the uncharacterised risk for breast cancer is related to endogenous factors, most probably genetic [Lichtenstein, et al., (2000), N Engl J Med, 343, 78-85; Peto and Mack, (2000), Nat Genet, 26, 411-4]. Knowledge of the genetic factors that underpin this widespread risk is very limited. Segregation analyses predict that the uncharacterised genetic risk for breast cancer is most likely to be polygenic in nature, with risk alleles that confer low to moderate risk and which may interact with each other and with hormonal risk factors. Nevertheless, these studies predict as much as 40-fold differences in relative risk between the highest and lowest quintiles of a distribution that could be defined by genetic profiling that captures these low to moderate risk alleles [Antoniou, et al., (2002), Br J Cancer, 86, 76-83; Pharoah, et al., (2002), Nat Genet, 31, 33-6]. 88% of all breast cancer cases are expected to arise amongst a predisposed 50% of the population and the 12% of the population at highest risk accounts for 50% of all breast cancer cases [Pharoah, et al., (2002), Nat Genet, 31, 33-6; Pharoah, (2003), Recent Results Cancer Res, 163, 7-18; discussion 264-6]. Much focus is therefore directed towards the identification of such genetically predisposed individuals and developing personalized medical management strategies for them.

We and others have shown that there is a significant familial risk of breast cancer in Iceland which extends to at least 5^{th} degree relatives [Amundadottir, et al., (2004), PLoS Med, 1, e65; Tulinius, et al., (2002), J Med Genet, 39, 457-62]. The contribution of BRCA1 mutations to familial risk in Iceland is thought to be minimal [Arason, et al., (1998), J Med Genet, 35, 446-9; Bergthorsson, et al., (1998), Hum Mutat, Suppl 1, S195-7]. A single founder mutation in the BRCA2 gene (999del5) is present at a carrier frequency of 0.6-0.8% in the general Icelandic population and 7.7-8.6% in female breast cancer patients [Thorlacius, et al., (1997), Am J Hum Genet, 60, 1079-84; Gudmundsson, et al., (1996), Am J Hum Genet, 58, 749-56]. This single mutation is estimated to account for approximately 40% of the inherited breast cancer risk to first through third degree relatives [Tulinius, et al., (2002), J Med Genet, 39, 457-62]. Although this estimate is higher than the 15-25% of familial risk attributed to all BRCA 1 and 2 mutations combined in non-founder populations, there is still some 60% of Icelandic familial breast cancer risk to be explained. First degree relatives of patients who test negative for BRCA2 999del5 remain at a 1.72 fold the population risk for breast cancer (95% CI 1.49-1.96) [Tulinius, et al., (2002), J Med Genet, 39, 457-62].

Understanding of the genetic factors contributing to the residual genetic risk for breast cancer is very limited. Variants in two genes have been rigorously confirmed as low penetrance breast cancer risk genes; CHEK2 and ATM [Renwick, et al., (2006), Nat Genet, 38, 873-5; (2004), Am J Hum Genet, 74, 1175-82]. Many other genes have been implicated however their contribution to breast cancer risk has not been confirmed in analyses employing very large sample sets [Breast Cancer Association, (2006), J Natl Cancer Inst, 98, 1382-96].

Genetic risk is conferred by subtle differences in genes among individuals in a population. Genes differ between individuals most frequently due to single nucleotide polymorphisms (SNP), although other variations are also important. SNP are located on average every 1000 base pairs in the human genome. Accordingly, a typical human gene containing 250,000 base pairs may contain 250 different SNP. Only a minor number of SNPs are located in exons and alter the amino acid sequence of the protein encoded by the gene. Most SNPs may have little or no effect on gene function, while others may alter transcription, splicing, translation, or stability of the mRNA encoded by the gene. Additional genetic polymorphism in the human genome is caused by insertion, deletion, translocation, or inversion of either short or long stretches of DNA. Genetic polymorphisms conferring disease risk may therefore directly alter the amino acid sequence of proteins, may increase the amount of protein produced from the gene, or may decrease the amount of protein produced by the gene.

As genetic polymorphisms conferring risk of common diseases are uncovered, genetic testing for such risk factors is becoming important for clinical medicine. Examples are apolipoprotein E testing to identify genetic carriers of the apoE4 polymorphism in dementia patients for the differential diagnosis of Alzheimer's disease, and of Factor V Leiden testing for predisposition to deep venous thrombosis. More importantly, in the treatment of cancer, diagnosis of genetic variants in tumor cells is used for the selection of the most appropriate treatment regime for the individual patient. In breast cancer, genetic variation in estrogen receptor expression or heregulin type 2 (Her2) receptor tyrosine kinase expression determine if anti-estrogenic drugs (tamoxifen) or anti-Her2 antibody (Herceptin) will be incorporated into the treatment plan. In chronic myeloid leukemia (CML) diagnosis of the Philadelphia chromosome genetic translocation fusing the genes encoding the Bcr and Abl receptor tyrosine kinases indicates that Gleevec (STI571), a specific inhibitor of the Bcr-Abl kinase should be used for treatment of the cancer. For CML patients with such a genetic alteration, inhibition of the Bcr-Abl kinase leads to rapid elimination of the tumor cells and remission from leukemia.

No universally successful method for the prevention or treatment of breast cancer is currently available. Management of breast cancer currently relies on a combination of primary prevention, early diagnosis, appropriate treatments and secondary prevention. There are clear clinical imperatives for integrating genetic testing into all aspects of these management areas. Identification of cancer susceptibility genes may also reveal key molecular pathways that may be manipulated (e.g., using small or large molecular weight drugs) and may lead to more effective treatments.

### SUMMARY OF THE INVENTION

The present invention relates to methods of risk assessment of breast cancer. This includes methods of determining an increased susceptibility to breast cancer in an individual, as well as methods of determining a decreased susceptibility to breast cancer or diagnosing a protection against cancer, in an individual, by evaluating certain markers or haplotypes that have been found to be associated with breast cancer, as described further herein.

In a first aspect, the invention pertains to a method for determining a susceptibility to breast cancer in a human individual, comprising determining the presence or absence of at least one allele of at least one polymorphic marker in a nucleic acid sample obtained from the individual or in a genotype dataset derived from the individual, wherein the at least one polymorphic marker is selected from the polymorphic markers set forth in any one of Table 10, Table 15 and Table 19, and markers in linkage disequilibrium therewith, and wherein the presence of the at least one allele is indicative of a susceptibility to breast cancer for the individual. The method may in one embodiment relate to determination of the presence or absence of at least one allele of at least one polymorphic marker in a nucleic acid sample obtained from the individual. In another embodiment, the method relates to determination of the presence or absence of at least one allele of at least one polymorhpic marker in a genotype dataset derived from the individual. The genotype dataset may, in certain embodiments, be derived from a particular individual by virtue of the information contained in the genotype dataset relating to a particular nucleic acid sample template, wherein the nucleic acid sample contains nucleic acid from a single individual.

Determination of the presence of a particular susceptibility allele of a polymorphic marker gives a direct indication of the presence of the particular susceptibility conferred by that allele. Determination of the absence of such a susceptibility allele gives on the other hand indication that the particular susceptibility is absent in the individual from which the genotype sample or gentotype datset is derived. In the particular case of a polymorphic marker with two possible alleles, such as a SNP or an insertion/deletion polymorphism, the determination of the absence of a particular allele implies the presence of two copies of the alternate allele in the individual (unless the particular genomic region contains a deletion or a duplication in the individual, in which there may be either only a single copy or more than two copies of the particular genomic region in the genome of the individual).

In a second aspect, the present invention relates to a method of diagnosing a susceptibility to breast cancer in a human individual, the method comprising determining the presence or absence of at least one allele of at least one polymorphic marker in a nucleic acid sample obtained from the individual, wherein the at least one polymorphic marker is selected from the group of markers associated with the rs4848543 LD block, the rs3803662 LD block, and the rs13387042 LD block, wherein the presence of the at least one allele is indicative of a susceptibility to breast cancer. In one embodiment, markers associated with the rs4848543 LD block, the rs3803662 LD block and/or the rs13387042 LD block are markers that are in linkage disequilibrium to at least one marker within one or more of these LD blocks.

In a further aspect, the invention relates to a method of diagnosing a susceptibility to breast cancer in a human individual, the method comprising determining the presence or absence of at least one allele of at least one polymorphic marker in a nucleic acid sample obtained from the individual, wherein the at least one polymorphic marker is selected from the group of markers consisting of the markers listed in Table 10, Table 15 and Table 19, and markers in linkage disequilibrium therewith. In one particular embodiment, the markers in linkage disequilibrium with the markers listed in Table 10, Table 15 and Table 19 are those markers with values of r² greater than 0.2. In another embodiment, the markers in linkage disequilibrium with the markers listed in Table 10, Table 15 and Table 19 are those markers with values of r² greater than 0.2 in a Caucasian population, such as the HapMap CEPH population.

In another aspect, the invention pertains to a method of identification of a marker for use in assessing susceptibility to breast cancer, the method comprising:
a. identifying at least one polymorphic marker in linkage disequilibrium with at least one of the markers within the genomic segments with sequence as set forth in SEQ ID NO:4, SEQ ID NO:5 and SEQ ID NO:6;
b. determining the genotype status of a sample of individuals diagnosed with, or having a susceptibility to, breast cancer; and
c. determining the genotype status of a sample of control individuals;
wherein a significant difference in frequency of at least one allele in at least one polymorphism in individuals diagnosed with, or having a susceptibility to, breast cancer, as compared with the frequency of the at least one allele in the control sample is indicative of the at least one polymorphism being useful for assessing susceptibility to breast cancer. In one embodiment, an increase in frequency of the at least one allele in the at least one polymorphism in individuals diagnosed with, or having a susceptibility to, breast cancer, as compared with the frequency of the at least one allele in the control sample is indicative of the at least one polymorphism being useful for assessing increased susceptibility to breast cancer. In another embodiment, a decrease in frequency of the at least one allele in the at least one polymorphism in individuals diagnosed with, or having a susceptibility to, breast cancer, as compared with the frequency of the at least one allele in the control sample is indicative of the at least one polymorphism being useful for assessing decreased susceptibility to, or protection against, breast cancer. In one embodiment, the at least one polymorphic marker in linkage disequilibrium with at least one of the markers within the genomic segments with sequence as set forth in SEQ ID NO:4, SEQ ID NO:5 and SEQ ID NO:6 is selected from rs4848543 (SEQ ID NO:1), rs3803662 (SEQ ID NO:3) and rs13387042 (SEQ ID NO:2).

The invention furthermore relates to a method of genotyping a nucleic acid sample obtained from a human individual at risk for, or diagnosed with, breast cancer, comprising determining the presence or absence of at least one allele of at least one polymorphic marker in the sample, wherein the at least one marker is selected from the group consisting of the markers set forth in Table 10, Table 15 and Table 19, and markers in linkage disequilibrium therewith, and wherein the presence or absence of the at least one allele of the at least one polymorphic marker is indicative of a susceptibility of breast cancer. In one embodiment, the at least one marker is selected from rs4848543 (SEQ ID NO:1), rs3803662 (SEQ ID NO:3) and rs13387042 (SEQ ID NO:2). In another embodiment, genotyping comprises amplifying a segment of a nucleic acid that comprises the at least one polymorphic marker by Polymerase Chain Reaction (PCR), using a nucleotide primer pair flanking the at least one polymorphic marker. In a further embodiment, genotyping is performed using a process selected from allele-specific probe hybridization, allele-specific primer extension, allele-specific amplification, nucleic acid sequencing, 5'-exonuclease digestion, molecular beacon assay, oligonucleotide ligation assay, size analysis, and single-stranded conformation analysis. In one preferred embodiment, the process comprises allele-specific probe hybridization. In another preferred embodiment, the process comprises DNA sequencing. In a particularly preferred embodiment, the process comprises the steps of
1) contacting copies of the nucleic acid with a detection oligonucleotide probe and an enhancer oligonucleotide probe under conditions for specific hybridization of the oligonucleotide probe with the nucleic acid;
   wherein
   a) the detection oligonucleotide probe is from 5-100 nucleotides in length and specifically hybridizes to a first segment of a nucleic acid whose nucleotide sequence is given by SEQ ID NO:4, SEQ ID NO:5 or SEQ ID NO:6;
   b) the detection oligonucleotide probe comprises a detectable label at its 3' terminus and a quenching moiety at its 5' terminus;
   c) the enhancer oligonucleotide is from 5-100 nucleotides in length and is complementary to a second segment of the nucleotide sequence that is 5' relative to the oligonucleotide probe, such that the enhancer oligonucleotide is located 3' relative to the detection oligonucleotide probe when both oligonucleotides are hybridized to the nucleic acid; and
   d) a single base gap exists between the first segment and the second segment, such that when the oligonucleotide probe and the enhancer oligonucleotide probe are both hybridized to the nucleic acid, a single base gap exists between the oligonucleotides;
2) treating the nucleic acid with an endonuclease that will cleave the detectable label from the 3' terminus of the detection probe to release free detectable label when the detection probe is hybridized to the nucleic acid; and
measuring free detectable label, wherein the presence of the free detectable label indicates that the detection probe specifically hybridizes to the first segment of the nucleic acid, and indicates the sequence of the polymorphic site as the complement of the detection probe.

A further aspect of the invention pertains to a method of assessing an individual for probability of response to a breast cancer therapeutic agent, comprising: determining the presence or absence of at least one allele of at least one polymorphic marker in a nucleic acid sample obtained from the individual, wherein the at least one polymorphic marker is selected from the group consisting of the polymorphic markers set forth in Table 10, Table 15 and Table 19, and markers in linkage disequilibrium therewith, wherein the presence of the at least one allele of the at least one marker is indicative of a probability of a positive response to the therapeutic agent. The therapeutic agent is in one embodiment a chemotherapeutic agent or hormonal therapy agent. In one embodiment, the hormonal therapy agent is a Selective Estrogen Receptor Modulator or an Aromatase inhibitor. In preferred embodiments, the Selective Estrogen Receptor Modulator is selected from Tamoxifen and Raloxifene. In certain embodiments, the aromatase inhibitor is selected from Exemestane, Anastrozole and Letrozole. In other embodiments, the Selective Estrogen Receptor Modulator is administered in combination with an aromatase inhibitor.

Yet another aspect of the invention pertains to a method of predicting prognosis of an individual diagnosed with breast cancer, the method comprising determining the presence or absence of at least one allele of at least one polymorphic marker in a nucleic acid sample obtained from the individual, wherein the at least one polymorphic marker is selected from the group consisting of the polymorphic markers set forth in Table 10, Table 15 and Table 19, and markers in linkage disequilibrium therewith, wherein the presence of the at least one allele is indicative of a worse prognosis of the breast cancer in the individual. The prognosis of the individual may in principle relate to any characteristic pattern of progression of the disease, including, but not limited to formation of a secondary tumor, rapid spread of the tumor, the grade of the tumor (Stage 0 through Stage IV) and the recurrence of the tumor.

The invention also relates to a method of monitoring progress of treatment of an individual undergoing treatment for breast cancer, the method comprising determining the presence or absence of at least one allele of at least one polymorphic marker in a nucleic acid sample obtained from the individual, wherein the at least one polymorphic marker is selected from the markers set forth in Table 10, Table 15 and Table 19, and markers in linkage disequilibrium therewith, wherein the presence of the at least one allele is indicative of the treatment outcome of the individual. Treatment can be any one of surgical treatment, chemotherapy treatment, radiotherapy, gene therapy or immunotherapy. Any of these therapies can be used alone or in combination. For example, surgical treatment may be followed by chemotherapy treatment and/or radiotherapy treatment. The treatment outcome of the individual relates to the tumor progression of the individual at the end of the treatment. Measures of such outcome include, but are not limited to, recurrence rate or likelihood of the tumor, spread of the tumor, and the formation of a secondary tumor.

Another aspect of the invention pertains to a kit for assessing susceptibility to breast cancer in a human individual, the kit comprising reagents for selectively detecting at least one allele of at least one polymorphic marker in the genome of the individual, wherein the polymorphic marker is selected from the markers set forth in Tables 10, 15 and 19, and markers in linkage disequilibrium therewith, and wherein the presence of the at least one allele is indicative of a susceptibility to breast cancer. In one embodiment, the at least one polymorphic marker is rs4848543 (SEQ ID NO:1), rs3803662 (SEQ ID NO:3) or rs13387042 (SEQ ID NO:2). In another embodiment, the reagents comprise at least one contiguous oligonucleotide that hybridizes to a fragment of the genome of the individual comprising the at least one polymorphic marker, a buffer and a detectable label. In another embodiment, the reagents comprise at least one pair of oligonucleotides that hybridize to opposite strands of a genomic nucleic acid segment obtained from the subject, wherein each oligonucleotide primer pair is designed to selectively amplify a fragment of the genome of the individual that includes one polymorphic marker, and wherein the fragment is at least 30 base pairs in size. In a further embodiment, the at least one oligonucleotide is completely complementary to the genome of the individual. In another embodiment, the oligonucleotide is from 18 to 50 nucleotides in size. In another embodiment, the oligonucleotide is from 20 to 30 nucleotides in size. In a preferred embodiment, the kit comprises:
a. a detection oligonucleotide probe that is from 5-100 nucleotides in length;
b. an enhancer oligonucleotide probe that is from 5-100 nucleotides in length; and
c. an endonuclease enzyme;
   wherein the detection oligonucleotide probe specifically hybridizes to a first segment of the nucleic acid whose nucleotide sequence is set forth in SEQ ID NO:4, SEQ ID NO: 5 or SEQ ID NO:6, and
   wherein the detection oligonucleotide probe comprises a detectable label at its 3' terminus and a quenching moiety at its 5' terminus;
   wherein the enhancer oligonucleotide is from 5-100 nucleotides in length and is complementary to a second segment of the nucleotide sequence that is 5' relative to the oligonucleotide probe, such that the enhancer oligonucleotide is located 3' relative to the detection oligonucleotide probe when both oligonucleotides are hybridized to the nucleic acid;
   wherein a single base gap exists between the first segment and the second segment, such that when the oligonucleotide probe and the enhancer oligonucleotide probe are both hybridized to the nucleic acid, a single base gap exists between the oligonucleotides; and
wherein treating the nucleic acid with the endonuclease will cleave the detectable label from the 3' terminus of the detection probe to release free detectable label when the detection probe is hybridized to the nucleic acid.

A further aspect of the invention pertains to an apparatus for determining a genetic indicator for breast cancer in a human individual, comprising:
a computer readable memory; and
a routine stored on the computer readable memory;
wherein the routine is adapted to be executed on a processor to analyze marker and/or haplotype information for at least one human individual with respect to at least one polymorphic marker selected from the markers set forth in Table 10, Table 15 and Table 19 and markers in linkage disequilibrium therewith, and generate an output based on the marker or haplotype information, wherein the output comprises an individual risk measure of the at least one marker or haplotype as a genetic indicator of breast cancer for the human individual. In one embodiment, the routine further comprises a risk measure for breast cancer associated with the at least one marker allele and/or haplotype, wherein the risk measure is based on a comparison of the frequency of at least one allele of at least one polymorphic marker and/or haplotype in a plurality of individuals diagnosed with breast cancer and an indicator of the frequency of the at least one allele of at least one polymorphic marker and/or haplotype in a plurality of reference individuals, and wherein the individual risk for the human individual is based on a comparison of the carrier status of the individual for the at least one marker allele and/or haplotype and the risk measure for the at least one marker allele and/or haplotype. In one embodiment, the at least one polymorphic marker is selected from rs4848543 (SEQ ID NO: 1), rs3803662 (SEQ ID NO:3) or rs13387042 (SEQ ID NO:2), and markers in linkage disequilibrium therewith

The invention also relates to the use of an oligonucleotide probe in the manufacture of a reagent for diagnosing and/or assessing susceptibility to breast cancer in a human individual, wherein the probe hybridizes to a segment of a nucleic acid whose nucleotide sequence is set forth in SEQ ID NO:4, SEQ ID NO:5 or SEQ ID NO:6, wherein the probe is 15-500 nucleotides in length.

The invention further relates to a computer-readable medium on which is stored:
a. an identifier for at least one polymorphic marker;
b. an indicator of the frequency of at least one allele of said at least one polymorphic marker in a plurality of individuals diagnosed with breast cancer; and
c. an indicator of the frequency of the least one allele of said at least one polymorphic markers in a plurality of reference individuals;
wherein the at least one polymorphic marker is selected from the polymorphic markers set forth in Table 10, Table 15 and Table 19, and polymorphic markers in linkage disequilibrium therewith.

In certain embodiments of the invention, the frequency of at least one haplotype in the individual is further assessed, wherein the haplotype comprises at least two markers, and wherein the presence of the at least one haplotype is indicative of a susceptibility to breast cancer. The haplotype is in one embodiment an at-risk haplotype for breast cancer, i.e., the haplotype confers an increased risk of developing breast cancer. The haplotype is in one embodiment selected from the group of haplotypes listed in Table 7, Table 8, Table 9, Table 13, Table 14 and Table 18.

In another embodiment of the invention, the at least one allele of at least one polymorphic marker is present on a haplotype background selected from the group of haplotype backgrounds defined by the haplotypes listed in Table 9 and Table 13. In another embodiment, the method of the present invention relates to further performing a step of assaying for a high penetrant genetic factor for breast cancer. Such a high penetrant genetic factor is in one embodiment BRCA2 999del5. Recent evidence suggests that risk associated with BRCA1 and BRCA2 variants are in some cases modified by other genetic or environmental factors that cluster in families (Antoniou, A.C., et al. Am J Hum Genet 2008 Mar 18 (Epub ahead of print)). Thus, in certain embodiments, other high penetrant mutations for breast cancer, such as those found in the BRCA1 and BRCA2 genes, which are well documented and known to the skilled person (see, e.g., Breast Cancer Mutation Data Base at: http://research.nhgri.nih.gov/bic/; see also Fackenthal, J.D. & Olopade, O.I., Nature Reviews Cancer 7:937-48 (2007) and references cited therein) may also be assessed and combined with the variants described herein to be associated with breast cancer.

In one embodiment of the invention, a further step of assessing the individual for estrogen receptor or progesterone receptor status is performed. In one such embodiment, estrogen positive status or progesterone positive status is associated with increased risk associated with rs13387042 allele A and rs3803662 allele T, and markers in association therewith, such as the markers as set forth in Table 15 and Table 19.

The present invention relates, in another aspect, to a method of assessing risk of developing at least a second primary tumor in an individual previously diagnosed with breast cancer, the method comprising determining the presence or absence of at least one allele of at least one polymorphic marker in a nucleic acid sample obtained from the individual, wherein the at least one polymorphic marker is selected from the group of markers associated with the rs4848543 LD Block, the rs13387042 LD block, and the rs3803662 LD block, wherein the presence of the at least one allele is indicative of risk of developing at least a second primary tumor. In one embodiment, the at least one polymorphic marker is selected from the markers set forth in any of the Tables 10, 15 and 19. In another embodiment, the at least one marker is associated with the rs4848543 LD block. In one embodiment, the at least one polymorphic marker is selected from the group consisting of the polymorphic markers listed in Table 10, Table 15, Table 19, Table 20, Table 21 and Table 22, and markers in linkage disequilibrium therewith. In another embodiment, the at least one polymorphic marker is selected from the group of markers in strong linkage disequilibrium, as defined by values of r² greater than 0.2, to marker rs4848543. In yet another embodiment, the at least one polymorphic marker is selected from the group of markers listed in Table7, Table 8 and Table 10. In another embodiment, the at least one polymorphic marker is selected from the markers set forth in Table 10. In a further embodiment, the at least one polymorphic marker is associated with the STEAP3/TSAP6 gene. In a preferred embodiment, marker is rs4848543. In other embodiments, additional steps of assessing the frequency of at least one haplotype in the individual are performed. This at least one haplotype is in one embodiment selected from the group of haplotypes listed in Table 7, Table 8 and Table 9. In other embodiments, high penetrant genetic factors for breast cancer, such as BRCA2 999del5, are also assessed.

The breast cancer phenotype according the invention as described herein can in certain embodiments be selected from All Breast Cancer, Multiple Primary Breast Cancer, early onset Breast Cancer, or other medically accepted diagnostic method of defining Breast Cancer. In another embodiment, the summed family history (FHS) is the phenotype associated with breast cancer.

In particular embodiments of the methods, uses, kits, or apparati of the invention, as described herein, the human individual that is assessed is female.

The markers disclosed herein to be useful for detecting a susceptibility to breast cancer can all be utilized in the various methods, kits, apparati and uses as described herein. Thus, in certain embodiments, the at least one polymorphic marker useful to practice the invention can be selected from the group consisting of the polymorphic markers listed in Tables 20, 21 and 22, and markers in linkage disequilibrium therewith. In another embodiment, the at least one polymorphic marker is selected from the group of markers listed in Table 10, Table 15 and Table 19, and markers in linkage disequilibrium therewith. In certain embodiments, the at least one polymorphic marker is located within the genomic segments with the sequence as set forth in any of SEQ ID NO:4, SEQ ID NO:5 and SEQ ID NO:6. In one preferred embodiment, the at least one marker within SEQ ID NO:4 is selected from the markers set forth in Table 20. In another preferred embodiment, the at least one marker within SEQ ID NO:5 is selected from the markers set forth in Table 21. In yet another preferred embodiment, the at least one marker within SEQ ID NO:6 is selected from the markers set forth in Table 22. In another embodiment, the at least one polymorphic marker is selected from the group of markers listed in Tables 7, 8, 10, 14, 15, 18 and 19. In another embodiment, the at least one polymorphic marker is selected from the group of markers in strong linkage disequilibrium, as defined by values of r² greater than 0.2, to marker rs4848543 (SEQ ID NO: 1), rs3803662 (SEQ ID NO:3) or rs13387042 (SEQ ID NO:2). In a preferred embodiment, the at least one polymorphic marker is selected from rs4848543 (SEQ ID NO: 1), rs3803662 (SEQ ID NO:3) and rs13387042 (SEQ ID NO: 2). In another preferred embodiment, the at least one polymorphic marker is rs4848543 (SEQ ID NO:1). In another preferred embodiment, the at least one polymorphic marker is rs3803662 (SEQ ID NO:3). In yet another preferred embodiment, the at least one polymorphic marker is rs13387042 (SEQ ID NO: 2). In a further embodiment, the at least one polymorphic marker is associated with (i.e., in linkage disequilibrium with) the STEAP3/TSAP6 gene, the LOC643714 gene, and/or the TNRC9 gene. In one such embodiment, the at least one polymorphic marker is located within the STEAP3/TSAP6 gene, the LOC643714 gene and/or the TNRC9 gene. In another preferred embodiment, the at least one polymorphic marker is selected from rs4848543, rs13387042, rs3803662, rs12922061, rs4784227 and rs17271951.

In certain methods, uses, kits or apparati of the invention, the presence of at least one allele or haplotype is indicative of increased susceptibility to breast cancer. In other methods of the invention, the presence of at least one allele or haplotype is indicative of decreased susceptibility to breast cancer. Increased susceptibility to breast cancer is in one embodiment present on a haplotype background selected from the group of haplotype backgrounds consisting of
(i) rs8955398 allele 2, rs4848543 allele 1, rs6759589 allele 1, rs838066 allele 2, rs838100 allele 3, rs838086 allele 1, rs12711924 allele 1 and rs3731603 allele 4;
(ii) rs8955398 allele 4, rs4848543 allele 1, rs6759589 allele 1, rs838066 allele 2, rs838100 allele 3, rs838086 allele 1, rs12711924 allele 1 and rs3731603 allele 4;
(iii) rs8955398 allele 4, rs4848543 allele 1, rs6759589 allele 1, rs838066 allele 2, rs838100 allele 3, rs838086 allele 2, rs12711924 allele 1 and rs3731603 allele 4;
(iv) rs8955398 allele 2, rs4848543 allele 1, rs6759589 allele 1, rs838066 allele 2, rs838100 allele 3, rs838086 allele 2, rs12711924 allele 1 and rs3731603 allele 3;
(v) rs8955398 allele 4, rs4848543 allele 1, rs6759589 allele 1, rs838066 allele 2, rs838100 allele 3, rs838086 allele 1, rs12711924 allele 1 and rs3731603 allele 3;
(vi) rs8955398 allele 2, rs4848543 allele 1, rs6759589 allele 1, rs838066 allele 2, rs838100 allele 3, rs838086 allele 1, rs12711924 allele 1 and rs3731603 allele 4.

In another embodiment, increased susceptibility to breast cancer is present on a haplotype background selected from the group of haplotype backgrounds consisting of
(i) rs10191184 allele 3, rs6435957 allele 4, rs10171745 allele 3, rs6716542 allele 1, rs4491709 allele 4, rs12621130 allele 1, rs6735174 allele 1, rs6435959 allele 2, rs2372943 allele 3, rs13387042 allele 1, rs10490444 allele 1, rs13011060 allele 3;
(ii) rs10191184 allele 3, rs6435957 allele 4, rs10171745 allele 3, rs6716542 allele 3, rs4491709 allele 4, rs12621130 allele 1, rs6735174 allele 2, rs6435959 allele 4, rs2372943 allele 3, rs13387042 allele 1, rs10490444 allele 3, rs13011060 allele 3;
(iii) rs10191184 allele 3, rs6435957 allele 2, rs10171745 allele 3, rs6716542 allele 3, rs4491709 allele 4, rs12621130 allele 1, rs6735174 allele 2, rs6435959 allele 4, rs2372943 allele 3, rs13387042 allele 1, rs10490444 allele 3, rs13011060 allele 3;
(iv) rs10191184 allele 3, rs6435957 allele 4, rs10171745 allele 3, rs6716542 allele 3, rs4491709 allele 4, rs12621130 allele 1, rs6735174 allele 1, rs6435959 allele 2, rs2372943 allele 3, rs13387042 allele 1, rs10490444 allele 3, rs13011060 allele 3;
(v) rs10191184 allele 3, rs6435957 allele 4, rs10171745 allele 1, rs6716542 allele 3, rs4491709 allele 2, rs12621130 allele 1, rs6735174 allele 1, rs6435959 allele 2, rs2372943 allele 3, rs13387042 allele 1, rs10490444 allele 1, rs13011060 allele 3;
(vi) rs10191184-allele 3, rs6435957 allele 4, rs10171745 allele 3, rs6716542-allele 3, rs4491709 allele 4, rs12621130 allele 1, rs6735174 allele 2, rs6435959 allele 4, rs2372943 allele 3, rs13387042 allele 1, rs10490444 allele 3, rs13011060 allele 1;
(vii) rs10191184 allele 3, rs6435957 allele 4, rs10171745 allele 3, rs6716542 allele 1, rs4491709 allele 4, rs12621130 allele 1, rs6735174 allele 1, rs6435959 allele 2, rs2372943 allele 3, rs13387042 allele 1, rs10490444 allele 1, rs13011060 allele 1;
(viii) rs10191184 allele 3, rs6435957 allele 2, rs10171745 allele 3, rs6716542 allele 1, rs4491709 allele 4, rs12621130 allele 1, rs6735174 allele 1, rs6435959 allele 2, rs2372943 allele 3, rs13387042 allele 1, rs10490444 allele 1, rs13011060 allele 3;
(ix) rs10191184 allele 3, rs6435957 allele 4, rs10171745 allele 3, rs6716542 allele 1, rs4491709 allele 4, rs12621130 allele 1, rs6735174 allele 1, rs6435959 allele 2, rs2372943 allele 3, rs13387042 allele 1, rs10490444 allele 3, rs13011060 allele 1;
(x) rs10191184 allele 3, rs6435957 allele 2, rs10171745 allele 3, rs6716542 allele 3, rs4491709 allele 4, rs12621130 allele 1, rs6735174 allele 1, rs6435959 allele 2, rs2372943 allele 3, rs13387042 allele 1, rs10490444 allele 1, rs13011060 allele 3.

In a preferred embodiment, the at least one allele or haplotype conferring increased risk of breast cancer is rs4848543 allele 1. In further embodiments, the increased risk is characterized by a relative risk or odds ratio of at least 1.2, including a risk of at least 1.25, a risk of at least 1.3, a risk of at least 1.4, a risk of at least 1.5, a risk of at least 1.6, a risk of at least 1.7, and a risk of at least 2.0.

In certain other embodiments of the invention, the presence of the at least one allele or haplotype is indicative of decreased susceptibility (decreased risk) to breast cancer. The at least one allele or haplotype may for example be selected from the group of marker alleles and haplotypes listed in Tables 8, 9, 13, 14 and 18 having a relative risk (RR) or odds ratio (OR) value of less than one. In one embodiment the at least one allele indicative of decreased susceptibility to breast cancer is present on a haplotype background selected from the group of haplotype backgrounds consisting of
(i) rs895398 allele 2, rs4848543 allele 2, rs6759589 allele 3, rs838066 allele 4, rs838100 allele 1, rs838086 allele 1, rs12711924 allele 3 and rs3731603 allele 3;
(ii) rs895398 allele 2, rs4848543 allele 2, rs6759589 allele 3, rs838066 allele 2, rs838100 allele 1, rs838086 allele 1, rs12711924 allele 3 and rs3731603 allele 3;
(iii) rs895398 allele 2, rs4848543 allele 2, rs6759589 allele 1, rs838066 allele 2, rs838100 allele 3, rs838086 allele 2, rs12711924 allele 3 and rs3731603 allele 3;
(iv) rs895398 allele 2, rs4848543 allele 2, rs6759589 allele 3, rs838066 allele 4, rs838100 allele 3, rs838086 allele 2, rs12711924 allele 3 and rs3731603 allele 3;
(v) rs895398 allele 2, rs4848543 allele 2, rs6759589 allele 3, rs838066 allele 2, rs838100 allele 3, rs838086 allele 2, rs12711924 allele 3 and rs3731603 allele 3;
(vi) rs895398 allele 2, rs4848543 allele 2, rs6759589 allele 1, rs838066 allele 2, rs838100 allele 3, rs838086 allele 1, rs12711924 allele 1 and rs3731603 allele 4;
(vii) rs895398 allele 2, rs4848543 allele 2, rs6759589 allele 3, rs838066 allele 4, rs838100 allele 3, rs838086 allele 1, rs12711924 allele 1 and rs3731603 allele 4;
(viii) rs895398 allele 2, rs4848543 allele 2, rs6759589 allele 1, rs838066 allele 2, rs838100 allele 3, rs838086 allele 1, rs12711924 allele 3 and rs3731603 allele 3; and
(ix) rs895398 allele 2, rs4848543 allele 2, rs6759589 allele 3, rs838066 allele 2, rs838100 allele 1, rs838086 allele 1, rs12711924 allele 1 and rs3731603 allele 4.

In another embodiment, decreased susceptibility to breast cancer is present on a haplotype background selected from the group of haplotype backgrounds consisting of
(i) rs10191184 allele 3, rs6435957 allele 2, rs10171745 allele 3, rs6716542 allele 3, rs4491709 allele 2, rs12621130 allele 3, rs6735174 allele 1, rs6435959 allele 2, rs2372943 allele 3, rs13387042 allele 3, rs10490444 allele 1, rs13011060 allele 3;
(ii) rs10191184 allele 3, rs6435957 allele 4, rs10171745 allele 3, rs6716542 allele 3, rs4491709 allele 4, rs12621130 allele 1, rs6735174 allele 1, rs6435959 allele 4, rs2372943 allele 1, rs13387042 allele 3, rs10490444 allele 3, rs13011060 allele 1;
(iii) rs10191184 allele 1, rs6435957 allele 4, rs10171745 allele 3, rs6716542 allele 3, rs4491709 allele 4, rs12621130 allele 1, rs6735174 allele 1, rs6435959 allele 4, rs2372943 allele 1, rs13387042 allele 3, rs10490444 allele 3, rs13011060 allele 1;
(iv) rs10191184 allele 3, rs6435957 allele 2, rs10171745 allele 3, rs6716542 allele 3, rs4491709 allele 2, rs12621130 allele 3, rs6735174 allele 1, rs6435959 allele 2, rs2372943 allele 3, rs13387042 allele 3, rs10490444 allele 1, rs13011060 allele 1;
(v) rs10191184 allele 1, rs6435957 allele 2, rs10171745 allele 1, rs6716542 allele 3, rs4491709 allele 2, rs12621130 allele 1, rs6735174 allele 1, rs6435959 allele 2, rs2372943 allele 3, rs13387042 allele 3, rs10490444 allele 3, rs13011060 allele 3;
(vi) rs10191184 allele 3, rs6435957 allele 4, rs10171745 allele 3, rs6716542 allele 3, rs4491709 allele 4, rs12621130 allele 1, rs6735174 allele 1, rs6435959 allele 4, rs2372943 allele 1, rs13387042 allele 3, rs10490444 allele 3, rs13011060 allele 3;
(vii) rs10191184 allele 1, rs6435957 allele 2, rs10171745 allele 3, rs6716542 allele 3, rs4491709 allele 4, rs12621130 allele 1, rs6735174 allele 1, rs6435959 allele 4, rs2372943 allele 1, rs13387042 allele 3, rs10490444 allele 3, rs13011060 allele 1;
(viii) rs10191184 allele 3, rs6435957 allele 4, rs10171745 allele 3, rs6716542 allele 3, rs4491709 allele 4, rs12621130 allele 1, rs6735174 allele 1, rs6435959 allele 2, rs2372943 allele 3, rs13387042 allele 3, rs10490444 allele 1, rs13011060 allele 3;
(ix) rs10191184 allele 3, rs6435957 allele 2, rs10171745 allele 3, rs6716542 allele 3, rs4491709 allele 2, rs12621130 allele 1, rs6735174 allele 1, rs6435959 allele 2, rs2372943 allele 3, rs13387042 allele 3, rs10490444 allele 1, rs13011060 allele 3;
(x) rs10191184 allele 1, rs6435957 allele 2, rs10171745 allele 3, rs6716542 allele 3, rs4491709 allele 2, rs12621130 allele 3, rs6735174 allele 1, rs6435959 allele 2, rs2372943 allele 3, rs13387042 allele 3, rs10490444 allele 1, rs13011060 allele 3;
(xi) rs10191184 allele 3, rs6435957 allele 4, rs10171745 allele 3, rs6716542 allele 3, rs4491709 allele 4, rs12621130 allele 1, rs6735174 allele 1, rs6435959 allele 4, rs2372943 allele 3, rs13387042 allele 3, rs10490444 allele 3, rs13011060 allele 1.

In certain embodiments, the decreased susceptibility is characterized by a risk (relative risk or odds ratio) of less than 0.9, including a risk of less than 0.8, a risk of less than 0.7, a risk of less than 0.6, and a risk of less than 0.5.

In principle, assessment of the markers and haplotypes shown herein to be associated with breast cancer can be combined with analysis of a sample comprising genomic DNA from a human individual or a genotype dataset derived from a human individual for the presence or absence of at least one at-risk allele of at least one at-risk variant for breast cancer not in linkage disequilibrium with any one of the markers set forth in Table 10, Table 15 and Table 19. In other words, genetic risk factors not linked to any of the risk factors described herein can be combined with the risk factors of the present invention, so as to obtain an estimate for overall risk for the individual based on a plurality of risk factors. Furthermore, analysis more than one risk factors described herein to be associated with breast cancer can be combined so as to obtain overall combined risk. In one such embodiment, analysis of rs4848543 (SEQ ID NO: 1), rs3803662 (SEQ ID NO:3) and rs13387042 (SEQ ID NO:2) is performed for an individual, or a sample from an individual, and overall risk analysis performed. In another embodiment, analysis of rs3803662 (SEQ ID NO:3) and rs13387042 (SEQ ID NO:2) is performed.

The methods, uses, kits and apparati of the invention can in certain embodiments further comprise analyzing non-genetic information to make risk assessment, diagnosis, or prognosis of the individual. Such non-genetic information can in certain embodiments include age, gender, ethnicity, socioeconomic status, previous disease diagnosis, medical history of subject, family history of breast cancer, biochemical measurements, and/or clinical measurements. Combined risk for the genetic and non-genetic risk factors can be performed using methods known to the skilled person.

Linkage disequilibrium (LD) between genetic segments (e.g., markers) is in certain embodiments of the invention characterized by certain values of a linkage disequilibrium measure. As further described herein, linkage disequilibrium can be characterized by particular numerical values of the LD measures r² and |D'|. In one preferred embodiment, linkage disequilibrium is characterized by values for r² of greater than 0.1. In another preferred embodiment, linkage disequilibrium is characterized by values for r² of greater than 0.2. Other cutoff values for r² are also possible, including, but not limited to, 0.3, 0.4, 0.5, 0.6, 0.7, 0.8, 0.9, 0.95, 0.96, 0.97, 0.98, 0.99. In another preferred embodiment, linkage disequilibrium is characterized by values for |D'| of greater than 0.5. In another preferred embodiment, linkage disequilibrium is characterized by values for |D'| of greater than 0.8. Other cutoff values for |D'| are also possible, including, but not limited to, 0.2, 0.3, 0.4, 0.6, 0.7, 0.8, 0.9, 0.95, 0.96, 0.97, 0.98 and 0.99. In certain embodiments, linkage disequilibrium is characterized by numeric cutoff values for either |D'| and r². In one such embodiment linkage disequilibrium is characterized by numeric cutoff values for either |D'| of greater than 0.8 and r² of greater than 0.2, or both. In certain embodiments, LD is determined in a particular population. In certain such embodiments, the population is selected from Caucasian, Chinese, Japanese, and African populations. In one embodiment, the population is the Caucasian CEPH population.

In certain other embodiments of the methods, uses, apparatus or kits of the invention, the individual is of a specific human ancestry. In one embodiment, the ancestry is selected from black African ancestry, Caucasian ancestry and Chinese ancestry. In another embodiment, the ancestry is black African ancestry. In another embodiment, the ancestry is African American ancestry. In another embodiment, the ancestry is European ancestry. In another embodiment, the ancestry is Caucasian ancestry. The ancestry is in certain embodiment self-reported by the individual who undergoes genetic analysis or genotyping. In other embodiments, the ancestry is determined by genetic determination comprising detecting at least one allele of at least one polymorphic marker in a nucleic acid sample from the individual, wherein the presence or absence of the allele is indicative of the ancestry of the individual.

### BRIEF DESCRIPTION OF THE DRAWINGS

The foregoing and other objects, features and advantages of the invention will be apparent from the following more particular description of preferred embodiments of the invention.

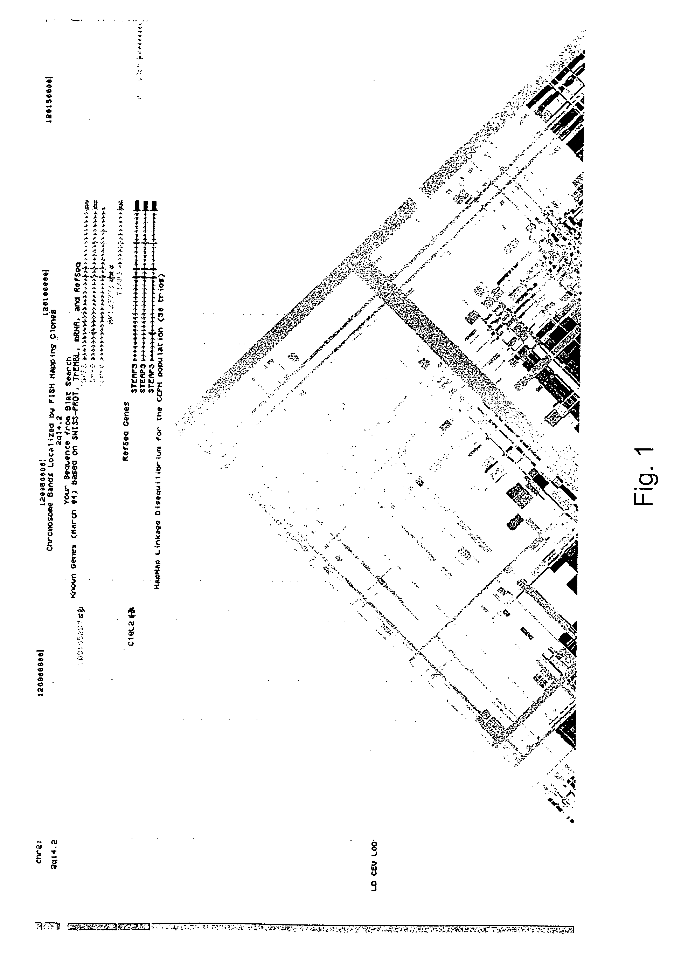

FIG 1 shows the genomic structure of region on chromosome 2 that includes marker rs4848543 that is shown herein to be associated with breast cancer. Markers in linkage disequilibrium with rs4848543 are also found within this region, which is also called rs4848543 LD block herein. The region is characterized by extensive linkage disequilibrium (high LD), and its the boundaries are characterized by regions of high recombination. Markers in linkage disequilibrium with rs4848543, as defined by values for the LD measure r² of greater than 0.2 in the Caucasian CEU HapMap population, span positions 120,023,583 - 120,117,062 on chromosome 2 in NCBI Build 34, and position 119,644,908-119,738,387 bp in NCBI Build 36. In both of these sequence builds, the region spanned by these markers is 93,479 bp.

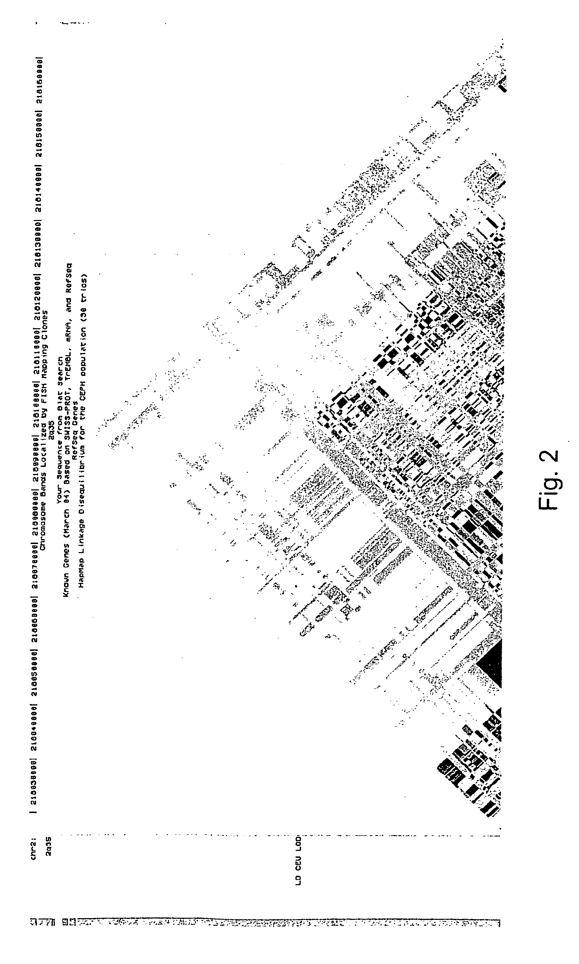

FIG 2 shows the genomic structure of the region on chromosome 2 that includes marker rs13387042. Markers in linkage disequilibrium with rs13387042 are also found within this region, which is also called rs13387042 LD block herein. The region is characterized by extensive linkage disequilibrium (high LD), and its the boundaries are characterized by regions of high recombination. Markers in linkage disequilibrium with rs13387042, as defined by values for the LD measure r² of greater than 0.2 in the Caucasian CEU HapMap population, span positions 218,059,508 - 218,141,061 on chromosome 2 in NCBI Build 34, and position 217,565,211-217,646,764 bp in NCBI Build 36. In both of these sequence builds, the region spans 81,553 bp.

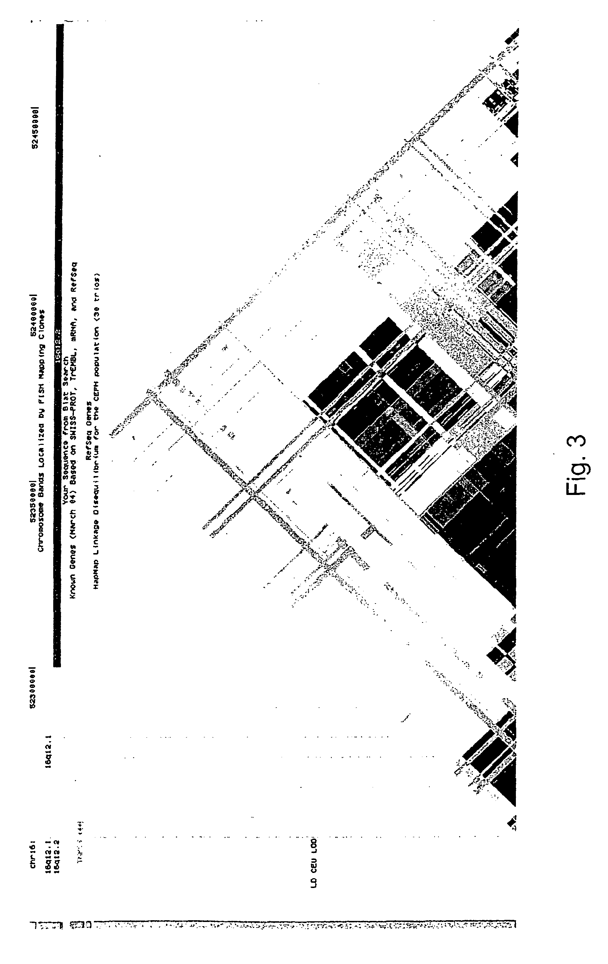

FIG 3 shows the genomic structure of the region on chromosome 16 that includes marker rs3803662. Markers in linkage disequilibrium with rs3803662 are also found within this region, which is also called rs3803662 LD block herein. The region is characterized by extensive linkage disequilibrium (high LD), and its the boundaries are characterized by regions of high recombination. Markers in linkage disequilibrium with rs3803662, as defined by values for the LD measure r² of greater than 0.2 in the Caucasian CEU HapMap population, span positions 52,314,403 - 52,413,602 on chromosome 16 in NCBI Build 34, and position 51,093,311-51,192,501 bp in NCBI Build 36. In both of these sequence builds, the region spans 99,190 bp.

### DETAILED DESCRIPTION OF THE INVENTION

A description of preferred embodiments of the invention follows.

The present invention discloses polymorphic variants and haplotypes that have been found to be associated with breast cancer. Particular alleles at certain polymorphic markers (e.g., marker rs4848543, marker rs13387042 and marker rs3803662, and markers in linkage disequilibrium therewith, the markers associated with the rs4848543 LD block, the rs13387042 LD block, and the rs3803662 LD block, e.g., the markers of Table 10, Table 15, Table 19, Table 20, Table 21 and Table 22, e.g., the markers listed in Table 10, Table 15 and Table 19) and haplotypes comprising such alleles have been found to be associated with breast cancer. Such markers and haplotypes are useful for risk management of breast cancer, as described in further detail herein. Further applications of the invention include kits for assessing the presence or absence of particular alleles of these markers..

### Definitions

Unless otherwise indicated, nucleic acid sequences are written left to right in a 5' to 3' orientation. Numeric ranges recited within the specification are inclusive of the numbers defining the range and include each integer or any non-integer fraction within the defined range. Unless defined otherwise, all technical and scientific terms used herein have the same meaning as commonly understood by the ordinary person skilled in the art to which the invention pertains.

The following terms shall, in the present context, have the meaning as indicated:

A "polymorphic marker", sometimes referred to as a "marker", as described herein, refers to a genomic polymorphic site. Each polymorphic marker has at least two sequence variations characteristic of particular alleles at the polymorphic site. Thus, genetic association to a polymorphic marker implies that there is association to at least one specific allele of that particular polymorphic marker. The marker can comprise any allele of any variant type found in the genome, including single nucleotide polymorphisms (SNPs), microsatellites, insertions, deletions, duplications and translocations.

An "allele" refers to the nucleotide sequence of a given locus (position) on a chromosome. A polymorphic marker allele thus refers to the composition (i.e., sequence) of the marker on a chromosome. Genomic DNA from an individual contains two alleles (e.g., allele-specific sequences) for any given polymorphic marker, representative of each copy of the marker on each chromosome. Sequence codes for nucleotides used herein are: A = 1, C = 2, G = 3, T = 4. For microsatellite alleles, the CEPH sample (Centre d'Etudes du Polymorphisme Humain, genomics repository, CEPH sample 1347-02) is used as a reference, the shorter allele of each microsatellite in this sample is set as 0 and all other alleles in other samples are numbered in relation to this reference. Thus, e.g., allele 1 is 1 bp longer than the shorter allele in the CEPH sample, allele 2 is 2 bp longer than the shorter allele in the CEPH sample, allele 3 is 3 bp longer than the lower allele in the CEPH sample, etc., and allele -1 is 1 bp shorter than the shorter allele in the CEPH sample, allele -2 is 2 bp shorter than the shorter allele in the CEPH sample, etc.

Sequence conucleotide ambiguity as described herein is as proposed by IUPAC-IUB. These codes are compatible with the codes used by the EMBL, GenBank, and PIR databases.

| **IUB code** | **Meaning** |
|---|---|
| A | Adenosine |
| C | Cytidine |
| G | Guanine |
| T | Thymidine |
| R | G or A |
| Y | T or C |
| K | G or T |
| M | A or C |
| S | G or C |
| W | A or T |
| B | C G or T |
| D | A G or T |
| H | A C or T |
| V | A C or G |
| N | A C G or T (Any base) |

A nucleotide position at which more than one sequence is possible in a population (either a natural population or a synthetic population, e.g., a library of synthetic molecules) is referred to herein as a "polymorphic site".

A "Single Nucleotide Polymorphism" or "SNP" is a DNA sequence variation occurring when a single nucleotide at a specific location in the genome differs between members of a species or between paired chromosomes in an individual. Most SNP polymorphisms have two alleles. Each individual is in this instance either homozygous for one allele of the polymorphism (i.e. both chromosomal copies of the individual have the same nucleotide at the SNP location), or the individual is heterozygous (i.e. the two sister chromosomes of the individual contain different nucleotides). The SNP nomenclature as reported herein refers to the official Reference SNP (rs) ID identification tag as assigned to each unique SNP by the National Center for Biotechnological Information (NCBI).

A "variant", as described herein, refers to a segment of DNA that differs from the reference DNA. A "marker" or a "polymorphic marker", as defined herein, is a variant. Alleles that differ from the reference are referred to as "variant" alleles.

A "microsatellite" is a polymorphic marker that has multiple small repeats of bases that are 2-8 nucleotides in length (such as CA repeats) at a particular site, in which the number of repeat lengths varies in the general population.

An "indel" is a common form of polymorphism comprising a small insertion or deletion that is typically only a few nucleotides long.

A "haplotype," as described herein, refers to a segment of genomic DNA within one strand of DNA that is characterized by a specific combination of alleles arranged along the segment. For diploid organisms such as humans, a haplotype comprises one member of the pair of alleles for each polymorphic marker or locus. In certain embodiments, the haplotype can comprise two or more alleles, three or more alleles, four or more alleles, or five or more alleles. Haplotypes are described herein in the context of the marker name and the allele of the marker in that haplotype, e.g., "1 rs4848543" or "1-rs4848543" refers to the 1 allele (A allele) of marker rs4848543 being in the haplotype, and is equivalent to "rs4848543 allele 1" or "rs4848543 allele A). Furthermore, allelic codes in haplotypes are as for individual markers, i.e. 1 = A, 2 = C, 3 = G and 4 = T.

The term "susceptibility", as described herein, refers to an individual (or group of individuals) being prone to developing breast cancer, or being less able to resist developing breast cancer, than the average individual. The term encompasses both increased susceptibility and decreased susceptibility. Thus, particular polymorphic markers and/or haplotypes of the invention may be characteristic of increased susceptibility (i.e., increased risk) of breast cancer, as characterized by a relative risk (RR) of greater than one or odds ratio (OR) of greater than one. Alternatively, the markers and/or haplotypes of the invention are characteristic of decreased susceptibility (i.e., decreased risk) of breast cancer, as characterized by a relative risk or odds ratio of less than one.

The term "and/or" shall in the present context be understood to indicate that either or both of the items connected by it are involved. In other words, the term herein shall be taken to mean "one or the other or both".

The term "associated with" shall, in the present context of linking genetic elements (genes and/or markers) refer to the elements being in linkage disequilibrium. Preferably, the term shall be taken to mean that the elements are in linkage disequilibrium as determined by values for the linkage disequilibrium measure r² of greater than 0.2.

The term "look-up table", as described herein, is a table that correlates one form of data to another form, or one or more forms of data to a predicted outcome to which the data is relevant, such as phenotype or trait. For example, a look-up table can comprise a correlation between allelic data for at least one polymorphic marker and a particular trait or phenotype, such as a particular disease diagnosis, that an individual who comprises the particular allelic data is likely to display, or is more likely to display than individuals who do not comprise the particular allelic data. Look-up tables can be multidimensional, i.e. they can contain information about multiple alleles for single markers simultaneously, or the can contain information about multiple markers, and they may also comprise other factors, such as particulars about diseases diagnoses, racial information, biomarkers, biochemical measurements, therapeutic methods or drugs, etc.

A "computer-readable medium", is an information storage medium that can be accessed by a computer using a commercially available or custom-made interface. Exemplary compute-readable media include memory (e.g., RAM, ROM, flash memory, etc.), optical storage media (e.g., CD-ROM), magnetic storage media (e.g., computer hard drives, floppy disks, etc.), punch cards, or other commercially available media. Information may be transferred between a system of interest and a medium, between computers, or between computers and the computer-readable medium for storage or acess of stored information. Such transmission can be electrical, or by other available methods, such as IR links, wireless connections, etc.

A "nucleic acid sample" is a sample obtained from an individual that contains nucleic acid (DNA or RNA). In certain embodiments, i.e. the detection of specific polymorphic markers and/or haplotypes, the nucleic acid sample comprises genomic DNA. Such a nucleic acid sample can be obtained from any source that contains genomic DNA, including as a blood sample, sample of amniotic fluid, sample of cerebrospinal fluid, or tissue sample from skin, muscle, buccal or conjunctival mucosa, placenta, gastrointestinal tract or other organs.

The term "breast cancer therapeutic agent" refers to an agent that can be used to ameliorate or prevent symptoms associated with breast cancer.

The term "breast cancer-associated nucleic acid", as described herein, refers to a nucleic acid that has been found to be associated to breast cancer. This includes, but is not limited to, the markers and haplotypes described herein and markers and haplotypes in strong linkage disequilibrium (LD) therewith. In one embodiment, a breast cancer-associated nucleic acid refers to a particular genomic region, such as an LD-block, found to be associated with breast cancer through at least one polymorphic marker located within the region or associated with (i.e., in linkage disequilibrium with) the region.

The term "All Breast Cancer", or *"All BC",* refers to all individuals diagnosed with breast cancer, irrespective of the particular subphenotype of the breast cancer.

The term "Medium Predisposition" breast cancer or *"MedPre"* breast cancer, refers to a subphenotype of breast cancer. The definition of this phenotype requires that the proband fulfills at least one of the following criteria:
1. The proband is a member of a cluster of breast cancer cases containing 3 or more affected relatives within a genetic distance of 3 meiotic events (3M).
2. The proband is a member of an affected pair related within 3M, one of whom was diagnosed when aged 50 or younger.
3. The proband is a member of an affected pair related within 3M, one of whom was diagnosed with a second primary tumor of any type.
4. The proband has been diagnosed with a second primary tumor of any type.

Out of the 1600 Icelandic patients analyzed in the study presented herein, 653 fulfills the *MedPre* criteria (40.8%).

The term "Multiple Primary Breast Tumor", or "MPBC", as described herein, refers to cases where at least one Primary tumor is diagnosed in addition to the first breast cancer diagnosis, and the two tumors confirmed both clinically and by histology to be independent primary tumors, arising simultaneously or subsequently to the first breast cancer and occurring in the contralateral or ipsilateral breast.

The term "family history score" or "FHS", as described herein, is defined based on the number of relatives affected with breast cancer for a proband with the disease. For each proband, a score of 1 is assigned for each affected first-degree relative, 0.5 for each affected second degree relative, and 0.25 for each third-degree relative. The total sum thus obtained over all affected relatives represents the summed family history score or FHS.

The term "rs4848543 LD block", or "rs4848543 linkage disequilibrium block", as described herein, refers to the genomic region on Chromosome 2 between positions 119,987,002 and 120,129,001 of NCBI (National Center for Biotechnology Information) Build 34, and between positions 119,608,327 and 120,129,001 in NCBI Build 36. The rs4848543 LD block is 141,999 bp in size.

The term "STEAP3 gene", or "TSAP6 gene" as described herein, refers to the Six-transmembrane Epithelial Antigen of the Prostate (STEAP3) gene, also called Tumor Suppressor Activated Pathway 6 (TSAP6) gene. The gene is located on chromosome 2q14.2, and spans positions 120076561-120118373 (NCBI Build 34).

The term "rs13387042 LD block", or "rs13387042 linkage disequilibrium block", as described herein, refers to the Linkage Disequilibrium (LD) block on Chromosome 2 between positions 218,062,001 and 218,141,002 of NCBI (National Center for Biotechnology Information) Build 34. The LD block spans 79,001 base pairs (Build 34).

The term "rs3803662 LD block", or "rs3803662 linkage disequilibrium block", as described herein, refers to the Linkage Disequilibrium (LD) block on Chromosome 16 between positions 52,291,041 and 52,436,127 of NCBI (National Center for Biotechnology Information) Build 34. The LD block spans 145,086 base pairs (Build 34).

The term "Daly haplotype", as described herein, refers two-marker haplotypes within a given LD block (region of high LD) that are identified as efficient surrogates (*r*²>0.8) for an additional set of SNPs typed in the HapMap project. These additional SNPs have a minor allele frequency of >5% in the UTAH CEPH (CEU) HapMap samples and they are neither on the Hap300 SNP chip nor are they efficiently tagged by SNPs that are on the chip [Pe'er, et al., (2006), Nat Genet, 38, 663-7].

The term "estrogen receptor positive breast cancer", or "ER positive breast cancer", as defined herein, refers to a sample of a breast cancer tissue determined to be positive for expression of estrogen receptor. The expression of estrogen receptor can for example be determined by radioimmunoassay or immunohistochemistry. A radioimmunometric measurement of > 10 fmol/mg or an immunohistochemical assessment of > 10% positive nuclei is considered to be positive.

The term "progesterone receptor positive breast cancer", or "PR positive breast cancer", as defined herein, refers to a sample of a breast cancer tissue determined to be positive for expression of progesterone receptor. The expression of progestrogen receptor can for example be determined by radioimmunoassay or immunohistochemistry. A radioimmunometric measurement of > 10 fmol/mg or an immunohistochemical assessment of > 10% positive nuclei is considered to be positive.

Through association analysis of a population of individuals diagnosed with breast cancer, it has been discovered that certain alleles at certain polymorphic markers are associated with breast cancer. A genome-wide analysis for variants associated with cancer revealed association of breast cancer to two distinct regions of chromosome 2 within the segments Chr2q14 and Chr2q35, as well as within a region on chromosome 16 (Chr16q12). Particular markers and haplotypes were found to be associated with an increased risk of breast cancer in these regions.

As indicated in Table 1, the A allele of marker rs4848543 on chromosome 2q14.2 (also called rs4848543 A allele or rs4848543 1 allele or A-rs4848543) has been found to be associated with an increased risk of breast cancer. Analysis of the MedPre breast cancer phenotype in 1598 patients in relation to 4477 cancer-free population controls, leads to an estimated Relative Risk (RR) value of 1.42, with a p-value of 8.3×10⁻⁸. Following a correction for 317,089 SNP markers tested as part of the genome-wide chip used in the experiment, the p-value is 0.026, i.e. the association is significant at the genome-wide level. The association to the broader All BC phenotype is slightly weaker, with an RR-value of 1.16 (see Table 1). These results have been replicated in an independent Icelandic Breast Cancer cohort.

The rs4848543 marker is located within a region called the rs4848543 LD block herein. This SNP marker, and markers that are correlated with the marker (e.g., markers as listed in Table 10), is useful in the methods of the present invention. Due to the local linkage disequilibrium pattern of the human genome, there are many polymorphic markers that are in strong LD with rs4848543 shown herein to be associated with cancer. These correlated markers (e.g., markers as listed in Table 10), including known SNPs or other polymorphic markers such as microsatellites or indels, as well as other correlated SNPs or other polymorphic markers, could therefore be used, alone or in combination, as surrogate markers for detecting the association to breast cancer described herein. In particular, it is expected that other polymorphic markers located within the rs4848543 LD block, may be used as surrogate markers in the methods of the invention.

A second region on Chromosome 2 (2q35) has been identified through genome-wide association analysis. Allele A of marker rs13387042 (also called rs13387042 A allele or rs13387042 1 allele or A-rs13387042) has been found to be associated with increased risk of breast cancer (see Table 11). This SNP is located at chromosome 2q35, at a distinct location from the STEAP3/TSAP6 locus. An analysis of 2,181 individuals with breast cancer (*All BC*) and 12,441 population controls reveals that the A allele of rs13387042 confers a risk of 1.19, with a p-value of 4.0×10-5. The risk (RR) in *MedPre* breast cancer was found to be comparable to that for *Any BC.* Surrogate markers that could also be used to detect the assocation to rs13387042 are listed in Table 15.

This result was replicated in a second independent sample from the Icelandic population (583 cases and 7966 controls), giving an estimate for RR for the A allele of rs13387042 in association with *AllBC* in this second group of 1.20 (P-value_{corr} = 3.8×10⁻³). Therefore the original finding was replicated significantly in an independent Icelandic sample, with a very similar point estimate of the RR, and an overall P-value_{corr} 2.0×10⁻⁷, which is close to the level of significance after Bonferroni correction for the 317,089 SNPs tested. The observed frequency and relative risk of the rs13387042 allele A variant corresponds to an estimated population attributable risk of 15.6% in the Icelandic population.

A replication study in Spanish and Swedish samples confirmed these findings. Analysis of 446 breast cancer cases and 977 control samples from Spain gave RR of 1.21 (P-value 1.8×10⁻²; Table 11). The frequency of the A-rs13387042 variant was higher in the Spanish control samples, suggesting that the variant may be more prevalent and thus contribute to a higher Breast Cancer burden in populations of Spanish extraction. Two Swedish cohorts, "Sweden Familial" and "Sweden Consecutive" were also analyzed. The "Sweden Consecutive" cohort demonstrated a significant RR for breast cancer of 1.31 (P-value= 2.0×10⁻⁴), while the "Sweden Familial" cohort gave a relative risk estimate of 1.11 that is not statistically significant. Overall, however, the combined Swedish cohorts returned a significant relative risk estimate of 1.22 (P-value= 8.1×10⁻⁴).

The estimates for the Icelandic, Spanish and Swedish cohorts were combined in a joint analysis, using the Mantel-Haenszel model. The result was an estimated relative risk of 1.20 with a P-value of 3.8×10⁻¹¹. This is well below the threshold for genome-wide significance using the Bonferroni method to correct for the 317,089 SNPs investigated. We therefore concluded that rs13387042 allele A confers a significant and reproducible risk for breast cancer in several populations samples of European descent. The overall population attributable risk was estimated to be 16.4%.

A further genome-wide SNP analysis using data from 1600 breast cancer patients and 11563 controls identified the T allele of SNP rs3803662 (T-rs3803662; rs3803662 allele T) as conferring an estimated 1.23-fold increased risk for *Any BC* (Table 16). This result was confirmed in a second, independent cohort of 594 Icelandic breast cancer patients and 1433 controls. The combined data from these two Icelandic samples gave a relative risk estimate of 1.23 and a P-value of 2.8×10⁻⁷ when corrected for relatedness between the individuals. This corresponded to an estimated population attributable risk of 10.1% (Table 16). Surrogate markers that can be used to detect the association to rs3803662 are listed in Table 19.

Replication analysis of this finding in the Swedish and Spanish cohorts described above was performed, as well as an analysis of 558 breast cancer cases and 1384 controls from Nijmegen, Holland. As shown in Table 16, a significantly increased risk for rs3803662 allele T was observed in all three of these replication cohorts. A combined analysis of these three non-Icelandic replication cohorts reveals an overall relative risk estimate of 1.35 and a P-value was 5.1×10⁻¹². A joint analysis of the Icelandic and replication cohorts indicates a combined relative risk estimate of 1.28 with a P-value of 2.7x10⁻¹⁷. This is well below the threshold for genome-wide significance when corrected for the number of SNPs tested. The corresponding overall population attributable risk estimate was 13.4% (Table 16).

The association between rs13387042 allele A and rs3803662 allele T, and estrogen receptor (ER) and progesterone receptor (PR) status was investigated. Significant breast cancer risk involving rs13387042 allele A and rs3803662 allele T was clearly confined to those patients diagnosed with ER positive tumors and the difference between the OR's for ER positive and ER negative tumors was also significant (Table 25). Similarly, there was a tendency towards breast cancer risk preferentially amongst patients diagnosed with PR positive tumors.

### Biology of STEAP3/TSAP6:

A cDNA for STEAP3/TSAP6 was first isolated from one of a series of mRNAs that were induced by p53 activation in the murine myeloid LTR6 cell line, which contains a temperature sensitive p53 gene [Amson, et al., (1996), Proc Natl Acad Sci U S A, 93, 3953-7]. This murine form was named Tumour Suppressor Activated Pathway 6 (TSAP6). The gene was later confirmed to be inducible by activated p53 in LTR6 cells [Passer, et al., (2003), Proc Natl Acad Sci U S A, 100, 2284-9]. A rat version, designated pHyde, was isolated from a prostatic cancer cell line during experiments designed to detect genes differentially expressed in two cell lines with different metastatic propensities [Rinaldy and Steiner, (1999), DNA Cell Biol, 18, 829-36]. The pHyde cDNA was shown to induce apoptosis in rat and human prostate cell lines and xenografts [Rinaldy, et al., (2000), Gan To Kagaku Ryoho, 27 Suppl 2, 215-22; Steiner, et al., (2000), Cancer Res, 60, 4419-25]. Apoptosis induction by pHyde was later shown to operate through the caspase-3 pathway [Zhang, et al., (2001), Oncogene, 20, 5982-90]. Down regulation of STEAP3/TSAP6 has also been associated with progression of hepatocellular carcinoma [Coulouarn, et al., (2005), J Hepatol, 42, 860-9].

Passer *et al.* isolated a cDNA for the human version of STEAP3/TSAP6 and showed that the mRNA was inducible by p53 activation in the breast cancer cell line MCF7 [Passer, et al., (2003), Proc Natl Acad Sci U S A, 100, 2284-9]. Induction by p53 appears to be at the transcriptional level and is mediated by a conserved p53 response element in the STEAP3/TSAP6 promoter between nucleotides -478 and -357 (sequence defined by accession number AY214461)[Passer, et al., (2003), Proc Natl Acad Sci U S A, 100, 2284-9]. The protein was identified as a 488 amino acid, 50-55kDa, 6-pass transmembrane protein [Passer, et al., (2003), Proc Natl Acad Sci U S A, 100, 2284-9]. Antisense RNA targeted on STEAP3/TSAP6 inhibited p53-dependent apoptosis, further supporting the view that STEAP3/TSAP6 acts as an effector of apoptosis [Passer, et al., (2003), Proc Natl Acad Sci U S A, 100, 2284-9]. STEAP3/TSAP6 interacts physically with two proteins involved in cell cycle regulation and apoptosis; Nix and Myt1[Passer, et al., (2003), Proc Natl Acad Sci U S A, 100, 2284-9]. Nix (also known as BNIP3L) is a mitochondrial, Bcl2-related, proapoptotic protein. Nix and STEAP3/TSAP6 accentuate each other's proapoptotic effects [Passer, et al., (2003), Proc Natl Acad Sci U S A, 100, 2284-9]. Myt1 is a dual specificity (Ser/Thr and Tyr) kinase that acts to block the cell cycle at the G2/M checkpoint by phosphorylating and thereby inhibiting the cyclin dependent kinase p34^{cdc2} . The interaction between Myt1 and TSAP6 promotes Myt1-dependent phosphorylation of p34^{cdc2} by holding Myt1 in its hypophosphorylated, active state [Passer, et al., (2003), Proc Natl Acad Sci U S A, 100, 2284-9].

Somewhat paradoxically, STEAP3/TSAP6 was recently identified as the gene responsible for the microcytic, hypochromic anemia in *nm1054* mutant stain of mice [Ohgami, et al., (2005), Nat Genet, 37, 1264-9]. Extracellular iron is carried by transferrin (Tf) from the intestine, reticuloendothelial system and liver to all proliferating cells in the body. Tf-bound iron enters the cell by transferrin-receptor (TfR1) mediated endocytosis. Iron is released from Tf in the endosome by acidification, where after it is delivered to the cytoplasm by the divalent metal transporter Dmt1. Tf and TfR1 are then recycled to the extracellular environment. Environmental and Tf-bound iron is present predominantly in the oxidized Fe³⁺ (ferric) state and must be reduced to the Fe²⁺ (ferrous) state before it can be transferred across the cytoplasmic membrane. Through its association with anemia, STEAP3/TSAP6 was identified as the major ferrireductase that carries out this reaction in erythroid lineage cells, implying that it has great importance for iron uptake [Ohgami, et al., (2005), Nat Genet, 37, 1264-9]. STEAP3/TSAP6 also as cupric reductase activity and these metalloreductase activities are shared by other members of the STEAP family STEAP2 and STEAP4 [Ohgami, et al., (2006), Blood, 108, 1388-94].

STEAP3/TSAP6 has also been shown to be involved in stimulation of secretion of proteins via a non-classical pathway. Classical protein secretion is mediated by an NH₂-terminal signal sequence on the protein destined for secretion. The signal sequence directs the protein through the endoplasmic reticulum/Golgi pathway followed by transport the plasma membrane in secretory vesicles. Upon fusion of the secretory vesicle with the plasma membrane, the secreted protein is released into the extracellular space. Protein secretion can also be carried out by a non-classical pathway in which intracellular vesicles can bud out into the lumen of endosomes forming so-called multivesicular structures. Fusion of the multivesicular structure with the plasma membrane results in the release of these membrane-encapsulated vesicles into the extracellular space. These vesicles are known as exosomes. STEAP3/TSAP6 has been shown to stimulate exosome production in a p53 dependent manner [Amzallag, et al., (2004), J Biol Chem, 279, 46104-12; Yu, et al., (2006), Cancer Res, 66, 4795-801]. Exosomes may be involved in important anti-oncogenic responses. One of the proteins secreted in exosomes in response to the p53-STEAP3/TSAP6 pathway stimulating is Maspin (mammary serine protease inhibitor) which has proven inhibitory effects on angiogenesis, tumor invasion and metastasis in mammary tumor cells [Sheng, et al., (1996), Proc Natl Acad Sci U S A, 93, 11669-74; Shi, et al., (2001), Cancer Res, 61, 6945-51; Zhang, et al., (1997), Mol Med, 3, 49-59; Zou, et al., (1994), Science, 263, 526-9]. Exosomes can contain pro-inflammatory factors such as Translationally Controlled Tumour Protein (TCTP)/Histamine Releasing Factor and tumor-specific antigens such as Her2/Neu and Mart1 [Amzallag, et al., (2004), J Biol Chem, 279, 46104-12; Andre, et al., (2002), Lancet, 360, 295-305]. It has been suggested that STEAP3/TSAP6-mediated endosome formation may be responsible for the so-called "bystander" effect wherein cells induced to express p53 can generate cell cycle arrest, proapoptotic responses and death in neighbouring cells [Yu, et al., (2006), Cancer Res, 66, 4795-801].

Almost nothing is known about the relationship between possible genetic variants of STEAP3/TSAP6 and cancer risk. A screen for mutations in prostate cancer cell lines, xenografts and tumor samples found 2 missense mutations (Ala184Thr and Ile305Thr) in xenografts out of a total of 4 cell lines, 8 xenografts and 56 tumor samples (total 68 samples). The authors concluded that STEAP3/TSAP6 is not a classical tumor suppressor gene in prostate cancer. No studies of STEAP3/TSAP6 genetic variants in any other type of cancer have been reported to our knowledge.

### Potential for utilization of the STEAP3/TSAP6 pathway in the development of improved therapies for breast cancer

Our observation that a genetic variant in the STEAP3/TSAP6 LD block implicates this gene as a common factor in the development of breast carcinoma. Therefore therapies which target STEAP3/TSAP6, its homologues and other components on the STEAP3/TSAP6 pathways can be considered as candidates for the treatment or prevention of breast cancer. Such targets include: STEAP3/TSAP6 itself (OMIM# 609671); the homologues STEAP1 (OMIM# 604415), STEAP2/TIARP/STAMP1 (OMIM# 605094), and STEAP4 (NM_024636); interacting proteins Nix/BNIP3L (OMIM# 605368), Myt1 (OMIM# 602474), TCTP/Histamine Releasing Factor (OMIM# 600763); pathway proteins p34^{cdc2} (OMIM# 116940), cyclin B1 (OMIM# 123836), HER2/Neu (OMIM# 164870), Maspin (OMIM# 154790); and other members of the STEP3/TSAP6 pathway yet to be identified or implicated. Therapies could include small molecule agonists or antagonists of STEAP3/TSAP6, its homologues or pathway components; macromolecular agents as agonists or antagonists of STEAP3/TSAP6, its homologues or pathway components; or the use of STEAP3/TSAP6, homologues or pathway components as transgenes in gene therapy or immunotherapy protocols. STEAP3/TSAP6, its homologues or pathway components and/or molecules that affect their activities could also be developed as adjuvants for cytostatic or cytotoxic therapies (chemotherapy, radiotherapy, gene or immunotherapy) through their potentiation of the "bystander effect".

### Assessment for markers and haplotypes

The genomic sequence within populations is not identical when individuals are compared. Rather, the genome exhibits sequence variability between individuals at many locations in the genome. Such variations in sequence are commonly referred to as polymorphisms, and there are many such sites within each genome For example, the human genome exhibits sequence variations which occur on average every 500 base pairs. The most common sequence variant consists of base variations at a single base position in the genome, and such sequence variants, or polymorphisms, are commonly called Single Nucelotide Polymorphisms ("SNPs"). These SNPs are believed to have occurred in a single mutational event, and therefore there are usually two possible alleles possible at each SNPsite; the original allele and the mutated allele. Due to natural genetic drift and possibly also selective pressure, the original mutation has resulted in a polymorphism characterized by a particular frequency of its alleles in any given population. Many other types of sequence variants are found in the human genome, including microsatellites, insertions, deletions, inversions and copy number variations. A polymorphic microsatellite has multiple small repeats of bases (such as CA repeats, TG on the complimentary strand) at a particular site in which the number of repeat lengths varies in the general population. In general terms, each version of the sequence with respect to the polymorphic site represents a specific allele of the polymorphic site. All sequence variants can be referred to as polymorphisms, occurring at specific polymorphic sites characteristic of the sequence variant in question. In general terms, polymorphisms can comprise any number of specific alleles. Thus in one embodiment of the invention, the polymorphism is characterized by the presence of two or more alleles in any given population. In another embodiment, the polymorphism is characterized by the presence of three or more alleles. In other embodiments, the polymorphism is characterized by four or more alleles, five or more alleles, six or more alleles, seven or more alleles, nine or more alleles, or ten or more alleles. All such polymorphisms can be utilized in the methods and kits of the present invention, and are thus within the scope of the invention.

In some instances, reference is made to different alleles at a polymorphic site without choosing a reference allele. Alternatively, a reference sequence can be referred to for a particular polymorphic site. The reference allele is sometimes referred to as the "wild-type" allele and it usually is chosen as either the first sequenced allele or as the allele from a "non-affected" individual (e.g., an individual that does not display a trait or disease phenotype).

Alleles for SNP markers as referred to herein refer to the bases A, C, G or T as they occur at the polymorphic site in the SNP assay employed. The allele codes for SNPs used herein are as follows: 1=A, 2=C, 3=G, 4=T. The person skilled in the art will however realize that by assaying or reading the opposite DNA strand, the complementary allele can in each case be measured. Thus, for a polymorphic site (polymorphic marker) characterized by an A/G polymorphism, the assay employed may be designed to specifically detect the presence of one or both of the two bases possible, i.e. A and G. Alternatively, by designing an assay that is designed to detect the opposite strand on the DNA template, the presence of the complementary bases T and C can be measured. Quantitatively (for example, in terms of relative risk), identical results would be obtained from measurement of either DNA strand (+ strand or - strand).

Typically, a reference sequence is referred to for a particular sequence. Alleles that differ from the reference are sometimes referred to as "variant" alleles. A variant sequence, as used herein, refers to a sequence that differs from the reference sequence but is otherwise substantially similar. Alleles at the polymorphic genetic markers described herein are variants. Additional variants can include changes that affect a polypeptide. Sequence differences, when compared to a reference nucleotide sequence, can include the insertion or deletion of a single nucleotide, or of more than one nucleotide, resulting in a frame shift; the change of at least one nucleotide, resulting in a change in the encoded amino acid; the change of at least one nucleotide, resulting in the generation of a premature stop codon; the deletion of several nucleotides, resulting in a deletion of one or more amino acids encoded by the nucleotides; the insertion of one or several nucleotides, such as by unequal recombination or gene conversion, resulting in an interruption of the coding sequence of a reading frame; duplication of all or a part of a sequence; transposition; or a rearrangement of a nucleotide sequence,. Such sequence changes can alter the polypeptide encoded by the nucleic acid. For example, if the change in the nucleic acid sequence causes a frame shift, the frame shift can result in a change in the encoded amino acids, and/or can result in the generation of a premature stop codon, causing generation of a truncated polypeptide. Alternatively, a polymorphism associated with a disease or trait can be a synonymous change in one or more nucleotides (*i.e*., a change that does not result in a change in the amino acid sequence). Such a polymorphism can, for example, alter splice sites, affect the stability or transport of mRNA, or otherwise affect the transcription or translation of an encoded polypeptide. It can also alter DNA to increase the possibility that structural changes, such as amplifications or deletions, occur at the somatic level. The polypeptide encoded by the reference nucleotide sequence is the "reference" polypeptide with a particular reference amino acid sequence, and polypeptides encoded by variant alleles are referred to as "variant" polypeptides with variant amino acid sequences.

A haplotype refers to a segment of DNA that is characterized by a specific combination of alleles arranged along the segment. For diploid organisms such as humans, a haplotype comprises one member of the pair of alleles for each polymorphic marker or locus. In a certain embodiment, the haplotype can comprise two or more alleles, three or more alleles, four or more alleles, or five or more alleles, each allele corresponding to a specific polymorphic marker along the segment. Haplotypes can comprise a combination of various polymorphic markers, e.g., SNPs and microsatellites, having particular alleles at the polymorphic sites. The haplotypes thus comprise a combination of alleles at various genetic markers.

Detecting specific polymorphic markers and/or haplotypes can be accomplished by methods known in the art for detecting sequences at polymorphic sites. For example, standard techniques for genotyping for the presence of SNPs and/or microsatellite markers can be used, such as fluorescence-based techniques (Chen, X. et al., Genome Res. 9(5): 492-98 (1999)), utilizing PCR, LCR, Nested PCR and other techniques for nucleic acid amplification. Specific methodologies available for SNP genotyping include, but are not limited to, TaqMan genotyping assays and SNPlex platforms (Applied Biosystems), mass spectrometry (e.g., MassARRAY system from Sequenom), minisequencing methods, real-time PCR, Bio-Plex system (BioRad), CEQ and SNPstream systems (Beckman), Molecular Inversion Probe array technology (e.g., Affymetrix GeneChip), and BeadArray Technologies (e.g., Illumina GoldenGate and Infinium assays). By these or other methods available to the person skilled in the art, one or more alleles at polymorphic markers, including microsatellites, SNPs or other types of polymorphic markers, can be identified.

In certain methods described herein, an individual who is at an increased susceptibility (i.e., increased risk) for any specific disease or trait under study (e.g., breast cancer), is an individual in whom at least one specific allele at one or more polymorphic marker or haplotype conferring increased susceptibility for the disease or trait is identified (i.e., at-risk marker alleles or haplotypes). In one aspect, the at-risk marker or haplotype is one that confers a significant increased risk (or susceptibility) of breast cancer. In one embodiment, significance associated with a marker or haplotype is measured by a relative risk (RR). In another embodiment, significance associated with a marker or haplotye is measured by an odds ratio (OR). In a further embodiment, the significance is measured by a percentage. In one embodiment, a significant increased risk is measured as a risk (relative risk and/or odds ratio) of at least 1.2, including but not limited to: at least 1.2, at least 1.3, at least 1.4, at least 1.5, at least 1.6, at least 1.7, at least 1.8, at least 1.9, at least 2.0, at least 2.5, at least 3.0, at least 4.0, and at least 5.0. In a particular embodiment, a risk (relative risk and/or odds ratio)of at least 1.2 is significant. In another particular embodiment, a risk of at least 1.3 is significant. In yet another embodiment, a risk of at least 1.4 is significant. In a further embodiment, a relative risk of at least about 1.5 is significant. In another further embodiment, a significant increase in risk is at least about 1.7 is significant. However, other cutoffs are also contemplated, e.g. at least 1.15, 1.25, 1.35, and so on, and such cutoffs are also within scope of the present invention. In other embodiments, a significant increase in risk is at least about 20%, including but not limited to about 25%, 30%, 35%, 40%, 45%, 50%, 55%, 60%, 65%, 70%, 75%, 80%, 85%, 90%, 95%, 100%, 150%, 200%, 300%, and 500%. In one particular embodiment, a significant increase in risk is at least 20%. In other embodiments, a significant increase in risk is at least 30%, at least 40%, at least 50%, at least 60%, at least 70%, at least 80%, at least 90% and at least 100%. Other cutoffs or ranges as deemed suitable by the person skilled in the art to characterize the invention are however also contemplated, and those are also within scope of the present invention. In certain embodiments, a significant increase in risk is characterized by a p-value, such as a p-value of less than 0.05, less than 0.01, less than 0.001, less than 0.0001, less than 0.00001, less than 0.000001, less than 0.0000001, less than 0.00000001, or less than 0.000000001.

An at-risk polymorphic marker or haplotype of the present invention is one where at least one allele of at least one marker or haplotype is more frequently present in an individual at risk for breast cancer (affected), compared to the frequency of its presence in a comparison group (control), and wherein the presence of the marker or haplotype is indicative of susceptibility to the disease or trait. The control group may in one embodiment be a population sample, i.e. a random sample from the general population. In another embodiment, the control group is represented by a group of individuals who are disease-free, e.g., indidividuals who have not been diagnosed with breast cancer. Such disease-free control may in one embodiment be characterized by the absence of one or more specific disesase-associated symptoms. In another embodiment, the disease-free control group is characterized by the absence of one or more disease-specific risk factors. Such risk factors are in one embodiment at least one environmental risk factor. Representative environmental factors are natural products, minerals or other chemicals which are known to affect, or contemplated to affect, the risk of developing the specific disease or trait. Other environmental risk factors are risk factors related to lifestyle, including but not limited to food and drink habits, geographical location of main habitat, and occupational risk factors. In another embodiment, the risk factors are at least one genetic risk factor.

As an example of a simple test for correlation would be a Fisher-exact test on a two by two table. Given a cohort of chromosomes, the two by two table is constructed out of the number of chromosomes that include both of the markers or haplotypes, one of the markers or haplotypes but not the other and neither of the markers or haplotypes. Other statistical tests of association known to the skilled person are also contemplated and are also within scope of the invention.

In other embodiments of the invention, an individual who is at a decreased susceptibility (i.e., at a decreased risk) for a disease or trait is an individual in whom at least one specific allele at one or more polymorphic marker or haplotype conferring decreased susceptibility for the disease or trait is identified. The marker alleles and/or haplotypes conferring decreased risk are also said to be protective. In one aspect, the protective marker or haplotype is one that confers a significant decreased risk (or susceptibility) of the disease or trait. In one embodiment, significant decreased risk is measured as a relative risk of less than 0.9, including but not limited to less than 0.9, less than 0.8, less than 0.7, less than 0.6, less than 0.5, less than 0.4, less than 0.3, less than 0.2 and less than 0.1. In one particular embodiment, significant decreased risk is less than 0.7. In another embodiment, significant decreased risk is less than 0.5. In yet another embodiment, significant decreased risk is less than 0.3. In another embodiment, the decrease in risk (or susceptibility) is at least 20%, including but not limited to at least 25%, at least 30%, at least 35%, at least 40%, at least 45%, at least 50%, at least 55%, at least 60%, at least 65%, at least 70%, at least 75%, at least 80%, at least 85%, at least 90%, at least 95% and at least 98%. In one particular embodiment, a significant decrease in risk is at least about 30%. In another embodiment, a significant decrease in risk at least about 50%. In another embodiment, the decrease in risk is at least about 70%. Other cutoffs or ranges as deemed suitable by the person skilled in the art to characterize the invention are however also contemplated, and those are also within scope of the present invention.

The person skilled in the art will appreciate that for markers with two alleles present in the population being studied, and wherein one allele is found in increased frequency in a group of individuals with a trait or disease in the population, compared with controls, the other allele of the marker will be found in decreased frequency in the group of individuals with the trait or disease, compared with controls. In such a case, one allele of the marker (the one found in increased frequency in individuals with the trait or disease) will be the at-risk allele, while the other allele will be a protective allele.

A genetic variant associated with a disease or a trait (e.g. breast cancer) can be used alone to predict the risk of the disease for a given genotype. For a biallelic marker, such as a SNP, there are 3 possible genotypes: homozygote for the at risk variant, heterozygote, and non carrier of the at risk variant. Risk associated with variants at multiple loci can be used to estimate overall risk. For multiple SNP variants, there are k possible genotypes *k* = 3ⁿ × 2*^{P}*; where n is the number autosomal loci and p the number of gonosomal (sex chromosomal) loci. Overall risk assessment calculations usually assume that the relative risks of different genetic variants multiply, i.e. the overall risk (e.g., RR or OR) associated with a particular genotype combination is the product of the risk values for the genotype at each locus. If the risk presented is the relative risk for a person, or a specific genotype for a person, compared to a reference population with matched gender and ethnicity, then the combined risk - is the product of the locus specific risk values - and which also corresponds to an overall risk estimate compared with the population. If the risk for a person is based on a comparison to non-carriers of the at risk allele, then the combined risk corresponds to an estimate that compares the person with a given combination of genotypes at all loci to a group of individuals who do not carry risk variants at any of those loci. The group of non-carriers of any at risk variant has the lowest estimated risk and has a combined risk, compared with itself (i.e., non-carriers) of 1.0, but has an overall risk, compare with the population, of less than 1.0. It should be noted that the group of non-carriers can potentially be very small, especially for large number of loci, and in that case, its relevance is correspondingly small.

The multiplicative model is a parsimonious model that usually fits the data of complex traits reasonably well. Deviations from multiplicity have been rarely described in the context of common variants for common diseases, and if reported are usually only suggestive since very large sample sizes are usually required to be able to demonstrate statistical interactions between loci.

By way of an example, let us consider a total of eight variants that have been described to associate with prostate cancer (Gudmundsson, J., et al., Nat Genet 39:631-7 (2007), Gudmundsson, J., et al., Nat Genet 39:977-83 (2007); Yeager, M., et al, Nat Genet 39:645-49 (2007), Amundadottir, L., el al., Nat Genet 38:652-8 (2006); Haiman, C.A., et al., Nat Genet 39:638-44 (2007)). Seven of these loci are on autosomes, and the remaining locus is on chromosome X. The total number of theoretical genotypic combinations is then 3⁷ × 2¹ = 4374. Some of those genotypic classes are very rare, but are still possible, and should be considered for overall risk assessment. It is likely that the multiplicative model applied in the case of multiple genetic variant will also be valid in conjugation with non-genetic risk variants assuming that the genetic variant does not clearly correlate with the "environmental" factor. In other words, genetic and non-genetic at-risk variants can be assessed under the multiplicative model to estimate combined risk, assuming that the non-genetic and genetic risk factors do not interact.

Using the same quantitative approach, the combined or overall risk associated with a plurality of variants associated with breast cancer may be assessed. In one such embodiment, the markers rs4848543, rs13387042 and rs3803662, or surrogate markers in linkage disequilibrium with these markers, are assessed and the risk for the genotype combinations (3³ = 27 possible combinations) calculated. In another embodiment, genotypes for two of the markers (*e.g.*, markers rs13387042 and rs3803662) are combined to give overall risk. In other embodiments, additional markers known to predispose to breast cancer (e.g., high penetrant risk factors, such as BRCA1, BRCA2, BARD1) are combined with one or more of the risk factors described herein.

### Linkage Disequilibrium

The natural phenomenon of recombination, which occurs on average once for each chromosomal pair during each meiotic event, represents one way in which nature provides variations in sequence (and biological function by consequence). It has been discovered that recombination does not occur randombly in the genome; rather, there are large variations in the frequency of recombination rates, resulting in small regions of high recombination frequency (also called recombination hotspots) and larger regions of low recombination frequency, which are commonly referred to as Linkage Disequilibrium (LD) blocks (Myers, S. et al., Biochem Soc Trans 34:526-530 (2006); Jeffreys, A.J., et al.,Nature Genet 29:217-222 (2001); May, C.A., et al., Nature Genet 31:272-275(2002)).

Linkage Disequilibrium (LD) refers to a non-random assortment of two genetic elements. For example, if a particular genetic element *(e.g.,* "alleles" of a polymorphic marker) occurs in a population at a frequency of 0.50 (50%) and another occurs at a frequency of 0.50 (50%), then the predicted occurrance of a person's having both elements is 0.25 (25%), assuming a random distribution of the elements. However, if it is discovered that the two elements occur together at a frequency higher than 0.25, then the elements are said to be in linkage disequilibrium since they tend to be inherited together at a higher rate than what their independent allele frequencies of occurrence (*e.g.*, allele or haplotype frequencies) would predict. Roughly speaking, LD is generally correlated with the frequency of recombination events between the two elements. Allele or haplotype frequencies can be determined in a population by genotyping individuals in a population and determining the occurence of each allele or haplotype in the population. For populations of diploids, *e.g.,* human populations, individuals will typically have two alleles for each genetic element (*e.g.*, a marker, haplotype or gene).

Many different measures have been proposed for assessing the strength of linkage disequilibrium (LD). Most capture the strength of association between pairs of biallelic sites. Two important pairwise measures of LD are r² (sometimes denoted Δ²) and |D'|. Both measures range from 0 (no disequilibrium) to 1 ('complete' disequilibrium), but their interpretation is slightly different. |D'| is defined in such a way that it is equal to 1 if just two or three of the possible haplotypes are present, and it is <1 if all four possible haplotypes are present. Therefore, a value of |D'| that is <1 indicates that historical recombination may have occurred between two sites (recurrent mutation can also cause |D'| to be <1, but for single nucleotide polymorphisms (SNPs) this is usually regarded as being less likely than recombination). The measure r² represents the statistical correlation between two sites, and takes the value of 1 if only two haplotypes are present.

The r² measure is arguably the most relevant measure for association mapping, because there is a simple inverse relationship between r² and the sample size required to detect association between susceptibility loci and SNPs. These measures are defined for pairs of sites, but for some applications a determination of how strong LD is across an entire region that contains many polymorphic sites might be desirable (e.g., testing whether the strength of LD differs significantly among loci or across populations, or whether there is more or less LD in a region than predicted under a particular model). Measuring LD across a region is not straightforward, but one approach is to use the measure r, which was developed in population genetics. Roughly speaking, r measures how much recombination would be required under a particular population model to generate the LD that is seen in the data. This type of method can potentially also provide a statistically rigorous approach to the problem of determining whether LD data provide evidence for the presence of recombination hotspots. For the methods described herein, a significant r² value can be at least 0.1 such as at least 0.1, 0.15, 0.2, 0.25, 0.3, 0.35, 0.4, 0.45, 0.5, 0.55, 0.6, 0.65, 0.7, 0.75, 0.8, 0.85, 0.9, 0.91, 0.92, 0.93, 0.94, 0.95, 0.96, 0.97, 0.98, 0.99 or 1.0. In one preferred embodiment, the significant r² value can be at least 0.2. Alternatively, linkage disequilibrium as described herein, refers to linkage disequilibrium characterized by values of |D'| of at least 0.2, such as 0.3, 0.4, 0.5, 0.6, 0.7, 0.8, 0.85, 0.9, 0.95, 0.96, 0.97, 0.98, 0.99. Thus, linkage disequilibrium represents a correlation between alleles of distinct markers. It is measured by correlation coefficient or |D'| (r² up to 1.0 and |D'| up to 1.0). In certain embodiments, linkage disequilibrium is defined in terms of values for both the r² and |D'| measures. In one such embodiment, a significant linkage disequilibrium is defined as r² > 0.1 and/or |D'| >0.8. In another embodiment, a significant linkage disequilibrium is defined as r² > 0.2 and/or |D'| >0.8. In another embodiment, a significant linkage disequilibrium is defined as r² > 0.2 and/or |D'| >0.9. Other combinations and permutations of values of r² and |D'| for determining linkage disequilibrium are also contemplated, and are also within the scope of the invention. Linkage disequilibrium can be determined in a single human population, as defined herein, or it can be determined in a collection of samples comprising individuals from more than one human population. In one embodiment of the invention, LD is determined in a sample from one or more of the HapMap populations (caucasian, african, japanese, chinese), as defined (http://www.hapmap.org). In one such embodiment, LD is determined in the CEU population of the HapMap samples. In another embodiment, LD is determined in the YRI population. In yet another embodiment, LD is determined in samples from the Icelandic population.

If all polymorphisms in the genome were identical at the population level, then every single one of them would need to be investigated in association studies. However, due to linkage disequilibrium between polymorphisms, tightly linked polymorphisms are strongly correlated, which reduces the number of polymorphisms that need to be investigated in an association study to observe a significant association. Another consequence of LD is that many polymorphisms may give an association signal due to the fact that these polymorphisms are strongly correlated.

Genomic LD maps have been generated across the genome, and such LD maps have been proposed to serve as framework for mapping disease-genes (Risch, N. & Merkiangas, K, Science 273:1516-1517 (1996); Maniatis, N., et al., Proc Natl Acad Sci USA 99:2228-2233 (2002); Reich, DE et al, Nature 411:199-204 (2001)).

It is now established that many portions of the human genome can be broken into series of discrete haplotype blocks containing a few common haplotypes; for these blocks, linkage disequilibrium data provides little evidence indicating recombination (see, *e.g.*, Wall., J.D. and Pritchard, J.K., Nature Reviews Genetics 4:587-597 (2003); Daly, M. et al., Nature Genet. 29:229-232 (2001); Gabriel, S.B. et al., Science 296:2225-2229 (2002); Patil, N. et al., Science 294:1719-1723 (2001); Dawson, E. et al., Nature 418:544-548 (2002); Phillips, M.S. et al., Nature Genet. 33:382-387 (2003)).

There are two main methods for defining these haplotype blocks: blocks can be defined as regions of DNA that have limited haplotype diversity (see, *e.g.*, Daly, M. et al., Nature Genet. 29:229-232 (2001); Patil, N. et al., Science 294:1719-1723 (2001); Dawson, E. et al., Nature 418:544-548 (2002); Zhang, K. et al., Proc. Natl. Acad. Sci. USA 99:7335-7339 (2002)), or as regions between transition zones having extensive historical recombination, identified using linkage disequilibrium (see, *e.g.,* Gabriel, S.B. et al., Science 296:2225-2229 (2002); Phillips, M.S. et al., Nature Genet. 33:382-387 (2003); Wang, N. et al., Am. J. Hum. Genet. 71:1227-1234 (2002); Stumpf, M.P., and Goldstein, D.B., Curr. Biol. 13:1-8 (2003)). More recently, a fine-scale map of recombination rates and corresponding hotspots across the human genome has been generated (Myers, S., et al., Science 310:321-32324 (2005); Myers, S. et al., Biochem Soc Trans 34:526530 (2006)). The map reveals the enormous variation in recombination across the genome, with recombination rates as high as 10-60 cM/Mb in hotspots, while closer to 0 in intervening regions, which thus represent regions of limited haplotype diversity and high LD. The map can therefore be used to define haplotype blocks/LD blocks as regions flanked by recombination hotspots. As used herein, the terms "haplotype block" or "LD block" includes blocks defined by any of the above described characteristics, or other alternative methods used by the person skilled in the art to define such regions.

Some representative methods for identification of haplotype blocks are set forth, for example, in U.S. Published Patent Application Nos. 20030099964, 20030170665, 20040023237 and 20040146870. Haplotype blocks can be used to map associations between phenotype and haplotype status, using single markers or haplotypes comprising a plurality of markers. The main haplotypes can be identified in each haplotype block, and then a set of "tagging" SNPs or markers (the smallest set of SNPs or markers needed to distinguish among the haplotypes) can then be identified. These tagging SNPs or markers can then be used in assessment of samples from groups of individuals, in order to identify association between phenotype and haplotype. If desired, neighboring haplotype blocks can be assessed concurrently, as there may also exist linkage disequilibrium among the haplotype blocks.

It is well known that linkage disequilibrium may vary between populations, due to differential historical rates of assortment in the various populations. In certain embodiments of the present invention, LD refers to LD as determined in Caucasian samples. In a particular embodiment, LD is determined in Caucasian CEPH samples from the HapMap consortium (as described on http://www.hapmap.org). In other embodiments, LD is determined in African populations, African-American populations, Hispanic populations, Japanese populations, Chinese populations. In certain embodiments, LD is determined in the HapMap samples from China, Japan, Africa, as described (http://www.hapmap.org).

It has thus become apparent that for any given observed association to a polymorphic marker in the genome, it is likely that additional markers in the genome also show association. This is a natural consequence of the uneven distribution of LD across the genome, as observed by the large variation in recombination rates. The markers used to detect association thus in a sense represent "tags" for a genomic region (i.e., a haplotype block or LD block) that is associating with a given disease or trait. One or more causative (functional) variants or mutations may reside within the region found to be associating to the disease or trait. Such variants may confer a higher relative risk (RR) or odds ratio (OR) than observed for the tagging markers used to detect the association. The present invention thus refers to the markers used for detecting association to the disease, as described herein, as well as markers in linkage disequilibrium with the markers. Thus, in certain embodiments of the invention, markers that are in LD with the markers and/or haplotypes of the invention, as described herein, may be used as surrogate markers. The surrogate markers have in one embodiment relative risk (RR) and/or odds ratio (OR) values smaller than for the markers or haplotypes initially found to be associating with the disease, as described herein. In other embodiments, the surrogate markers have RR or OR values greater than those initially determined for the markers initially found to be associating with the disease, as described herein. An example of such an embodiment would be a rare, or relatively rare (< 10% allelic population frequency) variant in LD with a more common variant (> 10% population frequency) initially found to be associating with the disease, such as the variants described herein. Identifying and using such markers for detecting the association discovered by the inventors as described herein can be performed by routine methods well known to the person skilled in the art, and are therefore within the scope of the present invention.

### Determination of haplotype frequency

The frequencies of haplotypes in patient and control groups can be estimated using an expectation-maximization algorithm (Dempster A. et al., J. R. Stat. Soc. B, 39:1-38 (1977)). An implementation of this algorithm that can handle missing genotypes and uncertainty with the phase can be used. Under the null hypothesis, the patients and the controls are assumed to have identical frequencies. Using a likelihood approach, an alternative hypothesis is tested, where a candidate at-risk-haplotype, which can include the markers described herein, is allowed to have a higher frequency in patients than controls, while the ratios of the frequencies of other haplotypes are assumed to be the same in both groups. Likelihoods are maximized separately under both hypotheses and a corresponding 1-df likelihood ratio statistic is used to evaluate the statistical significance.

To look for at-risk and protective markers and haplotypes within a linkage region, for example, association of all possible combinations of genotyped markers is studied, provided those markers span a practical region. The combined patient and control groups can be randomly divided into two sets, equal in size to the original group of patients and controls. The marker and haplotype analysis is then repeated and the most significant p-value registered is determined. This randomization scheme can be repeated, for example, over 100 times to construct an empirical distribution of p-values. In a preferred embodiment, a p-value of <0.05 is indicative of an significant marker and/or haplotype association.

### Haplotype analysis

One general approach to haplotype analysis involves using likelihood-based inference applied to NEsted MOdels (Gretarsdottir S., et al., Nat. Genet. 35:131-38 (2003)). The method is implemented in the program NEMO, which allows for many polymorphic markers, SNPs and microsatellites. The method and software are specifically designed for case-control studies where the purpose is to identify haplotype groups that confer different risks. It is also a tool for studying LD structures. In NEMO, maximum likelihood estimates, likelihood ratios and p-values are calculated directly, with the aid of the EM algorithm, for the observed data treating it as a missing-data problem.

Even though likelihood ratio tests based on likelihoods computed directly for the observed data, which have captured the information loss due to uncertainty in phase and missing genotypes, can be relied on to give valid p-values, it would still be of interest to know how much information had been lost due to the information being incomplete. The information measure for haplotype analysis is described in Nicolae and Kong (Technical Report 537, Department of Statistics, University of Statistics, University of Chicago; Biometrics, 60(2):368-75 (2004)) as a natural extension of information measures defined for linkage analysis, and is implemented in NEMO.

For single marker association to a disease, the Fisher exact test can be used to calculate two-sided p-values for each individual allele. Usually, all p-values are presented unadjusted for multiple comparisons unless specifically indicated. The presented frequencies (for microsatellites, SNPs and haplotypes) are allelic frequencies as opposed to carrier frequencies. To minimize any bias due the relatedness of the patients who were recruited as families for the linkage analysis, first and second-degree relatives can be eliminated from the patient list. Furthermore, the test can be repeated for association correcting for any remaining relatedness among the patients, by extending a variance adjustment procedure described in Risch, N. & Teng, J. (Genome Res., 8:1273-1288 (1998)), DNA pooling (*ibid*) for sibships so that it can be applied to general familial relationships, and present both adjusted and unadjusted p-values for comparison. The differences are in general very small as expected. To assess the significance of single-marker association corrected for multiple testing we can carry out a randomization test using the same genotype data. Cohorts of patients and controls can be randomized and the association analysis redone multiple times (e.g., up to 500,000 times) and the p-value is the fraction of replications that produced a p-value for some marker allele that is lower than or equal to the p-value we observed using the original patient and control cohorts.

For both single-marker and haplotype analyses, relative risk (RR) and the population attributable risk (PAR) can be calculated assuming a multiplicative model (haplotype relative risk model) (Terwilliger, J.D. & Ott, J., Hum. Hered. 42:337-46 (1992) and Falk, C.T. & Rubinstein, P, Ann. Hum. Genet. 51 (Pt 3):227-33 (1987)), i.e., that the risks of the two alleles/haplotypes a person carries multiply. For example, if RR is the risk of A relative to a, then the risk of a person homozygote AA will be RR times that of a heterozygote Aa and RR² times that of a homozygote aa. The multiplicative model has a nice property that simplifies analysis and computations — haplotypes are independent, i.e., in Hardy-Weinberg equilibrium, within the affected population as well as within the control population. As a consequence, haplotype counts of the affecteds and controls each have multinomial distributions, but with different haplotype frequencies under the alternative hypothesis. Specifically, for two haplotypes, *hᵢ* and *hⱼ*, risk(*hᵢ*)/risk(*hⱼ*) = (*fᵢ*/*pᵢ*)/(*fⱼ*/*pⱼ*)*,* where f and p denote, respectively, frequencies in the affected population and in the control population. While there is some power loss if the true model is not multiplicative, the loss tends to be mild except for extreme cases. Most importantly, p-values are always valid since they are computed with respect to null hypothesis.

### Linkage Disequilibrium using NEMO

LD between pairs of markers can be calculated using the standard definition of D' and r² (Lewontin, R., Genetics 49:49-67 (1964); Hill, W.G. & Robertson, A. Theor. Appl. Genet. 22:226-231 (1968)). Using NEMO, frequencies of the two marker allele combinations are estimated by maximum likelihood and deviation from linkage equilibrium is evaluated by a likelihood ratio test. The definitions of D' and r² are extended to include microsatellites by averaging over the values for all possible allele combination of the two markers weighted by the marginal allele probabilities. When plotting all marker combination to elucidate the LD structure in a particular region, we plot D' in the upper left corner and the p-value in the lower right corner. In the LD plots the markers can be plotted equidistant rather than according to their physical location, if desired.

### Risk assessment and diagnostics

Within any given population, there is an absolute risk of developing a disease or trait, defined as the chance of a person developing the specific disease or trait over a specified time-period. For example, a woman's lifetime absolute risk of breast cancer is one in nine. That is to say, one woman in every nine will develop breast cancer at some point in their lives. Risk is typically measured by looking at very large numbers of people, rather than at a particular individual. Risk is often presented in terms of Absolute Risk (AR) and Relative Risk (RR). Relative Risk is used to compare risks associating with two variants or the risks of two different groups of people. For example, it can be used to compare a group of people with a certain genotype with another group having a different genotype. For a disease, a relative risk of 2 means that one group has twice the chance of developing a disease as the other group. The Risk presented is usually the relative risk for a person, or a specific genotype of a person, compared to the population with matched gender and ethnicity. Risks of two individuals of the same gender and ethnicity could be compared in a simple manner. For example, if, compared to the population, the first individual has relative risk 1.5 and the second has relative risk 0.5, then the risk of the first individual compared to the second individual is 1.5/0.5 = 3.

As described herein, certain polymorphic markers and haplotypes comprising such markers are found to be useful for risk assessment of breast cancer. Risk assessment can involve the use of the markers for diagnosing a susceptibility to breast cancer. Particular alleles of polymorphic markers are found more frequently in individuals with breast cancer, than in individuals without diagnosis of breast cancer. Therefore, these marker alleles have predictive value for detecting breast cancer, or a susceptibility to breast cancer, in an individual. Tagging markers within haplotype blocks or LD blocks comprising at-risk markers, such as the markers of the present invention, can be used as surrogates for other markers and/or haplotypes within the haplotype block or LD block. Markers with values of *r*² equal to 1 are perfect surrogates for the at-risk variants, i.e. genotypes for one marker perfectly predicts genotypes for the other. Markers with smaller values of *r*² than 1 can also be surrogates for the at-risk variant, or alternatively represent variants with relative risk values as high or possibly even higher than the at-risk variant. The at-risk variant identified may not be the functional variant itself, but is in this instance in linkage disequilibrium with the true functional variant. The present invention encompasses the assessment of such surrogate markers for the markers as disclosed herein. Such markers are annotated, mapped and listed in public databases, as well known to the skilled person, or can alternatively be readily identified by sequencing the region or a part of the region identified by the markers of the present invention in a group of individuals, and identify polymorphisms in the resulting group of sequences. As a consequence, the person skilled in the art can readily and without undue experimentation genotype surrogate markers in linkage disequilibrium with the markers and/or haplotypes as described herein. The tagging or surrogate markers in LD with the at-risk variants detected, within the haplotype or LD block, also have predictive value for detecting association to breast cancer, or a susceptibility to breast cancer, in an individual. These tagging or surrogate markers that are in LD with the markers of the present invention can also include other markers that distinguish among haplotypes, as these similarly have predictive value for detecting susceptibility to breast cancer.

The present invention can in certain embodiments be practiced by assessing a sample comprising genomic DNA from an individual for the presence of variants described herein to be associated with breast cancer. Such assessment includes steps of detecting the presence or absence of at least one allele of at least one polymorphic marker, using methods well known to the skilled person and further described herein, and based on the outcome of such assessment, determine whether the individual from whom the sample is derived is at increased or decreased risk (increased or decreased susceptibility) of breast cancer. Alternatively, the invention can be practiced utilizing a dataset comprising information about the genotype status of at least one polymorphic marker described herein to be associated with breast cancer (or markers in linkage disequilibrium with at least one marker shown herein to be associated with breast cancer). In other words, a dataset containing information about such genetic status, for example in the form of genotype counts at a certain polymorphic marker, or a plurality of markers (e.g., an indication of the presence or absence of certain at-risk alleles), or actual genotypes for one or more markers, can be queried for the presence or absence of certain at-risk alleles at certain polymorphic markers shown by the present inventors to be associated with breast cancer. A positive result for a variant (*e.g.*, marker allele) associated with breast cancer, as shown herein, is indicative of the individual from which the dataset is derived is at increased susceptibility (increased risk) of breast cancer.

In certain embodiments of the invention, a polymorphic marker is correlated to breast cancer by referencing genotype data for the polymorphic marker to a look-up table that comprises correlations between at least one allele of the polymorphism and breast cancer. In some embodiments, the table comprises a correlation for one polymorhpism. In other embodiments, the table comprises a correlation for a plurality of polymorhpisms. In both scenarios, by referencing to a look-up table that gives an indication of a correlation between a marker and breast cancer, a risk for breast cancer, or a susceptibility to breast cancer, can be identified in the individual from whom the sample is derived. In some embodiments, the correlation is reported as a statistical measure. The statistical measure may be reported as a risk measure, such as a relative risk (RR), an absolute risk (AR) or an odds ratio (OR).

The markers and haplotypes of the invention, e.g., the polymorphic markers and haplotypes on chromosome 2q14.2, chromosome 2q35, and chromosome 16q12, *e.g.*, the markers presented in Table 10, Table 15, Table 19, Table 20, Table 21 and Table 22, and markers in linkage disequilibrium therewith, , e.g., markers rs4848543, rs13387042 and rs3803662, may be useful for risk assessment and diagnostic purposes for, either alone or in combination. Thus, even in cases where the increase in risk by individual markers is relatively modest, i.e. on the order of 10-30%, the association may have significant implications. Thus, relatively common variants may have significant contribution to the overall risk (Population Attributable Risk is high), or combination of markers can be used to define groups of individual who, based on the combined risk of the markers, is at significant combined risk of developing the disease.

Thus, in one embodiment of the invention, a plurality of variants (markers and/or haplotypes) is used for overall risk assessment. These variants are in one embodiment selected from the variants as disclosed herein. Other embodiments include the use of the variants of the present invention in combination with other variants known to be useful for diagnosing a susceptibility to breast cancer. In such embodiments, the genotype status of a plurality of markers and/or haplotypes is determined in an individual, and the status of the individual compared with the population frequency of the associated variants, or the frequency of the variants in clinically healthy subjects, such as age-matched and sex-matched subjects. Methods known in the art, such as multivariate analyses or joint risk analyses, may subsequently be used to determine the overall risk conferred based on the genotype status at the multiple loci. Assessment of risk based on such analysis may subsequently be used in the methods and kits of the invention, as described herein.

As described in the above, the haplotype block structure of the human genome has the effect that a large number of variants (markers and/or haplotypes) in linkage disequilibrium with the variant originally associated with a disease or trait may be used as surrogate markers for assessing association to the disease or trait. The number of such surrogate markers will depend on factors such as the historical recombination rate in the region, the mutational frequency in the region (i.e., the number of polymorphic sites or markers in the region), and the extent of LD (size of the LD block) in the region. These markers are usually located within the physical boundaries of the LD block or haplotype block in question as defined using the methods described herein, or by other methods known to the person skilled in the art. However, sometimes marker and haplotype association is found to extend beyond the physical boundaries of the haplotype block as defined. Such markers and/or haplotypes may in those cases be also used as surrogate markers and/or haplotypes for the markers and/or haplotypes physically residing within the haplotype block as defined. As a consequence, markers and haplotypes in LD (typically characterized by r² greater than 0.1, such as r² greater than 0.2, including r² greater than 0.3, also including r² greater than 0.4) with the markers and haplotypes of the present invention are also within the scope of the invention, even if they are physically located beyond the boundaries of the haplotype block as defined. This includes markers that are described herein (e.g., Table 10, Table 15 and Table 19), but may also include other markers that are in strong LD (e.g., characterized by r² greater than 0.1, r² greater than 0.2 and/or |D'| > 0.8) with one or more of the markers listed in Table 10, Table 15 and Table 19.

For the SNP markers described herein, the opposite allele to the allele found to be in excess in patients (at-risk allele) is found in decreased frequency in breast cancer. These markers and haplotypes in LD and/or comprising such markers, are thus protective for breast cancer, i.e. they confer a decreased risk.or susceptibility of individuals carrying these markers and/or haplotypes developing breast cancer.

Certain variants of the present invention, including certain haplotypes comprise, in some cases, a combination of various genetic markers, *e.g*., SNPs and microsatellites. Therefore, detecting haplotypes can be accomplished by methods known in the art and/or described herein for detecting sequences at polymorphic sites. Furthermore, correlation between certain haplotypes or sets of markers and disease phenotype can be verified using standard techniques. A representative example of a simple test for correlation would be a Fisher-exact test on a two by two table.

In specific embodiments, a marker allele or haplotype found to be associated with breast cancer, (e.g., marker alleles as listed in Table 10, Table 15, Table 19, Table 20, Table 21 and Table 22, and markers in linkage disequilibrium therewith) is one in which the marker allele or haplotype is more frequently present in an individual at risk for breast cancer (affected), compared to the frequency of its presence in a healthy individual (control), wherein the presence of the marker allele or haplotype is indicative of breast cancer or a susceptibility to breast cancer. In other embodiments, at-risk markers in linkage disequilibrium with one or more markers found to be associated with breast cancer (e.g., markers as listed in Table 10, Table 15, Table 19, Table 20, Table 21 and Table 22, and markers in linkage disequilibrium therewith) are tagging markers that are more frequently present in an individual at risk for breast cancer (affected), compared to the frequency of their presence in a healthy individual (control), wherein the presence of the tagging markers is indicative of increased susceptibility to breast cancer. In a further embodiment, at-risk markers alleles (i.e. conferring increased susceptibility) in linkage disequilibrium with one or more markers found to be associated with breast cancer (e.g., marker alleles as listed in Table 10, Table 15, Table 19, Table 20, Table 21 and Table 22, and markers in linkage disequilibrium therewith), are markers comprising one or more allele that is more frequently present in an individual at risk for breast cancer, compared to the frequency of their presence in a healthy individual (control), wherein the presence of the markers is indicative of increased susceptibility to breast cancer.

### Study population

In a general sense, the methods and kits of the invention can be utilized from samples containing genomic DNA from any source, i.e. any individual. In preferred embodiments, the individual is a human individual. The individual can be an adult, child, or fetus. The present invention also provides for assessing markers and/or haplotypes in individuals who are members of a target population. Such a target population is in one embodiment a population or group of individuals at risk of developing the disease, based on other genetic factors, biomarkers, biophysical parameters (e.g., weight, BMD, blood pressure), or general health and/or lifestyle parameters (e.g., history of disease or related diseases, previous diagnosis of disease, family history of disease).

The invention provides for embodiments that include individuals from specific age subgroups, such as those over the age of 40, over age of 45, or over age of 50, 55, 60, 65, 70, 75, 80, or 85. Other embodiments of the invention pertain to other age groups, such as individuals aged less than 85, such as less than age 80, less than age 75, or less than age 70, 65, 60, 55, 50, 45, 40, 35, or age 30. Other embodiments relate to individuals with age at onset of the disease in any of the age ranges described in the above. It is also contemplated that a range of ages may be relevant in certain embodiments, such as age at onset at more than age 45 but less than age 60. Other age ranges are however also contemplated, including all age ranges bracketed by the age values listed in the above.. The invention furthermore relates to individuals of either sex, males or females. In one embodiment, it relates to assessment of male subjects. In another embodiment, it relates to assessment of female subjects.

The Icelandic population is a Caucasian population of Northern European ancestry. A large number of studies reporting results of genetic linkage and association in the Icelandic population have been published in the last few years. Many of those studies show replication of variants, originally identified in the Icelandic population as being associating with a particular disease, in other populations (Stacey, S.N., et al., Nat Genet. May 27 2007 (Epub ahead of print; Helgadottir, A., et al., Science 316:1491-93 (2007); Steinthorsdottir, V., et al., Nat Genet. 39:770-75 (2007); Gudmundsson, J., et al., Nat Genet. 39:631-37 (2007); Amundadottir, L.T., et al., Nat Genet. 38:652-58 (2006); Grant, S.F., et al., Nat Genet. 38:320-23 (2006)). Thus, genetic findings in the Icelandic population have in general been replicated in other populations, including populations from Africa and Asia.

The markers of the present invention found to be associated with breast cancer are believed to show similar association in other human populations. Particular embodiments comprising individual human populations are thus also contemplated and within the scope of the invention. Such embodiments relate to human subjects that are from one or more human population including, but not limited to, Caucasian populations, European populations, American populations, Eurasian populations, Asian populations, Central/South Asian populations, East Asian populations, Middle Eastern populations, African populations, Hispanic populations, and Oceanian populations. European populations include, but are not limited to, Swedish, Norwegian, Finnish, Russian, Danish, Icelandic, Irish, Kelt, English, Scottish, Dutch, Belgian, French, German, Spanish, Portugues, Italian, Polish, Bulgarian, Slavic, Serbian, Bosnian, Chech, Greek and Turkish populations. The invention furthermore in other embodiments can be practiced in specific human populations that include Bantu, Mandenk, Yoruba, San, Mbuti Pygmy, Orcadian, Adygel, Russian, Sardinian, Tuscan, Mozabite, Bedouin, Druze, Palestinian, Balochi, Brahui, Makrani, Sindhi, Pathan, Burusho, Hazara, Uygur, Kalash, Han, Dai, Daur, Hezhen, Lahu, Miao, Oroqen, She, Tujia, Tu, Xibo, Yi, Mongolan, Naxi, Cambodian, Japanese, Yakut, Melanesian, Papuan, Karitianan, Surui, Colmbian, Maya and Pima.

In one embodiment, the invention relates to populations that include black African ancestry such as populations comprising persons of African descent or lineage. Black African ancestry may be determined by self reporting as African-Americans, Afro-Americans, Black Americans, being a member of the black race or being a member of the negro race. For example, African Americans or Black Americans are those persons living in North America and having origins in any of the black racial groups of Africa. In another example, self-reported persons of black African ancestry may have at least one parent of black African ancestry or at least one grandparent of black African ancestry.

The racial contribution in individual subjects may also be determined by genetic analysis. Genetic analysis of ancestry may be carried out using unlinked microsatellite markers such as those set out in Smith et al. Am J Hum Genet 74, 1001-13 (2004).

In certain embodiments, the invention relates to markers and/or haplotypes identified in specific populations, as described in the above. The person skilled in the art will appreciate that measures of linkage disequilibrium (LD) may give different results when applied to different populations. This is due to different population history of different human populations as well as differential selective pressures that may have led to differences in LD in specific genomic regions. It is also well known to the person skilled in the art that certain markers, e.g. SNP markers, are polymorphic in one population but not in another. The person skilled in the art will however apply the methods available and as thought herein to practice the present invention in any given human population. This may include assessment of polymorphic markers in the LD region of the present invention, so as to identify those markers that give strongest association within the specific population. Thus, the at-risk variants of the present invention may reside on different haplotype background and in different frequencies in various human populations. However, utilizing methods known in the art and the markers of the present invention, the invention can be practiced in any given human population.

### Utility of Genetic Testing

The person skilled in the art will appreciate and understand that the variants described herein in general do not, by themselves, provide an absolute identification of individuals who will develop breast cancer. The variants described herein do however indicate increased and/or decreased likelihood that individuals carrying the at-risk or protective variants of the invention will develop breast cancer. This information is however extremely valuable in itself, as outlined in more detail in the below, as it can be used for disease management and selection of appropriate treatment options.

The knowledge about a genetic variant that confers a risk of developing a disease (e.g., breast cancer) offers the opportunity to apply a genetic test to distinguish between individuals with increased risk of developing the disease (i.e. carriers of the at-risk variant) and those with decreased risk of developing the disease (i.e. carriers of the protective variant). The core values of genetic testing, for individuals belonging to both of the above mentioned groups, are the possibilities of being able to diagnose the disease, or a predisposition to the disease, at an early stage and provide information to the clinician about prognosis/aggressiveness of disease in order to be able to apply the most appropriate treatment.

### Models to predict inherited risk for breast cancer

The goal of breast cancer risk assessment is to provide a rational framework for the development of personalized medical management strategies for all women with the aim of increasing survival and quality of life in high-risk women while minimizing costs, unnecessary interventions and anxiety in women at lower risk. Risk prediction models attempt to estimate the risk for breast cancer in an individual who has a given set of congenital risk characteristics (*e.g*., family history, prior benign breast lesion, previous breast tumor). The breast cancer risk assessment models most commonly employed in clinical practice estimate inherited risk factors by considering family history. The risk estimates are based on the observations of increased risks to individuals with one or more close relatives previously diagnosed with breast cancer. They do not take into account complex pedigree structures. These models have the further disadvantage of not being able to differentiate between carriers and non-carriers of genes with breast cancer predisposing mutations.

More sophisticated risk models have better mechanisms to deal with specific family histories and have an ability to take into account carrier status for BRCA1 and BRCA2 mutations. For example, the Breast and Ovarian Analysis of Disease Incidence and Carrier Estimation Algorithm (BOADICEA) (Antoniou *et al.,* 2004) takes into account family history based on individual pedigree structures through the pedigree analysis program MENDEL. Information on known BRCA1 and BRCA2 status is also taken into account. The main limitations of the BOADICEA and all other breast cancer risk models currently in use are that they do not incorporate genotypic information from other predisposition genes. Current models depend strongly on family history to act as a surrogate to compensate for the lack of knowledge of non-BRCA genetic determinants of risk. Therefore the available models are limited to situations where there is a known family history of disease. Lower penetrance breast cancer predisposition genes may be relatively common in the population and may not show such strong tendencies to drive familial clustering as do the BRCA1 and BRCA2 genes. Patients with a relatively high genetic load of predisposition alleles may show little or no family history of disease. There is a need therefore to construct models which incorporate inherited susceptibility data obtained directly through gene-based testing. In addition to making the models more precise, this will reduce the dependency on family history parameters and assist in the extension of the risk profiling into the wider at-risk population where family history is not such a key factor.

### Integration of improved genetic risk models into clinical management of Breast Cancer primary prevention

Clinical primary prevention options currently can be classified as chemopreventative (or hormonal) treatments and prophylactic surgery. Patients identified as high risk can be prescribed long-term courses of chemopreventative therapies. This concept is well accepted in the field of cardiovascular medicine, but is only now beginning to make an impact in clinical oncology. The most widely used oncology chemopreventative is Tamoxifen, a Selective Estrogen Receptor Modulator (SERM). Initially used as an adjuvant therapy directed against breast cancer recurrence, Tamoxifen now has proven efficacy as a breast cancer preventative agent (Cuzick *et a*/*.,* 2003; Martino *et al.,* 2004). The FDA has approved the use of Tamoxifen as a chemopreventative agent in certain high risk women.

Unfortunately, long term Tamoxifen use increases risks for endometrial cancer approximately 2.5-fold, the risk of venous thrombosis approximately 2.0-fold. Risks for pulmonary embolism, stroke, and cataracts are also increased (Cuzick *et al.,* 2003). Accordingly, the benefits in Tamoxifen use for reducing breast cancer incidence may not be easily translated into corresponding decreases in overall mortality. Another SERM called Raloxifene may be more efficacious in a preventative mode, and does not carry the same risks for endometrial cancer. However risk for thrombosis is still elevated in patients treated long-term with Raloxifene (Cuzick *et al.,* 2003; Martino *et al.,* 2004). Moreover, both Tamoxifen and Raloxifene have quality of life issues associated with them. To make a rational risk: benefit analysis of SERM therapy in a chemopreventative mode, there is a clinical need to identify individuals who are most at risk for breast cancer. Given that a substantial proportion of risk for breast cancer is genetic, there is a clear clinical need for genetic tests to quantify individuals' risks in this context. One can anticipate similar issues arising from any future cancer chemo-preventative therapies that may become available, such as the aromatase inhibitors. Moreover, as chemopreventative therapies become safer, there is an increased need to identify patients who are genetically predisposed, but do not have massively elevated risks associated with BRCA1 & 2 carriers.

Patients who are identified as being at high risk for breast cancer are considered for prophylactic surgery; either bilateral mastectomy or oophorectomy or both. Clearly such drastic treatments are recommended only for patients who are perceived to be at extremely high risk. In practice, such risks can currently be identified only in individuals who carry mutations in BRCA1, BRCA2 or genes known to be involved in rare breast cancer predisposition syndromes like p53 in Li-Fraumeni Syndrome, PTEN in Cowden's Syndrome.

Estimates of the penetrance of BRCA1 and BRCA2 mutations tend to be higher when they are derived from multiple-case families than when they are derived from population-based estimates. This is because different mutation-carrying families exhibit different penetrances for breast cancer (see Thorlacius et *a*/*.,* 1997, for example). One of the major factors contributing to this variation is the action of as yet unknown predisposition genes whose effects modify the penetrance of BRCA1 and BRCA2 mutations. Therefore the absolute risk to an individual who carries a mutation in the BRCA1 or BRCA2 genes cannot be accurately quantified in the absence of knowledge of the existence and action of modifying genes. Since the treatment options for BRCA1 and BRCA2 carriers can be severe, it is important in this context to quantify the risks to individual BRCA carriers with the greatest accuracy possible. There is a need, therefore, to identify predisposition genes whose effects modify the penetrance of breast cancer in BRCA1 and BRCA2 carriers and to develop improved risk assessment models based on these genes.

Furthermore, there are individuals who are perceived to be at very high risk for breast cancer, perhaps because of a strong family history of breast cancer, but in whom no mutations in known predisposition genes can be identified. Consideration of prophylactic surgery is difficult in such cases because one cannot test the individual to discover whether or not she has inherited a high penetrance predisposition gene. Accordingly, the individual's risk cannot be assessed accurately. There is a clear clinical need, therefore, to identify any high penetrance predisposition genes that remain undiscovered and to develop associated genetic tests for use primary prevention strategies.

### Early diagnosis

Clinical screening for breast cancer in most western countries consists of periodic clinical breast examination (CBE) and X-ray mammography. There is good evidence to indicate that CBE has little added benefit when used in the context of a good mammographic screening program. In the United Kingdom, women between the ages of 50 and 70 are invited to undergo screening mammography every three years. The situation in the United States varies depending on healthcare provider, however the American Cancer Society recommends annual mammographic screening from age 40. Mammographic screening has proven effectiveness in reducing mortality amongst screened women over the age of 50.

It is unlikely that genetic testing would ever be employed as a means of reducing access to existing mammographic screening programs. However, mammographic screening is not without shortcomings and it is conceivable that genetic testing should be used to select people for augmented screening programs. One of the drawbacks of mammographic screening is that is has thus far not been possible to demonstrate a significant effect on improved survival for women screened under 50 years of age.

One reason that mammography is less effective in women under 50 may be that the density of breast tissue is higher in younger women, making mammographic detection of tumors more difficult. However, breast cancers in predisposed individuals tend to occur at early ages groups and there is a clear association between high breast density and breast cancer risk. Therefore there is a problem with simple increases in mammographic screening for individuals with high predisposition because they would be managed by a technique that performs sub-optimally in the group at highest risk. Recent studies have shown that contrast-enhanced magnetic resonance imaging (CE-MRI) is more sensitive and detects tumors at an earlier stage in this high-risk group than mammographic screening does (Warner *et al.,* 2004; Leach *et al.,* 2005). CE-MRI strategies work particularly well when used in combination with routine X-ray mammography (Leach *et al.,* 2005). Because CE-MRI requires specialist centers that incur high costs, screening of under-50's must be restricted to those individuals at the highest risk. Present CE-MRI trials restrict entry to those individuals with BRCA1, BRCA2 or p53 mutations or very strong family histories of disease. The extension of this screening modality to a wider range of high-risk patients would be greatly assisted by the provision of gene-based risk profiling tools.

There is good evidence to support the notion that early-onset breast cancers and cancers occurring in genetically predisposed women grow faster than cancers in older, less strongly predisposed women. This comes from observations of higher rates of interval cancers in younger women, that is, cancers that arise in the intervals between screening visits in a well-screened population are higher amongst younger women. Therefore there are suggestions that screening intervals, by whatever method, should be reduced for younger women. There is a paradox here in that more frequent screening using more expensive methodologies seems to be required for an age group in which the overall rates of breast cancer are comparatively low. There is a clear clinical need here to identify those young individuals who are most strongly predisposed to develop the disease early, and channel them into more expensive and extensive screening regimes.

### Treatment

Currently, primary breast cancer is treated by surgery, adjuvant chemotherapy, radiotherapy, followed by long term hormonal therapy. Often combinations of three or four therapies are used.

Breast cancer patients with the same stage of disease can have very different responses to adjuvant chemotherapy resulting in a broad variation in overall treatment outcomes. Consensus guidelines (the St Galen and NIH criteria) have been developed for determining the eligibility of breast cancer patients for adjuvant chemotherapy treatment. However, even the strongest clinical and histological predictors of metastasis fail to predict accurately the clinical responses of breast tumors (Goldhirsch *et al.,* 1998; Eifel *et al.,* 2001). Chemotherapy or hormonal therapy reduces the risk of metastasis only by approximately 1/3, however 70-80% of patients receiving this treatment would have survived without it. Therefore the majority of breast cancer patients are currently offered treatment that is either ineffective or unnecessary. There is a clear clinical need for improvements in the development of prognostic measures which will allow clinicians to tailor treatments more appropriately to those who will best benefit. It is reasonable to expect that profiling individuals for genetic predisposition may reveal information relevant to their treatment outcome and thereby aid in rational treatment planning.

Several previous studies exemplify this concept: Breast cancer patients who are BRCA mutation carriers appear to show better clinical response rates and survival when treated with adjuvant chemotherapies [Chappuis, et al., (2002), J Med Genet, 39, 608-10; Goffin, et al., (2003), Cancer, 97, 527-36]. BRCA mutation carriers demonstrate improved responses to platinum chemotherapy for ovarian cancer than non-carriers [Cass, et al., (2003), Cancer, 97, 2187-95]. Similar considerations may apply to predisposed patients in whom the genes involved are not known. For example, infiltrating lobular breast carcinoma (ILBC) is known to have a strong familial component but the genetic variants involved have not yet been identified. Patients with ILBC demonstrate poorer responses to common chemotherapy regimes [Mathieu, et al., (2004), Eur J Cancer, 40, 342-51].

Genetic predisposition models may not only aid in the individualization of treatment strategies, but may play an integral role in the design of these strategies. For example, BRCA1 and BRCA2 mutant tumor cells have been found to be profoundly sensitive to poly (ADP-ribose) polymerase (PARP) inhibitors as a result of their defective DNA repair pathway [Farmer, et al., (2005), Nature, 434, 917-21]. This has stimulated development of small molecule drugs targeted on PARP with a view to their use specifically in BRCA carrier patients. From this example it is clear that knowledge of genetic predisposition may identify drug targets that lead to the development of personalized chemotherapy regimes to be used in combination with genetic risk profiling. Cancer chemotherapy has well known, dose-limiting side effects on normal tissues particularly the highly proliferate hemopoetic and gut epithelial cell compartments. It can be anticipated that genetically-based individual differences exist in sensitivities of normal tissues to cytotoxic drugs. An understanding of these factors might aid in rational treatment planning and in the development of drugs designed to protect normal tissues from the adverse effects of chemotherapy.

Genetic profiling may also contribute to improved radiotherapy approaches: Within groups of breast cancer patients undergoing standard radiotherapy regimes, a proportion of patients will experience adverse reactions to doses of radiation that are normally tolerated. Acute reactions include erythema, moist desquamation, edema and radiation pneumatitis. Long term reactions including telangiectasia, edema, pulmonary fibrosis and breast fibrosis may arise many years after radiotherapy. Both acute and long-term reactions are considerable sources of morbidity and can be fatal. In one study, 87% of patients were found to have some adverse side effects to radiotherapy while 11% had serious adverse reactions (LENT/SOMA Grade 3-4); [Hoeller, et al., (2003), Int J Radiat Oncol Biol Phys, 55, 1013-8]. The probability of experiencing an adverse reaction to radiotherapy is due primarily to constitutive individual differences in normal tissue reactions and there is a suspicion that these have a strong genetic component. Several of the known breast cancer predisposition genes (e.g. BRCA1, BRCA2, ATM) affect pathways of DNA double strand break repair. DNA double strand breaks are the primary cytotoxic lesion induced by radiotherapy. This has led to concern that individuals who are genetically predisposed to breast cancer through carriage of variants in genes belonging to these pathways might also be at higher risk of suffering excessive normal tissue damage from radiotherapy.

The existence of constitutively radiosensitive individuals in the population means that radiotherapy dose rates for the majority of the patient population must be restricted, in order to keep the frequency of adverse reactions to an acceptable level. There is a clinical need, therefore, for reliable tests that can identify individuals who are at elevated risk for adverse reactions to radiotherapy. Such tests would indicate conservative or alternative treatments for individuals who are radiosensitive, while permitting escalation of radiotherapeutic doses for the majority of patients who are relatively radioresistant. It has been estimated that the dose escalations made possible by a test to triage breast cancer patients simply into radiosensitive, intermediate and radioresistant categories would result in an approximately 35% increase in local tumor control and consequent improvements in survival rates (Burnet *et al.,* 1996).

Exposure to ionizing radiation is a proven factor contributing to oncogenesis in the breast (Dumitrescu and Cotarla 2005). Known breast cancer predisposition genes encode pathway components of the cellular response to radiation-induced DNA damage (Narod and Foulkes 2004). Accordingly, there is concern that the risk for second primary breast tumors may be increased by irradiation of normal tissues within the radiotherapy field. There does not appear to be any measurable increased risk for BRCA carriers from radiotherapy, however their risk for second primary tumors is already exceptionally high. There is evidence to suggest that risk for second primary tumors is increased in carriers in breast cancer predisposing alleles of the ATM and CHEK2 genes who are treated with radiotherapy (Bernstein *et al.,* 2004; Broeks *et al.,* 2004). It is expected that the risk of second primary tumors from radiotherapy (and, possibly, from intensive mammographic screening) will be better defined by obtaining accurate genetic risk profiles from patients during the treatment planning stage.

### Secondary Prevention

Approximately 30% of patients who are diagnosed with a stage 1 or 2 breast cancer will experience either a loco-regional or distant metastatic recurrence of their original tumor. Patients who have had a primary breast cancer are also at greatly increased risk for being diagnosed with a second primary tumor, either in the contralateral breast or in the ipsilateral breast when breast-conserving surgery has been carried out. Secondary prevention refers to methods used to prevent recurrences or second primary tumors from developing. Methods currently in use comprise; long-term treatment with Tamoxifen or another SERM either alone or alternated with an aromatase inhibitor, risk-reducing mastectomy of the contralateral breast, and risk-reducing oophorectomy (in patients who are at risk for familial breast-ovarian cancer). Considerations regarding the use of Tamoxifen have been discussed above. With risk-reducing surgical options, it is clear that the risk needs to be quantified as well as possible in order to make an informed cost: benefit analysis.

There are some indications that patients with known genetic predispositions to breast cancer fare worse than the majority of patients. Patients carrying the CHEK2 gene 1100delC variant have an estimated 2.8-fold increased risk of distant metastasis and a 3.9-fold increased risk of disease recurrence compared to non-carriers [de Bock, et al., (2004), J Med Genet, 41, 731-5]. Patients with BRCA1 node-negative tumors have a greater risk of metastasis than similar patients who do not carry a BRCA1 mutatation[Goffin, et al., (2003), Cancer, 97, 527-36; Moller, et al., (2002), Int J Cancer, 101, 555-9; Eerola, et al., (2001), Int J Cancer, 93, 368-72]. Genetic profiling can therefore be used to help assess the risk of local recurrence and metastasis, thereby guiding the choice of secondary preventative treatment.

In general, patients with a primary tumor diagnosis are at risk from second primary tumors at a constant annual incidence of 0.7% (Peto and Mack 2000). Patients with BRCA mutations are at significantly greater risks for second primary tumors than most breast cancer patients, with absolute risks in the range 40-60% (Easton 1999). Carriers of BRCA mutations have a greatly increased risk for second primary tumors [Stacey, et al., (2006), PLoS Med, 3, e217; Metcalfe, et al., (2004), J Clin Oncol, 22, 2328-35]. Patients with mutations in the CHEK2 gene have and estimated 5.7-fold increased risk of contralateral breast cancer [de Bock, et al., (2004), J Med Genet, 41, 731-5]. Carriers of the BARD1 Cys557Ser variant are 2.7 fold more likely to be diagnosed with a second primary tumor [Stacey, et al., (2006), PLoS Med, 3, e217]. Genetic risk profiling can be used to assess the risk of second primary tumors in patients and will inform decisions on how aggressive the preventative measures should be.

### METHODS

Methods for risk assessment and diagnosis of breast cancer are described herein and are encompassed by the invention. The invention also encompasses methods of assessing an individual for probability of response to a therapeutic agent for breast cancer, as well as methods for predicting the effectiveness of a therapeutic agent for breast cancer. Kits for assaying a sample from a subject to detect susceptibility to breast cancer are also encompassed by the invention.

### Diagnostic and screening assays

In certain embodiments, the present invention pertains to methods of diagnosing, or aiding in the diagnosis of, breast cancer or a susceptibility to breast cancer, by detecting particular alleles at genetic markers that appear more frequently in breast cancer subjects or subjects who are susceptible to breast cancer. In a particular embodiment, the invention is a method of diagnosing a susceptibility to breast cancer by detecting at least one allele of at least one polymorphic marker (e.g., the markers described herein). The present invention describes methods whereby detection of particular alleles of particular markers or haplotypes is indicative of a susceptibility to breast cancer. Such prognostic or predictive assays can also be used to determine prophylactic treatment of a subject prior to the onset of symptoms of breast cancer. The present invention pertains in some embodiments to methods of clinical applications of diagnosis, *e.g.,* diagnosis performed by a medical professional. In other embodiments, the invention pertains to methods of diagnosis or determination of a susceptibility performed by a layman. Recent technological advances in genotyping technologies, including high-throughput genotyping of SNP markers, such as Molecular Inversion Probe array technology *(e.g.,* Affymetrix GeneChip), and BeadArray Technologies *(e.g.,* Illumina GoldenGate and Infinium assays) have made it possible for individuals to have their own genome assessed for up to one million SNPs simultaneously, at relatively low cost. The resulting genotype information, made available to the individual, can be compared to information from the public literature about disease or trait risk associated with various SNPs. The diagnostic application of disease-associated alleles as described herein, can thus be performed either by the individual, through analysis of his/her genotype data, or by a health professional based on results of a clinical test. In other words, the diagnosis or assessment of a susceptibility based on genetic risk can be made by health professionals, genetic counselors or by the layman, based on information about his/her genotype and publications on various risk factors. In the present context, the term "diagnosing", "diagnose a susceptibility" and "determine a susceptibility" is meant to refer to any available diagnostic method, including those mentioned above.

In addition, in certain other embodiments, the present invention pertains to methods of diagnosing, or aiding in the diagnosis of, a decreased susceptibility to breast cancer, by detecting particular genetic marker alleles or haplotypes that appear less frequently in breast cancer patients than in individual not diagnosed with breast cancer or in the general population.

As described and exemplified herein, particular marker alleles or haplotypes (e.g. markers located within the rs4848543 LD block, the rs13387042 LD block, the rs3803662 LD block, the STEAP3/TSAP6 gene, the markers and haplotypes as listed in Table 10, Table 15, Table 19, Table 20, Table 21 and Table 22, and markers in linkage disequilibrium therewith) are associated with breast cancer (e.g., All BC and/or MedPre breast cancer). In one embodiment, the marker allele or haplotype is one that confers a significant risk or susceptibility to breast cancer. In another embodiment, the invention relates to a method of diagnosing a susceptibility to breast cancer in a human individual, the method comprising determining the presence or absence of at least one allele of at least one polymorphic marker in a nucleic acid sample obtained from the individual, wherein the at least one polymorphic marker is selected from the group consisting of the polymorphic markers located within the rs4848543 LD block, the rs13387042 LD block, the rs3803662 LD block, the STEAP3/TSAP6 gene, the markers and haplotypes as listed in Table 10, Table 15, Table 19, Table 20, Table 21 and Table 22, and markers in linkage disequilibrium (e.g., defined as r² > 0.2) therewith. In another embodiment, the invention pertains to methods of diagnosing a susceptibility to breast cancer in a human individual, by screening for at least one marker allele or haplotype, e.g. the markers located within the rs4848543 LD block, the rs13387042 LD block, the rs3803662 LD block, the STEAP3/TSAP6 gene, the markers and haplotypes as listed in Table 10, Table 15, Table 19, Table 20, Table 21 and Table 22, or markers in linkage disequilibrium therewith. In another embodiment, the marker allele or haplotype is more frequently present in a subject having, or who is susceptible to, breast cancer (affected, e.g. All BC or MedPre breast cancer), as compared to the frequency of its presence in a healthy subject (control, such as population controls). In another embodiment, the invention relates to a method of diagnosing a susceptibility to breast cancer in a human individual, the method comprising determining the presence or absence of at least one allele of at least one polymorphic marker in a nucleic acid sample obtained from the individual, wherein the at least one polymorphic marker is selected from marker rs4848543, marker rs13387042, marker rs3803662, and markers in linkage disequilibrium therewith. In certain embodiments, the significance of association of the at least one marker allele or haplotype is characterized by a p value < 0.05. In other embodiments, the significance of association is characterized by smaller p-values, such as < 0.01, <0.001, <0.0001, <0.00001, <0.000001, <0.0000001, <0.00000001 or <0.000000001.

In these embodiments, the presence of the at least one marker allele or haplotype is indicative of a susceptibility to breast cancer. These diagnostic methods involve detecting the presence or absence of at least one marker allele or haplotype that is associated with breast cancer. The haplotypes described herein include combinations of alleles at various genetic markers (e.g., SNPs, microsatellites). The detection of the particular genetic marker alleles that make up the particular haplotypes can be performed by a variety of methods described herein and/or known in the art. For example, genetic markers can be detected at the nucleic acid level (e.g., by direct nucleotide sequencing or by other means known to the skilled in the art) or at the amino acid level if the genetic marker affects the coding sequence of a protein encoded by a breast cancer - associated nucleic acid (e.g., by protein sequencing or by immunoassays using antibodies that recognize such a protein). The marker alleles or haplotypes of the present invention correspond to fragments of a genomic DNA sequence associated with breast cancer. Such fragments encompass the DNA sequence of the polymorphic marker or haplotype in question, but may also include DNA segments in strong LD (linkage disequilibrium) with the marker or haplotype (e.g., as determined by a value of r² greater than 0.2 and/or |D'| > 0.8).

In one embodiment, diagnosis of a susceptibility to breast cancer can be accomplished using hybridization methods, such as Southern analysis, Northern analysis, and/or *in situ* hybridizations (see Current Protocols in Molecular Biology, Ausubel, F. et al., eds., John Wiley & Sons, including all supplements). A biological sample from a test subject or individual (a "test sample") of genomic DNA, RNA, or cDNA is obtained from a subject suspected of having, being susceptible to, or predisposed for breast cancer (the "test subject"). The subject can be an adult, child, or fetus. The test sample can be from any source that contains genomic DNA, such as a blood sample, sample of amniotic fluid, sample of cerebrospinal fluid, or tissue sample from skin, muscle, buccal or conjunctival mucosa, placenta, gastrointestinal tract or other organs. A test sample of DNA from fetal cells or tissue can be obtained by appropriate methods, such as by amniocentesis or chorionic villus sampling. The DNA, RNA, or cDNA sample is then examined. The presence of a specific marker allele can be indicated by sequence-specific hybridization of a nucleic acid probe specific for the particular allele. The presence of more than specific marker allele or a specific haplotype can be indicated by using several sequence-specific nucleic acid probes, each being specific for a particular allele. In one embodiment, a haplotype can be indicated by a single nucleic acid probe that is specific for the specific haplotype (i.e., hybridizes specifically to a DNA strand comprising the specific marker alleles characteristic of the haplotype). A sequence-specific probe can be directed to hybridize to genomic DNA, RNA, or cDNA. A "nucleic acid probe", as used herein, can be a DNA probe or an RNA probe that hybridizes to a complementary sequence. One of skill in the art would know how to design such a probe so that sequence specific hybridization will occur only if a particular allele is present in a genomic sequence from a test sample.

To diagnose a susceptibility to breast cancer, a hybridization sample is formed by contacting the test sample containing an breast cancer -associated nucleic acid, such as a genomic DNA sample, with at least one nucleic acid probe. A non-limiting example of a probe for detecting mRNA or genomic DNA is a labeled nucleic acid probe that is capable of hybridizing to mRNA or genomic DNA sequences described herein. The nucleic acid probe can be, for example, a full-length nucleic acid molecule, or a portion thereof, such as an oligonucleotide of at least 15, 30, 50, 100, 250 or 500 nucleotides in length that is sufficient to specifically hybridize under stringent conditions to appropriate mRNA or genomic DNA. For example, the nucleic acid probe can comprise all or a portion of the nucleotide sequence as set forth in SEQ ID NO:4, SEQ IN NO:5 or SEQ ID NO:6, the nucleotide sequence of the rs4848543 LD block, the rs13387042 LD block, the rs3803662 LD block, and/or the STEAP3/TSAP gene, as described herein, optionally comprising at least one allele of a marker described herein, or at least one marker or haplotype described herein (e.g., the markers and haplotypes as listed in Table 10, Table 15, Table 19, Table 20, Table 21 and Table 22, and markers in linkage disequilibrium therewith), or the probe can be the complementary sequence of such a sequence. In a particular embodiment, the nucleic acid probe is a portion of the nucleotide sequence as set forth in SEQ ID NO:4, SEQ ID NO: 5, or SEQ ID NO:6; or the nucleotide sequence of the rs4848543 LD block, the rs13387042 LD block, the rs3803662 LD block and/or the STEAP3/TSAP6 gene, as described herein, optionally comprising at least one allele of a marker described herein (e.g., the markers and haplotypes as listed in Table 10, Table 15, Table 19, Table 20, Table 21 and Table 22, and markers in linkage disequilibrium therewith), or at least one allele contained in the haplotypes described herein, or the probe can be the complementary sequence of such a sequence. Other suitable probes for use in the diagnostic assays of the invention are described herein. Hybridization can be performed by methods well known to the person skilled in the art (see, *e.g.,* Current Protocols in Molecular Biology, Ausubel, F. et al., eds., John Wiley & Sons, including all supplements). In one embodiment, hybridization refers to specific hybridization, i.e., hybridization with no mismatches (exact hybridization). In one embodiment, the hybridization conditions for specific hybridization are high stringency.

Specific hybridization, if present, is then detected using standard methods. If specific hybridization occurs between the nucleic acid probe and the breast cancer-associated nucleic acid in the test sample, then the sample contains the allele that is complementary to the nucleotide that is present in the nucleic acid probe. The process can be repeated for other markers of the present invention, or markers that make up a haplotype of the present invention, or multiple probes can be used concurrently to detect more than one marker alleles at a time. It is also possible to design a single probe containing more than one marker alleles of a particular haplotype (e.g., a probe containing alleles complementary to 2, 3, 4, 5 or all of the markers that make up a particular haplotype). Detection of the particular markers of the haplotype in the sample is indicative that the source of the sample has the particular haplotype (e.g., a haplotype) and therefore is susceptible to breast cancer.

In one preferred embodiment, a method utilizing a detection oligonucleotide probe comprising a fluorescent moiety or group at its 3' terminus and a quencher at its 5' terminus, and an enhancer oligonucleotide, is employed, as described by Kutyavin et al. (Nucleic Acid Res. 34:e128 (2006)). The fluorescent moiety can be Gig Harbor Green or Yakima Yellow, or other suitable fluorescent moieties. The detection probe is designed to hybridize to a short nucleotide sequence that includes the SNP polymorphism to be detected. Preferably, the SNP is anywhere from the terminal residue to -6 residues from the 3' end of the detection probe. The enhancer is a short oligonucleotide probe which hybridizes to the DNA template 3' relative to the detection probe. The probes are designed such that a single nucleotide gap exists between the detection probe and the enhancer nucleotide probe when both are bound to the template. The gap creates a synthetic abasic site that is recognized by an endonuclease, such as Endonuclease IV. The enzyme cleaves the dye off the fully complementary detection probe, but cannot cleave a detection probe containing a mismatch. Thus, by measuring the fluorescence of the released fluorescent moiety, assessment of the presence of a particular allele defined by nucleotide sequence of the detection probe can be performed.

The detection probe can be of any suitable size, although preferably the probe is relatively short. In one embodiment, the probe is from 5-100 nucleotides in length. In another embodiment, the probe is from 10-50 nucleotides in length, and in another embodiment, the probe is from 12-30 nucleotides in length. Other lengths of the probe are possible and within scope of the skill of the average person skilled in the art.

In a preferred embodiment, the DNA template containing the SNP polymorphism is amplified by Polymerase Chain Reaction (PCR) prior to detection. In such an embodiment, the amplified DNA serves as the template for the detection probe and the enhancer probe.

Certain embodiments of the detection probe, the enhancer probe, and/or the primers used for amplification of the template by PCR include the use of modified bases, including modified A and modified G. The use of modified bases can be useful for adjusting the melting temperature of the nucleotide molecule (probe and/or primer) to the template DNA, for example for increasing the melting temperature in regions containing a low percentage of G or C bases, in which modified A with the capability of forming three hydrogen bonds to its complementary T can be used, or for decreasing the melting temperature in regions containing a high percentage of G or C bases, for example by using modified G bases that form only two hydrogen bonds to their complementary C base in a double stranded DNA molecule. In a preferred embodiment, modified bases are used in the design of the detection nucleotide probe. Any modified base known to the skilled person can be selected in these methods, and the selection of suitable bases is well within the scope of the skilled person based on the teachings herein and known bases available from commercial sources as known to the skilled person.

In another hybridization method, Northern analysis (see Current Protocols in Molecular Biology, Ausubel, F. et al., eds., John Wiley & Sons, *supra)* is used to identify the presence of a polymorphism associated with breast cancer. For Northern analysis, a test sample of RNA is obtained from the subject by appropriate means. As described herein, specific hybridization of a nucleic acid probe to RNA from the subject is indicative of a particular allele complementary to the probe. For representative examples of use of nucleic acid probes, see, for example, U.S. Patent Nos. 5,288,611 and 4,851,330.

Additionally, or alternatively, a peptide nucleic acid (PNA) probe can be used in addition to, or instead of, a nucleic acid probe in the hybridization methods described herein. A PNA is a DNA mimic having a peptide-like, inorganic backbone, such as N-(2-aminoethyl)glycine units, with an organic base (A, G, C, T or U) attached to the glycine nitrogen via a methylene carbonyl linker (see, for example, Nielsen, P., et al., Bioconjug. Chem. 5:3-7 (1994)). The PNA probe can be designed to specifically hybridize to a molecule in a sample suspected of containing one or more of the marker alleles or haplotypes that are associated with breast cancer. Hybridization of the PNA probe is thus diagnostic for breast cancer or a susceptibility to breast cancer.

In one embodiment of the invention, a test sample containing genomic DNA obtained from the subject is collected and the polymerase chain reaction (PCR) is used to amplify a fragment comprising one ore more markers or haplotypes of the present invention. As described herein, identification of a particular marker allele or haplotype associated with breast cancer can be accomplished using a variety of methods (e.g., sequence analysis, analysis by restriction digestion, specific hybridization, single stranded conformation polymorphism assays (SSCP), electrophoretic analysis, etc.). In another embodiment, diagnosis is accomplished by expression analysis using quantitative PCR (kinetic thermal cycling). This technique can, for example, utilize commercially available technologies, such as TaqMan^{®} (Applied Biosystems, Foster City, CA), The technique can assess the presence of an alteration in the expression or composition of a polypeptide or splicing variant(s) that is encoded by a nucleic acid associated with breast cancer. Further, the expression of the variant(s) can be quantified as physically or functionally different.

In another method of the invention, analysis by restriction digestion can be used to detect a particular allele if the allele results in the creation or elimination of a restriction site relative to a reference sequence. Restriction fragment length polymorphism (RFLP) analysis can be conducted, e.g., as described in Current Protocols in Molecular Biology, *supra.* The digestion pattern of the relevant DNA fragment indicates the presence or absence of the particular allele in the sample.

Sequence analysis can also be used to detect specific alleles at polymorphic sites associated with breast cancer (e.g. the polymorphic markers and haplotypes of Table 10, Table 15, Table 19, Table 20, Table 21 and/or Table 22, and markers in linkage disequilibrium therewith). Therefore, in one embodiment, determination of the presence or absence of a particular marker alleles or haplotypes comprises sequence analysis of a test sample of DNA or RNA obtained from a subject or individual. PCR or other appropriate methods can be used to amplify a portion of a nucleic acid associated with breast cancer, and the presence of a specific allele can then be detected directly by sequencing the polymorphic site (or multiple polymorphic sites in a haplotype) of the genomic DNA in the sample.

Allele-specific oligonucleotides can also be used to detect the presence of a particular allele at a breast cancer-associated nucleic acid (e.g. the polymorphic markers and haplotypes of Table 10, Table 15, Table 19, Table 20, Table 21 and Table 22 and markers in linkage disequilibrium therewith), through the use of dot-blot hybridization of amplified oligonucleotides with allele-specific oligonucleotide (ASO) probes (see, for example, Saiki, R. et al., Nature, 324:163-166 (1986)). An "allele-specific oligonucleotide" (also referred to herein as an "allele-specific oligonucleotide probe") is an oligonucleotide of approximately 10-50 base pairs or approximately 15-30 base pairs, that specifically hybridizes to a breast cancer-associated nucleic acid, and which contains a specific allele at a polymorphic site (e.g., a marker or haplotype as described herein). An allele-specific oligonucleotide probe that is specific for one or more particular a breast cancer-associated nucleic acid can be prepared using standard methods (see, e.g., Current Protocols in Molecular Biology, *supra).* PCR can be used to amplify the desired region. The DNA containing the amplified region can be dot-blotted using standard methods *(see, e.g.,* Current Protocols in Molecular Biology, *supra),* and the blot can be contacted with the oligonucleotide probe. The presence of specific hybridization of the probe to the amplified region can then be detected. Specific hybridization of an allele-specific oligonucleotide probe to DNA from the subject is indicative of a specific allele at a polymorphic site associated with breast cancer (see, e.g., Gibbs, R. et al., Nucleic Acids Res., 17:2437-2448 (1989) and WO 93/22456).

With the addition of such analogs as locked nucleic acids (LNAs), the size of primers and probes can be reduced to as few as 8 bases. LNAs are a novel class of bicyclic DNA analogs in which the 2' and 4' positions in the furanose ring are joined via an O-methylene (oxy-LNA), S-methylene (thio-LNA), or amino methylene (amino-LNA) moiety. Common to all of these LNA variants is an affinity toward complementary nucleic acids, which is by far the highest reported for a DNA analog. For example, particular all oxy-LNA nonamers have been shown to have melting temperatures (Tₘ) of 64°C and 74°C when in complex with complementary DNA or RNA, respectively, as opposed to 28°C for both DNA and RNA for the corresponding DNA nonamer. Substantial increases in Tₘ are also obtained when LNA monomers are used in combination with standard DNA or RNA monomers. For primers and probes, depending on where the LNA monomers are included (e.g., the 3' end, the 5' end, or in the middle), the Tₘ could be increased considerably.

In another embodiment, arrays of oligonucleotide probes that are complementary to target nucleic acid sequence segments from a subject, can be used to identify polymorphisms in a nucleic acid associated with breast cancer (e.g. the polymorphic markers and haplotypes of Table 10, Table 15, Table 19, Table 20, Table 21 and Table 22 and markers in linkage disequilibrium therewith). For example, an oligonucleotide array can be used. Oligonucleotide arrays typically comprise a plurality of different oligonucleotide probes that are coupled to a surface of a substrate in different known locations. These oligonucleotide arrays, also described as "Genechips™," have been generally described in the art (see, e.g., U.S. Patent No. 5,143,854, PCT Patent Publication Nos. WO 90/15070 and 92/10092). These arrays can generally be produced using mechanical synthesis methods that incorporate a combination of photolithographic methods and solid phase oligonucleotide synthesis methods, or by other methods known to the person skilled in the art ( *see, e.g.,* Fodor, S. et al., Science, 251:767-773 (1991); Pirrung et al., U.S. Patent No. 5,143,854 (see also published PCT Application No. WO 90/15070); and Fodor. S. et al., published PCT Application No. WO 92/10092 and U.S. Patent No. 5,424,186, the entire teachings of each of which are incorporated by reference herein). Techniques for the synthesis of these arrays using mechanical synthesis methods are described in, e.g., U.S. Patent No. 5,384,261; the entire teachings of which are incorporated by reference herein. In another example, linear arrays can be utilized.

Once an oligonucleotide array is prepared, a nucleic acid of interest is allowed to hybridize with the array. Detection of hybridization is a detection of a particular allele in the nucleic acid of interest. Hybridization and scanning are generally carried out by methods described herein or by other methods known to those skilled in the art, for example as described in published PCT Application Nos. WO 92/10092 and WO 95/11995, and U.S. Patent No. 5,424,186, the entire teachings of each of which are incorporated by reference herein. In brief, a target nucleic acid sequence, which includes one or more previously identified polymorphic markers, is amplified by well-known amplification techniques (e.g., PCR). Typically this involves the use of primer sequences that are complementary to the two strands of the target sequence, both upstream and downstream, from the polymorphic site. Asymmetric PCR techniques can also be used. Amplified target, generally incorporating a label, is then allowed to hybridize with the array under appropriate conditions that allow for sequence-specific hybridization. Upon completion of hybridization and washing of the array, the array is scanned to determine the position on the array to which the target sequence hybridizes. The hybridization data obtained from the scan is typically in the form of fluorescence intensities as a function of location on the array.

Although primarily described in terms of a single detection block, e.g., for detection of a single polymorphic site, arrays can include multiple detection blocks, and thus be capable of analyzing multiple, specific polymorphisms (e.g., multiple polymorphisms of a particular haplotype). In alternate arrangements, it will generally be understood that detection blocks can be grouped within a single array or in multiple, separate arrays so that varying, optimal conditions can be used during the hybridization of the target to the array. For example, it will often be desirable to provide for the detection of those polymorphisms that fall within G-C rich stretches of a genomic sequence, separately from those falling in A-T rich segments. This allows for the separate optimization of hybridization conditions for each situation.

Additional descriptions of use of oligonucleotide arrays for detection of polymorphisms can be found, for example, in U.S. Patent Nos. 5,858,659 and 5,837,832, the entire teachings of both of which are incorporated by reference herein.

Other methods of nucleic acid analysis that are available to those skilled in the art can be used to detect a particular allele at a polymorphic site associated with breast cancer (e.g. the polymorphic markers and haplotypes of Table 10, Table 15, Table 19, Table 20, Table 21 and Table 22 and markers in linkage disequilibrium therewith). Representative methods include, for example, direct manual sequencing (Church and Gilbert, Proc. Natl. Acad. Sci. USA, 81: 1991-1995 (1988); Sanger, F., et al., Proc. Natl. Acad. Sci. USA, 74:5463-5467 (1977); Beavis, et al., U.S. Patent No. 5,288,644); automated fluorescent sequencing; single-stranded conformation polymorphism assays (SSCP); clamped denaturing gel electrophoresis (CDGE); denaturing gradient gel electrophoresis (DGGE) (Sheffield, V., et al., Proc. Natl. Acad. Sci. USA, 86:232-236 (1989)), mobility shift analysis (Orita, M., et al., Proc. Natl. Acad. Sci. USA, 86:2766-2770 (1989)), restriction enzyme analysis (Flavell, R., et al., Cell, 15:25-41 (1978); Geever, R., et al., Proc. Natl. Acad. Sci. USA, 78:5081-5085 (1981)); heteroduplex analysis; chemical mismatch cleavage (CMC) (Cotton, R., et al., Proc. Natl. Acad. Sci. USA, 85:4397-4401 (1985)); RNase protection assays (Myers, R., et al., Science, 230:1242-1246 (1985); use of polypeptides that recognize nucleotide mismatches, such as *E. coli* mutS protein; and allele-specific PCR.

In another embodiment of the invention, diagnosis of breast cancer or a susceptibility to breast cancer can be made by examining expression and/or composition of a polypeptide encoded by breast cancer-associated nucleic acid in those instances where the genetic marker(s) or haplotype(s) of the present invention result in a change in the composition or expression of the polypeptide. Thus, diagnosis of a susceptibility to breast cancer can be made by examining expression and/or composition of one of these polypeptides, or another polypeptide encoded by a breast cancer-associated nucleic acid, in those instances where the genetic marker or haplotype of the present invention results in a change in the composition or expression of the polypeptide. The haplotypes and markers of the present invention that show association to breast cancer may play a role through their effect on one or more of these nearby genes. Possible mechanisms affecting these genes include, e.g., effects on transcription, effects on RNA splicing, alterations in relative amounts of alternative splice forms of mRNA, effects on RNA stability, effects on transport from the nucleus to cytoplasm, and effects on the efficiency and accuracy of translation.

Thus, in another embodiment, the variants (markers or haplotypes) of the invention showing association to breast cancer affect the expression of a nearby gene. It is well known that regulatory element affecting gene expression may be located tenths or even hundreds of kilobases away from the promoter region of a gene. By assaying for the presence or absence of at least one allele of at least one polymorphic marker of the present invention, it is thus possible to assess the expression level of such nearby genes. It is thus contemplated that the detection of the markers or haplotypes of the present invention can be used for assessing expression for one or more of the STEAP3/TSAP6, LOC643714 , and TNRC9 genes.

A variety of methods can be used for detecting protein expression levels, including enzyme linked immunosorbent assays (ELISA), Western blots, immunoprecipitations and immunofluorescence. A test sample from a subject is assessed for the presence of an alteration in the expression and/or an alteration in composition of the polypeptide encoded by a breast cancer-associated nucleic acid. An alteration in expression of a polypeptide encoded by a breast cancer-associated nucleic acid can be, for example, an alteration in the quantitative polypeptide expression (i.e., the amount of polypeptide produced). An alteration in the composition of a polypeptide encoded by a breast cancer-associated nucleic acid is an alteration in the qualitative polypeptide expression (e.g., expression of a mutant polypeptide or of a different splicing variant). In one embodiment, diagnosis of a susceptibility to breast cancer is made by detecting a particular splicing variant encoded by a nucleic acid associated with breast cancer, or a particular pattern of splicing variants.

Both such alterations (quantitative and qualitative) can also be present. An "alteration" in the polypeptide expression or composition, as used herein, refers to an alteration in expression or composition in a test sample, as compared to the expression or composition of polypeptide encoded by a breast cancer-associated nucleic acid in a control sample. A control sample is a sample that corresponds to the test sample (e.g., is from the same type of cells), and is from a subject who is not affected by, and/or who does not have a susceptibility to, breast cancer. In one embodiment, the control sample is from a subject that does not possess a marker allele or haplotype as described herein. Similarly, the presence of one or more different splicing variants in the test sample, or the presence of significantly different amounts of different splicing variants in the test sample, as compared with the control sample, can be indicative of a susceptibility to breast cancer. An alteration in the expression or composition of the polypeptide in the test sample, as compared with the control sample, can be indicative of a specific allele in the instance where the allele alters a splice site relative to the reference in the control sample. Various means of examining expression or composition of a polypeptide encoded by a breast cancer-associated nucleic acid can be used, including spectroscopy, colorimetry, electrophoresis, isoelectric focusing, and immunoassays (e.g., David et al., U.S. Pat. No. 4,376,110) such as immunoblotting (see, e.g., Current Protocols in Molecular Biology, particularly chapter 10, *supra).*

For example, in one embodiment, an antibody (e.g., an antibody with a detectable label) that is capable of binding to a polypeptide encoded by a breast cancer-associated nucleic acid can be used. Antibodies can be polyclonal or monoclonal. An intact antibody, or a fragment thereof (e.g., Fv, Fab, Fab', F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a labeled secondary antibody (e.g., a fluorescently-labeled secondary antibody) and end-labeling of a DNA probe with biotin such that it can be detected with fluorescently-labeled streptavidin.

In one embodiment of this method, the level or amount of polypeptide encoded by a nucleic acid associated with breast cancer (*e.g.*, a nucleic acid encoding the STEAP3/TSAP6, LOC643714 or TNRC9 genes) in a test sample is compared with the level or amount of the polypeptide in a control sample. A level or amount of the polypeptide in the test sample that is higher or lower than the level or amount of the polypeptide in the control sample, such that the difference is statistically significant, is indicative of an alteration in the expression of the polypeptide encoded by the nucleic acid, and is diagnostic for a particular allele or haplotype responsible for causing the difference in expression. Alternatively, the composition of the polypeptide in a test sample is compared with the composition of the polypeptide in a control sample. In another embodiment, both the level or amount and the composition of the polypeptide can be assessed in the test sample and in the control sample.

In another embodiment, the diagnosis of a susceptibility to breast cancer is made by detecting at least one marker or haplotype of the present invention (e.g., associated alleles of the markers and haplotypes of Table 10, Table 15, Table 19, Table 20, Table 21 and Table 22 and markers in linkage disequilibrium therewith) in combination with an additional protein-based, RNA-based or DNA-based assay. The methods of the invention can also be used in combination with an analysis of a subject's family history and risk factors (e.g., environmental risk factors, lifestyle risk factors).

### Kits

Kits useful in the methods of the invention comprise components useful in any of the methods described herein, including for example, hybridization probes, restriction enzymes (e.g., for RFLP analysis), allele-specific oligonucleotides, antibodies that bind to an altered polypeptide encoded by a nucleic acid of the invention as described herein (e.g., a genomic segment comprising at least one polymorphic marker and/or haplotype of the present invention) or to a non-altered (native) polypeptide encoded by a nucleic acid of the invention as described herein, means for amplification of a nucleic acid associated with breast cancer, means for analyzing the nucleic acid sequence of a nucleic acid associated with breast cancer, means for analyzing the amino acid sequence of a polypeptide encoded by a nucleic acid associated with breast cancer, etc. The kits can for example include necessary buffers, nucleic acid primers for amplifying nucleic acids of the invention (e.g., one or more of the polymorphic markers as described herein), and reagents for allele-specific detection of the fragments amplified using such primers and necessary enzymes (e.g., DNA polymerase). Additionally, kits can provide reagents for assays to be used in combination with the methods of the present invention, e.g., reagents for use with breast cancer diagnostic assays.

In one embodiment, the invention is a kit for assaying a sample from a subject to detect the presence of a breast cancer or a susceptibility breast cancer in a subject, wherein the kit comprises reagents necessary for selectively detecting at least one allele of at least one polymorphism of the present invention in the genome of the individual. In a particular embodiment, the reagents comprise at least one contiguous oligonucleotide that hybridizes to a fragment of the genome of the individual comprising at least one polymorphism of the present invention. In another embodiment, the reagents comprise at least one pair of oligonucleotides that hybridize to opposite strands of a genomic segment obtained from a subject, wherein each oligonucleotide primer pair is designed to selectively amplify a fragment of the genome of the individual that includes at least one polymorphism, wherein the polymorphism is selected from the group consisting of the polymorphisms as listed in Table 10, Table 15, Table 19, Table 20, Table 21 and Table 22, and polymorphic markers in linkage disequilibrium therewith. In yet another embodiment the fragment is at least 20 base pairs in size. Such oligonucleotides or nucleic acids (e.g., oligonucleotide primers) can be designed using portions of the nucleic acid sequence flanking polymorphisms (e.g., SNPs or microsatellites) that are indicative of breast cancer. In another embodiment, the kit comprises one or more labeled nucleic acids capable of allele-specific detection of one or more specific polymorphic markers or haplotypes associated with breast cancer, and reagents for detection of the label. Suitable labels include, e.g., a radioisotope, a fluorescent label, an enzyme label, an enzyme co-factor label, a magnetic label, a spin label, an epitope label.

In particular embodiments, the polymorphic marker or haplotype to be detected by the reagents of the kit comprises one or more markers, two or more markers, three or more markers, four or more markers or five or more markers selected from the group consisting of the markers in Table 10, Table 15, Table 19, Table 20, Table 21 and Table 22. In one embodiment, the marker to be detected is selected from the markers in Table 10, Table 15 or Table 19. In one embodiment, the marker to be detected is selected from the markers in Table 10. In another embodiment, the marker to be detected is selected from the markers in Table 15 In another embodiment, the marker to be detected is selected from the markers in Table 19. In a preferred embodiment, the marker to be detected is rs4848543, rs13387042 and/or rs3803662. Other preferred embodiments include those that include reagents for detecting marker rs4848543, those that include reagents for detecting marker rs13387042 and those that include reagents for detecting marker rs3803662. In another embodiment, the marker or haplotype to be detected comprises at least one marker from the group of markers in strong linkage disequilibrium, as defined by values of r² greater than 0.2, to at least one of the group of markers consisting of the markers listed in Table 10, Table 15, Table 19, Table 20, Table 21 and Table 22. In yet another embodiment, the marker or haplotype to be detected comprises at least one marker selected from the group of markers consisting of markers rs4848543, rs13387042 and rs3803662, and markers in linkage disequilibrium therewith.

In one of such embodiments, the presence of the marker or haplotype is indicative of a susceptibility (increased susceptibility or decreased susceptibility) to breast cancer. In another embodiment, the marker is rs4848543 allele A, rs13387042 allele A, and/or rs3803662 allele T, the presence of which is indicative of increased risk of breast cancer (e.g., All BC and/or MedPre breast cancer). In still another embodiment, the presence of the marker or haplotype is indicative of response to a breast cancer therapeutic agent. In another embodiment, the presence of the marker or haplotype is indicative of breast cancer prognosis. In yet another embodiment, the presence of the marker or haplotype is indicative of progress of breast cancer treatment. Such treatment may include intervention by surgery, medication, radiation therapy or by other means (e.g., lifestyle changes).

### Diagnosis of Breast Cancer associated with polymorphisms of the invention

Although the methods of diagnosis have been generally described in the context of diagnosing susceptibility to breast cancer, the methods can also be used to diagnose breast cancer associated with the polymorphic markers of the present invention. For example, an individual having breast cancer or risk factors associated with breast cancer can be assessed to determine whether the presence in the individual of a polymorphism or haplotype of the present invention could have been a contributing factor to breast cancer in the individual. In one embodiment, identification of breast cancer associated with the markers and/or haplotypes of the present invention facilitates treatment planning. For example, preventive treatment to minimize the occurrence of the individual developing breast cancer can be administered. Such preventive treatment can also include assessment of whether the individual is heterozygous or homozygous for the at-risk variants of the invention. In other embodiments of the invention, therapies can be designed and therapeutics selected to target the appropriate gene or protein associated with the polymorphism and/or haplotype of the present invention

In other embodiments, the invention pertains to a method for the diagnosis and identification of breast cancer associated with the polymorphisms of the invention in a subject, by identifying the presence of a polymorphic marker or haplotype of the invention, as described in detail herein. For example, the polymorphic markers and/or haplotypes described herein are found more frequently in subjects with breast cancer than in subjects not affected by breast cancer. Therefore, these markers and/or haplotypes have predictive value for diagnosing breast cancer. In one embodiment, the marker or haplotype having predictive value for detecting breast cancer comprises one or more markers selected from the group consisting of the markers in Table 10, Table 15, Table 19, Table 20, Table 21 and Table 22. In another embodiment, the marker having predictive value for diagnosing breast cancer is selected from markers rs4848543, rs13387042 and rs3803662, and markers in linkage disequilibrium therewith.

In another embodiment, the marker allele having predictive value for diagnosing breast cancer comprises rs4848543 allele A, rs13387042 allele A, and rs3803662 allele T, and markers in linkage disequilibrium therewith.

The methods described herein can thus be used to assess a sample from a subject for the presence or absence of a specific allele of a marker, or a haplotype; the presence or absence of a specific allele of a marker, or a haplotype, is indicative of a susceptibility to breast cancer.

In one embodiment of the invention, diagnosis of breast cancer associated with the polymorphisms and/or haplotypes of the present invention is made by detecting a polymorphism of the present invention. Particular polymorphisms are described herein (see, *e.g.,* Table 10, Table 15, Table 19, Table 20, Table 21 and Table 22, and markers in linkage disequilibrium therewith). A test sample of genomic DNA, RNA, or cDNA, is obtained from a subject having breast cancer to determine whether the disease is associated with one or more polymorphisms of the invention. The DNA, RNA or cDNA sample is then examined to determine whether a specific allele of a polymorphism or a specific haplotype of the invention is found to be present in the sample. If the nucleic acid sample is found to contain the specific allele of the polymorphism or the haplotype, then the presence of the allele or haplotype is indicative of breast cancer associated with the polymorphism and/or haplotype.

Methods known to the person skilled in the art, including but not limited to hybridization methods, such as Southern analysis, Northern analysis, quantitative PCR, *in situ* hybridization, restriction digestion or sequence analysis, can be used to detect the polymorphism.. Diagnosis of breast cancer associated with the polymorphisms of the invention can also be made by examining expression and/or composition of a polypeptide encoded by a nucleic acid associated with the polymorphisms of the invention, using a variety of methods, including enzyme linked immunosorbent assays (ELISAs), Western blots, immunoprecipitations and immunofluorescence, or other methods well known to the skilled person.

### Therapeutic agents

Variants of the present invention (e.g., the markers and/or haplotypes of the invention, e.g., the markers listed in any one of Table 10, Table 15 and Table 19) can be used to identify novel therapeutic targets for breast cancer. For example, genes containing, or in linkage disequilibrium with, variants (markers and/or haplotypes) associated with breast cancer, or their products (e.g., one or more of the STEAP3/TSAP6 gene, the LOC643714 gene, and/or the TNRC9 gene), as well as genes or their products that are directly or indirectly regulated by or interact with these variant genes or their products, can be targeted for the development of therapeutic agents to treat breast cancer. Therapeutic agents may comprise one or more of, for example, small non-protein and non-nucleic acid molecules, proteins, peptides, protein fragments, nucleic acids (DNA, RNA), PNA (peptide nucleic acids), or their derivatives or mimetics which can modulate the function and/or levels of the target genes or their gene products.

The nucleic acids and/or variants of the invention, or nucleic acids comprising their complementary sequence, may be used as antisense constructs to control gene expression in cells, tissues or organs. The methodology associated with antisense techniques is well known to the skilled artisan, and is described and reviewed in AntisenseDrug Technology: Principles, Strategies, and Applications, Crooke, ed., Marcel Dekker Inc., New York (2001). In general, antisense nucleic acid molecules are designed to be complementary to a region of mRNA expressed by a gene, so that the antisense molecule hybridizes to the mRNA, thus blocking translation of the mRNA into protein. Several classes of antisense oligonucleotide are known to those skilled in the art, including cleavers and blockers. The former bind to target RNA sites, activate intracellular nucleases (e.g., RnaseH or Rnase L), that cleave the target RNA. Blockers bind to target RNA, inhibit protein translation by steric hindrance of the ribosomes. Examples of blockers include nucleic acids, morpholino compounds, locked nucleic acids and methylphosphonates (Thompson, Drug Discovery Today, 7:912-917 (2002)). Antisense oligonucleotides are useful directly as therapeutic agents, and are also useful for determining and validating gene function, for example by gene knock-out or gene knock-down experiments. Antisense technology is further described in Lavery et al., Curr. Opin. Drug Discov. Devel. 6:561-569 (2003), Stephens et al., Curr. Opin. Mol. Ther. 5:118-122 (2003), Kurreck, Eur. J. Biochem. 270:1628-44 (2003), Dias et al., Mol. Cancer Ter. 1:347-55 (2002), Chen, Methods Mol. Med. 75:621-636 (2003), Wang et al., Curr. Cancer Drug Targets 1:177-96 (2001), and Bennett, Antisense Nucleic Acid Drug.Dev. 12:215-24 (2002)

The variants described herein can be used for the selection and design of antisense reagents that are specific for particular variants. Using information about the variants described herein, antisense oligonucleotides or other antisense molecules that specifically target mRNA molecules that contain one or more variants of the invention can be designed. In this manner, expression of mRNA molecules that contain one or more variant of the present invention (markers and/or haplotypes) can be inhibited or blocked. In one embodiment, the antisense molecules are designed to specifically bind a particular allelic form (i.e., one or several variants (alleles and/or haplotypes)) of the target nucleic acid, thereby inhibiting translation of a product originating from this specific allele or haplotype, but which do not bind other or alternate variants at the specific polymorphic sites of the target nucleic acid molecule.

As antisense molecules can be used to inactivate mRNA so as to inhibit gene expression, and thus protein expression, the molecules can be used to treat breast cancer. The methodology can involve cleavage by means of ribozymes containing nucleotide sequences complementary to one or more regions in the mRNA that attenuate the ability of the mRNA to be translated. Such mRNA regions include, for example, protein-coding regions, in particular protein-coding regions corresponding to catalytic activity, substrate and/or ligand binding sites, or other functional domains of a protein.

The phenomenon of RNA interference (RNAi) has been actively studied for the last decade, since its original discovery in C. *elegans* (Fire et al.,Nature 391:806-11 (1998)), and in recent years its potential use in treatment of human disease has been actively pursued (reviewed in Kim & Rossi, Nature Rev. Genet. 8:173-204 (2007)). RNA interference (RNAi), also called gene silencing, is based on using double-stranded RNA molecules (dsRNA) to turn off specific genes. In the cell, cytoplasmic double-stranded RNA molecules (dsRNA) are processed by cellular complexes into small interfering RNA (siRNA). The siRNA guide the targeting of a protein-RNA complex to specific sites on a target mRNA, leading to cleavage of the mRNA (Thompson, Drug Discovery Today, 7:912-917 (2002)). The siRNA molecules are typically about 20, 21, 22 or 23 nucleotides in length. Thus, one aspect of the invention relates to isolated nucleic acid molecules, and the use of those molecules for RNA interference, i.e. as small interfering RNA molecules (siRNA). In one embodiment, the isolated nucleic acid molecules are 18-26 nucleotides in length, preferably 19-25 nucleotides in length, more preferably 20-24 nucleotides in length, and more preferably 21, 22 or 23 nucleotides in length.

Another pathway for RNAi-mediated gene silencing originates in endogenously encoded primary microRNA (pri-miRNA) transcripts, which are processed in the cell to generate precursor miRNA (pre-miRNA). These miRNA molecules are exported from the nucleus to the cytoplasm, where they undergo processing to generate mature miRNA molecules (miRNA), which direct translational inhibition by recognizing target sites in the 3' untranslated regions of mRNAs, and subsequent mRNA degradation by processing P-bodies (reviewed in Kim & Rossi, Nature Rev. Genet. 8:173-204 (2007)).

Clinical applications of RNAi include the incorporation of synthetic siRNA duplexes, which preferably are approximately 20-23 nucleotides in size, and preferably have 3' overlaps of 2 nucleotides. Knockdown of gene expression is established by sequence-specific design for the target mRNA. Several commercial sites for optimal design and synthesis of such molecules are known to those skilled in the art.

Other applications provide longer siRNA molecules (typically 25-30 nucleotides in length, preferably about 27 nucleotides), as well as small hairpin RNAs (shRNAs; typically about 29 nucleotides in length). The latter are naturally expressed, as described in Amarzguioui et al. (FEBS Lett. 579:5974-81 (2005)). Chemically synthetic siRNAs and shRNAs are substrates for *in* vivo processing, and in some cases provide more potent gene-silencing than shorter designs (Kim et al., Nature Biotechnol. 23:222-226 (2005); Siolas et al., Nature Biotechnol. 23:227-231 (2005)). In general siRNAs provide for transient silencing of gene expression, because their intracellular concentration is diluted by subsequent cell divisions. By contrast, expressed shRNAs mediate long-term, stable knockdown of target transcripts, for as long as transcription of the shRNA takes place (Marques et al., Nature Biotechnol. 23:559-565 (2006); Brummelkamp et al., Science 296: 550-553 (2002)).

Since RNAi molecules, including siRNA, miRNA and shRNA, act in a sequence-dependent manner, the variants of the present invention (e.g., the markers and haplotypes set forth in Table 10, Table 15 and Table 19) can be used to design RNAi reagents that recognize specific nucleic acid molecules comprising specific alleles and/or haplotypes (e.g., the alleles and/or haplotypes of the present invention), while not recognizing nucleic acid molecules comprising other alleles or haplotypes. These RNAi reagents can thus recognize and destroy the target nucleic acid molecules. As with antisense reagents, RNAi reagents can be useful as therapeutic agents (i.e., for turning off disease-associated genes or disease-associated gene variants), but may also be useful for characterizing and validating gene function (e.g., by gene knock-out or gene knock-down experiments).

Delivery of RNAi may be performed by a range of methodologies known to those skilled in the art. Methods utilizing non-viral delivery include cholesterol, stable nucleic acid-lipid particle (SNALP), heavy-chain antibody fragment (Fab), aptamers and nanoparticles. Viral delivery methods include use of lentivirus, adenovirus and adeno-associated virus. The siRNA molecules are in some embodiments chemically modified to increase their stability. This can include modifications at the 2' position of the ribose, including 2'-O-methylpurines and 2'-fluoropyrimidines, which provide resistance to Rnase activity. Other chemical modifications are possible and known to those skilled in the art.

The following references provide a further summary of RNAi, and possibilities for targeting specific genes using RNAi: Kim & Rossi, Nat. Rev. Genet. 8:173-184 (2007), Chen & Rajewsky, Nat. Rev. Genet. 8: 93-103 (2007), Reynolds, et al., Nat. Biotechnol. 22:326-330 (2004), Chi et al., Proc. Natl. Acad. Sci. USA 100:6343-6346 (2003), Vickers et al., J. Biol. Chem. 278:7108-7118 (2003), Agami, Curr. Opin. Chem. Biol. 6:829-834 (2002), Lavery, et al., Curr. Opin. Drug Discov. Devel. 6:561-569 (2003), Shi, Trends Genet. 19:9-12 (2003), Shuey et al., Drug Discov. Today 7:1040-46 (2002), McManus et al., Nat. Rev. Genet. 3:737-747 (2002), Xia et al., Nat. Biotechnol. 20:1006-10 (2002), Plasterk et al., curr. Opin. Genet. Dev. 10:562-7 (2000), Bosher et al., Nat. Cell Biol. 2:E31-6 (2000), and Hunter, Curr. Biol. 9:R440-442 (1999).

A genetic defect leading to increased predisposition or risk for development of breast cancer, or a genetic defect causing breast cancer, may be corrected permanently by administering to a subject carrying the defect a nucleic acid fragment that incorporates a repair sequence that supplies the normal/wild-type nucleotide(s) at the site of the genetic defect. Such site-specific repair sequence may concompass an RNA/DNA oligonucleotide that operates to promote endogenous repair of a subject's genomic DNA. The administration of the repair sequence may be performed by an appropriate vehicle, such as a complex with polyethelenimine, encapsulated in anionic liposomes, a viral vector such as an adenovirus vector, or other pharmaceutical compositions suitable for promoting intracellular uptake of the adminstered nucleic acid. The genetic defect may then be overcome, since the chimeric oligonucleotides induce the incorporation of the normal sequence into the genome of the subject, leading to expression of the normal/wild-type gene product. The replacement is propagated, thus rendering a permanent repair and alleviation of the symptoms associated with the disease or condition.

The present invention provides methods for identifying compounds or agents that can be used to treat and/or prevent breast cancer. Thus, the variants of the invention are useful as targets for the identification and/or development of therapeutic agents. In certain embodiments, such methods include assaying the ability of an agent or compound to modulate the activity and/or expression of a nucleic acid that includes at least one of the variants (markers and/or haplotypes) of the present invention, or the encoded product of the nucleic acid, for example one or more of the STEAP3/TSAP6 gene, the LOC643714 gene, and/or the TNRC9 gene. This in turn can be used to identify agents or compounds that inhibit or alter the undesired activity or expression of the encoded nucleic acid product. Assays for performing such experiments can be performed in cell-based systems or in cell-free systems, as known to the skilled person. Cell-based systems include cells naturally expressing the nucleic acid molecules of interest, or recombinant cells that have been genetically modified so as to express a certain desired nucleic acid molecule.

Variant gene expression in a patient can be assessed by expression of a variant-containing nucleic acid sequence (for example, a gene containing at least one variant of the present invention, which can be transcribed into RNA containing the at least one variant, and in turn translated into protein), or by altered expression of a normal/wild-type nucleic acid sequence due to variants affecting the level or pattern of expression of the normal transcripts, for example variants in the regulatory or control region of the gene. Assays for gene expression include direct nucleic acid assays (mRNA), assays for expressed protein levels, or assays of collateral compounds involved in a pathway, for example a signal pathway. Furthermore, the expression of genes that are up- or down-regulated in response to the signal pathway can also be assayed. One embodiment includes operably linking a reporter gene, such as luciferase, to the regulatory region of the gene(s) of interest.

Modulators of gene expression can in one embodiment be identified when a cell is contacted with a candidate compound or agent, and the expression of mRNA is determined. The expression level of mRNA in the presence of the candidate compound or agent is compared to the expression level in the absence of the compound or agent. Based on this comparison, candidate compounds or agents for treating and/or preventing breast cancer can be identified as those modulating the gene expression of the variant gene. When expression of mRNA or the encoded protein is statistically significantly greater in the presence of the candidate compound or agent than in its absence, then the candidate compound or agent is identified as a stimulator or up-regulator of expression of the nucleic acid. When nucleic acid expression or protein level is statistically significantly less in the presence of the candidate compound or agent than in its absence, then the candidate compound is identified as an inhibitor or down-regulator of the nucleic acid expression.

The invention further provides methods of treatment using a compound identified through drug (compound and/or agent) screening as a gene modulator (i.e. stimulator and/or inhibitor of gene expression).

### Methods of assessing probability of resonse to therapeutic agents, methods of monitoring progress of treatment and methods for treating Breast Cancer

As is known in the art, individuals can have differential responses to a particular therapy (e.g., a therapeutic agent or therapeutic method, as described further herein). The basis of the differential response may be genetically determined in part. Pharmacogenomics addresses the issue of how genetic variations (e.g., the variants (markers and/or haplotypes) of the present invention) affect drug response, due to altered drug disposition and/or abnormal or altered action of the drug. Thus, the basis of the differential response may be genetically determined in part. Clinical outcomes due to genetic variations affecting drug response may result in toxicity of the drug in certain individuals (e.g., carriers or non-carriers of the genetic variants of the present invention), or therapeutic failure of the drug. Therefore, the variants of the present invention may determine the manner in which a therapeutic agent and/or method acts on the body, or the way in which the body metabolizes the therapeutic agent.

Accordingly, in one embodiment, the presence of a particular allele at a polymorphic site or haplotype is indicative of a different response rate to a particular treatment modality. This means that a patient diagnosed with breast cancer, and carrying a certain allele at a polymorphic or haplotype of the present invention (e.g., the at-risk and protective alleles and/or haplotypes of the invention) would respond better to, or worse to, a specific therapeutic, drug and/or other therapy used to treat the disease. Therefore, the presence or absence of the marker allele or haplotype could aid in deciding what treatment should be used for a the patient. For example, for a newly diagnosed patient, the presence of a marker or haplotype of the present invention may be assessed (e.g., through testing DNA derived from a blood sample, as described herein). If the patient is positive for a marker allele or haplotype at (that is, at least one specific allele of the marker, or haplotype, is present), then the physician recommends one particular therapy, while if the patient is negative for the at least one allele of a marker, or a haplotype, then a different course of therapy may be recommended (which may include recommending that no immediate therapy, other than serial monitoring for progression of the disease, be performed). Thus, the patient's carrier status could be used to help determine whether a particular treatment modality should be administered. The value lies within the possibilities of being able to diagnose the disease at an early stage, to select the most appropriate treatment, and provide information to the clinician about prognosis/aggressiveness of the disease in order to be able to apply the most appropriate treatment.

As described further herein, current clinical preventive options for breast cancer are mainly chemopreventive (chemotherapy, or hormonal therapy) and prophylactic surgery. The most common chemopreventive is Tamoxifen and Raloxifene; other options include the aromatase inihibitors. Treatment options also include radiation therapy, for which a proportion of patients experience adverse symptoms. The markers of the invention, as described herein, may be used to assess response to these therapeutic options, or to predict the progress of therapy using any one of these treatment options. Thus, genetic profiling can be used to select the appropriate treatement strategy based on the genetic status of the individual, or it may be used to predict the outcome of the particular treatment option, and thus be useful in the strategic selection of treatment options or a combination of available treatment options.

The present invention also relates to methods of monitoring progress or effectiveness of a treatment for a breast cancer. This can be done based on the genotype and/or haplotype status of the markers and haplotypes of the present invention, i.e., by assessing the absence or presence of at least one allele of at least one polymorphic marker as disclosed herein, or by monitoring expression of genes that are associated with the variants (markers and haplotypes) of the present invention. The risk gene mRNA or the encoded polypeptide can be measured in a tissue sample (e.g., a peripheral blood sample, or a biopsy sample). Expression levels and/or mRNA levels can thus be determined before and during treatment to monitor its effectiveness. Alternatively, or concomitantly, the genotype and/or haplotype status of at least one risk variant for breast cancer as presented herein is determined before and during treatment to monitor its effectiveness.

Alternatively, biological networks or metabolic pathways related to the markers and haplotypes of the present invention can be monitored by determining mRNA and/or polypeptide levels. This can be done for example, by monitoring expression levels or polypeptides for several genes belonging to the network and/or pathway, in samples taken before and during treatment. Alternatively, metabolites belonging to the biological network or metabolic pathway can be determined before and during treatment. Effectiveness of the treatment is determined by comparing observed changes in expression levels/metabolite levels during treatment to corresponding data from healthy subjects.

In a further aspect, the markers of the present invention can be used to increase power and effectiveness of clinical trials. Thus, individuals who are carriers of the at-risk variants of the present invention, i.e. individuals who are carriers of at least one allele of at least one polymorphic marker conferring increased risk of developing breast cancer may be more likely to respond to a particular treatment modality. In one embodiment, individuals who carry at-risk variants for gene(s) in a pathway and/or metabolic network for which a particular treatment (e.g., small molecule drug) is targeting, are more likely to be responders to the treatment. In another embodiment, individuals who carry at-risk variants for a gene, which expression and/or function is altered by the at-risk variant, are more likely to be responders to a treatment modality targeting that gene, its expression or its gene product.

In a further aspect, the markers and haplotypes of the present invention can be used for targeting the selection of pharmaceutical agents for specific individuals. Personalized selection of treatment modalities, lifestyle changes or combination of the two, can be realized by the utilization of the at-risk variants of the present invention. Thus, the knowledge of an individual's status for particular markers of the present invention, can be useful for selection of treatment options that target genes or gene products affected by the at-risk variants of the invention. Certain combinations of variants may be suitable for one selection of treatment options, while other gene variant combinations may target other treatment options. Such combination of variant may include one variant, two variants, three variants, or four or more variants, as needed to determine with clinically reliable accuracy the selection of treatment module.

### Computer-implemented aspects

The present invention also relates to computer-implemented applications of the polymorphic markers and haplotypes described herein to be associated with breast cancer. Such applications can be useful for storing, manipulating or otherwise analyzing genotype data that is useful in the methods of the invention, as described herein. One example pertains to storing genotype information derived from an individual on readable media, so as to be able to provide the genotype information to a third party *(e.g.,* the individual), or for deriving information from the genotype data, *e.g.,* by comparing the genotype data to information about genetic risk factors contributing to increased susceptibility to breast cancer, and reporting results based on such comparison.

One such aspect relates to computer-readable media. In general terms, such medium has capabilities of storing (i) identifer information for at least one polymorphic marker or a haplotye; (ii) an indicator of the frequency of at least one allele of said at least one marker, or the frequency of a haplotype, in individuals with breast cancer; and an indicator of the frequency of at least one allele of said at least one marker, or the frequency of a haplotype, in a reference population. The reference population can be a disease-free population of individuals. Alternatively, the reference population is a random sample from the general population, and is thus representativ of the population at large. The frequency indicator may be a calculated frequency, a count of alleles and/or haplotype copies, or normalized or otherwise manipulated values of the actual frequencies that are suitable for the particular medium.

Additional information about the individual can be stored on the medium, such as ancestry information, information about sex, physical attributes or charactersitics (including height and weight), biochemical measurements, treatment administration, treatment outcome, medication, or other useful information that is desirable to store or manipulate in the context of the genotype status of a particular individual.

The invention furthermore relates to an apparatus that is suitable for determination or manipulation of genetic data useful for determining a susceptibility to breast cancer in a human individual. Such an apparatus can include a computer-readable memory, a routine for manipulating data stored on the computer-readable memory, and a routine for generating an output that includes a measure of the genetic data. Such measure can include values such as allelic or haplotype frequencies, genotype counts, sex, age, phenotype information, values for odds ratio (OR) or relative risk (RR), population attributable risk (PAR), or other useful information that is either a direct statistic of the original genotype data or based on calculations based on the genetic data.

The markers and haplotypes shown herein to be associated with increased susceptibility (e.g., increased risk) of breast cancer, are in certain embodiments useful for interpretation and/or analysis of genotype data. Thus in certain embodiments, an identification of an at-risk allele or an at-risk haplotype for breast cancer, as shown herein, or an allele at a polymorphic marker or a haplotype in LD with any one of the markers and/or haplotypes shown herein to be associated with breast cancer, is indicative of the individual from whom the genotype data originates is at increased risk of breast cancer. In one such embodiment, genotype data is generated for at least one polymorphic marker shown herein to be associated with breast cancer, or a marker in linkage disequilibrium therewith. The genotype data is subsequently made available to a third party, such as the individual from whom the data originates, for example via a user interface accessable over the internet, together with an interpretation of the genotype data, e.g., in the form of a risk measure (such as an absolute risk (AR), risk ratio (RR) or odds ration (OR)) for the disease (e.g., breast cancer. In another embodiment, at-risk markers identified in a genotype dataset derived from an individual are assessed and results from the assessment of the risk conferred by the presence of such at-risk varians in the dataset are made available to the individual, for example via a secure web interface, or by other communication means. The results of such risk assessment can be reported in numeric form (e.g., by risk values, such as absolute risk, relative risk, and/or an odds ratio, or by a percentage increase in risk compared with a reference), by graphical means, or by other means suitable to illustrate the risk to the individual from whom the genotype data is derived. In particular embodiments, the results of risk assessment is made available to a third party, e.g., a physician, other healthcare worker or genetic counselor.

### Markers useful in various aspects of the invention

The above-described methods and applications can all be practiced with the markers and haplotypes of the invention that have in more detail been described herein in general terms as being useful for assessing susceptibility to breast cancer. Thus, these applications can in general be reduced to practice using markers as set forth in any of the Tables 10, 15, 19, 20, 21 and 22, and markers in linkage disequilibrium therewith. In certain embodiments, the markers or haplotypes are present within the genomic segments whose sequences are set forth in SEQ ID NO:4, SEQ ID NO:5 or SEQ ID NO:6. In certain embodiments, the markers are present within the rs4848543 LD block, the rs13387042 LD block or the rs3803662 LD block. In particular embodiments, the markers are selected from the markers set forth in Table 10. In other embodiments, the markers are selected from the markers set forth in Table 15. In other embodiments, the markers are selected from the markers set forthin in Table 19. In some embodiments, the markers are selected from the markers set forth in Tables 7 and 8. In some other embodiments, the markers are selected from the markers set forth in Table 14. In some other embodiments, the markers are selected from the markers set forth in Table 18. In other embodiments, the markers are one of rs4848543 (SEQ ID NO:1), rs13387042 (SEQ ID NO:2), or rs3803662 (SEQ ID NO:3), optionally including markers in linkage disequlibrium therewith. In one embodiment, the markers are one of rs4848543 (SEQ ID NO: 1), rs13387042 (SEQ ID NO:2), and rs3803662 (SEQ ID NO:3). In certain preferred embodiments, the marker is rs4848543 (SEQ ID NO: 1). In other preferred embodiments, the marker is rs13387042 (SEQ ID NO:2). In other preferred embodiments, the marker is rs3803662 (SEQ ID NO:3). In particular embodiments, linkage disequilibrium is defined by numerical values for r² of greater than 0.2. In another embodiment, the marker or haplotype comprises at least one marker selected from rs4848543 allele A, rs13387042 allele A, and rs3803662 allele T.

### Nucleic acids and polypeptides

The nucleic acids and polypeptides described herein can be used in methods and kits of the present invention, as described in the above. An "isolated" nucleic acid molecule, as used herein, is one that is separated from nucleic acids that normally flank the gene or nucleotide sequence (as in genomic sequences) and/or has been completely or partially purified from other transcribed sequences (e.g., as in an RNA library). For example, an isolated nucleic acid of the invention can be substantially isolated with respect to the complex cellular milieu in which it naturally occurs, or culture medium when produced by recombinant techniques, or chemical precursors or other chemicals when chemically synthesized. In some instances, the isolated material will form part of a composition (for example, a crude extract containing other substances), buffer system or reagent mix. In other circumstances, the material can be purified to essential homogeneity, for example as determined by polyacrylamide gel electrophoresis (PAGE) or column chromatography (e.g., HPLC). An isolated nucleic acid molecule of the invention can comprise at least about 50%, at least about 80% or at least about 90% (on a molar basis) of all macromolecular species present. With regard to genomic DNA, the term "isolated" also can refer to nucleic acid molecules that are separated from the chromosome with which the genomic DNA is naturally associated. For example, the isolated nucleic acid molecule can contain less than about 250 kb, 200 kb, 150 kb, 100 kb, 75 kb, 50 kb, 25 kb, 10 kb, 5 kb, 4 kb, 3 kb, 2 kb, 1 kb, 0.5 kb or 0.1 kb of the nucleotides that flank the nucleic acid molecule in the genomic DNA of the cell from which the nucleic acid molecule is derived.

The nucleic acid molecule can be fused to other coding or regulatory sequences and still be considered isolated. Thus, recombinant DNA contained in a vector is included in the definition of "isolated" as used herein. Also, isolated nucleic acid molecules include recombinant DNA molecules in heterologous host cells or heterologous organisms, as well as partially or substantially purified DNA molecules in solution. "Isolated" nucleic acid molecules also encompass *in vivo* and *in vitro* RNA transcripts of the DNA molecules of the present invention. An isolated nucleic acid molecule or nucleotide sequence can include a nucleic acid molecule or nucleotide sequence that is synthesized chemically or by recombinant means. Such isolated nucleotide sequences are useful, for example, in the manufacture of the encoded polypeptide, as probes for isolating homologous sequences (e.g., from other mammalian species), for gene mapping (e.g., by *in situ* hybridization with chromosomes), or for detecting expression of the gene in tissue (e.g., human tissue), such as by Northern blot analysis or other hybridization techniques.

The invention also pertains to nucleic acid molecules that hybridize under high stringency hybridization conditions, such as for selective hybridization, to a nucleotide sequence described herein (e.g., nucleic acid molecules that specifically hybridize to a nucleotide sequence containing a polymorphic site associated with a marker or haplotype described herein). Such nucleic acid molecules can be detected and/or isolated by allele- or sequence-specific hybridization (e.g., under high stringency conditions). Stringency conditions and methods for nucleic acid hybridizations are well known to the skilled person (see, *e.g.,* Current Protocols in Molecular Biology, Ausubel, F. et al, John Wiley & Sons, (1998), and Kraus, M. and Aaronson, S., Methods Enzymol., 200:546-556 (1991), the entire teachings of which are incorporated by reference herein.

The percent identity of two nucleotide or amino acid sequences can be determined by aligning the sequences for optimal comparison purposes (e.g., gaps can be introduced in the sequence of a first sequence). The nucleotides or amino acids at corresponding positions are then compared, and the percent identity between the two sequences is a function of the number of identical positions shared by the sequences (i.e., % identity = # of identical positions/total # of positions x 100). In certain embodiments, the length of a sequence aligned for comparison purposes is at least 30%, at least 40%, at least 50%, at least 60%, at least 70%, at least 80%, at least 90%, or at least 95%, of the length of the reference sequence. The actual comparison of the two sequences can be accomplished by well-known methods, for example, using a mathematical algorithm. A non-limiting example of such a mathematical algorithm is described in Karlin, S. and Altschul, S., Proc. Natl. Acad. Sci. USA, 90:5873-5877 (1993). Such an algorithm is incorporated into the NBLAST and XBLAST programs (version 2.0), as described in Altschul, S. et al., Nucleic Acids Res., 25:3389-3402 (1997). When utilizing BLAST and Gapped BLAST programs, the default parameters of the respective programs (e.g., NBLAST) can be used. See the website on the world wide web at ncbi.nlm.nih.gov. In one embodiment, parameters for sequence comparison can be set at score=100, wordlength=12, or can be varied (e.g., W=5 or W=20).

Other examples include the algorithm of Myers and Miller, CABIOS (1989), ADVANCE and ADAM as described in Torellis, A. and Robotti, C., Comput. Appl. Biosci. 10:3-5 (1994); and FASTA described in Pearson, W. and Lipman, D., Proc. Natl. Acad. Sci. USA, 85:2444-48 (1988). In another embodiment, the percent identity between two amino acid sequences can be accomplished using the GAP program in the GCG software package (Accelrys, Cambridge, UK).

The present invention also provides isolated nucleic acid molecules that contain a fragment or portion that hybridizes under highly stringent conditions to a nucleic acid that comprises, or consists of, a nucleotide sequence comprising the polymorphic markers listed Table 10, Table 15, Table 19, Table 20, Table 21 and Table 22, and markers in linkage disequilibrium therewith, and the nucleotide sequence of the STEAP3/TSAP6, LOC643714 and TNRC9 genes, or a nucleotide sequence comprising, or consisting of, the complement of the nucleotide sequence of a nucleotide sequence comprising the polymorphic markers listed in Table 10, Table 15, Table 19, Table 20, Table 21 and Table 22, and markers in linkage disequilibrium therewith, and the nucleotide sequence of the STEAP3/TSAP6, LOC643714 and TNRC9 genes, or fragments thereof , wherein the nucleotide sequence comprises at least one polymorphic allele contained in the markers and haplotypes described herein. The nucleic acid fragments of the invention are at least about 15, at least about 18, 20, 23 or 25 nucleotides, and can be 30, 40, 50, 100, 200, 500, 1000, 10,000 or more nucleotides in length.

The nucleic acid fragments of the invention are used as probes or primers in assays such as those described herein. "Probes" or "primers" are oligonucleotides that hybridize in a base-specific manner to a complementary strand of a nucleic acid molecule. In addition to DNA and RNA, such probes and primers include polypeptide nucleic acids (PNA), as described in Nielsen, P. et al., Science 254:1497-1500 (1991). A probe or primer comprises a region of nucleotide sequence that hybridizes to at least about 15, typically about 20-25, and in certain embodiments about 40, 50 or 75, consecutive nucleotides of a nucleic acid molecule. In one embodiment, the probe or primer comprises at least one allele of at least one polymorphic marker or at least one haplotype described herein, or the complement thereof. In particular embodiments, a probe or primer can comprise 100 or fewer nucleotides; for example, in certain embodiments from 6 to 50 nucleotides, or, for example, from 12 to 30 nucleotides. In other embodiments, the probe or primer is at least 70% identical, at least 80% identical, at least 85% identical, at least 90% identical, or at least 95% identical, to the contiguous nucleotide sequence or to the complement of the contiguous nucleotide sequence. In another embodiment, the probe or primer is capable of selectively hybridizing to the contiguous nucleotide sequence or to the complement of the contiguous nucleotide sequence. Often, the probe or primer further comprises a label, e.g., a radioisotope, a fluorescent label, an enzyme label, an enzyme co-factor label, a magnetic label, a spin label, an epitope label.

The nucleic acid molecules of the invention, such as those described above, can be identified and isolated using standard molecular biology techniques well known to the skilled person. The amplified DNA can be labeled (e.g., radiolabeled) and used as a probe for screening a cDNA library derived from human cells. The cDNA can be derived from mRNA and contained in a suitable vector. Corresponding clones can be isolated, DNA can obtained following *in vivo* excision, and the cloned insert can be sequenced in either or both orientations by art-recognized methods to identify the correct reading frame encoding a polypeptide of the appropriate molecular weight. Using these or similar methods, the polypeptide and the DNA encoding the polypeptide can be isolated, sequenced and further characterized.

In general, the isolated nucleic acid sequences of the invention can be used as molecular weight markers on Southern gels, and as chromosome markers that are labeled to map related gene positions. The nucleic acid sequences can also be used to compare with endogenous DNA sequences in patients to identify breast cancer or a susceptibility to breast cancer, and as probes, such as to hybridize and discover related DNA sequences or to subtract out known sequences from a sample (e.g., subtractive hybridization). The nucleic acid sequences can further be used to derive primers for genetic fingerprinting, to raise anti-polypeptide antibodies using immunization techniques, and/or as an antigen to raise anti-DNA antibodies or elicit immune responses.

### An tibodies

Polyclonal antibodies and/or monoclonal antibodies that specifically bind one form of the gene product but not to the other form of the gene product are also provided. Antibodies are also provided which bind a portion of either the variant or the reference gene product that contains the polymorphic site or sites. The term "antibody" as used herein refers to immunoglobulin molecules and immunologically active portions of immunoglobulin molecules, i.e., molecules that contain antigen-binding sites that specifically bind an antigen. A molecule that specifically binds to a polypeptide of the invention is a molecule that binds to that polypeptide or a fragment thereof, but does not substantially bind other molecules in a sample, e.g., a biological sample, which naturally contains the polypeptide. Examples of immunologically active portions of immunoglobulin molecules include F(ab) and F(ab')₂ fragments which can be generated by treating the antibody with an enzyme such as pepsin. The invention provides polyclonal and monoclonal antibodies that bind to a polypeptide of the invention. The term "monoclonal antibody" or "monoclonal antibody composition", as used herein, refers to a population of antibody molecules that contain only one species of an antigen binding site capable of immunoreacting with a particular epitope of a polypeptide of the invention. A monoclonal antibody composition thus typically displays a single binding affinity for a particular polypeptide of the invention with which it immunoreacts.

Polyclonal antibodies can be prepared as described above by immunizing a suitable subject with a desired immunogen, *e.g.,* polypeptide of the invention or a fragment thereof. The antibody titer in the immunized subject can be monitored over time by standard techniques, such as with an enzyme linked immunosorbent assay (ELISA) using immobilized polypeptide. If desired, the antibody molecules directed against the polypeptide can be isolated from the mammal (e.g., from the blood) and further purified by well-known techniques, such as protein A chromatography to obtain the IgG fraction. At an appropriate time after immunization, e.g., when the antibody titers are highest, antibody-producing cells can be obtained from the subject and used to prepare monoclonal antibodies by standard techniques, such as the hybridoma technique originally described by Kohler and Milstein, Nature 256:495-497 (1975), the human B cell hybridoma technique (Kozbor et al., Immunol. Today 4: 72 (1983)), the EBV-hybridoma technique (Cole et al., Monoclonal Antibodies and Cancer Therapy, Alan R. Liss,1985, Inc., pp. 77-96) or trioma techniques. The technology for producing hybridomas is well known (see generally Current Protocols in Immunology (1994) Coligan et al., (eds.) John Wiley & Sons, Inc., New York, NY). Briefly, an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds a polypeptide of the invention.

Any of the many well known protocols used for fusing lymphocytes and immortalized cell lines can be applied for the purpose of generating a monoclonal antibody to a polypeptide of the invention (see, *e.g., Current Protocols in Immunology, supra;* Galfre et al., Nature 266:55052 (1977); R.H. Kenneth, in Monoclonal Antibodies: A New Dimension In Biological Analyses, Plenum Publishing Corp., New York, New York (1980); and Lerner, Yale J. Biol. Med. 54:387-402 (1981)). Moreover, the ordinarily skilled worker will appreciate that there are many variations of such methods that also would be useful.

Alternative to preparing monoclonal antibody-secreting hybridomas, a monoclonal antibody to a polypeptide of the invention can be identified and isolated by screening a recombinant combinatorial immunoglobulin library (e.g., an antibody phage display library) with the polypeptide to thereby isolate immunoglobulin library members that bind the polypeptide. Kits for generating and screening phage display libraries are commercially available (e.g., the Pharmacia *Recombinant Phage Antibody System,* Catalog No. 27-9400-01; and the Stratagene *Surf*ZAP™ Phage Display Kit, Catalog No. 240612). Additionally, examples of methods and reagents particularly amenable for use in generating and screening antibody display library can be found in, for example, U.S. Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al., Bio/Technology 9: 1370-1372 (1991); Hay et al., Hum. Antibod. Hybridomas 3:81-85 (1992); Huse et al., Science 246: 1275-1281 (1989); and Griffiths et al., EMBO J. 12:725-734 (1993).

Additionally, recombinant antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art.

In general, antibodies of the invention (e.g., a monoclonal antibody) can be used to isolate a polypeptide of the invention by standard techniques, such as affinity chromatography or immunoprecipitation. A polypeptide-specific antibody can facilitate the purification of natural polypeptide from cells and of recombinantly produced polypeptide expressed in host cells. Moreover, an antibody specific for a polypeptide of the invention can be used to detect the polypeptide (e.g., in a cellular lysate, cell supernatant, or tissue sample) in order to evaluate the abundance and pattern of expression of the polypeptide. Antibodies can be used diagnostically to monitor protein levels in tissue as part of a clinical testing procedure, e.g., to, for example, determine the efficacy of a given treatment regimen. The antibody can be coupled to a detectable substance to facilitate its detection. Examples of detectable substances include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Antibodies may also be useful in pharmacogenomic analysis. In such embodiments, antibodies against variant proteins encoded by nucleic acids according to the invention, such as variant proteins that are encoded by nucleic acids that contain at least one polymorpic marker of the invention, can be used to identify individuals that require modified treatment modalities.

Antibodies can furthermore be useful for assessing expression of variant proteins in disease states, such as in active stages of a disease, or in an individual with a predisposition to a disease related to the function of the protein, in particular breast cancer. Antibodies specific for a variant protein of the present invention that is encoded by a nucleic acid that comprises at least one polymorphic marker or haplotype as described herein can be used to screen for the presence of the variant protein, for example to screen for a predisposition to breast cancer as indicated by the presence of the variant protein.

Antibodies can be used in other methods. Thus, antibodies are useful as diagnostic tools for evaluating proteins, such as variant proteins of the invention, in conjunction with analysis by electrophoretic mobility, isoelectric point, tryptic or other protease digest, or for use in other physical assays known to those skilled in the art. Antibodies may also be used in tissue typing. In one such embodiment, a specific variant protein has been correlated with expression in a specific tissue type, and antibodies specific for the variant protein can then be used to identify the specific tissue type.

Subcellular localization of proteins, including variant proteins, can also be determined using antibodies, and can be applied to assess aberrant subcellular localization of the protein in cells in various tissues. Such use can be applied in genetic testing, but also in monitoring a particular treatment modality. In the case where treatment is aimed at correcting the expression level or presence of the variant protein or aberrant tissue distribution or developmental expression of the variant protein, antibodies specific for the variant protein or fragments thereof can be used to monitor therapeutic efficacy.

Antibodies are further useful for inhibiting variant protein function, for example by blocking the binding of a variant protein to a binding molecule or partner. Such uses can also be applied in a therapeutic context in which treatment involves inhibiting a variant protein's function. An antibody can be for example be used to block or competitively inhibit binding, thereby modulating (i.e., agonizing or antagonizing) the activity of the protein. Antibodies can be prepared against specific protein fragments containing sites required for specific function or against an intact protein that is associated with a cell or cell membrane. For administration in *vivo,* an antibody may be linked with an additional therapeutic payload, such as radionuclide, an enzyme, an immunogenic epitope, or a cytotoxic agent, including bacterial toxins (diphtheria or plant toxins, such as ricin). The *in vivo* half-life of an antibody or a fragment thereof may be increased by pegylation through conjugation to polyethylene glycol.

The present invention further relates to kits for using antibodies in the methods described herein. This includes, but is not limited to, kits for detecting the presence of a variant protein in a test sample. One preferred embodiment comprises antibodies such as a labelled or labelable antibody and a compound or agent for detecting variant proteins in a biological sample, means for determining the amount or the presence and/or absence of variant protein in the sample, and means for comparing the amount of variant protein in the sample with a standard, as well as instructions for use of the kit.

The present invention will now be exemplified by the following non-limiting example.

### EXEMPLIFICATION

**Patient and Control Selection:** Approval for this study was granted by the National Bioethics Committee of Iceland and the Icelandic Data Protection Authority. Records of breast cancer diagnoses were obtained from the Icelandic Cancer Registry (ICR). Records included all cases of invasive breast tumors and ductal or lobular carcinoma in-situ diagnosed in Iceland from 1 January 1955 to 31 December 2005. The ICR contained records of 4603 individuals diagnosed during this period. A prevalence cohort comprising all living patients (approximately 2840) were eligible for recruitment into the study. We obtained informed consent, a blood sample, and clinical information from 2210 patients, a yield of approximately 78%. The initial group of controls was comprised of all individuals from other ongoing deCODE Genetics genome-wide association studies and who had been genotyped on Illumina SNP chips by the time the breast cancer patient group had been genotyped. Individuals with entries in the Icelandic Cancer Registry indicating a diagnosis of breast, prostate or colorectal cancer up to the end of 2005 were excluded from the control group, resulting in a set of 4477 controls. An independent replication set of controls was comprised of a further 7406 individuals from non-breast cancer projects who were typed after the 4477 set. In this 7406 set, prostate and colorectal cancer patients were not excluded because we had obtained interim data indicating that the frequencies of the SNPs in question did not differ from controls in these patient samples. In each control sets there was no difference between genders in the frequencies of the SNPs listed in Tables 8,9, & 14 below. Therefore these control groups provided a reasonable representation of the population frequencies of the SNPs under investigation.

**Genealogical Database:** deCODE Genetics maintains a computerized database of the genealogy of Icelanders. The records include almost all individuals born in Iceland in the last two centuries and for that period around 95% of the parental connections are known [Sigurdardottir, et al., (2000), Am J Hum Genet, 66, 1599-609]. In addition, a county of residence identifier is recorded for most individuals, based on census and parish records. The information is stored in a relational database with encrypted personal identifiers that match those used on the biological samples and ICR records, allowing cross-referencing of the genotypes and phenotypes of the study participants with their genealogies.

**Sample Handling:** Blood samples were preserved in EDTA at -20°C. DNA was isolated from whole blood using a Qiagen (http://www.quiagen.com) extraction column method. DNA was stored at 4°C.

**Genotyping:** A set of 1600 patient samples was then randomly selected and genotyped on Illumina Infinium Hap300 SNP bead microarrays (Illumina, San Diego, CA, USA), containing 317,511 SNPs derived from Phase I of the International HapMap project. 4480 cancer-free control samples were genotyped on the same platform. Of the total number of SNPs on the chip, 170 generated no genotypes and an additional 24 SNPs had yields lower than 80%. An additional 61 SNPs showed very significant distortion from Hardy-Weinberg equilibrium in the controls (p < 1x10⁻¹⁰). 104 SNPs were monomorphic and 58 SNP's were nearly monomorphic (i.e. the minor allele frequency in the combined cohort of patients and controls was less than 0.001). Lastly, 5 markers were determined to have genotyping problems after investigation of particular regions and possible signals in several different on-going genomewide association studies inhouse. All of these problematic SNPs were removed from the analysis. Thus, the final analyses presented in the text utilizes 317,089 SNPs. Any chips with a call rate below 98% were also excluded from the analysis.

The sequence context of the three key SNPs examined in this study are given below:
rs4848543 (SEQ ID NO: 1): 
caggataagattatttctgaggctgcttccatcagtggttctgtgagctggcccatgtattcagccagtcagtaggccatcaagcatgcatct
acgcaaggcactagaagggttacatacaatgggaaactgaggcccagaaggaggaaagatgaagtgcccaaagttaaggagataaa
agcagaagtgcaggtaagagaaggagtattcatattactgcaggaatccaaattctacaagaacatacgcttgttctttaaagggagccc
tcgctttaagcattatttgccgatcaatacc[A/C]attaataattacaggacatgggcaaatattctagcaaaggtcctgaggtcttcctag
atgtgcccaaaataacaaggactccccccgcaggtggcaccaaaagtcggaggatgtgttggaagctgaccttccccacctgtccctgcc
tcacccacagtcacccacccctctaggatgtaatggtgaatcattcaacacatgggccaccttgggaagtcgcttaacctctctcccttttcac
gttataaaatgggcatagtcattacacaaatgcataaatcaacctcaattttctcatct
rs13387042 (SEQ ID NO:2): 
ctgacatagccgcctgtgcaggtttttgggaccaggtctgccatctgtattcccaggctgggctggagcacctgaggctactatggttacag
aaaggaacctggcatccgaaggtataggctcaaatcccagctctcatgattgctagctttgaggtttcaggttcatttcttaagtgctctgag
ctttggtgccctcctcttcaaaatggaatgaggagcatgtctagctccctgttttgttgcagtgaatagctacatgaagaacagctaaacc
agaacagaaagaaggcaaatgga[A/G]gctacagaaaccaaggatttccttgttgaatcgaatcttccttcaatcttccttcaccacact
agtggatctccctgtgggagggatgttgagagtgctccgtgtttttttgtttttttttttttttttgtgatggagtctcactctgtcacccaga
ctggaatgcagtggtgcgatctcggctcactgcaacctctgcctcccaggttcaagtgattctcctgcctcagcttcccaagtagccaggact
acaggcatatgccatcacacccagctaatttttgtatttttagtag
rs3803662 (SEQ ID NO:3):

gctagtccttggctgttctgtgatcaaatgcaattgtttcccatttcttaagaaacagaatctttattagactcagttcttttcttggttatttttac
aattttattgttctatggttattaaaaaataacatgtcatataaattaagcctagaaacatggatgtttatcaaagaaaggattgtcatccaa
agcaccaactatgagagatatctatgtgcaatggtatatagatctgtcatagaagggtttaattatatctgcctaatgatttctctccttaatg
cctctatagctgtc[C/T]cttagcgaagaataaaactgtggactgacccccacccatttgcgaagaaagtactgggtcttcagctttcattg
ttcagccggtggtctttgtggacaacaccaggggcccctcctgctgaagactgtctggacttcctgattttcactcctgtataaacccccctccc
ccattactacctttgtctccttggggaaaaaaaatgtttttcacttaagcaaaataataactacccagtatttgtttctggagtgaaattgtaa
acttcattgtacattcttcctggctttgatgcttatcg

We designed and tested assays to detect a number of SNPs in the rs4848543 LD block. These assays were based on the Centaurus SNP assay technology described in [Kutyavin, et al., (2006), Nucleic Acids Res, 34, e128]. The assays, and their primers were:
SG02S733
rs895398
(Build 34) chr2:120,058,180
Forward Primer: TTGGAACCTCCCACTGCCACA
Reverse Primer: ACAGCCCAGGTTTCAGGTTGGCAT
VIC-Probe: AGCGACACTG
FAM-Probe: GCGACACCG
Enhancer: CCTG*AGATCCAGGCA

SG02S738
rs4848543
(Build 34) chr2:120,061,096
Forward Primer: AGGGAGCCCTCGCTTTAAGCATTA
Reverse Primer: TTTGGGCACATCTAGGAAGACCTC
VIC-Probe: GAT*CAATACCC
FAM-Probe: CGAT*CAATACCA
Enhancer: TTAATAATTACAGGACATG

SG02S739
rs6759589
(Build 34) chr2:120,064,976
Forward Primer: TCAGCAGCCTCTTGTCTCACTAAT
Reverse Primer: CTTCTTGCCCAGGCAGGACAGA
VIC-Probe: TAGACTGAGCC
FAM-Probe: AAT*AGACTAAGCC
Enhancer: CCATCCCCAACTAA

SG02S753
rs895397
(Build 34) chr2:120,072,400

Forward Primer: CACTACTAGATCATGGGAGATGCCT
Reverse Primer: GTTTCCTCAGTTGTAATGTAGACC
VIC-Probe: TT*T*A*ACCCTT
FAM-Probe: GTTTTAACCCCT
Enhancer: GCTGCTCGGAAG

SG02S740
rs838102
(Build 34) chr2:120,079,878
Forward Primer: GGTTTTGCATCCTTGCTCACTCACAT
Reverse Primer: GTGCCCTCTGCGTACTTGCGAAT
VIC-Probe: TACTGCCACC
FAM-Probe: CTTACTGCCACT
Enhancer: TGTCTCCTCACTC

SG02S741
rs838100
(Build 34) chr2:120,083,710
Forward Primer: ACTTACCAACAGCAGGCTGGTG
Reverse Primer: CCAGAAAGGGTGGCTCACCTCA
VIC-Probe: GTCTGAGGCT
FAM-Probe: GGTCTGAGGTT
Enhancer: TCAGGTCACTCG

SG02S734
rs12711924
(Build 34) chr2:120,107,316
Forward Primer: AACGCTTATCACAGAGCCAGGTAG
Reverse Primer: GGAAAACCATGAACACTGTGGCAA
VIC-Probe: CCCGCCGT
FAM-Probe: GCCCGCCAT
Enhancer: TTGTCTTTCTTGTGTC

SG02S742
rs3731603
(Build 34) chr2:120,117,062
Forward Primer: AACA*CTCTAGGGAGTAAAGCTC
Reverse Primer: GAGGA*CATTTGGAGTCCCCAAT
VIC-Probe: GACTGTCTTCAA
FAM-Probe: ACTGT*CT*TCAC
Enhancer: AAGTCAGTCCTGAG

SG02S728
rs13387042
(Build 34) chr2:218,108,374
Forward Primer: CCACTAGTGTGGTGA*AGGAAGATT
Reverse Primer: GCTACATGA*AGAA*CAGCTAAACC
VIC-Probe: TTTCTGTAGCCT
FAM-Probe: TT*T*CTGTAGCT*T
Enhancer: CATTTGCCTTCTTTC
* indicates a modified base, as described [Kutyavin, et al., (2006), Nucleic Acids Res, 34, e128].

A single founder mutation of the BRCA2 gene (999del5) is present at a carrier frequency of 0.6%-0.8% in the general Icelandic population and 7.7%-8.6% in female breast cancer patients [Thorlacius, et al., (1997), Am J Hum Genet, 60, 1079-84; Gudmundsson, et al., (1996), Am J Hum Genet, 58, 749-56]. The possible presence of BRCA2 999del5 mutations was determined in 1499 of the cases using a microsatellite-type PCR assay, designated DG13S3727. Primers used were
Forward TGTGAAAAGCTATTTTTCCAATC
Reverse: ATCACGGGTGACAGAGCAA

The sequence amplified by DG13S3727 is:
DG13S3727 location=chr13:30703058-30703261

tgtgaaaagctatttttccaatcatgatgaaagtctgaagaaaaatgata

gatttatcgcttctgtgacagacagtgaaaacacaaatcaaagagaagct

gcaagtcatggtaagtcctctgtttagttgaactacaggtttttttgttg

ttgttgttttgatttttttttttgaggtggagtcttgctctgtcacccg

tgat

**Statistical Methods:** Assuming the multiplicative model, we calculate the relative risk (RR) of a SNP allele as RR=[p/(1-p)]/[s/(1-s)] where p and s are the frequencies of the variant in the patients and in the controls respectively. P-values associated with RR's were calculated based on a standard likelihood ratio Chi-squared statistic. Confidence intervals were calculated assuming that the estimate of RR has a log-normal distribution.

Haplotype frequencies were estimated by maximum likelihood and tests of differences between cases and controls, performed using a generalized likelihood ratio test. The haplotype analysis program called NEMO, which stands for NEsted Models, was used to calculate all the haplotype results. To handle uncertainties with phase and missing genotypes, it is emphasized that NEMO does not use a common two-step approach to association tests, where haplotype counts are first estimated, possibly with the use of the EM algorithm, and then tests are performed treating the estimated counts as though they are true counts, a method that can sometimes be problematic and may require randomization to properly evaluate statistical significance. Instead, with NEMO, maximum likelihood estimates, likelihood ratios and p-values are computed directly for the observed data, and hence the loss of information due to uncertainty in phase and missing genotypes is automatically captured by the likelihood ratios.

Some Icelandic patients and controls are related, both within and between groups. To correct the association results for relatedness, we simulated genotypes by propagating alleles through the Icelandic genealogy. For each simulation we performed the case-control association test in the same way as for the real genotypes, i.e. the Chi-squared likelihood ratio test. We calculated the average of the Chi-squared statistics over N simulations (which is identical to the variance of the corresponding Z-score), which for independent individuals under the null hypothesis has to be 1, whereas for related individuals it is expected to be slightly larger. The correction for relatedness was then applied by dividing the Chi-square statistics in the real association by the average obtained from the simulations.

Joint analyses of multiple case-control replication groups were carried out using a Mantel-Haenszel model in which the groups were allowed to have different population frequencies for alleles, haplotypes and genotypes but were assumed to have common relative risks.

All P-values are reported as two-sided.

**Analysis of Multiple Primary Breast Tumors:** Diagnostic records of multiple primary breast cancer (MPBC) were obtained from the ICR. Primary tumors diagnosed in addition to the first breast cancer diagnosis were confirmed both clinically and by histology to be independent primary tumors, arising simultaneously or subsequently to the first breast cancer and occurring in the contra lateral or ipsilateral breast. Tumors that were classified clinically or by histology as recurrences of the original tumor or multifocal single primary tumors were excluded. Patients with diagnoses of 2 or more independent primary tumors appearing in the registry data up until the end of December 2005 were considered to be MPBC. Patients who did not have a diagnosis of a second primary tumor in the ICR to 31 December 2005 were designated as Single Primary Breast Cancer (SPBC) cases. The risk of MPBC relative to SPBC was determined by logistic regression, taking into account the number of years of follow up time from the first breast cancer diagnosis as a covariate. Age of onset comparisons were assessed by linear regression using NEMO software.

**Family History Analysis:** For each affected proband, a family history score was assigned using the Genealogical Database and ICR records. The circle of first to third degree relatives around the proband was determined by reference to the Genealogical Database. Then, the affection status of the relatives was determined from ICR records. Probands were assigned a family history score of 1 for each affected first degree relative, 0.5 for each affected second-degree relative, and 0.25 for each affected third-degree relative. The total score was then summed for each proband. Potential relationship between SNP marker genotype and family history score was then tested by Wilcoxon tests run on JMP v4 software (S.A.S. Institute Inc.).

**Association Analysis Phenotypes:** We used two related phenotypes for the association analysis. The first phenotype included all 1600 individuals diagnosed with breast cancer. This phenotype is designated "All Breast Cancer" *(All BC*). The second phenotype selected individuals with moderate to high predisposition characteristics and was designated "Medium Predisposition" *(MedPre).* The definition of this phenotype required that the proband fulfilled one of the following criteria:
1. The proband was a member of a cluster of breast cancer cases containing 3 or more affected relatives within a genetic distance of 3 meiotic events (3M).
2. The proband was a member of an affected pair related within 3M, one of whom was diagnosed when aged 50 or younger.
3. The proband was a member of an affected pair related within 3M, one of whom was diagnosed with a second primary tumor of any type.
4. The proband had been diagnosed with a second primary tumor of any type.

Out of the 1600 patients typed on the Illumina Hap300 chip, 653 fulfilled the *MedPre* criteria (40.8%).

### RESULTS

### Section 1: rs4848543 on Chromosome 2q14.2

### The rs4848543 allele A allele is associated with an increased risk for breast cancer:

Genome wide association analysis showed that rs4848543 allele A was associated with *MedPre* breast cancer. Illumina chip data for this SNP were obtained for 1598 patients (653 of whom fulfilled the *MedPre* phenotype criteria) and 4477 cancer-free controls. The RR estimate for rs4848543 allele A in association with *MedPre* breast cancer was 1.42 with a P-value of 8.3x10⁻⁸ when corrected for relatedness between individuals in the case and control groups (Table 1). Following Bonferroni correction for 317,089 SNPs tested, the overall corrected P-value was 0.026. The A allele of rs4848543 also showed nominally significant association with the *AllBC* phenotype, with a relative risk estimate of 1.16 (Table 1). In order to confirm this result in an independent sample from the Icelandic population, we designed and validated a Centaurus SNP assay, SG02S738, to test for the rs4848543 SNP. The SG02S738 assay was run on an additional 573 BC patients, of whom 198 fitted the *MedPre* phenotype definition. These patients were tested against an independent control group comprised of 7406 individuals who were typed for rs4848543 using the Illumina chip or the SG02S738 assay. The RR estimate for rs4848543 allele A in association with *MedPre* breast cancer in this second group was 1.26 (p-value=3.4×10⁻²). Therefore the original finding was replicated to nominal significance in an independent Icelandic sample. The results of a joint analysis, in which the original 1598 patients and the 573 replication patient set are combined and compared with 11883 controls is shown in Table 1. The joint analysis showed nominally significant risk for the *All8C* phenotype, and genome-wide significant risk for the *MedPre* breast cancer phenotype. The overall Bonferroni corrected P-value for *MedPre* breast cancer was 0.0095 (correcting for 317,089 SNPs). This value is genome-wide significant, even if testing of the two phenotypes of *AllBC* and *MedPre* are taken into account.

Because the ICR records go back to 1955, some of the patients we recruited were long-term cancer survivors. If patients who carried the rs4848543 allele A variant had different probabilities of long-term survival than non-carriers, then the frequency of the variant amongst prevalent cases might be affected. To investigate this, we identified subset of patients composed of 833 individuals who were diagnosed after 1 January 2000 and had times from diagnosis to recruitment of less than 5 years. In this cohort of recently diagnosed patients, the frequency of the rs4848543 allele A allele was 0.372945. We also identified a group of 1338 patients who had been diagnosed before 1 January 2000 and had survived to recruitment. The frequency of the rs4848543 allele A amongst this group was 0.372749, which is not significantly different from the frequency amongst recently diagnosed patients (P value = 0.99). Therefore there is no compelling evidence that differential survivorship affected the risk estimates shown in Table 1.

We noted additionally that breast cancer patients who did not have the *MedPre* phenotype did not display any increased frequency of rs4848543 allele A (Table 1). The implications of this observation are discussed further below. We went on to examine the relationships between rs4848543 allele A and various components of the *MedPre* phenotype definition:

### rs4848543 allele A is not significantly associated with early onset breast cancer:

Because a young age at diagnosis was one of the criteria used in the definition of the *MedPre* phenotype, we looked for an association between age at diagnosis and rs4848543 genotype. Amongst the 1598 patients who were analysed on the Illumina chips, there was no significant association between rs4848543 genotype and age at diagnosis (Table 2).

### The risk from rs4848543 allele A is associated with a family history of breast cancer:

A family history score was calculated for each patient proband in the study. Using the Genealogical Database, we identified a circle of first- through third-degree relatives of each proband. Then, by reference to the ICR records, we identified all those relatives who had themselves been diagnosed with breast cancer. We then generated a summed family history score (FHS) for each proband, assigning a score of 1 for each affected first-degree relative, 0.5 for each affected second degree relative, and 0.25 for each third-degree relative. The 1598 probands who had been typed on the Illumina chip were then evaluated to look for an association between FHS and rs4848543 genotype. As shown in Table 2, a tendency for increased FHS was associated with carriage of the A allele of rs4848543. This result would not be expected based only on the observation that the rs4848543 allele A variant has a relative risk amongst *AllBC* patients of only 1.16; this relative risk alone could not generate a familial clustering of breast cancer cases [Stacey, et al., (2006), PLoS Med, 3, e217]. However, rs4848543 allele A was discovered using the *MedPre* phenotype, which has an substantial element of family history in its definition. These observations suggest that rs4848543 allele A may be in linkage disequilibrium with a less common, higher penetrance variant. Alternatively rs4848543 allele A might show interactions with another high penetrance predisposition determinants, increasing their penentrance and thereby the association with family history of breast cancer.

*The association between risk from rs4848543 allele A and family history of Breast Cancer is partly (but not completely) explained by families carrying the BRCA2 999del5 mutation:*

In order to investigate further the relationship between rs4848543 allele A and family history of breast cancer, we asked whether families carrying the well characterized Icelandic BRCA2 999del5 mutation are responsible for the familial clustering exhibited by patients carrying rs4848543 allele A. We typed 1499 patients out of the set of 1600 for the BRCA2 999del5 mutation. We re-calculated the FHS as described above, this time excluding all probands who had been shown to carry the BRCA2 999del5 mutation. As shown in Table 2, the association between rs4848543 allele A and FHS was still significant after removal of the 999del5 mutation carriers, suggesting that the association cannot be fully explained by BRCA999del5 mutation families. However, the P-value was substantially higher than when 999del5 carriers had not been excluded, leading us to suspect that BRCA2 999del5 might be involved in some of the familial clustering exhibited by rs4848543 allele A.

*The risk from rs4848543 allele A extends to carriers of the BRCA2 999del5 mutation:*

Since the BRCA2 999del5 mutation has such a substantial impact on familial breast cancer in Iceland, we considered its relationship with the rs4848543 allele A variant. Once possible scenario would be that the rs4848543 allele A variant confers negligible additional risk to BRCA2 999del5 carriers, as has been suggested for the interaction between CHEK2 and BRCA mutations [(2004), Am J Hum Genet, 74, 1175-82; Meijers-Heijboer, et al., (2002), Nat Genet, 31, 55-9]. If this were so, then the frequency of the rs4848543 allele A variant amongst affected BRCA2999del5 carriers would approximate the control frequency. Conversely, if the frequency of the rs4848543 allele A variant in affected BRCA2 999del5 carriers is greater than in the population controls, it would imply that the rs4848543 allele A confers risk on the BRCA2 carriers over and above the risk conferred by the 999del5 mutation. In order to maximize the number of BRCA2 999del5 carriers, the following analysis was carried out on the joint Icelandic Illumina and Replication patient sets. Examination of the data showed that the frequency of the rs4848543 allele A variant was 0.463 in BRCA2 999del5 carriers with breast cancer, significantly greater than the control frequency and corresponding to an RR of 1.65 (Table 3). Therefore, BRCA2 999del5 carriers (who are already at a high risk of breast cancer) have their risk multiplied by an estimated factor of 1.65 for each rs4848543 allele A allele carried. There was no evidence for a deviation of the rs4848543 genotypes from the multiplicative model. These observations demonstrate that the increased risk of breast cancer conferred by the rs4848543 allele A variant extends to BRCA2 999del5 carriers. Since BRCA2 999del5 is a null mutation that produces a non-functional protein, these results suggest that the risk confers by rs4848543 allele A extends to all carriers of BRCA2 mutations where the mutation renders the protein non-functional.

### rs4848543 allele A may interact synergistically with BRCA2 999del5:

The observation that the risk from rs4848543 allele A extends to BRCA2 999del5 carriers raises the question of whether the variant confers the same relative risk upon 999del5 carriers as it does upon 999del5 non-carriers. We noted that the relative risk estimate for rs4848543 allele A in a BRCA2 999del5 carrier (1.65) was somewhat higher than the relative risk estimate for rs4848543 allele A in non-carriers of the BRCA2 mutation (1.13, Table 3). We therefore tested whether the relative risk conferred by rs4848543 allele A was different in a BRCA2 999del5 background than it was in a non-999del5 background. As shown in Table 3, the relative risk conferred by rs4848543 allele A was an estimated 1.46-fold higher in 999del5 carriers than in non-carriers. These observations are consistent with the interpretation that rs4848543 allele A interacts in a synergistic manner with BRCA2 999del5. Since BRCA2 999del5 is a null mutation that produces a non-functional protein, these results suggest that rs4848543 allele A interacts in a synergistic manner with all BRCA2 mutations where the mutation renders the protein non-functional.

The potential interaction with BRCA2 999del5 was also investigated by evaluating whether, amongst patients, there was a significant correlation between numbers of variant alleles of rs4848543 and 999del5 carried by patients. As shown in Table 4, there was, in patients, a significant correlation between the number of rs4848543 allele A alleles carried and the carriage of BRCA2 999del5 alleles. In order to investigate whether the correlation between rs4848543 allele A and BRCA2 999del5 alleles in patients might have arisen due to an underlying population stratification, we examined whether the correlation existed in the control population. There was no correlation amongst 5938 controls who had been tested for both variants. Breast cancer patients were excluded from this group of 5938 controls. Because BRCA2 999del5 is such-a strong risk factor for breast cancer, it might be argued that the control group was depleted of 999del5 carriers (since relatively few female carriers might be unaffected with breast cancer). We therefore repeated the test using a control group comprised of 2925 male subjects who had been tested for both variants. There was no evidence of a correlation between rs4848543 allele A and 999del5 carriage in this group. Therefore we concluded that there is no evidence of an underlying population stratification that might account for the co-inheritance of rs4848543 allele A and 999del5 amongst patients. Accordingly, the most likely explanation is that rs4848543 allele A and 999del 5 interact in an synergistic manner.

We then returned to our observation that rs4848543 allele A shows an association with family history when known BRCA2 999del5 patients are removed from the analysis (Table 2). We asked whether high risk patients who were not BRCA2 999del5 carriers but who fitted the *MedPre* phenotype criteria demonstrated an increased frequency of rs4848543 allele A. The frequency was higher in these patients than in controls, corresponding to a relative risk of 1.35 (Table 3). Thus, the risk association with rs4848543 allele A extends to patients who are not 999del5 carriers but who nevertheless have the *MedPre* characteristics.

As shown in Table 1, there was no detectable risk associated with rs4848543 allele A in patients who do not fit the *MedPre* phenotype definition. This suggests that rs4848543 allele A only confers risk in the context of a *MedPre* phenotype background. This could occur if a relatively rare, high penetrance mutation exists on the background of the rs4848543 allele A variant. Alternatively, the rs4848543 allele A variant could be indicative of a more common allele that modifies the penetrance of BRCA2 and other, as yet unidentified, high penetrance risk determinants that have a familial association. Such unidentified risk determinants might be high penetrance variants of other genes, polygenic combinations of variants, or even environmental risk factors that cluster in families. For convenience, we refer to such hypothetical high penetrance risk determinants as BRCAx. To further investigate the notion that rs4848543 allele A might act as a modifier gene, we asked whether rs4848543 allele A occurred in the same frequency amongst affected BRCA2 999del5 carriers as it does on *MedPre* affected, non-BRCA carriers. This, in effect, queries whether the rs4848543 allele A variant confers a similar risk on carriers of 999del5 as it does on individuals with BRCAx. As shown in Table 3, there was no significant difference in frequencies of the rs4848543 allele A variant between these groups. However the point estimate of the relative risk was 1.22, suggesting that the effect might be greater in 999del5 carriers.

### Carriers of rs4848543 allele A and a number of other markers within the rs4848543 LD block (in linkage disequilibrium with rs4848543) are at increased risk for multiple primary breast cancer:

Occurrence of multiple primary breast tumors is an indicator of hereditary predisposition and has clinical relevance. Patients who have had breast cancer are at greatly increased risk for developing a second primary tumor. Genetic predisposition may form a substantial component of this increased risk. We therefore investigated whether multiple primary breast cancer (MPBC) occurred at higher than expected frequencies in rs4848543 allele A carriers than non-carriers. Data from the Illumina chip indicated that other markers in the rs4848543 LD block might predict MPBC at least as well as rs4848543. We therefore designed and tested a battery of 8 assays for markers within the block. In an evaluation of the risk for MPBC using logistic regression and correcting for the length of patients' follow-up times, 7 out of 8 SNPs showed an increased risk of MPBC in carriers when compared with patients who had only experienced a single primary breast cancer (SPBC) diagnosis (Table 5). We concluded that these 7 SNP assays can determine whether a patient is at an increased risk of MPBC.

### Multiple SNPs in one linkage disequilibrium block show associations with All BC and MedPre Breast Cancer phenotypes:

The SNP rs4848543 allele A is found in a linkage disequilibrium (LD) block whose delimiting coordinates, based on NCBI Build 34, are shown in Table 6. The LD block was defined as extending between recombination hotspots as defined by the Mathematical Genetics Group at the University of Oxford using the likelihood ratio test described in [McVean, et al., (2004), Science, 304, 581-4; Winckler, et al., (2005), Science, 308, 107-11]. Data from the Illumina Hap300 chip revealed a number of SNPs that are in the same linkage disequilibrium block as rs4848543 allele A and that showed nominally significant associations with the two breast cancer predispositions tested. Tables 7 & 8 show a lists of all of the SNPs in the LD block that are present on the Illumina Hap300 chip, and the association values obtained from them for the two phenotypes. We also considered two-marker haplotypes within the block that were identified as efficient surrogates (r²>0.8) for an additional set of SNPs typed in the HapMap project (all references to HapMap data are with respect to release 20). These additional SNPs have a minor allele frequency of >5% in the UTAH CEPH (CEU) HapMap samples and they are neither on the Hap300 SNP chip nor are they efficiently tagged by SNPs that are on the chip [Pe'er, et al., (2006), Nat Genet, 38, 663-7]. Results for these so-called "Daly" haplotypes are also shown in Tables 7 & 8. For *MedPre* breast cancer, 29 individual SNPs or two-point "Daly" haplotypes showed nominally significant p-values.

### rs4848543 allele A-associated risk is present on multiple haplotype backgrounds.

In order to try to differentiate whether rs4848543 allele A tags a relatively rare, high penetrance variant or behaves as a common modifier of other high penetrance determinants, we investigated the diversity of haplotype backgrounds in which rs4848543 allele A appears. The rs4848543 LD block contains a region of relatively high recombination, with its distal end at around nucleotide 120034174. The SNPs that confer nominally significant breast cancer risk are located distal (rightwards) to this region of recombination. We therefore limited our analysis of the haplotype diversity to this right-hand sub-block region. We used NEMO software to identify haplotypes within the defined region (nt 120034174 to nt 120129001) using SNPs and samples that were typed on the Illumina chips. Redundant SNPs that were not necessary to differentiate haplotypes were eliminated, then relative risks and frequencies were evaluated for each resulting haplotype. The results are shown in Table 9. It is apparent that all identified haplotypes that carry the rs4848543 A allele had a relative risk estimate greater than 1 and, in most cases the RR estimate was significantly greater than 1. Conversely, no haplotype that carried the rs4848543 C allele had a RR estimate greater than 1, and all the significant estimates were less than 1 (i.e. they were protective). Thus we were unable to separate the risk from rs4848543 allele A-bearing haplotypes. This suggests that the pathogenic variant (i.e. the variant that, mechanistically, confers risk) is rs4848543 allele A or a variant in strong linkage disequilibrium with rs4848543 allele A. The presence of the pathogenic variant on multiple haplotype backgrounds suggests that its frequency might closely approximate that of rs4848543 allele A (i.e. the r² value between rs4848543 allele A and the pathogenic variant is high). This observation favors the notion that rs4848543 allele A does not indicate the presence of a rare, high penetrance variant within the rs4848543 LD block, but rather it marks a rather common modifier of high penetrance determinants such as BRCA2 and the previously defined BRCAx.

### Numerous markers could show BC risk association through their correlation with rs4848543 allele A:

Reference to the HapMap project data (release 20) revealed a number of known SNPs that are correlated with rs4848543 with r² values greater than 0.2 in the CEU white European population sample. Since these SNPs are significantly correlated with rs4848543, they could be used to measure the same BC risk as is measured by rs4848543 itself. These SNPs are listed in Table 10.

### The STEAP3/TSAP6 gene is the most probable gene involved in the observed risk for breast cancer:

There are three presently known genes in the rs4848543 LD block region. These are;
1. The Complement Component 1q Subcomponent-like 2 gene C1Ql2 (NM_182528)
2. The Six Transmembrane Epithelial Antigen of Prostate 3 (STEAP3) gene, otherwise known as Tumour Suppressor Activated Pathway 6 (TSAP6), pHyde, and Dudulin-2. There are three major known alternative transcripts of this gene identified by RefSeq numbers NM_018234 (isoform b), NM_182915 (isoform a), and NM_001008410 (also designated AF262322). A fourth transcript of this gene has been described and assigned the identifier AK024163.
3. A transcript, AK127773, overlaps with the STEAP3 gene and is transcribed from the opposite (leftwards) strand. This gene has been designated Hypothetical protein FU45874.

The rs4848543 LD block contains a region of relatively high recombination, with its distal end at around nucleotide 120034174. The majority of SNPs and Daly 2-point haplotypes that confer nominally significant breast cancer risk are located distal (rightwards) to this region of recombination. This suggests that variants in and around C1Ql2 are not the source of the association signal. The variants that do associate strongly with risk are located 5' to and in the STEAP3/TSAP6 gene. Indeed two of the nominally significant SNPs, rs838100 and rs3731603 are within the transcribed sequences of the gene and another two, rs838102 and rs12711924 are located in STEAP3/TSAP6 introns. The latter of these two SNPs is located near the 3' splice site of the IVS4 gene. Given these observations, and the known biology of the STEAP3/TSAP6 gene (see below), the most likely gene to be involved in the observed increased risks for breast cancer is STEAP3/TSAP6.

### Section 2: rs13387042 on Chromosome 2q35

*The rs13387042 A allele is associated with an increased risk for Breast Cancer in Iceland:*

Genome-wide association analysis showed that rs13387042 allele A was associated with the *Any* BC phenotype (Table 11). This SNP is located at chromosome 2q35, at a distinct location from the STEAP3/TSAP6 locus. Illumina chip data for this SNP were obtained for 1598 patients and 4475 cancer-free controls. The RR estimate for A-rs13387042 was 1.19 with a P-value of 4.0x10⁻⁵ when corrected for relatedness between individuals in the case and control groups. The RR was similar in the *MedPre* breast cancer, with a P-value of 4.4x10⁻³.

In itself, this result did not reach a level that would be considered genome-wide significant after Bonferroni correction for the 317,089 SNPs tested. We therefore sought to confirm the result in an independent sample from the Icelandic population, as we did for rs4848543 allele A. We designed and tested a Centaurus SNP assay, designated SG02S728, to test for the rs13387042 SNP. The SG02S728 assay was run on an additional 583 patients and an independent control group comprised of 7966 individuals who were typed for rs13387042 allele A, either by the Illumina chip or by the Centaurus methods. The 7966 control group contained individuals who had been diagnosed with prostate cancer or colorectal cancer but not individuals who had been diagnosed with breast cancer. This was justified by our interim observations that rs13387042 allele A did not confer detectable risk for these cancers (data not presented). The RR estimate for the A allele of rs13387042 in association with *AnyBC* in this second group was 1.20 (P-value_{corr} = 3.8x10⁻³). Therefore the original finding was replicated significantly in an independent Icelandic sample, with a very similar point estimate of the RR. The results of a joint analysis, in which the original 1598 and the 583 replication samples are combined and compared with 12441 controls is shown in Table 11. The joint analysis showed a significant relative risk estimate of 1.20 (P-value_{corr} = 2.0x10⁻⁷), which is close to the level of significance after Bonferroni correction for the 317,089 SNPs tested. The observed frequency and relative risk of the rs13387042 allele A variant corresponds to an estimated population attributable risk of 15.6% in the Icelandic population.

Because the ICR records go back to 1955, some of the patients we recruited were long-term cancer survivors. If patients who carried the rs13387042 allele A variant had different probabilities of long-term survival than non-carriers, then the frequency of the variant amongst prevalent cases might be affected. To investigate this, we identified subset of patients composed of 837 individuals who were diagnosed after 1 January 2000 and had times from diagnosis to recruitment of less than 5 years. In this cohort of recently diagnosed patients, the frequency of rs13387042 allele A was 0.498. We also identified a group of 1344 patients who had been diagnosed before 1 January 2000 and had survived to recruitment. The frequency of rs13387042 allele A amongst this group was 0.492, which is not significantly different from the frequency amongst recently diagnosed patients (P value = 0.71). Therefore there is no compelling evidence that differential survivorship affected the risk estimates shown in Table 11.

### The rs13387042 A allele is associated with an increased risk for Breast Cancer in a Spanish case: control sample

In order to further investigate the role of rs13387042 allele A in breast cancer risk, we examined an independent set of BC case:control samples collected in Spain. The Spanish study population consisted of 446 breast cancer cases recruited from the Oncology Department of Zaragoza Hospital in Zaragoza, Spain. A set of 977 control individuals were approached at the University Hospital in Zaragoza, Spain, and were confirmed to be free of breast cancer before they were included in the study. All subjects gave written informed consent.

The Spanish case and control samples were genotyped for rs13387042 using the SG02S728 Centaurus assay. The results are presented in Table 11. Allele A of rs13387042 showed significant association with Breast Cancer risk in this population, with a point estimate of the RR of 1.21 (P-value 1.8x10⁻²). The frequency of the rs13387042 allele A variant was higher in the Spanish control samples, suggesting that the variant may be more prevalent and thus contribute to a higher Breast Cancer burden in populations of Spanish extraction.

### The rs13387042 A allele is associated with an increased risk for breast cancer in Swedish case/control samples:

To seek further confirmation of the role of the A allele of the rs13387042 variant in risk for Breast Cancer, we typed the SNP in two Swedish cohorts: The "Sweden Familial" cohort was comprised of 346 BC patients who had been referred to the oncogenetic counseling clinic of the Karolinska Institute, Stockholm for investigation of a family history of breast cancer. Each patient came from a distinct family. The "Sweden Consecutive" cohort was comprised of 482 consectively recruited BC patients who attended the Karolinska Institute breast cancer clinic. Family history was not taken into account in the selection of this second cohort. Controls were 1300 blood donors from the Stockholm area and 434 cancer-free spouses of colorectal cancer cases. There was no significant difference between genders in the frequency of A-rs13387042 in the controls, nor was there a significant difference between the blood donor and cancer-free control sets.

As shown in Table 11, the "Sweden Consecutive" cohort demonstrated a significant relative risk for breast cancer of 1.31 (P-value= 2.0x10⁻⁴). The "Sweden Familial" cohort relative risk estimate of 1.11 was not significant, perhaps because this group may carry high-penetrance genes that overshadow the effect of A-rs13387042. Overall, the combined Swedish cohorts returned a significant relative risk estimate of 1.22 (P-value= 8.1x10⁻⁴).

The estimates for the Icelandic, Spanish and Swedish cohorts were combined in a joint analysis, using the Mantel-Haenszel model. The result was an estimated relative risk of 1.20 with a P-value of 3.8x10⁻¹¹. This is well below the threshold for genome-wide significance using the Bonferroni method to correct for the 317,089 SNPs investigated. We therefore concluded that A-rs13387042 confers a significant and reproducible risk for breast cancer in several population samples of European descent. The overall population attributable risk was estimated to be 16.4%.

### rs13387042 allele A - associated risk is present on multiple haplotype backgrounds:

The rs13387042 SNP is found in a linkage disequilibrium (LD) block whose delimiting coordinates, based on NCBI Build 34, are shown in Table 12. The LD block was defined as extending between recombination hotspots as defined by the Mathematical Genetics Group at the University of Oxford using the likelihood ratio test described in [McVean, et al., (2004), Science, 304, 581-4; Winckler, et al., (2005), Science, 308, 107-11]. In order to try to differentiate whether the A allele of marker rs13387042 tags a relatively rare, high penetrance variant or a more common, lower penetrance variant we investigated the diversity of haplotype backgrounds in which rs13387042 allele A appears. We used NEMO software to identify haplotypes within the LD block using NSPs and samples that were typed on the Illumina chips. Redundant SNPs that were not necessary to differentiate haplotypes were eliminated, then relative risks and frequencies were evaluated for each resulting haplotype. The results are shown in Table 13. It is apparent that several identified haplotypes that carry the A allele of rs13387042 allele confer relative risk estimates greater than 1 and, in several cases the RR estimate was significantly greater than 1. Conversely, only one, rare haplotype that carried the C allele of rs13387042 allele showed a RR estimate greater than 1, and the two nominally significant RR estimates for rs13387042 allele C-containing haplotypes were less than 1 (i.e. they were protective). Two haplotypes that carry the A allele of rs13387042 did not produce RR estimates greater than 1, and one of these estimates was nominally significant. Taken together, these observations suggests that the pathogenic variant (i.e. the variant that, mechanistically, confers risk) is in strong LD with rs13387042 allele A but may not be rs13387042 allele A itself.

### Multiple SNPs in the rs13387042 LD block show association with BC risk:

Data from the Illumina Hap300 chip revealed a number of SNPs that are in the same LD block as rs13387042. Table 14 shows a list of all the SNPs in the LD block that are present on the Illumina Hap300 chip and the association values obtained from them for single point and two point "Daly" haplotype analysis. It can be seen that 21 SNPs or Daly haplotypes showed nominally significant P-values when corrected for familial relationships amongst the subjects.

### Numerous markers could show BC risk association through their correlation with rs13387042 allele A:

Reference to the HapMap project data (release 20) revealed a number of known SNPs that are correlated with rs13387042 with r² values greater than 0.2 in the CEU white European population sample. Since these SNPs are significantly correlated with rs13387042, they could be used to measure the same BC risk as is measured by rs13387042 itself. These SNPs are listed in Table 15.

### Section 3: rs3803662 on Chromosome 16q12

### The T allele of rs3803662 is associated with risk for Breast Cancer:

Further genome wide SNP analysis using data from 1600 breast cancer patients and 11563 controls identified the T allele of SNP rs3803662 as conferring and estimated 1.23-fold increased risk for *Any BC* (Table 16). This result was confirmed in a second, independent cohort of 594 Icelandic breast cancer patients and 1433 controls. The combined data from these two Icelandic samples gave a relative risk estimate of 1.23 and a P-value of 2.8x10⁻⁷ when corrected for relatedness between the individuals. This corresponded to an estimated population attributable risk of 10.1% (Table 16).

### Association rs3803662 allele T with risk for Breast Cancer is replicated in independent breast cancer case: control cohorts from Sweden, Spain, and Holland.

In order to confirm the association of rs3803662 allele T with breast cancer risk, we sought to replicate the finding in three independent, foreign cohorts. We genotyped individuals from the Swedish and Spanish cohorts described above. We also genotyped 558 breast cancer cases and 1384 controls from Nijmegen, Holland. This cohort was drawn from a population-based registry ascertainment of breast cancer patients diagnosed in the Eastern part of the Netherlands in the years 2005-2006. All patients diagnosed with breast cancer before the age of 70 years during this period were invited to participate in the study. The control group was collected in a survey conducted in 2002-2003 (the Nijmengen Biomedical Study) based on a random sample of the population registration. As shown in Table 16, a significantly increased risk for rs3803662 allele T was observed in all three of these replication cohorts. In a combined analysis of these three non-Icelandic replication cohorts, the overall relative risk estimate was 1.35 and the P-value was 5.1x10⁻¹². A joint analysis of the Icelandic and replication cohorts indicated a combined relative risk estimate of 1.28 with a P-value of 2.7x10⁻¹⁷. This is well below the threshold for genome-wide significance when corrected for the number of SNPs tested. The corresponding overall population attributable risk estimate was 13.4% (Table 16).

### Definition of the LD block containing rs3803662 and identification of potential candidate genes.

The rs3803662 SNP is found in a linkage disequilibrium (LD) block whose delimiting coordinates, based on NCBI Build 34, are shown in Table 17. The LD block was defined as extending between recombination hotspots as defined by the Mathematical Genetics Group at the University of Oxford using the likelihood ratio test described in [McVean, et al., (2004), Science, 304, 581-4; Winckler, et al., (2005), Science, 308, 107-11]. The rs3803662 is present in the transcript of a hypothetical protein designated LOC643714. This hypothetical protein is thus implicated as a potential cancer predisposition gene. The 5' end of known gene TNRC9 is also present in the block. TNRC9 is a member of the chromatin associated high mobility group (HMG) protein family, a group of proteins which is known to include transcription factors and chromatin remodeling agents. Reduced expression of the TNRC9 protein has been observed in metastatic breast cancer when compared to primary breast cancers (Olendrowitz, C. 2006, Bachelor of Science Bioinformatics Thesis, Charité-Universitatsmedizin, Berlin). The identification of a SNP variant near the 5' end of this gene suggests that the variant may be in linkage disequilibrium with genetic factors that alter the expression of function of the TNRC9 protein, leading to an increased breast cancer risk.

### Multiple SNPs in the rs3803662 LD block show association with BC risk:

Data from the Illumina Hap300 chip revealed a number of SNPs that are in the same LD block as rs3803662. Table 18 shows a list of all the SNPs in the LD block that are present on the Illumina Hap300 chip and the association values obtained from them for single point and two point "Daly" haplotype analysis. It can be seen that 80 SNPs or Daly haplotypes showed nominally significant P-values.

### Numerous markers could show BC risk association through their correlation with rs3803662 allele T:

Reference to the HapMap project data (release 20) revealed a number of known SNPs that are correlated with rs3803662 with r² values greater than 0.2 in the CEU white European population sample. Since these SNPs are significantly correlated with rs3803662, they could be used to measure the same BC risk as is measured by rs3803662 itself. These SNPs are listed in Table 19.

### rs3803662 allele T and/or related SNPs confer risk for Breast Cancer in several different ethnicities:

In order to investigate the generality of the findings with rs3803662 allele T in patients from different ethnic background, we tested the SNP for association in a sample of breast cancer patients and controls from the Multiethnic Cohort study (MEC). The MEC consists of over 215,000 men and women in Hawaii and Los Angeles (with additional African-Americans from elsewhere in California) and has been described in detail elsewhere [Kolonel, et al., (2000), Am J Epidemiol, 151, 346-57]. The cohort is comprised predominantly of African Americans, Native Hawaiians, Japanese Americans, Latinos, and European Americans who entered the study between 1993 and 1996 by completing a 26-page self-administered questionnaire that asked detailed information about dietary habits, demographic factors, personal behavior, history of prior medical conditions, family history of common cancers, and for women, reproductive history and exogenous hormone use. The participants were between the ages of 45 and 75 at enrolment. Incident cancers in the MEC were identified by cohort linkage to population-based cancer Surveillance, Epidemiology, and End Results (SEER) registries covering Hawaii and Los Angeles County and to the California State Cancer Registry covering all of California. Beginning in 1994, blood samples were collected from incident breast cancer cases and a random sample of MEC participants to serve as a control pool for genetic analysis in the cohort. Eligible cases in the nested breast cancer case-control study consisted of women with incident invasive cancer diagnosed after enrolment into the MEC through December 31, 2002. Controls were participants without breast cancer, respectively, prior to entry into the cohort and without a diagnosis up to December 31, 2002. Controls were frequency matched to case based on race/ethnicity and age (in 5-year intervals). The study was approved by the Institutional Review Boards at the University of Southern California and at the University of Hawaii.

We sought to investigate the risk associated with rs3803662 in European Americans, Latina, and African Americans from the MEC. Because the correlations between the rs13387042 SNP and other SNPs might be different in different ethnicities, we selected a group of SNPs to test in these samples. The SNPs, their r² and D' values derived from HapMap project data (release 20) for European Americans and Yoruba (Africans) are shown in Table 23. Centaurus assays were generated for these SNPs, and genotyping was carried out in the MEC samples. To be better able to compare results between SNPs, we used the generalized likelihood ratio test taking into account missing genotypes by haplotype analysis. This had the effect of equalizing the number of cases and controls for different SNPs, allowing for an easier comparison between them. Results are shown in Table 24. Firstly it is evident that the risk from rs3803662 allele T is replicated significantly in European Americans. Two of the related SNPs, rs4784227 allele T and rs17271951 allele C show nominally higher relative risks and lower P-values than rs3803662 allele T in European Americans. This exemplifies the concept that related to the SNPs originally identified might have higher relative risks and thus provide superior disease risk markers.

The rs3803662 allele T signal is also replicated significantly in Latina. The T allele of marker rs3803662 did not replicate significantly in African Americans, indeed the T-allele was significantly protective in the African American sample. This suggests the LD relation between the rs3803662 allele T and the putative pathogenic mutation is quite different in African Americans. We noted that the three related SNP alleles; rs12922061 allele T, rs4784227 allele T, and rs17271951 allele C are strongly correlated with rs3803662 allele T in Europeans, but this correlation is not maintained in Yoruban Africans (Table 24). However, unlike rs3803662 allele T, the related SNP alleles rs12922061 allele T, rs4784227 allele T, and rs17271951 allele C all show nominally increased relative risks for breast cancer, two of which achieve statistical significance at the 1-sided level (Table 24). This shows that using sets of related SNPs can provide risk markers suitable for use in a variety of ethnic groups.

### rs13387042 allele A and rs3803662 allele T confer increased risk for Estrogen Receptor Positive Breast Cancer:

Medical records of patients in the study were reviewed if they were available. Using the combined sample sets, we looked for associations between rs13387042 allele A and rs3803662 allele T, age at diagnosis, estrogen receptor (ER) and progesterone receptor (PR) status. Neither variant showed an association with age at diagnosis. Significant breast cancer risk involving rs13387042 allele A and rs3803662 allele T was clearly confined to those patients diagnosed with ER positive tumors and the difference between the OR's for ER positive and ER negative tumors was significant (Table 24). Similarly, there was a tendency towards breast cancer risk preferentially amongst patients diagnosed with PR positive tumors, however the difference between PR positive and PR negative OR's was not significant. This suggests that ER positive and negative tumors may have somewhat different genetic etiologies.

### TABLES:

**Table 1: Association of SNP SG02S738 (rs4848543) allele A with risk for breast cancer:**

| | **p-val** | **p_corr*** | **r** | **95%Cl** | **#aff** | **aff.freq** | **#con** | **con.freq** |
|---|---|---|---|---|---|---|---|---|
| MedPre Illumina | 6.70E-09 | 8.30E-08 | 1.42 | (1.25,1.62) | 653 | 0.423 | 4477 | 0.341 |
| MedPre Iceland Replication | 2.90E-02 | 3.40E-02 | 1.26 | (1.02,1.55) | 198 | 0.399 | 7406 | 0.346 |
| MedPre joint | 9.70E-10 | 3.00E-08 | 1.37 | (1.23,1.53) | 851 | 0.418 | 11883 | 0.344 |
| All Illumina | 7.10E-04 | 1.10E-03 | 1.16 | (1.06,1.26) | 1598 | 0.374 | 4477 | 0.341 |
| All Iceland Replication | 9.70E-02 | 1.00E-01 | 1.11 | (0.98,1.26) | 573 | 0.37 | 7406 | 0.346 |
| All joint | 2.20E-04 | 3.50E-04 | 1.14 | (1.06,1.22) | 2171 | 0.373 | 11883 | 0.344 |
| non-MedPre vs. CTR | 9.80E-01 | | 1 | | 1320 | 0.344 | 11883 | 0.344 |

| | | | | | | | | |
|---|---|---|---|---|---|---|---|---|
| *corrected for relationships between individuals | | | | | | | | |

**Table 2: Association of SNP SG02S738 (rs4848543) with age at diagnosis and family history of breast cancer:**

| **Comparison** | **P-value (Wilcoxon)** |
|---|---|
| rs4848543 Genotype vs Age at Diagnosis | 0.5139 |
| rs4848543 Genotype vs Family History Score | <0.001 |
| Non-BRCA2 rs4848543 Genotype vs Family History Score | 0.0044 |

**Table 3: Association of SNP SG02S738 (rs4848543) with risk for breast cancer amongst carriers of BRCA2 999del5:**

| | **p_corr*** | **p-val** | **r** | **95%Cl** | **#aff** | **aff.freq** | **#con** | **con.freq** |
|---|---|---|---|---|---|---|---|---|
| BC BRCA2 carr vs. CTR | 3.67E-03 | 8.02E-04 | 1.65 | (1.18,2.30) | 94 | 0.463 | 9452 | 0.344 |
| BC BRCA2 non carr vs. CTR | 2.95E-03 | 2.19E-03 | 1.13 | (1.04,1.22) | 1621 | 0.371 | 9452 | 0.344 |
| BC BRCA2 carr vs. BC BRCA2 non car | 3.06E-02 | 1.29E-02 | 1.46 | (1.04,2.05) | 94 | 0.463 | 1621 | 0.371 |
| MedPre BRCA2 non-carr vs. CTR | 4.71 E-06 | 5.92E-07 | 1.35 | (1.19,1.55) | 622 | 0.414 | 9452 | 0.344 |
| BC BRCA2 carr vs MedPre BRCA2 non-carr | n.a. | 2.08E-01 | 1.22 | n.a. | 94 | 0.463 | 622 | 0.414 |

| | | | | | | | | |
|---|---|---|---|---|---|---|---|---|
| *corrected for relationships between individuals | | | | | | | | |

**Table 4 : Correlation between rs4848543 allele A and BRCA2 999del5 Genotypes:**

| **BRCA2 - STEAP3 genotype correlation (Pearson test)** | | | |
|---|---|---|---|
| | **P-val** | **corr.coeff.** | **#indiv** |
| **BC patients** | 1.4E-02 | 0.059 | 1715 |
| **controls** | 8.0E-01 | -0.0033 | 5938 |
| **male controls** | 6.7E-01 | -0.0079 | 2925 |

**Table 5: Association of SNPs with risk for multiple primary breast cancer:**

| **#MPBC** | **MPBC.freq** | **#SPBC** | **SPBC.freq** | **P carrier** | **RR carrier** | **Ale Mrk** | **rs SNP** | **Pos in Seq ID 4** |
|---|---|---|---|---|---|---|---|---|
| 128 | 0.258 | 1566 | 0.189 | 0.0111 | 1.615 | 4 SG02S733 | rs895398 | 34898 |
| 160 | 0.409 | 2010 | 0.370 | 0.0433 | 1.419 | 1 SG02S738 | rs4848543 | 37814 |
| 160 | 0.547 | 2019 | 0.488 | 0.0438 | 1.498 | 1 SG02S739 | rs6759589 | 41694 |
| 163 | 0.414 | 2001 | 0.371 | 0.0389 | 1.427 | 4 SG02S753 | rs895397 | 49118 |
| 162 | 0.645 | 2020 | 0.575 | 0.0072 | 1.928 | 3 SG02S740 | rs838102 | 56596 |
| 160 | 0.650 | 2014 | 0.575 | 0.0050 | 2.012 | 3 SG02S741 | rs838100 | 60428 |
| 127 | 0.445 | 1565 | 0.380 | 0.0176 | 1.602 | 1 SG02S734 | rs12711924 | 84034 |
| 159 | 0.387 | 2004 | 0.343 | 0.1741 | 1.258 | 4 SG02S742 | rs3731603 | 93780 |

**Table 6: Definition of the rs4848543 LD block on Chromosome 2:**

| **NCBI Build** | **Start** | **End** |
|---|---|---|
| 34 | 119,987,002 | 120,129,001 |
| 36 | 119,608,327 | 119,750,326 |

**Table 7: Association of SNP markers and 2-marker haplotypes in the rs4848543 LD-block with risk for breast cancer (phenotype Any Breast Cancer): The allele codes are as follows: 1=A, 2=C, 3=G, 4=T.**

| **p-value** | **RR** | **#aff** | **aff.freq** | **#con** | **con.freq** | **H0.freq** | **X2** | **info_alt** | **all** | **marker** | **all** | **marker** |
|---|---|---|---|---|---|---|---|---|---|---|---|---|
| 0.0000825 | 1.1852 | 1557 | 0.377649 | 4626 | 0.33863 | 0.348455 | 15.5001 | 1 | 1 | rs4848543 | | |
| 0.00015 | 0.8545 | 1558 | 0.498176 | 4627 | 0.537411 | 0.527529 | 14.3718 | 0.999171 | 2 | rs895398 | 3 | rs6759589 |
| 0.0001842 | 1.1677 | 1558 | 0.500642 | 4626 | 0.461954 | 0.471701 | 13.9859 | 1 | 1 | rs6759589 | | |
| 0.0004919 | 1.2131 | 1558 | 0.18702 | 4627 | 0.159399 | 0.166396 | 12.1462 | 0.966913 | 2 | rs4849765 | 4 | rs895398 |
| 0.0006022 | 1.1539 | 1558 | 0.560738 | 4627 | 0.525225 | 0.534172 | 11.7693 | 0.994181 | 2 | rs4849760 | 3 | rs838102 |
| 0.0006816 | 1.1529 | 1558 | 0.587612 | 4625 | 0.552757 | 0.56154 | 11.5388 | 1 | 3 | rs838102 | | |
| 0.0007483 | 1.1517 | 1556 | 0.587082 | 4622 | 0.552466 | 0.561185 | 11.3653 | 1 | 3 | rs838100 | | |
| 0.0007856 | 0.8681 | 1558 | 0.401958 | 4627 | 0.436379 | 0.427706 | 11.2749 | 0.995483 | 3 | rs6759589 | 1 | rs838102 |
| 0.0009395 | 1.1929 | 1557 | 0.196853 | 4626 | 0.17045 | 0.177098 | 10.9433 | 1 | 4 | rs895398 | | |
| 0.0009835 | 1.1959 | 1556 | 0.204802 | 4619 | 0.177192 | 0.184711 | 10.8584 | 0.936107 | 2 | rs4849765 | 4 | rs2084154 |
| 0.0019878 | 1.1829 | 1558 | 0.187099 | 4626 | 0.162884 | 0.168984 | 9.5608 | 1 | 4 | rs935361 | | |
| 0.0039725 | 1.3609 | 1558 | 0.067214 | 4627 | 0.050286 | 0.054483 | 8.2963 | 0.668984 | 2 | rs838086 | 1 | rs12711924 |
| 0.0044063 | 1.1296 | 1558 | 0.387356 | 4627 | 0.358872 | 0.366047 | 8.1083 | 1 | 1 | rs12711924 | | |
| 0.0053777 | 1.1574 | 1558 | 0.202503 | 4627 | 0.179922 | 0.18561 | 7.7478 | 1 | 2 | rs7564432 | 2 | rs11680207 |
| 0.0076689 | 1.1238 | 1558 | 0.349807 | 4627 | 0.323752 | 0.330315 | 7.1092 | 1 | 4 | rs3731603 | | |
| 0.0092636 | 0.895 | 1558 | 0.58833 | 4626 | 0.614901 | 0.608199 | 6.7713 | 0.984119 | 2 | rs6542504 | 2 | rs12711923 |
| 0.0119574 | 1.1118 | 1557 | 0.418433 | 4626 | 0.392888 | 0.399321 | 6.3172 | 1 | 4 | rs1530561 | | |
| 0.0120093 | 1.1289 | 1558 | 0.253591 | 4627 | 0.231338 | 0.236949 | 6.3095 | 0.998425 | 1 | rs745888 | 2 | rs838087 |
| 0.0127242 | 1.1276 | 1558 | 0.253851 | 4625 | 0.231784 | 0.237344 | 6.2071 | 1 | 1 | rs745888 | | |
| 0.0140734 | 0.8975 | 1554 | 0.330746 | 4621 | 0.355095 | 0.348951 | 6.0289 | 0.987772 | 3 | rs2084154 | 4 | rs838066 |
| 0.0143097 | 0.8986 | 1558 | 0.337519 | 4627 | 0.361823 | 0.355701 | 5.9995 | 0.993263 | 3 | rs6759589 | 4 | rs838066 |
| 0.0158735 | 0.9024 | 1558 | 0.586151 | 4627 | 0.610816 | 0.60459 | 5.8168 | 0.983893 | 2 | rs6542504 | 2 | rs4849760 |
| 0.0166389 | 1.1057 | 1558 | 0.432285 | 4627 | 0.407824 | 0.413985 | 5.7341 | 1 | 3 | rs838069 | | |
| 0.0199778 | 1.1096 | 1558 | 0.332002 | 4626 | 0.309345 | 0.315063 | 5.4138 | 0.982361 | 4 | rs6542504 | 2 | rs12711923 |
| 0.02322 | 0.9 | 1558 | 0.716624 | 4627 | 0.737523 | 0.732257 | 5.152 | 0.999896 | 2 | rs4849760 | 3 | rs745888 |
| 0.0234152 | 0.9054 | 1558 | 0.339147 | 4627 | 0.361769 | 0.356092 | 5.1374 | 0.982618 | 2 | rs895398 | 4 | rs838066 |
| 0.0236887 | 1.1011 | 1555 | 0.397106 | 4610 | 0.374295 | 0.380049 | 5.1173 | 1 | 4 | rs6542504 | | |
| 0.0346288 | 1.0972 | 1522 | 0.658673 | 4559 | 0.63753 | 0.642822 | 4.4634 | 1 | 2 | rs838066 | | |
| 0.0376797 | 0.9131 | 1558 | 0.444559 | 4627 | 0.467115 | 0.461494 | 4.3194 | 0.903903 | 1 | rs838086 | 1 | rs838069 |
| 0.0612227 | 0.9009 | 1558 | 0.161601 | 4627 | 0.176246 | 0.172556 | 3.5039 | 0.98925 | 3 | rs6759589 | 2 | rs838066 |
| 0.0675678 | 1.0805 | 1558 | 0.403402 | 4627 | 0.384915 | 0.389572 | 3.3411 | 1 | 2 | rs11680207 | | |
| 0.0742724 | 1.1028 | 1558 | 0.20821 | 4627 | 0.192542 | 0.196469 | 3.186 | 0.887553 | 3 | rs1867750 | 2 | rs11680207 |
| 0.1275005 | 1.0674 | 1511 | 0.527134 | 3962 | 0.510853 | 0.515348 | 2.3227 | 1 | 4 | rs2084154 | | |
| 0.1817962 | 0.9326 | 1558 | 0.209889 | 4627 | 0.221696 | 0.218727 | 1.7829 | 0.931297 | 1 | rs10496555 | 3 | rs745888 |
| 0.2193745 | 1.056 | 1542 | 0.679313 | 4602 | 0.667319 | 0.670329 | 1.5085 | 1 | 2 | rs4849765 | | |
| 0.2349078 | 1.0589 | 1548 | 0.251292 | 4614 | 0.240681 | 0.243346 | 1.4109 | 1 | 2 | rs838086 | | |
| 0.2688289 | 0.9532 | 1558 | 0.456896 | 4627 | 0.468807 | 0.465537 | 1.2227 | 0.919405 | 3 | rs7562894 | 3 | rs2084154 |
| 0.2941036 | 0.9538 | 1558 | 0.331602 | 4627 | 0.342173 | 0.339281 | 1.1007 | 0.945087 | 4 | rs2084154 | 2 | rs895398 |
| 0.455931 | 1.0331 | 1558 | 0.354244 | 4627 | 0.346821 | 0.34869 | 0.5559 | 0.98416 | 2 | rs330763 | 3 | rs725346 |
| 0.4591499 | 1.033 | 1558 | 0.358115 | 4627 | 0.350681 | 0.352554 | 0.548 | 0.971987 | 3 | rs1439945 | 3 | rs725346 |
| 0.4793247 | 1.0562 | 1557 | 0.079641 | 4622 | 0.075725 | 0.076711 | 0.5004 | 1 | 1 | rs12711923 | | |
| 0.497656 | 1.03 | 1558 | 0.356544 | 4627 | 0.349799 | 0.351497 | 0.4599 | 0.989528 | 2 | rs330763 | 3 | rs10496556 |
| 0.5015258 | 0.9669 | 1558 | 0.216303 | 4627 | 0.222066 | 0.220614 | 0.4517 | 1 | 2 | rs11680207 | 2 | rs935361 |
| 0.5315142 | 1.0273 | 1553 | 0.370573 | 4610 | 0.364317 | 0.365893 | 0.3915 | 1 | 3 | rs725346 | | |
| 0.5977098 | 1.0229 | 1557 | 0.376365 | 4619 | 0.371076 | 0.372409 | 0.2785 | 1 | 3 | r10496556 | | |
| 0.6233236 | 1.0256 | 1558 | 0.799101 | 4627 | 0.795007 | 0.796039 | 0.2412 | 1 | 2 | rs7564432 | | |
| 0.7140552 | 1.0158 | 1558 | 0.33668 | 4627 | 0.333192 | 0.334071 | 0.1343 | 1.054416 | 3 | rs10496555 | 3 | rs725346 |
| 0.7338904 | 0.9756 | 1558 | 0.084777 | 4627 | 0.086711 | 0.086224 | 0.1156 | 1.040852 | 3 | rs12711924 | 3 | rs838069 |
| 0.7373932 | 1.0483 | 1554 | 0.977479 | 4049 | 0.976416 | 0.976709 | 0.1124 | 1 | 2 | rs838087 | | |
| 0.7425392 | 1.0407 | 1558 | 0.970475 | 4627 | 0.96931 | 0.969604 | 0.1079 | 1 | 2 | rs4849760 | | |
| 0.8144482 | 1.01 | 1558 | 0.341581 | 4627 | 0.339335 | 0.339901 | 0.0551 | 1.052093 | 3 | rs10496555 | 3 | rs10496556 |
| 0.8352386 | 0.9855 | 1558 | 0.092358 | 4627 | 0.093587 | 0.093277 | 0.0433 | 1.03608 | 4 | rs330773 | 3 | rs745888 |
| 0.8666485 | 1.0069 | 1558 | 0.421023 | 4627 | 0.419347 | 0.419769 | 0.0282 | 1.049887 | 3 | rs10496555 | 1 | rs725346 |
| 0.8876881 | 1.0095 | 1558 | 0.106083 | 4627 | 0.10519 | 0.105415 | 0.0199 | 1.01195 | 1 | rs1867750 | 2 | rs7564432 |
| 0.915614 | 1.0149 | 1557 | 0.977521 | 4626 | 0.977194 | 0.977276 | 0.0112 | 1 | 3 | rs1545360 | | |
| 0.9646863 | 0.998 | 1558 | 0.700899 | 4627 | 0.701318 | 0.701213 | 0.002 | 1 | 3 | rs1867750 | | |
| 0.9767802 | 0.9959 | 1558 | 0.021916 | 4627 | 0.022003 | 0.021981 | 0.0008 | 1.023095 | 1 | rs1545360 | 1 | rs10165398 |

**Table 8: Association of SNP markers and 2-marker haplotypes in the rs4848543 LD block with risk for breast cancer (phenotype Medium Predisposition Breast Cancer): The allele codes are as follows: 1=A, 2=C, 3=G, 4=T.**

| **p-value** | **RR** | **#aff** | **aff.freq** | **#con** | **con.freq** | **H0.freq** | **X2** | **info_alt** | **all** | **mrk** | **all** | **mrk** |
|---|---|---|---|---|---|---|---|---|---|---|---|---|
| 9.40E-10 | 1.4567 | 632 | 0.427215 | 4626 | 0.33863 | 0.349277 | 37.4465 | 1 | 1 | rs4848543 | | |
| 1.33E-06 | 0.748 | 632 | 0.464983 | 4627 | 0.537453 | 0.528745 | 23.3794 | 0.999257 | 2 | rs895398 | 3 | rs6759589 |
| 1.51E-06 | 1.3348 | 632 | 0.534019 | 4626 | 0.461954 | 0.470616 | 23.1368 | 1 | 1 | rs6759589 | | |
| 1.62E-06 | 1.3411 | 632 | 0.428798 | 4627 | 0.358872 | 0.367275 | 22.9946 | 1 | 1 | rs12711924 | | |
| 2.57E-06 | 1.4384 | 632 | 0.214003 | 4627 | 0.159157 | 0.165836 | 22.1127 | 0.970516 | 2 | rs4849765 | 4 | rs895398 |
| 4.01E-06 | 1.4104 | 632 | 0.224684 | 4626 | 0.17045 | 0.176968 | 21.2621 | 1 | 4 | rs895398 | | |
| 1.00E-05 | 1.3099 | 632 | 0.61788 | 4622 | 0.552466 | 0.560335 | 19.5116 | 1 | 3 | rs838100 | | |
| 0.0000125 | 1.3136 | 632 | 0.386076 | 4627 | 0.323752 | 0.331242 | 19.0859 | 1 | 4 | rs3731603 | | |
| 1.398E-05 | 1.3039 | 632 | 0.617089 | 4625 | 0.552757 | 0.560491 | 18.8713 | 1 | 3 | rs838102 | | |
| 0.0000142 | 0.7657 | 632 | 0.37216 | 4627 | 0.43636 | 0.428644 | 18.8419 | 0.995144 | 3 | rs6759589 | 1 | rs838102 |
| 1.579E-05 | 1.388 | 632 | 0.230115 | 4619 | 0.177183 | 0.184123 | 18.6401 | 0.943665 | 2 | rs4849765 | 4 | rs2084154 |
| 0.0000258 | 1.2911 | 632 | 0.588229 | 4627 | 0.525264 | 0.532825 | 17.7045 | 0.993478 | 2 | rs4849760 | 3 | rs838102 |
| 0.0004697 | 1.2366 | 631 | 0.444533 | 4626 | 0.392888 | 0.399087 | 12.2324 | 1 | 4 | rs1530561 | | |
| 0.0007986 | 0.7553 | 632 | 0.139112 | 4627 | 0.176242 | 0.171752 | 11.2444 | 0.995218 | 3 | rs6759589 | 2 | rs838066 |
| 0.0025521 | 1.2002 | 632 | 0.452532 | 4627 | 0.407824 | 0.413196 | 9.1029 | 1 | 3 | rs838069 | | |
| 0.0027736 | 1.2608 | 632 | 0.196994 | 4626 | 0.162884 | 0.166984 | 8.9507 | 1 | 4 | rs935361 | | |
| 0.0038501 | 1.5279 | 632 | 0.074928 | 4627 | 0.050342 | 0.053363 | 8.3532 | 0.690307 | 2 | rs838086 | 1 | rs12711924 |
| 0.0086964 | 0.8452 | 631 | 0.317499 | 4621 | 0.355011 | 0.350481 | 6.8841 | 0.989658 | 3 | rs2084154 | 4 | rs838066 |
| 0.0109182 | 1.1925 | 632 | 0.264011 | 4627 | 0.231241 | 0.235192 | 6.4786 | 0.998578 | 1 | rs745888 | 2 | rs838087 |
| 0.0116941 | 1.1903 | 632 | 0.264241 | 4625 | 0.231784 | 0.235686 | 6.3567 | 1 | 1 | rs745888 | | |
| 0.0125611 | 0.8469 | 632 | 0.704114 | 4627 | 0.737523 | 0.733507 | 6.2299 | 0.99995 | 2 | rs4849760 | 3 | rs745888 |
| 0.0140093 | 0.8557 | 632 | 0.326665 | 4627 | 0.361823 | 0.357582 | 6.037 | 0.997006 | 3 | rs6759589 | 4 | rs838066 |
| 0.0167713 | 1.1975 | 632 | 0.20807 | 4627 | 0.179922 | 0.183305 | 5.7202 | 1 | 2 | rs7564432 | 2 | rs11680207 |
| 0.0206458 | 0.8629 | 632 | 0.328408 | 4627 | 0.36172 | 0.357717 | 5.3565 | 0.986182 | 2 | rs895398 | 4 | rs838066 |
| 0.0250242 | 0.8675 | 632 | 0.431088 | 4627 | 0.466247 | 0.462051 | 5.0222 | 0.905031 | 1 | rs838086 | 1 | rs838069 |
| 0.0262532 | 0.772 | 632 | 0.068325 | 4627 | 0.086756 | 0.084542 | 4.9393 | 0.958473 | 3 | rs12711924 | 3 | rs838069 |
| 0.0331553 | 1.1454 | 621 | 0.668277 | 4559 | 0.63753 | 0.641216 | 4.5377 | 1 | 2 | rs838066 | | |
| 0.0420433 | 0.8752 | 632 | 0.312809 | 4627 | 0.342145 | 0.33827 | 4.1335 | 0.956363 | 4 | rs2084154 | 2 | rs895398 |
| 0.0490954 | 1.1279 | 632 | 0.413766 | 4627 | 0.384915 | 0.388382 | 3.8721 | 1 | 2 | rs11680207 | | |
| 0.0545878 | 1.1328 | 625 | 0.6944 | 4602 | 0.667319 | 0.670557 | 3.6946 | 1 | 2 | rs4849765 | | |
| 0.0565963 | 1.1614 | 632 | 0.216878 | 4627 | 0.19254 | 0.195451 | 3.6344 | 0.887609 | 3 | rs1867750 | 2 | rs11680207 |
| 0.1002333 | 1.1066 | 609 | 0.536125 | 3962 | 0.510853 | 0.51422 | 2.7018 | 1 | 4 | rs2084154 | | |
| 0.1098139 | 1.4252 | 630 | 0.983333 | 4049 | 0.976414 | 0.977346 | 2.5569 | 1 | 2 | rs838087 | | |
| 0.2444521 | 0.9299 | 632 | 0.450621 | 4627 | 0.46866 | 0.466267 | 1.3547 | 0.930417 | 3 | rs7562894 | 3 | rs2084154 |
| 0.2972591 | 1.0681 | 632 | 0.36608 | 4627 | 0.350928 | 0.352761 | 1.0865 | 0.9763 | 3 | rs1439945 | 3 | rs725346 |
| 0.324048 | 0.9409 | 632 | 0.600571 | 4626 | 0.615101 | 0.613351 | 0.9725 | 0.985757 | 2 | rs6542504 | 2 | rs12711923 |
| 0.3389162 | 1.0619 | 632 | 0.363391 | 4627 | 0.349623 | 0.351284 | 0.9145 | 0.992987 | 2 | rs330763 | 3 | rs10496556 |
| 0.3390482 | 0.9427 | 632 | 0.596933 | 4627 | 0.611053 | 0.609353 | 0.914 | 0.984363 | 2 | rs6542504 | 2 | rs4849760 |
| 0.3489783 | 1.0609 | 632 | 0.360144 | 4627 | 0.346646 | 0.348274 | 0.8772 | 0.98708 | 2 | rs330763 | 3 | rs725346 |
| 0.363347 | 1.0608 | 632 | 0.321891 | 4626 | 0.309141 | 0.310678 | 0.8263 | 0.984258 | 4 | rs6542504 | 2 | rs12711923 |
| 0.4496627 | 0.9444 | 632 | 0.212841 | 4627 | 0.222576 | 0.221424 | 0.5715 | 0.927154 | 1 | rs10496555 | 3 | rs745888 |
| 0.457771 | 1.047 | 631 | 0.385103 | 4610 | 0.374295 | 0.375596 | 0.5513 | 1 | 4 | rs6542504 | | |
| 0.4606554 | 0.9296 | 632 | 0.098529 | 4627 | 0.105201 | 0.104399 | 0.5443 | 1.01297 | 1 | rs1867750 | 2 | rs7564432 |
| 0.47764 | 1.0451 | 630 | 0.374603 | 4610 | 0.364317 | 0.365553 | 0.5042 | 1 | 3 | rs725346 | | |
| 0.4879223 | 1.1566 | 632 | 0.980221 | 4626 | 0.977194 | 0.977558 | 0.4811 | 1 | 3 | rs1545360 | | |
| 0.498098 | 1.045 | 632 | 0.343589 | 4627 | 0.333725 | 0.334913 | 0.459 | 0.948386 | 3 | rs10496555 | 3 | rs725346 |
| 0.5144968 | 1.0412 | 632 | 0.380538 | 4619 | 0.371076 | 0.372215 | 0.4249 | 1 | 3 | rs10496556 | | |
| 0.5466543 | 1.0398 | 632 | 0.348742 | 4627 | 0.339941 | 0.341001 | 0.3633 | 0.950967 | 3 | rs10496555 | 3 | rs10496556 |
| 0.5484699 | 0.8812 | 632 | 0.019407 | 4627 | 0.021965 | 0.021657 | 0.3601 | 1.017257 | 1 | rs1545360 | 1 | rs10165398 |
| 0.6700167 | 0.9696 | 632 | 0.216772 | 4627 | 0.222066 | 0.22143 | 0.1816 | 1 | 2 | rs11680207 | 2 | rs935361 |
| 0.7411272 | 0.9806 | 632 | 0.413979 | 4627 | 0.41875 | 0.418177 | 0.1091 | 1.048229 | 3 | rs10496555 | 1 | rs725346 |
| 0.7734425 | 0.971 | 632 | 0.090995 | 4627 | 0.093457 | 0.093161 | 0.0829 | 1.032733 | 4 | rs330773 | 3 | rs745888 |
| 0.8205628 | 1.0258 | 632 | 0.07753 | 4622 | 0.075725 | 0.075942 | 0.0514 | 1 | 1 | rs12711923 | | |
| 0.8541421 | 0.9688 | 632 | 0.968355 | 4627 | 0.969313 | 0.969196 | 0.0338 | 1 | 2 | rs4849760 | | |
| 0.9360615 | 0.9944 | 628 | 0.23965 | 4614 | 0.240681 | 0.240557 | 0.0064 | 1 | 2 | rs838086 | | |
| 0.9536622 | 0.9957 | 632 | 0.794304 | 4627 | 0.795007 | 0.794923 | 0.0034 | 1 | 2 | rs7564432 | | |
| 0.9754573 | 1.002 | 632 | 0.70174 | 4627 | 0.701318 | 0.701369 | 0.0009 | 1 | 3 | rs1867750 | | |

**Table 9: Haplotype Diversity of the rs4848543 Right-half Sub-block:**

| (The allele codes are as follows: 1=A, 2=C, 3=G, 4=T) | | | | | | | | | | | | | | |
|---|---|---|---|---|---|---|---|---|---|---|---|---|---|---|
| p-val | r | #aff | aff.freq | #con | con.freq | info_alt | | | | | | | | |
| 2.65E-02 | 1.23 | 653 | 0.131 | 4480 | 0.109 | 0.96 | 2 rs895398 1 | rs4848543 1 | rs6759589 2 | rs838066 3 | rs838100 1 | rs838086 1 | rs12711924 4 | rs3731603 |
| 1.51E-02 | 1.26 | 653 | 0.123 | 4480 | 0.100 | 0.94 | 4 rs895398 1 | rs4848543 1 | rs6759589 2 | rs838066 3 | rs838100 1 | rs838086 1 | rs12711924 4 | rs3731603 |
| 1.70E-02 | 1.43 | 653 | 0.051 | 4480 | 0.036 | 0.90 | 4 rs895398 1 | rs4848543 1 | rs6759589 2 | rs838066 3 | rs838100 2 | rs838086 1 | rs12711924 4 | rs3731603 |
| 2.97E-01 | 1.17 | 653 | 0.043 | 4480 | 0.037 | 0.99 | 2 rs895398 1 | rs4848543 1 | rs6759589 2 | rs838066 3 | rs838100 1 | rs838086 1 | rs12711924 3 | rs3731603 |
| 1.19E-02 | 1.55 | 653 | 0.039 | 4480 | 0.026 | 0.89 | 4 rs895398 1 | rs4848543 1 | rs6759589 2 | rs838066 3 | rs838100 2 | rs838086 3 | rs12711924 3 | rs3731603 |
| 3.10E-01 | 1.36 | 653 | 0.015 | 4480 | 0.011 | 0.76 | 2 rs895398 1 | rs4848543 1 | rs6759589 2 | rs838066 3 | rs838100 2 | rs838086 1 | rs12711924 4 | rs3731603 |
| | | | | | | | | | | | | | | |
| 2.08E-02 | 0.85 | 653 | 0.244 | 4480 | 0.274 | 0.97 | 2 rs895398 2 | rs4848543 3 | rs6759589 4 | rs838066 1 | rs838100 1 | rs838086 3 | rs12711924 3 | rs3731603 |
| 6.56E-03 | 0.77 | 653 | 0.107 | 4480 | 0.134 | 0.96 | 2 rs895398 2 | rs4848543 3 | rs6759589 2 | rs838066 1 | rs838100 1 | rs838086 3 | rs12711924 3 | rs3731603 |
| 5.95E-02 | 0.81 | 653 | 0.072 | 4480 | 0.088 | 0.96 | 2 rs895398 2 | rs4848543 1 | rs6759589 2 | rs838066 3 | rs838100 2 | rs838086 3 | rs12711924 3 | rs3731603 |
| 3.91E-01 | 0.88 | 653 | 0.043 | 4480 | 0.049 | 0.90 | 2 rs895398 2 | rs4848543 3 | rs6759589 4 | rs838066 3 | rs838100 2 | rs838086 3 | rs12711924 3 | rs3731603 |
| 3.00E-02 | 0.59 | 653 | 0.014 | 4480 | 0.024 | 0.83 | 2 rs895398 2 | rs4848543 3 | rs6759589 2 | rs838066 3 | rs838100 2 | rs838086 3 | rs12711924 3 | rs3731603 |
| 8.84E-01 | 1.03 | 653 | 0.022 | 4480 | 0.021 | 0.91 | 2 rs895398 2 | rs4848543 1 | rs6759589 2 | rs838066 3 | rs838100 1 | rs838086 1 | rs12711924 4 | rs3731603 |
| 8.33E-01 | 1.05 | 653 | 0.022 | 4480 | 0.021 | 0.91 | 2 rs895398 2 | rs4848543 3 | rs6759589 4 | rs838066 3 | rs838100 1 | rs838086 1 | rs12711924 4 | rs3731603 |
| 7.42E-01 | 1.09 | 653 | 0.015 | 4480 | 0.013 | 0.88 | 2 rs895398 2 | rs4848543 1 | rs6759589 2 | rs838066 3 | rs838100 1 | rs838086 3 | rs12711924 3 | rs3731603 |
| 6.26E-01 | 0.86 | 653 | 0.010 | 4480 | 0.012 | 0.91 | 2 rs895398 2 | rs4848543 3 | rs6759589 2 | rs838066 1 | rs838100 1 | rs838086 1 | rs12711924 4 | rs3731603 |

**Table 10: HapMap SNP markers that are correlated with rs4848543 with r² value greater than 0.2:**

| **Surrogate SNP** | **Ref SNP** | **D'** | **R²** | **p-value** | **Pos in Build 34** | **Pos in Build 36** | **Pos in SEQ ID 4** |
|---|---|---|---|---|---|---|---|
| rs11680207 | rs4848543 | 0.653174 | 0.309094 | 4.55E-09 | 120023583 | 119644908 | 301 |
| rs7583824 | rs4848543 | 0.655931 | 0.322684 | 3.29E-09 | 120027724 | 119649049 | 4442 |
| rs4848541 | rs4848543 | 0.656671 | 0.243502 | 8.45E-06 | 120032169 | 119653494 | 8887 |
| rs1446128 | rs4848543 | 0.628705 | 0.317935 | 8.08E-09 | 120032378 | 119653703 | 9096 |
| rs4849766 | rs4848543 | 0.851071 | 0.31689 | 7.16E-09 | 120034354 | 119655679 | 11072 |
| rs6715243 | rs4848543 | 0.923886 | 0.362632 | 3.83E-10 | 120034730 | 119656055 | 11448 |
| rs4142753 | rs4848543 | 0.934092 | 0.413776 | 2.96E-12 | 120051961 | 119673286 | 28679 |
| rs10188745 | rs4848543 | 0.934092 | 0.413776 | 2.96E-12 | 120057058 | 119678383 | 33776 |
| rs895398 | rs4848543 | 0.934092 | 0.413776 | 2.96E-12 | 120058180 | 119679505 | 34898 |
| rs4848543 | rs4848543 | 1 | 1 | 0 | 120061096 | 119682421 | 37814 |
| rs11684731 | rs4848543 | 1 | 0.319149 | 2.08E-12 | 120062319 | 119683644 | 39037 |
| rs12464139 | rs4848543 | 1 | 0.294118 | 1.69E-11 | 120062996 | 119684321 | 39714 |
| rs6754664 | rs4848543 | 1 | 0.963415 | 4.90E-33 | 120063070 | 119684395 | 39788 |
| rs6754799 | rs4848543 | 1 | 0.673648 | 6.09E-22 | 120063176 | 119684501 | 39894 |
| rs6759589 | rs4848543 | 1 | 0.534483 | 2.36E-19 | 120064976 | 119686301 | 41694 |
| rs1562256 | rs4848543 | 0.951061 | 0.50597 | 1.03E-15 | 120066318 | 119687643 | 43036 |
| rs1446125 | rs4848543 | 1 | 1 | 4.29E-31 | 120066620 | 119687945 | 43338 |
| rs1446124 | rs4848543 | 1 | 0.963415 | 4.90E-33 | 120066705 | 119688030 | 43423 |
| rs11677262 | rs4848543 | 1 | 0.30137 | 6.60E-12 | 120067290 | 119688615 | 44008 |
| rs7565771 | rs4848543 | 1 | 0.307692 | 2.81E-12 | 120070400 | 119691725 | 47118 |
| rs7595954 | rs4848543 | 1 | 0.289474 | 5.19E-12 | 120070666 | 119691991 | 47384 |
| rs838066 | rs4848543 | 1 | 0.3 | 2.40E-12 | 120071508 | 119692833 | 48226 |
| rs895397 | rs4848543 | 1 | 1 | 4.32E-35 | 120072400 | 119693725 | 49118 |
| rs838059 | rs4848543 | 0.841262 | 0.353861 | 2.97E-10 | 120075395 | 119696720 | 52113 |
| rs838102 | rs4848543 | 1 | 0.321918 | 4.92E-13 | 120079878 | 119701203 | 56596 |
| rs3769659 | rs4848543 | 0.849119 | 0.718225 | 2.57E-20 | 120081128 | 119702453 | 57846 |
| rs865108 | rs4848543 | 1 | 0.376923 | 4.62E-14 | 120081529 | 119702854 | 58247 |
| rs708670 | rs4848543 | 1 | 0.302817 | 4.27E-12 | 120082382 | 119703707 | 59100 |
| rs708672 | rs4848543 | 1 | 0.632075 | 3.65E-22 | 120082499 | 119703824 | 59217 |
| rs708673 | rs4848543 | 1 | 0.3125 | 1.13E-12 | 120082871 | 119704196 | 59589 |
| rs838100 | rs4848543 | 1 | 0.3 | 2.40E-12 | 120083710 | 119705035 | 60428 |
| rs838098 | rs4848543 | 1 | 0.3125 | 1.13E-12 | 120084226 | 119705551 | 60944 |
| rs838096 | rs4848543 | 1 | 0.310811 | 1.10E-12 | 120084741 | 119706066 | 61459 |
| rs838092 | rs4848543 | 1 | 0.321918 | 4.92E-13 | 120091298 | 119712623 | 68016 |
| rs838090 | rs4848543 | 0.780443 | 0.224773 | 5.57E-06 | 120092304 | 119713629 | 69022 |
| rs12467944 | rs4848543 | 0.865803 | 0.535809 | 9.44E-15 | 120094306 | 119715631 | 71024 |
| rs10196144 | rs4848543 | 0.84638 | 0.686857 | 4.46E-19 | 120095312 | 119716637 | 72030 |
| rs6728660 | rs4848543 | 0.872481 | 0.70845 | 3.68E-18 | 120095756 | 119717081 | 72474 |
| rs10188946 | rs4848543 | 0.74499 | 0.446479 | 2.07E-12 | 120098925 | 119720250 | 75643 |
| rs895406 | rs4848543 | 0.790753 | 0.304687 | 1.52E-09 | 120104365 | 119725690 | 81083 |
| rs708675 | rs4848543 | 0.707874 | 0.418005 | 1.81E-11 | 120104502 | 119725827 | 81220 |
| rs1867856 | rs4848543 | 0.77707 | 0.512514 | 2.87E-13 | 120104672 | 119725997 | 81390 |
| rs3754847 | rs4848543 | 0.717855 | 0.22469 | 4.70E-07 | 120105720 | 119727045 | 82438 |
| rs12711924 | rs4848543 | 0.792931 | 0.561965 | 3.43E-15 | 120107316 | 119728641 | 84034 |
| rs1867749 | rs4848543 | 0.75345 | 0.214069 | 6.37E-07 | 120109057 | 119730382 | 85775 |
| rs838073 | rs4848543 | 0.709981 | 0.247536 | 5.75E-07 | 120109754 | 119731079 | 86472 |
| rs3731603 | rs4848543 | 0.784597 | 0.392356 | 2.49E-10 | 120117062 | 119738387 | 93780 |

**Table 11: Association of SNP SG02S728 (rs13387042) allele A with risk for breast cancer:**

| **Cohort** | **p-val.corr*** | **p-val** | **r** | **95%Cl** | **#aff** | **aff.freq** | **#con** | **con.freq** |
|---|---|---|---|---|---|---|---|---|
| MedPre BC Illumina | 4.4E-03 | 2.1E-03 | 1.20 | | 652 | 0.496 | 4475 | 0.451 |
| MedPre BC Iceland Replication | 2.3E-03 | 1.8E-03 | 1.37 | | 200 | 0.528 | 7966 | 0.449 |
| MedPre BC All Icelandic | 8.5E-05 | 1.5E-05 | 1.24 | (1.12,1.38) | 852 | 0.504 | 12441 | 0.449 |
| Any BC Illumina | 4.0E-05 | 2.1E-05 | 1.19 | | 1598 | 0.494 | 4475 | 0.451 |
| Any BC Icelandic Replication | 3.8E-03 | 3.3E-03 | 1.20 | | 583 | 0.493 | 7966 | 0.449 |
| Any BC All Icelandic | 2.0E-07 | 5.0E-08 | 1.20 | (1.12,1.28) | 2181 | 0.494 | 12441 | 0.449 |
| Spain | | 1.8E-02 | 1.21 | (1.03,1.42) | 446 | 0.581 | 977 | 0.533 |
| Sweden Familial | | 2.2E-01 | 1.11 | | 346 | 0.516 | 1734 | 0.490 |
| Sweden Consecutive | | 2.0E-04 | 1.31 | | 482 | 0.558 | 1734 | 0.490 |
| Sweden All | | 8.1E-04 | 1.22 | (1.09,1.37) | 828 | 0.540 | 1734 | 0.490 |
| All cohorts joint | 3.8E-11 | | 1.20 | (1.14,1.27) | 3455 | 0.516 | 15152 | 0.460 |

| | | | | | | | | |
|---|---|---|---|---|---|---|---|---|
| *corrected for relationships between individuals | | | | | | | | |

**Table 12: Definition of the rs13387042 LD-block on Chromosome 2:**

| **NCBI Build** | **Start** | **End** |
|---|---|---|
| 34 | 218,062,001 | 218,141,002 |
| 36 | 217,567,902 | 217,646,679 |

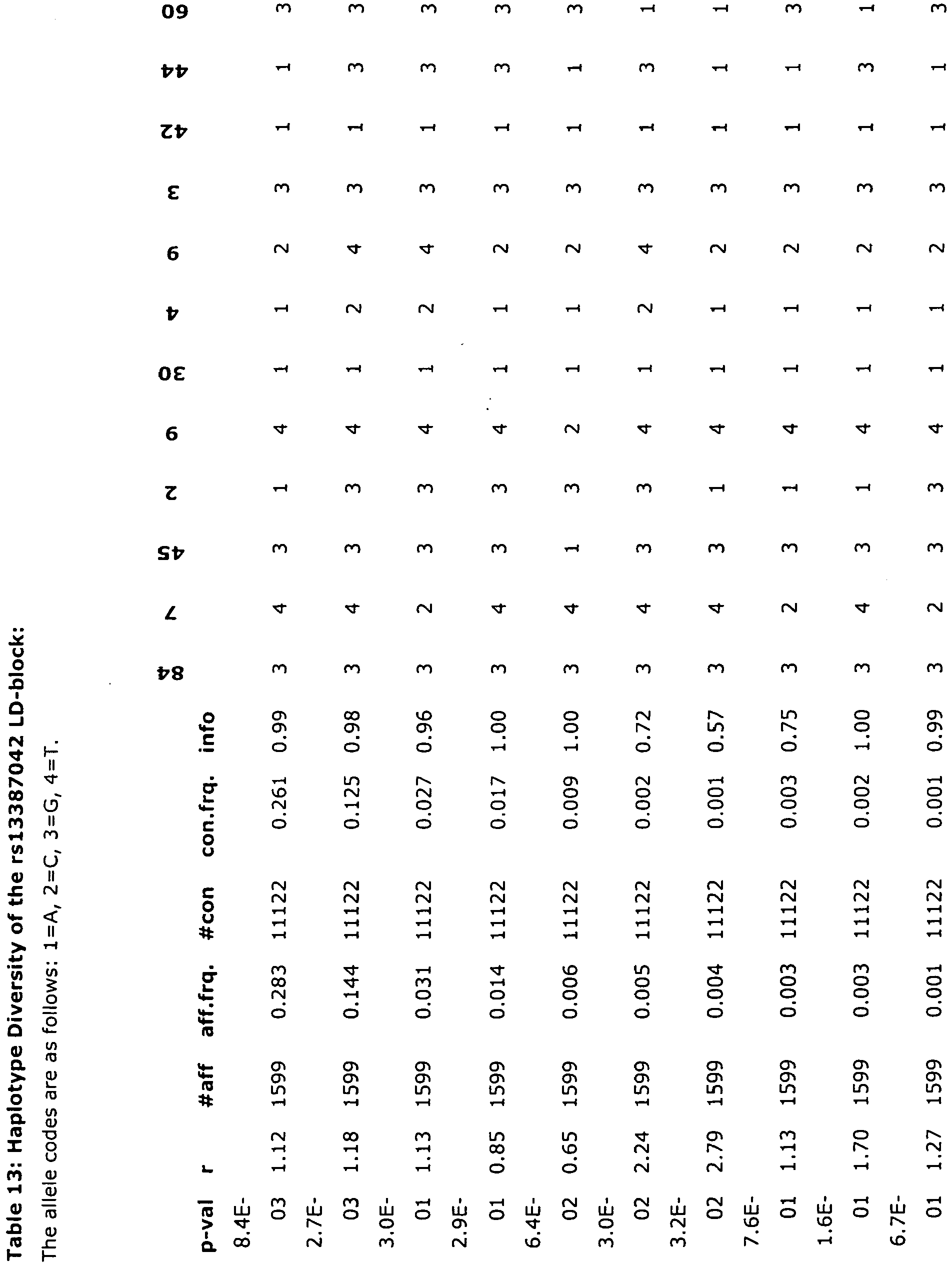

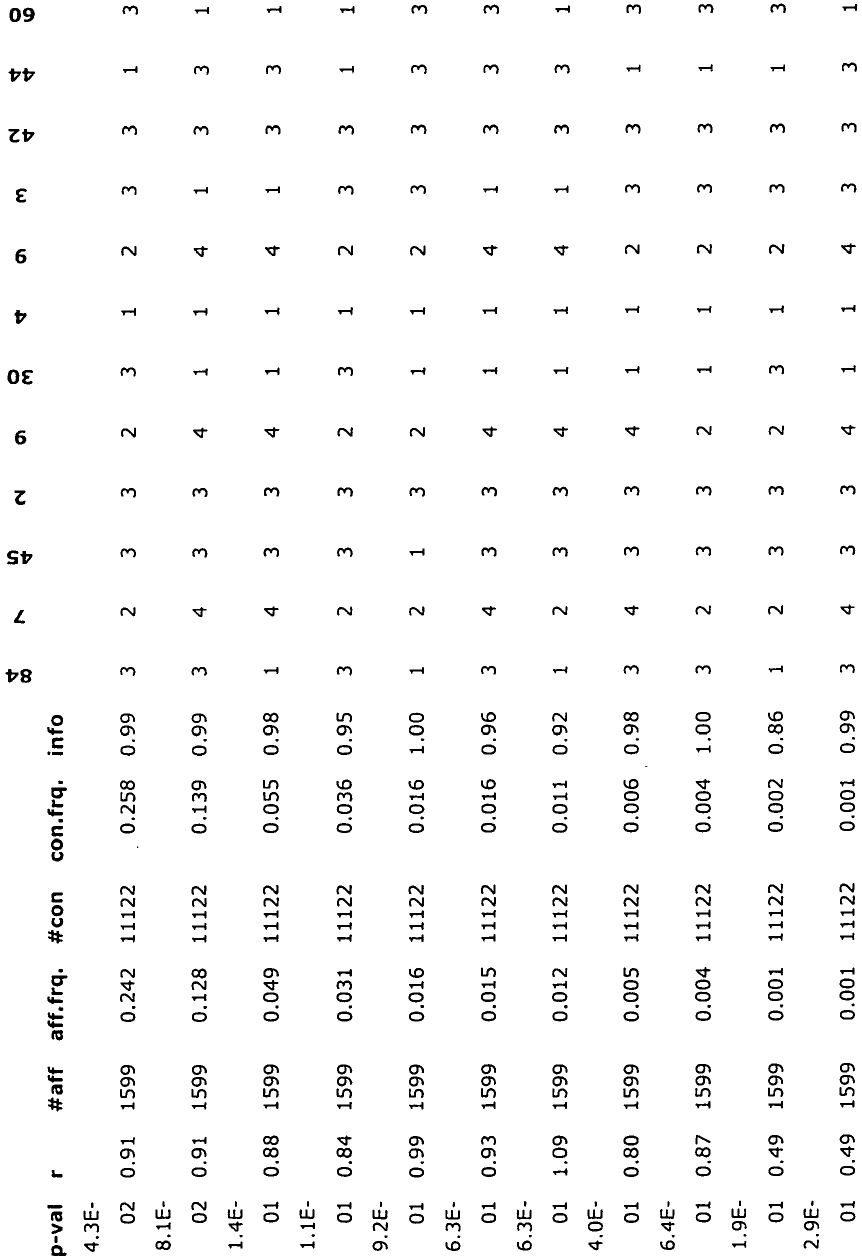

**Table 14: Association of SNP markers and 2-marker haplotypes in the rs13387042 LD-block with risk for breast cancer (phenotype Any Breast Cancer): The allele codes are as follows: 1=A, 2=C, 3=G, 4=T.**

| **pvcorr** | **r** | **#aff** | **aff.freq** | **#con** | **con.freq** | **allele** | **marker** | **allele** | **marker** |
|---|---|---|---|---|---|---|---|---|---|
| 7.58E-06 | 1.1913 | 1658 | 0.493969 | 11547 | 0.450377 | 1 | rs13387042 | | |
| 8.59E-04 | 1.1892 | 1660 | 0.178916 | 11556 | 0.154855 | 2 | rs6735174 | | |
| 8.04E-03 | 1.1216 | 1660 | 0.29247 | 11563 | 0.269307 | 1 | rs6716542 | | |
| 1.17E-02 | 1.1281 | 1660 | 0.796385 | 11561 | 0.776144 | 3 | rs2372943 | | |
| 1.24E-02 | 1.1214 | 1660 | 0.769879 | 11560 | 0.748962 | 3 | rs13011060 | | |
| 1.54E-02 | 1.1074 | 1659 | 0.6956 | 11560 | 0.673573 | 4 | rs4491709 | | |
| 2.65E-02 | 1.1007 | 1659 | 0.723026 | 11561 | 0.703399 | 1 | rs12621130 | | |
| 1.03E-01 | 1.0691 | 1660 | 0.655723 | 11563 | 0.640491 | 4 | rs6435957 | | |
| 2.37E-01 | 1.088 | 1659 | 0.920735 | 11560 | 0.91436 | 3 | rs10191184 | | |
| 7.63E-01 | 1.0122 | 1660 | 0.383133 | 11560 | 0.380277 | 4 | rs6435959 | | |
| 7.74E-01 | 1.0367 | 1660 | 0.975602 | 11555 | 0.974729 | 3 | rs10171745 | | |
| 8.45E-01 | 1.0078 | 1660 | 0.417169 | 11563 | 0.41529 | 3 | rs10490444 | | |
| 9.54E-06 | 0.8405 | 1660 | 0.489993 | 11563 | 0.533373 | 3 | rs10171745 | 3 | rs13387042 |
| 4.24E-03 | 0.8771 | 1659 | 0.22895 | 11562 | 0.25292 | 1 | rs12621130 | 3 | rs13387042 |
| 6.79E-03 | 1.1253 | 1660 | 0.288931 | 11563 | 0.265289 | 4 | rs6435957 | 1 | rs6716542 |
| 7.96E-03 | 1.1219 | 1660 | 0.29198 | 11563 | 0.268789 | 1 | rs6716542 | 4 | rs4491709 |
| 8.04E-03 | 1.1216 | 1660 | 0.29247 | 11563 | 0.269307 | 1 | rs6716542 | 2 | rs6435959 |
| 8.04E-03 | 1.1216 | 1660 | 0.29247 | 11563 | 0.269307 | 3 | rs10171745 | 1 | rs6716542 |
| 9.38E-03 | 0.8938 | 1660 | 0.286833 | 11563 | 0.310333 | 2 | rs4491709 | 1 | rs10490444 |
| 1.03E-02 | 1.118 | 1660 | 0.289694 | 11563 | 0.267286 | 1 | rs6716542 | 1 | rs10490444 |
| 1.29E-02 | 0.9003 | 1660 | 0.683133 | 11563 | 0.705426 | 3 | rs10171745 | 3 | rs6716542 |
| 1.80E-02 | 0.8986 | 1660 | 0.304278 | 11563 | 0.327368 | 4 | rs2705593 | 3 | rs13387042 |
| 2.92E-02 | 1.0966 | 1660 | 0.315096 | 11562 | 0.295536 | 1 | rs6735174 | 1 | rs13387042 |
| 3.27E-02 | 1.0947 | 1660 | 0.312348 | 11563 | 0.293242 | 4 | rs4491709 | 2 | rs6435959 |
| 3.40E-02 | 0.9119 | 1660 | 0.275355 | 11563 | 0.294127 | 3 | rs10191184 | 3 | rs12621130 |
| 3.60E-02 | 0.9128 | 1660 | 0.274287 | 11563 | 0.29282 | 4 | rs2705593 | 3 | rs12621130 |
| 6.69E-02 | 1.0758 | 1660 | 0.405656 | 11563 | 0.388167 | 3 | rs10171745 | 1 | rs4674132 |
| 1.08E-01 | 0.9339 | 1660 | 0.315371 | 11563 | 0.33033 | 3 | rs10191184 | 2 | rs6435957 |
| 1.27E-01 | 1.0702 | 1660 | 0.293447 | 11563 | 0.279572 | 4 | rs6435957 | 1 | rs10490444 |
| 1.53E-01 | 1.0653 | 1660 | 0.270411 | 11563 | 0.258112 | 4 | rs2705593 | 4 | rs6435957 |
| 3.74E-01 | 1.0381 | 1660 | 0.335709 | 11563 | 0.327421 | 2 | rs2705593 | 3 | rs10191184 |
| 6.52E-01 | 0.9825 | 1660 | 0.544171 | 11563 | 0.548548 | 1 | rs12621130 | 1 | rs6735174 |
| 6.88E-01 | 0.984 | 1660 | 0.61747 | 11563 | 0.621271 | 1 | rs6735174 | 3 | rs2372943 |
| 7.59E-01 | 0.958 | 1660 | 0.028119 | 11563 | 0.029317 | 1 | rs10191184 | 2 | rs6435957 |
| 8.45E-01 | 0.9923 | 1660 | 0.582831 | 11563 | 0.58471 | 1 | rs6735174 | 1 | rs10490444 |
| 8.97E-01 | 0.9947 | 1660 | 0.355026 | 11563 | 0.356245 | 2 | rs2705593 | 3 | rs10490444 |
| 9.07E-01 | 0.9954 | 1660 | 0.403688 | 11563 | 0.404807 | 3 | rs6716542 | 4 | rs4491709 |
| 9.61 E-01 | 1.0068 | 1660 | 0.026373 | 11563 | 0.026199 | 4 | rs2705593 | 1 | rs10191184 |

**Table 15: HapMap SNP markers that are correlated with rs13387042 with an r² value greater than 0.2:**

| **Surrogate SNP** | **Ref SNP** | **D'** | **R2** | **p-value** | **Pos in Build 34** | **Pos in Build 36** | **Pos in SEQ ID 5** |
|---|---|---|---|---|---|---|---|
| rs1882419 | rs13387402 | 0.692153 | 0.331807 | 1.92E-09 | 218059508 | 217565211 | 301 |
| rs2252488 | rs13387402 | 1 | 0.258085 | 5.60E-11 | 218059992 | 217565695 | 785 |
| rs2272525 | rs13387402 | 0.849123 | 0.271771 | 1.07E-08 | 218061250 | 217566904 | 1994 |
| rs4396687 | rs13387402 | 1 | 0.268293 | 1.29E-10 | 218063010 | 217568713 | 3803 |
| rs12614767 | rs13387402 | 0.785371 | 0.245063 | 1.01E-07 | 218063508 | 217569211 | 4301 |
| rs4571035 | rs13387402 | 1 | 0.344501 | 1.91E-13 | 218064351 | 217570054 | 5144 |
| rs10195963 | rs13387402 | 1 | 0.324895 | 7.05E-13 | 218065238 | 217570941 | 6031 |
| rs10207736 | rs13387402 | 0.573221 | 0.236068 | 4.26E-07 | 218066096 | 217571799 | 6889 |
| rs12464018 | rs13387402 | 0.847812 | 0.263762 | 1.33E-08 | 218067143 | 217572846 | 7936 |
| rs10932689 | rs13387402 | 0.880377 | 0.389169 | 5.54E-12 | 218068532 | 217574235 | 9325 |
| rs12613030 | rs13387402 | 0.917661 | 0.281049 | 3.18E-09 | 218068906 | 217574609 | 9699 |
| rs2888450 | rs13387402 | 1 | 0.323308 | 4.19E-13 | 218069383 | 217575086 | 10176 |
| rs4254482 | rs13387402 | 1 | 0.304413 | 5.14E-12 | 218069959 | 217575662 | 10752 |
| rs2372932 | rs13387402 | 1 | 0.268293 | 2.98E-11 | 218069975 | 217575678 | 10768 |
| rs4255939 | rs13387402 | 1 | 0.300466 | 2.28E-12 | 218070431 | 217576134 | 11224 |
| rs10192415 | rs13387402 | 1 | 0.33518 | 1.75E-13 | 218073196 | 217578899 | 13989 |
| rs7595393 | rs13387402 | 1 | 0.319631 | 7.19E-13 | 218073796 | 217579499 | 14589 |
| rs2372935 | rs13387402 | 1 | 0.331897 | 4.68E-13 | 218076131 | 217581834 | 16924 |
| rs6733648 | rs13387402 | 1 | 0.318182 | 6.69E-13 | 218077438 | 217583141 | 18231 |
| rs6435957 | rs13387402 | 0.586033 | 0.244261 | 1.46E-07 | 218080751 | 217586454 | 21544 |
| rs12614773 | rs13387402 | 0.911373 | 0.292062 | 1.74E-08 | 218084485 | 217590188 | 25278 |
| rs17833842 | rs13387402 | 1 | 0.345455 | 5.50E-12 | 218087663 | 217593366 | 28456 |
| rs10177578 | rs13387402 | 1 | 0.331897 | 2.95E-13 | 218088201 | 217593904 | 28994 |
| rs12623304 | rs13387402 | 0.919746 | 0.300741 | 1.15E-09 | 218090342 | 217596045 | 31135 |
| rs6716542 | rs13387402 | 1 | 0.33518 | 1.75E-13 | 218093185 | 217598888 | 33978 |
| rs17777330 | rs13387402 | 1 | 0.33518 | 1.75E-13 | 218094234 | 217599937 | 35027 |
| rs10211546 | rs13387402 | 1 | 0.312977 | 1.07E-12 | 218094939 | 217600642 | 35732 |
| rs2372937 | rs13387402 | 0.818494 | 0.335949 | 6.72E-10 | 218095256 | 217600959 | 36049 |
| rs4315498 | rs13387402 | 1 | 0.345455 | 5.50E-12 | 218095412 | 217601115 | 36205 |
| rs12998806 | rs13387402 | 1 | 0.33395 | 1.54E-11 | 218096305 | 217602008 | 37098 |
| rs12465515 | rs13387402 | 1 | 0.347368 | 7.21E-14 | 218096945 | 217602648 | 37738 |
| rs4491709 | rs13387402 | 0.81627 | 0.334787 | 8.36E-10 | 218097298 | 217603001 | 38091 |
| rs2372938 | rs13387402 | 0.818494 | 0.335949 | 6.72E-10 | 218097651 | 217603354 | 38444 |
| rs12621130 | rs13387402 | 1 | 0.345455 | 5.50E-12 | 218098168 | 217603871 | 38961 |
| rs13399995 | rs13387402 | 1 | 0.321168 | 7.70E-13 | 218100083 | 217605786 | 40876 |
| rs12052807 | rs13387402 | 0.788298 | 0.255063 | 4.96E-08 | 218100783 | 217606486 | 41576 |
| rs10199394 | rs13387402 | 1 | 0.347368 | 7.21E-14 | 218101273 | 217606976 | 42066 |
| rs2372940 | rs13387402 | 1 | 0.354179 | 2.00E-13 | 218101566 | 217607269 | 42359 |
| rs4583440 | rs13387402 | 0.818494 | 0.335949 | 6.72E-10 | 218101794 | 217607497 | 42587 |
| rs2372941 | rs13387402 | 1 | 0.315673 | 1.25E-12 | 218101850 | 217607553 | 42643 |
| rs6721811 | rs13387402 | 1 | 0.347368 | 7.21E-14 | 218102838 | 217608541 | 43631 |
| rs12615418 | rs13387402 | 1 | 0.345455 | 5.50E-12 | 218104345 | 217610048 | 45138 |
| rs12621884 | rs13387402 | 1 | 0.340033 | 2.71E-11 | 218104452 | 217610155 | 45245 |
| rs2372943 | rs13387402 | 1 | 0.345455 | 5.50E-12 | 218106318 | 217612021 | 47111 |
| rs4522583 | rs13387402 | 1 | 0.431818 | 1.56E-14 | 218106548 | 217612251 | 47341 |
| rs2888451 | rs13387402 | 1 | 0.33518 | 1.75E-13 | 218107055 | 217612758 | 47848 |
| rs2270398 | rs13387402 | 1 | 0.33395 | 1.84E-11 | 218107518 | 217613221 | 48311 |
| rs13412666 | rs13387402 | 1 | 1 | 5.65E-36 | 218108321 | 217614024 | 49114 |
| rs13387402 | rs13387402 | 1 | 1 | 0 | 218108374 | 217614077 | 49167 |
| rs13426489 | rs13387402 | 1 | 1 | 1.42E-35 | 218108788 | 217614491 | 49581 |
| rs6713249 | rs13387402 | 1 | 0.345455 | 5.50E-12 | 218109630 | 217615333 | 50423 |
| rs12620095 | rs13387402 | 1 | 0.863636 | 3.14E-29 | 218110056 | 217615759 | 50849 |
| rs4621152 | rs13387402 | 0.782931 | 0.262305 | 4.22E-09 | 218111527 | 217617230 | 52320 |
| rs6721996 | rs13387402 | 1 | 0.964912 | 4.45E-34 | 218112005 | 217617708 | 52798 |
| rs12622764 | rs13387402 | 1 | 0.345455 | 5.50E-12 | 218112403 | 217618106 | 53196 |
| rs6723013 | rs13387402 | 1 | 0.331897 | 4.68E-13 | 218113452 | 217619155 | 54245 |
| rs2372945 | rs13387402 | 1 | 0.385965 | 4.45E-15 | 218116654 | 217622357 | 57447 |
| rs12694403 | rs13387402 | 0.934804 | 0.388184 | 4.75E-12 | 218117956 | 217623659 | 58749 |
| rs12613955 | rs13387402 | 1 | 0.863636 | 4.73E-29 | 218118594 | 217624297 | 59387 |
| rs11894340 | rs13387402 | 0.781203 | 0.257717 | 6.21E-09 | 218119709 | 217625412 | 60502 |
| rs17778329 | rs13387402 | 1 | 0.35514 | 5.16E-12 | 218122353 | 217628056 | 63146 |
| rs4442975 | rs13387402 | 1 | 0.964912 | 4.45E-34 | 218123311 | 217629014 | 64104 |
| rs10932693 | rs13387402 | 1 | 0.324895 | 7.05E-13 | 218123663 | 217629366 | 64456 |
| rs17778427 | rs13387402 | 1 | 0.345455 | 5.50E-12 | 218125555 | 217631258 | 66348 |
| rs17835044 | rs13387402 | 1 | 0.345455 | 5.50E-12 | 218126147 | 217631850 | 66940 |
| rs7562029 | rs13387402 | 1 | 0.33518 | 1.75E-13 | 218126803 | 217632506 | 67596 |
| rs13000023 | rs13387402 | 0.923607 | 0.31255 | 5.05E-10 | 218126936 | 217632639 | 67729 |
| rs735361 | rs13387402 | 1 | 0.345455 | 5.50E-12 | 218127665 | 217633368 | 68458 |
| rs13409592 | rs13387402 | 1 | 0.210526 | 2.27E-09 | 218128870 | 217634573 | 69663 |
| rs2287289 | rs13387402 | 0.856459 | 0.284427 | 2.96E-09 | 218134039 | 217639742 | 74832 |
| rs12329133 | rs13387402 | 0.722969 | 0.239375 | 1.69E-07 | 218137658 | 217643361 | 78451 |
| rs13022815 | rs13387402 | 0.722969 | 0.239375 | 1.69E-07 | 218138666 | 217644369 | 79459 |
| rs13011060 | rs13387402 | 0.849849 | 0.279401 | 1.41E-08 | 218140719 | 217646422 | 81512 |
| rs13011326 | rs13387402 | 0.853195 | 0.289251 | 9.17E-09 | 218140952 | 217646655 | 81745 |
| rs4674132 | rs13387402 | 0.725082 | 0.249037 | 1.45E-07 | 218141061 | 217646764 | 81854 |

**Table 16: Association of SNP SG16S299 (rs3803662) allele T with risk for breast cancer: Association results for the allele T of the SNP rs3803662 on 16q12. Iceland 1 is the initial discovery cohort and Iceland 2 the independent replication group. All P-values are two-sided and are adjusted for relatedness and other potential population stratification of the Icelandic cases and controls. For combined non-Icelanders and for all cohorts combined the OR and P-values are calculated using the Mantel-Haenszel method and the displayed frequencies are obtained as a simple (arithmetic) average over the frequencies of individual groups.**

| **rs3803662 (T)** | **Frequency** | | | | | |
|---|---|---|---|---|---|---|
| **Cohort (Cases/Controls)** | **Cases** | **Controls** | **OR** | **(95% CI)** | **P** | **PAR** |
| | | | | | | |
| **Iceland 1 (1600/11563)** | 0.278 | 0.238 | 1.23 | (1.13,1.34) | 4.7E-06 | |
| **Iceland 2 (594/1433)** | 0.274 | 0.227 | 1.28 | (1.08,1.52) | 4.9E-03 | |
| **Iceland combined** | | | | | | |
| **(2194/12996)** | 0.276 | 0.237 | 1.23 | (1.14,1.33) | 2.8E-07 | 0.101 |
| | | | | | | |
| **Sweden (819/1723)** | 0.333 | 0.250 | 1.50 | (1.32,1.71) | 8.3E-10 | 0.209 |
| **Spain (455/1002)** | 0.348 | 0.308 | 1.20 | (1.02,1.42) | 3.1E-02 | 0.114 |
| **Holland (558/1384)** | 0.306 | 0.255 | 1.29 | (1.10,1.50) | 1.4E-03 | 0.131 |
| | | | | | | |
| **Non-Icelanders** | | | | | | |
| **(1832/4109)** | 0.329 | 0.271 | 1.35 | (1.24,1.47) | 5.1E-12 | 0.166 |
| | | | | | | |
| **All cohorts** | | | | | | |
| **(4026/17105)** | 0.316 | 0.262 | 1.28 | (1.21,1.36) | 2.7E-17 | 0.134 |

**Table 17: Definition of the rs3803662 LD-block on Chromosome 16:**

| **NCBI Build** | **Start** | **End** |
|---|---|---|
| 34 | 52,291,041 | 52,436,127 |
| 36 | 51,069,957 | 51,215,026 |

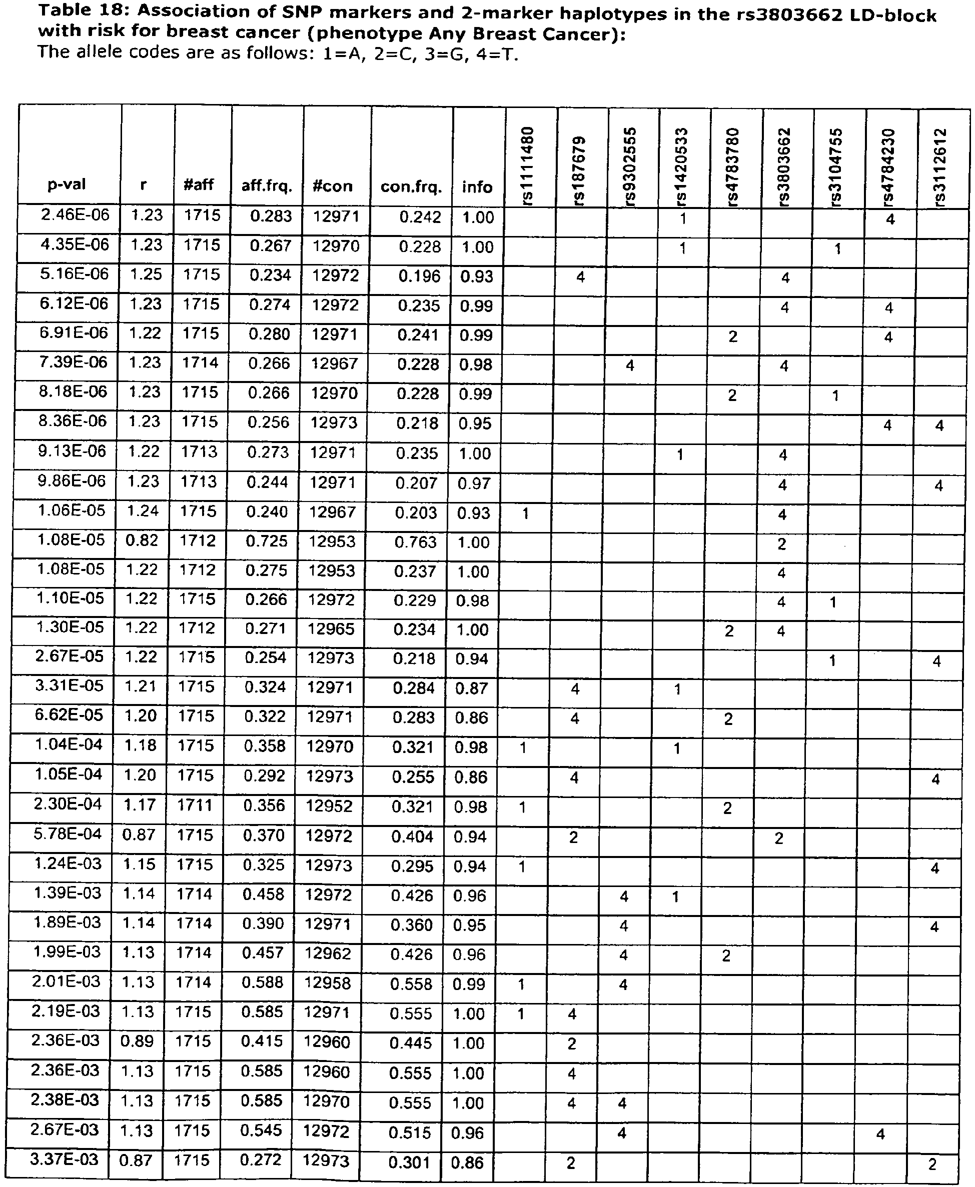

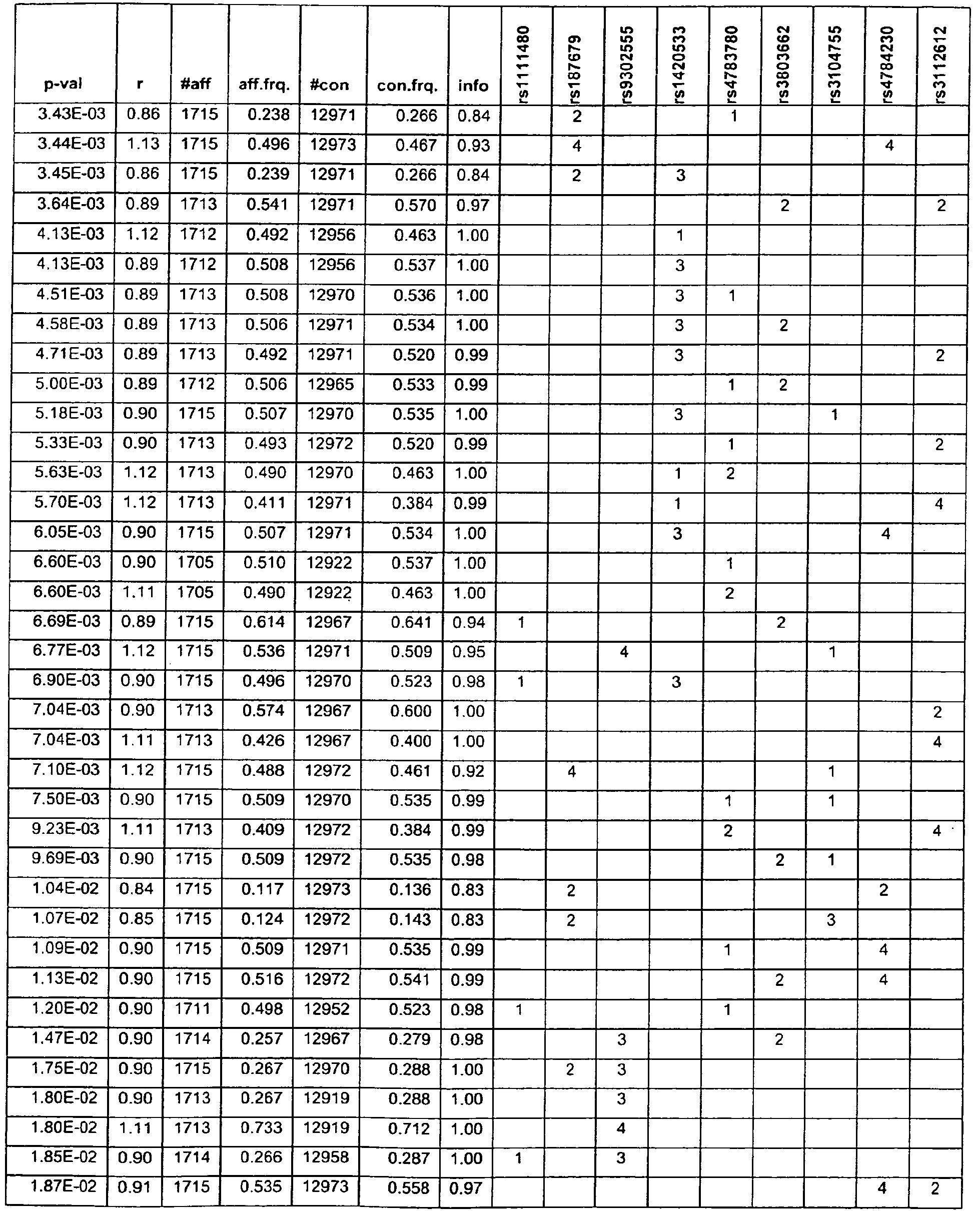

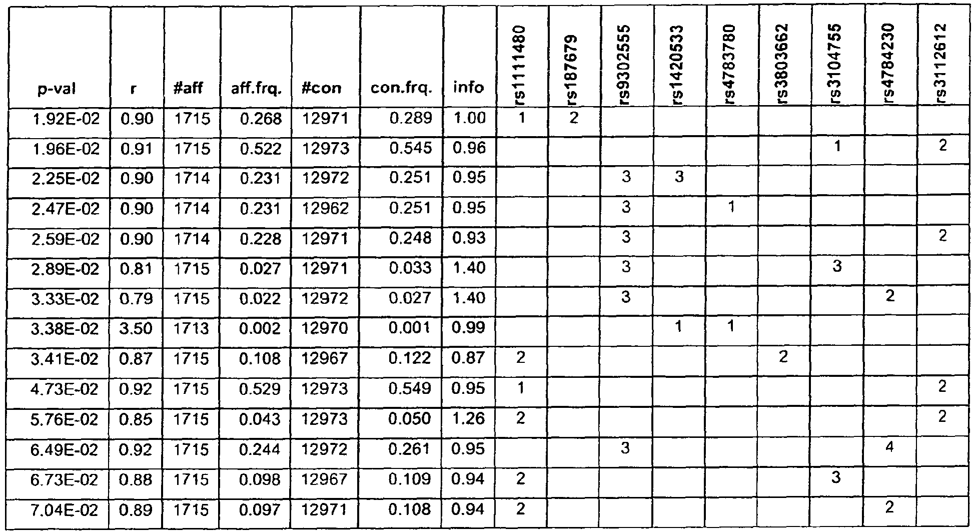

**Table 19: HapMap SNP markers that are correlated with rs3803662 with an r² value greater than 0.2:**

| **Surrogate SNP** | **Ref SNP** | **D'** | **R2** | **p-value** | **Pos in Build 34** | **Pos in Build 36** | **Pos in SEQ ID 6** |
|---|---|---|---|---|---|---|---|
| rs4784220 | rs3803662 | 0.675773 | 0.314847 | 8.65E-09 | 52314403 | 51093311 | 301 |
| rs12598982 | rs3803662 | 0.939522 | 0.331013 | 2.84E-11 | 52315478 | 51094386 | 1376 |
| rs4784222 | rs3803662 | 0.93875 | 0.331747 | 4.06E-11 | 52316179 | 51095087 | 2077 |
| rs17271951 | rs3803662 | 0.959839 | 0.88572 | 9.27E-27 | 52316633 | 51095541 | 2531 |
| rs9933638 | rs3803662 | 0.935921 | 0.321096 | 2.07E-10 | 52318190 | 51097098 | 4088 |
| rs9302556 | rs3803662 | 0.936406 | 0.334336 | 1.07E-10 | 52322238 | 51101146 | 8136 |
| rs7190749 | rs3803662 | 0.939522 | 0.331013 | 2.84E-11 | 52322671 | 51101579 | 8569 |
| rs12443621 | rs3803662 | 0.939123 | 0.326882 | 4.01E-11 | 52326630 | 51105538 | 12528 |
| rs9933556 | rs3803662 | 0.937324 | 0.325388 | 9.50E-11 | 52326895 | 51105803 | 12793 |
| rs1362546 | rs3803662 | 0.935967 | 0.329172 | 9.67E-11 | 52328528 | 51107436 | 14426 |
| rs1075367 | rs3803662 | 0.939522 | 0.331013 | 2.84E-11 | 52333595 | 51112503 | 19493 |
| rs8046979 | rs3803662 | 0.932586 | 0.342705 | 3.56E-10 | 52334335 | 51113243 | 20233 |
| rs1420529 | rs3803662 | 0.931004 | 0.310285 | 1.29E-09 | 52337266 | 51116174 | 23164 |
| rs11642645 | rs3803662 | 0.936189 | 0.329297 | 5.09E-11 | 52337500 | 51116408 | 23398 |
| rs1420533 | rs3803662 | 1 | 0.362637 | 3.83E-14 | 52342219 | 51121127 | 28117 |
| rs1362548 | rs3803662 | 1 | 0.924812 | 8.14E-30 | 52342544 | 51121452 | 28442 |
| rs2193094 | rs3803662 | 1 | 0.362637 | 3.83E-14 | 52345047 | 51123955 | 30945 |
| rs4783780 | rs3803662 | 1 | 0.362637 | 3.83E-14 | 52350025 | 51128937 | 35927 |
| rs3112581 | rs3803662 | 1 | 0.358407 | 5.51E-14 | 52350122 | 51129034 | 36024 |
| rs3112580 | rs3803662 | 1 | 0.370787 | 2.40E-14 | 52350194 | 51129106 | 36096 |
| rs9931232 | rs3803662 | 1 | 0.362637 | 3.83E-14 | 52351417 | 51130333 | 37323 |
| rs1123428 | rs3803662 | 1 | 0.366516 | 3.46E-14 | 52356420 | 51135336 | 42326 |
| rs3095604 | rs3803662 | 1 | 1 | 9.56E-34 | 52360562 | 51139480 | 46470 |
| rs3803662 | rs3803662 | 1 | 1 | 0 | 52364943 | 51143842 . | 50832 |
| rs4784227 | rs3803662 | 1 | 0.812734 | 3.45E-25 | 52377790 | 51156689 | 63679 |
| rs12922061 | rs3803662 | 0.813366 | 0.393164 | 1.28E-08 | 52413602 | 51192501 | 99491 |

**Table 20: List of all dbSNP125 markers in the rs4848543 Linkage Disequilibrium block (positions are from NCBI Build 34):**

| **Pos Build 34** | **Name** | **Pos Build 34** | **Name** | **Pos Build 34** | **Name** |
|---|---|---|---|---|---|
| 119987193 | rs11883887 | 119994803 | rs1374121 | 120003189 | rs11884636 |
| 119987218 | rs7601379 | 119994954 | rs1530522 | 120003199 | rs4849760 |
| 119987236 | rs10189928 | 119995078 | rs10205049 | 120003758 | rs7562612 |
| 119987488 | rs17012230 | 119995182 | rs1530523 | 120004530 | rs745884 |
| 119987701 | rs17798163 | 119995238 | rs5018771 | 120004532 | rs745885 |
| 119988214 | rs6542503 | 119995420 | rs13417283 | 120004821 | rs745886 |
| 119988417 | rs7606204 | 119995638 | rs2860753 | 120005202 | rs745887 |
| 119988662 | rs17012243 | 119995975 | rs4447611 | 120005459 | rs745888 |
| 119988746 | rs10174027 | 119996042 | rs4485581 | 120005770 | rs13395242 |
| 119988777 | rs17012263 | 119996150 | rs10864979 | 120005783 | rs7560869 |
| 119988845 | rs12623644 | 119996950 | rs13433007 | 120005868 | rs11885255 |
| 119988905 | rs10184937 | 119996967 | rs6748754 | 120005995 | rs17012332 |
| 119989602 | rs7570704 | 119997458 | rs28574898 | 120006051 | rs11900137 |
| 119989690 | rs7573686 | 119998119 | rs4848537 | 120006067 | rs11884243 |
| 119990027 | rs6542504 | 119998463 | rs13409086 | 120006186 | rs2197538 |
| 119990110 | rs7557802 | 119998504 | rs13411872 | 120006239 | rs11889299 |
| 119990364 | rs12987676 | 119998551 | rs13420759 | 120006306 | rs970013 |
| 119990863 | rs12993619 | 119999028 | rs11895163 | 120007130 | rs7562248 |
| 119991237 | rs6737956 | 119999114 | rs7597287 | 120007189 | rs12468740 |
| 119991499 | rs10529569 | 120000098 | rs13020121 | 120007959 | rs6542507 |
| 119991618 | rs6741215 | 120000215 | rs9308770 | 120008874 | rs4848538 |
| 119991739 | rs11677153 | 120000292 | rs13425098 | 120009246 | rs6758051 |
| 119991908 | rs6542505 | 120000559 | rs895404 | 120010134 | rs7593041 |
| 119991993 | rs6728751 | 120000798 | rs10207133 | 120010278 | rs11308896 |
| 119992039 | rs6542506 | 120000837 | rs10207198 | 120010394 | rs7556873 |
| 119992209 | rs11386094 | 120000969 | rs11898182 | 120010590 | rs3980309 |
| 119993577 | rs6721529 | 120001134 | rs9308771 | 120010592 | rs3980308 |
| 119993825 | rs6739574 | 120001139 | rs9308772 | 120010654 | rs1317848 |
| 119993914 | rs12711922 | 120001461 | rs6146899 | 120010949 | rs4643545 |
| 119993983 | rs13397501 | 120001581 | rs17012317 | 120011133 | rs6705476 |
| 119994264 | rs12711923 | 120001748 | rs9308773 | 120011458 | rs2121217 |
| 119994321 | rs13017443 | 120001768 | rs9308774 | 120011699 | rs5833777 |
| 119994574 | rs6740231 | 120002407 | rs10202747 | 120011761 | rs7600045 |
| 119994784 | rs10716622 | 120002993 | rs17190912 | 120012134 | rs7563952 |
| 120012410 | rs4849761 | 120023516 | rs2901763 | 120037714 | rs7584975 |
| 120012531 | rs1446130 | 120023583 | rs11680207 | 120037998 | rs7598853 |
| 120012783 | rs1446129 | 120024083 | rs6722032 | 120038031 | rs11123504 |
| 120013821 | rs4849762 | 120024822 | rs7592528 | 120038363 | rs11384356 |
| 120014233 | rs6542508 | 120024900 | rs6542512 | 120038373 | rs11464659 |
| 120014416 | rs11299793 | 120025087 | rs7371346 | 120038374 | rs11393319 |
| 120014424 | rs6724034 | 120025150 | rs10864980 | 120038698 | rs1446126 |
| 120014425 | rs6706020 | 120026424 | rs10188216 | 120038699 | rs10168744 |
| 120014553 | rs6542509 | 120026569 | rs2044428 | 120039011 | rs4848542 |
| 120014683 | rs6542510 | 120026637 | rs11123503 | 120039313 | rs4277535 |
| 120015600 | rs1867750 | 120026789 | rs2044427 | 120039785 | rs13416713 |
| 120015668 | rs5019100 | 120027283 | rs10203658 | 120040375 | rs13417356 |
| 120015928 | rs1867751 | 120027724 | rs7583824 | 120040703 | rs12614389 |
| 120016277 | rs7420091 | 120028226 | rs10174654 | 120040851 | rs2084154 |
| 120016397 | rs4390775 | 120028314 | rs10186930 | 120040967 | rs12621053 |
| 120016788 | rs6732650 | 120028820 | rs6542513 | 120041245 | rs10167806 |
| 120016904 | rs6742613 | 120028843 | rs6542514 | 120041777 | rs10641092 |
| 120017026 | rs7579840 | 120029239 | rs6542515 | 120041779 | rs895400 |
| 120017542 | rs2197539 | 120029586 | rs10532262 | 120041797 | rs7599963 |
| 120017573 | rs10205385 | 120029885 | rs4849764 | 120041798 | rs10658512 |
| 120017765 | rs6542511 | 120030487 | rs17804511 | 120041840 | rs7560645 |
| 120018695 | rs6716071 | 120030834 | rs935362 | 120041953 | rs10551390 |
| 120018841 | rs10597874 | 120030947 | rs935361 | 120041983 | rs7600179 |
| 120019534 | rs13407241 | 120031161 | rs2860752 | 120042110 | rs13017664 |
| 120019716 | rs4849763 | 120031486 | rs11893808 | 120042545 | rs10206284 |
| 120019836 | rs4848539 | 120031828 | rs12623976 | 120042980 | rs3053193 |
| 120019839 | rs4848540 | 120032169 | rs4848541 | 120042985 | rs5833778 |
| 120019852 | rs1867752 | 120032367 | rs28653619 | 120043240 | rs13024716 |
| 120020267 | rs10531839 | 120032378 | rs1446128 | 120044181 | rs7607939 |
| 120020619 | rs2197540 | 120033081 | rs1838763 | 120044182 | rs13405317 |
| 120020703 | rs6727359 | 120033129 | rs1470474 | 120044735 | rs13020459 |
| 120020806 | rs2197541 | 120033135 | rs4528761 | 120045023 | rs13015999 |
| 120020814 | rs6737324 | 120033150 | rs1838762 | 120045027 | rs12052957 |
| 120020831 | rs2197542 | 120033268 | rs2121216 | 120045116 | rs12468083 |
| 120021103 | rs7577922 | 120033353 | rs3980311 | 120045623 | rs963662 |
| 120021157 | rs7575097 | 120033359 | rs1446127 | 120046099 | rs12472931 |
| 120021526 | rs6713238 | 120033749 | rs13024710 | 120046932 | rs7579602 |
| 120021548 | rs7564432 | 120034173 | rs4849765 | 120047249 | rs5833779 |
| 120021672 | rs11682799 | 120034354 | rs4849766 | 120047386 | rs13030441 |
| 120021695 | rs11687834 | 120034730 | rs6715243 | 120047590 | rs991844 |
| 120022550 | rs2121218 | 120036029 | rs10166010 | 120048218 | rs13000062 |
| 120022556 | rs2121219 | 120036225 | rs7561309 | 120048226 | rs13022674 |
| 120022644 | rs13418186 | 120036417 | rs11886602 | 120048234 | rs28579959 |
| 120022871 | rs2166408 | 120036954 | rs6542516 | 120048244 | rs28719630 |
| 120022882 | rs2166409 | 120037241 | rs7601541 | 120048434 | rs13027917 |
| 120022986 | rs6732687 | 120037242 | rs7562256 | 120048457 | rs13027932 |
| 120023118 | rs12474874 | 120037469 | rs7557444 | 120048632 | rs12466475 |
| 120023404 | rs6748897 | 120037538 | rs6735202 | 120049031 | rs4849767 |
| 120023422 | rs4322838 | 120037631 | rs7584874 | 120049111 | rs4849768 |
| 120049158 | rs4849769 | 120064976 | rs6759589 | 120077721 | rs838112 |
| 120049468 | rs13017938 | 120065127 | rs10221610 | 120077754 | rs838111 |
| 120049808 | rs11680905 | 120065310 | rs11689491 | 120077769 | rs838110 |
| 120049895 | rs2028852 | 120066060 | rs7560972 | 120077991 | rs838109 |
| 120050448 | rs2028851 | 120066128 | rs7558272 | 120078174 | rs838108 |
| 120050503 | rs13410792 | 120066241 | rs12463977 | 120078320 | rs12373823 |
| 120050761 | rs13402151 | 120066318 | rs1562256 | 120078344 | rs838107 |
| 120050907 | rs7586792 | 120066453 | rs1562255 | 120078395 | rs865688 |
| 120051111 | rs11675684 | 120066620 | rs1446125 | 120078483 | rs12373677 |
| 120051961 | rs4142753 | 120066705 | rs1446124 | 120078615 | rs7578870 |
| 120052171 | rs4849770 | 120066972 | rs1838761 | 120078754 | rs838106 |
| 120052802 | rs2084155 | 120066974 | rs7601127 | 120079222 | rs838105 |
| 120053731 | rs11691024 | 120067290 | rs11677262 | 120079745 | rs838104 |
| 120054452 | rs11681612 | 120067736 | rs10204391 | 120079819 | rs838103 |
| 120054477 | rs11692475 | 120067927 | rs6753314 | 120079878 | rs838102 |
| 120054484 | rs13400379 | 120068051 | rs6735649 | 120080519 | rs10182677 |
| 120054490 | rs11692479 | 120068477 | rs28455610 | 120080542 | rs10625730 |
| 120055083 | rs4142752 | 120068761 | rs6709777 | 120081128 | rs3769659 |
| 120056573 | rs11695796 | 120069635 | rs10208455 | 120081529 | rs865108 |
| 120057058 | rs10188745 | 120069642 | rs838067 | 120082110 | rs838101 |
| 120057116 | rs11685085 | 120069925 | rs4848544 | 120082382 | rs708670 |
| 120057332 | rs12475875 | 120070154 | rs12466050 | 120082432 | rs708671 |
| 120057333 | rs12463432 | 120070400 | rs7565771 | 120082499 | rs708672 |
| 120057493 | rs5833780 | 120070666 | rs7595954 | 120082585 | rs6146900 |
| 120057623 | rs746945 | 120070908 | rs708666 | 120082871 | rs708673 |
| 120057815 | rs5833781 | 120071508 | rs838066 | 120083300 | rs1106771 |
| 120057816 | rs3053192 | 120071996 | rs708667 | 120083710 | rs838100 |
| 120057829 | rs5833782 | 120072367 | rs708668 | 120084104 | rs3223038 |
| 120058071 | rs876062 | 120072376 | rs708669 | 120084105 | rs6146901 |
| 120058160 | rs895399 | 120072400 | rs895397 | 120084215 | rs838099 |
| 120058174 | rs10186657 | 120072476 | rs838065 | 120084226 | rs838098 |
| 120058180 | rs895398 | 120072631 | rs6755420 | 120084235 | rs13410686 |
| 120058601 | rs5833783 | 120072650 | rs838064 | 120084331 | rs3769656 |
| 120060148 | rs10181114 | 120072686 | rs838063 | 120084565 | rs838097 |
| 120060448 | rs2860751 | 120072737 | rs838062 | 120084736 | rs13400593 |
| 120060852 | rs11693876 | 120073503 | rs838061 | 120084741 | rs838096 |
| 120061012 | rs11683050 | 120073744 | rs11683528 | 120084781 | rs838095 |
| 120061029 | rs10184332 | 120073904 | rs10209847 | 120085360 | rs13425946 |
| 120061096 | rs4848543 | 120074221 | rs1562310 | 120085486 | rs838094 |
| 120061139 | rs10184438 | 120074355 | rs838060 | 120085502 | rs13010169 |
| 120061708 | rs11123505 | 120074581 | rs7557910 | 120085910 | rs12470404 |
| 120062319 | rs11684731 | 120075395 | rs838059 | 120086734 | rs895396 |
| 120062891 | rs6754436 | 120075418 | rs838058 | 120086898 | rs5833784 |
| 120062996 | rs12464139 | 120076234 | rs11676144 | 120086917 | rs13432674 |
| 120063070 | rs6754664 | 120076668 | rs2289894 | 120086997 | rs13408034 |
| 120063176 | rs6754799 | 120076919 | rs13003725 | 120087374 | rs708674 |
| 120063963 | rs12997936 | 120076920 | rs13034799 | 120087538 | rs2121358 |
| 120064284 | rs10206700 | 120077150 | rs3795927 | 120087544 | rs1963247 |
| 120064289 | rs10194572 | 120077389 | rs3795926 | 120087718 | rs12465926 |
| 120088448 | rs7577784 | 120103130 | rs12990907 | 120116464 | rs3769653 |
| 120088805 | rs13391380 | 120103529 | rs7561146 | 120116484 | rs10171408 |
| 120089829 | rs838093 | 120103701 | rs734846 | 120116695 | rs7590206 |
| 120090797 | rs10190429 | 120103880 | rs734845 | 120116930 | rs7581339 |
| 120090847 | rs10182241 | 120104365 | rs895406 | 120117062 | rs3731603 |
| 120091298 | rs838092 | 120104472 | rs895405 | 120117527 | rs6753005 |
| 120091323 | rs13401854 | 120104502 | rs708675 | 120117530 | rs6753006 |
| 120091399 | rs13386059 | 120104672 | rs1867856 | 120117532 | rs12476967 |
| 120091931 | rs838091 | 120105219 | rs838078 | 120117536 | rs10571222 |
| 120092184 | rs864505 | 120105720 | rs3754847 | 120117768 | rs10204553 |
| 120092304 | rs838090 | 120105721 | rs12467089 | 120118122 | rs3731602 |
| 120092610 | rs7567193 | 120105810 | rs11888609 | 120118333 | rs4849771 |
| 120092737 | rs838089 | 120105981 | rs838077 | 120118405 | rs11694139 |
| 120092937 | rs838088 | 120106044 | rs7596511 | 120118612 | rs11682650 |
| 120093034 | rs10174436 | 120106433 | rs838076 | 120119587 | rs1530561 |
| 120093360 | rs838087 | 120107316 | rs12711924 | 120119648 | rs6721852 |
| 120094306 | rs12467944 | 120108033 | rs838075 | 120119899 | rs13016861 |
| 120095038 | rs17804991 | 120108409 | rs838074 | 120120260 | rs13034803 |
| 120095312 | rs10196144 | 120109057 | rs1867749 | 120120268 | rs13034810 |
| 120095426 | rs13027072 | 120109148 | rs12104548 | 120120378 | rs13012570 |
| 120095756 | rs6728660 | 120109236 | rs1045623 | 120120617 | rs6707849 |
| 120096263 | rs13027293 | 120109381 | rs3731604 | 120120725 | rs12165162 |
| 120096647 | rs838086 | 120109485 | rs12104639 | 120121081 | rs6723312 |
| 120096791 | rs838085 | 120109754 | rs838073 | 120121286 | rs3054836 |
| 120096949 | rs10600943 | 120109782 | rs12619779 | 120122044 | rs1545360 |
| 120096982 | rs10526493 | 120109902 | rs6742425 | 120122260 | rs6740573 |
| 120097896 | rs10196222 | 120110130 | rs838072 | 120122331 | rs6730921 |
| 120097915 | rs838084 | 120110342 | rs6542517 | 120122555 | rs13004570 |
| 120097952 | rs3731606 | 120110521 | rs838071 | 120122566 | rs5028235 |
| 120098787 | rs838083 | 120111317 | rs12991476 | 120122626 | rs11395093 |
| 120098925 | rs10188946 | 120111319 | rs12991477 | 120123526 | rs2860792 |
| 120099084 | rs838082 | 120111544 | rs12105674 | 120123689 | rs1867855 |
| 120099515 | rs838081 | 120111606 | rs838070 | 120123862 | rs5833787 |
| 120099779 | rs10202732 | 120111771 | rs838069 | 120124074 | rs28497293 |
| 120099996 | rs6738309 | 120111887 | rs838068 | 120124123 | rs12990281 |
| 120100457 | rs17013371 | 120112630 | rs13400106 | 120124221 | rs12995866 |
| 120100570 | rs1665025 | 120112767 | rs13388672 | 120125144 | rs11692352 |
| 120100571 | rs1624263 | 120112793 | rs7575387 | 120125454 | rs13402922 |
| 120100714 | rs17805141 | 120113041 | rs1610469 | 120126304 | rs6729196 |
| 120100985 | rs5833785 | 120113297 | rs11891374 | 120126843 | rs13034935 |
| 120100993 | rs1530524 | 120114676 | rs6720040 | 120127261 | rs7599282 |
| 120101012 | rs4592854 | 120114755 | rs6723169 | 120127470 | rs7582316 |
| 120101809 | rs2592075 | 120115056 | rs3054837 | 120127897 | rs11123506 |
| 120102502 | rs838080 | 120115077 | rs5833786 | 120128212 | rs2084201 |
| 120102508 | rs6735209 | 120115093 | rs13014052 | 120128319 | rs7592522 |
| 120102857 | rs838079 | 120115134 | rs1374314 | 120128879 | rs10864981 |
| 120103067 | rs17013379 | 120115780 | rs11885203 | 120128983 | rs11123507 |

**Table 21: List of all dbSNP125 markers in the rs13387042 Linkage Disequilibrium block (positions are from NCBI Build 34):**

| **Pos in Build 34** | **Name** | **Pos in Build 34** | **Name** | **Pos in Build 34** | **Name** |
|---|---|---|---|---|---|
| 218062199 | rs10191184 | 218070433 | rs2372933 | 218086701 | rs11677001 |
| 218063010 | rs4396687 | 218070638 | rs10200456 | 218086856 | rs10171745 |
| 218063034 | rs5838616 | 218071353 | rs7426087 | 218087663 | rs17833842 |
| 218063341 | rs10932687 | 218071353 | rs4542823 | 218087886 | rs13393574 |
| 218063429 | rs12614802 | 218071539 | rs4456677 | 218087900 | rs13406843 |
| 218063508 | rs12614767 | 218071539 | rs7422519 | 218088071 | rs10198696 |
| 218063667 | rs11889406 | 218071631 | rs4264545 | 218088201 | rs10177578 |
| 218063784 | rs16856807 | 218071948 | rs10169454 | 218088354 | rs10177962 |
| 218064064 | rs2888449 | 218073196 | rs10192415 | 218089512 | rs4435428 |
| 218064289 | rs10192649 | 218073796 | rs7595393 | 218089563 | rs4276003 |
| 218064351 | rs4571035 | 218073891 | rs10169372 | 218089745 | rs13425828 |
| 218064664 | rs2372931 | 218074854 | rs13430080 | 218089919 | rs3084560 |
| 218065238 | rs10195963 | 218075719 | rs10175911 | 218089928 | rs13414201 |
| 218065423 | rs17833456 | 218075836 | rs12165215 | 218090342 | rs12623304 |
| 218065966 | rs10207650 | 218076028 | rs17833745 | 218091057 | rs2372936 |
| 218066023 | rs10932688 | 218076072 | rs2372934 | 218092002 | rs7425648 |
| 218066050 | rs13403428 | 218076131 | rs2372935 | 218092308 | rs13410769 |
| 218066096 | rs10207736 | 218076869 | rs12612444 | 218092732 | rs10191536 |
| 218066454 | rs28663434 | 218076958 | rs13423850 | 218092782 | rs10203987 |
| 218067143 | rs12464018 | 218077438 | rs6733648 | 218093185 | rs6716542 |
| 218067861 | rs16856812 | 218077473 | rs13389571 | 218093638 | rs10207757 |
| 218068232 | rs12477491 | 218077681 | rs12165173 | 218093795 | rs10173250 |
| 218068256 | rs12477493 | 218077715 | rs13389783 | 218094186 | rs28525300 |
| 218068532 | rs10932689 | 218078368 | rs6737508 | 218094234 | rs17777330 |
| 218068574 | rs13413746 | 218078384 | rs10209168 | 218094822 | rs10211354 |
| 218068580 | rs10932690 | 218079285 | rs13383189 | 218094838 | rs10187690 |
| 218068673 | rs16856816 | 218079764 | rs11884129 | 218094913 | rs10211289 |
| 218068844 | rs13414233 | 218080177 | rs6714036 | 218094939 | rs10211546 |
| 218068906 | rs12613030 | 218080751 | rs6435957 | 218095256 | rs2372937 |
| 218069197 | rs11903648 | 218081124 | rs10192982 | 218095412 | rs4315498 |
| 218069383 | rs2888450 | 218082238 | rs10932691 | 218096305 | rs12998806 |
| 218069406 | rs11688968 | 218082405 | rs10932692 | 218096485 | rs12999224 |
| 218069916 | rs4994976 | 218082754 | rs10188365 | 218096566 | rs17833945 |
| 218069959 | rs4254482 | 218083373 | rs6723019 | 218096592 | rs12465396 |
| 218069975 | rs2372932 | 218083726 | rs7349199 | 218096776 | rs12465506 |
| 218070046 | rs6723386 | 218083877 | rs13404341 | 218096945 | rs12465515 |
| 218070106 | rs6435956 | 218084046 | rs16856821 | 218097298 | rs4491709 |
| 218070227 | rs6713022 | 218084485 | rs12614773 | 218097359 | rs4674130 |
| 218070431 | rs4255939 | 218086136 | rs6732152 | 218097448 | rs3084573 |
| 218097540 | rs13417588 | 218105260 | rs5838618 | 218111343 | rs13423473 |
| 218097624 | rs13392238 | 218105268 | rs6711784 | 218111527 | rs4621152 |
| 218097651 | rs2372938 | 218105513 | rs9973728 | 218111631 | rs13386262 |
| 218098168 | rs12621130 | 218105657 | rs9973894 | 218111703 | rs12329160 |
| 218098897 | rs11387578 | 218105711 | rs9973730 | 218111876 | rs12329163 |
| 218099133 | rs4471865 | 218105920 | rs13402323 | 218112005 | rs6721996 |
| 218099154 | rs6435958 | 218106201 | rs5838619 | 218112094 | rs12329172 |
| 218099578 | rs6722681 | 218106318 | rs2372943 | 218112403 | rs12622764 |
| 218100083 | rs13399995 | 218106548 | rs4522583 | 218112445 | rs10206095 |
| 218100630 | rs7564605 | 218106783 | rs13431146 | 218112816 | rs10206577 |
| 218100783 | rs12052807 | 218107055 | rs2888451 | 218113130 | rs17778091 |
| 218101273 | rs10199394 | 218107332 | rs13383392 | 218113325 | rs13411606 |
| 218101451 | rs10210524 | 218107518 | rs2270398 | 218113452 | rs6723013 |
| 218101477 | rs2372939 | 218107985 | rs6734131 | 218113636 | rs13386291 |
| 218101566 | rs2372940 | 218108214 | rs13386831 | 218113745 | rs10685531 |
| 218101794 | rs4583440 | 218108244 | rs13412479 | 218113820 | rs10192175 |
| 218101850 | rs2372941 | 218108254 | rs13412482 | 218113854 | rs10192253 |
| 218101894 | rs10187530 | 218108321 | rs13412666 | 218113962 | rs10192501 |
| 218101902 | rs6760041 | 218108374 | rs13387042 | 218114027 | rs10204872 |
| 218102145 | rs10190181 | 218108601 | rs13426268 | 218114030 | rs10192589 |
| 218102201 | rs10190349 | 218108615 | rs13387273 | 218114046 | rs6708579 |
| 218102294 | rs17834221 | 218108788 | rs13426489 | 218114076 | rs10192446 |
| 218102294 | rs10202715 | 218108844 | rs13413328 | 218114103 | rs10192609 |
| 218102377 | rs6705818 | 218108906 | rs13413357 | 218114176 | rs4672818 |
| 218102717 | rs6706491 | 218108957 | rs13387717 | 218114436 | rs10195111 |
| 218102822 | rs6735174 | 218108958 | rs13426688 | 218114560 | rs10171550 |
| 218102838 | rs6721811 | 218109040 | rs13413523 | 218114629 | rs10195322 |
| 218102872 | rs6735190 | 218109091 | rs17777938 | 218114644 | rs10195505 |
| 218102939 | rs6735298 | 218109320 | rs13416240 | 218114737 | rs10195441 |
| 218103034 | rs17777767 | 218109630 | rs6713249 | 218114786 | rs10207917 |
| 218103042 | rs12464375 | 218109728 | rs13391022 | 218115389 | rs10198604 |
| 218103061 | rs12476702 | 218109746 | rs13416773 | 218115494 | rs10198699 |
| 218103151 | rs6435959 | 218109779 | rs13416605 | 218115540 | rs10198621 |
| 218103234 | rs6435960 | 218109902 | rs13430215 | 218115617 | rs10174948 |
| 218103592 | rs2372942 | 218109976 | rs13416831 | 218115747 | rs10175133 |
| 218104122 | rs4594417 | 218109979 | rs13416834 | 218115929 | rs10198996 |
| 218104281 | rs10709272 | 218110056 | rs12620095 | 218116467 | rs10167002 |
| 218104345 | rs12615418 | 218110145 | rs13433000 | 218116654 | rs2372945 |
| 218104452 | rs12621884 | 218110379 | rs12327908 | 218116683 | rs10201966 |
| 218104650 | rs7572103 | 218110607 | rs2372944 | 218116715 | rs10201982 |
| 218104906 | rs7421376 | 218110643 | rs2888452 | 218116795 | rs10178467 |
| 218105047 | rs11681010 | 218110863 | rs4618027 | 218117052 | rs16856849 |
| 218105186 | rs5838617 | 218111014 | rs10490444 | 218117184 | rs10204819 |
| 218105193 | rs3084579 | 218111297 | rs13397674 | 218117267 | rs5838620 |
| 218117273 | rs10566949 | 218126446 | rs7561690 | 218134805 | rs13018411 |
| 218117273 | rs3084597 | 218126695 | rs7561918 | 218134901 | rs7599066 |
| 218117443 | rs11901909 | 218126803 | rs7562029 | 218135359 | rs13415112 |
| 218117759 | rs11901972 | 218126936 | rs13000023 | 218135444 | rs6754177 |
| 218117956 | rs12694403 | 218127398 | rs17835187 | 218135688 | rs4361105 |
| 218118356 | rs11892687 | 218127601 | rs3085312 | 218135854 | rs13025492 |
| 218118594 | rs12613955 | 218127603 | rs3085315 | 218136008 | rs13025714 |
| 218118809 | rs10208746 | 218127665 | rs735361 | 218137327 | rs12328709 |
| 218119095 | rs10185401 | 218128212 | rs17835248 | 218137594 | rs16825209 |
| 218119709 | rs11894340 | 218128870 | rs13409592 | 218137658 | rs12329133 |
| 218119744 | rs6435961 | 218129396 | rs16856867 | 218137683 | rs12329135 |
| 218120136 | rs10191172 | 218129599 | rs2372957 | 218137973 | rs6734010 |
| 218120424 | rs2372946 | 218129616 | rs2372958 | 218138557 | rs12622536 |
| 218120624 | rs10168238 | 218129746 | rs5838621 | 218138666 | rs13022815 |
| 218120863 | rs7586158 | 218129748 | rs3085335 | 218138690 | rs12996888 |
| 218121113 | rs7600412 | 218129820 | rs16856873 | 218138928 | rs12997141 |
| 218121293 | rs10183545 | 218129963 | rs17778798 | 218139101 | rs12328842 |
| 218121786 | rs7589722 | 218130041 | rs16856877 | 218139274 | rs12328926 |
| 218122009 | rs6740850 | 218130142 | rs7600279 | 218139288 | rs13002378 |
| 218122107 | rs17778299 | 218130798 | rs11682933 | 218139595 | rs16856893 |
| 218122353 | rs17778329 | 218130803 | rs4608489 | 218140109 | rs12478570 |
| 218123052 | rs10198784 | 218131549 | rs13034362 | 218140238 | rs12467063 |
| 218123311 | rs4442975 | 218132117 | rs10211044 | 218140453 | rs11690147 |
| 218123663 | rs10932693 | 218133211 | rs16856888 | 218140482 | rs13010040 |
| 218124158 | rs11291245 | 218133717 | rs5838622 | 218140537 | rs12467163 |
| 218124274 | rs2888456 | 218133723 | rs5838623 | 218140719 | rs13011060 |
| 218124419 | rs2372956 | 218133948 | rs2287288 | 218140839 | rs4672819 |
| 218124426 | rs2888457 | 218134039 | rs2287289 | 218140888 | rs4672820 |
| 218125505 | rs11674902 | 218134273 | rs16856890 | 218140952 | rs13011326 |
| 218125555 | rs17778427 | 218134618 | rs7598618 | 218140976 | rs11690464 |
| 218126147 | rs17835044 | 218134620 | rs7598805 | | |
| 218126358 | rs7588345 | 218134761 | rs7598926 | | |

**Table 22: List of all dbSNP125 markers in the rs3803662 Linkage Disequilibrium block (positions are from NCBI Build 34):**

| **Pos in Build 34** | **Name** | **Pos in Build 34** | **Name** | **Pos in Built 34** | **Name** |
|---|---|---|---|---|---|
| 52291041 | rs1076479 | 52293430 | rs11454608 | 52295650 | rs7198218 |
| 52292193 | rs7188075 | 52293504 | rs9937283 | 52295682 | rs11337972 |
| 52292669 | rs28720857 | 52294560 | rs12921126 | 52295683 | rs5816850 |
| 52293212 | rs11642868 | 52295118 | rs189851 | 52296050 | rs7203597 |
| 52293398 | rs5816849 | 52295192 | rs28558853 | 52296074 | rs11304815 |
| 52296100 | rs7203778 | 52314189 | rs11427470 | 52328944 | rs12932707 |
| 52296956 | rs4784218 | 52314348 | rs16951213 | 52329434 | rs9939775 |
| 52297566 | rs12444429 | 52314403 | rs4784220 | 52329968 | rs8048043 |
| 52297574 | rs12447254 | 52314941 | rs8046985 | 52333201 | rs3086693 |
| 52297595 | rs12447113 | 52315230 | rs28665779 | 52333595 | rs1075367 |
| 52297649 | rs28689711 | 52315478 | rs12598982 | 52334335 | rs8046979 |
| 52297698 | rs3095662 | 52316033 | rs10451108 | 52334886 | rs1345388 |
| 52297718 | rs28493012 | 52316119 | rs4784221 | 52335310 | rs9941100 |
| 52298081 | rs4493036 | 52316179 | rs4784222 | 52337266 | rs1420529 |
| 52298807 | rs7205259 | 52316601 | rs7195542 | 52337322 | rs1420530 |
| 52299817 | rs11464652 | 52316633 | rs17271951 | 52337427 | rs1420531 |
| 52299822 | rs10641032 | 52316669 | rs12934513 | 52337486 | rs10708737 |
| 52299825 | rs11427655 | 52316689 | rs12934308 | 52337500 | rs11642645 |
| 52299952 | rs1111480 | 52316930 | rs7184835 | 52338806 | rs12918816 |
| 52300224 | rs12447430 | 52317345 | rs10545412 | 52340832 | rs4280232 |
| 52301045 | rs43143 | 52317493 | rs12600239 | 52341080 | rs7197481 |
| 52301313 | rs187679 | 52318190 | rs9933638 | 52341404 | rs12929984 |
| 52302076 | rs42542 | 52318304 | rs28530752 | 52342090 | rs1420532 |
| 52302193 | rs4784219 | 52318333 | rs7186498 | 52342219 | rs1420533 |
| 52302954 | rs11649553 | 52318957 | rs13337151 | 52342476 | rs1362547 |
| 52303160 | rs3086679 | 52321003 | rs8063421 | 52342544 | rs1362548 |
| 52303163 | rs9923098 | 52321733 | rs11326759 | 52342856 | rs10538952 |
| 52303361 | rs8061425 | 52321900 | rs4541074 | 52343467 | rs28555277 |
| 52303886 | rs1978343 | 52321923 | rs4517793 | 52343743 | rs7193453 |
| 52304653 | rs3095595 | 52322238 | rs9302556 | 52344234 | rs9926163 |
| 52304699 | rs12102837 | 52322671 | rs7190749 | 52345047 | rs2193094 |
| 52305016 | rs10637120 | 52322726 | rs16951220 | 52345209 | rs28580623 |
| 52305075 | rs9931647 | 52323043 | rs1362544 | 52345455 | rs3095598 |
| 52305085 | rs28484983 | 52323132 | rs1819860 | 52346438 | rs12929605 |
| 52305086 | rs10637121 | 52323870 | rs7500427 | 52346440 | rs12929606 |
| 52305875 | rs7188610 | 52325826 | rs16951223 | 52347923 | rs28750243 |
| 52305996 | rs7186907 | 52326630 | rs12443621 | 52349175 | rs3112583 |
| 52306637 | rs9923960 | 52326776 | rs9923558 | 52349177 | rs3112582 |
| 52306726 | rs2193092 | 52326895 | rs9933556 | 52349414 | rs28435355 |
| 52306777 | rs2216188 | 52327297 | rs1362545 | 52349558 | rs11648477 |
| 52306900 | rs9939506 | 52327389 | rs4538010 | 52350025 | rs4783780 |
| 52307320 | rs9926539 | 52327503 | rs12922895 | 52350122 | rs3112581 |
| 52307440 | rs9302555 | 52327601 | rs12923628 | 52350194 | rs3112580 |
| 52308124 | rs12597838 | 52327690 | rs11415785 | 52350390 | rs11431881 |
| 52308541 | rs28562017 | 52327938 | rs7198754 | 52350614 | rs9921569 |
| 52309356 | rs8064219 | 52328239 | rs9936081 | 52351417 | rs9931232 |
| 52309429 | rs11287146 | 52328264 | rs8051286 | 52352109 | rs11075535 |
| 52309445 | rs8044985 | 52328472 | rs10647102 | 52353864 | rs12597887 |
| 52310401 | rs9928129 | 52328482 | rs5816851 | 52354487 | rs3112579 |
| 52310846 | rs3086687 | 52328483 | rs10645216 | 52354492 | rs4784223 |
| 52310948 | rs1111481 | 52328493 | rs3086690 | 52355066 | rs9933351 |
| 52311095 | rs2077653 | 52328528 | rs1362546 | 52355242 | rs1548910 |
| 52312052 | rs8061860 | 52328629 | rs2193093 | 52355727 | rs8052175 |
| 52312760 | rs8051542 | 52328900 | rs2160474 | 52355897 | rs28523905 |
| 52356089 | rs1420534 | 52371458 | rs28595571 | 52381091 | rs3104750 |
| 52356352 | rs7195388 | 52372064 | rs3104812 | 52381630 | rs3104751 |
| 52356420 | rs1123428 | 52372504 | rs16951246 | 52381735 | rs3104752 |
| 52356800 | rs1420535 | 52372582 | rs7201350 | 52381752 | rs3112566 |
| 52357133 | rs1477074 | 52372830 | rs3104818 | 52381753 | rs3112565 |
| 52358132 | rs3891563 | 52373327 | rs16951251 | 52381770 | rs3112564 |
| 52358441 | rs13336638 | 52373331 | rs3104827 | 52381945 | rs5011736 |
| 52358491 | rs8044585 | 52373794 | rs28715219 | 52382513 | rs3104753 |
| 52358571 | rs8045285 | 52373795 | rs28759054 | 52382735 | rs11864809 |
| 52358832 | rs4784224 | 52373894 | rs28491753 | 52382879 | rs16951272 |
| 52358870 | rs3095601 | 52374821 | rs28397852 | 52382932 | rs11859735 |
| 52359085 | rs3095602 | 52375020 | rs28561462 | 52383045 | rs16951279 |
| 52359653 | rs3095603 | 52375173 | rs28366779 | 52383698 | rs3481 |
| 52360007 | rs12930156 | 52375215 | rs28690015 | 52383902 | rs16951281 |
| 52360562 | rs3095604 | 52375335 | rs28472996 | 52384112 | rs3104754 |
| 52360995 | rs9921056 | 52375668 | rs28437002 | 52384265 | rs3112563 |
| 52361357 | rs1362549 | 52375894 | rs3104743 | 52384839 | rs3104755 |
| 52361398 | rs1362550 | 52376140 | rs9922732 | 52385009 | rs3104756 |
| 52361636 | rs28463809 | 52376155 | rs3112576 | 52386865 | rs3112562 |
| 52361725 | rs4784226 | 52376446 | rs4594251 | 52387168 | rs3104757 |
| 52362126 | rs13335680 | 52376504 | rs28685217 | 52387274 | rs10521267 |
| 52362449 | rs3095605 | 52376699 | rs3112575 | 52387635 | rs3104758 |
| 52362775 | rs3095606 | 52377560 | rs3112574 | 52387700 | rs9921358 |
| 52362897 | rs3095607 | 52377659 | rs9937101 | 52388131 | rs3104759 |
| 52363302 | rs12926842 | 52377665 | rs16951253 | 52388189 | rs3112561 |
| 52364042 | rs3112578 | 52377790 | rs4784227 | 52388416 | rs3104760 |
| 52364943 | rs3803662 | 52377970 | rs16951254 | 52388424 | rs3104761 |
| 52365079 | rs3803661 | 52377996 | rs3104744 | 52388458 | rs3112560 |
| 52365170 | rs3803660 | 52378688 | rs3104745 | 52388606 | rs3112559 |
| 52365183 | rs3803659 | 52378902 | rs3112573 | 52389436 | rs3104762 |
| 52365510 | rs8053294 | 52378941 | rs13334992 | 52389606 | rs8055502 |
| 52365977 | rs12446056 | 52379032 | rs28503301 | 52390581 | rs5816853 |
| 52366465 | rs4322659 | 52379049 | rs3112572 | 52390586 | rs5816854 |
| 52366478 | rs28409238 | 52379220 | rs4784228 | 52390646 | rs1109951 |
| 52367399 | rs13335398 | 52379400 | rs12919508 | 52390740 | rs8061255 |
| 52367692 | rs7189773 | 52379702 | rs3104746 | 52391109 | rs28636558 |
| 52367823 | rs7191347 | 52379713 | rs3112571 | 52391155 | rs5816855 |
| 52367830 | rs13332085 | 52380052 | rs11372822 | 52391221 | rs3112638 |
| 52368150 | rs3104771 | 52380131 | rs11646414 | 52391267 | rs9708611 |
| 52369037 | rs28478483 | 52380217 | rs8053784 | 52391639 | rs12935019 |
| 52369057 | rs28418561 | 52380227 | rs11342984 | 52392183 | rs3104763 |
| 52369268 | rs8057406 | 52380243 | rs3112570 | 52393309 | rs5816856 |
| 52369377 | rs7199494 | 52380252 | rs9934281 | 52394968 | rs5816857 |
| 52369741 | rs28636082 | 52380383 | rs3112569 | 52395219 | rs5816858 |
| 52370476 | rs11437687 | 52380473 | rs3112568 | 52395420 | rs11284113 |
| 52370482 | rs3112577 | 52380526 | rs3104747 | 52395423 | rs11326465 |
| 52370737 | rs28719661 | 52380961 | rs3104748 | 52395448 | rs6498958 |
| 52370774 | rs28700577 | 52380977 | rs3104749 | 52396694 | rs3112637 |
| 52371185 | rs3104808 | 52381045 | rs3112567 | 52396715 | rs4784230 |
| 52396799 | rs13330592 | 52406977 | rs7198019 | 52416037 | rs4578643 |
| 52397331 | rs5816859 | 52407020 | rs11860998 | 52416145 | rs3104787 |
| 52397555 | rs11645620 | 52407095 | rs7192828 | 52416382 | rs12597685 |
| 52398233 | rs3112636 | 52407116 | rs7193515 | 52416795 | rs11374841 |
| 52398720 | rs3112635 | 52407139 | rs11860769 | 52417105 | rs3104788 |
| 52398860 | rs3112634 | 52407166 | rs11861036 | 52417150 | rs3104789 |
| 52399117 | rs16951316 | 52407928 | rs3104772 | 52417212 | rs12599893 |
| 52399179 | rs3112633 | 52408038 | rs3104773 | 52417265 | rs3104790 |
| 52399652 | rs28567699 | 52408105 | rs3112622 | 52417361 | rs4290467 |
| 52399807 | rs3112632 | 52408239 | rs10692294 | 52417477 | rs4467067 |
| 52399850 | rs3112631 | 52408304 | rs3104774 | 52417564 | rs3104791 |
| 52399948 | rs5816861 | 52408429 | rs3104775 | 52417836 | rs4238749 |
| 52400091 | rs13335960 | 52408504 | rs7200735 | 52417838 | rs4238750 |
| 52400138 | rs3112630 | 52408747 | rs7203671 | 52418217 | rs8048809 |
| 52400341 | rs3104764 | 52408951 | rs3112621 | 52418357 | rs4459541 |
| 52400714 | rs11640537 | 52409006 | rs7206289 | 52418357 | |
| 52401021 | rs3112629 | 52409053 | rs7206313 | 52419612 | rs1112135 |
| 52401314 | rs3112628 | 52409255 | rs7204612 | 52419788 | rs3104792 |
| 52402239 | rs6498960 | 52409498 | rs3112620 | 52420562 | rs3104793 |
| 52402254 | rs5816863 | 52409610 | rs3112619 | 52420628 | rs3104794 |
| 52402438 | rs4262942 | 52409628 | rs8062962 | 52420979 | rs3104795 |
| 52402568 | rs3104765 | 52409779 | rs12597100 | 52422526 | rs3104796 |
| 52403048 | rs2335 | 52409788 | rs3112618 | 52422537 | rs8046994 |
| 52403100 | rs10653756 | 52410064 | rs3104776 | 52422543 | rs7193357 |
| 52403137 | rs3104766 | 52410319 | rs3112617 | 52423052 | rs7186461 |
| 52403194 | rs7201410 | 52410349 | rs3112616 | 52423497 | rs3104797 |
| 52403223 | rs12934093 | 52410566 | rs11075551 | 52424706 | rs3104798 |
| 52403340 | rs3104767 | 52410952 | rs3112615 | 52424978 | rs3104799 |
| 52403795 | rs28400414 | 52411233 | rs3104777 | 52425772 | rs3104800 |
| 52403802 | rs3112626 | 52411546 | rs12929797 | 52427035 | rs3112611 |
| 52404000 | rs7190208 | 52411635 | rs3112614 | 52427036 | rs12921031 |
| 52404011 | rs28379686 | 52412049 | rs7202142 | 52427156 | rs12921032 |
| 52404038 | rs17292403 | 52412254 | rs3104778 | 52427333 | rs3112610 |
| 52404154 | rs3112625 | 52412259 | rs3104779 | 52427444 | rs3112609 |
| 52404215 | rs12920540 | 52412298 | rs8044824 | 52427468 | rs8045104 |
| 52404998 | rs3104768 | 52412310 | rs10665605 | 52427539 | rs3112608 |
| 52405153 | rs3112624 | 52412516 | rs3104780 | 52427910 | rs12921801 |
| 52405181 | rs5816864 | 52412602 | rs3104781 | 52428293 | rs6498966 |
| 52405833 | rs3104769 | 52412765 | rs3112613 | 52428649 | rs4488440 |
| 52405970 | rs3104770 | 52412855 | rs8044760 | 52429238 | rs3112607 |
| 52406122 | rs11645834 | 52413602 | rs12922061 | 52429511 | rs5816865 |
| 52406128 | rs3112623 | 52413719 | rs28709052 | 52429906 | rs3112606 rs5002422 |
| 52406232 | rs8046543 | 52413766 | rs3112612 | 52429965 | rs3112605 |
| 52406857 | rs12930738 | 52413932 | rs9925692 | 52430198 | rs3112604 |
| 52406859 | rs12926392 | 52414779 | rs3104782 | 52430316 | rs11075561 |
| 52406869 | rs12926394 | 52414844 | rs3104783 | 52430713 | rs12599215 |
| 52406870 | rs12930747 | 52414967 | rs3104784 | 52431082 | rs4640177 |
| 52406881 | rs12102546 | 52415402 | rs3104785 | 52431277 | rs5816867 |
| 52406905 | rs28642024 | 52415965 | rs3104786 | 52431322 | rs4638564 |
| .52431382 | rs11647405 | 52432454 | rs3104802 | 52433898 | rs12924425 |
| 52431582 | rs11075562 | 52432459 | rs3104803 | 52434496 | rs28680336 |
| 52431715 | rs3112603 | 52432718 | rs7198792 | 52434497 | rs5816868 |
| 52431739 | rs4638563 | 52432830 | rs13338987 | 52434960 | rs7202932 |
| 52431878 | rs4426347 | 52433165 | rs11643841 | 52435190 | rs3112600 |
| 52431941 | rs3104801 | 52433198 | rs3104804 | 52435864 | rs3104806 |
| 52432313 | rs3112602 | 52433468 | rs3104805 | 52436127 | rs3104807 |
| 52432373 | rs11434012 | 52433888 | rs3112601 | | |
| 52432387 | rs6498968 | 52433897 | rs12924424 | | |

**Table 23: HapMap SNP markers used for investigation in the Multiethnic Cohort:**

| SNP | European Americans | | Yoruba (African) | |
|---|---|---|---|---|
| | r²* | D' | r² | D' |
| rs12922061 | 0.39 | 0.81 | 0.03 | 1 |
| rs4784227 | 0.81 | 1 | 0.03 | 1 |
| rs17271951 | 0.88 | 0.96 | 0.03 | 1 |

| | | | | |
|---|---|---|---|---|
| *r² and D' values with respect to rs3803662 | | | | |

**Table 24: Breast Cancer Association Data from the Multiethnic Cohort:**

| **European Americans** | | | | | | |
|---|---|---|---|---|---|---|
| SNP | p-val | r | #aff | aff.freq | #con | con.freq |
| T-rs3803662 | 0.0214 | 1.23 | 534 | 0.3408 | 572 | 0.2951 |
| T-rs12922061 | 0.0724 | 1.19 | 534 | 0.2799 | 572 | 0.2462 |
| T-rs4784227 | 0.0036 | 1.32 | 534 | 0.3034 | 572 | 0.2480 |
| C-rs17271951 | 0.0154 | 1.26 | 534 | 0.3096 | 572 | 0.2630 |
| | | | | | | |

| **Latina** | | | | | | |
|---|---|---|---|---|---|---|
| SNP | p-val | r | #aff | aff.freq | #con | con.freq |
| T-rs3803662 | 0.0498 | 1.22 | 425 | 0.4190 | 426 | 0.3723 |
| T-rs12922061 | 0.2487 | 1.13 | 425 | 0.3231 | 426 | 0.2972 |
| T-rs4784227 | 0.1373 | 1.17 | 425 | 0.3429 | 426 | 0.3091 |
| C-rs17271951 | 0.4867 | 1.08 | 425 | 0.2905 | 426 | 0.2753 |
| | | | | | | |

| **African Americans** | | | | | | |
|---|---|---|---|---|---|---|
| SNP | p-val | r | #aff | aff.freq | #con | con.freq |
| T-rs3803662 | 0.0069 | 0.77 | 432 | 0.4747 | 457 | 0.5395 |
| T-rs12922061 | 0.0358 | 1.41 | 432 | 0.1065 | 457 | 0.0777 |
| T-rs4784227 | 0.0853 | 1.34 | 432 | 0.0972 | 457 | 0.0744 |
| C-rs17271951 | 0.2060 | 1.24 | 432 | 0.0922 | 457 | 0.0755 |

**Table 25: Breast Cancer Risk Associated with rs13387042 allele A and rs3803662 allele T in Estrogen Receptor and Progesterone Receptor Positive and Negative Tumors.**

| | **rs13387042 (A)** | | | **rs3803662 (T)** | | |
|---|---|---|---|---|---|---|
| | **Cases** | **OR** | ***P*** | **Cases** | **OR** | ***P*** |
| | | | | | | |

| **Estrogen Receptor (ER)** | | | | | | |
|---|---|---|---|---|---|---|
| *ER pos* | 2124 | 1.22 | 4.3E-09 | 2128 | 1.32 | 5.8E-13 |
| *ER neg* | 589 | 1.06 | 0.37 | 589 | 1.07 | 0.30 |
| *ER pos vs ER neg* | | 1.15 | 0.036 | | 1.21 | 0.0098 |

| **Progesterone receptor (PR)** | | | | | | |
|---|---|---|---|---|---|---|
| *PR pos* | 1802 | 1.23 | 2.8E-08 | 1805 | 1.30 | 9.3E-11 |
| *PR neg* | 816 | 1.12 | 0.036 | 817 | 1.16 | 0.015 |
| *PR pos vs PR neg* | | 1.09 | 0.150 | | 1.13 | 0.057 |

**Table 26. Flanking sequences for rs4848543 and correlated HapMap SNP markers with r^{2>} 0.2**

| |
|---|
| >rs4848543 |
| GCACTAGAAGGTTACATACAATGGAAACTGAGGCCCAGAAGGAGGAAA GATGAAGTGCCCAAAGTTAAGGAGATAAAAGCAGAAGTGCAGGTAAGAGA AGGAGTATTCATATTACTGCAGGAATCCAAATTCTACAAGAACATACGCT TGTTCTTTAAAGGGAGCCCTCGCTTTAAGCATTATTTGCCGATCAATACC [A/C] ATTAATAATTACAGGACATGGGCAAATATTCTAGCAAAGGTCCTGAGGT CTTCCTAGATGTGCCCAAAATAACAAGGACTCCCCCGCAGGTGGCACCA AAAGTCGGAGGATGTGTTGGAAGCTGACCTTCCCCACCTGTCCCTGCCTC ACCCACAGTCACCCACCCCTCTAGGATGTAATGGTGAATCATTCAACACAT |
| >rs11680207 |
| GTGACACTAGGGACGTGGGGGAATGCAAGTGGGTCCAGACCTGGGGGCC ACCAGTGCAGGAGCAAGGGCAGGCCGGCCATCAGAGCTGGGCGGGAGGG GGGCGCTGAGCTTGGCGTGGGAGTTTCCCCAACGGACTGTGTCTTCCAGG GACCCTCGCCCCTCATGCCCCTCTGCGTCCTGAAGGAGGAGGCACCACC [C/T] CTCTGTGGCATCTGCTTTTTAGGGCTGACGATGGAACAAGCTCATGGAC AGGAGTGAAGCCCCTGGCACATAGCAGGTGCCACAGAAGGGTGGTGAAGT CTGCCCTAGATCAGAGATCATTCAGGGGGTCTGTTAACTTGGATGAAGAA GAAATACACTCTTATCACTAACATCAGGAAGGTAAGCAAGGAACCACACAT |
| >rs7583824 |
| AAAGGAAGGGATTTCTGGGCCTCAGAAAACCACATGCCAACGCATGGGGG TATTAAGGATAGGGGCTCTTGAACGCCCAGGCTGGTAAAATCTAGAACGG CAGAGGGAGGAGGGGTAATTTTTCTGGTTGGTAGAAATAAAGTGGGGATG |
| AGGAAGAGCACGAAAGTCTGTCGGCAAACCTGAAAGTGGGAGCGAGCTTT [C/T] GTCAAATCCAGGGTCAACCTGAGCTCTCATTAATTGGGAAACTGGAGAG TCAGATCCTCTAAAGAAAGTGGAAATATTTGTTCTAAAGAACCCCTGCCA GGTCCCCGTTTACGTGACTCATAAAGAGGCCTTCAGGCAGAAAGGATCCC AGTGCACCAGTAACAGAAGGGCTCCAGGACCAGAGGGAATGGACAGGGAAC |
| >rs4848541 |
| TGGCCTATAGTCGAGTAGATAGAGGGAGGGCTGATAACTAAGTGACTACA ACAGGACCTCCCTGTGTAAGGGTTCTAGCTTATTTCCACACTCTTCTGGC ACAGACAGGTTGGCAGGGCAGCTGGTAGCAGGCACAGCAGATGGAATGTC AGGAATGAAATCCACAAGACTCCAAGCCCCTCCCAGGGTGAGCTTGCCAC [A/G] GGCCACACCAGGAAGCTGAGCTCCTCACCCAAAGCCTGCCAGTGCCTAC GTGGCAGTCATTCCCTTGTCAACAGACTTCCCACTGGCTTCTCTTGCTCC ATTATTAAAAGCAAGAACCAAAAACTTCACTGAGGGGATAGAATCCCACT AATAAGCATGTCCACGCTTTGGGTCATCTTAGGGGCACAGAAAATCTGAGT |
| >rs1446128 |
| GAGACACTTTTCTGCCTGCCCAAAGTCACATAGCTCATTAGAGGCCAGGT TGGGCCTGGAGTCTCTGGCAGGAATAATAGCTGAACTTTCATTCATTCCC TAACCCACCTGTGTTGTGAGGGCCCTGAGGCCAGCATGTTCATGGCTGCA GCCTCCAACCAACTGAGCTAATTGACAAGAAATCTGCTCTGCCTGATGCT [A/G] TGGGGGATACTCAGATTTTCTGTGCCCCTAAGATGACCCAAAGCGTGGA CATGCTTATTAGTGGGATTCTATCCCCTCAGTGAAGTTTTTGGTTCTTGC TTTTAATAATGGAGCAAGAGAAGCCAGTGGGAAGTCTGTTGACAAGGGAA TGACTGCCACGTAGGCACTGGCAGGCTTTGGGTGAGGAGCTCAGCTTCCTG |
| >rs4849766 |
| CCAGGCTCAAGAGAGAAAGCCAGACCCTCTCTCCTTCCACCTACCTGAGA GAGGCCTGTCCTCAGGTGTCTGGCTCACAGATGGGGGACCCAACTGGGAG TCACTCCCTTCTGGAAGCCCCAGGCCCTATAAACAGGTCCCCGGCCTCAC TGCTGGGCCTCGGCCTGGAGACAGCGGCTCAGTGCAACACAACCACGCCA [C/G] AGCTGATCAGGGGAAACCCACGCAGCAGTTGAGGGGGCTGGGGCTTTGC GGGTGCAGTGAGGGGACTGCTTCCCTTCAGTGGAGTTGCTGCCGCAGGTG AGGGGAGGAAGATGAATCTGAAGGTTCTAACGTCAGCCCACAGGCTGAAG CGAAGGCTGGCCTGAAGTGTTCCACCTGGGCCCCCACCTGAGCGGCTGAAG |
| >rs6715243 |
| TGGGCCCCCACCTGAGCGGCTGAAGAGTTCTGTTTCCTCAAGGCCCACAG AGCTCCTCAGAGGGAAGCTTTAGTAGCTATTTCCTTAGGAAGGCAAGTAT GTGTGCAACATTTCCTAATAAAATGCAAAGGCTGTTGGAGGCACTCCAGC CCCCTCATTTAGCTCTGAGGTGTAATTAATACATAAATTAAGCTCCTGCA [G/T] ACTCACAAATGTCCAAGAAAAAAAATGAAAAATATAATTAGTTTGCCCA TCTGACTCCATCTGTCAGCTCTGAGCTCAATGCTCAACGTAGCAAATCCT GAGGGCTTTGGTCTCGCATTGGCTGGGCCCGGCGCTGCTCCTGCCCTGCT CCAGGGAAGTGGGCCCTCCGCAGCAGCTGCCCAGAGCCCGGGCTCTGGACT |
| >rs895398 |
| TTACCAGCCCTCTCTCTCCCTTCTCCCCTTTGCCTTCCACACACAGCCTC TTGTCCAGCCACTCCCTCTTCCTGGAAGGGCCTTACTGACAGAGTGCTGG GGAGCCCTGTCAGCTGGCCACAGGTCTTAGCAAGGGCCACCCCCTCCTTC AGGCCTTCACCGTGTCTTGGAACCTCCCACTGCCACAGCCTCAGCGACAC [C/T] |
| GTCCTTAGATCCAGGCAGGTGGGCTGTGACCCGGACTGCACTTTGGAA GGCCTTGCTATCCTCCTCTGGCCATCTCCTTCTCCAGGGCCCGAGGGATC TGCAGAACCGGAGGAACATGCCAACCTGAAACCTGGGCTGTCTTTATCCG GCTGTACTCCACCTGGGATTTTCTGCCCGGATGGCATCAAGATCGCTTCCA |
| >rs11684731 |
| GCCTAAGCCAGAGGAGGCAGGAGGGTTTAGGGCACCAGCCAAGAAGTGCT TTCCAGATGCTGAGTGGGATCCCGAATAAGGGAAAGGGCATCTAACATAG AATGAGGAGGAGATAACACAAGGGTAGAAATAACCAAGAAAGGGGATTTC TCTCAGATGGCTTTAGAGGTGCTCCCAGCCATTGCTGGGCTCACATGGCT [C/T] ACATGTCATAATTTTTGCCAAGGCGGTTTCACTGCCACCACCACCCCGC TCAGCCTCCAGTGGCTTGTCCACCCATGGCTGTGGCCTCTAACTGGGCAG GATTTTATAGGCTAGGAAGAAAAGTAGGGAATGTTAAGGGAGACGGAAGT CAAGTTCAACTTGGAACCATCCCAGAATGTCCAGCAGGAAAGGCCCATGGA |
| >rs12464139 |
| GACACCTCACCTTGACACGCAGCCACCTGCCAGTGTTCACAAATGTTGGT TTTCAGCTTTATGCGAGGGTAGGATCCAACAAGCTCATTCTTTAATGAAA AAGAATGAAATTGTAAAATATCAGATCTTATTACATGTCCTAAGGGTAAC TTTTATCTCATAACACTTTTGCTTCAATCATGAGTGTATAATTTCTATTT [G/T] ATTTTGTGTCTATGGGGTTAGGTGTGCAATGTAGAAAAGCTTGAAAACC ATTGCCTTATACCCATTTGAAAAATGAACCACCAAACAAAGGCTTAAAAA CATGAATATTTGTCAACCAAAAGGAGAACTCCTGGGCCTGGAAGAAACAT CACGATTCAAGTGCTGCAGCACGAAAGAAGTTGAGGCTGGATTTGTATTCA |
| >rs6754664 |
| TCCAACAAGCTCATTCTTTAATGAAAAAGAATGAAATTGTAAAATATCAG ATCTTATTACATGTCCTAAGGGTAACTTTTATCTCATAACACTTTTGCTT CAATCATGAGTGTATAATTTCTATTTGATTTTGTGTCTATGGGGTTAGGT GTGCAATGTAGAAAAGCTTGAAAACCATTGCCTTATACCCATTTGAAAAA [G/T] GAACCACCAAACAAAGGCTTAAAAACATGAATATTTGTCAACCAAAAGG AGAACTCCTGGGCCTGGAAGAAACATCACGATTCAAGTGCTGCAGCACGA AAGAAGTTGAGGCTGGATTTGTATTCAGCTTGACTGTTTGTAAAGCACCA TGCACCATGCAACTGCCTTCAGTTCCTTTTTTTTTTTCTTTCTTTTTTTTTT |
| >rs6754799 |
| TGAGTGTATAATTTCTATTTGATTTTGTGTCTATGGGGTTAGGTGTGCAA TGTAGAAAAGCTTGAAAACCATTGCCTTATACCCATTTGAAAAATGAACC ACCAAACAAAGGCTTAAAAACATGAATATTTGTCAACCAAAAGGAGAACT CCTGGGCCTGGAAGAAACATCACGATTCAAGTGCTGCAGCACGAAAGAAG [C/T] TGAGGCTGGATTTGTATTCAGCTTGACTGTTTGTAAAGCACCATGCACC ATGCAACTGCCTTCAGTTCCTTTTTTTTTTCTTTCTTTTTTTTTTTTTTT TTTTTTGAGATGGAGTTTCGTTCTTGTCGCCCAGGCTGGAGTGCAATGGC ACAATCTCGGCTCACTGCAATCTCCGCCTCCCAGGTTCAAGAGATTCTGCT |
| >rs6759589 |
| GTGGGGCAGCTCCTTGTTTGTTCCTCACTGTCATTTCCAAAAGGCAG ACAAGCCCTGGCCGCCTTATGCCCAGGGCTGAGTCCACACCATGCTACAC GAGCACCTGAGGGGAGGCTCCTCTCCAAAGTGCATTACCAGGGCCTGTGC TAAATTTTTCAGCAGCCTCTTGTCTCACTAATGGTCCCGGGAAATAGACT [A/G] AGCCTCCATCCCCAACTAAGTGCTCAGGCCTCCCCCACCTCTGTCCTGC CTGGGCAAGAAGCCCAGCGCCGCCCCACAGCCCAGCTCTCCATGCAGCCT |
| CAGCTCCCAGTGGCCCTCTGCCAAGGACTTGCGCCTCTGCAGTGGGATGC AGTGCCTCATTTTATCCATAATTGTACGTGGTCCCTGTGTGATGTCCGGGG |
| >rs1562256 |
| GGCCAGACCACACTCTGTGGGGTGTTGGGAGTTGGGTTTTCTTGCTGCAA GGAATATATGTTCTTTAAGGCAGTCATCTGCCTTAAAGATCCTAAGGTGA GGAATTAGGGGAGCCTTATACTTGAAAGGGAGGTCCCCTTTGGGCCTGGG GAGCATTGGTCTGAAGGCTCAGGTATGGAGGATGCAGGAATATCTGCTGG [C/T] GAGGATTGAGCTGGAACCTCCCTCCTTCCCACTCCCATCTTAGTCCCTC CGGCCTTTAAAAAAATTTATTTTATTTTATTTTTATTTTTTAAGATAGAG TTTCACTCTTGTCACCTAGGCTGGAGCGCAATGGCTCGATCTCGGCTCAC TGCAATTTCCACCTCCTGGGTTCAAGTGATTCTCCTCCCTTAGCCTCCCAA |
| >rs1446125 |
| GCTGGTCTCGAGCTGCAGACCTCAGGTGATCTGCCTGCCTCGGCCTCCCT AAGTGCTGGGATTACAGGTGAGAGCCACCGCGCCCAGCAGACCAGACCCT TTCTAATACCTTCTGACCCCAGCAGTGGGGCCCAGGCCCACCTAACATGG CAGCCTGGATGCACCTCTCTGTTCCTAGGCCTGGTGCCTGGCTCAGTAAA [C/T] GTTTTTTTTTTTAATGATTGAAAGCTCATCTTTATGGTACTTAAAGTCT GTTTCAATTACTAAGGGTCTTTGTCGCTTTTTTCTTAGGGCAGACTTGTC CAGGCCAGACCACACTCTGTGGGGTGTTGGGAGTTGGGTTTTCTTGCTGC AAGGAATATATGTTCTTTAAGGCAGTCATCTGCCTTAAAGATCCTAAGGTG |
| >rs1446124 |
| GCTGGCTGGGATTACAGGTGCCCATCACTATGCCCAACTAATTTTTGTAT TTTTAGTAGAGACGGGGTTTCACCATGTTGGCCAGGCTGGTCTCGAGCTG CAGACCTCAGGTGATCTGCCTGCCTCGGCCTCCCTAAGTGCTGGGATTAC AGGTGAGAGCCACCGCGCCCAGCAGACCAGACCCTTTCTAATACCTTCTG [A/T] CCCCAGCAGTGGGGCCCAGGCCCACCTAACATGGCAGCCTGGATGCACC TCTCTGTTCCTAGGCCTGGTGCCTGGCTCAGTAAACGTTTTTTTTTTTAA TGATTGAAAGCTCATCTTTATGGTACTTAAAGTCTGTTTCAATTACTAAG GGTCTTTGTCGCTTTTTTCTTAGGGCAGACTTGTCCAGGCCAGACCACACT |
| >rs11677262 |
| TGATACATTATTTATGGATCAGGTGGCACAGCATTGCAGGCAGCAGCAGG GCAAAGACAGTGTGGAATCAGAGTCGGGCAGCGTTGGATGGGATTCCGGT TCTGCCACCACTGGCTATGTGATGGAAATCTACCACATCTCTCCAAGCCT TGCTGTCCTGTTTTATCAAATGAGGACATTGTACTGAATCACAAGGTCTG [C/T] ACAATGCCCGTGCATGTGCCTGGAATCTTCCCCTCTGGTGTCCCCTCAA TGCCCATGGTGCTTCCTGCACAGGGTGTACCATAGTTGCTTGCTGTCTCC TGCTGAAGCCTGCTGTGAAGAGGGACTGTGTGGAGGTAAGGCAGTGGGCC TCTCCCTGTGAGGTCCTCAGGGGGCTGAAGGCAAAGTCAGAAGTAAAAAA |
| >rs7565771 |
| TCTTCTTCAGTATTTCCCTTCCTCTCTGAACACCGTTTCTTTTCCTTTCA GATTATCTGACAGCCTTTTAATGAACACCTACTAAGTGCCCAGCACTTTC CTGGGAACTTAATTCTTACAACCTGTTAAGAGGTCTATACACTCATTTTA CAGTGGAGGAAATGGAGACTTAGCGGAGATGTGAAATTTGAACCAGGTCC [A/G] TGTGGTTTCAAAGCCTGCGCATGTTCCTTTACCCTATGGAAGCCTCCAG AAGCCAAGGTCTCATCTCCCCTCTTCCCTGGAGGCCCCAAACCTGGGTAG CTGTGCCCCTAGACCTTTGAGGGAGGGTCTCGAGCCATTATCTCCCTTAC CTTTTCTTGCAGTGACTTGGCTTCTCCCAGGCTGCCGTAACCAGCAAACGC |
| >rs838066 |
| TCACCTTTCGTGGTCGTGGCACGTCCTCTGCTGGATTTTGAGCCAAGGA CAGAGGGTTTGGGAGAAGGAGGCCTCCTTGGACTTCTGCCAGGAGTTACT GAGAAAAGAAAACCCAACCAGCTGGGGGGCCAGTTGGGGGGAAATCTGCA CAGGATGTTCACACTGTGCCCCTCCCTGCTTCTTTCCTGACCCCCAGAGC [C/T] CACCTCAACACATCTGGAGTGTAGGGTGTTGTCTGCCCTGTCAGACCAC TCTCCACACACAGCCTGGGGCAGGAACAATGGGACAGGTGGCTGGTGCTC ACTCAGCACAGCAGGGGTGGTGGAAAGACACATGTCAGCTGAGGCTTGGC TTCAGGGCAGGCAAGTCACAGCCAAGTCACAGCCCAGCCCAGCATAGGCAA |
| >rs895397 |
| TCAACGGTCTTGGCTTGTGACTAGACCAAGATCGCCAACCAGAATGGAGG GAGGCTGACCCAGCACATCCCGGAAGCATTTACAGAGCCAAGGAAGAGAT TCTTCTTGCCCGTCTATGATAACATTGCATTGCCTTTCAGCTGGGGGCGA TTAGAAAGGCACTCACTACTAGATCATGGAGATGCCTGGGTTTTAACCC [C/T] TTGCTGCTCGGAAGGTGGTGATCATGATGGCCTGGGAGGGTTTTAGAAT GGCAGAGTGTCAGGCCACCCCAAAGCTCCCAAATTAGAATGGCATTTTAA CAAGACCCCCTAGGAGGTTGTATACGTGTTTAAGTTTGAGAAGCGCTGGT CTACATTACAACTGAGGAAACTCACTACTCCCTGAACACCTGCATCAAGGT |
| >rs838059 |
| ATAAGGAGCTTCAGGTTATTTTAGGAAGTCTAAGAATCCTGTTTCTGG GGAGCCAGCCTCTGTTGGGGAGATAAGCCTCCGGAGGCCAGAGGCAGGAG CTGGGCCTCAAGGAAGGCCCCACAACTGGACCCCAAAGGGCACGGTGGGG AGGAAGAGGCAGAGCTCTGGTCTCCCTGTGGAGGGAGTGCTGCTGTAAAG [C/G] GCCTTTCCCTCCACCCCCAACTCCCGCCGAGAACCCCCCCACCCCCGCA GGCAGTTTGCTTTCCCCCTCCACAGCTTTCCTCCTACCCGAATACCCACC TCCCAGCTCTCTGCTCAGCCAGGGCCCAAGGCCAGAGCCTCTCTTCTCTT GCAAGAACTTTCCTGTCCCACCTTGCCCCAGGGCAACACCTCTTATGGCAA |
| >rs838102 |
| CTAACTAACTGCCACATTCTCCTGTCCAAATTCTGCCTCATCGGTTATTC ATTCAACAAACACTTACCGAGCACTAACTTTGAGCCAGGCACTCTAAGAC CAGCTGAGTCCCACTTCTGGCAGGATGAGTGTTGTTGTCATCAACCACCC CACAAACACCCGTGCCCTCTGCGTACTTGCGAATCTGAGTGAGGAGACAC [A/G] GTGGCAGTAAGGCTCAGGACCTATAGCTCATGTGAGTGAGCAAGGATGC AAAACCAAAGACACCCGCTCCCCTCACCCACTGAGCTGGAGGGATCAACC ACTGTGCCTGCCATCAGGGCTGATGGGCGACTGCTTGTGGATCACAAACT CTTGCGTTGTCACTTCAACCCAGGGGCAGAGTGTGTTCCTCTTCAGGTGTG |
| >rs3769659 |
| GGGCCCAGCACTGTCTTGCTTCCTTCCCTTCCAGTTCCAGGCACCACCTT CTTTCTGGCCAGTCCAGAGACATCTCCAAGGGCCAGAGGGTCATCTCCAG AGGGCCTGGTGTCACTTCCCGCAGCTGCTCTGGCCTTTCACCCAGGTTGG GGCGATTTGATGGACAGTGGTGGGTCCCCTCAAACTCCACTGCATTCAGC [G/T] TGCTACTGACTCATGTGCAGGCCTGGGCAGTCACCCAGAAAGCCCCCCT GGATGCCAGCAAGCCCACTGTGGCAATGGGCAGGCCAGACCTCACCTGGA GGGCCCAGCTCACAGCCACTGTATCCTCTAGCTGTGCACCCAGAGTTCAC CTTCAGAGAGACACTTGCCCTGACTGGGGGTATACTGTTACTCTCTCAAGC |
| >rs865108 |
| AGCAGCCAGAAATCAGGGTGATGGGTGGGTGGAGGGGCAGGCATCTGGGA AAGCCCTGCCCTCCTGGTGTTCCCAGATCTGAAGGCTGGAGGCTGGAAAC CTTGTGTCACCTGTGCTGGTGTGAGTTCCATTGGGGCTGCCGCTTACCCT CCCATGACCTTTAACATCTGGCATGGCTTGTGGGGGGTGCTGAGAGGAGG [A/G] GAAGTCTCAAGGCTGAGGGAGCTCAGGGTAGAGCCAAGGTCTCAGTGAG AACCCCAGGGGGCAGCTGTCACAGCTGACCTGGAGCCCTCCAGGAACGCG TACTCTCCAGCTCCTGTACTCTCAAAAGCAACAGAAAGGTTTGCTTTCCT CAGGTGCGGTTTCCTCACAGTGCAGCTTCTCCAAAGAGATCTGGAGACCCC |
| >rs708670 |
| GCAGGGACTGACTTCCACTACCCCTCCTTCCAGTCCCTGCCCACTCCACC CCCCACCACAGCCAATGGCATTCTTTCCATCTCCAATCTTGACAACCTCC TGGCCCATCCCAATCCCCCTTCCTTAAAGATCCAGCCCAAGGCCTTGGGC ACTTAAGGCACCTGCCATCTCCCCCAACGCCCACCCCTGACAATCTCTCC [C/T] GGCTTTGAATTCAGACAGGCAGCTGTGTTAGTTGGGGGACCTAGCTGGA CAGAGATGTGTGTGAATTCCGGTCCAGCCATGTGCTAGCTGTGTGACGTT GGGTAGGTTGCTTTCCCTCTCTGTCCTGATGATTAAACAGTGAATATGAA ACCCCTAAACACATGTCTTCCTTAGGACTGTTCTTGTGTGCACCTTTCCTT |
| >rs708672 |
| CCTCATTGTTCTCTGACTGCTGTGGCCCCCTGGGTCTCCTCTCTTCTACT GGAGTGAAAGCCCCTGTGGGCAGGGCTAGATTCCTGAATCTCCGCGGAGT CTAACACAGGCCTGTAGGCAGGGACTGACTTCCACTACCCCTCCTTCCAG TCCCTGCCCACTCCACCCCCCACCACAGCCAATGGCATTCTTTCCATCTC [A/C] AATCTTGACAACCTCCTGGCCCATCCCAATCCCCCTTCCTTAAAGATCC AGCCCAAGGCCTTGGGCACTTAAGGCACCTGCCATCTCCCCCAACGCCCA CCCCTGACAATCTCTCCCGGCTTTGAATTCAGACAGGCAGCTGTGTTAGT TGGGGGACCTAGCTGGACAGAGATGTGTGTGAATTCCGGTCCAGCCATGTG |
| >rs708673 |
| AAACAGATGACAGCACTTGTCTCCAGGGCAAAGCATGGACGGGGGCTGAA GAAGACACAGGCATGTTTCTGCTGGCTGGGGATGGCTTTTCAGTGGACCT TGGAGGCCACACCGGCAGCCCAAAGGAGGCTGGAGGTTTGGAAGCTTTTG CATTGTTGAAGTGAATTATGTGCAAGCCCATTCTTAGGGATAAGTGTATT [C/G] TATTGTTTTCAACTTGTTTGGAACCTAGCGATCTTGGAATAGGGCACAT CTAAGTATCCCGGGCATCTTTATCTCAAGCCCACATGCAGAACCTGTTTT TCAGATCCCTGAGCATCTGATAAACAGGTTCTGGGCAGGTTTCCCCTATC ATCACCAAACACCAAGCCAAGGCCTCATTGTTCTCTGACTGCTGTGGCCCC |
| >rs838100 |
| ATCAGTCACCACTCCCGGTCCAGCCCCTGTGGCCAAGAGCTGGCGTGCAG GCTGCGGGAGGCAGCTGGCTGTGCAAGACCCTGGCAGGGCCCTCGCCTCC TGAGAAACCGAGAGTCAGAACCAAAGCCAGGCTGTCCTGGTTGGAGACTG AGCCAGAAAGGGTGGCTCACCTCACGGTGAGGCTGTCGAGTGACCTGAGA [A/G] CCTCAGACCCTCACGTCAGCCGGATGTCGCACCAGCCTGCTGTTGGTAA GTCTGGCTAGGACGCAGATCCAAGGGGGCATGGGTCGTGTGCAAACCCCT CCCACCAGCTCCCTCACCACCTGACCCCTGCCCTGCTGGAGGGTGCCCCT TGAATCTCCAGGAGGTCCGAGGAACTCGACACCTCCCAGAGATCTGTACTG |
| >rs838098 |
| AAATTTCAGGGGTTCAAGGATTTATCACACACAGTTGCGTGTGTGTGTGT GTGTGTGTGTGTGTGTGTGTGTGTGTGTGTGTTTAAGGATGTTAATGTTTAT |
| GATGCATTTCTGTGGAATGCAGGCCCATTGGGAATTGTAAATTTTTCTTA TTGGTAACCATTTCTGCATTGGTTTAGCACGGAACTGCTGCTGCAAGTAA [A/T] TGAACCAGATTTTTCTGGAATGATTCGAAAATCTAGCTGTTTGTAAAAA GCTCTCTAAATTATTCATTCACTTTTATTTGCACAGTTGTGTGACGGTA AACTCTTTTCCATCAGACGAAACTTGCGCGCACGTGTATTTCTGCGGGGA TTGGCTCGAGTGTGCTTGAGTTTTCTGTATTCTCTTTTGTAGGAACTTCTC |
| >rs838096 |
| ATGCCCACAGAGAAAATAGAAATCTACCAATTAGATGTGTGTGGCTATA AGTGAGCTTTTAAACCATGATTTGAGACTGGCATCCTCTGGCCTCTCTGC CCTCAGGTCAGCTGTGGGAGGCTAGGGGTGGATAGCAAAAGGGACCTGG GATCAGGTGGGGCTTCCTGGACCCCAGGCCCAGTAAGAATGAAGCCTAGG [A/G] GTTCTAGGATAGGGGGCAGCCCTGGATTTGCAGAAACAGCCTGAGAATG AGGGGCTGGAAAGTATGAGTTGGAGCAGCTCAGTGAGGAGACAGAAAACT GAAGCAGAAGAAAGCAGAAGACACCAGAGTCTGGTGCTGACTGGGTGACT GGCTGTTACTCACATCCTGAGCAATGAGCCACAGACCCTCCCCACCCCAGA |
| >rs838092 |
| CATTTCCCTGAGGGCTAATGATGCTGAACATCTTTTCATGTGCATTTGTA TATCTTTTTTGGAGAAGTGTCTTTTCCAGTCTTTTGCCCACTTTATGTAG GTTGTTTGTCTTTTTAATACCGAGGTGTAAGAGTTCTTCAGATATTTAGA AACAGGTCCCCTTATCAGTTATATGATTTCCTGAACTTTTCTCCATTCTG [A/T] GTTGGCTTTGCACTTTCTCGATGCTGTGCTCTTTCGATTTCGTCAGCAG CGAATTAGCAGGACCTGGGGGGCTTTAAGAGGTGAGCAGGCAGACAGACA GCGCCCACAAAGTCCTGAGGATGAGAGAAGAGGCAGAGCTTACACAAAGG CTCTGTGAAAACTGAAGATGGACCCCAGGCAGGTTGGGGTGATCCCTGGGG |
| >rs838090 |
| TCAGCCTCCGGAGTAGCTGGGATTACAGGCACGTGCCAACACACCCAGGT AATTTTTGTATTTTTAGTAGAGATGGGGTATCACCATGTTGGCCAGGCTG TGTGCAAGAATTTATGTTTTCACTTGTGGCTTGCTGAGCCCTGGTGCTGG CTGTGGGCACATACTCGTTCACCCAGGGACATGCACTGTTGATGGGTCCT [C/T] GCACTCTGGGCTATCTTGGGCCCCGTTTGCTCAATGCAGGCCCCTCTTT CTGAAGCGCTTTTCTTGGGCTCACTCTCAATTCGTCAAAGTTCGGCTCAG GCCCACCTCCTGCAGGAATCCTCCCAGTCCCCGCTGTCCGAAGTCAGTAC CCTTGTCAGGAGACCTTAAACACTTTAGTCCACTGCCTGGCCCTCCCATGG |
| >rs12467944 |
| AAGAGGCCGCCTAGGGAGCAGTACAAGGCAGCATTACAACCTGGCATTTT CCTGTTGCAGACTGCATTTTGGTGGCAAGAGAAGCTTTGGAGGTGCTTAA CGTTTAGTTTGCATTTATCCTACCGCTGAAAAGCAGGGACCCTTAGGACC ATAAGTCTAGGACTTTTCATAGCACCCTAGACTGCTGGGGATGAAAGGGG [C/T] TTGGAGCTGAGCTGGTGCAGCAGCTCCTGTTAGAGAAGCCACCCAGAGA GGCCCAGGGACCTCCTATGTCCCAGCGCCAGTTAGGGGCAGACGTGGGAC CAGCTCCCCCTCGGCTGCAGCGGCCTCCATATCAGCTCCCTGGAAGGGAGC AGATGCCAAGTCAGGTTCTGAGCAACACCCTCAAGGCCCAGCCCTGGCAAT |
| >rs10196144 |
| GACTTGGCCCTCCCGACCACAGGAGAAGAGGGATTTGAATTCACCTCGGC CCAAATTGCAGATGCTGGCCTGCGAGCCCTGAGGCCATCTGCAAGGCTCC TGGGCTGACATAACTGTCACCCAGCTCTTCATCCTCCTTCCCCGGGGCTG AAGGGGTGCCTGCAGGGTCTTCTGCCCTCCCTCCACCTGGGCCCTACTGC |
| [C/T] GGCACGGAGCTTCATTTCTGTTGGCAGCCAGTGTTTGCTTAGTGATTAC TGAATGCCGTTTGCTGGGTGCTTATGATGTGCTCAGCACTGTTCTAAGTG CTTTACACAATTGACTTATTTACTCCTACAACCTGCCCATAAGGAAGTG CTGCCATGGTCTCCCTTTTATAGCTGAGAAAACTGAGGCACAGATAGGTCC |
| >rs6728660 |
| AGGCGGACTGGCTCCAGAGCCCGGCTCCTGGACCGAGCAGCCTTGAGTAG GGACCTGGTGCAGACCCGCCTGCCGTGGACTCACTATAGGATGGGTCAGG AGGCCCGTTGTCACCTACAAAATGTAGCCCACCACCCAGGAAACGCTCCC AGAACCAGGCAGAAAATACTGTCTCCTATTCACAGGGTGGCTGTGCCTGG [C/T] GTGAGTGTCTTGGCGGGTGCCTGCATGTCTGTGCAGGAGTGCCACCCCC ATCTGTGGACTCGGAAAGCATGATCCCCACAAAAGCAACCCCTCAGAGTC TCAGCTCAGCCAATCCCTGTTATCCTGTGACAGGCAGGGCCATGTTTCAA AACCATGTTAAAGATGAGCAAGAGGCTAAGGAAGTTTCCCATGCTGGCAGA |
| >rs10188946 |
| ATAATACCCATCGTAACAATAAATATAAATGGCTAATTTTCATCGAGTGC TGCCAGCATGCTCTTTTTGATATGTTATCATAACTAATCCTGCATCACTG CAAAATTCCATTTTACAGAACAGGAAATTGTGGCTCAAAGACATTAATGA TCTTACTGAAAATTCCATAGCTGGTAAGTTGGGATTTGAACCCAGTTCCT [A/G] TGGATCTGAAACCCATGTTCTTTCTGCTATGGTAACAGGGTTTCCTAGA GGGCAAGGAGGAAAAGGTTGATGGCTTTTTGCTATTTGGAGCCAAGAAGG AGAGAGACTTGGCTTTTAGAATTGGACTCAGAAGGAGGAAAGTAACACAC TTATGTACCCGGCCCTGAGGAAGGCAGGCATGTCTCAAATTTTATCGTATT |
| >rs895406 |
| AGGAGGAATAGGAGTTTGCAAGCTGTCAAGGTGTCCTGGGTGGGCGTTC CAAGCCGAGAGGAATGAGCTCTGCCCATTTAGAGGACAGTATGGGACCCA AGTGTTGACACGTCACAGGAGAAGGGGAGATGAGGCTGCGGAGGGAGTCT AGCTGCATAGTGAGGGTTCTACCATTATCCCAAGGACACTGGGGTCACTG [A/G] ATCTTTTCAGCAGTGACATGGTCAGATGAGAGTTTCAGACCATCCTGTG CCAGCCATGTGCAGATGTAGCTTGGGGTGAAGGTAATAGGAGAACGGGGT GAAGGTAACAGGAGAACAGTGTGCAGGTTAAACATTTCTTTGATCTTGAT CTTTGAGTTTGATGTTGAAATGTGTATTTCCAGGGAAATACACAAGCAAAT |
| >rs708675 |
| TGGACCTCTGAGTCGGGTATGAAGTAGTGTGTAAGAAAAATAATAAAAAA GTATTTACAAAATGCAACAGATTACAGAGGCAGGTATGGGGAAGTAAGGG GATAGTCTGGGGGAGCTTAACAGAGGTAGGGTTTTGAAGGAGGAATAGGA GTTTGCAAGCTGTCAAGGTGTCCTGGGGTGGCGTTCCAAGCCGAGAGGA [A/G] TGAGCTCTGCCCATTTAGAGGACAGTATGGGACCCAAGTGTTGACACGT CACAGGAGAAGGGGAGATGAGGCTGCGGAGGGAGTCTAGCTGCATAGTGA GGGTTCTACCATTATCCCAAGGACACTGGGGTCACTGGATCTTTTCAGCA GTGACATGGTCAGATGAGAGTTTCAGACCATCCTGTGCCAGCCATGTGCAG |
| >rs1867856 |
| CCATACTGTCCTCTAAATGGGCAGAGCTCATTCCTCTCGGCTTGGAACGC CCACCCCAGGACACCTTGACAGCTTGCAAACTCCTATTCCTCCTTCAAAA CCCTACCTCTGTTAAGCTCCCCCAGACTATCCCCTTACTTCCCCATACCT GCCTCTGTAATCTGTTGCATTTTGTAAATACTTTTTTATTATTTTTCTTA [C/T] ACACTACTTCATACCCGACTCAGAGGTCCATGACTGGGTCTTCTACACC |
| TTGGGGTCCATGGTTCCTAACACAGGTTCACAGTGCATGCTCACTGAGTG ACTGATTTTTAAATTGGCCACCACAGAGCCAATATCAGGCATCTTAAGAG GAGTGCACGTAGAGCTTGGAGCAAGAACTAGTCTAACTGCCTTGCTGCCAC |
| >rs12711924 |
| CCTGATCCCAGCTCTGCATCTGTTAGCTACATGTGACCTTGGGAAAGTGA TTTGATCTTTGTGCCTCATTTCCTCCATCTCTAAAATGGGCTAATAGTGA TCTCTACTTTGTGGGGTGGTTTTGAGGATTCGATGAGTTAATGCCTGAGA AACGCTTATCACAGAGCCAGGTAGCGTCAGGTGCAGTGTGAGCGCCCGCC [A/G] TCTTGTCTTTCTTGTGTCCTTCATCATTGCCCTCCCGGGGCACCAGCCT TTGCCACAGTGTTCATGGTTTTCCTTCCATCCATGTCAGGTCTTGGCCAA CAAGAGCCACCTCTGGGTGGAGGAGGAGGTCTGGCGGATGGAGATCTACC TCTCCCTGGGAGTGCTGGCCCTCGGCACGTTGTCCCTGCTGGCCGTGACCT |
| >rs1867749 |
| GGACTCAGGGACAGTGACAGCGTAATGCTCTTACGTAATCCAAATTAATT GCTCCTGACCAGGGATGCTTCACATCCATTGGAACAACACAGAGTTGTAG CCGTGTGTTTTTTAAAGTGTGTCTGCCTCTGAATCTCCCTGGCTCTGCTC ATCTCAGAAGGTCCCTGGAGGAGGGCTCATGGCTGACACATGTTAGCATA [C/G] GAGAAGGGGAAGAATTTTATTTATCTGCCACTAATCCCCATGTGTCTCA TACCCAAATCTAGGATTTTCCTCTTCCCACCTGGAGTATCCGGCGTGCAT GGTTTATCCACAATTAAAGCAGACTGCAGGCTGCTCCCCTCACCAGAAAG CCGCTCCCTTAAGCAGCCATAGATTACATGCCCTGGCTCTGCCTGCCAGAG |
| >rs838073 |
| TGCTCCAAGAGCCTCAGTTTACACCCCTGAAAAATGGAGTTAATGCCTTA CAGGGATTTTATGAGCCACAGTAAGAGATTATGTGTGAAAGAAAAATAAA AAGCATTTTGCTATTATTCTTAGGGTATCTGGCCACATTTCCATTATCTC TGACTAAGGTACCAACCACCGATTTCTGAGTTCAGTAGGAGACAGTGAGA [C/T] AGAGGCCAAGGAACCCCAACCCTGGGCCTAAAAGTGGTCCAGTCAGTGT TTGCAAACTGGCGGACACTCACTAGTGGGTCATGCAATTGATTCCATGGA TCACAAGCAGCATTTTAAAAATGAAATAAAATAGAATGACAAAGAAAACA GTGGAGTGTTTTGCATGGTGTCGGGATACTTTTTCAGTTATATGGAATTGC |
| >rs838069 |
| CTGACCTCATGATCCGCCTGCCTCGGCCTTCCAAAGTGCTGGGATTATAG GCGTGAGCCACCGCACCCAGCCTTCCTTTGGTATTTTTTTGTTATTGTTT TGGTTTGGTTTGGGTTGTGCTAAGTGTTTCAATTCAGCCAGTTTTAACTG GCTGTTAGATATTAGACTGCAAAATACTCCAGTCAGAGCAGCTCACCTGC [A/G] CAGAAAAATGAGGGTTCAGCTTTTAGAACCTAAGTTCTTTCCCAGAGTT AGAGGAACTGCCTGTGACCTTGCCCACTCCAGCCACCTGCCTGAAATAAT GACAAGACCAGCCTGACCCCAGCTTCATGAGCAATTGTCCAAAGCCCCTT TGTGCCGTAGTAATTGAGCACCAGCAGGAGCTACAACGACCTGGCTGTTTC |
| >rs3731603 |
| TCCTGTGGCCCCGGCGGTGCTCTTCCCTCTTGTGCTTTGGGGCAGCAGCA TTTTAGGTTTGTAGCTTTGGTGGAAAAGTTGCTCCCCACTGTTTTGAAGT GTAATGCCCCATGAGGAGGAAGTTGGAGCCAGTGTGGTTTTCTGGGTCCT CTGCCAGAGGACATTTGGAGTCCCCAATATTTTTCCTCAGGACTGACTTC [G/T] TGAAGACAGTCCTTCAGCCCAGGCAGGGCTCAACTCTGACGCCCGGGGA GCTTTACTCCCTAGAGTGTTTTCTCTTGCTCTCCCCCTTGGGCTGATTTC TAAAGGGTCTGCTCTAGGCCTTCCTGGGAGTCATTCAAACTGAAGATGGA |
| AAGGGTGAGAAGGCACAGTGGTTTAAAGGACGCTCATCGCCAGGTTAAAGG |

**Table 27. Flanking sequences for rs13387042 and correlated HapMap SNP markers with r²> 0.2**

| |
|---|
| >rs13387042 |
| CTGGCATCCGAAGGTATAGGCTCAAATCCCAGCTCTCATGATTGCTAGCT TTGAGGTTTCAGGTTCATTTCTTAAGTGCTCTGAGCTTTGGTGCCCTCCT CTTCAAAATGGGAATGAGGAGCATGTCTAGCTCCCTGTTTTGTTGCAGTG AATAGCTACATGAAGAACAGCTAAACCAGAACAGAAAGAAGGCAAATGGA [A/G] GCTACAGAAACCAAGGATTTCCTTGTTGAATCGAATCTTCCTTCAATCT TCCTTCACCACACTAGTGGATCTCCCTGTGGGAGGGATGTTGAGAGTGCT CCGTGTTTTTTTTGTTTTTTTTTTTTTTTTTTTTGTGATGGAGTCTCACT CTGTCACCCAGACTGGAATGCAGTGGTGCGATCTCGGCTCACTGCAACCTC |
| >rs1882419 |
| AGCCATGGGGGTGACACAAACTCCAACAAGCACCAGGGTCAAGTAGAAGC TAACTGACCCAGGAGAGAAAATAATTCTGTCAAGTTAAGTAATTTGCTGG TGTTCTCTGCTAACTCACATCTGCGTCCTGGGTCTCCTTAGCTCTTCCTT GAAAAAGCAATGAATGTTTTCTAACTGTAGCTACCCCTAGTTCTTCAGCA [C/T] GTCTGTGGCTGGCTGCTTGCCACCCAGGCATGAGAGGATGCTGTCCTCT GGGAGTCCCAGCCTTGTGGTAAAGAATGCAGGCTGAGAAAGACTGTAAGA TTCATATTCAGGATCTTCCACTTGGTGGGCAACCCTAAGAAACTCATTTA ATCTTTATTAGCCTCCGTTTCCCCTTCTGTAATAGTACCTGCCTCACAGGG |
| >rs2252488 |
| AGACATGGTCAGGGCCTCAGACCCTGTGTGTGGCCATTCAGCTTTGCCCT GTTCTGTGGCCACAGTCCATGCTCTAAGTACTGACCAGATGTCTTACAAT GCACACGGCACACAGTTATGGTCTCTGTGGGGATGGGGGAATAAGGTGTG GACAAATCTGTTCAGCTCTAAATCCTTGACCAGAGGAGCAGCTCAGAGCA [C/T] GAATCCACAGGGCAGTGGAGCCATGTGCAGGATCAAGCACAGCGGCATC ACTGACTTGCAGGACACTGGAGTGCTGGGGCGTGGTTCTAGAGAAATGCC CTCAAGTCCTCGATAGATCCTAAATGTGGTTAGTTCTCTTCATTACAAAG ACAGTGGAAGCTGAGGTCCATGCAGATTAAAAGGCTAAGCTGTACAGTAAT |
| >rs2272525 |
| GGACCCTGGTCAGCCTTAACACAGGCTGTGATCATCTCAAACCTTTCGAG ACCTTTATCCATAAGAAAATAAGAGGAAATTTTATTTCAAAGTAGCATGA AGTGATATGCCAACTCACTTGAACAGCATGACTATTTTCTGGTTCTGTTT TGTAGTTACTTTTCACGTGGAGGCCCCATCTGCAAAATAGGACAAAGGAA [A/G] ACTCACTGAGACCAAGTCTGGGATTCTGAACCCCAGACCACTTTCTTCC TCCTTCCCTCAAACACACTCACTCTCTCTCACTCTCTTCTCTTTCTCTCT CTCTTTTTTCTCTTTCTTTCTCTCTCTCTCTCTCAGGTCCTGGGGCACTC TGGACACAGTCTGAAAACCATTGAGGTACAGCAAGTACCACAGAATGGAAT |
| >rs4396687 |
| TTTCCAAGGACTTAAGCAAACAGAGTCAACTCCTCTGTTTTCCTTGTTTG GGTATATTTTTGAAAATGTCTCTTCTTCTTTCCTTTCATTTACATTTTAC CGTTGTTTATCCACCTTTTCCTCTTCCTTCAAAGCTCTAGACCTGCCTCC CTGCCTGTTCTTAGCCCACTTTTGAGAAAGGACTTTCCAACCCCATCCTC [C/G] |
| AAAGGGCAGGGTCTCTCTCCAGGGCTCCAACAGAGTTCTGAAAGAACAC GGCTTCCACCATTGTGTCTTCTCTGTTTTGTCAACTGCTGAAAGACTGAC ACAGTAGACAATGCTGCAGACCCCTTGGGAGATAGTCCAAGTTTAGGGTG GGGAGGGAATGGTGCATTCTGGGTGTCCACATATTTGTTGTTGCTTTAGGA |
| >rs12614767 |
| ACAAGAAAAGGGAGTGAAGTGTGTGGGGTTTTTAAAGTGGTGGTGGGGGG GCATTAGGAGATACCCAGAAGCTCTCAAGATTCCAAACAGTCTGCTTTTC TTTTCAGACATTACACTCCAAGAAAAGAAACTCTAGTGTATAACAGAGGG GCTCTGTCAAAATTTAACAACATAAATATTGGGCAAATGAAGTTGTTTCT [C/T] TGGTGCTATCTCAACCTGCCAATGGAGAAATATCTCAAAAAGTCAAGAA TTGAAGCCATTACTCAGAGACAGCATCCTATGGCATGGTGATCCAAAACA AGTACACCTCTACCTTTTCCCTTTCAGCCCCAAACCCCAAAACACTGGAG AATATTGGGGCAGGCAAGGCCCGTGAATAGGTGAGCAGAGCTGGAAGAAGA |
| >rs4571035 |
| AAAGGCTTTGCCAGTAGTGCAGCCCTCTCCTCCCAAAGTCCTAGAGGCAA TTACTCTGAAATATGGAAGAAAGAGGCACATCTGAAAGCCCAGTGCTGTC TTCTTGTCCTCACATCCCTCAGGATCTCAAGGTCAGCCTAGTAGAGGCAT TGCTTTACCATAGTAGCAACTCAGGGAATATCTGTGGAACTGGATTCCTC [C/T] GAAGTTGGACAGTTGCTTTATCCCTGCTAGAGGCTCTCACTACTTAGAC CCACACAGTCCTATAGATAGAAGAAACCTAGAGAGAACCACCTCTCACAC TTTTATTTTATACATGGAGAACCAAAGGCCTCAAATGGTGGAGTGATTTG CTGGGGTGCTACAGTCATTGTCACAACTTACATTGTGACATGGGCTTCATA |
| >rs10195963 |
| GCTGCAAGTCAAGTTCGACATTCAACTATTTCCTCGGGGTCCTTTTTTGG GAGAAGCAAGGCTTTGAGTTCACTCTTTGACTTCAAGTGAAGTCCATTTT CCTCTTAGACACTACCCCAGTGGTTCTCACACTCAAGGAAGCACCAAAGT CCCTGGAGGACCCGTCTAAATGAAGGATGCTAAGCCCCACCTCACAGAGA [G/T] TGATGCAGTACGTCCCAGGAATCTGCATGTTAAAGGAGCTCTGCAGGTG ATTCTGATGGAGGTGGCCCTTGCAGGATGCTTTGAGAAACTATATGTTCT ATCCCTCGAACTCTGCCTGCAGAACAGCCTGAGATGGCTCTTCCATGCTT TGACCATTTGGCCCTCCACAGTAGTCCCTGAGCTGCGTCTTCTTCGGACCA |
| >rs10207736 |
| TGTCTAACCCCCCTTTCATCTGGTTATAATGAATGCCATTGCTTGTGTAT AGTTGCCTACATTAATAGACAACAATGCAATATTCTCCTTATGGAAACCC TACCTAGATTGGGAGGCACCATGAAGCCACTTTTTCTTCTTTCTCCATCT CTACCCTGATTAGCAAACATCTCTAATTATTGTGACTCCCAACCCCAGTC [A/T] GATCATTTTATTCTTTTTCCAGTATCTATGCAGGGAAGCCTGGCAAAGG CTTAAGGGATGGGGTGGGGTGTAGCATGTCAGCAGGATTTGGAACTATGG GCCCCATACTTTTGGCACCTGCTGCATTTTCTCCCCACCTGCTGCACCAT AATTCATCTGCTTGTATGAGTTTAGCAGAAAGAGCCCCCAGTTCCCTGTAA |
| >rs12464018 |
| GGGAAATTTCAGTATACATAGAAGGCTTAATTCAAAATGATATAATGTCA ACATTTGGTATAAGAAATTATTTATCTGGCAGAATATATACTCACAGATG TCAAAATGGGTCTAGAGAGTAGAGAATGGAGGCAATTATCCAGACACTGA GGCCCAAGGCACAATACTTTTAAGTAATCATGCCCTCAACTGGCATTTCC [A/G] TAGTCTTAAGCCCAAAAGCATTGGGTGGAACCATTCTATAAGCAAAGCC TCAGCTATGAAAACAACTTTTAAAATTGACATTCAAAAGATAAGATCAAC |
| AACAGGTGCCCTTTGCTTTCGGCAGTAGGTGTGTTCTTGAAAAGTTGGTT GTCAATATTTCCTTTTCCCATTAATCTAATCACATTCTAAATATAACAGGG |
| >rs10932689 |
| GGCTCGAATTATGCTTGCACTGAACAAGGCTAGTCTAAGGTATGCAATTG CCCTGCTTCTTCACTACTTTCAAGTGTAAACAAATATGTCAGCATTATCC CTAGAAGGAGGGTTTAGGGGTTTTAAGTTGAATACTCTTCAGCTGAAGAA AATCCATTAATCTCCAGTTTTATTCTTGAGTTAGCTTTCAAATCTACATT [G/T] CGACAATAATTACTTTGGGATCTTTGAATTTTCTTTCATGCCTTTCTGT TCTATGAAGCCCACATGCGTAGTTTATGCATTTTCCAATTTAGGCTAGAA GTCCAATGTGCTGTCCATTGCACCACAGAGCCTGTTTGCTATGCATTTTT AAGAGTAAAATTTTCAGAAAATACTTAAATTAAAAAGATAGATTTGGAAAG |
| >rs12613030 |
| TTAAATTAAAAAGATAGATTTGGAAAGCATATCCAAAATGTTGCTTTGAA CTTCTTATTCTGGACAATTCCTGGACTGGGGAGAAGGCATGCCCAAGTAC ACATTGCTTGCAGTGGCACCTCCAAACCAATGCTGGTCGGGACTCTTCCT ATTACCTCGCAACAGCAATGACTGGGCCCCATAATACAGCCACGGTCATT [A/G] TAAAAGATCATTACAAAGCATCAAATTAACTGCTATTAATGAAAGCCAG AAACACGTCATTCAGGAGGTCGAATGGCTGGGATGGAACATACAGGGTTG AATAATGGTAGGAGCCTGGGTTTGGAATTAGAAAACTGGGACAGGAATCC TACTTCTGCTATGAATCCATTGCAAGTGACTGGGCAAGTTGTTCCTCACTC |
| >rs2888450 |
| CAGATCTCAATCCGTCTTATCCTACCCTCTTCCTGACCTCTAGCTACAAG AACAGCAAGGCACAGCCCCCTGGTTTTGAGAATGCTAATGCTTGCCAGCA TTCTTGTCCTGCAGGGCATGAAGTATAGTCCTGCACAACCAGCAGGCTCT GTGACTGCCTAACCTCATCCTTGGTCACTGACCTCTGTCTTAGTAGCAGT [A/T] CGTGCCATCCTACCTAGCTTTGCTGGGTCAAGTGCGTTCACATCTTAAA ACCGTTTGAAGCGTTTGTCTAATTAACATGCAGAAAGCATATACTGATTT TTATTTTCACCACCATGTATGTGAGGGCCTATTTTATGGCATGCAAAGCA TCTCATTTTTATTTCAATTCTCTGTCTTGTAACTTTAAATGAGGCTGAAAT |
| >rs4254482 |
| TTTTCCGGTTTGTGACTTACCTTTTAATTTTGCTCATAAATTACTTTTAA CTTACAGATAGTAATATTTTTGAAAGGTTACATTCATCAGTCTTTGTATT GTTAAAAAGAGTCTTTATTGTTATACTTTTAAAGTAGCTTTTCCACTCCT AGTCAGATACTTTCACATATAATAGTTGTCTACTTTCCTCCTGAATTAAT [A/G] GGGATTTATTTTGGCATATGGTGTGAAGTAAGAAGCTAAATTCTTTTTC TTTTCTCTGCATCATCAATGATCTCTTACACTGTTAAATAAACTAGTCTT TTCCTTCTGCTTTCCACACCCATTTAACCTAGCCCACTGTTGACATCGTC AGAGAAAACTTTTCATCAGAAAGTCTGAACAGCACAATTATACTGTATCAA |
| >rs2372932 |
| TTACCTTTTAATTTGCTCATAAATTACTTTTAACTTACAGATAGTAATA TTTTTGAAAGGTTACATTCATCAGTCTTTGTATTGTTAAAAAGAGTCTTT ATTGTTATACTTTTAAAGTAGCTTTTCCACTCCTAGTCAGATACTTTCAC ATATAATAGTTGTCTACTTTCCTCCTGAATTAATGGGGATTTATTTTGGC [A/G] TATGGTGTGAAGTAAGAAGCTAAATTCTTTTTCTTTTCTCTGCATCATC AATGATCTCTTACACTGTTAAATAAACTAGTCTTTTCCTTCTGCTTTCCA CACCCATTTAACCTAGCCCACTGTTGACATCGTCAGAGAAAACTTTTCAT CAGAAAGTCTGAACAGCACAATTATACTGTATCAATCCAGCTTAAATTACA |
| >rs4255939 |
| TGATCATCGATATACATGATGTCAAAAATTTGGGTATAACTGAAGAATTT TAGTAATTTAAAGATTTCAGAATTTAAATAATGAAATATCTGATACATTC AATTGGAAATTTGGTTGAGATCAGCTGACTTGTTAATTGGACTTCACTGA TAAACATTTGCCTTAAAATCTGTAGAATCTAAAGCAGGGTATACCTAGTA [G/T] GCGAGTAAGACATCATTTCTGGAATCAAGGGGGCTTTTCAGTGTTGACC AGATGTTTATCACTGCATGGGCAAATGGCCCAAGGAGTATGGTTGTGGCT TGGCTTATTTCTTCTTAACGCAGATGCAGTTGCTTTCTTTCTGGGTGGCA GTGTGGAGGATAACTGGGGTCGGTGGGGACATAAAGTCTCACAAGGGGAGG |
| >rs10192415 |
| TTCTTTATTGTATCCAGTATAACATACTCGAAGATGTATATATACACATA TATAGATATACAAAGCATCTATACATCTAGACATGTATATATACATATAT ATATACACACACGTATATACTGTACTTCAATCATCTATATTAAATTGTGA TAATATCACATTACCACATGTTGATGGCATTCTTGTAATCTGCCTGAAAT [C/T] CATGAAAACACATGCAGTGGTCTGGCTCATGTTGTCTCAGGGAGTGCAT TCTGGCTCCCAGTGATCATCAATTACTTTTATAAAAGTTCACCAATCACT TGTTGGTTTCCGGAAAACAAATTTAAAGCAAACATTATTAATGTCAAACT TACACATTTTGCTTTCTGGAATCAACTTTTTTATTTTAAAAATTGAGAATA |
| >rs7595393 |
| CAGGGTCCACGGTGTCCTTCGTGTCCCTCCCTGCCCAGCCTCAGCAGGAG CCCCCTGCTCCTAGACCACTCCCTTACTGAGTCCTGTAGATGTTCCAGCA CACACAGTGCCTCCCTTCTCAAGGCCCCAGCCCCAGCCCCTTCTCAGGTC ATTTGGGGGCTTACATCACCTGCTACCTGTGTGGAGATGGAGGTCTGTTT [C/G] CCAGCACTGCCTTGCTTTTCCTCCTGCCCCACTGCATTTAATCACTGTG ATAAGAGGAAACAAAGAGGGAACGTCATTGGTCCCATTCCTCTGAATCTT GACCACGAAAATATCTTGAATTGCCTCCGTGTCCCCACCCAACATCGCCT CTTGCACTGCTACCTCTGCCTGAGTGCACATCAGCCAAGAGCCAGGAACCA |
| >rs2372935 |
| CCCAGCCTAATGTGTACTTTTAAAAGCACTTTTCAAATACTTCATCTTGT TTAATCTTCTTGGCAGTTCTGTAAAGTGTGAAGCATTGTACAAAGTTAAC TCTCATTATTATATTGGAACCCAGGGTTTCAGAGGAGAATCCTCAAGGA GATAGGGCTTTCCTGCCTTGTGCCCATATAAGGTTGTGGATCCCAAGGGA [C/G] GAAAGCATTTTCATCAGACTGGGAGAGGCAAGAAAAGGTCACTGGCAGT ATAGATTCTTCTGGAGTTCACTGGCAACTTGTCCCACTTCAGTTCTCCCT TAATCTTTCCAGATGTGCAGATGTATAGCCTGGGTGGATGAGCCAAAATG AATTCAAGGCTAAACAGGTCAGCTGGCTCAGCCCATGGGAAGCATGCTCAT |
| >rs6733648 |
| TGGAGAAACCAAGAGGTTGATTTCCTCAGCCTTCAAGAGAGCTCATCCTC AGCTCTCTAAAGCTGACCAGAAAGAGGAGAGTGGGACCTATGAAAACAGC AGGAGATGGGAGGGCTTGAATTCCTAATGCTATACCCATAGGTCATTGTG CCATGGTCCTCTGCACCCCCAACACCCCCAACACTGATCCTGAAGGTCTC [A/C] ACAATCTTCATCTGCCTCTCTCCTTTCACCTTCTTTACACTCTCATTGA TAGCCTTGGTTTTTTCTATACCTCCAGTATTCCCATTCCCATGATAGCCA TTTAAGGACAGGGCACTTCCTGGTCACCATAAGGAATATTGTGGGAATGT TCTGCTCATGGATATTGCATCAGAATAGGTCTGGAAATGAAACACACCAAC |
| >rs6435957 |
| GAGACAGATGGAGAGTGAGAGAAGGGAGGCAGGTAGGTAGGCAGAAGAAT GAGGAAGAAAGCAAAGGAGTTGAGACTGGGAATGGACAGAAGGAAACCTG TACATGCTGTGATCTGGGCTTTCTATGTTCCCTGATCCAGGGTCCTGAGA TCTTGGGGTACTCGCCACTGCATTGTAAGTTTCTGTGGATGAAGCTGGCA [C/T] ATAAATACATCTGTAAAGCTGTAACTCCTGGGCATTGTCCCTGAAATTT TACTGATGTCTAGATTCTAGGTATGGGATTAAATGGGTCCACATTTTGTG TTTTTCTGAGTCACCAATTACAACAAAACTTGCTCATATACTTATGCCTA TCTTAAAAAATAATTTATTTTAATAAAATAATAATAAGGCAAGAAAAAAAG |
| >rs6723019 |
| TCATTATCTCCAGAAAGCTTTCCAGCTTCCAGTCACTGCTTCACTCCAG TTTTCTTTCTTCTTTTTTCATCTTGGCTTATTTTAGTTGGCTTCTATGCC CATGACTATCACCCTCAACCAAAGACAGAGAGGCTCCAATGCAGGAGGCA GACAGATTTGGGCTTGAACCCAATGCCGCTTCCTTGCTGTGTGAGCTTTG [G/T] CAAGTTATTCTATTGCCTAATTTTTGGGGATTGTATTATGTTATGTTAC ATGTTATGTTTTGTTACATGATATTGTGTTATATTACTATCTTCTTCATG GTGTTATTATATTATTAAGATTGATGAGATAATGTGTGTCAGATTTCTAG CAGGTTTCTTGGCATATTGCAACCACCAATACACTGAACATATACTGTCTA |
| >rs12614773 |
| CCACATGTTTCTCATCCTCCTCCCAGGATTAGTGGGCAGCCCAAGCATGT CTTTCTTCAAAATGATGCTGGATACATAAGAGAGCAAGCAAAAACATACA TGGCTTCTTGAAGCCCAGGCTTGGAATTGGTATGCCATTGCTTTTACTGG CCAAGCAAGTCACATGGGTGGCATCAGGGATGGGCTTATGCATCATCTAC [C/T] GTGGGAGGAGTTTTCTAAGTATGATGAGGGCACAGATACAGGGAGCGGT GACCATTACGAACATTAATGCGAATCCTCCACACTGTGTTTCTTGATCTG CCAGGACAGGGTTCTCTGAAGGCCAGTAGAGGTTCAGTGCTTCCAAAAAA GGATGCTGGGCTCAGGTAGGTAGACAGAGTTCATCACCTCTATCATGACTG |
| >rs17833842 |
| TAACTTATAAATTGACTACAATCCTAATAATCACATTAAAATTTTTTATA GTGAAATTGATACTGAAGTTATATGGAAAAAATAAGAATAACTAGAGAAA TATTAAGAAATTAAAAGAGAGCTACAATGAAAGATTAACTCTGCCTAAAT TTAAAAGGCATTCTAAAGTTTCTGTGATTGAACTAGTAAGAACCTTGTGC [A/G] TAAGTGGAAAGACCAACTAATGAAATAAAATATGATCTGAAAGAGACCC ATATGCATGTGAAAATTTAGTATATAATAAAAATGCAAGTTCTAATCACT TGATAGTCATTCCTTTAAAGAGATACAATGAAATCCATACCTTAAACCAT ATAAAAGAATAAACCTCAAATGTATGAAAGATAGTATATATAGAAAATAAA |
| >rs10177578 |
| TGACTAGGTAAAGTTTAGAAACAACAAATTTGAATGTTCAAAAATATTAT CAGCAGTAAAGTCAAAAGCCAAGTGACGAACTGGTAGAAAATGTTTCTAT ATATATCAGAGATAAAAAGTTAATGTCCCCAAAGTATAAAGAATTTTTGA AATTGAGAATAAGCGGAAAACCAAAAACCAATATAAATGGGCAAAAGATA [C/T] GTTCATATAATTTAAGTGGTCCCTAAATATATAAAAATATCTTCAACTT TCAAATATCCTTAAACATATAAACATACATTCAAATATGTTTCTTGTAAT AAGAGGAATGAAAATTAAATATAATGAGATACCATTTTTCACCTATCAGA ATGGTAAAACAATTTTTTTTAAACTCTTAACACACTGTTGACAAAGCTGTG |
| >rs12623304 |
| AAAAAAAAACTATGAAAAATGATGGCGATATATCAAAAAGACTCAGGAT CCAGTGTGAGCAGCTCTTATTGGGCAAGTCTTGGAAATTTGAACAACAAA |
| ATCAACAACGGTTAGTTACACATTAACATGTTTGACAAAATAAGAATCCA TGAGTCCAGACTGATATAAATTAATGAGTGAATAAATGAATAGGTGATTG [A/G] GAAGAAAGGAAAGCTTCTCTTCACAATAGAATGCCAATTAACAAATACA AAAGGAACAATGGAGTTAGAAAAGAGTTAGAACACTACCATTTTGCAGCA ATTACAATAATAATTAGTTTAGGTAAGAATCATGAATGGGTGATACATTT ACGGAGACTGTTTGATGAGGAACTGGATATTACATAGCCTCACAGTGTTTC |
| >rs6716542 |
| AGAACACCTTCCGCACAAGCTCTTCAAATATCTCAAGTTCTCTTTTCCAG TATGGTGTTGCAGGGTGTAGTGAAAAATGCATGAGTTTGGAGTCAAACAG ATTCCCGTTCCATTTCTGGTTGTATCCTATATTACCACTTGGAGTTGTTG GCAGTCTTAGGTGAACACAGGAGGCAATTCTTTTTTACAGTGACTGACA [A/G] AGAGGTGAGCAAGAAATGTGAGTCTCTTATTTATTCCCAGCCCCTCTAT CTTAACTTGTTGAAGGTAAAGACCAGTGGATCTGAATTTAGATTATTTAC CCTCCCATGTGTGGGGAACACACACACGTGCACACACACACCAGGCTGGA ATTTCTTCAGTTTGTTCCTCCGGGTCTACTCTATACTTCATGGAAGCTGAT |
| >rs17777330 |
| AGTCAGCAACATGTCTCCAATTAACATGACAGATTTTTGGACCACAGAT CTCTTTGGGAATCTTATGTAAGCTGTGAAACTTCTCCCTTAAAAAACCTA CATATTTAATTCTGTCTGTAATTTTAGGACTTCACATATCCCCAAAGACC ATTCATGATCCACCAGGGTTAAAGACTTGTTTTAAGTAAAGAAAATATTC [A/G] CAGCCTCTGGAGTAGTAGACATCTCCAGAGAAAGTCTGGCATTCTTTGG TAACTCATGGCATTTATTACATTAGCTTTGGATGGAACAACCCCCAACCC CCAAGTCTGAGCATGTGGTTTGATGCTGCTTCCTTTCCATTTTCACTCTG CTCTCTTGGATTCTATGTAGCAAAAAGGTACCTTTTCTAGGGTCACATGTC |
| >rs10211546 |
| ATATTTCTAGGGTAGTTTAGGTAACATAGAACTTTGTAGCTCAGCAGTCT TGGAATCACTAGACTCTAAGCCCCATGTAGTCAGAGACTGCCTGCTTTTA TAATTCATCCCCAACAGCAGGCTATTGCATAACACATTGTTACCACCCAC ACCATTTTGTTGAGTAAATGAATGCATGCATGAACGAATCAGAGGATTCT [A/G] TTGTCTAAATAAACTTTGGAGGTGACTTAGTCTTCTCTTCCCTCTGATG CTTAGATCTTCTTTATCTCTCAGTTCAACATATATTGCTCAAATGGTTCC TAGGTGCAAGGGATCTGGAAGTTATTAAAATGAAATAGATTTTGGACCAT GACCTTGACAAATTATGATCTACTAGGAGAGATAATATGGAAATCAGTATA |
| >rs2372937 |
| GAAGTTATTAAAATGAAATAGATTTTGGACCATGACCTTGACAAATTATG ATCTACTAGGAGAGATAATATGGAAATCAGTATAATACTACAGAGACAGT AAAAAGGAAAAAGAAATGTTTGCACTTAATAATTATTCCACCATTGTTCC AAAAGTTTCAATTATAGGGAACTAAGTTCCACTACTAATAAAAGTCAATA [C/T] GCATTGTATGCTTACTGCATGCTAAATATATTATGTCATTATAATATTT ATGTTCAGGTGTATACTATGATTGTATGTATTTTACGACAAGGAAATTGA GGCTCAGAGAGGTTAAAGAACTTGCTCAAGGTGACAGAGCCAGTAGGTGG TAGAACAAAGACTCCTACATTCTGACCTAGTCCATATGTAAGCAGATTTAT |
| >rs4315498 |
| TTCAATTATAGGGAACTAAGTTCCACTACTAATAAAAGTCAATACGCATT GTATGCTTACTGCATGCTAAATATATTATGTCATTATAATATTTATGTTC AGGTGTATACTATGATTGTATGTATTTTACGACAAGGAAATTGAGGCTCA GAGAGGTTAAAGAACTTGCTCAAGGTGACAGAGCCAGTAGGTGGTAGAAC |
| [A/G] AAGACTCCTACATTCTGACCTAGTCCATATGTAAGCAGATTTATTGCTA GAAATATCTTGATGTTGCATTGAAATCTGCCTCTCCTTATTTTCCATTTG GAACTAATTCCACTCTCCCGAGCCAGAAAAACCAAGCCTCTCATTACTTG AAAAACCAATTAATGAGTTAAATTACATCAAGTTAGTTTAACAGTCAGCAA |
| >rs12998806 |
| TAGGTAAGTCTTCACAGAGAAACTGGTATTAGGGCTAAGATCTAGCGGAG GACTAGGGGCTTTCTAGACAGACAATGGAGGCAGGACACTTCTGAGAGAG GAATGAAGCCTTGAGGTGTGAGACAGCATGTGGTAGAGCATGGAGGGTTC AGGCACTTGGAGGAGTTGGGTATGGGGAGAAATACAGAGCACCTGTGAGA [A/G] CCTGGGGCATGGCTGGAGAGGTAGGCAGGGGCCGGCTTGCAAAGGGCTT TACCTGTCATGCTGAAGGGCTAACTGCTGTTCTGGAGAATTTGCAGAGCC ACTGAAGGTTTTGAAGCATCCTAATGTTAGGATTCACTTCAAGTTTTAGA AAGTTTAGTCTTTTGGCTCAAGGGTGGGGAGGGAATAAAAGAAGCGAGAGT |
| >rs12465515 |
| CACCTACTCAGGAGGCTGAAGCGGGAGAATCGCTTGAACCTGGGAGGCGG AGGTTGCAGTGAGCTGAGTTGGCACCACTGCACCCCAGCCTGGGCAAAAA GAGTGAAACTCTGTCTCATAATAAATAAATAGAGTGGACAAGGAAATACT GATGGGGGTGAGAAAACAATTTGAGAGACATTTGAGAAGTAGTGAGATAA [C/G] ATTTGGTTACTGATTGAATATGGAGTGTTAGGATAACCCTGGGATCCTC ATTTTGGGCAATCACAAAGCTACTGGCTACAGCAAAAGGAGGGGAAGAAA AGTGGTGGTGGGAGGGAGGCATGAGAAATTTGCTGCTGGCCATGTTAAGC TCCACCTCCTGTCACCAAGTGGTGATGCTCAGGAGATGTTTGTGTCCACAG |
| >rs4491709 |
| ACCTCCTGTCACCAAGTGGTGATGCTCAGGAGATGTTTGTGTCCACAGGA GGAGAGTGATCTGGGTGGGAGGTTTACATGTCCCTCCTTCCATCTCCCGG TCCTTCTTTCCCTCCTCCCTCTTTCTTTGTTTGGTACTTCCTTTCTTCTC TCTTTTCTTTTTCTGCCTTCTTTTGAGAATGAAACCTAAGAGTGTTTATA [C/T] GTAGAGGGAAAAAGTGGAGCTAGAAGTTTAAGACACAGGAAAGGGAGGA AAAATGTCCTAGTGTGAGATTCCAAGGAGGAAGAAGGGCCAGGATAAGGT GGGGAGAGGGATGCCTCTTTTGCTCATTGCCTTTATCTGTCTTTGTCTGT CCTGGATTGATTGTCCTATCCTTTTCTTCCACTTCGTGTCTTCTCCCGTAT |
| >rs2372938 |
| GGATTGATTGTCCTATCCTTTTCTTCCACTTCGTGTCTTCTCCCGTATTT CTACACAAGGCACAGTCAAATAAGGGCTGGCACGTGCGCATCTTTGTGCA CACATGTGTGTGCACAAGCATTGAGGGATGCACAGGTGCACACACCTTGT AGAGCCTCTAGTAGCTCAAGTCACATTTTTTCGCATTTCCAGATTGTAAG [C/G] CTTACTGAGGGGCAGATGGACGTTCTCACAGGAAAAAGGCTGACCTCA GGGAGAAAGATGTGGCCCCCACCTACAAACCAATGATGTCTACTCCCAAC AGGGGACGTCTTTCCCCTGCGTGAAGCAACAGGACAGATTTGGTTAGGTG TGGCATCGATGTCCTAATCTCCTATGAGCACACAATGAATGAAGGTGGGGG |
| >rs12621130 |
| GATGGGGCTCTGACCCAGTTTCCATTTGGCCAGCCGACTGGAAACAGCTA CTCACTGGGCCACTGGGTTCTTTCCAAAGAACAGTGAAGAACAGTGGTTT GGGGTAGGTGGGGGCAGGGGCTGGTGGGGCAAGAATGTCAGAAGCAAATG CAGGCGTGGCTGGTCCAGTAGTTCCAGTACCAAAGAAGCCGCTATCTGGA [A/G] GAGTGGGACAACCCATCCTAAACATGGGAAGAAGAAACAGGAAATTTCA |
| AAAGATACAACTACTAATTAAATGAAAGCACTCCTTAACCACCCCTTTGA TAATTAGATCATCTTCTCCTAGAATGAGAAGGAATATTAGTCTGACTTTG CCCTCCATATGGAGAAACATGGCAGGCAGGCTCTCTGTGCCTGGAGGGTCT |
| >rs13399995 |
| GAAGGACACTGCAGCCAAGCTGAGCCTGTGTCCAGCTGAACACGGACATT TACTTCTGCTTCTGAGGGGAAATATTGTCATGTCTTATGGGTGTACCCAG CAAAAGATGTTTCCACTAAAAGACTATAGAGAATTGCTTATTTCTAAGGA CTGAATTCCGAATGTTTTTGGTCAGGTTCTCTCTGAACAGAGAACCATTG [C/T] CCCTGAGCAGAAGCGGTCTTCACACAACTAGCAACCATGTAGTGTTTTC TCATTCAAATGGCGTTTGGTATTCACCTGCCTGTCCTGGAACAAATGCAA AGTCAGGGTTGTTTCTAGCTCTCCCCCAATATTGGAAAAATGCTGATGAA CTGAGCCTGGTTCCATTTTACACCTGACAAAAGGAACTCAGATGAGAGTGT |
| >rs12052807 |
| TGCTCTCATGTGTCTCCAGGAGAGTGTGGCCTGGGTTAGCTTAAGACTAG GAATGTGCTGACAGCTGGGAAGAAAGTCAAAAGAAGGCATGGCATGTGGT TATGGGGCCGGAGGGACCGCTTGGGGGGAGAGGTGGAAGCGCTGACATGA GCTTGCTCTGCATCGCTGGTTCCCAGAGGTGGCCTGATAGGAGAGTGCCA [A/G] CTGGTGGTATGCCGTGGGCTCTGGGGAAGGGGCTAGAATGTTGTTGCAG CCCAGAGGGGCTGGGCTAGTGAGGAGCAAAAGAAAACATAAGCTGGGGGG AAACTGCCTAATAGACCAATTAGGAGACAGGGTTGTGCCCTAAGGATGG GTGGGAGCAGGGGGTGCTAGAAGTAGACAGAGAGTAACTCTCAGCTCTGAA |
| >rs10199394 |
| GCAAACTCATCTCATTTGCTACTTGTCCTATAGGTAAGGATGGACACCAA ACCCAAAGAGACTTTTGGGATGTGGATAAAAATTGAAAACTTTGCCTCTC ATTAATCCCTTGGAGAATATAGCATCTCCTTCAAACTGGACTGCTCTCCT TGATCCCTTGTCTTTACCTGAAAATATCTTTCAAGGCCTAGCTCATGCTA [C/T] CTTGGCCATAGGAACTTTCCTTATCACCTCCATCAATGCTGTTTGTCCC TCCACTAGAATCTTCTGTTTTGGTACTCCTCTTACTCAGCCTCAAGTTAA ATTTATGTGTACTGGTTTTAACCCCAGTACCCAGTAAAGGTTCTGAATAC ACGTGATGAAATAAGTCTCTCCTATCCCATGGCACCTAGTATAGCATTTAG |
| >rs2372940 |
| AAGTTAAATTTATGTGTACTGGTTTTAACCCCAGTACCCAGTAAAGGTTC TGAATACACGTGATGAAATAAGTCTCTCCTATCCCATGGCACCTAGTATA GCATTTAGTTGCTATACTAATTATTATAATTAGCATTTATTACGGGCTTA CTATGTGCAAAGTGCCATTATTCCATTTATTATTCACAAAAATATTTAGT [C/T] TCTACTATTCTCTAATTTATTAGATAACCTAATATAAATCTTTTTTGTT TTGATTGTTGCTAGCTGATTGAAGGAAATAGAATTGTGGTGTGGTAGTAG GGGATGTTGAGTGAGTCTTCCTCATTCTTAATATTTCCTTCCACTAGGAT AACGCCCCCTGGGCTCTCTTCATCAGGGGAGGAGGAGTGACTTCATCTGATA |
| >rs4583440 |
| CCTAATATAAATCTTTTTTGTTTTGATTGTTGCTAGCTGATTGAAGGAAA TAGAATTGTGGTGTGGTAGTAGGGGATGTTGAGTGAGTCTTCCTCATTCT TAATATTTCCTTCCACTAGGATAACGCCCCTGGGCTCTCTTCATCAGGGG AGGAGGAGTGACTTCATCTGATAGATTCGGAAGGGAAAATGTTACCCAGT [C/G] GCTTCTCAGTCCTCTTTCTCTTGGAGCGAGCCCATCTCTGGAGCACATA CACTCTCCCCTGCTCCTGTAGGTCTCGAGTTCTCAGGGGAAGGGTTTGCC CTGTAAGAGAGCTTGTCCTTCTCCAGTGAGGCCTGTCACTGGGAAGATAA |
| TTCTGTACAGTGCTGGGATTCAAATTTAGAAAGCCCACTTGCCCCATAATT |
| >rs2372941 |
| TGTGGTGTGGTAGTAGGGGATGTTGAGTGAGTCTTCCTCATTCTTAATAT TTCCTTCCACTAGGATAACGCCCCTGGGCTCTCTTCATCAGGGGAGGAGG AGTGACTTCATCTGATAGATTCGGAAGGGAAAATGTTACCCAGTCGCTTC TCAGTCCTCTTTCTCTTGGAGCGAGCCCATCTCTGGAGCACATACACTCT [C/G] CCCTGCTCCTGTAGGTCTCGAGTTCTCAGGGGAAGGGTTTGCCCTGTAA GAGAGCTTGTCCTTCTCCAGTGAGGCCTGTCACTGGGAAGATAATTCTGT ACAGTGCTGGGATTCAAATTTAGAAAGCCCACTTGCCCCATAATTAAGCC ACATTCACCAGCTTTAACAATGACAGAGGTGATATTGAGTTCTCCAGTGAT |
| >rs6721811 |
| TACCAGGTCCTTCATTATCATATTCTACACATTGAAAATGAAGGTAAGAG AGCAAGCAACTTGCCCTAGGCTACCCAGGGAATAGGGAGGAAAGTCTGGA TTTGAACCTACTGCTGCCTGACTCCGAAGCCCTGTTCTTTCTCTCACCCT GGAGGATGGATGAGGGTGGATGGGGCAGTTAAAAGTTGAGGGGTATCTC [C/T] AGGAGAAAGATGAGACACATGACCATTGGGTCTAAGGAGCCAGAGAGAG GCCATTGTGTCTGAGCCTGGACAGCCAAGCTTCCTAGAGACAGAGGATGG GATGAGATGACTTCTGGCTGACTCTGCCTCCTTGAGCTCTTTAGGATCCC CTGCAGAATCTCCCAAAATGATGCTCATTGGAAACAGAGAGAGCACATTCA |
| >rs12615418 |
| TAGAGATGGGGTTTCTCCATGTTGGTCAGCCTGTTCTCAAACTCCCGACC TCAGATGATCCACCTGCCTCAGCCTCCCAAAGTGCTGGGATTACAGGTGT GAGCCACCACACCCGGCCAATTTTTTTTTTTTTAAAATATGGCATTAAAA TGGTATTTATCTTGACTACTGAGTTTTTGGTTAACCCCTTACATTTTGTG [C/T] CCAAGACATCTCACTCATGTTATCCTAGGCCCAGCATGGCTGGCCTTGT CTCTTAAATTCAAATTTTTAAATTTTTCATTCCTCAGGCTTCTCACTTGT AAAATAGAAATAATAATAGCATCTCCCCCACAGGCCTGTGCTGAGAGTGA AATGTGTTACTACACGTGCCTCACCTTCATTTTCAACAGGACCTATTGCCA |
| >rs12621884 |
| CACACCCGGCCAATTTTTTTTTTTTTAAAATATGGCATTAAAATGGTATT TATCTTGACTACTGAGTTTTTGGTTAACCCCTTACATTTTGTGCCCAAGA CATCTCACTCATGTTATCCTAGGCCCAGCATGGCTGGCCTTGTCTCTTAA ATTCAAATTTTTAAATTTTTCATTCCTCAGGCTTCTCACTTGTAAAATAG [A/G] AATAATAATAGCATCTCCCCCACAGGCCTGTGCTGAGAGTGAAATGTGT TACTACACGTGCCTCACCTTCATTTTCAACAGGACCTATTGCCAGTAAGT GCTCGTTAATGTTAGCCATTATTCTTGGCATTGCTGTTGTTATTAGTGTC AGCCATCATTCTCTCAGCCAAGAGATCCTTCCAGAAGAGAAGTTCTCCATG |
| >rs2372943 |
| CTTGAGTCTCAATTTCTTCAAGTTGGGAACATTACATGACAGGACAGGAC TGTGAGGATTAAATGTAAACAACATTTTTTTTTTTCTATAGAACATACT GTGGGGCCAGCACCATGCAAGGTGCTTTTATCTACAATTTATTTCTTTCC CTTCCCATGCCTTCTGCCTCTTTGAATAACTGCTGCAATGGCTGTAGAGA [A/G] ATAAATGCAGTAGACCAAGACTTTTAGTGATTTTTAAACAATGTTCTAG GGCAAATACATGGGTATGTTTACATACTCCACAAAGTGAAAAAATTATGC TAGTCAGTGTGCAAAGTGTGTTTTTCCAGACCTGCAAAAACTGAGATCAA CTTCCATATTTCCAACTTCCATATTCCCTTCATCCCTTAAGCTACCAGGCA |
| ATTTTTAAACAATGTTCTAGGGCAAATACATGGGTATGTTTACATACTCC ACAAAGTGAAAAAATTATGCTAGTCAGTGTGCAAAGTGTGTTTTTCCAGA CCTGCAAAAACTGAGATCAACTTCCATATTTCCAACTTCCATATTCCCTT CATCCCTTAAGCTACCAGGCAGAGGATGGAGATAAAATTTGGCCATAAA [C/T] TTGTCATGGGTCCATCTTGGTCCTAATTAGAATTATCTACTTCCTAATC CATTAACTGCATGGATAGATAGTCTGAGGGCCTCAAGGGCAAGAGATGAC TTCTACCCCTCCACCCATCCAGCCCTGACCCACCCTTCTCATATACATTG GAGAAAGCTCTGCTCTGTGTAAAACACATCAAGTGCAGGACATGGTTCTTT >rs4522583 |
| >rs2888451 |
| GTAGAGTTTGGGAAATGAGTAACATGTAGATCTGTGAATAGATATGTCTG AGGAAGCACCAGGCAGCGGAAATAGCTGGTTGCAGGAGCAGTTTGTCCTG CCCACCTGAATCTTCAGGTTCCTTCAGCTAAGCCTTAGGAGAGGAGGCTG GAGAGCTGCTTTGCTGGATCATCTCCTTTGGCCCCCTCAGACTAGAACT [C/G] CACCTCCTCTCTACCTAGCTCTGTGCTCCACAGGCTGCCTCTGGGCTGC ACTGATGGGCTCCCTTGACTGCTGGCTCCTGTTCAGGGTTGGAGGAGGAG GATGAAGTCATGGAATAGTGTCCCCATGCTGGGCTGCAGGTACAGTTGCT GCTTCTCTACTGAAGACCCCTGACAGCAACCCTGTCTTACAGCCAGGGTGT |
| >rs2270398 |
| TCAGAGCAGGAGACATCATTTCCCTTAATTCTATTCTGCCTTGTAACCC CTTTCTTAGACTCTCTGCTGAGTCTCTGCTTATGTGTTGGGATTGCATTG ATGCAGATGCTTTAGGGCCACGTTGTGGAGGATACAAGATGATGAGACAG GAGGCAGCGTATGGTGAATCACTTCTCACAGCTCCTAACTAAGTTCTGGG [C/T] ACATGAATGGGTACTCAGCACATACCTACTGACCAAGCCTATGGAGAGC TGAAAACTCATAGGCTGACTTTGAAGCGAGGCCTTTGAAACCATCGTCCC AGGGGAAATAATCTAGGTCAAAGAGACGGCTGTGGCAAGGGTGCGAGCTG GCAAGCATGAGGGCTGACAAAGCAGGCAGCACTCCTCCTGAGCAGATGACC |
| >rs13412666 |
| TATTCCCAGGCTGGGCTGGAGCACCTGAGGCTACTATGGTTACAGAAAGG AACCTGGCATCCGAAGGTATAGGCTCAAATCCCAGCTCTCATGATTGCTA GCTTTGAGGTTTCAGGTTCATTTCTTAAGTGCTCTGAGCTTTGGTGCCCT CCTCTTCAAAATGGGAATGAGGAGCATGTCTAGCTCCCTGTTTTGTTGCA [A/G] TGAATAGCTACATGAAGAACAGCTAAACCAGAACAGAAAGAAGGCAAAT GGAAGCTACAGAAACCAAGGATTTCCTTGTTGAATCGAATCTTCCTTCAA TCTTCCTTCACCACACTAGTGGATCTCCCTGTGGGAGGGATGTTGAGAGT GCTCCGTGTTTTTTTTTGTTTTTTTTTTTTTTTTTTTTTTGTGATGGAGTCTCA |
| >rs13426489 |
| CAAGTGATTCTCCTGCCTCAGCTTCCCAAGTAGCCAGGACTACAGGCATA TGCCATCACACCCAGCTAATTTTTGTATTTTTAGTAGAGACGGGGTTTCA CCATGTTGGCCAGGATGGTCTCGATTTCTTGACCTTGTGATCTGCGCACC TCTGCCTCCCAAAGTGCTGGGATTACAGGTGTTAGCCACCACACCAGCCT [G/T] CTCTGTGTGTTTTTAATCAATAGCAATATGTACCATATTTAGTAGGGAC TATAAAGAAATTGTGCTAGAAACACCTGGTTATTTTACAGAACTTTGCCA GTGAGTACAAAAATATGCGTAGCCCACAACCTCTCTCCCCTAGAAAATAC CTCCTGTGGGTAGAATGAAATGGGTAGGTGTTCTTACCATTCATTAGCATT |
| >rs6713249 |
| ATTAAACCCCTTCCTTATACATTACCCAGTCTCAGCTAGTTCTTTATAGC |
| AGTGTGAAAACAGACTAACACACTCTTAAAAGTAAATGTTATTACCCACC TTTTACAGATTAAGAAACTTAATATTACATCAAGGAATTAGTTTATAGCA CTGGATGATCTGGCCTCAAATCTCCAGTGTGATCAGTATCAAGCTTTTCT [C/G] ACTGTATCAACAGAGTTTTCCTAACAAATCATAAGACACAAGTAGTTTC TCCAACTTAATCTATTATACCCATAATTCACTTGCCTCCAGGGAAAAAA TTAGATGAAACAGACTGTAGTGGCTTTGTGTAAAACAGCAGCTCCATTCC TTTAGCCCTATTCTAAAACAATAGACCATGTGCTGATAGCTAGCTAACCTC |
| >rs12620095 |
| GCATTTGTTCCAAACACATTTTACATGGATTATCCCATTTAATCTTTACA CTAAAGTATAATACTCTTATTACCCCATTTTAAAGAAGAGAAAACTGAGG CACAGTGAGATTAAGGAATTCATCCAGTTTCTGCTGCAAGTAGGGTGGCT AGCATTGTAATCTGGGCATTTGCCATGAAGCTGTGCCCTTAACCACTCCA [C/G] TATGTGCTATAAGGTGGAGCTGACTTCATGGGGGCTAATTCAACCAGGG GAGTGTGCTCATATTAACAGATCTGTGAACTCCCTGGTTTGTTTGTGAGA CAATTATCTATTTGCTTACCTGAATCACAGCAATAATAGTTATACCTCCC ACTTACTGCATGCCTACTTGGTAGCATGTACCTGGACAGAAGGTTTTCATT |
| >rs4621152 |
| GGCGTGAGCCACCCCGGCTGGTCGCATTCCTCTATTTAGTGTTAATACCC TAAGACTCCCCAGCTGACCACCAAATGTAGGCAGGTGGAGGGACTGACCT CCAGGGGGCACATGTCTCAGGTTGACAAACACATTTTCTCTTGATCCTTT TTTTCAGTATGATGAAGTATTCTACAAGACTCTCATTGAACCTTCTGATA [C/T] CACATTCTAACCATTTATATAAAAGTCAAAAGCTCCTTGTCTTCAGGAT TCCAAAGTTCTTAGCACAAAGGTCCAGGAAGTGTCTGAATTCTTTGGTGT CTGGTTCAGTATGGATGTTCGGGTCCCTCTTAGACTTGAGATAGAACAGG AAAGAGATGAATAGACAATGACAGAGGTAGAGAAAGTACCAACCCTTCCCA |
| >rs12622764 |
| CCTCTCATCTCCTGAATGGCTGACCAGTGCTTGCTTCAAGTTAGATTAGA GTTTGAGCTTTGTAACATAAGTCCCCTTCTGTCTGATCAATGAAATGAGG GATAGGTACTTGCCCGAGAGTTATGGGACCTGGCATTTAACGTGAGAACA TGCATCTTCAGAAGCACTGGTGAGACTTCAGAATTCCTGAAAGCACTATT [C/G] TTGCAAGGCTTGAATGTACCTTCTCCACTCCACCATGTCCTCTCCCAAC CCTACTAGCATGATATCAAGCTTGGGTGGAGAGCTCGCTTGCAACTTTGT CAGATACCCATGAGCCACTCTTTGAATTTCTGACAATGAACAAGTATGTG GGAGGCCAACCCCATCCAACCTGACCCATCTGAACCCAATCACATGTTTTC |
| >rs6723013 |
| AGAAGAGTCCTTTGTGACTCTGATTGGCTTTGTGACTTCCTTGGGGCTGG TGTTGGTCTCAGGCTTGTGTCTCGCAGTCATCCAAAATGTGGGTGGGCCA TGTCCCACCTTAACTGTGGTTGTGAACGCAGAGGATTCACTCAACTTTCA GCATGCAACAATAATCAAATGCAGTTTTATGGTTCACAGAGGGCTCCACC [G/T] TCATGATTTCATTTGATCCTCCTAACAACCCTGGGAGCTCAGAGAGACT AAGGAACTTGTCTCATATCACATACCCAGGAGTTGACTGCACGGCATCTG ATGCCAGCTTCTGTACCACGTGTCCTCCTGGTGATACTAGAGTAAATAAA ACATAACTAGACAAGTAAATATTTTGTACAAAGTAATCTGCTAAAAATGT |
| >rs2372945 |
| GAGCATTTTCTCTTGGTAAGTTCTCATTTTCCCTTAATCATTAGAAGACA CGGCACAGTAAACGCGAGCTCATGAGCAGAAGGCTACATAGTTGTATACA TGTGTGTGCTTTTTTTCACACCAGAGTTTGATGAAGCAAAATACCCAAAG |
| GTCTTTTAAAAGTAAAGAGGTTTTAAGAGTAAAGAAATTTAAGAAAAAAA [A/C] CAAAAAGAAGTAAAGATGGTTAGGTCTCCTGTTTGCTCTCCTTTTGCAA TCAGTCTGTCTCGCTCTGTGAGCTGACTATGCTGCAGACTTTTCTTTTAC CTCCAGCCTTTCGCTTGTAGGGTTTGAGGCTAGGGGCGGGGAGAGAGAGT TCAGCCCAGCAGGCAGCAAACCCTATGTGCAAACACGATGTGTGAACATT |
| >rs12694403 |
| TCACTCTCTACCCCTGCTGCCCTCCCAATGCTCAGGTGGGCCCTAGATTC ATCCAGAAGCCAGGGTGTTGTGCTTCATGAAAGGGCCAATACCCCTAATG TGGGACCTATACAATAAAAGGCAAGGGCAGAAAGAGAGAAGAAATAAAAG GAAAAGGGGTGTCTTGAGGGGAGTACATGATAGACTCCTGTGCCTGGAAG [A/G] GACTAGGAAACTTATACTGACTGGTTCACTGCATTTGCTCAAAGAGTAT TCATCTTCTTGAGTTTGTCAGGTGACTGTAAGAGCTGGGTTCAAGTTCTT TGGTTCACTATTATATGCCTAGCTGAGCACAATGCCTGGGACTTAGAGGG AAGGTCTGGGACTTCACCAGGATTCCCAAAATAGAACAAAGCATTTGTTA |
| >rs12613955 |
| TGAAATATGCCAGCCAGCTTAAATTAGCAAAGCTGGAACTGTGGCAGAAT GAATAGCCCGTGTCTGAGTGAGAGGGAAGGAAAGGGTCTACTGTGTGATA GGCAATGGTTCCAAAGTAGGAGTAGGGGCCTAGTGTGGTCATGCCTTCTG ATATGTCAAGAAAAGGCAAAAAGTTGATATTTATTTGAAATATTCTGATT [G/T] TATAGGTTGGTGATCAATTAAAAAACTAACAAATACAGTTAACAAACAT GTTTTAATGCTTGCAGACAGGATCCTTCCTGCCGATGTTTGGCCTTTGTC TTTAATGGAATTTGGGCTGCCCCATAACAGCCCACCCTAAGCAAAACTTC TTTTCTGCCTTTCTTTTATATTGTTTTTTATGGAATGTAGAGGGATTTTT |
| >rs11894340 |
| CTGCTTCTATGCTGTATTCTAGTCATTAAAGGAGACCATCTCACACTCAA GGGGAGAGACTACACAAGCCATAGTGCTGCCATGGCAACACAACCATTAG GATGATGCCTTGAGCCTTCCTTCTTCATATTTCTTAGACTTCCTTTCTGA CCTAGAGGAGCCAGAAGGACTTGGGACTTGTCATACCGTCCAATCCCAAG [A/G] AATGACAGGAATGAAGGAATTTTTAAAAATTCAACTCATTTCAACACAC ACACAAAAAATGAGAGAAGTGAGTCAATTTGAGAACTGAAATTCTGGTAG ACTTTTGGGGAGACACTAGAAGGGCCATGTTCATGAGTAGGAACCATAGA TTCTGAGTCAATTGGGCATGAAAGTTTTTCTCCTGGTGTCCCGATCTTGG |
| >rs17778329 |
| ATTGTGTTGGGGGAAGGGAGGCTTCTGAGACAGAGCTGAGAGTCCCCAGC AATGCCATCCCTGGCTTCTGGCCCATCTGAATGCCTTACTCTTTCATGTA AGCAAGACATGAGGTCCTGCTGTTTGAGTTGGCTTTGCCCTGCTCCGTGA TTTCAGTTCCTTGTCTCTCCAGCGTGTGGGCTCATTTCCGTCCTCTGAGC [A/G] CTATTGTTTTTCTGACTTGAACAGTCATCCAGAAAACCTCAGTAATAAG GCTTTTGTATCATTATTTACTTTGCTAATTATATATGTCTTTCCCATCCC CAGTGCTTGAAGACATCAAATTGCAGTTGAAGTAACGGCAGAGGAAAAG CAAGGGCAACAACAACAAACCACTTCACTCCTCTGGGTGGAAACATGCAA |
| >rs4442975 |
| AATTTGGTGATGGGAGGTGTAGAGGGTAAACTCTTGATGATATCATTTG TGTACCTGGGTCCAGCCATGCCTGAACTTTTCAATTACTTGACCCAGTAA ATTTCCTTATTCATTTAAATGTTAGAGTTGAGTTTTGTCTCTTATAACTG AAAGAGTGACATGTGTTTATATTAAGTGCAGGTCACTAATATAGTCTATT [G/T] |
| GTTTCCCCAGGCTTGTTCTCCTTTTCTCAAATAGGACTGATCAGATAGT AAGTGCTCCATACCTGTAGGATACATAAAAGAGCAAATGAACAAGGAGCA GGAGATATGGTGGCTGACGGTTGGACTATAATTTTTTCTTGATTTTTCTG ACTTTATTTTGGCTGTGATCTAGGAAGTGAAATCCAGGTCCTTCTAAACC |
| >rs10932693 |
| TTTATTTTGGCTGTGATCTAGGAAGTGAAATCCAGGTCCTTCTAAACCCC AATTTAACTGTGGCTCCTGAGGGAGATTTTCAGATCTCAAATGATCTGTA AGTTTAAGGGAGGATGATTGGTCCCTGTACACTCTCTGTGAGAGTTCCTT TGGAATACTGGTTTCAGAATGTACAGCCACACAGGTAACTGGATTAGGGA [C/G] GGCCAGTGGTTTTTTATGAATGCTATTGAAGACACTGAATGGAAGCAAT TCTCACCAGAACTCTTTTTGCTGTCATCTTTCAGGGGCTAAGTTGACACT AGAACCAATCAATGATCCCTGAACTGGGAGAAATGGGTGGCCTTGGCTGT GATTTTTGGTTGTGCTGCACATTTCAAGATGACAATATTGTTTTAAATTA |
| >rs17778427 |
| TCATCCTGTTATCCATAATGAAGGAAGATTTAGGATATTCCAGCTTAGA TCCAGGTGTCCCCAGCTTCTATACTCACTCTGAAGAAGTGTACTTTATCT ACTCTTTTTACACCATTTATCCCATTTTGGCCATTTCTGAAAGGAAGATC CTAGAAGCCAGGCCTCTTGGAAGAAGTTGGAGCTACAGCATTGATAGGAA [A/T] ACCTGACACCTAGAAAGGCAAGTCAGTTCTTCCCATGCAGTGGCTTTGG GGACTGTAATTTAACAAAGCTCTTTTTACTTGTGTAAAGTGATCTCCAT TCCACTTTTTCTTGCACTGATTCTAATTAAGTGGAAGCAATTGTTATCA TCATTTAGGATTATAGTTAAAATACCCCTCAAATTGTTTCCATTTGGAAT |
| >rs17835044 |
| AAATATTTCTCTTGGTCACTTGAGTCTAGCATCTAAGTCATATAATGCTA TTATAGCCTTGTTTTGCTTTTGATTTAGGTCATATCTTCTTTTTCTGATC TTTCTTAAAGTGAAGGGACCATTTGCTATGCTCACAAAAATTCTCAAGGC TGGAACTACAGCATGCTCTGGTTTCTAAGCCATACACATCCAGGTAGAAG [G/T] GTCAATTTAATTGCCAATGATCTCAGCCTTTTCTAACACTGTTCCTTAA GTTTCTTTGGCACTGCATGGCAAGCAACTGAGTCATGCATGAAAATTTCT CTTCTAGACAACCCATACCTACAGAGGGAGGATTTGGTTTACTGCAGATG CTGAGTGCTTCTGATGGCACACATTCCTTCTTATTAATATCCCCCTGGCC |
| >rs7562029 |
| GAACAGTCAGCATTGTCTCCGCTTCCGCTCCACCCTGCAGTTCTGAGCTG AACAGATAATTTATCTGTGGCACAACAAAGTATATGACTTGGAAGAATGT TAAAGATCAAGTCCACCTTCTCCTTGTATTATATGGCTCTGAGAGCCAGA GAGGGGAGGTGACCTTCCCAGAATGTGAATGTGGTAGAGCCTAGCTTACA [A/G] TTCATGTCCAACTTAGGTGTGAAGTTCTATGAGACTATGCTGCCCTCTG CCTCACAATGCTTTCTCCCATGGGAGAGGAGGCTCAGAGGTACAAAGCAG CTGCAGAAAGACTCTCTCAAGCTATTGACTCTTGGATGACAACAATTAAT GTTCAACTTGGCTTTACCCCCAACATGCACTGCACAGATGCCTGCAGGTC |
| >rs13000023 |
| TGGCTCTGAGAGCCAGAGAGGGGAGGTGACCTTCCCAGAATGTGAATGTG GTAGAGCCTAGCTTACAATTCATGTCCAACTTAGGTGTGAAGTTCTATGA GACTATGCTGCCCTCTGCCTCACAATGCTTTCTCCCATGGGAGAGGAGGC TCAGAGGTACAAAGCAGCTGCAGAAAGACTCTCTCAAGCTATTGACTCTT [A/G] GATGACAACAATTAATGTTCAACTTGGCTTTACCCCCAACATGCACTGC ACAGATGCCTGCAGGTCTACAATGCCGAGACCAAGTTTGGGGGGCATTTC |
| CGCAGCTCTGTCTATTGTCCTCCTTTCCTCTTCTGTGGTCATTCTTCCCA AGGGATAGGCCTAGTCCAGGGTGGAGAAAAACAATTCTTGGTGGTAGGGG |
| >rs735361 |
| GGTTTATAAGGCAACTCTTAGCTTGACCCACTCTACTCCTCCATCAAAT AAAGAAATAAATAAAGTTTTCAAACTAAAGAGCATTAAAGAATGATAACT TTTCTTTTTCTTGGTACTATCTATTGACCTATACCTGGTTTTCACTGTAA CACATTTGTGGTATGAAAAATGACAATATGATGTCAGAGAAAATGAGAGA [C/G] ACATTTTAGAATTTATCAGAATGTGACTATGGTCACTTAAAGTCCAACT TTCTCATGTGGGTTTCAGCCTGTGCTGGAAACAAATAGCCTCTCTTACCT CTAAAGTACTTGGTTCTGTGCTCAAAGTGGCATATTAAGTTCTGCTGATT ACATCAGAATTTGGATGGTGTCTCAGGATCTCAGTTTCCTATCAGTGGAA |
| >rs13409592 |
| TTAGAATAAGACTTCCCTAAGCAAATCAATGTGATGATTGATACAAACAG CCATCACTGGGGCCTCCTCTATTTCCAATCTCTTCCTCAGTTTCCCAGTT CTTTCAGAACCTCACTGGGAAGTTCAGGGATATATGCCTGCAGGCTGGCC TGGCGCAGATGGATGATGAAGGGTCCTGCCTTGAGTGTAGGGTAGAGTGA [C/T] TCTGAGTGCTTTCCAACAGACTGTAAATTAAAGACACTCCAAAAAGCCC CTGGTGGGGATAGATGGGGAGAAGGGGTATCACTAAGTCTTCATCTTTTC TTCTAGGGCCAGGGTCAGTGGTTCAAATAAACATGGTGCCCCACAGAGAT GGCCTGTGGCCAAGTAGCTCTCAGTGGGTTGACTCAAAGTCACCTGGACA |
| >rs2287289 |
| GATCCGGGATCTGGTGATCCTCGGCAGGACAACTCACCATTCTGTTCGGT TGCCCGCTTGCCTCCTCCCGCTGGCTCCAGCTTCTGTGCCTACAATGAAG GCTGTGCCCATTCCCTCACGTGCCCGCCGTGGCCTGACACCTTCCTGGGC CCACCCTAACACATTCCTCCGTTGCTTTTAACGACTGTTTACAGCTCGCT [A/T] CCTCTAGGACGTGGCTTCTGATGAAAACTGTCCAGACGCTCTGCCTCCG GTGTAATTCAGAGCCCAGCTCTCCTCCTTAAAATTCTTTTACGCTATTTT CCACAAGCATGTGCTTCTGTTGGCAGTGCCATAAGTGTGATTGATCTTTA CCATAAGTTCCTTGAACTTTGCTGTGACCTGGATGCTAAGGTGGCGGGAG |
| >rs12329133 |
| AAAGTCCACGGAGACACAGCTTCAGCCACCACCGGGGCTGCGACCTGTC CTCAGGATCCTCCTCAGCCCCCTCCAGGAGTCCTCAGCTTTCTGCTTTTA GCCCCAAACCCAGATTCACTTTCCCAGGCCATTCACCGTCTGAGGCAGCC CAGTCCGCTTTGTCCTTCCTCCTTCCCGAAGGCTCTCAGCCCCTGGGCAG [C/T] ATCAGAATTTCACTTTGATCTCCTTTTCCCTGGGAGCCAGAATAGCTTA GTCTCTTCAAAAGGAGAGTTAGAAAGTGTGCCGTTCATCTAATAAAAATT AACTCCTGAGAGTTATCCAATCCCAATACAGGTCATTTCGCTGGAGGCTA CCCCGACTAGGAAACACATTTTAGGACATCAGCAGAGGTAAAGATGAGAG |
| >rs13011060 |
| TAAATTTCAGCACATGCTATATAGTTTTCAGGATTAGTCCCTCTCTAGGT ATTCATCTGGGGCTGGCGCCTCCTTGGCTGGCAGGGGCTGGTGATCGGGA CTCCCCAGCGAGACAGATTGCACCCAGAGCAGTGAGGCCCCGAGCGCTTC CTGGAGCCTGGGGAAGATGGCATTCATTTTTAAAGTCCATTTATTGTGCA [A/G] TTGTTGAAAGTGGGACACAGAAATTTGGACTGATATGCCTAGGCCACAT GACTACTGATTGGGCCAGGGTAAATGCAGTCTTTTTGTTGATGGAGAAGG TCTTTTCTGGGCCCAGCACAAAGCAATGGATCTTCAGTCTTAAGAAATGC ATTGAAAACCATCCTTGTGCAGTTTGGGGTCCCTGGTAGGCCAAGAGCTG |
| >rs13011326 |
| ATATGCCTAGGCCACATGACTACTGATTGGGCCAGGGTAAATGCAGTCTT TTTGTTGATGGAGAAGGTCTTTTCTGGGCCCAGCACAAAGCAATGGATCT TCAGTCTTAAGAAATGCATTGAAAACCATCCTTGTGCAGTTTGGGGTCCC TGGTAGGCCAAGAGCTGGAAAGGCCCTTAGACTCTGAGTGCTGTAGCCCC [C/T] CTTCTAGGTGATGGTCAAAACTTGAAAACGGCTCTAGACTCTGGACAGA AATGCATGAGAATTGAGTGTTCAAAGCTTGGTGCTTTGACTTCTTTTAAC TTTCTGTAGACTGAAATATGAATAAGAGGAAGGCATGAGGCCCCTCTCAG TTTATGGAGCCATAGCTCCAAACCTCCCACTTTCCAACCCAGCAACCCTG |
| >rs4674132 |
| AGAAATGCATTGAAAACCATCCTTGTGCAGTTTGGGGTCCCTGGTAGGCC AAGAGCTGGAAAGGCCCTTAGACTCTGAGTGCTGTAGCCCCCCTTCTAGG TGATGGTCAAAACTTGAAAACGGCTCTAGACTCTGGACAGAAATGCATGA GAATTGAGTGTTCAAAGCTTGGTGCTTTGACTTCTTTTAACTTTCTGTAG [A/G] CTGAAATATGAATAAGAGGAAGGCATGAGGCCCCTCTCAGTTTATGGAG CCATAGCTCCAAACCTCCCACTTTCCAACCCAGCAACCCTGTGTGTCCTA GGAATTGTAGCGGGTGTCCCAGAATGCAAAAATCACTCTGCTAGAACTGG AAAAGGAGACGCCTGCCTTTTCTAGCACGATCTTTAGTAAATAGATTCAG |

**Table 28. Flanking sequences for rs3803662 and correlated HapMap SNP markers with r² > 0.2**

| |
|---|
| >rs3803662 |
| TTTTATTGTTCTATGGTTATTAAAAAATAACATGTCATATAAATTAAGCC TAGAAACATGGATGTTTATCAAAGAAAGGATTGTCATCCAAAGCACCAAC TATGAGAGATATCTATGTGCAATGGTATATAGATCTGTCATAGAAGGGTT TAATTATATCTGCCTAATGATTTTCTCTCCTTAATGCCTCTATAGCTGTC [C/T] CTTAGCGAAGAATAAAACTGTGGACTGACCCCCACCCATTTGCGAAGAA AGTACTGGGTCTTCAGCTTTCATTGTTCAGCCGGTGGTCTTTGTGGACAA CACCAGGGGCCCCTCCTGCTGAAGACTGTCTGGACTTCCTGATTTTCACT CCTGTATAAACCCCCTCCCCCATTACTACCTTTGTCTCCTTGGGGAAAAA |
| >rs4784220 |
| AGAGAATTACTAATATTTTCTTCTCCAACCTTAATGCTCACTCCCTGAGC TATGTGGATTATACTAGTGAAAGTACTGCCTTAACCCACTAATGTAAACA GATTCCTTTTAAGAAGCCTCCCTATCGAGAATGGTCTGTAAGAGACAACC ATGTCAACAAGTAAAGTTCCTTCTGCCTTCCCTCCTGGGGTGGGCTAAGA [C/T] GGACCCTTCTCACCATTCCCAATTACCTGCAGGAAGGCAGCAACAGAAT AGAGAAGGAGGGTAACACAGTTTCCTGTACTTTAAATACCTCCCTGCCTC CGCAAGTAAAACTTGAGCCATGCCAATGAGGGAAAAAGTGGCCGCGATTT CCCAGGTGGCTTAGTAATACACAGTTTTCTGACACACTGCAACTCCATTC |
| >rs12598982 |
| CACTACCAAATAATATTGCTTCTTGCATTAAGTATAGCATTGGTTTGCTA GAATAATCCTAACAATGACAATGCAATGTACTTTAAATTGGGGTCAAAAA ACTTTTTTCTTAAAGTACCAGATAGTCAAGCTTTTAAGCTCTATGGGTCA TCCACAGACACACTGCAACTATACAAACTATATCACCCCTGTTGCAACTA [C/T] |
| ACGAAAAGCAGGAAAGCAACCACAGAAGACAGACAATACATAAACAAAT GAGTATGACTGTGTTCCAATAACATTTTATTAATAAAAACAGGCAACTGG CCCATGGGTGGTAATTTGCTGAAACTTGGTTTAAATTATTATTAGTATCT ACCTTTTTTTAAAAGATGATGTTTTTTAGGGTCTTTCCATCATGAAAATT |
| >rs4784222 |
| TAATGATACTATAAAAACACCCAGCATAACGCCAGCATAAGGATGTGAAT GAATGAGCAAATGAAAGGAGGGAGGGCAATGGCAAAATTTTACCTTGGTT TCCAAGCTAAAGTATAACCTGAGTTTTTTAATGCTTTACAAAAAGGAAAG GCAATCAGAACCATAATTAATATTTAAGCTGTACTGACAGGTTTTCCAGG [C/G] AAAATTCTATAGAACTTGTTATTCTCCAACTTCCAAAAATAAGCAATAA ATTTTTTTTAAAGAGCTGTTATTTTAAGACTTTATTTGAACCAAAGAATG TGTTTATGGTAACAACATACATGTTGTAAATTAATCAGTTTCAATGTTAT AAAATAATACTTCCAAGAATAAATGTATCATATAATCTATGTACCTCATG |
| >rs17271951 |
| TCATTGTTGTAAAATCCAATTGTCATACTCTGGTATTTGTTCTCCAAAA GAATCATCACTTTCATATTCACTACAGCATGAGATGAGATATTTAATAAA TAATAGGACATCATAATCCAGGGATTAAGTAGTTTCTTTTAGCAAAATTT AATATGAGGGTAGCATATGCCAGCCGTTCCTAGTTGAGCAACAATTTCTG [C/T] GTTGGGAGAGGATAACAAAGCCAAGTATTTTCCCATAAGAAGTTCACAC TCTAAAAGGCCAGGCGCGGTGGCTCACACCTATAATCCCAGCACTTTGGG TGGCTGAGGCAGGCGGATCACTAGGTCAGGAGATCGAGACCATCCTGGCT AAGACGGTGAAACCCCGTCTCTACTAAAAATACAAAAAAATTAGCCAGGC |
| >rs9933638 |
| GGAATAATTACAGATGATGCTAGGCAATATCACTAGATTGGGTTTTCATG AAGCTCGTGACTGGATTTTGCCACAAAAATCACTAACTGCTTTGGCTTCC AGAGAATGGCCTCATGGATAAGCCCCAAGTTCCAAAAACTACAAAATGTC AAAATCACATTCAACTAATTAATAATTTTATGTCACAATAAGAATTAGGA [A/G] TCTCTAGAAGGCTGGGTACAGTAGCTCATGTCTGTGATCCCAGCACTTT GGGAGATCGAGGCAGGCAGATCACTTAAGCCCAGCAGTTCAAGACCAGCC TGGGCAACATAGGGAGACCCCGTCCTACAAAAAATATAAAAATCAGCTGG GCATGGTGGCACATGCCTGTAGTCCCAGCTACCCAGGAGGCTGAGGTGGG |
| >rs9302556 |
| AACACATAAGCCATTTTTTCCAAAGCTAGACAGCTTTTCTCTATCCACGT TCTTTCAAATATTTTACCTAAGACAGAGAATCTCATATTAAATTCAATTT CACACTTGCCAATCACACACGCCCACTCACAATTCCTCCAGATGATGCAA TGTGGCCTAATGTGTATGCTTATGTATGTACATGTGCACACACAAACAGG [A/C] ATAATGTTTAAATACATATAAACACATTCATTGCTAGGTATTGGTTAAA ACAAACAGGCTTACAGCATACCAACTTATTAAACATAATGATTGATATTA GTAGTTTCATTAATGTAATTAACCTTTAGATTTGAATTATCAAGTTCAAA GTCTAAGACCGAGAGCAGTGGTTCTCAGGCCGGTACAATAATGGCAAGTC |
| >rs7190749 |
| CAGTCCTGCCCCTACCAGAAACCAGAGATATTTGCTGCTGGTAATAATGA GGAGGGGGTTTCTAAGTACAAGATCAGCTCTATTCTTTCATTCCCTGGAA ATCTGCCCTCGCCTCTGATACCTCAAAAAATATCTGTTGCATTTCCAAGG GAAAGAACAATGATATAAGCCTTGCTGTTAAAAAAAAAAAAAAAAATGCC [A/G] CTACTTACAAGATAAAAGGCAAGGTGTTTTGTTTTTTCCCCTTTCTCT TAGACTAGGCATGAATAATCTCAAAATATTTGTTTGTCAATAAAGTGATT |
| AAAATAAAGTGATAGACAAGACATAATGATTAGAGAAATGAATCAAAGTA ATATTTGGGACCAGGGTCTTACAAATCTAGAGTAAGTATGTTGAGCAGAA |
| >rs12443621 |
| AGATCAAGATGTCTAGCATAATTTGAAAGTATGTTTCTATTTGACAGAAA CCTTGGCTTGGAAAAGTTAAACTCTAAATATTTGTACAGAAAGAAGTATT CTGGGTTTCATTCATTAAAAGTCATTGACCACTGCAGAAAAGGGAGAGCA AGAGATTTACATATATTTTTCTTGACGTTTTATATGCATTAGGCCTGGCA [A/G] TGAACTTGAGGTAGGTATTACTATCTCCTTATTTCTAAGGGAATCAGAA TACTGTAAACAAAGACATGGAGCTAAATAATGGAGTCAAATCCTGGCTTA CTAGGCTCCACATCCATGTTGCTCCACTTATCAGACGGCCACCCAAACAT ACAAGTTTAACAAATTCCAAATTATTGGGGCCTAAATAGACACTTTTTTG |
| >rs9933556 |
| CATGGAGCTAAATAATGGAGTCAAATCCTGGCTTACTAGGCTCCACATCC ATGTTGCTCCACTTATCAGACGGCCACCCAAACATACAAGTTTAACAAAT TCCAAATTATTGGGGCCTAAATAGACACTTTTTTGTTTCAATATTGCCCT TCTTCCCTTGTATTCTGAATATCATGTCAACCATGAATCATAAGCCAATG [C/T] TTACAAATTTAACTTCACATTATGAGTTTTGAGCCCAACTGATGCCTAA CCACTAAACCATTCATATAACACACTGAAAAGAAGCCATTTAAGAAATGA GAAAAATAGGTATTGTTTTCTCTGTATGGCTGTAAAAGCTGAGATAAGAA CATTTTGGCAAGTTCAATAGAGCCGAAAGGCTTGTGAAGGCTATGGGAGG |
| >rs1362546 |
| TCCACTATTTGCACTAGCATTAATCAACATCCAAAAAGTGTACAAATTAT TTTATCTTTATAATCAATCTTCTGAAATGCTAACTTGAAAATATAAACAG CACAATTACTTTTTTCTCTTTTCTTTCCCATTTGAAATGTGTGCTTGTGT GTGTGTGTGTGTGTGTATCTGAATCTGTACATATAAATCAAATACACACA [C/T] ATATACTTGTTTTAAAAAAATCCTCAAAGAATCAATCACATATTACAGA ACTGAAGGAATATTGGAGTTCATCTGGTCCAAAGTGTTGGTATTGTAAAA TCACTTTCAAATTTCACAGTTATCCAAATGGTAATGAATATTAATACCTA AGAAAATCAATCAAAGATTAACATTAAGACATTAGATTTTAAAAATCATT |
| >rs1075367 |
| TTTTTAGCTACAAGGATATGTTTTTAAACCATATTGAATTACATAGAAAG CATTTCCATTTTCCTTTCAGGCTTTCTTATTACATCAGTGAGAAAATCTC AGTTTGGTGCGAATATGTCTTTAACACCTAATACTTGCTAATCTCTTTTT TAGCAGAGAGATTGAGACACATTATAGGGGAAGAGAGAGAGGGAGAGA [C/T] GGAGGAGATCATACAGGACCCAGTTACAACCCTTCTGTAGCTTTGGTTT GCTAGAATTAAATAGATTATTTTGTATTTATTGTATTTATTTTGATAGTT ATGTGCTATTTATAGAAAGGAATACCAGTTTTCCATGTATGAAATAAAGT GTCCTTTTGTTAACAAAAAAGCATATTAATTTTTATGCAAATGCATGAAT |
| >rs8046979 |
| CAGCTTCAGTGGATGCAAGTATGATCTCTCCGATGATCCTGGGTCCCTTC AGAGGCCTTTGGATGGTTCTGAGCAGTAGAACTCAAACTGTGATCCCAGG AAGGCAGCATCAGCATCACCTAGCAACTTGTTAGAAATGCAAATTCTTGG GCCCTACCCCAGACCTACTGAATCAGAAACTCTGGGTGTGGAGCGGCAAC [A/G] TGGATTCTAACAAGGCGTTCAGGTGACTCTCATGCTCAGTGAAGTTTGA AAAGCCTGTGGGTAACGAGCACAGTCTCTGGAACCTGCCTGTTGATTTGA AGGAATCCCAGATCAATCACCTCCTCACCTGAGACTTTGAACAAGTTATT TAATTTCAGCCATGCTTCCATTTCCGTTTCTGTTAACTGGGACATTAATA |
| >rs1420529 |
| ATGAACTCTTCAGTGCCCTCCCCTTCCCACAGAGGGCTGAAAGGACTCA CTCTTGTTACAAGAACACGGCCACTCCTCCCTCTCTTCCTCTTCCCAGTC CATCCCTCACCCCAGTTTCAGCCACCATCCAAAAAGAAATCGCAGCATGG CCCTTGAAGCACATTGAGCTTAAAAGTGCCTAGAAGATAAAGCCTAAACT [G/T] CAGCACGACAGGCAAGACCTTTCAAAATCTGGCCTCGACCATCCTCTCT GGGTCTCCTTTCTGAGAACCCCTATTTTCTGCCTTTTCACAACTCCACAC TTATGCATGATGCTGTTTTCCCTCCCTGGCTCACCCACCCCAGTGTTCTT CTTCTGTGATGGTAACCTCCCCAGAAAGTCTCTTGAGCTAGAGTGATCAT |
| >rs11642645 |
| CTCGACCATCCTCTCTGGGTCTCCTTTCTGAGAACCCCTATTTCTGCCT TTTCACAACTCCACACTTATGCATGATGCTGTTTTCCCTCCCTGGCTCAC CCACCCCAGTGTTCTTCTTCTGTGATGGTAACCTCCCCAGAAAGTCTCTT GAGCTAGAGTGATCATTATTAAATTAAAAAAAAAAAAATCCTACTTTGTT [A/C] TATTCTGATTTCAAAAACCTGAAAGGCAAGATATTTGAGGGAAAAAAGA GAATATTGTTTTGTTGCTTTTTAAAAAATAAATTTGCAGGTCCTGACAAT CCCGTGCTTTGTGTGTGCTGATCACACTGCAATCTAATCAGGTACAGTAA ATAAAATGACTTGAGAACCTTTCTGACGAATGCAAAGGCTGATGATATTT |
| >rs1420533 |
| TGTGATTTTTAAAAGGTAAGTAACAGGTGAATCAAAGCAAGGAAAAAGTA AATTTGAGAAAATAATAAAGCCTAGATGGCCAGCACACAGGAATGCATAT CCTACTGCCCTAGACAGCTACTAAATGGGTTTCCTGTTTGACTCCAAGCT TCTTGGCCATCAATGCAAAGAAAGAACAAGAATGGTAAGATTAATGCCCA [A/G] TAAGATACAGCGAAGTGGATTTTTTTCAGTTCTTCTTGATATAGTAACC TAAGAAGATTCTCTGTAAATCATCATTTAAAAAGACACCATGAGGTGTAA TGAGCTATACATTGTAAATAGAACTTTTTAAAAAAAATGATGTTCACGAA ACTATTACTCATAATCCTCTCAATACAAAGTGAAGCCTTAATCAGTGTGC |
| >rs1362548 |
| TTTTAAAAAAAATGATGTTCACGAAACTATTACTCATAATCCTCTCAATA CAAAGTGAAGCCTTAATCAGTGTGCAATTCAATAAAGCAGAGAGGGCCTG TGAAGGCAACTGTGAAGTGGGGTGCCACATAATATGCCATCGTGAGCATT CAGCTCTCTCACAAGTCTGGCAGAAATCTCTTATCAAATTAGGTTATCTG [C/G] ATGAACGGAAGGAAGCAAGGCATCTTCTTCATGCCATTCTCCTTAGATA TATTATTTCTTCCAACAGATTTTATTAACACAAACTGGGCAGCAGGGACT TCAAGAAGGACAAAAATCCTGTGTCAAAACCATTACATATGTTGGCAACA. TTTATCATCAGGAAAAGCGTCCCAGGAAGTTGAATTCTAATGTATGTGTG |
| >rs2193094 |
| CTGGGCCTGGACTCAAGGGTTCTTGTGTACTTTCCTGCTGTCCGAAATAC TGCCATCATGAACAAGCCCAGGCTGGCGTGCTGGAGGTGAAAGATACATG CCAGAGAACTGAGGGACCCCGGTGGACAGCCAGCCAACCTCCTGAAGCAA ACCCTCCCTGCCAATCTGCAGTTGACTGCAAACACATAAGAGACCCAGAA [G/T] AGGATAAGCACTGCCCTTCTGAGCCCAACCTAAATTGCTGACATGCAGA ATCAGAAACAAAATATGTGTTTTTGAAGTCACAAAGTTTAGAGGCGGTTT ATCACTCGCGATAGATAACTGGTACAGACAACACGGGTCTGCCTAGAAAG TGAAAAACATACGAAGGCAAAACAGTAAGAGGTCAAGCTTTAGGAGGATA |
| >rs4783780 |
| TCTCATATCCATAGAATATCCAGGTGACCTATAGGTTTCTTGAAGGCAGA CCACATCCAATCTCAGCCTCAACAAGAAGGGCGTTCCTAATATAAACAAT ATTTATGATATTTACATTACATAAGCACAGGCTCATTTTGGTTTCCACAA AGAATAATAATAAACATAGACAATGGAAATCCTCAGAAATCTTCAAAATC [A/C] TCAAGCTTCACTCCAGTTAGCCGTTTCAATGGACTTTGCTTTCCTATCC TACACCCACATTCTCTCATTGTGACATCAGCACTTTGTTTTTCCTCCGAA GAACCACTGCTCCATACTCTTAGCTCCTGTGGCTTCCAAGAAGATGACAG TGTCCAGCTCCCCCACTGGCATGCCTACGTGGCTACGAGCCTGGGCATGG |
| >rs3112581 |
| TTACATTCTCACAGCTCAGCCTCTTCAAGAGGGAGAGATTCTTTCTTGCA TGTTTCAGCAGCTTTTCTGGATCAAGTCTCATTGGATAGACTTGGGTCCC ATGCCCAGGCTCGTAGCCACGTAGGCATGCCAGTGGGGGAGCTGGACACT GTCATCTTCTTGGAAGCCACAGGAGCTAAGAGTATGGAGCAGTGGTTCTT [C/T] GGAGGAAAAACAAAGTGCTGATGTCACAATGAGAGAATGTGGGTGTAGG ATAGGAAAGCAAAGTCCATTGAAACGGCTAACTGGAGTGAAGCTTGATGA TTTTGAAGATTTCTGAGGATTTCCATTGTCTATGTTTATTATTATTCTTT GTGGAAACCAAAATGAGCCTGTGCTTATGTAATGTAAATATCATAAATAT |
| >rs3112580 |
| TTTTTTTTTTTTTTTATCATATCAGCCAGGCTGTACTCAAGTAGTGGGAA GTTGTCCAGCAGCAGCTCCAAGTTACATTCTCACAGCTCAGCCTCTTCAA GAGGGAGAGATTCTTTCTTGCATGTTTCAGCAGCTTTTCTGGATCAAGTC TCATTGGATAGACTTGGGTCCCATGCCCAGGCTCGTAGCCACGTAGGCAT [A/G] CCAGTGGGGGAGCTGGACACTGTCATCTTCTTGGAAGCCACAGGAGCTA AGAGTATGGAGCAGTGGTTCTTCGGAGGAAAAACAAAGTGCTGATGTCAC AATGAGAGAATGTGGGTGTAGGATAGGAAAGCAAAGTCCATTGAAACGGC TAACTGGAGTGAAGCTTGATGATTTTGAAGATTTCTGAGGATTTCCATTG |
| >rs9931232 |
| TTGAATTAAGATTAAAGATAGAATGTTAATCATACAAGAAGGGCCATTTT CCAGGGCCTGGCATGTTTTACACAAGCATGAAACATATTTAAGAGAACAT GGCCTCCCTGAGGAAGAGATTAAGGATACAAACCTAGGAAACAAAGAAAA TAAAACGATCTACCTCAAAGGGATAAGATTAACCTGTGAGAAGCTCATGC [A/G] GACCTTAAATAAAAGCCTCTACTCCTTATATTTTTAAACAGGATTTTTC TTTTTTTTTTAGAGCACTAATTCCACCTTCAAAATATATTTGAGTTTGTA AGTATACTCAACAAAAATCCTGTCCTTTCTTATTAACCATACTCAAAACA GATTCTCTAAAATAGTTATGACACCATTAACATTTCATGAAATCCAAAAC |
| >rs1123428 |
| CCTGGCTATATTTTATTTGAAGTTATTCTCCTACTTGCAACGACTTTTGA ACCCACAAAACTCTTTCATCCATTCTCCACAGACACAGTACACTTTCATG AGCACTGACATTTGAAAAGGATCCATATACTCACAGAATGTAAAGATAAT ACACATGCATATAATTGATACAAAGGACTCTGGTCATTTGTTAAAGAGAG [A/T] AATGAATGAAACCCTATTATTCACGGTTTGTAAATAAATTTAAAGATAA GCGCTAACTTTCACTTGTTAAGCCTAGAGCCAAGAGTTCGGTGTTTCTTC TAAAGTTTCCCAGATGGTCATTAAAACAAGAGAGGGAAGAAACATACTTT CTTTGAAATCCATCATTTCCCATCCAGAAAGATAATACATGTCATCAAAA |
| >rs3095604 |
| TCATTCACTGCTCTGGGAGAGAAAATGTCCCTATTTTCCTAAAAGGTCCT |
| AGCTGATTAACTTCCCCACTCTACACTCACCCACCCCTCTTTATTTCTTT ATCTTTCTTAAGCTCCATCTTTTTATTTAATTTTTTTTCTCATCGGCTTT ATTGCTTTGAGTTAATCTGAGAGTTTGGCCTTCAGCCTTGGCGAAGGAA [C/G] GGGCTTTATACTAAATGGGAACGTTGTGGAGAGGCCGTGAGATCTGCGC CCTCCCCAGTTTAGTTATCTGCCAGCTACTTTAACCAGAGTGGCGACATT CTGAAATATTTCCTATGACACTCGCTAACAGTTTCATCCCCTGGTCACAC CTTGCTGGCCCAGGGCCTGAGCTTTTTCAGACAAGCATGACATGGGTTTT |
| >rs4784227 |
| GAGTTGATCAGTAAATATTTGCTGAATGAAAGAATACATGAATGAAAAGT CAGAGCCCTATAGGTCAGCATGGACGGCGGTAAAGGAACCTGGCTGAGCC TGAAAGAGAATGTGATCTAAGATTAAATCCAGGATATGCTGGTAAATGTT TAACAGCCAACTCTTTGGGGAGGAAAAAAGTCCCAATTTGTAGTGTTTGC [C/T] GATTATTGTGATGTAAATACTCCCATCATGACCAATTTCAAGCTACCAA CATGCTGACACTGAACTTGGAGTTGGAAGGAGATGAACAGGCATAATCAG GTCTCGTGAGATGGCCCAAGCCGGCCCCAGCACTCCACTGTTATATATGA GGCTAGAATTACTACATAACTGGAATAGCAACTTTCTGGACCATATGCCT |
| >rs12922061 |
| ACTTCATTTTGAGGAAAAGAACAGTTACTACCATTCATTCATTCACTCAT CACACCAATATTACTGAATCCTACCCTGGACCAGCATTGTTATGGTAGAT CTTGATAATATAGTGACAGACAATACCATCCTTCCCACCATTTACATAGT AGACTGCCTGGGGCTCATAGTCTACTCACATCTCAGCACATGAGATAGTC [C/T] CATTATCACATTCTCTTCTATCTCTCTCACAGGCACTCACTCACCAGCC ATGGAGAACTTAATGGGCATTAGGTTCTTAATCCTGCTGGGTATAATTGG AGATAACCACCCTGGCTGACTGAGCACTATACCAGGCTACTCATGGATGA TTTGGTTATTAGACGTAATCAGAGTGGCCCTTGTATGCTAAAAGGAGAC |

**Table 29. Multi-Ethnic cohorts:**

| |
|---|
| >rs4784227 |
| GTAAATATTTGCTGAATGAAAGAATACATGAATGAAAAGTCAGAGCCCTA TAGGTCAGCATGGACGGCGGTAAAGGAACCTGGCTGAGCCTGAAAGAGAA TGTGATCTAAGATTAAATCCAGGATATGCTGGTAAATGTTTAACAGCCAA CTCTTTGGGGAGGAAAAAAGTCCCAATTTGTAGTGTTTGC [C/T] GATTATTGTGATGTAAATACTCCCATCATGACCAATTTCAAGCTACCAA CATGCTGACACTGAACTTGGAGTTGGAAGGAGATGAACAGGCATAATCAG GTCTCGTGAGATGGCCCAAGCCGGCCCCAGCACTCCACTGTTATATATGA GGCTAGAATTACTACATAACTGGAATAGCAACTTTCTGGAC |
| >rs17271951 |
| TAAAATCCAATTGTCATACTCTGGTATTTGTTCTCCAAAAGAATCATCAC TTTCATATTCACTACAGCATGAGATGAGATATTTAATAAATAATAGGACA TCATAATCCAGGGATTAAGTAGTTTCTTTTAGCAAAATTTAATATGAGGG TAGCATATGCCAGCCGTTCCTAGTTGAGCAACAATTTCTG [C/T] GTTGGGAGAGGATAACAAAGCCAAGTATTTTCCCATAAGAAGTTCACAC TCTAAAAGGCCAGGCGCGGTGGCTCACACCTATAATCCCAGCACTTTGGG TGGCTGAGGCAGGCGGATCACTAGGTCAGGAGATCGAGACCATCCTGGCT AAGACGGTGAAACCCCGTCTCTACTAAAAATACAAAAAAAT |

## Claims

1. A method for determining a susceptibility to breast cancer in a human individual, comprising determining the presence or absence of at least one allele of at least one polymorphic marker in a nucleic acid sample obtained from the individual or in a genotype dataset obtained from a nucleic acid sample from the individual, wherein the at least one polymorphic marker is selected from the polymorphic markers rs13387042, and markers in linkage disequilibrium therewith **characterized by** values of r² greater than 0.2, and wherein the presence of the at least one allele is indicative of a susceptibility to breast cancer for the individual.

2. The method according to Claim 1, wherein the at least one polymorphic marker is located within a genomic segment with the sequence as set forth in SEQ ID NO:5.

3. The method according to Claim 1 or Claim 2, wherein the at least one polymorphic marker is selected from the markers set forth in Table 15.

4. The method of any of the preceding claims, wherein the susceptibility conferred by the presence of the at least one allele is increased susceptibility.

5. The method according to Claim 4, wherein the presence of allele A in marker rs13387042 is indicative of increased susceptibility to breast cancer.

6. The method according to any of the preceding claims, further comprising a step of determining the presence or absence of at least one high penetrant genetic factor for breast cancer in a nucleic acid sample obtained from the individual or in a genotype dataset obtained from a nucleic acid sample from the individual.

7. A method of determining risk of developing at least a second primary tumor in an individual previously diagnosed with breast cancer, the method comprising determining the presence or absence of at least one allele of at least one polymorphic marker in a nucleic acid sample obtained from the individual, or in a genotype dataset obtained from a nucleic acid sample from the individual, wherein the at least one polymorphic marker is selected from the polymorphic markers rs13387042, and markers in linkage disequilibrium therewith **characterized by** values of r² greater than 0.2,
and wherein the presence of the at least one allele is indicative of risk of developing at least a second primary tumor.

8. An apparatus for determining a genetic indicator for breast cancer in a human individual, comprising:
a computer readable memory; and
a routine stored on the computer readable memory;
wherein the routine is adapted to be executed on a processor to analyze marker information for at least one human individual with respect to at least one polymorphic marker selected from the markers set rs13387042, and markers in linkage disequilibrium therewith **characterized by** values of r² greater than 0.2, and generate an output based on the marker information, wherein the output comprises an individual risk measure of the at least one marker as a genetic indicator of breast cancer for the human individual.

9. A method of identification of a marker for use in assessing susceptibility to breast cancer, the method comprising:
a. identifying at least one polymorphic marker in linkage disequilibrium, **characterized by** values of r² greater than 0.2, with rs13387042;
b. determining the genotype status of a sample of individuals diagnosed with, or having a susceptibility to, breast cancer; and
c. determining the genotype status of a sample of control individuals;
wherein a significant difference in frequency of at least one allele in at least one polymorphism in individuals diagnosed with, or having a susceptibility to, breast cancer, as compared with the frequency of the at least one allele in the control sample is indicative of the at least one polymorphism being useful for assessing susceptibility to breast cancer.

10. The method according to Claim 9, wherein an increase in frequency of the at least one allele in the at least one polymorphism in individuals diagnosed with, or having a susceptibility to, breast cancer, as compared with the frequency of the at least one allele in the control sample is indicative of the at least one polymorphism being useful for assessing increased susceptibility to breast cancer, and wherein a decrease in frequency of the at least one allele in the at least one polymorphism in individuals diagnosed with, or having a susceptibility to, breast cancer, as compared with the frequency of the at least one allele in the control sample is indicative of the at least one polymorphism being useful for assessing decreased susceptibility to, or protection against, breast cancer.

11. A method of genotyping a nucleic acid sample obtained from a human individual at risk for, or diagnosed with, breast cancer, comprising determining the presence or absence of at least one allele of at least one polymorphic marker in the sample, wherein the at least one marker is selected from the group consisting of the markers rs13387042, and markers in linkage disequilibrium therewith **characterized by** values of r² greater than 0.2, and wherein a determination of the presence or absence of the at least one allele of the at least one polymorphic marker is indicative of a susceptibility of breast cancer for the individual.

12. A method of assessing an individual for probability of response to a breast cancer therapeutic agent, comprising determining the presence or absence of at least one polymorphic marker in a nucleic acid sample obtained from the individual, wherein the at least one polymorphic marker is selected from the group consisting of the polymorphic markers rs13387042, and markers in linkage disequilibrium therewith **characterized by** values of r² greater than 0.2, wherein the presence of the at least one allele of the at least one marker is indicative of a probability of a positive response to the therapeutic agent.

13. A method of predicting prognosis of an individual diagnosed with breast cancer, the method comprising determining the presence or absence of at least one polymorphic marker in a nucleic acid sample obtained from the individual, wherein the at least one polymorphic marker is selected from the group consisting of the polymorphic markers rs13387042, and markers in linkage disequilibrium therewith **characterized by** values of r² greater than 0.2, wherein the presence of the at least one allele is indicative of a worse prognosis of the breast cancer in the individual.

14. A method of monitoring progress of treatment of an individual undergoing treatment for breast cancer, the method comprising determining the presence or absence of at least one allele of at least one polymorphic marker in a nucleic acid sample obtained from the individual, wherein the at least one polymorphic marker is selected from the markers rs13387042, and markers in linkage disequilibrium therewith **characterized by** values of r² greater than 0.2, wherein the presence of the at least one allele is indicative of the treatment outcome of the individual.
